(19) Europäisches Patentamt
European Patent Office
Office européen des brevets

(11) EP 1 243 580 A1

(12) **EUROPEAN PATENT APPLICATION**
published in accordance with Art. 158(3) EPC

(43) Date of publication:
**25.09.2002 Bulletin 2002/39**

(21) Application number: **00985987.7**

(22) Date of filing: **28.12.2000**

(51) Int Cl.[7]: **C07D 213/74**, C07D 277/18, C07D 277/42, C07D 277/60, C07D 277/82, C07D 263/48, C07D 263/58, C07D 233/50, C07D 233/88, C07D 271/10, C07D 285/12, C07D 327/04, C07D 339/06, C07D 279/06, C07D 279/12, C07D 239/46, C07D 235/30, C07D 487/04, C07D 417/04, C07D 285/10

(86) International application number:
**PCT/JP00/09411**

(87) International publication number:
**WO 01/047888 (05.07.2001 Gazette 2001/27)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **28.12.1999 JP 37404099**
**08.08.2000 JP 2000239624**
**01.11.2000 JP 2000334442**

(71) Applicant: **Nissan Chemical Industries, Ltd.**
**Chiyoda-ku, Tokyo 101-0054 (JP)**

(72) Inventors:
- **NIKI, Toshio, Nissan Chem. Ind. Ltd. Ctr. Res. In**
  **Funabashi-shi, Chiba 274-8507 (JP)**
- **MIZUKOSHI, Takashi**
  **Funabashi-shi, Chiba 274-8507 (JP)**
- **TAKAHASHI, Hiroaki**
  **Funabashi-shi, Chiba 274-8507 (JP)**
- **SATOW, Jun**
  **Funabashi-shi, Chiba 274-8507 (JP)**
- **OGURA, Tomoyuki,**
  **Nissan Chemical Industries Ltd.**
  **Tokyo 101-0054 (JP)**
- **YAMAGISHI, Kazuhiro, Nissan Chem. Ind. Ltd.**
  **Minamisaitama-gun, Saitama-349-0218 (JP)**
- **SUZUKI, Hiroyuki,**
  **Nissan Chemical Industries Ltd.**
  **Minamisaitama-gun, Saitama 349-0218 (JP)**
- **HAYASAKA, Fumio,**
  **Nissan Chemical Industries Ltd.**
  **Minamisaitama-gun, Saitama 349-0218 (JP)**

(74) Representative: **Hartz, Nikolai F., Dr. et al**
**Wächtershäuser & Hartz,**
**Patentanwälte,**
**Tal 29**
**80331 München (DE)**

# (54) HETEROCYCLIC IMINO COMPOUNDS AND FUNGICIDES AND INSECTICIDES FOR AGRICULTURAL AND HORTICULTURAL USE

(57) A heterocyclic imino compound of the formula (1) and an agrochemically acceptable salt thereof; and an agricultural chemical, fungicide and insecticide containing at least one member selected from the group of such compounds as an active ingredient:

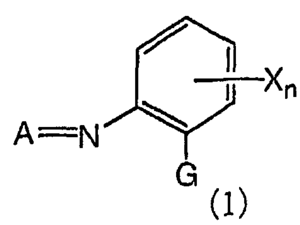
(1)

wherein G is

EP 1 243 580 A1

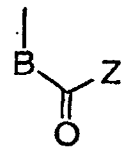

$G^1$

or the like, A is a 3- to 13-membered, mono-, di- or tricyclic ring which contains at least one hetero atom selected from among oxygen atoms, sulfur atoms and nitrogen atoms, which is composed of from 3 to 13 atoms arbitrarily selected from among carbon atoms, oxygen atoms, sulfur atoms and nitrogen atoms and which may be substituted by from 1 to 13 Ys, provided that when A is a quinolone ring, the nitrogen atom in the quinolone ring is present at the α-position to the imino bond,

Z is $-OR^1$ or the like,

B is $-CH_2-$ or the like,

Y is $Y'-D-(CH_2)_p-$ or the like,

D is a single bond or the like,

X is halogen or the like, and

$R^1$ is a hydrogen atom, $C_1$-$C_6$ alkyl, $C_1$-$C_6$ haloalkyl or the like.

**Description**

TECHNICAL FIELD

The present invention relates to novel heterocyclic imino compounds and their salts, and plant disease and plant insect pest controlling agents containing at least one member selected from such heterocyclic imino compounds and their salts, as an active ingredient.

BACKGROUND ART

Conventional fungicides and insecticides for agricultural and horticultural use are not satisfactory from the viewpoint of the effects or residual effects in view of an increase of resistant strains or narrowness of spectra of such conventional agents. Accordingly, development of a plant disease and plant insect pest controlling agent which is highly effective at a low dose and which is highly safe to desired crop plants, is desired.

Meanwhile, certain imino compounds are known in publication of an international patent application (WO-95/27693) and publication of an European patent application (EP-254426), and their application as a fungicide for agricultural and horticultural use, is disclosed. Whereas, the heterocyclic imino compounds as the compounds of the present invention, are novel compounds not disclosed in literatures.

DISCLOSURE OF THE INVENTION

Under these circumstances, the present inventors have conducted various studies to find out an excellent fungicide and insecticide and as a result, have found that the novel heterocyclic imino compounds and their salts have remarkable activities as plant disease and plant insect pest controlling agents and are safe to desired crop plants, and thus, they have arrived at the present invention.

Namely, the present invention relates to the following (1) to (7).

(1) A heterocyclic imino compound of the formula (1) and an agrochemically acceptable salt thereof:

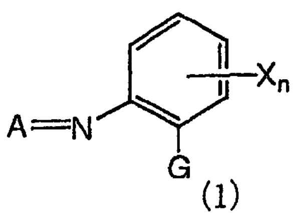

(1)

wherein G is a group selected from $G^1$ to $G^{14}$:

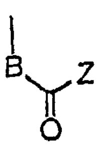

$G^1$

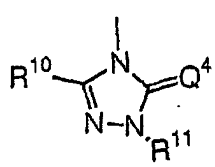

$G^2$

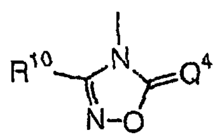

$G^3$

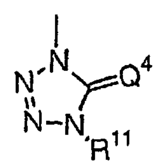

$G^4$.

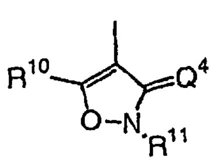

$G^5$

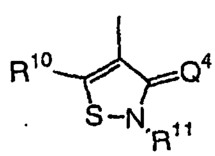

$G^6$.

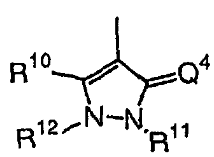

$G^7$

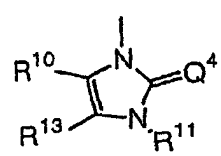

$G^8$

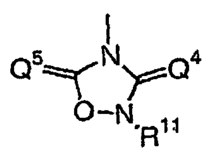

G9

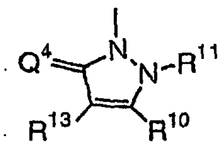

G10

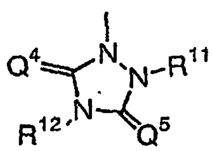

G11.

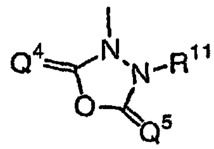

G12:

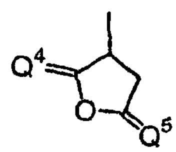

G13

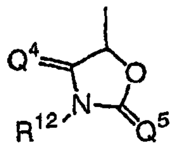

G14

A is a 3- to 13-membered, mono-, di- or tri-cyclic ring which contains at least one hetero atom selected from among oxygen atoms, sulfur atoms and nitrogen atoms, which is composed of from 3 to 13 atoms arbitrarily selected from among carbon atoms, oxygen atoms, sulfur atoms and nitrogen atoms and which is substituted by from 0 to 13 Ys, provided that when A is a quinolone ring, the nitrogen atom in the quinolone ring is present at the $\alpha$-position to the imino bond,

Z is $-OR^1$, $-SR^1$ or $-NR^2R^3$,

B is $-CH_2-$, $-C(=CH-OR^4)-$ or $-C(=N-OR^4)-$,

Y is $Y'-D-(CH_2)_p-$ or $=Q^1$ (provided that in the case of 2 or more Ys, they may be the same or different), or 2 Ys substituted on the same carbon atom of A, may, together with the carbon atom, form a 3- to 7-membered ring which may contain from 1 to 3 oxygen atoms, nitrogen atoms or sulfur atoms,

provided that when Y is a substituent on a carbon atom, Y may be a hydrogen atom,

D is a single bond, $-NR^5-$, $-C(=Q^2)-$, $-C(=Q^2)-C(=Q^3)-$, $-CR^6=N-$, $-N=CR^6-$, $-CR^6=N-N=CR^6-$, $-N=CR^6-O-N=CR^6-$, $-CR^6=N-O-$, $-CR^6=N-O-CR^6=N-O-$, $-O-N=CR^6-CR^6=N-O-$, $-CR^6=N-NR^5-$ or $-O-N=CR^6-CR^6=N-NR^5-$,

$Q^1$, $Q^2$ and $Q^3$, each independently is $=O$, $=S$, $=N-R^7$ or $=C(R^8)(R^9)$,

$Q^4$ and $Q^5$, each independently is $=O$ or $=S$,

X is halogen, $C_1$-$C_6$ alkyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, ($C_1$-$C_6$ alkyl)$_2$ amino, $NO_2$, CN, formyl, OH, SH, $NU^1U^2$, $C_1$-$C_6$ alkoxycarbonyl, $C_1$-$C_6$ alkylcarbonyl, $C_1$-$C_6$ haloalkylcarbonyl, phenylcarbonyl which may be substituted by $R^a$, or $C_1$-$C_6$ alkylcarbonyloxy (provided that in the case of two or more Xs substituted, they may be the same or different),

$R^1$, $R^2$ and $R^4$, each independently is a hydrogen atom, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_1$-$C_6$ alkylsulfenyl $C_1$-$C_6$ alkyl, phenyl $C_1$-$C_6$ alkyl which may be substituted by $R^a$, or heteroaryl $C_1$-$C_6$ alkyl which may be substituted by $R^a$,

$R^3$ is a hydrogen atom, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_1$-$C_6$ alkylsulfenyl $C_1$-$C_6$ alkyl, phenyl which may be substituted by $R^a$, phenyl $C_1$-$C_6$ alkyl which may be substituted by $R^a$, or heteroaryl $C_1$-$C_6$ alkyl which may be substituted by $R^a$,

$R^5$ and $R^6$, each independently is halogen, $C_1$-$C_6$ alkyl, $C_1$-$C_6$ haloalkyl, $C_3$-$C_6$ cycloalkyl, $C_1$-$C_6$ alkoxy, $C_1$-$C_6$ alkoxy $C_1$-$C_6$ alkyl, $C_1$-$C_6$ alkylsulfenyl $C_1$-$C_6$ alkyl, $C_1$-$C_6$ haloalkoxy, $C_1$-$C_6$ alkylsulfenyl, $C_1$-$C_6$ alkylsulfinyl, $C_1$-$C_6$ alkylsulfonyl, $C_1$-$C_6$ haloalkylsulfenyl, $C_1$-$C_6$ haloalkylsulfinyl, $C_1$-$C_6$ haloalkylsulfonyl, $C_2$-$C_6$ alkenyl, $C_2$-$C_6$ haloalkenyl, $C_2$-$C_6$ alkenyloxy, $C_2$-$C_6$ haloalkenyloxy, $C_2$-$C_6$ alkenylsulfenyl, $C_2$-$C_6$ alkenylsulfinyl, $C_2$-$C_6$ alkenylsulfonyl, $C_2$-$C_6$ haloalkenylsulfenyl, $C_2$-$C_6$ haloalkenylsulfinyl, $C_2$-$C_6$ haloalkenylsulfonyl, $C_2$-$C_6$ alkynyl, $C_2$-$C_6$ haloalkynyl, $C_2$-$C_6$ alkynyloxy, $C_2$-$C_6$ haloalkynyloxy, $C_2$-$C_6$ alkynylsulfenyl, $C_2$-$C_6$ alkynylsulfinyl, $C_2$-$C_6$ alkylsulfonyl, $C_2$-$C_6$ haloalkynylsulfenyl, $C_2$-$C_6$ haloalkynylsulfinyl, $C_2$-$C_6$ haloalkynylsulfonyl, $NO_2$, CN, formyl, OH, SH, SCN, $C_1$-$C_6$ alkoxycarbonyl, $C_1$-$C_6$ haloalkoxycarbonyl, $C_1$-$C_6$ alkylcarbonyl, $C_1$-$C_6$ haloalkylcarbonyl, $C_1$-$C_6$ alkylcarbonyloxy, phenyl which may be substituted by $R^a$, phenyl $C_1$-$C_6$ alkyl which may be substituted by $R^a$, phenylsulfonyl which may be substituted $R^a$, phenyl $C_1$-$C_6$ alkylsulfonyl which may be substituted by $R^a$, heteroaryl which may be substituted by $R^a$, heteroaryl $C_1$-$C_6$ alkyl which may be substituted by $R^a$, heteroarylsulfonyl which may be substituted by $R^a$, phenylcarbonyl which may be substituted by $R^a$, phenyl $C_1$-$C_6$ alkylcarbonyl which may be substituted by $R^a$, heteroarylcarbonyl which may be substituted by $R^a$, or $-NU^1U^2$, provided that $R^6$ may be a hydrogen atom,

$R^7$ is hydrogen atom, $C_1$-$C_6$ alkyl, $C_1$-$C_6$ haloalkyl, $C_3$-$C_6$ cycloalkyl, $C_1$-$C_6$ alkoxy, $C_1$-$C_6$ alkoxy $C_1$-$C_6$ alkyl,

$C_1$-$C_6$ alkylsulfenyl $C_1$-$C_6$ alkyl, $C_1$-$C_6$ alkylsulfonyl, $C_1$-$C_6$ haloalkylsulfonyl, $C_1$-$C_6$ alkylcarbonyl, $C_1$-$C_6$ haloalkylcarbonyl, phenyl which may be substituted by $R^a$, phenoxy which may be substituted by $R^a$, phenyl $C_1$-$C_6$ alkyl which may be substituted by $R^a$, phenyl $C_1$-$C_6$ alkoxy which may be substituted by $R^a$, phenylsulfonyl which may be substituted by $R^a$, phenyl $C_1$-$C_6$ alkylsulfonyl which may be substituted by $R^a$, heteroaryl which may be substituted by $R^a$, heteroaryloxy which may be substituted by $R^a$, heteroaryl $C_1$-$C_6$ alkyl which may be substituted by $R^a$, heteroarylsulfonyl which may be substituted by $R^a$, phenylcarbonyl which may be substituted by $R^a$, phenoxycarbonyl which may be substituted by $R^a$, phenyl $C_1$-$C_6$ alkylcarbonyl which may be substituted by $R^a$, heteroarylcarbonyl which may be substituted by Ra, heteroaryloxycarbonyl which may be substituted by $R^a$, or heteroaryl $C_1$-$C_6$ alkylcarbonyl which may be substituted by $R^a$,

$R^8$ and $R^9$, each independently is a hydrogen atom, halogen, $C_1$-$C_6$ alkyl, $C_1$-$C_6$ alkoxy, $C_1$-$C_6$ alkylsulfenyl, $C_2$-$C_6$ alkenyl, $NO_2$, CN, formyl, or $C_1$-$C_6$ alkoxycarbonyl,

$R^{10}$ is a hydrogen atom, halogen, $R^{14}$, -$OR^{14}$, -$SR^{14}$, -$SOR^{14}$, or -$SO_2R^{14}$,

$R^{11}$ is a hydrogen atom, $R^{14}$ or CN,

$R^{12}$ is a hydrogen atom or $R^{14}$,

$R^{13}$ is a hydrogen atom, halogen, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_2$-$C_6$ alkenyl, or $C_2$-$C_6$ alkynyl,

$R^{14}$ is $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_3$-$C_6$ cycloalkyl, $C_1$-$C_6$ alkylcarbonyl, or $C_1$-$C_6$ alkoxycarbonyl,

Y' is halogen, $C_1$-$C_{12}$ alkyl which may be substituted by $R^b$, $C_3$-$C_6$ cycloalkyl which may be substituted by $R^b$, $C_2$-$C_{12}$ alkenyl which may be substituted by $R^b$, $C_2$-$C_{12}$ alkynyl which may be substituted by $R^b$, $C_1$-$C_{12}$ alkoxy which may be substituted by $R^b$, $C_1$-$C_6$ alkoxy $C_1$-$C_6$ alkoxy which may be substituted by $R^b$, $C_2$-$C_6$ alkenyloxy which may be substituted by $R^b$, $C_2$-$C_6$ alkynyloxy which may be substituted by $R^b$, $C_1$-$C_6$ alkylsulfenyl which may be substituted by $R^b$, $C_2$-$C_6$ alkenylsulfenyl which may be substituted by $R^b$, $C_2$-$C_6$ alkynylsulfenyl which may be substituted by $R^b$, $C_1$-$C_6$ alkylsulfinyl which may be substituted by $R^b$, $C_2$-$C_6$ alkenylsulfinyl which may be substituted by $R^b$, $C_2$-$C_6$ alkynylsulfinyl which may be substituted by $R^b$, $C_1$-$C_6$ alkylsulfonyl which may be substituted by $R^b$, $C_2$-$C_6$ alkenylsulfonyl which may be substituted by $R^b$, $C_2$-$C_6$ alkynylsulfonyl which may be substituted by $R^b$, $C_1$-$C_6$ alkoxycarbonyl which may be substituted by $R^b$, $C_1$-$C_6$ alkylcarbonyl which may be substituted by $R^b$, $C_1$-$C_6$ alkylcarbonyloxy which may be substituted by $R^b$, phenyl which may be substituted by $R^c$, phenoxy which may be substituted by $R^c$, phenyl $C_1$-$C_6$ alkyl which may be substituted by $R^c$, phenyl $C_1$-$C_6$ alkoxy which may be substituted by $R^c$, phenylsulfonyl which may be substituted by $R^c$, phenylsulfinyl which may be substituted by $R^c$, phenylsulfenyl which may be substituted by $R^c$, phenyl $C_1$-$C_6$ alkylsulfenyl which may be substituted by $R^c$, phenyl $C_1$-$C_6$ alkylsulfinyl which may be substituted by $R^c$, phenyl $C_1$-$C_6$ alkylsulfonyl which may be substituted by $R^c$, heteroaryl which may be substituted by $R^c$, heteroaryloxy which may be substituted by $R^c$, heteroaryl $C_1$-$C_6$ alkyl which may be substituted by $R^c$, heteroaryl $C_1$-$C_6$ alkoxy which may be substituted by $R^c$, heteroarylsulfinyl which may be substituted by $R^c$, heteroarylsulfenyl which may be substituted by $R^c$, heteroarylsulfonyl which may be substituted by $R^c$, heteroaryl $C_1$-$C_6$ alkylsulfenyl which may be substituted by $R^c$, heteroaryl $C_1$-$C_6$ alkylsulfinyl which may be substituted by $R^c$, heteroaryl $C_1$-$C_6$ alkylsulfonyl which may be substituted by $R^c$, phenylcarbonyl which may be substituted by $R^c$, phenylcarbonylxoy which may be substituted by $R^c$, phenoxycarbonyl which may be substituted by $R^c$, phenyl $C_1$-$C_6$ alkylcarbonyl which may be substituted by $R^c$, phenyl $C_1$-$C_6$ alkylcarbonyloxy which may be substituted by $R^c$, heteroarylcarbonyl which may be substituted by $R^c$, heteroarylcarbonyloxy which may be substituted by $R^c$, heteroaryloxycarbonyl which may be substituted by $R^c$, heteroaryl $C_1$-$C_6$ alkylcarbonyl which may be substituted by $R^c$, heteroaryl $C_1$-$C_6$ alkylcarbonyloxy which may be substituted by $R^c$, $NO_2$, CN, formyl, or naphthyl,

$R^a$ is halogen, $C_1$-$C_6$ alkyl, $C_1$-$C_6$ haloalkyl, $C_3$-$C_6$ cycloalkyl, $C_1$-$C_6$ alkoxy, $C_1$-$C_6$ alkoxy $C_1$-$C_6$ alkyl, $C_1$-$C_6$ alkylsulfenyl $C_1$-$C_6$ alkyl, $C_1$-$C_6$ haloalkoxy, $C_1$-$C_6$ alkylsulfenyl, $C_1$-$C_6$ alkylsulfinyl, $C_1$-$C_6$ alkylsulfonyl, $C_1$-$C_6$ haloalkylsulfenyl, $C_1$-$C_6$ haloalkylsulfinyl, $C_1$-$C_6$ haloalkylsulfonyl, $C_2$-$C_6$ alkenyl, $C_2$-$C_6$ haloalkenyl, $C_2$-$C_6$ alkenyloxy, $C_2$-$C_6$ haloalkenyloxy, $C_2$-$C_6$ alkenylsulfenyl, $C_2$-$C_6$ alkenylsulfinyl, $C_2$-$C_6$ alkenylsulfonyl, $C_2$-$C_6$ haloalkenylsulfenyl, $C_2$-$C_6$ haloalkenylsulfinyl, $C_2$-$C_6$ haloalkenylsulfonyl, $C_2$-$C_6$ alkynyl, $C_2$-$C_6$ haloalkynyl, $C_2$-$C_6$ alkynyloxy, $C_2$-$C_6$ haloalkynyloxy, $C_2$-$C_6$ alkynylsulfenyl, $C_2$-$C_6$ alkynylsulfinyl, $C_2$-$C_6$ alkynylsulfonyl, $C_2$-$C_6$ haloalkynylsulfenyl, $C_2$-$C_6$ haloalkynylsulfinyl, $C_2$-$C_6$ haloalkynylsulfonyl, $NO_2$, CN, formyl, SH, OH, SCN, $C_1$-$C_6$ alkoxycarbonyl, $C_1$-$C_6$ alkylcarbonyl, $C_1$-$C_6$ haloalkylcarbonyl, $C_1$-$C_6$ alkylcarbonyloxy, phenyl, or -$NU^1U^2$, the number of $R^a$ for substitution being from 1 to 5 (provided that in the case of two or more $R^a$, they may be the same or different),

$R^b$ is halogen, $C_3$-$C_6$ cycloalkyl, $C_1$-$C_6$ alkoxy, $C_1$-$C_6$ alkoxy $C_1$-$C_6$ alkoxy, $C_1$-$C_6$ alkylsulfenyl $C_1$-$C_6$ alkoxy, $C_1$-$C_6$ haloalkoxy, $C_1$-$C_6$ alkylsulfenyl, $C_1$-$C_6$ alkylsulfinyl, $C_1$-$C_6$ alkylsulfonyl, $C_1$-$C_6$ haloalkylsulfenyl, $C_1$-$C_6$ haloalkylsulfinyl, $C_1$-$C_6$ haloalkylsulfonyl, $C_2$-$C_6$ alkenyloxy, $C_2$-$C_6$ haloalkenyloxy, $C_2$-$C_6$ alkenylsulfenyl, $C_2$-$C_6$ alkenylsulfinyl, $C_2$-$C_6$ alkenylsulfonyl, $C_2$-$C_6$ haloalkenylsulfenyl, $C_2$-$C_6$ haloalkenylsulfinyl, $C_2$-$C_6$ haloalkenylsulfonyl, $C_2$-$C_6$ alkynyloxy, $C_2$-$C_6$ haloalkynyloxy, $C_2$-$C_6$ alkynylsulfenyl, $C_2$-$C_6$ alkynylsulfinyl, $C_2$-$C_6$ alkynylsulfonyl, $C_2$-$C_6$ haloalkynylsulfenyl, $C_2$-$C_6$ haloalkynylsulfinyl, $C_2$-$C_6$ haloalkynylsulfonyl, $NO_2$, CN, formyl, OH, SH, SCN, $C_1$-$C_6$ alkoxycarbonyl, $C_1$-$C_6$ alkylcarbonyl, $C_1$-$C_6$ haloalkylcarbonyl, $C_1$-$C_6$ alkylcarbonyloxy, phenyl which may be substituted by $R^a$, phenoxy which

may be substituted by $R^a$, phenyl $C_1$-$C_6$ alkoxy which may be substituted by $R^a$, phenylsulfonyl which may be substituted by $R^a$, phenyl $C_1$-$C_6$ alkylsulfonyl which may be substituted by $R^a$, heteroaryl which may be substituted by $R^a$, heteroaryloxy which may be substituted by $R^a$, heteroarylsulfonyl which may be substituted by $R^a$, phenylcarbonyl which may be substituted by $R^a$, phenoxycarbonyl which may be substituted by $R^a$, phenyl $C_1$-$C_6$ alkylcarbonyl which may be substituted by $R^a$, heteroarylcarbonyl which may be substituted by $R^a$, heteroaryloxycarbonyl which may be substituted by $R^a$, or heteroaryl $C_1$-$C_6$ alkylcarbonyl which may be substituted by $R^a$, or $-NU^1U^2$, or a 3- to 7-membered ring which may contain from 1 to 4 hetero atoms selected from among oxygen atoms, nitrogen atoms and sulfur atoms, the number of $R^b$ for substitution being from 1 to 8 (provided that in the case of two or more $R^b$, they may be the same or different),

$R^c$ is halogen, $C_1$-$C_{12}$ alkyl which may be substituted by $R^b$, $C_3$-$C_6$ cycloalkyl which may be substituted by $R^b$, $C_2$-$C_{12}$ alkenyl which may be substituted by $R^b$, $C_2$-$C_{12}$ alkynyl which may be substituted by $R^b$, $C_1$-$C_{12}$ alkoxy which may be substituted by $R^b$, $C_1$-$C_6$ alkoxy $C_1$-$C_6$ alkoxy which may be substituted by $R^b$, $C_2$-$C_6$ alkenyloxy which may be substituted by $R^b$, $C_2$-$C_6$ alkynyloxy which may be substituted by $R^b$, $C_1$-$C_6$ alkylsulfenyl which may be substituted by $R^b$, $C_2$-$C_6$ alkenylsulfenyl which may be substituted by $R^b$, $C_2$-$C_6$ alkynylsulfenyl which may be substituted by $R^b$, $C_1$-$C_6$ alkylsulfinyl which may be substituted by $R^b$, $C_2$-$C_6$ alkenylsulfinyl which may be substituted by $R^b$, $C_2$-$C_6$ alkynylsulfinyl which may be substituted by $R^b$, $C_1$-$C_6$ alkylsulfonyl which may be substituted by $R^b$, $C_2$-$C_6$ alkenylsulfonyl which may be substituted by $R^b$, $C_2$-$C_6$ alkynylsulfonyl which may be substituted by $R^b$, $C_1$-$C_6$ alkoxycarbonyl which may be substituted by $R^b$, $C_1$-$C_6$ alkylcarbonyl which may be substituted by $R^b$, $C_1$-$C_6$ alkylcarbonyloxy which may be substituted by $R^b$, $NO_2$, CN, formyl, OH, SH, SCN, $C_1$-$C_6$ alkoxycarbonyl, $C_1$-$C_6$ alkylcarbonyl, $C_1$-$C_6$ haloalkylcarbonyl, $C_1$-$C_6$ alkylcarbonyloxy, phenyl which may be substituted by $R^a$, phenoxy which may be substituted by $R^a$, phenyl $C_1$-$C_6$ alkyl which may be substituted by $R^a$, phenyl $C_1$-$C_6$ alkoxy which may be substituted by $R^a$, phenylsulfonyl which may be substituted by $R^a$, phenylsulfinyl which may be substituted by $R^a$, phenylsulfenyl which may be substituted by $R^a$, phenyl $C_1$-$C_6$ alkylsulfenyl which may be substituted by $R^a$, phenyl $C_1$-$C_6$ alkylsulfinyl which may be substituted by $R^a$, phenyl $C_1$-$C_6$ alkylsulfonyl which may be substituted by $R^a$, heteroaryl which may be substituted by $R^a$, heteroaryloxy which may be substituted by $R^a$, heteroaryl $C_1$-$C_6$ alkyl which may be substituted by $R^a$, heteroaryl $C_1$-$C_6$ alkoxy which may be substituted by $R^a$, heteroarylsulfinyl which may be substituted by $R^a$, heteroarylsulfenyl which may be substituted by $R^a$, heteroarylsulfonyl which may be substituted by $R^a$, heteroaryl $C_1$-$C_6$ alkylsulfenyl which may be substituted by $R^a$, heteroaryl $C_1$-$C_6$ alkylsulfinyl which may be substituted by $R^a$, heteroaryl $C_1$-$C_6$ alkylsulfonyl which may be substituted by $R^a$, phenylcarbonyl which may be substituted by $R^a$, phenylcarbonyloxy which may be substituted by $R^a$, phenoxycarbonyl which may be substituted by $R^a$, phenyl $C_1$-$C_6$ alkylcarbonyl which may be substituted by $R^a$, phenyl $C_1$-$C_6$ alkylcarbonyloxy which may be substituted by $R^a$, heteroarylcarbonyl which may be substituted by $R^a$, heteroarylcarbonyloxy which may be substituted by $R^a$, heteroaryloxycarbonyl which may be substituted by $R^a$, heteroaryl $C_1$-$C_6$ alkylcarbonyl which may be substituted by $R^a$, heteroaryl $C_1$-$C_6$ alkylcarbonyloxy which may be substituted by $R^a$, or $-NU^1U^2$, the number of $R^c$ for substitution being from 1 to 5 (provided that in the case of two or more $R^c$, they may be the same or different),

$U^1$ and $U^2$, each independently is a hydrogen atom, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_1$-$C_6$ alkylsulfenyl $C_1$-$C_6$ alkyl, formyl, $C_1$-$C_6$ alkylsulfonyl, $C_1$-$C_6$ haloalkylsulfonyl, $C_1$-$C_6$ alkoxycarbonyl, $C_1$-$C_6$ alkylcarbonyl, or $C_1$-$C_6$ haloalkylcarbonyl, or $U^1$ and $U^2$ together form a 3- to 7-membered ring which may contain from 1 to 4 hetero atoms selected from among oxygen atoms, nitrogen atoms and sulfur atoms,

n represents the number of substituents and is from 0 to 4, and

p represents the number of repeating units and is from 0 to 2.

(2) A hydrochloride, a hydrobromide, a hydroiodide, a formate, an acetate or an oxalate of the heterocyclic imino compound according to (1).

(3) The heterocyclic imino compound and an agrochemically acceptable salt thereof, according to (1), wherein A is

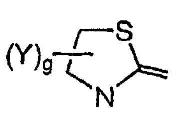 , 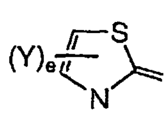 , 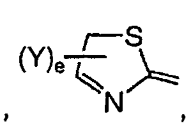 , 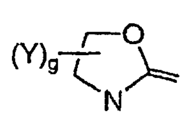 , 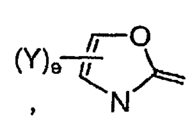 ,

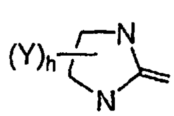 , 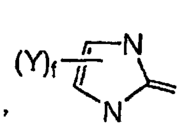 , 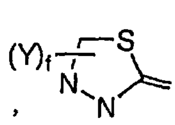 , 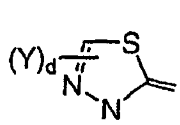 , 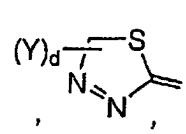 ,

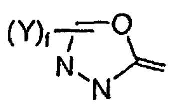 , 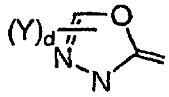 , 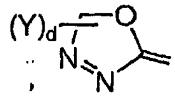 , 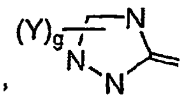 , 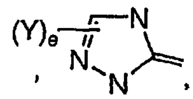 ,

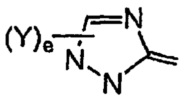 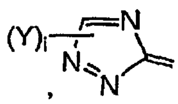 , 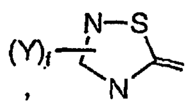 , 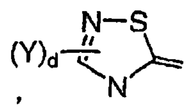 , 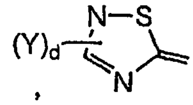 ,

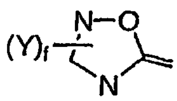 , 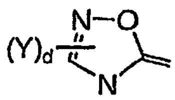 , 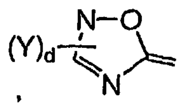 , 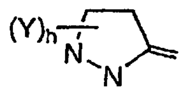 , 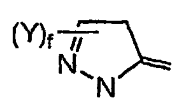 ,

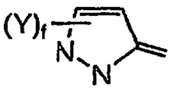 , 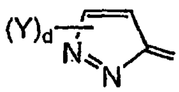 , 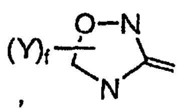 , 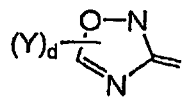 , 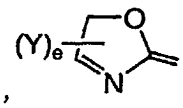 ,

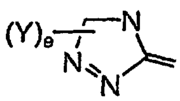 , 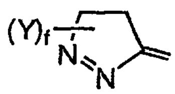 , 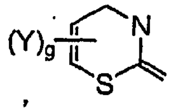 , 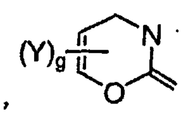 , 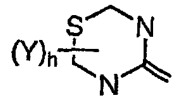 ,

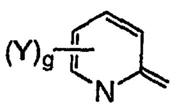 , 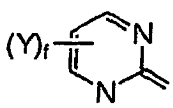 , 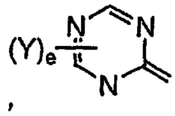 , 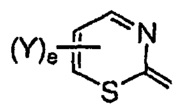 , 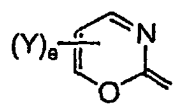 ,

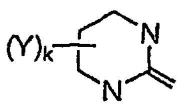 , 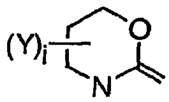 , 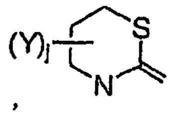 , 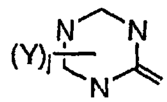 , 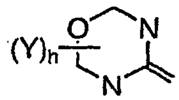 ,

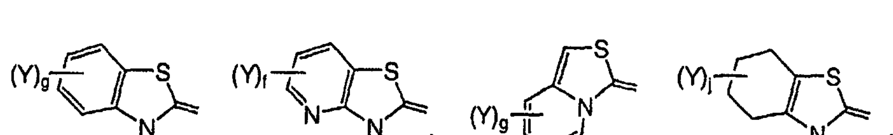

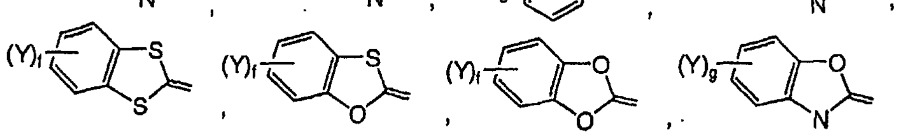

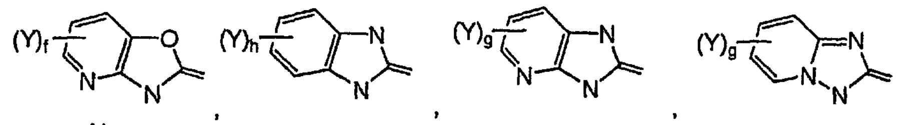
, , , ,

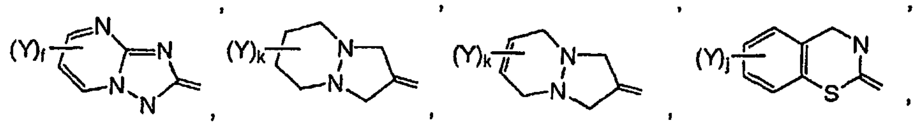
, , , ,

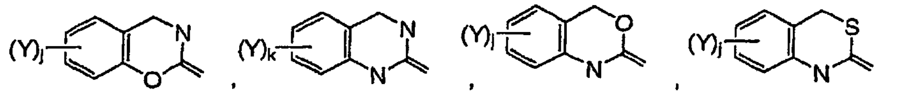
, , , ,

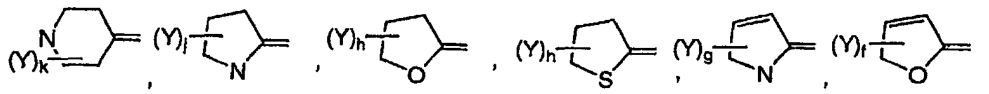
, , , , , ,

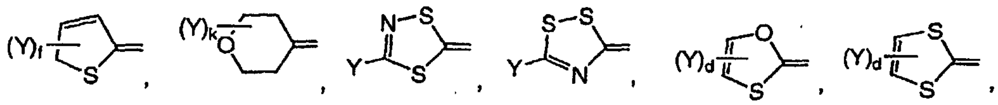
, , , , , ,

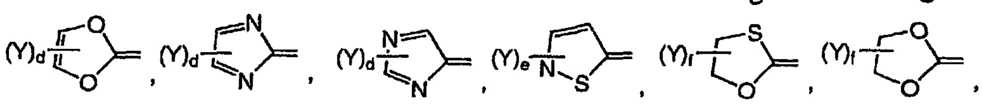
, , , , , ,

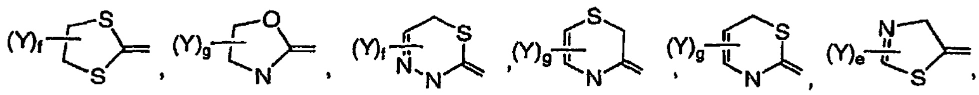
, , , , , ,

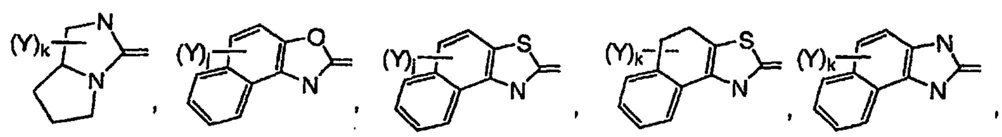
, , , , ,

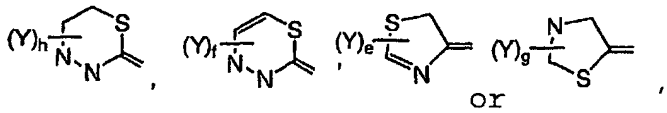
, , or ,

d represents the number of substituents and is from 0 to 2,
e represents the number of substituents and is from 0 to 3,
f represents the number of substituents and is from 0 to 4,
g represents the number of substituents and is from 0 to 5,
h represents the number of substituents and is from 0 to 6,
i represents the number of substituents and is from 0 to 1,
j represents the number of substituents and is from 0 to 7, and
k represents the number of substituents and is from 0 to 8.

(4) The heterocyclic imino compound according to any one of (1) to (3), wherein G is $G^1$.

(5) An agricultural chemical containing at least one member selected from the heterocyclic imino compound and an agrochemically acceptable salt thereof according to any one of (1) to (4), as an active ingredient.

(6) A fungicide containing at least one member selected from the heterocyclic imino compound and an agrochemically acceptable salt thereof according to any one of (1) to (4), as an active ingredient.

(7) An insecticide containing at least one member selected from the heterocyclic imino compound and an agrochemically acceptable salt thereof according to any one of (1) to (4), as an active ingredient.

However, when the present compounds have stereo isomers, geometrical isomers, tautomeric isomers and diastereomers, the present invention covers all of the respective isomers and their mixtures.

BEST MODE FOR CARRYING OUT THE INVENTION

Each substituent in the compound of the formula (1) of the present invention will be exemplified below.
Here, abbreviations have the following meanings respectively.
Me represents a methyl group, Et an ethyl group, Pr a propyl group, Bu a butyl group, Pen a pentyl group, Hex a hexyl group, Hep a heptyl group, Oct an octyl group, Non a nonyl group, Dec a decyl group, Undec an undecanyl group, Dodec a dodecyl group, n normal, i iso, s secondary, t tertiary and c cyclo, respectively, and Ph represents a phenyl group, and in the representation of a phenyl group, e.g. 2-Cl-Ph represents a 2-chlorophenyl group, and 2-MeO-3-Me-Ph represents a 2-methoxy-3-methylphenyl group.
The $C_1$-$C_6$ alkyl in the definitions of $R^1$, $R^2$, $R^3$, $R^4$, $R^5$, $R^6$, $R^7$, $R^8$, $R^9$, $R^{13}$, $R^{14}$, $R^a$, X, $U^1$ and $U^2$, may, for example, be a linear or branched alkyl, such as, methyl, ethyl, n-propyl, i-propyl, n-butyl, i-butyl, t-butyl, s-butyl, n-pentyl, n-hexyl, 2-ethylpropyl, 2,2-dimethylpropyl, 1,2-dimethylpropyl, 1,1,2-trimethylpropyl, 1,2,2-trimethylpropyl, 1-ethyl-1-methylpropyl, 1-ethyl-2-methylpropyl, 1-methylbutyl, 2-methylbutyl, 3-methylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1-methylpentyl, 2-methylpentyl, 3-methylpentyl and 4-methylpentyl.
The halogen atom in the definitions of $R^5$, $R^6$, $R^8$, $R^9$, $R^{10}$, $R^{13}$, $R^a$, $R^b$, $R^c$, X and Y', may, for example, be a fluorine atom, a chlorine atom, a bromine atom and an iodine atom.
The $C_1$-$C_6$ haloalkyl in the definitions of $R^1$, $R^2$, $R^3$, $R^4$, $R^5$, $R^6$, $R^7$, $R^{13}$, $R^{14}$, $R^a$, X, $U^1$ and $U^2$, may, for example, be a linear or branched haloalkyl, such as fluoromethyl, chloromethyl, bromomethyl, iodomethyl, difluoromethyl, chlorodifluoromethyl, bromodifluoromethyl, trifluoromethyl, dichloromethyl, trichloromethyl, 1-chloroethyl, 1-bromoethyl, 1-iodoethyl, 1-fluoroethyl, 2-chloroethyl, 2-bromoethyl, 2-iodoethyl, 2-fluoroethyl, 2,2-difluoroethyl, 2,2,2-trifluoroethyl, 2,2,2-trichloroethyl, pentafluoroethyl, 2,2,2-trifluoro-1-chloroethyl, 3-fluoropropyl, 3-chloropropyl, 1-fluoro-i-propyl, 1-chloro-i-propyl, heptafluoropropyl, 1,1,2,2,3,3-hexafluoro-n-propyl, 4-chlorobutyl, 4-fluorobutyl, 5-chloropentyl, 5-fluoropentyl, 6-chlorohexyl and 6-fluorohexyl.
The $C_3$-$C_6$ cycloalkyl in the definitions of $R^1$, $R^2$, $R^3$, $R^4$, $R^5$, $R^6$, $R^7$, $R^{13}$, $R^{14}$, $R^a$, $R^b$, $U^1$ and $U^2$, may, for example, be cyclopropyl, cyclobutyl, cyclopentyl and cyclohexyl.
The $C_1$-$C_6$ alkoxy in the definitions of $R^5$, $R^6$, $R^7$, $R^8$, $R^9$, $R^a$, $R^b$ and X, may, for example, be a linear or branched alkoxy, such as methoxy, ethoxy, n-propoxy, i-propoxy, n-butoxy, i-butoxy, s-butoxy, t-butoxy, n-pentyloxy, n-hexyloxy, 1,1-dimethylpropoxy, 1,2-dimethylpropoxy, 2,2-dimethylpropoxy, 1-ethylpropoxy, 1,1,2-trimethylpropoxy, 1,2,2-trimethylpropoxy, 1-ethyl-1-methylpropoxy, 1-ethyl-2-methylpropoxy, 1-methylbutoxy, 2-methylbutoxy, 3-methylbutoxy, 1-ethylbutoxy, 2-ethylbutoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-methylpentyloxy, 2-methylpentyloxy, 3-methylpentyloxy and 4-methylpentyloxy.
The $C_1$-$C_6$ haloalkoxy in the definitions of $R^5$, $R^6$, $R^a$, $R^b$ and X, may, for example, be a $C_1$-$C_6$ linear or branched haloalkoxy, such as fluoromethoxy, chloromethoxy, bromomethoxy, iodomethoxy, dichloromethoxy, trichloromethoxy, difluoromethoxy, trifluoromethoxy, chlorodifluoromethoxy, bromodifluoromethoxy, dichlorofluoromethoxy, 1-chloroethoxy, 1-bromoethoxy, 1-iodoethoxy, 1-fluoroethoxy, 2-chloroethoxy, 2-bromoethoxy, 2-iodoethoxy, 2-fluoroethoxy, 2,2-difluoroethoxy, 2,2,2-trifluoroethoxy, 2,2,2-trichloroethoxy, pentafluoroethoxy, 2,2,2-trifluoro-1-chloroethoxy, 1,1,2,2-tetrafluoroethoxy, 3-bromopropoxy, 1-fluoro-i-propoxy, 1-chloro-i-propoxy, 3-fluoropropoxy, 3-chloropropoxy, heptafluoropropoxy, 1,1,2,2,3,3-hexafluoropropoxy, 4-chlorobutoxy, 4-fluorobutoxy, 5-chloropentyloxy, 5-fluoropentyloxy, 6-chlorohexyloxy and 6-fluorohexyloxy.
The $C_1$-$C_6$ alkylsulfenyl in the definitions of $R^5$, $R^6$, $R^8$, $R^9$, $R^a$, $R^b$ and X, may, for example, be a linear or branched alkylsulfenyl, such as methylthio, ethylthio, n-propylthio, i-propylthio, n-butylthio, i-butylthio, s-butylthio, t-butylthio, n-pentylthio and n-hexylthio.
The $C_1$-$C_6$ alkylsulfinyl in the definitions of $R^5$, $R^6$, $R^a$ and $R^b$, may, for example, a linear or branched alkylsulfinyl, such as methylsulfinyl, ethylsulfinyl, n-propylsulfinyl, i-propylsulfinyl, n-butylsulfinyl, i-butylsulfinyl, s-butylsulfinyl, t-butylsulfinyl, n-pentylsulfinyl and n-hexylsulfinyl.
The $C_1$-$C_6$ alkylsulfonyl in the definitions of $R^5$, $R^6$, $R^7$, $R^a$, $R^b$, $U^1$ and $U^2$, may, for example, be a linear or branched alkylsulfonyl, such as methylsulfonyl, ethylsulfonyl, n-propylsulfonyl, i-propylsulfonyl, n-butylsulfonyl, i-butylsulfonyl, s-butylsulfonyl, t-butylsulfonyl, n-pentylsulfonyl and n-hexylsulfonyl.
The $C_1$-$C_6$ alkoxy $C_1$-$C_6$ alkyl in the definitions of $R^1$, $R^2$, $R^3$, $R^4$, $R^5$, $R^6$, $R^7$, $R^{13}$, $R^a$, $U^1$ and $U^2$, may, for example, be methoxymethyl, ethoxymethyl, n-propoxymethyl, i-propoxymethyl, n-butoxymethyl, i-butoxymethyl, s-butoxymethyl, t-butoxymethyl, n-pentyloxymethyl, 2-methoxyethyl, 3-ethoxypropyl and 3-methoxypropyl.
The $C_1$-$C_6$ alkoxy $C_1$-$C_6$ alkoxy in the definition of $R^b$, may, for example, be methoxymethoxy, ethoxymethoxy, n-propoxymethoxy, i-propoxymethoxy, n-butoxymethoxy, i-butoxymethoxy, s-butoxymethoxy, t-butoxymethoxy, n-pentyloxymethoxy, 2-methoxyethoxy, 3-ethoxypropoxy and 3-methoxypropoxy.

The $C_1$-$C_6$ alkylsulfenyl $C_1$-$C_6$ alkyl in the definitions of $R^1$, $R^2$, $R^3$, $R^4$, $R^5$, $R^6$, $R^7$, $R^a$, $U^1$ and $U^2$, may, for example, be a linear or branched alkylsulfenylalkyl, such as methylthiomethyl, ethylthiomethyl, n-propylthiomethyl, i-propylthiomethyl, n-butylthiomethyl, i-butylthiomethyl, s-butylthiomethyl, t-butylthiomethyl, n-pentylthiomethyl, 2-methylthioethyl, 3-ethylthiopropyl and 3-methylthiopropyl.

The $C_1$-$C_6$ alkylsulfenyl $C_1$-$C_6$ alkoxy in the definition of $R^b$, may, for example, be methylthiomethoxy, ethylthiomethoxy, n-propylthiomethoxy, i-propylthiomethoxy, n-butylthiomethoxy, i-butylthiomethoxy, s-butylthiomethoxy, t-butylthiomethoxy, n-pentylthiomethoxy, 2-methylthioethoxy, 3-ethylthiopropoxy and 3-methylthiopropoxy.

The $C_1$-$C_6$ haloalkylsulfenyl in the definitions of $R^5$, $R^6$, $R^a$ and $R^b$, may, for example, be a linear or branched haloalkylthio, such as fluoromethylthio, chlorodifluoromethylthio, bromodifluoromethylthio, trifluoromethylthio, trichloromethylthio, 2,2,2-trifluoroethylthio, 1,1,2,2-tetrafluoroethylthio, 2-fluoroethylthio, pentafluoroethylthio and 1-fluoro-i-propylthio.

The $C_1$-$C_6$ haloalkylsulfinyl in the definitions of $R^5$, $R^6$, $R^a$ and $R^b$, may, for example, be a linear or branched haloalkylsulfinyl, such as fluoromethylsulfinyl, chlorodifluoromethylsulfinyl, bromodifluoromethylsulfinyl, trifluoromethylsulfinyl, trichloromethylsulfinyl, 2,2,2-trifluoroethylsulfinyl, 1,1,2,2-tetrafluoroethylsulfinyl, 2-fluoroethylsulfinyl, pentafluoroethylsulfinyl and 1-fluoro-i-propylsulfinyl.

The $C_1$-$C_6$ haloalkylsulfonyl in the definitions of $R^5$, $R^6$, $R^7$, $R^a$, $R^b$ $U^1$ and $U^2$, may, for example, be a linear or branched haloalkylsulfonyl, such as fluoromethylsulfonyl, chlorodifluoromethylsulfonyl, bromodifluoromethylsulfonyl, trifluoromethylsulfonyl, trichloromethylsulfonyl, 2,2,2-trifluoroethylsulfonyl, 1,1,2,2-tetrafluoroethylsulfonyl, 2-fluoroethylsulfonyl, pentafluoroethylsulfonyl and 1-fluoro-i-propylsulfonyl.

The $C_2$-$C_6$ alkenyl in the definitions of $R^5$, $R^6$, $R^8$, $R^9$, $R^{13}$, $R^{14}$ and $R^a$, may, for example, be a linear or branched alkenyl such as ethenyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-methyl-2-propenyl, 2-methyl-2-propenyl, 1,1-dimethyl-2-propenyl, 1,2-dimethyl-2-propenyl, 1-ethyl-2-propenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,3-dimethyl-2-butenyl, 2,3-dimethyl-3-butenyl, 1-ethyl-2-butenyl, 1-ethyl-3-butenyl, 2-ethyl-2-butenyl, 2-ethyl-3-butenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1,2-trimethyl-2-propenyl and 1-ethyl-1-methyl-2-propenyl.

The $C_2$-$C_6$ haloalkenyl in the definitions of $R^5$, $R^6$, $R^{14}$ and $R^a$, may, for example, be a linear or branched haloalkenyl, such as 2-chloroethenyl, 2-bromoethenyl, 2,2-dichloroethenyl, 3-chloro-2-propenyl, 3-fluoro-2-propenyl, 3-bromo-2-propenyl, 3-iodo-2-propenyl, 3,3-dichloro-2-propenyl, 3,3-difluoro-2-propenyl, 4-chloro-2-butenyl, 4,4-dichloro-3-butenyl and 4,4-difluoro-3-butenyl.

The $C_2$-$C_6$ alkenyloxy in the definitions of $R^5$, $R^6$, $R^a$ and $R^b$, may, for example, be a linear or branched alkenyloxy, such as 1-methylethenyloxy, 2-propenyloxy, 1-methyl-2-propenyloxy, 2-butenyloxy, 3-butenyloxy and 2-methyl-2-propenyloxy.

The $C_2$-$C_6$ haloalkenyloxy in the definitions of $R^5$, $R^6$, $R^a$, $R^b$ and X, may, for example, be a linear or branched haloalkenyloxy, such as 2-chloroethenyloxy, 2-bromoethenyloxy, 2,2-dichloroethenyloxy, 3-chloro-2-propenyloxy, 3-fluoro-2-propenyloxy, 3-bromo-2-propenyloxy, 3-iodo-2-propenyloxy, 3,3-dichloro-2-propenyloxy, 3,3-difluoro-2-propenyloxy, 4-chloro-2-butenyloxy, 4,4-dichloro-3-butenyloxy and 4,4-difluoro-3-butenyloxy.

The $C_2$-$C_6$ alkenylsulfenyl in the definitions of $R^5$, $R^6$, $R^a$ and $R^b$, may, for example, be a linear or branched alkenylsulfenyl, such as 1-methylethenylthio, 2-propenylthio, 1-methyl-2-propenylthio, 2-butenylthio, 3-butenylthio and 2-methyl-2-propenylthio.

The $C_2$-$C_6$ alkenylsulfinyl in the definitions of $R^5$, $R^6$, $R^a$ and $R^b$, may, for example, be a linear or branched alkenylsulfinyl, such as 1-methylethenylsulfinyl, 2-propenylsulfinyl, 1-methyl-2-propenylsulfinyl, 2-butenylsulfinyl, 3-butenylsulfinyl and 2-methyl-2-propenylsulfinyl.

The $C_2$-$C_6$ alkenylsulfonyl in the definitions of $R^5$, $R^6$, $R^a$ and $R^b$, may, for example, be a linear or branched alkenylsulfonyl, such as 1-methylethenylsulfonyl, 2-propenylsulfonyl, 1-methyl-2-propenylsulfonyl, 2-butenylsulfonyl, 3-butenylsulfonyl and 2-methyl-2-propenylsulfonyl.

The $C_2$-$C_6$ haloalkenylsulfenyl in the definitions of $R^5$, $R^6$, $R^a$ and $R^b$, may, for example, be a linear or branched haloalkenylsulfenyl, such as 2-chloroethenylthio, 2-bromoethenylthio, 2,2-dichloroethenylthio, 3-chloro-2-propenylthio, 3-fluoro-2-propenylthio, 3-bromo-2-propenylthio, 3-iodo-2-propenylthio, 3,3-dichloro-2-propenylthio, 3,3-difluoro-2-propenylthio, 4-chloro-2-butenylthio, 4,4-dichloro-3-butenylthio and 4,4-difluoro-3-butenylthio.

The $C_2$-$C_6$ haloalkenylsulfinyl in the definitions of $R^5$, $R^6$, $R^a$ and $R^b$, may, for example, be a linear or branched haloalkenylsulfinyl, such as 2-chloroethenylsulfinyl, 2-bromoethenylsulfinyl, 2,2-dichloroethenylsulfinyl, 3-chloro-2-propenylsulfinyl, 3-fluoro-2-propenylsulfinyl, 3-bromo-2-propenylsulfinyl, 3-iodo-2-propenylsulfinyl, 3,3-dichloro-2-propenylsulfinyl, 3,3-difluoro-2-propenylsulfinyl, 4-chloro-2-butenylsulfinyl, 4,4-dichloro-3-butenylsulfinyl and 4,4-difluoro-

3-butenylsulfinyl.

The $C_2$-$C_6$ haloalkenylsulfonyl in the definitions of $R^5$, $R^6$, $R^a$ and $R^b$, may, for example, be a linear or branched haloalkenylsulfonyl, such as 2-chloroethenylsulfonyl, 2-bromoethenylsulfonyl, 2,2-dichloroethenylsulfonyl, 3-chloro-2-propenylsulfonyl, 3-fluoro-2-propenylsulfonyl, 3-bromo-2-propenylsulfonyl, 3-iodo-2-propenylsulfonyl, 3,3-dichloro-2-propenylsulfonyl, 3,3-difluoro-2-propenylsulfonyl, 4-chloro-2-butenylsulfonyl, 4,4-dichloro-3-butenylsulfonyl and 4,4-difluoro-3-butenylsulfonyl.

The $C_2$-$C_6$ alkynyl in the definitions of $R^5$, $R^6$, $R^{13}$, $R^{14}$ and $R^a$, may, for example, be a linear or branched alkynyl, such as ethynyl, 1-propynyl, 2-propynyl, 1-methyl-2-propynyl, 1,1-dimethyl-2-propynyl, 1-methyl-1-ethyl-2-propynyl, 1-butynyl, 2-butynyl, 3-butynyl, 1-methyl-2-butynyl, 1-methyl-3-butynyl, 2-methyl-3-butynyl, 1,1-dimethyl-2-butynyl, 1,1-dimethyl-3-butynyl, 1,2-dimethyl-3-butynyl, 2,2-dimethyl-3-butynyl, 1-ethyl-2-butynyl, 1-ethyl-3-butynyl, 2-ethyl-3-butynyl, 1-pentynyl, 2-pentynyl, 3-pentynyl, 4-pentynyl, 1-methyl-3-pentynyl, 1-methyl-4-pentynyl, 2-methyl-3-pentynyl, 2-methyl-4-pentynyl, 3-methyl-4-pentynyl, 4-methyl-2-pentynyl and hexynyl.

The $C_2$-$C_6$ haloalkynyl in the definitions of $R^5$, $R^6$, $R^{14}$ and $R^a$, may, for example, be a linear or branched haloalkynyl, such as chloroethynyl, bromoethynyl, iodoethynyl, 3-chloro-2-propynyl, 3-bromo-2-propynyl, 3-iodo-2-propynyl, 4-bromo-3-butynyl, 4-iodo-3-butynyl and 6-iodo-5-hexynyl.

The $C_2$-$C_6$ alkynyloxy in the definitions of $R^5$, $R^6$, $R^a$ and $R^b$, may, for example, be a linear or branched alkynyloxy, such as ethynyloxy, 2-propynyloxy, 1-methyl-2-propynyloxy, 1,1-dimethyl-2-propynyloxy, 1-methyl-1-ethyl-2-propynyloxy, 2-butynyloxy, 3-butynyloxy, 1-methyl-2-butynyloxy, 1,1-dimethyl-2-butynyloxy, 1-pentynyloxy, 2-pentynyloxy, 3-pentynyloxy, 4-pentynyloxy and hexynyloxy.

The $C_2$-$C_6$ haloalkynyloxy in the definitions of $R^5$, $R^6$, $R^a$ and $R^b$, may, for example, be a linear or branched haloalkynyloxy, such as chloroethynyloxy, bromoethynyloxy, iodoethynyloxy, 3-chloro-2-propynyloxy, 3-bromo-2-propynyloxy, 3-iodo-2-propynyloxy, 4-bromo-3-butynyloxy, 4-iodo-3-butynyloxy and 6-iodo-5-hexynyloxy.

The $C_2$-$C_6$ alkynylsulfenyl in the definitions of $R^5$, $R^6$, $R^a$ and $R^b$, may, for example, be a linear or branched alkynylsulfenyl, such as ethynylthio, 2-propynylthio, 1-methyl-2-propynylthio, 1,1-dimethyl-2-propynylthio, 1-methyl-1-ethyl-2-propynylthio, 2-butynylthio, 3-butynylthio, 1-methyl-2-butynylthio, 1,1-dimethyl-2-butynylthio, 1-pentynylthio, 2-pentynylthio, 3-pentynylthio, 4-pentynylthio and hexynylthio.

The $C_2$-$C_6$ alkynylsulfinyl in the definitions of $R^5$, $R^6$, $R^a$ and $R^b$, may, for example, be a linear or branched alkynylsulfinyl, such as ethynylsulfinyl, 2-propynylsulfinyl, 1-methyl-2-propynylsulfinyl, 1,1-dimethyl-2-propynylsulfinyl, 1-methyl-1-ethyl-2-propynylsulfinyl, 2-butynylsulfinyl, 3-butynylsulfinyl, 1-methyl-2-butynylsulfinyl, 1,1-dimethyl-2-butynylsulfinyl, 1-pentynylsulfinyl, 2-pentynylsulfinyl, 3-pentynylsulfinyl, 4-pentynylsulfinyl and hexynylsulfinyl.

The $C_2$-$C_6$ alkynylsulfonyl in the definitions of $R^5$, $R^6$, $R^a$ and $R^b$, may, for example, be a linear or branched alkynylsulfonyl, such as ethynylsulfonyl, 2-propynylsulfonyl, 1-methyl-2-propynylsulfonyl, 1,1-dimethyl-2-propynylsulfonyl, 1-methyl-1-ethyl-2-propynylsulfonyl, 2-butynylsulfonyl, 3-butynylsulfonyl, 1-methyl-2-butynylsulfonyl, 1,1-dimethyl-2-butynylsulfonyl, 1-pentynylsulfonyl, 2-pentynylsulfonyl, 3-pentynylsulfonyl, 4-pentynylsulfonyl and hexynylsulfonyl.

The $C_2$-$C_6$ haloalkynylsulfenyl in the definitions of $R^5$, $R^6$, $R^a$ and $R^b$, may, for example, be a linear or branched haloalkynylsulfenyl, such as chloroethynylthio, bromoethynylthio, iodoethynylthio, 3-chloro-2-propynylthio, 3-bromo-2-propynylthio, 3-iodo-2-propynylthio, 4-bromo-3-butynylthio, 4-iodo-3-butynylthio and 6-iodo-5-hexynylthio.

The $C_2$-$C_6$ haloalkynylsulfinyl in the definitions of $R^5$, $R^6$, $R^a$ and $R^b$, may, for example, be a linear or branched haloalkynylsulfinyl, such as chloroethynylsulfinyl, bromoethynylsulfinyl, iodoethynylsulfinyl, 3-chloro-2-propynylsulfinyl, 3-bromo-2-propynylsulfinyl, 3-iodo-2-propynylsulfinyl, 4-bromo-3-butynylsulfinyl, 4-iodo-3-butynylsulfinyl and 6-iodo-5-hexynylsulfinyl.

The $C_2$-$C_6$ haloalkynylsulfonyl in the definitions of $R^5$, $R^6$, $R^a$ and $R^b$, may, for example, be a linear or branched haloalkynylsulfonyl, such as chloroethynylsulfonyl, bromoethynylsulfonyl, iodoethynylsulfonyl, 3-chloro-2-propynylsulfonyl, 3-bromo-2-propynylsulfonyl, 3-iodo-2-propynylsulfonyl, 4-bromo-3-butynylsulfonyl, 4-iodo-3-butynylsulfonyl and 6-iodo-5-hexynylsulfonyl.

The $C_1$-$C_6$ alkoxycarbonyl in the definitions of $R^5$, $R^6$, $R^8$, $R^9$, $R^{14}$, $R^a$, $R^b$, X, $U^1$ and $U^2$, may, for example, be a linear or branched alkoxycarbonyl, such as methoxycarbonyl, ethoxycarbonyl, n-propoxycarbonyl, i-propoxycarbonyl, n-butoxycarbonyl, i-butoxycarbonyl, s-butoxycarbonyl, t-butoxycarbonyl, n-pentyloxycarbonyl and n-hexyloxycarbonyl.

The $C_1$-$C_6$ alkylcarbonyl in the definitions of $R^5$, $R^6$, $R^7$, $R^{14}$, $R^a$, $R^b$, X, $U^1$ and $U^2$, may, for example, be a linear or branched alkylcarbonyl, such as acetyl, propionyl, n-propylcarbonyl, i-propylcarbonyl, n-butylcarbonyl, i-butylcarbonyl, s-butylcarbonyl, t-butylcarbonyl, n-pentylcarbonyl and n-hexylcarbony.

The $C_1$-$C_6$ haloalkylcarbonyl in the definitions of $R^5$, $R^6$, $R^7$, $R^a$, $R^b$, X, $U^1$ and $U^2$, may, for example, be a linear or branched haloalkylcarbonyl, such as chloroacetyl, fluoroacetyl, chlorofluoroacetyl, chlorodifluoroacetyl, dichloroacetyl, difluoroacetyl, trifluoroacetyl, 3,3,3-trifluoropropionyl and pentafluoropropionyl.

The $C_1$-$C_6$ alkylcarbonyloxy in the definitions of $R^5$, $R^6$, $R^a$, $R^b$, X, $U^1$ and $U^2$, may, for example, be a linear or branched alkylcarbonyloxy, such as acetyloxy, propionyloxy, n-propylcarbonyloxy, i-propylcarbonyloxy, n-butylcarbonyloxy, i-butylcarbonyloxy, s-butylcarbonyloxy, t-butylcarbonyloxy, n-pentylcarbonyloxy and n-hexylcarbonyloxy.

The phenyl $C_1$-$C_6$ alkyl which may be substituted by $R^a$ in the definitions of $R^1$, $R^2$, $R^3$, $R^4$, $R^5$, $R^6$, $R^7$ and $R^c$, may, for example, be a linear or branched phenylalkyl, such as benzyl, 2-chlorobenzyl, 3-bromobenzyl, 4-chlorobenzyl, 4-methylbenzyl, 4-t-butylbenzyl, 2-methylbenzyl, 2-methoxybenzyl, 1-phenylethyl, 1-(3-chlorophenyl)ethyl, 2-phenylethyl, 1-methyl-l-phenylethyl, 1-(4-chlorophenyl)-1-methylethyl, 1-(3-chlorophenyl)-1-methylethyl, 1-phenylpropyl, 2-phenylpropyl, 3-phenylpropyl, 1-phenylbutyl, 2-phenylbutyl, 3-phenylbutyl, 4-phenylbutyl, 1-methyl-1-phenylpropyl, 1-methyl-2-phenylpropyl, 1-methyl-3-phenylpropyl, 2-methyl-2-phenylpropyl, 2-(4-chlorophenyl)-2-methylpropyl, 2-methyl-2-(3-methylphenyl)propyl, 1-phenylpentyl, 2-phenylpentyl, 3-phenylpentyl, 4-phenylpentyl, 5-phenylpentyl, 1-methyl-1-phenylbutyl, 1-methyl-2-phenylbutyl, 1-methyl-3-phenylbutyl, 1-methyl-4-phenylbutyl, 2-methyl-2-phenylbutyl, 2-(4-chlorophenyl)-2-methylbutyl, 2-methyl-2-(3-methylphenyl)butyl, 1-phenylhexyl, 2-phenylhexyl, 3-phenylhexyl, 4-phenylhexyl, 5-phenylhexyl, 6-phenylhexyl, 1-methyl-1-phenylpentyl, 1-methyl-2-phenylpentyl, 1-methyl-3-phenylpentyl, 1-methyl-4-phenylpentyl, 2-methyl-2-phenylpentyl, 2-(4-chlorophenyl)-2-methylpentyl and 2-methyl-2-(3-methylphenyl)pentyl.

The heteroaryl $C_1$-$C_6$ alkyl which may be substituted by $R^a$ in the definitions of $R^1$, $R^2$, $R^3$, $R^4$, $R^5$, $R^6$, $R^7$ and $R^c$, may, for example, be a linear or branched heteroarylalkyl, such as pyridin-2-ylmethyl, 5-chlorothiophen-2-ylmethyl, 1-methyl-3-chloropyrazol-5-ylmethyl, 2-(3-methylfuran-2-yl)ethyl, 3-(6-trifluoromethylpyridin-2-yl)propyl, 4-(pyrimidin-2-yl)butyl, 5-(1,2,4-triazol-1-yl)pentyl, 2-(pyrrol-1-yl)hexyl.

The phenyl which may be substituted by $R^a$ in the definitions of $R^3$, $R^5$, $R^6$, $R^7$, $R^b$ and $R^c$, may, for example, be Ph, 2-Cl-Ph, 3-Cl-Ph, 4-Cl-Ph, 2-F-Ph, 3-F-Ph, 4-F-Ph, 2-Me-Ph, 3-Me-Ph, 4-Me-Ph, 2-MeO-Ph. 3-MeO-Ph, 4-MeO-Ph, 4-Br-Ph, 2,4-$Cl_2$-Ph, 3,4-$Cl_2$-Ph, 2,4,6-$Cl_3$-Ph, 3,4-(MeO) $_2$-Ph, 2-Cl-4-Me-Ph, 2-MeO-4-Me-Ph, 2-Cl-4-i-PrO-Ph, 3-Cl-4-$PhCH_2O$-Ph, 2,4-$Me_2$-Ph, 2,5-$Me_2$-Ph, 2,6-$F_2$-Ph, 2,3,4,5,6-$F_5$-Ph, 4-Et-Ph, 4-i-Pr-Ph, 4-n-Bu-Ph, 4-s-Bu-Ph, 4-t-Bu-Ph, 4-(t-$BuCH_2$)-Ph, 4-Et$(Me)_2$-Ph, 4-n-Hex-Ph, 4-$((Me)_2$ (CN)C)-Ph, 4-(MeCH=CH)-Ph, 4-(MeC≡C) -Ph, 4-$CF_3$-Ph, 4-$CF_3$ $CH_2$-Ph, 4-($Cl_2C$=$CHCH_2$)-Ph, 4-(BrC=C)-Ph, 4-(2,2-$F_2$-c-$BuCH_2$)-Ph, 4-(1-Me-c-Pr)-Ph, 4-i-PrO-Ph, 4-t-BuO-Ph, 4-n-HexO-Ph, 4-MeCC(O)Ph, 4-($CH_2$=$CHCH_2O$)-Ph, 4-$CHF_2O$-Ph, 4-$CBrF_2O$-Ph, 4-$CF_3O$-Ph, 4-$CF_3CH_2O$-Ph, 4-($CF_2$=$CHCH_2CH_2O$)-Ph, 4-$CCl_3CCH_2O$-Ph, 4-MeS-Ph, 4-s-BuS-Ph, 4-EtSO-Ph, 4-$MeSO_2$-Ph, 4-$EtSO_2$-Ph, 4-i-$PrSO_2$-Ph, 4-t-$BuSO_2$-Ph, 4-(MeCH=$CHCH_2$ S)-Ph. 4-($CH_2$=$CHCH_2SO$)-Ph, 4-(ClCH=$CHCH_2SO_2$) -Ph, 4-(HC≡ $CCH_2S$)-Ph, 4-(HC≡$CCH_2SO$-Ph), 4-(HC≡$CCH_2SO_2$)-Ph, 4-$CHF_2S$-Ph, 4-$CBrF_2S$-Ph, 4-$CF_3S$-Ph, 4-$CF_3CH_2S$-Ph, 4-$CHF_2CF_2S$-Ph, 4-$CHF_2SO$-Ph, 4-$CBrF_2SO$-Ph, 4-$CF_3SO$-Ph, 4-$CF_3CH_2SO_2$-Ph, 4-$CHF_2$ $CF_2SO_2$-Ph, 4-$CHF_2SO_2$-Ph, 4-$CBrF_2SO_2$-Ph, 4-$CF_3SO_2$-Ph, 4-($Cl_2$ C=$CHCH_2S$)-Ph, 4-($Cl_2C$=$CHCH_2SO$)-Ph, 4-($Cl_2C$=$CHCH_2SO_2$)-Ph, 4-(BrO≡$CCH_2S$)-Ph, 4-(BrC≡$CCH_2SO$)-Ph, 4-(BrC≡$CCH_2SO_2$)-Ph, 4-CHO-Ph, 4-$NO_2$-Ph, 3-CN-Ph, 4-CN-Ph, 4-$(Me)_2N$-Ph, 4-Me(MeC(O))N-Ph, 4-PhN(Me)-Ph, 4-$PhCH_2$(MeC(O))N-Ph, 4-MeC(O)-Ph, 4-EtC (O)-Ph, 4-n-PrC(O)-Ph, 4-i-PrC(O)-Ph, 4-i-BuC(O)-Ph, 4-t-BuC(O)-Ph, 4-i-$BuCH_2C$(O)-Ph, 4-Et$(Me)_2$ C(O)-Ph, 4-n-HexC(O)-Ph, 4-$MeOCH_2$-Ph, 4-$EtOCH_2$-Ph, 4-i-$PrOCH_2$-Ph, 4-$MeSCH_2$-Ph, 4-$EtSCH_2$-Ph, 4-i-$PrSCH_2$-Ph, 4-$CF_3$ C (O)-Ph, 4-$CF_3CF_2C$(O)-Ph, 4-MeC(O)O-Ph, 4-EtC(O)O-Ph, 4-n-PrC(O)O-Ph, 4-i-PrC(O)O-Ph, 4-i-BuC(O)O-Ph, 4-t-BuC(O)O-Ph, 4-i-$BuOH_2C$(O)O-Ph, 4-Et$(Me)_2C$(O)O-Ph, 4-n-HexC(O)O-Ph, 4-$CF_3C$(O)O-Ph, 4-$CF_3CF_2C$(O)O-Ph, 3,5-$Cl_2$-Ph, 2,6-$Cl_2$-Ph, 2,5-$Cl_2$-Ph, 2,3-$Cl_2$-Ph, 2,3-$F_2$-Ph, 2,5-$F_2$-Ph, 3,4-$F_2$-Ph, 3,5-$F_2$-Ph, 2,4-$F_2$-Ph, 2-$CF_3$-Ph, 3-(3-Cl-Ph, $CH_2$ O)-Ph, 2-F-6-$CF_3$-Ph, 2-F-6-Cl-Ph, 2-F-6-Me-Ph, 2-F-6-MeO-Ph, 2-F-6-OH-Ph, 2-F-6-MeS-Ph, 2-F-5-Cl-Ph, 2-F-5-$CF_3$-Ph, 2-F-5-Me-Ph, 2-F-5-MeO-Ph, 2-F-5-OH-Ph, 2-F-5-MeS-Ph, 2-F-4-Cl-Ph, 2-F-4-$CF_3$-Ph, 2-F-4-Me-Ph, 2-F-4-MeO-Ph, 2-F-3-Cl-Ph, 2-F-3-Me-Ph, 2-F-3-MeO-Ph, 3-F-2-Cl-Ph, 3-F-2-Me-Ph, 3-F-2-MeO-Ph, 3-F-4-Cl-Ph, 3-F-4-Me-Ph, 3-F-4-MeO-Ph, 3-F-5-Cl-Ph, 3-F-5-Me-Ph, 3-F-5-MeO-Ph, 3-F-6-Cl-Ph, 3-F-6-Me-Ph, 3-F-6-MeO-Ph, 4-F-2-Cl-Ph, 4-F-2-Me-Ph, 4-F-2-MeO-Ph, 4-F-3-Cl-Ph, 4-F-3-Me-Ph, 4-F-3-MeO-Ph, 2,4,6-$F_3$-Ph, 2-OH-Ph, 4-I-Ph, 4-MeOC(O)-Ph, 4-MeNHC(O)-Ph, 2,6-$Me_2$-Ph, 3-$CF_3$-Ph, 2-Br-Ph, 3-Br-Ph, 2-MeO(O)-Ph, 2-I-Ph, 3-I-Ph, 4-c-Pr-Ph, 4-(2-Cl-c-Pr)-Ph, 4-(2,2-$Cl_2$-c-Pr)-Ph, 4-(Ph-CH=CH)-Ph, 4-(Ph-C=O)-Ph, 4-PhS-Ph, 4-HO-Ph, 4-EtO-Ph, 4-PenO-Ph, 2-F-3-$CF_3$-Ph, 2,3-$Me_2$-Ph, 3,4-$Me_2$-Ph, 3,5-$Me_2$-Ph, 2,3-$(MeO)_2$-Ph, 2,4-$(MeO)_2$-Ph, 2,5-$(MeO)_2$-Ph, 3,5-$(MeO)_2$-Ph, 2-F-3-I-Ph, 2-F-4-I-Ph, 2-F-5-I-Ph, 2-F-6-I-Ph, 2-F-4-EtO-Ph, 2-F-4-PrO-Ph, 2-F-4-i-PrO-Ph, 2-F-4-BuO-Ph, 2-F-4-s-BuO-Ph, 2-F-4-i-BuO-Ph, 2-F-4-t-BuO-Ph, 2-F-4-PenO-Ph, 2-F-4-(2-Me-BuO)-Ph, 2-F-4-(2,2-$Me_2$-PrO)-Ph, 2-F-4-HexO-Ph, 2-F-4-(2-Et-Hex)O-Ph, 2-F-4-Et-Ph, 2-F-4-Pr-Ph, 2-F-4-i-Pr-Ph, 2-F-4-Bu-Ph, 2-F-4-s-Bu-Ph, 2-F-4-i-Bu-Ph, 2-F-4-t-Bu-Ph, 2-F-4-Pen-Ph, 2-F-4-(2-Me-Bu)-Ph, 2-F-4-(2,2-$Me_2$-Pr)-Ph, 2-F-4-Hex-Ph, 2-F-4-(2-Et-Hex)-Ph, 2-F-6-PhS-Ph, 2-F-6-$Me_2N$-Ph, 2-F-6-MeNH-Ph, 2-F-6-Ph-Ph, 3,4-methylenedioxy-Ph, 3,4-ethylenedioxy-Ph, 2-F-3-Br-Ph, 2-F-4-Br-Ph, 2-F-5-Br-Ph, 2-F-6-Br-Ph, 3-F-2-Br-Ph, 3-F-4-Br-Ph, 3-F-5-Br-Ph, 3-F-6-Br-Ph, 4-F-2-Br-Ph, 4-F-3-Br-Ph, 2-Cl-3-Me-Ph, 2-Cl-4-Me-Ph, 2-Cl-S-Me-Ph, 2-Cl-6-Me-Ph, 3-Cl-2-Me-Ph, 3-Cl-4-Me-Ph, 3-Cl-5-Me-Ph, 3-Cl-6-Me-Ph, 4-Cl-2-Me-Ph, 4-Cl-3-Me-Ph, 2,3-$F_2$-4-Me-Ph, 2,3-$F_2$-5-Me-Ph, 2,3-$F_2$-6-Me-Ph, 2,4-$F_2$-3-Me-Ph, 2,4-$F_2$-5-Me-Ph, 2,4-$F_2$-6-Me-Ph, 2,5-$F_2$-3-Me-Ph, 2,5-$F_2$-4-Me-Ph, 2,5-$F_2$-6-Me-Ph, 2,6-$F_2$-3-Me-Ph, 2,6-$F_2$-4-Me-Ph, 2,3-$F_2$-4-Cl-Ph, 2,3-$F_2$-5-Cl-Ph, 2,3-$F_2$-6-Cl-Ph, 2,4-$F_2$-3-Cl-Ph, 2,4-$F_2$-5-Cl-Ph, 2,4-$F_2$-6-Cl-Ph, 2,5-$F_2$-3-Cl-Ph, 2,5-$F_2$-4-Cl-Ph, 2,5-$F_2$-6-Cl-Ph, 2,6-$F_2$-3-Cl-Ph, 2,6-$F_2$-4-Cl-Ph, 2,3-$F_2$-4-MeO-Ph, 2,3-$F_2$-5-MeO-Ph, 2,3-$F_2$-6-MeO-Ph, 2,4-$F_2$-3-MeO-Ph, 2,4-$F_2$-5-MeO-Ph, 2,4-$F_2$-6-MeO-Ph, 2,5-$F_2$-3-MeO-Ph, 2,5-$F_2$-4-MeO-Ph, 2,5-$F_2$-6-MeO-Ph, 2,6-$F_2$-3-MeO-Ph, 2,6-$F_2$-4-MeO-Ph, 2,3-$F_2$-4-EtO-Ph, 2,3-$F_2$-5-EtO-Ph, 2,3-$F_2$-6-EtO-Ph, 2,4-$F_2$-3-EtO-Ph, 2,4-$F_2$-5-EtO-Ph, 2,4-$F_2$-6-EtO-Ph, 2,5-$F_2$-3-EtO-Ph, 2,5-$F_2$-4-EtO-Ph, 2,5-$F_2$-6-EtO-Ph,

2,6-$F_2$-3-EtO-Ph, 2,6-$F_2$-4-EtO-Ph, 2,3-$F_2$-4-Et-Ph, 2,3-$F_2$-5-Et-Ph, 2,3-$F_2$-6-Et-Ph, 2,4-$F_2$-3-Et-Ph, 2,4-$F_2$-5-Et-Ph, 2,4-$F_2$-6-Et-Ph, 2,5-$F_2$-3-Et-Ph, 2,5-$F_2$-4-Et-Ph, 2,5-$F_2$-6-Et-Ph, 2,6-$F_2$-3-Et-Ph, 2,6-$F_2$-4-Et-Ph, 2,3-$F_2$-4-Br-Ph, 2,3-$F_2$-5-Br-Ph, 2,3-$F_2$-6-Br-Ph, 2,4-$F_2$-3-Br-Ph, 2,4-$F_2$-5-Br-Ph, 2,4-$F_2$-6-Br-Ph, 2,5-$F_2$-3-Br-Ph, 2,5-$F_2$-4-Br-Ph, 2,5-$F_2$-6-Br-Ph, 2,6-$F_2$-3-Br-Ph, 2,6-$F_2$-4-Br-Ph, 2,6-$F_2$-4-Pr-Ph, 2,6-$F_2$-4-i-Pr-Ph, 2,6-$F_2$-4-c-Pr-Ph, 2,6-$F_2$-4-Bu-Ph, 2,6-$F_2$-4-i-Bu-Ph, 2,6-$F_2$-4-s-Bu-Ph, 2,6-$F_2$-4-t-Bu-Ph, 2,6-$F_2$-4-Pen-Ph, 2,6-$F_2$-4-Hex-Ph, 2,6-$F_2$-4-Ph-Ph, 2,6-$F_2$-4-$PhCH_2$-Ph, 2,6-$F_2$-4-PrO-Ph, 2,6-$F_2$-4-i-PrO-Ph, 2,6-$F_2$-4-c-PrO-Ph, 2,6-$F_2$-4-BuO-Ph, 2,6-$F_2$-4-i-BuO-Ph, 2,6-$F_2$-4-s-BuO-Ph, 2,6-$F_2$-4-t-BuO-Ph, 2,6-$F_2$-4-PenO-Ph, 2,6-$F_2$-4-HexO-Ph, 2,6-$F_2$-4-PhO-Ph, 2,6-$F_2$-4-$PhCH_2O$-Ph, 2-F-6-Cl-3-MeO-Ph, 2-F-6-Cl-4-MeO-Ph, 2-F-6-Cl-5-MeO-Ph, 2-F-6-Cl-3-Me-Ph, 2-F-6-Cl-4-Me-Ph, 2-F-6-Cl-5-Me-Ph, 2-F-6-MeO-3-Cl-Ph, 2-F-6-MeO-4-Cl-Ph, 2-F-6-MeO-5-Cl-Ph, 2-F-6-MeO-3-Me-Ph, 2-F-6-MeO-4-Me-Ph, 2-F-6-MeO-5-Me-Ph, 2,4,6-$Me_3$-Ph, 2-Cl-3-MeO-Ph, 2-Cl-4-MeO-Ph, 2-Cl-5-MeO-Ph, 2-Cl-6-MeO-Ph, 3-Cl-2-MeO-Ph, 3-Cl-4-MeO-Ph, 3-Cl-5-MeO-Ph, 3-Cl-6-MeO-Ph, 4-Cl-2-MeO-Ph, 4-Cl-3-MeO-Ph, 2-Me-3-MeO-Ph, 2-Me-4-MeO-Ph, 2-Me-5-MeO-Ph, 2-Me-6-MeO-Ph, 3-Me-2-MeO-Ph, 3-Me-4-MeO-Ph, 3-Me-5-MeO-Ph, 3-Me-g-MeO-Ph, 4-Me-3-MeO-Ph and 2,6-$(MeO)_2$-Ph.

The heteroaryl which may be substituted by $R^a$ in the definitions of the heteroaryl which may be substituted by $R^a$, the heteroarylsulfonyl which may be substituted by $R^a$ and the heteroarylcarbonyl which may be substituted by $R^a$, in $R^5$, $R^6$, $R^7$, $R^b$ and $R^c$, may, for example, be 5-chlorothiophen-2-yl, 3,5-dimethylfuran-2-yl, 3-cyanopyrrol-1-yl, oxazol-2-yl, 2-methylsulfenyloxazol-4-yl, 4-methylthiazol-2-yl, 2-trifluoromethylimidazol-1-yl, isoxazol-3-yl, 3-chloroisoxazol-4-yl, 3-methylisothiazol-5-yl, 3-phenylpyrazol-1-yl, 1-methylpyrazol-5-yl, 2-methylsulfonyl-1,3,4-oxadiazol-5-yl, 2-bromo-1,3,4-thiadiazol-2-yl, 1,2,4-oxadiazol-3-yl, 1,2,4-thiadiazol-5-yl, 1,2,4-triazol-1-yl, 1,2,3-thiadiazol-5-yl, 1,2,3-triazol-1-yl, 1,2,3,4-tetrazol-1-yl, 6-phenoxypyridin-2-yl, 6-methoxypyrimidin-2-yl, pyrazin-2-yl, pyridazin-3-yl, 1,3,5-triazin-2-yl and 1,2,4-triazin-6-yl.

The phenyl which may be substituted by $R^a$ in the definitions of the phenylsulfonyl which may be substituted by $R^a$ and the phenylcarbonyl which may be substituted by $R^a$, in $R^5$, $R^6$, $R^7$, $R^b$, $R^c$ and X, may, for example, be phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 4-iodophenyl, 2,4-dichlorophenyl, 3,4-dichlorophenyl, 2,6-difluorophenyl, 2,6-dichlorophenyl, 2-fluoro-4-chlorophenyl, 2,3,4,5,6-pentafluorophenyl, 2-methylphenyl, 3-methylphenyl, 4-methylphenyl, 2,5-dimethylphenyl, 4-methyl-2,3,5,6-tetrafluorophenyl, 2-methoxyphenyl, 3-methoxyphenyl, 4-methoxyphenyl, 2,6-dimethoxyphenyl, 3,4-dimethoxyphenyl, 3,4,5-trimethoxyphenyl, 2-trifluoromethylphenyl, 3-trifluoromethylphenyl and 4-trifluoromethylphenyl.

The phenyl $C_1$-$C_6$ alkyl which may be substituted by $R^a$ in the definitions of the phenyl $C_1$-$C_6$ alkylsulfonyl which may be substituted by $R^a$ and the phenyl $C_1$-$C_6$ alkylcarbonyl which may be substituted by $R^a$, in $R^5$, $R^6$, $R^7$, $R^b$ and $R^c$, may, for example, be benzyl, 2-chlorobenzyl, 3-bromobenzyl, 4-chlorobenzyl, 4-methylbenzyl, 4-t-butylbenzyl, 2-methylbenzyl, 2-methoxybenzyl, 1-phenylethyl, 1-(3-chlorophenyl)ethyl, 2-phenylethyl, 1-methyl-1-phenylethyl, 1-(4-chlorophenyl)-1-methylethyl, 1-(3-chlorophenyl)-1-methylethyl, 1-phenylpropyl, 2-phenylpropyl, 3-phenylpropyl, 1-phenylbutyl, 2-phenylbutyl, 3-phenylbutyl, 4-phenylbutyl, 1-methyl-1-phenylpropyl, 1-methyl-2-phenylpropyl, 1-methyl-3-phenylpropyl, 2-methyl-2-phenylpropyl, 2-(4-chlorophenyl) -2-methylpropyl, 2-methyl-2-(3-methylphenyl)propyl, 1-phenylpentyl, 2-phenylpentyl, 3-phenylpentyl, 4-phenylpentyl, 5-phenylpentyl, 1-methyl-1-phenylbutyl, 1-methyl-2-phenylbutyl, 1-methyl-3-phenylbutyl, 1-methyl-4-phenylbutyl, 2-methyl-2-phenylbutyl, 2-(4-chlorophenyl)-2-methylbutyl, 2-methyl-2-(3-methylphenyl)butyl, 1-phenylhexyl, 2-phenylhexyl, 3-phenylhexyl, 4-phenylhexyl, 5-phenylhexyl, 6-phenylhexyl, 1-methyl-1-phenylpentyl, 1-methyl-2-phenylpentyl, 1-methyl-3-phenylpentyl, 1-methyl-4-phenylpentyl, 2-methyl-2-phenylpentyl, 2-(4-chlorophenyl)-2-methylpentyl and 2-methyl-2-(3-methylphenyl)pentyl.

The phenoxy which may be substituted by $R^a$ in the definitions of the phenoxy which may be substituted by $R^a$ and the phenoxycarbonyl which may be substituted by $R^a$, in $R^7$, $R^b$ and $R^c$, may, for example, be phenoxy, 2-fluorophenoxy, 3-fluorophenoxy, 4-fluorophenoxy, 2-chlorophenoxy, 3-chlorophenoxy, 4-chlorophenoxy, 2-bromophenoxy, 3-bromophenoxy, 4-bromophenoxy, 4-iodophenoxy, 2,4-dichlorophenoxy, 3,4-dichlorophenoxy, 2,6-difluorophenoxy, 2,6-dichlorophenoxy, 2-fluoro-4-chlorophenoxy, 2,3,4,5,6-pentafluorophenoxy, 2-methylphenoxy, 3-methylphenoxy, 4-methylphenoxy, 2,5-dimethylphenoxy, 4-methyl-2,3,5,6-tetrafluorophenoxy, 2-methoxyphenoxy, 3-methoxyphenoxy, 4-methoxyphenoxy, 2,6-dimethoxyphenoxy, 3,4-dimethoxyphenoxy, 3,4,5-trimethoxyphenoxy, 2-trifluoromethylphenoxy, 3-trifluoromethylphenoxy and 4-trifluoromethylphenoxy.

The phenyl $C_1$-$C_6$ alkoxy which may be substituted by $R^a$ in the definitions of $R^7$, $R^b$ and $R^c$, may, for example, be a linear or branched phenylalkoxy, such as benzyloxy, 2-chlorobenzyloxy, 3-bromobenzyloxy, 4-chlorobenzyloxy, 4-methylbenzyloxy, 4-t-butylbenzyloxy, 2-methylbenzyloxy, 2-methoxybenzyloxy, 1-phenylethyloxy, 1-(3-chlorophenyl) ethyloxy, 2-phenylethyloxy, 1-methyl-1-phenylethyloxy, 1-(4-chlorophenyl)-1-methylethyloxy, 1-(3-chlorophenyl)-1-methylethyloxy, 1-phenylpropyloxy, 2-phenylpropyloxy, 3-phenylpropyloxy, 1-phenylbutyloxy, 2-phenylbutyloxy, 3-phenylbutyloxy, 4-phenylbutyloxy, 1-methyl-1-phenylpropyloxy, 1-methyl-2-phenylpropyloxy, 1-methyl-3-phenylpropyloxy, 2-methyl-2-phenylpropyloxy, 2-(4-chlorophenyl)-2-methyl-propyloxy, 2-methyl-2-(3-methylphenyl)propyloxy, 1-phenylpentyloxy, 2-phenylpentyloxy, 3-phenylpentyloxy, 4-phenylpentyloxy, 5-phenylpentyloxy, 1-methyl-1-phenylbutyloxy, 1-methyl-2-phenylbutyloxy, 1-methyl-3-phenylbutyloxy, 1-methyl-4-phenylbutyloxy, 2-methyl-2-phenylbutyloxy, 2-(4-chlorophenyl)-2-methylbutyloxy, 2-methyl-2-(3-methylphenyl)butyloxy, 1-phenylhexyloxy, 2-phenylhexyloxy, 3-phenylhexyloxy, 4-phenylhexyloxy, 5-phenylhexyloxy, 6-phenylhexyloxy, 1-methyl-l-phenylpentyloxy, 1-methyl-2-phenylpentylxoy, 1-methyl-3-phenylpentyloxy, 1-methyl-4-phenylpentyloxy, 2-methyl-2-phenylpentyloxy, 2-(4-chlorophenyl)-2-methylpentyloxy and 2-methyl-2-(3-methylphenyl)pentyloxy.

The heteroaryloxy which may be substituted by $R^a$ in the definitions of the heteroaryloxy which may be substituted by $R^a$ and the heteroaryloxycarbonyl which may be substituted by $R^a$, in $R^7$, $R^b$ and $R^c$, may, for example, be 5-chlorothiophen-2-yloxy, 3,5-dimethylfuran-2-yloxy, 3-cyano-pyrrol-1-yloxy, oxazol-2-yloxy, 2-methylsulfenyloxazol-4-yloxy, 4-methylthiazol-2-yloxy, 2-trifluoromethylimidazol-4-yloxy, isoxazol-3-yloxy, 3-chloroisoxazol-4-yloxy, 3-methylisothiazol-5-yloxy, 1-benzyl-3-phenylpyrazol-5-yloxy, 1-methylpyrazol-5-yloxy, 2-methylsulfonyl-1,3,4-oxadiazol-5-yloxy, 2-bromo-1,3,4-thiadiazol-2-yloxy, 1,2,4-oxadiazol-3-yloxy, 1,2,4-thiadiazol-5-yloxy, 1,2,4-triazol-3-yloxy, 1,2,3-thiadiazol-5-yloxy, 1,2,3-triazol-5-yloxy, 1,2,3,4-tetrazol-5-yloxy, 6-phenoxypyridin-2-yloxy, 6-methoxypyridin-2-yloxy, pyrazin-2-yloxy, pyridazin-3-yloxy, 1,3,5-triazin-2-yloxy and 1,2,4-triazin-6-yloxy.

The phenyl which may be substituted by $R^a$ in the definitions of the phenylsulfenyl which may be substituted by $R^a$, the phenylsulfinyl which may be substituted by $R^a$ and the phenylcarbonyloxy which may be substituted by $R^a$, in $R^c$, may, for example, be phenyl, 2-fluorophenyl, 3-fluorophenyl, 4-fluorophenyl, 2-chlorophenyl, 3-chlorophenyl, 4-chlorophenyl, 2-bromophenyl, 3-bromophenyl, 4-bromophenyl, 4-iodophenyl, 2,4-dichlorophenyl, 3,4-dichlorophenyl, 2,6-difluorophenyl, 2,6-dichlorophenyl, 2-fluoro-4-chlorophenyl, 2,3,4,5,6-pentafluorophenyl, 2-methylphenyl, 3-methylphenyl, 4-methylphenyl, 2,5-dimethylphenyl, 4-methyl-2,3,5,6-tetrafluorophenyl, 2-methoxyphenyl, 3-methoxyphenyl, 4-methoxyphenyl, 2,6-dimethoxyphenyl, 3,4-dimethoxyphenyl, 3,4,5-trimethoxyphenyl, 2-trifluoromethylphenyl, 3-trifluoromethylphenyl and 4-trifluoromethylphenyl.

The phenyl $C_1$-$C_6$ alkyl which may be substituted by $R^a$ in the definitions of the phenyl $C_1$-$C_6$ alkylsulfenyl which may be substituted by $R^a$, the phenyl $C_1$-$C_6$ alkylsulfinyl which may be substituted by $R^a$ and the phenyl $C_1$-$C_6$ alkylcarbonyloxy which may be substituted by $R^a$, in $R^c$, may, for example, be a linear or branched phenylalkyl, such as benzyl, 2-chlorobenzyl, 3-bromobenzyl, 4-chlorobenzyl, 4-methylbenzyl, 4-t-butylbenzyl, 2-methylbenzyl, 2-methoxybenzyl, 1-phenylethyl, 1-(3-chlorophenyl)ethyl, 2-phenylethyl, 1-methyl-1-phenylethyl, 1-(4-chlorophenyl)-1-methylethyl, 1-(3-chlorophenyl)-1-methylethyl, 1-phenylpropyl, 2-phenylpropyl, 3-phenylpropyl, 1-phenylbutyl, 2-phenylbutyl, 3-phenylbutyl, 4-phenylbutyl, 1-methyl-1-phenylpropyl, 1-methyl-2-phenylpropyl, 1-methyl-3-phenylpropyl, 2-methyl-2-phenylpropyl, 2-(4-chlorophenyl)-2-methylpropyl, 2-methyl-2-(3-methylphenyl)propyl, 1-phenylpentyl, 2-phenylpentyl, 3-phenylpentyl, 4-phenylpentyl, 5-phenylpentyl, 1-methyl-1-phenylbutyl, 1-methyl-2-phenylbutyl, 1-methyl-3-phenylbutyl, 1-methyl-4-phenylbutyl, 2-methyl-2-phenylbutyl, 2-(4-chlorophenyl)-2-methylbutyl, 2-methyl-2-(3-methylphenyl)butyl, 1-phenylhexyl, 2-phenylhexyl, 3-phenylhexyl, 4-phenylhexyl, 5-phenylhexyl, 6-phenylhexyl, 1-methyl-1-phenylpentyl, 1-methyl-2-phenylpentyl, 1-methyl-3-phenylpentyl, 1-methyl-4-phenylpentyl, 2-methyl-2-phenylpentyl, 2-(4-chlorophenyl)-2-methylpentyl and 2-methyl-2- (3-methylphenyl)pentyl.

The heteroaryl which may be substituted by $R^a$ in the definitions of the heteroarylsulfinyl which may be substituted by $R^a$, the heteroarylsulfenyl which may be substituted by $R^a$ and the heteroarylcarboxyoxy which may be substituted by $R^a$, in $R^c$, may, for example, be 5-chlorothiophen-2-yl, 3,5-dimethylfuran-2-yl, 3-cyanopyrrol-1-yl, oxazol-2-yl, 2-methylsulfenyloxazol-4-yl, 4-methylthiazol-2-yl, 2-trifluoromethylimidazol-1-yl, isoxazol-3-yl, 3-chloroisoxazol-4-yl, 3-methylisothiazol-5-yl, 3-phenylpyrazol-1-yl, 1-methylpyrazol-5-yl, 2-methylsulfonyl-1,3,4-oxadiazol-5-yl, 2-bromo-1,3,4-thiadiazol-2-yl, 1,2,4-oxadiazol-3-yl, 1,2,4-thiadiazol-5-yl, 1,2,4-triazol-1-yl, 1,2,3-thiadiazol-5-yl, 1,2,3-triazol-1-yl, 1,2,3,4-tetrazol-1-yl, 6-phenoxypyrimidin-2-yl, 6-methoxypyrimidin-2-yl, pyrazin-2-yl, pyridazin-3-yl, 1,3,5-triazin-2-yl and 1,2,4-triazin-6-yl.

The heteroaryl $C_1$-$C_6$ alkoxy which may be substituted by $R^a$ in the definition of $R^c$, may, for example, be a linear or branched heteroarylalkoxy, such as pyridin-2-ylmethyloxy, 5-chlorothiophen-2-ylmethyloxy, 1-methyl-3-chloropyrazol-5-ylmethyloxy, 2-(3-methylfuran-2-yl)ethyloxy, 3-(6-trifluoromethylpyridin-2-yl)propyloxy, 4-(pyrimidin-2-yl)butyloxy, 5-(triazol-1-yl)pentyloxy and 6-(pyrrol-1-yl)hexyloxy.

The heteroaryl $C_1$-$C_6$ alkyl which may be substituted by $R^a$ in the definitions of the heteroaryl $C_1$-$C_6$ alkylsulfenyl which may be substituted by $R^a$, the heteroaryl $C_1$-$C_6$ alkylsulfinyl which may be substituted by $R^a$, the heteroaryl $C_1$-$C_6$ alkylsulfonyl which may be substituted by $R^a$, and the heteroaryl $C_1$-$C_6$ alkylcarbonyloxy which may be substituted by $R^a$, in $R^c$, may, for example, be a linear or branched heteroarylalkyl, such as pyridin-2-ylmethyl, 5-chlorothiophen-2-ylmethyl, 1-methyl-3-chloropyrazol-5-ylmethyl, 2-(3-methylfuran-2-yl)ethyl, 3-(6-trifluoromethylpyridin-2-yl)propyl, 4-(pyrimidin-2-yl)butyl, 5-(triazol-1-yl)pentyl and 6-(pyrrol-1-yl)hexyl.

The phenyl which may be substituted by $R^c$ in the definitions of the phenyl which may be substituted by $R^c$, the phenylsulfenyl which may be substituted by $R^c$, the phenylsulfinyl which may be substituted by $R^c$, the phenylsulfonyl which may be substituted by $R^c$, the phenylcarbonyl which may be substituted by $R^c$ and the phenylcarbonyloxy which may be substituted by $R^c$, in Y', may, for example, be Ph, 2-Cl-Ph, 3-Cl-Ph, 4-Cl-Ph, 2-F-Ph, 3-F-Ph, 4-F-Ph, 2-Me-Ph, 3-Me-Ph, 4-Me-Ph, 2-MeO-Ph, 3-MeO-Ph, 4-MeO-Ph, 4-Br-Ph, 2,4-$Cl_2$-Ph, 3,4-$Cl_2$-Ph, 2,4,6-$Cl_3$-Ph, 3,4-$(MeO)_2$-Ph, 2-Cl-4-Me-Ph, 2-MeO-4-Me-Ph, 2-Cl-4-i-PrO-Ph, 3-Cl-4-$PhCH_2O$-Ph, 2,4-$Me_2$-Ph, 2,5-$Me_2$-Ph, 2,6-$F_2$-Ph, 2,3,4,5,6-$F_5$-Ph, 4-Et-Ph, 4-i-Pr-Ph, 4-n-Bu-Ph, 4-s-Bu-Ph, 4-t-Bu-Ph, 4-(t-$BuCH_2$)-Ph, 4-$Et(Me)_2$-Ph, 4-n-Hex-Ph,

4-((Me)$_2$(CN)C)-Ph, 4-PhCH$_2$-Ph, 4-(4-F-Ph)(Me)$_2$-Ph, 4-(MeCH=CH)-Ph, 4-(MeC≡C)-Ph, 4-CF$_3$-Ph, 4-CF$_3$CH$_2$-Ph, 4-(Cl$_2$C=CHCH$_2$)-Ph, 4-(BrC≡C)-Ph, 4-(2,2-F$_2$-c-BuCH$_2$)-Ph, 4-(1-Me-c-Pr)-Ph, 4-i-PrO-Ph, 4-t-BuO-Ph, 4-n-HexO-Ph, 4-MeCC(O)Ph , 4-(CH$_2$=CHCH$_2$O)-Ph, 4-CHF$_2$O-Ph, 4-CBrF$_2$O-Ph, 4-CF$_3$O-Ph, 4-CF$_3$CH$_2$O-Ph, 4-(CF$_2$=CHCH$_2$CH$_2$O)-Ph, 4-CCl$_3$CCH$_2$O-Ph, 4-MeS-Ph, 4-s-BuS-Ph, 4-EtSO-Ph, 4-MeSO$_2$-Ph, 4-EtSO$_2$-Ph, 4-i-PrSO$_2$-Ph, 4-t-BuSO$_2$-Ph, 4-(MeOH=CHCH$_2$S)-Ph, 4-(CH$_2$ =CHCH$_2$SO)-Ph, 4- (ClCH=CHCH$_2$SO$_2$) -Ph, 4-(HC≡CCH$_2$S)-Ph, 4-(HC≡CCH$_2$SO-Ph), 4-(HC≡CCH$_2$SO$_2$)-Ph, 4-CHF$_2$S-Ph, 4-CBrF$_2$S-Ph, 4-CF$_3$S-Ph, 4-CF$_3$CH$_2$S-Ph, 4-CHF$_2$CF$_2$S-Ph, 4-CHF$_2$SO-Ph, 4-CBrF$_2$SO-Ph, 4-CF$_3$SO-Ph, 4-CF$_3$CH$_2$SO$_2$-Ph, 4-CHF$_2$CF$_2$SO$_2$-Ph, 4-CHF$_2$SO$_2$-Ph, 4-CBrF$_2$SO$_2$-Ph, 4-CF$_3$SO$_2$-Ph, 4-(Cl$_2$C=CHCH$_2$S)-Ph, 4-(Cl$_2$C=CHCH$_2$SO)-Ph, 4-(Cl$_2$C=CHCH$_2$SO$_2$)-Ph, 4-(BrC≡CCH$_2$S)-Ph, 4-(BrC≡CCH$_2$SO)-Ph, 4-(BrC≡CCH$_2$SO$_2$)-Ph, 4-CHO-Ph, 4-NO$_2$-Ph, 3-CN-Ph, 4-CN-Ph, 4-(Me)$_2$N-Ph, 4-Me(MeC(O))N-Ph, 4-PhMeN-Ph, 4-PhCH$_2$(MeO(O))N-Ph, 4-PhCH$_2$O-Ph, 4-(2-Cl-Ph)CH$_2$O-Ph, 4-(3-Cl-Ph)CH$_2$O-Ph, 4-(4-Cl-Ph)CH$_2$O-Ph, 4-(2-Me-Ph)CH$_2$O-Ph, 4-(3-Me-Ph)CH$_2$O-Ph, 4-(4-F-Ph) CH$_2$O-Ph, 4-(4-Et-Ph)CH$_2$O-Ph, 4-(2-Cl-Ph)CH$_2$S-Ph, 4-(3-Cl-Ph)CH$_2$S-Ph, 4-(4-Cl-Ph)CH$_2$SO-Ph, 4-(2-Me-Ph) CH$_2$S-Ph, 4-(3-Me-Ph)CH$_2$ SO$_2$-Ph, 4-(2,4-F$_2$-Ph)CH$_2$O-Ph, 3-(3,4-Cl$_2$-Ph)CH$_2$O-Ph, 4-(2,5-Me$_2$-Ph)CH$_2$O-Ph, 4-(2,3,5,6-F$_5$-Ph)CH$_2$O-Ph, 4-MeO(O)-Ph, 4-EtC(O)-Ph, 4-n-PrC(O)-Ph, 4-i-PrC(O)-Ph, 4-i-BuC(O)-Ph, 4-t-BuC(O) -Ph, 4-i-BuCH$_2$C(O)-Ph, 4-Et(Me)$_2$C(O)-Ph, 4-n-HexC(O)-Ph, 4-PhC(O)-Ph, 4-(2-Cl-Ph)C(O)- 4-(3-Br-Ph)C(O)-Ph, 4-(4-Cl-Ph)C(O)-Ph, 4-(2-Me-Ph)C(O)-Ph, 4-MeOCH$_2$-Ph, 4-EtOCH$_2$-Ph, 4-i-PrOCH$_2$-Ph, 4-MeSCH$_2$-Ph, 4-EtSCH$_2$-Ph, 4-i-PrSCH$_2$-Ph, 4-CF$_3$C(O)-Ph, 4-CF$_3$CF$_2$C(O)-Ph, 4-MeC(O)O-Ph, 4-EtC(O)O-Ph, 4-n-PrC(O)O-Ph, 4-i-PrC(O) O-Ph, 4-i-BuC(O)O-Ph, 4-t-BuC(O)O-Ph, 4-i-BuCH$_2$C(O)O-Ph, 4-Et(Me)$_2$C(O)O-Ph, 4-n-HexC(O)O-Ph, 4-CF$_3$C(O) O-Ph, 4-CF$_3$CF$_2$ C(O)O-Ph, 4-PhC(O)O-Ph, 3-Ph-Ph, 4-Ph-Ph, 4-(4-Cl-Ph)-Ph, 4-(2,5-Me$_2$-Ph)-3-Me-Ph, 3-PhO-Ph, 4-PhO-Ph, 4-(4-Cl-Ph)O-Ph, 4-(4-Me-Ph)O-Ph, 4-(4-F-Ph)O-Ph, 4-(4-MeO-Ph)O-Ph, 4-(2,4-Cl$_2$-Ph)O-Ph, 4-(3,4-Cl$_2$-Ph)O-Ph, 4-(2-Pyridyl)-Ph, 4-(5-Cl-2-Pyridyl)-Ph, 2,3-Cl$_2$-Ph, 3,5-Cl$_2$-Ph, 2,6-Cl$_2$-Ph, 2,5-Cl$_2$-Ph, 2,3-F$_2$-Ph, 2,5-F$_2$-Ph, 3,4-F$_2$-Ph, 3,5-F$_2$-Ph, 2,4-F$_2$-Ph, 2-CF$_3$-Ph, 3-(3-Cl-PhCH$_2$O)-Ph, 2-F-6-CF$_3$-Ph, 2-F-6-Cl-Ph, 2-F-6-Me-Ph, 2-F-6-MeO-Ph, 2-F-6-OH-Ph, 2-F-6-MeS-Ph, 2-F-5-Cl-Ph, 2-F-5-CF$_3$-Ph, 2-F-5-Me-Ph, 2-F-5-MeO-Ph, 2-F-5-OH-Ph, 2-F-5-MeS-Ph, 2-F-4-Cl-Ph, 2-F-4-CF $_3$-Ph, 2-F-4-Me-Ph, 2-F-4-MeO-Ph, 2-F-3-Cl-Ph, 2-F-3-Me-Ph, 2-F-3-MeO-Ph, 3-F-2-Cl-Ph, 3-F-2-Me-Ph, 3-F-2-MeO-Ph, 3-F-4-Cl-Ph, 3-F-4-Me-Ph, 3-F-4-MeO-Ph, 3-F-5-Cl-Ph, 3-F-5-Me-Ph, 3-F-5-MeO-Ph, 3-F-6-Cl-Ph, 3-F-6-Me-Ph, 3-F-6-MeO-Ph, 4-F-2-Cl-Ph, 4-F-2-Me-Ph, 4-F-2-MeO-Ph, 4-F-3-Cl-Ph, 4-F-3-Me-Ph, 4-F-3-MeO-Ph, 2,4,6-F$_3$-Ph, 2-OH-Ph, 4-I-Ph, 4-MeOC(O)-Ph, 4-MeNHCO-Ph, 2,6-Me$_2$-Ph, 2,6-(MeO)$_2$-Ph, 4-(6-F-5-CF$_3$-2-Pyridyl)-Ph, 4-(2-Pyridyl)O-Ph, 4-(5-Cl-2-Pyridyl)O-Ph, 4-(3-Cl-5-F-2-Pyridyl)O-Ph, 4-(5-Cl-2-Thienyl) O-Ph, 3-CF$_3$-Ph, 2-Br-Ph, 3-Br-Ph, 2-MeC(O)-Ph, 2-I-Ph, 3-I-Ph, 4-c-Pr-Ph, 4-(2-Cl-c-Pr)-Ph, 4-(2,2-Cl$_2$-c-Pr)-Ph, 4-(Ph-CH=CH)-Ph, 4-(Ph-C≡C)-Ph, 4-PhS-Ph, 4-HO-Ph, 4-EtO-Ph, 4-PenO-Ph, 2-F-3-CF$_3$-Ph, 2,3-Me$_2$-Ph, 3,4-Me$_2$-Ph, 3,5-Me$_2$-Ph, 2,3-(MeO)$_2$-ph, 2,4-(MeO)$_2$-Ph, 2,5-(MeO)$_2$-Ph, 3,5-(MeO)$_2$-Ph, 2-F-3-I-Ph, 2-F-4-I-Ph, 2-F-5-I-Ph, 2-F-6-I-Ph, 2-F-4-EtO-Ph, 2-F-4-PrO-Ph, 2-F-4-i-PrO-Ph, 2-F-4-BuO-Ph, 2-F-4-s-BuO-Ph, 2-F-4-i-BuO-Ph, 2-F-4-t-BuO-Ph, 2-F-4-PenO-Ph, 2-F-4-(2-Me-BuO)-Ph, 2-F-4-(2,2-Me$_2$-PrO)-Ph, 2-F-4-HexO-Ph, 2-F-4-(2-Et-Hex)O-Ph, 2-F-4-Et-Ph, 2-F-4-Pr-Ph, 2-F-4-i-Pr-Ph, 2-F-4-Bu-Ph, 2-F-4-s-Bu-Ph, 2-F-4-i-Bu-Ph, 2-F-4-t-Bu-Ph, 2-F-4-Pen-Ph, 2-F-4-(2-Me-Bu)-Ph, 2-F-4-(2,2-Me$_2$-Pr)-Ph, 2-F-4-Hex-Ph, 2-F-4-(2-Et-Hex)-Ph, 2-F-6-PhS-Ph, 2-F-6-Me$_2$N-Ph, 2-F-6-MeNH-Ph, 2-F-6-Ph-Ph, 3,4-methylenedioxy-Ph, 3,4-ethylenedioxy-Ph, 2-F-3-Br-Ph, 2-F-4-Br-Ph, 2-F-5-Br-Ph, 2-F-6-Br-Ph, 3-F-2-Br-Ph, 3-F-4-Br-Ph, 3-F-5-Br-Ph, 3-F-6-Br-Ph, 4-F-2-Br-Ph, 4-F-3-Br-Ph, 2-Cl-3-Me-Ph, 2-Cl-4-Me-Ph, 2-Cl-5-Me-Ph, 2-Cl-6-Me-Ph, 3-Cl-2-Me-Ph, 3-C1-4-Me-Ph, 3-Cl-5-Me-Ph, 3-Cl-6-Me-Ph, 4-Cl-2-Me-Ph, 4-C1-3-Me-Ph, 2,3-F$_2$-4-Me-Ph, 2,3-F$_2$-5-Me-Ph, 2,3-F$_2$-6-Me-Ph, 2,4-F$_2$-3-Me-Ph, 2,4-F$_2$-5-Me-Ph, 2,4-F$_2$-6-Me-Ph, 2,5-F$_2$-3-Me-Ph, 2,5-F$_2$-4-Me-Ph, 2,5-F$_2$-6-Me-Ph, 2,6-F$_2$-3-Me-Ph, 2,6-F$_2$-4-Me-Ph, 2,3-F$_2$-4-Cl-Ph, 2,3-F$_2$-5-Cl-Ph, 2,3-F$_2$-6-Cl-Ph, 2,4-F$_2$-3-Cl-Ph, 2,4-F$_2$-5-Cl-Ph, 2,4-F$_2$-6-Cl-Ph, 2,5-F$_2$-3-Cl-Ph, 2,5-F$_2$-4-Cl-Ph, 2,5-F$_2$-6-Cl-Ph, 2,6-F$_2$-3-Cl-Ph, 2,6-F$_2$-4-Cl-Ph, 2,3-F$_2$-4-MeO-Ph, 2,3-F$_2$-5-MeO-Ph, 2,3-F$_2$-6-MeO-Ph, 2, 4-F$_2$-3-MeO-Ph, 2,4-F$_2$-5-MeO-Ph, 2,4-F$_2$-6-MeO-Ph, 2,5-F$_2$-3-MeO-Ph, 2,5-F$_2$-4-MeO-Ph, 2,5-F$_2$-6-MeO-Ph, 2,6-F$_2$-3-MeO-Ph, 2,6-F$_2$-4-MeO-Ph, 2,3-F$_2$-4-EtO-Ph, 2,3-F$_2$-5-EtO-Ph, 2,3-F$_2$-6-EtO-Ph, 2,4-F$_2$-3-EtO-Ph, 2,4-F$_2$-5-EtO-Ph, 2,4-F$_2$-6-EtO-Ph, 2,5-F$_2$-3-EtO-Ph, 2,5-F$_2$-4-EtO-Ph, 2,5-F$_2$-6-EtO-Ph, 2,6-F$_2$-3-EtO-Ph, 2,6-F$_2$-4-EtO-Ph, 2,3-F$_2$-4-Et-Ph, 2,3-F$_2$-5-Et-Ph, 2,3-F$_2$-6-Et-Ph, 2,4-F$_2$-3-Et-Ph, 2,4-F$_2$-5-Et-Ph, 2,4-F$_2$-6-Et-Ph, 2,5-F$_2$-3-Et-Ph, 2,5-F$_2$-4-Et-Ph, 2,5-F$_2$-6-Et-Ph, 2,6-F$_2$-3-Et-Ph, 2,6-F$_2$-4-Et-Ph, 2,3-F$_2$-4-Br-Ph, 2,3-F$_2$-5-Br-Ph, 2,3-F$_2$-6-Br-Ph, 2,4-F$_2$-3-Br-Ph, 2,4-F$_2$-5-Br-Ph, 2,4-F$_2$-6-Br-Ph, 2,5-F$_2$-3-Br-Ph, 2,5-F$_2$-4-Br-Ph, 2,5-F$_2$-6-Br-Ph, 2,6-F$_2$-3-Br-Ph, 2,6-F$_2$-4-Br-Ph, 2,6-F$_2$-4-Pr-Ph, 2,6-F$_2$-4-i-Pr-Ph, 2,6-F$_2$-4-c-Pr-Ph, 2,6-F$_2$-4-Bu-Ph, 2,6-F$_2$-4-i-Bu-Ph, 2,6-F$_2$-4-s-Bu-Ph, 2,6-F$_2$-4-t-Bu-Ph, 2,6-F$_2$-4-Pen-Ph, 2,6-F$_2$-4-Hex-Ph, 2,6-F$_2$-4-Ph-Ph, 2,6-F$_2$-4-PhCH$_2$-Ph, 2,6-F$_2$-4-PrO-Ph, 2,6-F$_2$-4-i-PrO-Ph, 2,6-F$_2$-4-c-PrO-Ph, 2,6-F$_2$-4-BuO-Ph, 2,6-F$_2$-4-i-BuO-Ph, 2,6-F$_2$-4-s-BuO-Ph, 2,6-F$_2$-4-t-BuO-Ph, 2,6-F$_2$-4-PenO-Ph, 2,6-F$_2$-4-HexO-Ph, 2,6-F$_2$-4-PhO-Ph, 2,6-F$_2$-4-PhCH$_2$O-Ph, 2-F-6-Cl-3-MeO-Ph, 2-F-6-Cl-4-MeO-Ph, 2-F-6-Cl-5-MeO-Ph, 2-F-6-Cl-3-Me-Ph, 2-F-6-Cl-4-Me-Ph, 2-F-6-Cl-5-Me-Ph, 2-F-6-MeO-3-Cl-Ph, 2-F-6-MeO-4-Cl-Ph, 2-F-6-MeO-5-Cl-Ph, 2-F-6-MeO-3-Me-Ph, 2-F-6-MeO-4-Me-Ph, 2-F-6-MeO-5-Me-Ph, 4-HepO-Ph, 4-OctO-Ph, 4-NonO-Ph, 4-DecO-Ph, 4-UndecO-Ph, 4-DodecO-Ph, 4-Hep-Ph, 4-Oct-Ph, 4-Non-Ph, 4-Dec-Ph, 4-Undec-Ph, 4-Dodec-Ph, 2-Cl-4-HepO-Ph, 2-C1-4-OctO-Ph, 2-Cl-4-NonO-Ph, 2-Cl-4-DecO-Ph, 2-Cl-4-UndecO-Ph, 2-Cl-4-DodecO-Ph, 2-Cl-4-Hep-Ph, 2-Cl-4-Oct-Ph, 2-C1-4-Non-Ph, 2-Cl-4-Dec-Ph, 2-Cl-4-Undec-Ph, 2-Cl-4-Dodec-Ph, 3-Cl-4-HepO-Ph, 3-Cl-4-OctO-Ph, 3-Cl-4-NonO-Ph, 3-Cl-4-DecO-Ph, 3-Cl-4-UndecO-Ph, 3-Cl-4-DodecO-Ph, 3-Cl-4-Hep-Ph, 3-Cl-4-Oct-Ph,

3-Cl-4-Non-Ph, 3-Cl-4-Dec-Ph, 3-C1-4-Undec-Ph, 3-Cl-4-Dodec-Ph, 2-F-4-HepO-Ph, 2-F-4-OctO-Ph, 2-F-4-NonO-Ph, 2-F-4-DecO-Ph, 2-F-4-UndecO-Ph, 2-F-4-DodecO-Ph, 2-F-4-Hep-Ph, 2-F-4-Oct-Ph, 2-F-4-Non-Ph, 2-F-4-Dec-Ph, 2-F-4-Undec-Ph, 2-F-4-Dodec-Ph, 3-F-4-HepO-Ph, 3-F-4-OctO-Ph, 3-F-4-NonO-Ph, 3-F-4-DecO-Ph, 3-F-4-UndecO-Ph, 3-F-4-DodecO-Ph, 3-F-4-Hep-Ph, 3-F-4-Oct-Ph, 3-F-4-Non-Ph, 2-Cl-3-MeO-Ph, 2-Cl-4-MeO-Ph, 2-Cl-5-MeO-Ph, 2-Cl-6-MeO-Ph, 3-Cl-2-MeO-Ph, 3-Cl-4-MeO-Ph, 3-Cl-5-MeO-Ph, 3-Cl-6-MeO-Ph, 4-Cl-2-MeO-Ph, 4-Cl-3-MeO-Ph, 2-Me-3-MeO-Ph, 2-Me-4-MeO-Ph, 2-Me-5-MeO-Ph, 2-Me-6-MeO-Ph, 3-Me-2-MeO-Ph, 3-Me-4-MeO-Ph, 3-Me-5-MeO-Ph, 3-Me-6-MeO-Ph, 4-Me-3-MeO-Ph, 3-F-4-Dec-Ph, 3-F-4-Undec-Ph, 3-F-4-Dodec-Ph and 2,4,6-$Me_3$-Ph.

The phenyl $C_1$-$C_6$ alkyl which may be substituted by $R^c$ in the definitions of the phenyl $C_1$-$C_6$ alkyl which may be substituted by $R^c$, the phenyl $C_1$-$C_6$ alkylsulfenyl which may be substituted by $R^c$, the phenyl $C_1$-$C_6$ alkylsulfinyl which may be substituted by $R^c$, the phenyl $C_1$-$C_6$ alkylsulfonyl which may be substituted by $R^c$, the phenyl $C_1$-$C_6$ alkylcarbonyl which may be substituted by $R^c$ and the phenyl $C_1$-$C_6$ alkylcarbonyloxy which may be substituted by $R^c$, in Y', may, for example, be a linear or branched phenylalkyl, such as benzyl, 2-chlorobenzyl, 3-bromobenzyl, 4-chlorobenzyl, 4-methylbenzyl, 4-t-butylbenzyl, 2-methylbenzyl, 2-methoxybenzyl, 1-phenylethyl, 1-(3-chlorophenyl)ethyl, 2-phenylethyl, 1-methyl-1-phenylethyl, 1-(4-chlorophenyl)-1-methylethyl, 1-(3-chlorophenyl)-1-methylethyl, 1-phenylpropyl, 2-phenylpropyl, 3-phenylpropyl, 1-phenylbutyl, 2-phenylbutyl, 3-phenylbutyl, 4-phenylbutyl, 1-methyl-1-phenylpropyl, 1-methyl-2-phenylpropyl, 1-methyl-3-phenylpropyl, 2-methyl-2-phenylpropyl, 2-(4-chlorophenyl)-2-methylpropyl, 2-methyl-2-(3-methylphenyl)propyl, 1-phenylpentyl, 2-phenylpentyl, 3-phenylpentyl, 4-phenylpentyl, 5-phenylpentyl, 1-methyl-1-phenylbutyl, 1-methyl-2-phenylbutyl, 1-methyl-3-phenylbutyl, 1-methyl-4-phenylbutyl, 2-methyl-2-phenylbutyl, 2-(4-chlorophenyl)-2-methylbutyl, 2-methyl-2-(3-methylphenyl)butyl, 1-phenylhexyl, 2-phenylhexyl, 3-phenylhexyl, 4-phenylhexyl, 5-phenylhexyl, 6-phenylhexyl, 1-methyl-l-phenylpentyl, 1-methyl-2-phenylpentyl, 1-methyl-3-phenylpentyl, 1-methyl-4-phenylpentyl, 2-methyl-2-phenylpentyl, 2-(4-chlorophenyl)-2-methylpentyl and 2-methyl-2-(3-methylphenyl)pentyl.

The phenyl $C_1$-$C_6$ alkoxy which may be substituted by $R^c$ in the definition of Y', may, for example, be a linear or branched phenylalkoxy, such as benzyloxy, 2-chlorobenzyloxy, 3-bromobenzyloxy, 4-chlorobenzyloxy, 4-methylbenzyloxy, 4-t-butylbenzyloxy, 2-methylbenzyloxy, 2-methoxybenzyloxy, 1-phenylethyloxy, 1-(3-chlorophenyl)ethyloxy, 2-phenylethyloxy, 1-methyl-1-phenylethyloxy, 1-(4-chlorophenyl)-1-methylethyloxy, 1-(3-chlorophenyl)-1-methylethyloxy, 1-phenylpropyloxy, 2-phenylpropyloxy, 3-phenylpropyloxy, 1-phenylbutyloxy, 2-phenylbutyloxy, 3-phenylbutyloxy, 4-phenylbutyloxy, 1-methyl-1-phenylpropyloxy, 1-methyl-2-phenylpropyloxy, 1-methyl-3-phenylpropyloxy, 2-methyl-2-phenylpropyloxy, 2-(4-chlorophenyl)-2-methyl-propyloxy, 2-methyl-2-(3-methylphenyl)propyloxy, 1-phenylpentyloxy, 2-phenylpentyloxy, 3-phenylpentyloxy, 4-phenylpentyloxy, 5-phenylpentyloxy, l-methyl-1-phenylbutyloxy, 1-methyl-2-phenylbutyloxy, 1-methyl-3-phenylbutyloxy, 1-methyl-4-phenylbutyloxy, 2-methyl-2-phenylbutyloxy, 2-(4-chlorophenyl)-2-methylbutyloxy, 2-methyl-2-(3-methylphenyl)butyloxy, 1-phenylhexyloxy, 2-phenylhexyloxy, 3-phenylhexyloxy, 4-phenylhexyloxy, 5-phenylhexyloxy, 6-phenylhexyloxy, 1-methyl-1-phenylpentyloxy, 1-methyl-2-phenylpentyloxy, 1-methyl-3-phenylpentyloxy, 1-methyl-4-phenylpentyloxy, 2-methyl-2-phenylpentyloxy, 2-(4-chlorophenyl)-2-methylpentyloxy and 2-methyl-2-(3-methylphenyl)pentyloxy.

The phenoxy which may substituted by $R^c$ in the definitions of the phenoxy which may be substituted by $R^c$ and the phenoxycarbonyl which may be substituted by $R^c$, in Y', may, for example, be phenoxy, 2-fluorophenoxy, 3-fluorophenoxy, 4-fluorophenoxy, 2-chlorophenoxy, 3-chlorophenoxy, 4-chlorophenoxy, 2-bromophenoxy, 3-bromophenoxy, 4-bromophenoxy, 4-iodophenoxy, 2,4-dichlorophenoxy, 3,4-dichlorophenoxy, 2,6-difluorophenoxy, 2,6-dichlorophenoxy, 2-fluoro-4-chlorophenoxy, 2,3,4,5,6-pentafluorophenoxy, 2-methylphenoxy, 3-methylphenoxy, 4-methylphenoxy, 2,5-dimethylphenoxy, 4-methyl-2,3,5,6-tetrafluorophenoxy, 2-methoxyphenoxy, 3-methoxyphenoxy, 4-methoxyphenoxy, 2,6-dimethoxyphenoxy, 3,4-dimethoxyphenoxy, 3,4,5-trimethoxyphenoxy, 2-trifluoromethylphenoxy, 3-trifluoromethylphenoxy and 4-trifluoromethylphenoxy.

The heteroaryl which may be substituted by $R^c$ in the definitions of the heteroaryl which may be substituted by $R^c$, the heteroarylsulfinyl which may be substituted by $R^c$, the heteroarylsulfenyl which may be substituted by $R^c$, the heteroarylsulfonyl which may be substituted by $R^c$, the heteroarylcarbonyl which may be substituted by $R^c$ and the heteroarylcarbonyloxy which may be substituted by $R^c$, in Y', may, for example, be 2-fluorofuran-3-yl, 3-cyanopyrrol-1-yl, oxazol-2-yl, 2-methylsulfenyloxazol-4-yl, 2-methylsulfonyl-1,3,4-oxadiazol-5-yl, 2-bromo-1,3,4-thiadiazol-5-yl, 1,2,4-oxadiazol-3-yl, 1,2,4-thiadiazol-5-yl, 1,2,4-triazol-1-yl, 1,2,3-triazol-1-yl, 1,2,3,4-tetrazol-1-yl, 6-methoxypyrimidin-2-yl, pyridazin-3-yl, 1,3,5-triazin-2-yl, 1,2,4-triazin-6-yl, 1-methylpyrazol-5-yl, 1-methylpyrazol-4-yl, 1-methylpyrazol-3-yl, 1-phenylpyrazol-5-yl, 1-phenylpyrazol-4-yl, 1-phenylpyrazol-3-yl, 1-methyl-4-fluoropyrazol-5-yl, 1-methyl-4-fluoropyrazol-3-yl, 1-methyl-3-fluoropyrazol-4-yl, l-methyl-3-fuoropyrazol-5-yl, 1-methyl-5-fluoropyrazol-3-yl, 1-methyl-5-fluoropyrazol-4-yl, 1-methyl-4-chloropyrazol-5-yl, 1-methyl-4-chloropyrazol-3-yl, 1-methyl-3-chloropyrazol-4-yl, 1-methyl-3-chloropyrazol-5-yl, 1-methyl-5-chloropyrazol-3-yl, 1-methyl-5-chloropyrazol-4-yl, 1-methyl-3-bromopyrazol-4-yl, 1-methyl-3-phenylpyrazol-4-yl, 1-methyl-5-nitropyrazol-4-yl, 1-methyl-3-trifluoromethylpyrazol-4-yl, 1-methyl-3-difluorochloromethylpyrazol-4-yl, 1-methyl-3-trifluoromethyl-5-methoxypyrazol-4-yl, 1-methyl-5-trifluoromethylpyrazol-3-yl, 1-methyl-4-methoxycarbonylpyrazol-5-yl, 1-methyl-4-methoxycarbonylpyrazol-3-yl, 1-methyl-5-methoxycarbonylpyrazol-3-yl, 1-methyl-3-chloro-4-methoxycarbonylpyrazol-5-yl, 1-methyl-3-chloro-4-ethoxycarbonylpyrazol-5-yl, 1-methyl-4-ethoxycarbonylpyrazol-3-yl, 1,4-dimethylpyrazol-5-yl, 1,4-dimethylpyrzol-3-yl, 1,3-dimethylpyrazol-4-yl, 1,3-dimethylpyrazol-5-yl, 1,5-dimethylpyrazol-3-yl, 1/5-dimethylpyrazol-4-yl, 1,5-dimethyl-4-chloropyrazol-3-yl, 1,3-dimethyl-5-chloropyrazol-4-yl, 1,3-dimethyl-5-fluoropyrazol-4-yl, 1,3-dimethyl-5-methoxypyrazol-4-yl, 1,3,5-trimethylpyrazol-4-yl, 1,3-dimethyl-4-chloropyrazol-5-yl, 1,3-dimethyl-4-fluoropyrazol-5-yl, 1,3-dimethyl-4-nitropyrazol-5-yl, 1,3-dimethyl-4-methoxypyrazol-5-yl, 1-methyl-3,5-dichloropyrazol-4-yl, 1-methyl-3,5-difluoropyrazol-4-yl, 1-phenyl-3,5-dichloropyrazol-4-yl, 1-phenyl-3,5-difluoropyrazol-4-yl, 1-(2-pyridyl)-3,5-dichloropyrazol-4-yl, 1-phenyl-5-methylpyrazol-4-yl, 1-phenyl-5-trifluoromethylpyrazol-4-yl, 1-phenyl-5-difluorochloromethylpyrazol-4-yl, 1-t-butyl-5-methylpyrazol-4-yl, 1-methyl-3-chloro-5-methylthiopyrazol-4-yl, 1-methyl-pyrrol-2-yl, 1-methyl-pyrrol-3-yl, 1-methyl-4-trifluoromethylpyrrol-5-yl, furan-2-yl, furan-3-yl, 5-methylfuran-2-yl, 5-phenylfuran-2-yl, 2,5-dimethylfuran-3-yl, 2,4-dimethylfuran-3-yl, thiophen-2-yl, thiophen-3-yl, 5-phenylthiophen-2-yl, 5-methylthiophen-2-yl, 5-bromothiophen-2-yl, 3-bromothiophen-2-yl, 4,5-dibromothiophen-2-yl, 5-iodothiophen-2-yl, 5-chlorothiophen-2-yl, 5-phenyl-2-methylthiophen-3-yl, 5-nitrothiophen-3-yl, 3-methylthiophen-2-yl, 3-chlorothiophen-2-yl, 3-methoxythiophen-2-yl, 3-fluorothiophen-2-yl, thiazol-4-yl, thiazol-5-yl, thiazol-2-yl, 2,4-dimethylthiazol-5-yl, 2-bromo-4-methylthiazol-5-yl, 2-chloro-4-methylthiazol-5-yl, 2-chloro-4-ethylthiazol-5-yl, 2-chloro-4-trifluoromethylthiazol-5-yl, 2-methyl-4-trifluoromethylthiazol-5-yl, 2-methyl-4-ethylthiazol-5-yl, 2-bromo-4-ethylthiazol-5-yl, 2-ethyl-4-methylthiazol-5-yl, 2-methoxy-4-methylthiazol-5-yl, 2-chloro-4-fluorothiazol-5-yl, 2-phenyl-4-ethoxycarbonylthiazol-5-yl, 2-chlorothiazol-4-yl, 2-methylthiazol-4-yl, 1-phenyl-5-methyloxazol-4-yl, 1,3-dimethyloxazol-5-yl, 3-methylisothiazol-5-yl, 3-benzyloxy-5-methylisothiazol-4-yl, 4-chloro-5-ethoxycarbonylisothiazol-3-yl, isoxazol-5-yl, 3,5-dimethylisoxazol-4-yl, 5-methylisoxazol-3-yl, 3-phenyl-5-methylisoxazol-4-yl, 4-cyanoisoxazol-3-yl, 1-methylimidazol-5-yl, 1-methyl-4,5-dichloroimidazol-2-yl, 1,5-dimethyl-2-chloroimidazol-4-yl, 1-phenyl-5-methyl-1,2,3-triazol-4-yl, 1-phenyl-5-ethyl-1,2,3-triazol-4-yl, 1-phenyl-5-dibromomethyl-1,2,3-triazol-4-yl, 4-methyl-1,2,3-thiadiazol-5-yl, 4-ethyl-1,2,3-thiadiazol-5-yl, 1,2,3-thiadiazol-5-yl, 1,2,3-thiadiazol-4-yl, pyridin-2-yl, pyridin-3-yl, pyridin-4-yl, 6-methylpyridin-3-yl, 6-chloropyridin-2-yl, 6-phenoxypyridin-2-yl, 2-chloropyridin-4-yl, 2-fluoropyridin-4-yl, 2,6-dichloropyridin-4-yl, 2-methoxypyridin-4-yl, 3,6-dichloropyridin-2-yl, 2-chloro-6-methylpyridin-4-yl, 3-fluoropyridin-2-yl, 3-fluoropyridin-4-yl, quinoxalin-2-yl, 6-chloroquinoxalin-2-yl, 6-fluoroquinoxalin-2-yl, 6-methoxyquinoxalin-2-yl, 5-chloroquinoxalin-2-yl, 5-fluoroquinoxalin-2-yl, 5-methoxyquinoxalin-2-yl, 1-methylindol-3-yl, 1-methyl-2-chloroindol-3-yl, 1-methyl-2-fluoroindol-3-yl, benzothiazol-2-yl, 5-fluorobenzothiazol-2-yl, 6-fluorobenzothiazol-2-yl, quinolin-4-yl, pyrazin-2-yl, 3-chloropyrazin-2-yl, 3-methylpyrazin-2-yl, 3-ethylpyrazin-2-yl, 2-phenyl-4-methylpyrimidin-5-yl, 2,4-dimethylpyrimidin-5-yl, 4-trifluoromethylpyrimidin-5-yl, 4-difluorochloromethylpyrimidin-5-yl, 4-pentafluoroethylpyrimidin-5-yl, 4-methylthiopyrimidin-5-yl, 4-bromodifluoromethylpyrimidin-5-yl and 2-methyl-4-chlorodifluoromethylpyrimidin-5-yl.

The heteroaryl $C_1$-$C_6$ alkoxy which may be substituted by $R^c$ in the definition of Y', may, for example, be a linear or branched heteroarylalkoxy, such as pyridin-2-ylmethyloxy, 5-chlorothiophen-2-ylmethyloxy, 1-methyl-3-chloropyrazol-5-ylmethyloxy, 2-(3-methylfuran-2-yl)ethyloxy, 3-(6-trifluoromethylpyridin-2-yl)propyloxy, 4-(pyrimidin-2-yl)butyloxy, 5-(triazol-1-yl)pentyloxy and 6-(pyrrol-1-yl)hexyloxy.

The heteroaryl $C_1$-$C_6$ alkyl which may be substituted by $R^c$ in the definitions of the heteroaryl $C_1$-$C_6$ alkyl which may be substituted by $R^c$, the heteroaryl $C_1$-$C_6$ alkylsulfenyl which may be substituted by $R^c$, the heteroaryl $C_1$-$C_6$ alkylsulfinyl which may be substituted by $R^c$, the heteroaryl $C_1$-$C_6$ alkylsulfonyl which may be substituted by $R^c$, the heteroaryl $C_1$-$C_6$ alkylcarbonyl which may be substituted by $R^c$ and the heteroaryl $C_1$-$C_6$ alkylcarbonyloxy which may be substituted by $R^c$, in Y', may, for example, be a linear or branched heteroarylalkyl, such as pyridin-2-ylmethyl, 5-chlorothiophen-2-ylmethyl, 1-methyl-3-chloropyrazol-5-ylmethyl, 2-(3-methylfuran-2-yl)ethyl, 3-(6-trifluoromethylpyridin-2-yl)propyl, 4-(pyrimidin-2-yl)butyl, 5-(triazol-1-yl)pentyl and 6-(pyrrol-1-yl)hexyl.

The heteroaryloxy which may be substituted by $R^c$ in the definitions of the heteroaryloxy which may be substituted by $R^c$ and the heteroaryloxycarbonyl which may be substituted by $R^c$, in Y', may, for example, be 5-chlorothiophen-2-yloxy, 3,5-dimethylfuran-2-yloxy, 3-cyano-1-methylpyrrol-1-yloxy, oxazol-2-yloxy, 2-methylsulfenyloxazol-4-yloxy, 4-methylthiazol-2-yloxy, 2-trifluoromethylimidazol-4-yloxy, isoxazol-3-yloxy, 3-chloroisoxazol-4-yloxy, 3-methylisothiazol-5-yloxy, 1-benzyl-3-phenylpyrazol-5-yloxy, 1-methylpyrazol-5-yloxy, 2-methylsulfonyl-1,3,4-oxadiazol-5-yloxy, 2-bromo-1,3,4-thiadiazol-5-yloxy, 1,2,4-oxadiazol-3-yloxy, 1,2,4-thiadiazol-5-yloxy, 1,2,4-triazol-3-yloxy, 1,2,3-thiadiazol-5-yloxy, 1,2,3-triazol-5-yloxy, 1,2,3,4-tetrazol-5-yloxy, 6-phenoxypyrimidin-2-yloxy, 6-methoxypyrimidin-2-yloxy, pyrazin-2-yloxy, pyridazin-3-yloxy, 1,3,5-triazin-2-yloxy and 1,2,4-triazin-6-yloxy.

The naphthyl in the definition of Y', may, for example, be 1-naphthyl and 2-naphthyl.

The $C_1$-$C_6$ alkyl which may be substituted by $R^b$ in the definitions of the $C_1$-$C_6$ alkylsulfenyl which may be substituted by $R^b$, the $C_1$-$C_6$ alkylsulfinyl which may be substituted by $R^b$, the $C_1$-$C_6$ alkylsulfonyl which may be substituted by $R^b$, the $C_1$-$C_6$ alkylcarbonyl which may be substituted by $R^b$ and the $C_1$-$C_6$ alkylcarbonyloxy which may be substituted by $R^b$, in $R^c$ and Y', may, for example, be a linear or branched alkyl, such as methyl, ethyl, n-propyl, i-propyl, n-butyl, i-butyl, t-butyl, s-butyl, n-pentyl, n-hexyl, 2-ethylpropyl, 2,2-dimethylpropyl, 1,2-dimethylpropyl, 1,1,2-trimethylpropyl, 1,2,2-trimethylpropyl, 1-ethyl-1-methylpropyl, 1-ethyl-2-methylpropyl, 1-methylbutyl, 2-methylbutyl, 3-methylbutyl, 1,1-dimethylbutyl, 1,2-dimethylbutyl, 1,3-dimethylbutyl, 2,2-dimethylbutyl, 2,3-dimethylbutyl, 3,3-dimethylbutyl,

1-ethylbutyl, 2-ethylbutyl, 1-methylpentyl, 2-methylpentyl, 3-methylpentyl, 4-methylpentyl, fluoromethyl, chloromethyl, bromomethyl, iodomethyl, difluoromethyl, chlorodifluoromethyl, bromodifluoromethyl, trifluoromethyl, dichloromethyl, trichloromethyl, 1-chloroethyl, 1-bromoethyl, 1-iodoethyl, 1-fluoroethyl, 2-chloroethyl, 2-bromoethyl, 2-iodoethyl, 2-fluoroethyl, 2,2-difluoroethyl, 2,2,2-trifluoroethyl, 2,2,2-trichloroethyl, pentafluoroethyl, 2,2,2-trifluoro-1-chloroethyl, 3-fluoropropyl, 3-chloropropyl, 1-fluoro-i-propyl, 1-chloro-i-propyl, heptafluoropropyl, 1,1,2,2,3,3-hexafluoropropyl, 4-chlorobutyl, 4-fluorobutyl, 5-chloropentyl, 5-fluoropentyl, 6-chlorohexyl, 6-fluorohexyl, methoxymethyl, ethoxymethyl, n-propoxymethyl, i-propoxymethyl, n-butoxymethyl, i-butoxymethyl, s-butoxymethyl, t-butoxymethyl, n-pentyloxymethyl, 2-methoxyethyl, 3-ethoxypropyl, 3-methoxypropyl, methylthiomethyl, ethylthiomethyl, n-propylthiomethyl, i-propylthiomethyl, n-butylthiomethyl, i-butylthiomethyl, s-butylthiomethyl, t-butylthiomethyl, n-pentylthiomethyl, 2-methylthioethyl, 3-ethylthiopropyl, 3-methylthiopropyl, benzyl, 2-chlorobenzyl, 3-bromobenzyl, 4-chlorobenzyl, 4-methylbenzyl, 4-t-butylbenzyl, 2-methylbenzyl, 2-methoxybenzyl, 1-phenylethyl, 1-(3-chlorophenyl)ethyl, 2-phenylethyl, 1-methyl-1-phenylethyl, 1-(4-chlorophenyl)-1-methylethyl, 1-(3-chlorophenyl)-1-methylethyl, 1-phenylpropyl, 2-phenylpropyl, 3-phenylpropyl, 1-phenylbutyl, 2-phenylbutyl, 3-phenylbutyl, 4-phenylbutyl, 1-methyl-1-phenylpropyl, 1-methyl-2-phenylpropyl, 1-methyl-3-phenylpropyl, 2-methyl-2-phenylpropyl, 2-(4-chlorophenyl)-2-methylpropyl, 2-methyl-2-(3-methylphenyl)propyl, 1-phenylpentyl, 2-phenylpentyl, 3-phenylpentyl, 4-phenylpentyl, 5-phenylpentyl, 1-methyl-1-phenylbutyl, 1-methyl-2-phenylbutyl, 1-methyl-3-phenylbutyl, 1-methyl-4-phenylbutyl, 2-methyl-2-phenylbutyl, 2-(4-chlorophenyl)-2-methylbutyl, 2-methyl-2-(3-methylphenyl)butyl, 1-phenylhexyl, 2-phenylhexyl, 3-phenylhexyl, 4-phenylhexyl, 5-phenylhexyl, 6-phenylhexyl, 1-methyl-1-phenylpentyl, 1-methyl-2-phenylpentyl, 1-methyl-3-phenylpentyl, 1-methyl-4-phenylpentyl, 2-methyl-2-phenylpentyl, 2-(4-chlorophenyl)-2-methylpentyl, 2-methyl-2-(3-methylphenyl)pentyl, pyridin-2-ylmethyl, 5-chlorothiophen-2-ylmethyl, 1-methyl-3-chloropyrazol-5-ylmethyl, 2-(3-methylfuran-2-yl)ethyl, 3-(6-trifluoromethylpyridin-2-yl)propyl, 4-(pyrimidin-2-yl)butyl, 5-(triazol-1-yl)pentyl and 6-(pyrrol-1-yl) hexyl.

The $C_2$-$C_6$ alkenyl which may be substituted by $R^b$ in the definitions of the $C_2$-$C_6$ alkenyloxy which may be substituted by $R^b$, the $C_2$-$C_6$ alkenylsulfenyl which may be substituted by $R^b$, the $C_2$-$C_6$ alkenylsulfinyl which may be substituted by $R^b$ and the $C_2$-$C_6$ alkenylsulfonyl which may be substituted by $R^b$, in $R^c$ and Y', may, for example, be a linear or branched alkenyl, such as ethenyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 1-methyl-2-propenyl, 2-methyl-2-propenyl, 1,1-dimethyl-2-propenyl, 1,2-dimethyl-2-propenyl, 1-ethyl-2-propenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,3-dimethyl-2-butenyl, 2,3-dimethyl-3-butenyl, 3,3-dimethyl-2-butenyl, 1-ethyl-2-butenyl, 1-ethyl-3-butenyl, 2-ethyl-2-butenyl, 2-ethyl-3-butenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1,2-trimethyl-2-propenyl, 1-ethyl-1-methyl-2-propenyl, 2-chloroethenyl, 2-bromoethenyl, 2,2-dichloroethenyl, 3-chloro-2-propenyl, 3-fluoro-2-propenyl, 3-bromo-2-propenyl, 3-iodo-2-propenyl, 3,3-dichloro-2-propenyl, 3,3-difluoro-2-propenyl, 4-chloro-2-butenyl, 4,4-dichloro-3-butenyl and 4,4-difluoro-3-butenyl.

The $C_2$-$C_6$ alkynyl which may be substituted by $R^b$ in the definitions of the $C_2$-$C_6$ alkynyloxy which may be substituted by $R^b$, the $C_2$-$C_6$ alkynylsulfenyl which may be substituted by $R^b$, the $C_2$-$C_6$ alkynylsulfinyl which may be substituted by $R^b$ and the $C_2$-$C_6$ alkynylsulfonyl which may be substituted by $R^b$, in $R^c$ and Y', may, for example, be a linear or branched alkynyl, such as ethynyl, 1-propynyl, 2-propynyl, 1-methyl-2-propynyl, 1,1-dimethyl-2-propynyl, 1-methyl-1-ethyl-2-propynyl, 1-butynyl, 2-butynyl, 3-butynyl, 1-methyl-2-butynyl, 1-methyl-3-butynyl, 2-methyl-3-butynyl, 1,1-dimethyl-2-butynyl, 1,1-dimethyl-3-butynyl, 1,2-dimethyl-3-butynyl, 2,2-dimethyl-3-butynyl, 1-ethyl-2-butynyl, 1-ethyl-3-butynyl, 2-ethyl-3-butynyl, 1-pentynyl, 2-pentynyl, 3-pentynyl, 4-pentynyl, 1-methyl-3-pentynyl, 1-methyl-4-pentynyl, 2-methyl-3-pentynyl, 2-methyl-4-pentynyl, 3-methyl-4-pentynyl, 4-methyl-2-pentynyl, hexynyl, chloroethynyl, bromoethynyl, iodoethynyl, 3-chloro-2-propynyl, 3-bromo-2-propynyl, 3-iodo-2-propynyl, 4-bromo-3-butynyl, 4-iodo-3-butynyl and 6-iodo-5-hexynyl.

The $C_1$-$C_6$ alkoxy which may be substituted by $R^b$ in the definition of the $C_1$-$C_6$ alkoxycarbonyl which may be substituted by $R^b$, in $R^c$ and Y', may, for example, be a linear or branched alkoxy, such as methoxy, ethoxy, n-propoxy, i-propoxy, n-butoxy, i-butoxy, s-butoxy, t-butoxy, n-pentyloxy, n-hexyloxy, 1,1-dimethylpropoxy, 1,2-dimethylpropoxy, 2,2-dimethylpropoxy, 1-ethylpropoxy, 1,1,2-trimethylpropoxy, 1,2,2-trimethylpropoxy, 1-ethyl-1-methylpropoxy, 1-ethyl-2-methylpropoxy, 1-methylbutoxy, 2-methylbutoxy, 3-methylbutoxy, 1-ethylbutoxy, 2-ethylbutoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-methylpentyloxy, 2-methylpentyloxy, 3-methylpentyloxy, 4-methylpentyloxy, fluoromethoxy, chloromethoxy, bromomethoxy, iodomethoxy, dichloromethoxy, trichloromethoxy, difluoromethoxy, trifluoromethoxy, chlorodifluoromethoxy, bromodifluoromethoxy, dichlorofluoromethoxy, 1-chloroethoxy, 1-bromoethoxy, 1-iodoethoxy, 1-fluoroethoxy, 2-chloroethoxy, 2-bromoethoxy, 2-iodoethoxy, 2-fluoroethoxy, 2,2-difluoroethoxy, 2,2,2-trifluoroethoxy, 2,2,2-trichloroethoxy, pentafluoroethoxy, 2,2,2-trifluoro-1-chloroethoxy, 1,1,2,2-tetrafluoroethoxy, 3-bromopropoxy, 1-fluoro-i-propoxy, 1-chloro-i-propoxy, 3-fluoropropoxy, 3-chloropropoxy, heptafluoropropoxy, 1,1,2,2,3,3-hexafluoropropoxy, 4-chlorobutoxy, 4-fluorobutoxy, 5-chloropentyloxy, 5-fluoropentyloxy, 6-chlorohexyloxy, 6-fluorohexyloxy, benzyloxy, 2-chlorobenzyloxy, 3-bromobenzyloxy, 4-chlorobenzyloxy, 4-methylbenzyloxy, 4-t-butylbenzyloxy, 2-methylbenzyloxy, 2-methoxybenzyloxy, 1-phenylethyloxy, 1-(3-chlorophenyl)ethyloxy, 2-phenylethyloxy, 1-methyl-1-phenylethyloxy, 1-(4-chlorophenyl)-1-methylethyloxy, 1-(3-chlorophenyl)-1-methylethyloxy, 1-phenylpropyloxy, 2-phenylpropyloxy, 3-phenylpropyloxy, 1-phenylbutyloxy, 2-phenylbutyloxy, 3-phenylbutyloxy, 4-phenylbutyloxy, 1-metkyl-1-phenylpropyloxy, 1-methyl-2-phenylpropyloxy, 1-methyl-3-phenylpropyloxy, 2-methyl-2-phenylpropyloxy, 2-(4-chlorophenyl)-2-methylpropyloxy, 2-methyl-2-(3-methylphenyl)propyloxy, 1-phenylpentyloxy, 2-phenylpentyloxy, 3-penylpentyloxy, 4-phenylpentyloxy, 5-phenylpentyloxy, 1-methyl-1-phenylbutyloxy, 1-methyl-2-phenylbutyloxy, 1-methyl-3-phenylbutyloxy, 1-methyl-4-phenylbutyloxy, 2-methyl-2-phenylbutyloxy, 2-(4-chlorophenyl)-2-methylbutyloxy, 2-methyl-2-(3-methylphenyl)butyloxy, 1-phenylhexyloxy, 2-phenylhexyloxy, 3-phenylhexyloxy, 4-phenylhexyloxy, 5-phenylhexyloxy, 6-phenylhexyloxy, 1-methyl-1-phenylpentyloxy, 1-methyl-2-phenylpentyloxy, 1-methyl-3-phenylpentyloxy, 1-methyl-4-phenylpentyloxy, 2-methyl-2-phenylpentyloxy, 2-(4-chlorophenyl)-2-methylpentyloxy, 2-methyl-2-(3-methylphenyl)pentyloxy, pyridin-2-ylmethyloxy, 5-chlorothiophen-2-ylmethyloxy, 1-methyl-3-chloropyrazol-5-ylmethyloxy, 2-(3-methylfuran-2-yl)ethyloxy, 3-(6-trifluoromethylpyridin-2-yl)propyloxy, 4-(pyrimidin-2-yl)butyloxy, 5-(1,2,4-triazol-1-yl)pentyloxy and 6-(pyrrol-1-yl)hexyloxy.

The $C_1$-$C_{12}$ alkyl which may be substituted by $R^b$ in the definitions of $R^c$ and Y', may, for example, be a linear or branched alkyl, such as methyl, ethyl, n-propyl, i-propyl, n-butyl, i-butyl, t-butyl, s-butyl, n-pentyl, n-hexyl, n-heptyl, n-octyl, n-nonyl, n-decanyl, 2-ethylpropyl, 2,2-dimethylpropyl, 1,2-dimethylpropyl, 1,1,2-trimethylpropyl, 1,2,2-trimethylpropyl, 1-ethyl-1-methylpropyl, 1-ethyl-2-methylpropyl, 1-methylbutyl, 2-methylbutyl, 3-methylbutyl, 1,1-dimethylbutyl, 1,2-dimethylbutyl, 1,3-dimethylbutyl, 2,2-dimethylbutyl, 2,3-dimethylbutyl, 3,3-dimethylbutyl, 1-ethylbutyl, 2-ethylbutyl, 1-methylpentyl, 2-methylpentyl, 3-methylpentyl, 4-methylpentyl, 2-ethylhexyl, fluoromethyl, chloromethyl, bromomethyl, iodomethyl, difluoromethyl, chlorodifluoromethyl, bromodifluoromethyl, trifluoromethyl, dichloromethyl, trichloromethyl, 1-chloroethyl, 1-bromoethyl, 1-iodoethyl, 1-fluoroethyl, 2-chloroethyl, 2-bromoethyl, 2-iodoethyl, 2-fluoroethyl, 2,2-difluoroethyl, 2,2,2-trifluoroethyl, 2,2,2-trichloroethyl, pentafluoroethyl, 2,2,2-trifluoro-1-chloroethyl, 3-fluoropropyl, 3-chloropropyl, 1-fluoro-i-propyl, 1-chloro-i-propyl, heptafluoropropyl, 1,1,2,2,3,3-hexafluoropropyl, 4-chlorobutyl, 4-fluorobutyl, 5-chloropentyl, 5-fluoropentyl, 6-chlorohexyl, 6-fluorohexyl, 7-fluoroheptyl, 8-chlorooctyl, methoxymethyl, ethoxymethyl, n-propoxymethyl, i-propoxymethyl, n-butoxymethyl, i-butoxymethyl, s-butoxymethyl, t-butoxymethyl, n-pentyloxymethyl, 2-methoxyethyl, 3-ethoxypropyl, 3-methoxypropyl, methylthiomethyl, ethylthiomethyl, n-propylthiomethyl, i-propylthiomethyl, n-butylthiomethyl, i-butylthiomethyl, s-butylthiomethyl, t-butylthiomethyl, n-pentylthiomethyl, 2-methylthioethyl, 3-ethylthiopropyl, 3-methylthiopropyl, benzyl, 2-chlorobenzyl, 3-bromobenzyl, 4-chlorobenzyl, 4-methylbenzyl, 4-t-butylbenzyl, 2-methylbenzyl, 2-methoxybenzyl, 1-phenylethyl, 1-(3-chlorophenyl) ethyl, 2-phenylethyl, 1-methyl-1-phenylethyl, 1-(4-chlorophenyl)-1-methylethyl, 1-(3-chlorophenyl)-1-methylethyl, 1-phenylpropyl, 2-phenylpropyl, 3-phenylpropyl, 1-phenylbutyl, 2-phenylbutyl, 3-phenylbutyl, 4-phenylbutyl, 1-methyl-2-phenylpropyl, 1-methyl-2-phenylpropyl, 1-methyl-3-phenylpropyl, 2-methyl-2-phenylpropyl, 2-(4-chlorophenyl)-2-methylpropyl, 2-methyl-2-(3-methylphenyl)propyl, 1-phenylpentyl, 2-phenylpentyl, 3-phenylpentyl, 4-phenylpentyl, 5-phenylpentyl, 1-methyl-1-phenylbutyl, 1-methyl-2-phenylbutyl, 1-methyl-3-phenylbutyl, 1-methyl-4-phenylbutyl, 2-methyl-2-phenylbutyl, 2-(4-chlorophenyl)-2-methylbutyl, 2-methyl-2-(3-methylphenyl)butyl, 1-phenylhexyl, 2-phenylhexyl, 3-phenylhexyl, 4-phenylhexyl, 5-phenylhexyl, 6-phenylhexyl, 1-methyl-1-phenylpentyl, 1-methyl-2-phenylpentyl, 1-methyl-3-phenylpentyl, 1-methyl-4-phenylpentyl, 2-methyl-2-phenylpentyl, 2-(4-chlorophenyl)-2-methylpentyl, 2-methyl-2-(3-methylphenyl)pentyl, pyridin-2-ylmethyl, 5-chlorothiophen-2-ylmethyl, 1-methyl-3-chloropyrazol-5-ylmethyl, 2-(3-methylfuran-2-yl)ethyl, 3-(6-trifluoromethylpyridin-2-yl)propyl, 4-(pyrimidin-2-yl)butyl, 5-(1,2,4-triazol-1-yl)pentyl, 6-(pyrrol-1-yl)hexyl, cyclopropylmethyl, cyclobutylmethyl, cyclopentylmethyl, cyclohexylmethyl, 2,2-dichlorocyclopropylmethyl, 1-phenylpyrazole-5-carboxymetyl, tetrahydropyran-2-ylmethyl, imidazole-1-ylmethyl, 2-difluoromethoxyethyl, 2-methylsulfenylethyl, 3-cyanopropyl, 2-formyl-2-methylpropyl, 4-methoxycarbonyl-4-cyanobutyl, 5-(2-chlorophenyl)pentyl, 1-phenyl-1-methoxymethyl, 1-phenyl-1-ethoxymethyl, 1-(2-chlorophenyl)-1-methoxymethyl, 1-(3-chlorophenyl)-1-methoxymethyl, 1- (4-chlorophenyl)-1-methoxymethyl, 1-(2-fluorophenyl)-1-methoxymethyl, 1-(3-fluorophenyl)-1-methoxymethyl, 1- (4-fluorophenyl)-1-methoxymethyl, 1-(2-methylphenyl)-1-methoxymethyl, 1-(3-methylphenyl)-1-methoxymethyl, 1-(4-methylphenyl)-1-methoxymethyl, 1-phenyl-1-chloromethyl, 1-phenyl-1,1-dimethoxymethyl and 6-morpholinohexyl.

The $C_1$-$C_{12}$ alkenyl which may be substituted by $R^b$ in the definitions of $R^c$ and Y', may, for example, be a linear or branched alkenyl, such as ethenyl, 1-propenyl, 2-propenyl, 1-butenyl, 2-butenyl, 3-butenyl, 1-pentenyl, 2-pentenyl, 3-pentenyl, 4-pentenyl, 1-hexenyl, 2-hexenyl, 3-hexenyl, 4-hexenyl, 5-hexenyl, 6-heptenyl, 7-octenyl, 8-nonenyl, 9-decenyl, 1-methyl-2-propenyl, 2-methyl-2-propenyl, 1,1-dimethyl-2-propenyl, 1,2-dimethyl-2-propenyl, 1-ethyl-2-propenyl, 1-methyl-2-butenyl, 2-methyl-2-butenyl, 3-methyl-2-butenyl, 1-methyl-3-butenyl, 2-methyl-3-butenyl, 3-methyl-3-butenyl, 1,1-dimethyl-2-butenyl, 1,1-dimethyl-3-butenyl, 1,2-dimethyl-2-butenyl, 1,2-dimethyl-3-butenyl, 1,3-dimethyl-2-butenyl, 1,3-dimethyl-3-butenyl, 2,3-dimethyl-2-butenyl, 2,3-dimethyl-3-butenyl, 3,3-dimethyl-2-butenyl, 1-ethyl-2-butenyl, 1-ethyl-3-butenyl, 2-ethyl-2-butenyl, 2-ethyl-3-butenyl, 1-methyl-2-pentenyl, 2-methyl-2-pentenyl, 3-methyl-2-pentenyl, 4-methyl-2-pentenyl, 1-methyl-3-pentenyl, 2-methyl-3-pentenyl, 3-methyl-3-pentenyl, 4-methyl-3-pentenyl, 1-methyl-4-pentenyl, 2-methyl-4-pentenyl, 3-methyl-4-pentenyl, 4-methyl-4-pentenyl, 1,1,2-trimethyl-2-propenyl, 1-ethyl-1-methyl-2-propenyl, 2-chloroethenyl, 2-bromoethenyl, 2,2-dichloroethenyl, 3-chloro-2-propenyl, 3-fluoro-2-propenyl, 3-bromo-2-propenyl, 3-iodo-2-propenyl, 3,3-dichloro-2-propenyl, 3,3-difluoro-2-propenyl, 4-chloro-2-butenyl, 4,4-dichloro-3-butenyl, 4,4-difluoro-3-butenyl, 2-phenylethenyl, 3-cyano-2-propenyl, 4-(4-chlorophenyl)-4-ethoxycarbonyl-3-butenyl, 3-(thiazole-2-carbonyloxy)-4-methoxy-3-butenyl, 2-phenylethenyl, 2-(4-chlorophenyl)ethenyl, 2-(3-chlorophenyl)ethenyl, 2-(2-chlorophenyl)ethenyl, 2-(4-fluorophenyl)ethenyl, 2-(3-fluorophenyl)ethenyl, 2-(2-fluorophenyl)ethenyl, 2-(4-methylphenyl)ethenyl, 2-(3-methylphenyl)ethenyl, 2-(2-methylphenyl)ethenyl, 2-phenyl-1,2-dibromoethenyl and 6-(pyrazol-1-yl)-3-hexenyl.

The $C_1$-$C_{12}$ alkynyl which may be substituted by $R^b$ in the definitions of $R^c$ and Y', may, for example, be a linear or branched alkynyl, such as ethynyl, 1-propynyl, 2-propynyl, 1-methyl-2-propynyl, 1,1-dimethyl-2-propynyl, l-methyl-1-ethyl-2-propynyl, 1-butynyl, 2-butynyl, 3-butynyl, 1-methyl-2-butynyl, 1-methyl-3-butynyl, 2-methyl-3-butynyl, 1,1-dimethyl-2-butynyl, 1,1-dimethyl-3-butynyl, 1,2-dimethyl-3-butynyl, 2,2-dimethyl-3-butynyl, 1-ethyl-2-butynyl, 1-ethyl-3-butynyl, 2-ethyl-3-butynyl, 1-pentynyl, 2-pentynyl, 3-pentynyl, 4-pentynyl, 1-methyl-3-pentynyl, 1-methyl-4-pentynyl, 2-methyl-3-pentynyl, 2-methyl-4-pentynyl, 3-methyl-4-pentynyl, 4-methyl-2-pentynyl, hexynyl, chloroethynyl, bromoethynyl, iodoethynyl, 3-chloro-2-propynyl, 3-bromo-2-propynyl, 3-iodo-2-propynyl, 4-bromo-3-butynyl, 4-iodo-3-butynyl, 6-iodo-5-hexynyl, 4-(2-chlorothiazol-5-yl)-3-butynyl, 5-formyl-3-pentynyl, 6-methylsulfenyl-5-hexynyl, 2-phenylethynyl and 3-cyano-5-hexynyl.

The $C_1$-$C_{12}$ alkoxy which may be substituted by $R^b$ in the definitions of $R^c$ and Y', may, for example, be a linear or branched alkoxy, such as methoxy, ethoxy, n-propoxy, i-propoxy, n-butoxy, i-butoxy, s-butoxy, t-butoxy, n-pentyloxy, n-hexyloxy, 1,1-dimethylpropoxy, 1,2-dimethylpropoxy, 2,2-dimethylpropoxy, 1-ethylpropoxy, 1,1,2-trimethylpropoxy, 1,2,2-trimethylpropoxy, 1-ethyl-1-methylpropoxy, 1-ethyl-2-methylpropoxy, 1-methylbutoxy, 2-methylbutoxy, 3-methylbutoxy, 1-ethylbutoxy, 2-ethylbutoxy, 1,1-dimethylbutoxy, 1,2-dimethylbutoxy, 1,3-dimethylbutoxy, 2,2-dimethylbutoxy, 2,3-dimethylbutoxy, 3,3-dimethylbutoxy, 1-methylpentyloxy, 2-methylpentyloxy, 3-methylpentyloxy, 4-methylpentyloxy, n-heptyloxy, n-octyloxy, n-nonyloxy, n-decanyloxy, fluoromethoxy, chloromethoxy, bromomethoxy, iodomethoxy, dichloromethoxy, trichloromethoxy, difluoromethoxy, trifluoromethoxy, chlorodifluoromethoxy, bromodifluoromethoxy, dichlorofluoromethoxy, 1-chloroethoxy, 1-bromoethoxy, 1-iodoethoxy, 1-fluoroethoxy, 2-chloroethoxy, 2-bromoethoxy, 2-iodoethoxy, 2-fluoroethoxy, 2,2-difluoroethoxy, 2,2,2-trifluoroethoxy, 2,2,2-trichloroethoxy, pentafluoroethoxy, 2,2,2-trifluoro-1-chloroethoxy, 1,1,2,2-tetrafluoroethoxy, 3-bromopropoxy, 1-fluoro-i-propoxy, 1-chloro-i-propoxy, 3-fluoropropoxy, 3-chloropropoxy, heptafluoropropoxy, 1,1,2,2,3,3-hexafluoropropoxy, 4-chlorobutoxy, 4-fluorobutoxy, 5-chloropentyloxy, 5-fluoropentyloxy, 6-chlorohexyloxy, 6-fluorohexyloxy, benzyloxy, 2-chlorobenzyloxy, 3-bromobenzyloxy, 4-chlorobenzyloxy, 4-methylbenzyloxy, 4-t-butylbenzyloxy, 2-methylbenzyloxy, 2-methoxybenzyloxy, 1-phenylethyloxy, 1-(3-chlorophenyl)ethyloxy, 2-phenylethyloxy, 1-methyl-1-phenylethyloxy, 1-(4-chlorophenyl)-1-methylethyloxy, 1-(3-chlorophenyl)-1-methylethyloxy, 1-phenylpropyloxy, 2-phenylpropyloxy, 3-phenylpropyloxy, 1-phenylbutyloxy, 2-phenylbutyloxy, 3-phenylbutyloxy, 4-phenylbutyloxy, 1-methyl-1-phenylpropyloxy, 1-methyl-2-phenylpropyloxy, 1-methyl-3-phenylpropyloxy, 2-methyl-2-phenylpropyloxy, 2-(4-chlorophenyl)-2-methylpropyloxy, 2-methyl-2-(3-methylphenyl)propyloxy, 1-phenylpentyloxy, 2-phenylpentyloxy, 3-phenylpentyloxy, 4-phenylpentyloxy, 5-phenylpentyloxy, 1-methyl-1-phenylbutyloxy, 1-methyl-2-phenylbutyloxy, 1-methyl-3-phenylbutyloxy, 1-methyl-4-phenylbutyloxy, 2-methyl-2-phenylbutyloxy, 2-(4-chlorophenyl)-2-methylbutyloxy, 2-methyl-2-(3-methylphenyl)butyloxy, 1-phenylhexyloxy, 2-phenylhexyloxy, 3-phenylhexyloxy, 4-phenylhexyloxy, 5-phenylhexyloxy, 6-phenylhexyloxy, 1-methyl-l-phenylpentyloxy, 1-methyl-2-phenylpentyloxy, 1-methyl-3-phenylpentyloxy, 1-methyl-4-phenylpentyloxy, 2-methyl-2-phenylpentyloxy, 2-(4-chlorophenyl)-2-methylpentyloxy, 2-methyl-2-(3-methylphenyl)pentyloxy, pyridin-2-ylmethyloxy, 5-chlorothiophen-2-ylmethyloxy, 1-methyl-3-chloropyrazol-5-ylmethyloxy, 2-(3-methylfuran-2-yl)ethyloxy, 3-(6-trifluoromethylpyridin-2-yl)propyloxy, 4-(pyrimidin-2-yl)butyloxy, 5-(triazol-1-yl)pentyloxy, 6-(pyrrol-1-yl)hexyloxy, 1-phenylpyrazole-5-carboxymethyloxy, tetrahydropyran-2-ylmethyloxy, imidazol-1-ylmethyloxy, 2-difluoromethoxyethyloxy, 2-methylsulfenylethyloxy, 3-cyanopropyloxy, 2-formyl-2-methylpropyloxy, 4-methoxycarbonyl-4-cyanobutyloxy, 5-(2-chlorophenyl)pentyloxy and 6-morpholinohexyloxy.

The $C_1$-$C_6$ alkoxy $C_1$-$C_6$ alkoxy which may be substituted by $R^b$ in the definitions of $R^c$ and Y', may, for example, be methoxymethoxy, ethoxymethoxy, n-propoxymethoxy, i-propoxymethoxy, n-butoxymethoxy, i-butoxymethoxy, s-butoxymethoxy, t-butoxymethoxy, n-pentyloxymethoxy, 2-methoxyethoxy, 3-ethoxypropoxy, 3-methoxypropoxy, cyanomethoxymethoxy, 2-(2-nitroethoxy)ethoxy, 3-(1-methylpyrazol-5-ylmethoxy)propyloxy, 4-(3-cyano-2-methylpropyloxy)butoxy, 5-benzyloxypentyloxy, and 5-(2-trifluoromethylthiazol-5-yl)methoxyhexyloxy.

The 3- to 7-membered ring which may contain from 1 to 3 oxygen atoms, nitrogen atoms or sulfur atoms, formed by two Ys substituted on the same carbon atom of A, together with the carbon atom, in the definition of Y, may, for example, be cyclopropyl, 2,2-dichlorocyclopropyl, cyclobutyl, oxetane and cyclopentyl.

The 3- to 7-membered ring which may contain from 1 to 4 hetero atoms selected from among oxygen atoms, nitrogen atoms and sulfur atoms, formed by $R^2$ and $R^3$ together, in the definition of $R^2$ and $R^3$, may, for example, be aziridine, morpholine, hexamethyleneimine and 4-benzylpiperazine.

The 3- to 7-membered ring which may contain from 1 to 4 hetero atoms selected from among oxygen atoms, nitrogen atoms and sulfur atoms, formed by $U^1$ and $U^2$ together, in the definition of $U^1$ and $U^2$, may, for example, be aziridine, morpholine, hexamethyleneimine and 4-benzylpiperazine.

A may, for example, be a 5-memebered cyclic hetero ring, a 6-membered cyclic hetero ring and a 7-membered cyclic hetero ring, preferably,

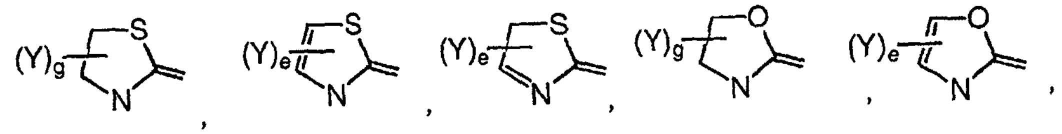

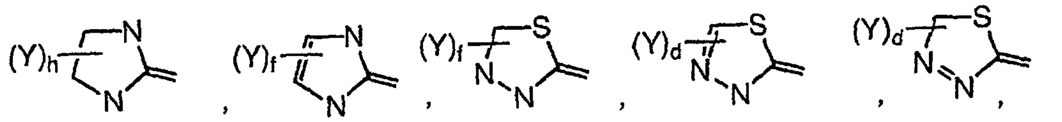

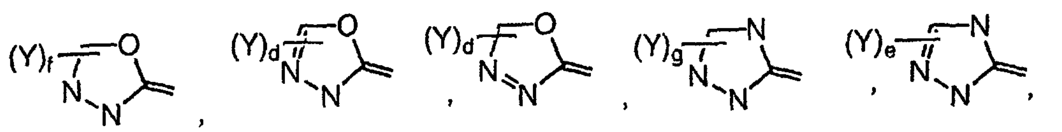

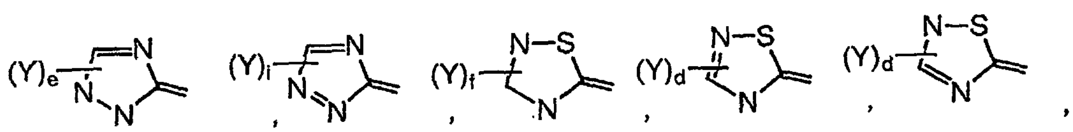

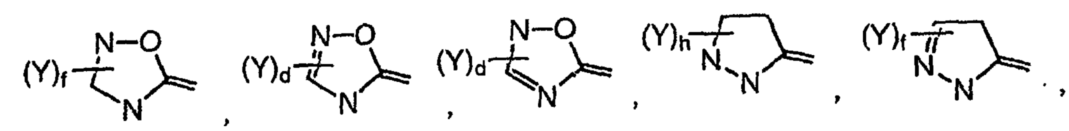

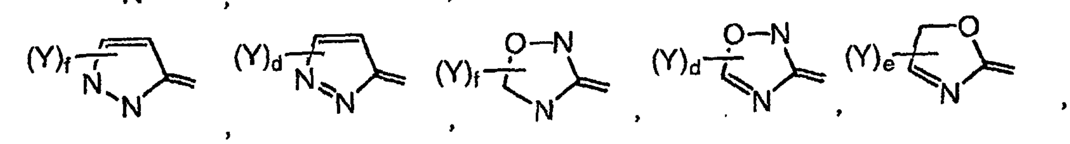

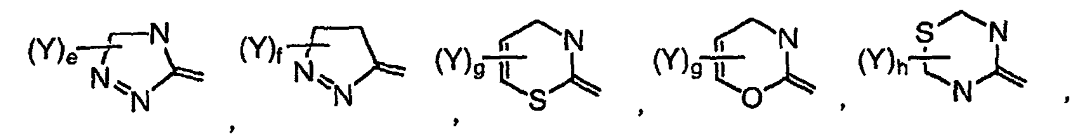

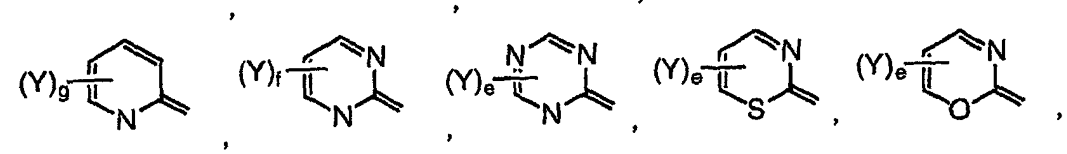

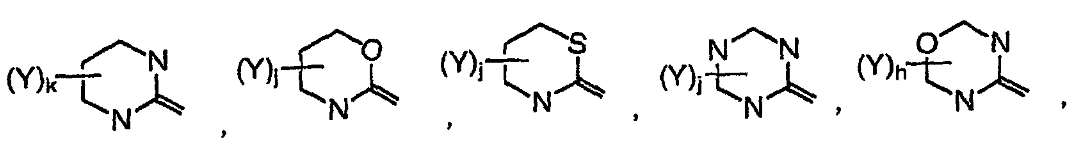

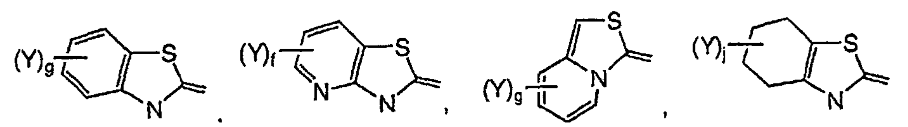

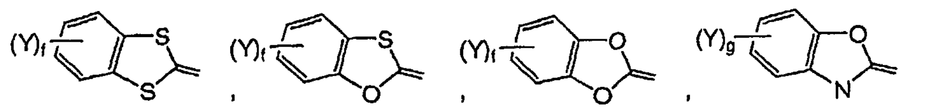

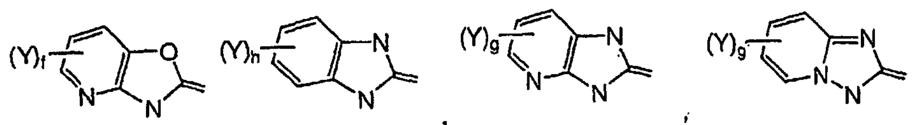

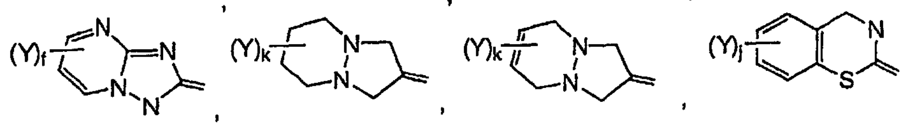

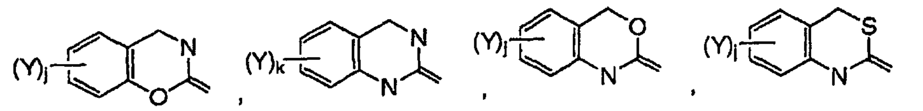

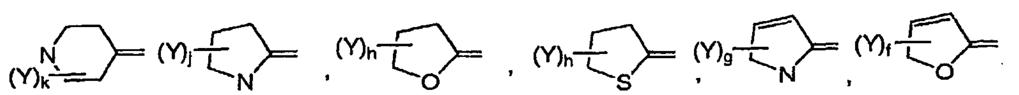

,

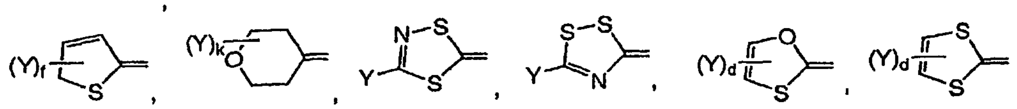

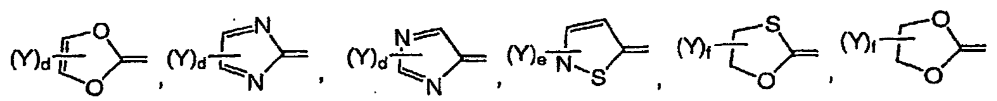

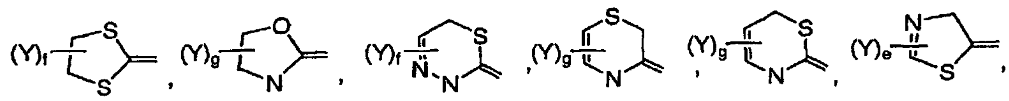

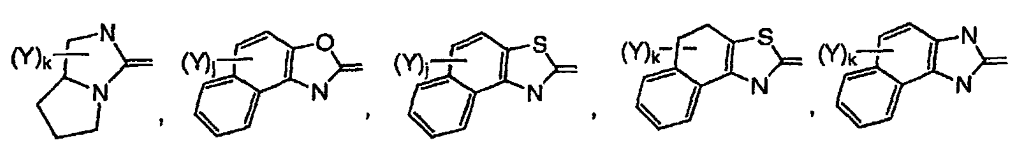

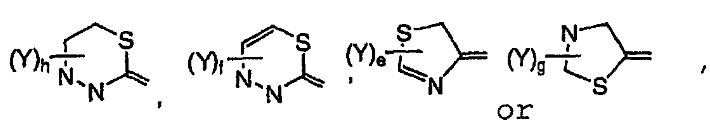

or

more preferably

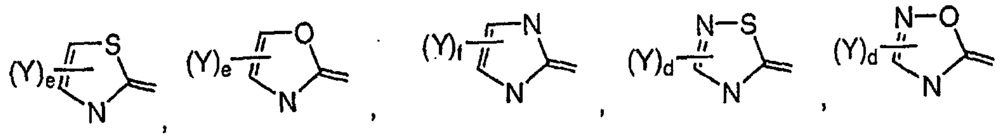

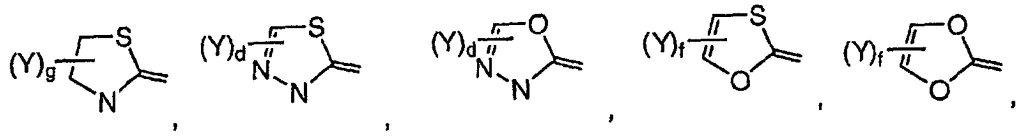

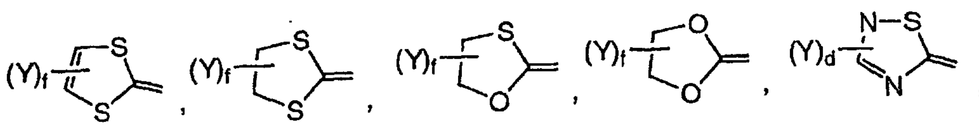

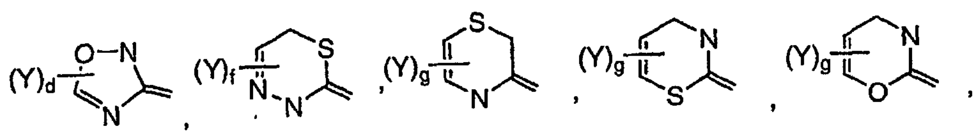

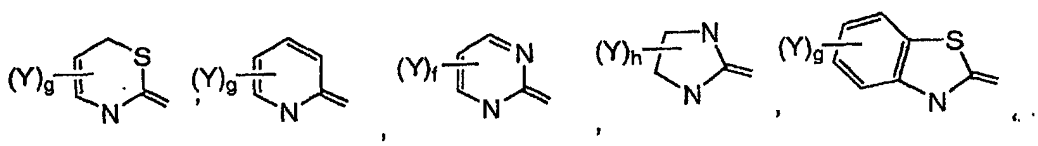

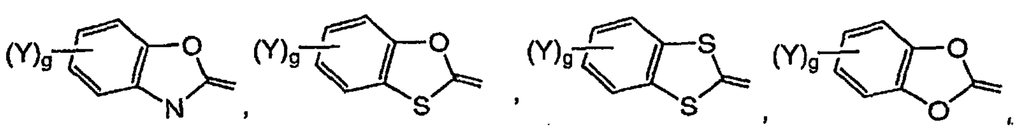

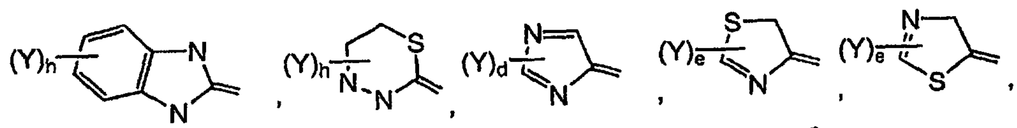

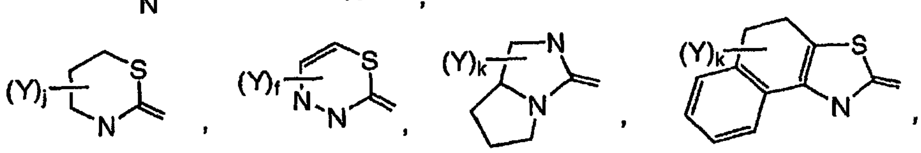

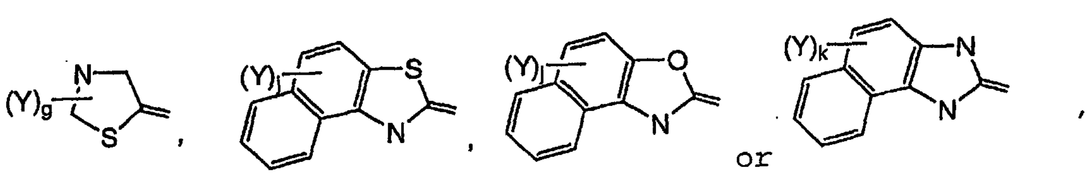

particularly preferably

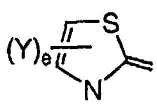, 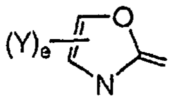, 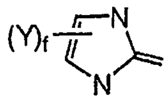, 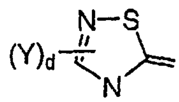, 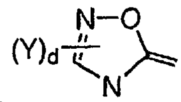,

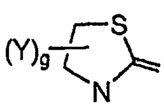, 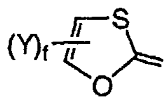, 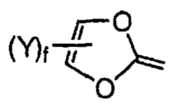, 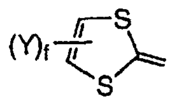, 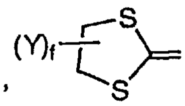,

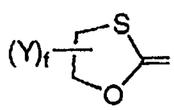, 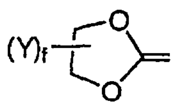, 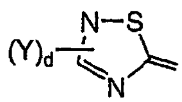, 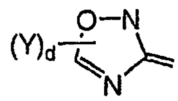, 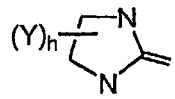,

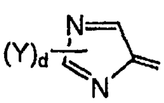, 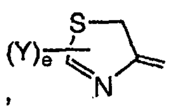, 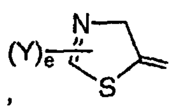, 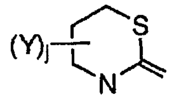, 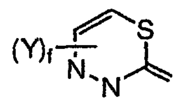,

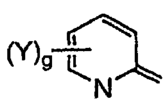, 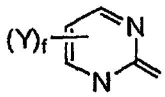, 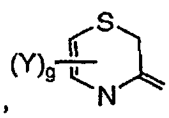, 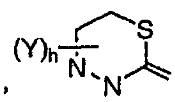, 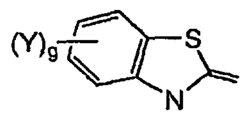,

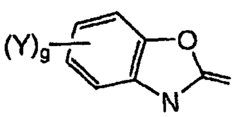, 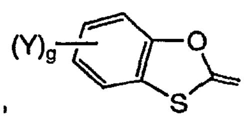, 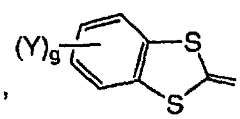 or 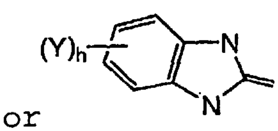,

wherein Y, d, e, f, g, h, i, j and k have the same meanings as above.

B may, for example, be $-CH_2-$, $-C(=CH-OR^4)-$ or $-C(N=OR^4)-$.

$R^1$ may, for example, be preferably a hydrogen atom, methyl, ethyl, n-propyl, i-propyl, n-butyl, i-butyl, s-butyl, n-pentyl, 3-methylbutyl, n-hexyl and benzyl, more preferably methyl.

$R^2$ may, for example, be preferably a hydrogen atom, methyl, ethyl, n-propyl, i-propyl, n-butyl, i-butyl, s-butyl, n-pentyl, 3-methylbutyl, n-hexyl and benzyl, more preferably methyl.

$R^3$ may, for example, be preferably a hydrogen atom, methyl, ethyl, phenyl which may be substituted by $R^a$ and benzyl which may be substituted by $R^a$, more preferably a hydrogen atom, phenyl which may be substituted by $R^a$, and methyl.

$R^4$ may, for example, be a hydrogen atom, methyl, ethyl and benzyl, more preferably methyl.

$R^5$ may, for example, be a hydrogen atom, methyl, acetyl, phenyl and benzyl, more preferably methyl and acetyl.

$R^6$ may, for example, be a hydrogen atom, a chlorine atom, methyl, ethyl, methoxycarbonyl, methylsulfenyl, phenyl which may be substituted by $R^a$, and benzyl.

$R^7$ may, for example, be phenyl which may be substituted by $R^a$, heteroaryl which may be substituted by $R^a$, a hydrogen atom, methyl, ethyl, methoxy, benzyloxy, acetyl, and benzyl which may be substituted by $R^a$.

$R^8$ and $R^9$ may, for example, be a hydrogen atom, a chlorine atom, methyl, ethyl and benzyl.

$R^{10}$ may, for example, be a hydrogen atom, a chlorine atom, methyl and methoxy.

$R^{11}$ may, for example, be a hydrogen atom, methyl and ethyl.

$R^{12}$ may, for example, be a hydrogen atom and methyl.

$R^{13}$ may, for example, be a hydrogen atom, a chlorine atom, a bromine atom, methyl and methoxy.

$R^a$ may, for example, be preferably a halogen atom, a $C_1$-$C_6$ alkyl, $C_1$-$C_6$ alkoxy, $C_1$-$C_6$ haloalkyl, $C_1$-$C_6$ haloalkoxy, CN, nitro and $C_1$-$C_6$ alkoxycarbonyl, more preferably Cl, F, Br, trifluoromethyl, methoxy, ethoxy, ethyl, propyl and methyl.

$R^b$ may, for example, be preferably a halogen atom, $C_1$-$C_6$ alkoxy, $C_1$-$C_6$ alkylsulfenyl, phenyl which may be substituted by $R^a$, heteroaryl which may be substituted by $R^a$, CN, nitro and $C_1$-$C_6$ alkoxycarbonyl.

$R^c$ may, for example, be preferably a halogen atom, phenyl which may be substituted by $R^a$, heteroaryl which may be substituted by $R^a$, phenylcarbonyl which may be substituted by $R^a$, phenylsulfonyl which may be substituted by $R^a$, $C_1$-$C_6$ alkyl which may be substituted by $R^b$, $C_2$-$C_6$ alkenyl which may be substituted by $R^b$, $C_2$-$C_6$ alkynyl which may be substituted by $R^b$, $C_1$-$C_6$ alkoxy which may be substituted by $R^b$, $C_1$-$C_6$ alkylsulfenyl which may be substituted by $R^b$, CN, nitro, OH, SH, SCN and $C_1$-$C_6$ alkoxycarbonyl.

X may, for example, be preferably a halogen atom, $C_1$-$C_4$ alkyl, $C_1$-$C_4$ alkoxy, $C_1$-$C_2$ haloalkyl, $C_1$-$C_2$ haloalkoxy, CN, nitro, S-R, $NU^1U^2$, phenylcarbonyl which may be substituted by $R^a$ and $C_1$-$C_4$ alkoxycarbonyl, more preferably Cl, F, I, Br, methoxy, ethyl, n-propyl, ethoxy, n-propoxy, chlorodifluoromethyl, trifluoromethyl, trifluoromethoxy, difluoromethoxy, methoxycarbonyl, pentafluoroethyl, ethoxycarbonyl, CN, acetyl and methyl.

Y' may, for example, be preferably a hydrogen atom, a halogen atom, $C_1$-$C_6$ alkyl which may be substituted by $R^b$, $C_1$-$C_6$ alkoxy which may be substituted by $R^b$, phenyl which may be substituted by $R^c$, phenoxy which may be substituted by $R^c$, phenyl $C_1$-$C_6$ alkyl which may be substituted by $R^c$, heteroaryl which may be substituted by $R^c$, CN, nitro and $C_1$-$C_6$ alkoxycarbonyl.

$U^1$ and $U^2$ may, for example, be preferably a hydrogen atom, $C_1$-$C_4$ alkyl, $C_1$-$C_2$ haloalkyl, phenyl, heteroaryl, $C_1$-$C_4$ alkylcarbonyl and $C_1$-$C_4$ alkoxycarbonyl, more preferably H, methyl, phenyl, benzyl, acetyl and methoxycarbonyl.

D may, for example, be preferably a single bond, -C(=$Q^2$)- and -C($R^6$)=N-O-.

$Q^1$, $Q^2$ and $Q^3$ may, for example, be preferably =O, =S, =N-$R^7$ and =$CH_2$.

$Q^4$ and $Q^5$ may, for example, be preferably =O and =S.

G may, for example, be $G^1$, $G^2$, $G^3$, $G^4$, $G^5$, $G^6$, $G^7$ and $G^8$, preferably $G^1$, $G^2$, $G^3$ and $G^4$, more preferably $G^1$.

n is preferably 0, 1 or 2.

p is preferably 0 or 1.

Further, the agrochemically acceptable salt of the heterocyclic imino compound of the present invention may, for example, be a hydrochloride, a hydrobromide, a hydroiodide, a formate, an acetate, an ammonium salt, an isopropylamine salt and an oxalate.

Now, plant diseases to be controlled by the compounds of the present invention include, for example:

Rice: blast (Pyricularia oryzae), sesame leaf blotch (Cochliobolms miyabeanus), sheath blight (Rhizoctonia solani),
Barley, wheat, etc.: powdery mildew (Erysiphe graminis f. sp. hordei, f. sp. tritici), stripe (Pyrenophora graminea), net blotch (Pyrenophora teres), scab (Gibberella zeae), stripe rust (Puccinia striiformis, P. graminis, P. recondita, P. hordei), snow-rot (Tipula sp., Micronectria nivalis), loose smut (Ustilago tritici, U. nuda), ice pot (Pseudocercosporella herpotrichoides), scald (Rhynchosporium secalis), speckled leaf blotch (Septoria tritici), glume-blotch (Leptosphaeria nodorum),
Citrus fruit: phoma rot (Diaporthe citri), scab (Elsinoe fawcetti), common green mold (Penicillium digitalum, P. italicum),
Apple: blossom blight (Sclerotinia mali), canker (Valsa mali), powdery mildew (Podosphaera Icuchotricha), alternaria leaf spot (Alternaria mali) , scab (Ventura inaequalis),
Pear: scab (Venturia nashicola), black scab (Alternaria kikuchiana), rust (Gymnosporangium haraenum),
Peach: brown rot (Sclerotinia cinerea), scab (Cladosporium carpophilum), phomopsis (Phomopsis sp.),
Grape: downy mildew (Plasmopara viticola), anthracnose (Elsinoe ampelina), ripe rot (Glomerella cingulate), powdery mildew (Uncinula necator), rust (Phakopsora ampelopsidis),
Japanese persimmon: anthracnose (Gloeosporium kaki), angular leaf spot (Cercospora kaki, Mycosphaerella hawae),
Cucumber: downy mildew (Pseudoperonospora cubensis), anthracnose (Colletotrichum lagenarium), powdery mildew (Sphaerotheca fuliginea), gummy stem blight (Mycosphaerella melonis),
Tomato: late blight (Phytophthora infestans), early blight (Alternaria solani), leaf mold (Cladosporium fulvum),
Eggplant: early blight (Phomopsis vexans), powdery mildew (Erysiphe cichoracoarum),
Rape: black rot (Alternaria japonica), white spot (Cercosporella brassicae),
Welsh onion: rust (Puccinia allii),
Soy bean: purple speck (Cercospora kikuchii), sphaceloma scab (Elsinoe glycines), pod and stem blight (Diaporthe phaseololum),
Kidney bean: anthracnose (Colletotrichum lindemuthianum),
Peanut: leaf spot (Mycosphaerella personatum), leaf spot (Cercospora arachidicola),
Garden pea: powdery mildew (Erysiphe pisi),

Potato: early blight (Alternaria solani),
Strawberry: powdery mildew (Sphaerotheca humuli),
Tea: net blister blight (Exobasidium reticulatum), scab (Elsinoe leucospila),
Tobacco: brown-spot (Alternaria lingipes), powdery mildew (Erysiphe cichoracearum), anthracnose (Colletotrichum tabacum),
Sugar beet: cercospora leaf spot (Cercospora beticola),
Rose: black spot (Diplocarpon rosae), powdery mildew (Sphaerotheca pannosa),
Chrysanthemum: leaf blight (Septoria chrysanthemiindici), rust (Puccinia horiana),
Various crop plants: gray mold (Botrytis cinerea),
Various crop plants: sclerotinia rot (Sclerotinia sclerotiorum).

Even at low concentration, the compounds of the present invention effectively prevent various pests, which include, for example, so-called agricultural insect pests that injure agricultural and horticultural crops and trees, so-called livestock insect pests that live on livestock and poultry, so-called sanitary insect pests that have various negative influences on the human living environment including houses, so-called stored products insect pests that injure grains stored in storehouses, and also acarids, nematodes, molluscs and crustaceans that live in the same sites as above and injure those mentioned above.

Examples of insect pests, acarids, nematodes, molluscs and crustaceans capable of being exterminated by the compounds of the present invention are mentioned below, which, however, are not limitative. Insect pests of Lepidoptera, such as rice stem borer (Chilo suppressalis Walker), rice leafroller (Cnaphalocrocis medinalis Guenee), green rice caterpillar (Naranga aenescens Moore), rice skipper (Parnara guttata Bremer et Gvey), diamond back moths (Plutella xylostella Linne), cabbage armyworms (Mamestra brassicae Linnee), common white (Pieris rapae crucivora Boisduval), turnip moth (Agrotis segetum Denis et Schiffermuller), common cutworm (Spodptera litura Fabricius), beet armyworm (Spodoptera exigua Hubner), smaller tea tortrix (Adoxophyes sp.), oriental tea tortrix (Homona magnanima Diakonoff), peach fruit moth (Carposina niponensis Walsingham), oriental fruit moth (Grapholita molesta Busck), summer fruit tortrix (Adoxophyes orana fasciata Walsingham), apple leafminers (Phyllonorycter ringoniella Matsumura), corn earworm (Helicoverpa zea Boddie), tobacco bad worms (Heliothis virescens Fabricius), European corn borer (Ostrinia nubilalis Hubner), fall armyworm (Spodoptera frugiperda J. E. Smith), Codling moth moth (Cydia pomonella Linnee), fall webworms (Hyphantria cunea Drury), etc.;

Insect pests of Hemiptera, such as green rice leafhopper (Nephotettix cincticeps Uhler), brown rice planthoppers (Nilaparvata lugens Stal), green peach aphid (Myzus persicae Sulzer), cotton aphid (Aphis gossypii Glover), greenhouse whitefly (Trialeurodes vaporariorum Westwood), sweetpotato white fly (Bemisia tabaci Gennadius), pear psylla (Psylla pyricola Forster), azalea lace bug (Stephanitis pyrioides Scott), arrowhead scale (Unaspis yanonensis Kuwana), comstock mealybug (Pseudococcus comstocki Kuwana), red wax scale (Ceroplastes rubens Maskell), brown-marmorated stinkbug (Halyomorpha mista Uhler), cabbage bug (Eurydema rugosam Motschulsky), bed bug (Cimex lectularius Linnee), etc.;

Insect pests of Coleoptera, such as twenty-eight-spotted ladybird (Henosepilachna vigintioctopunctata Fabricius), cupreous chafers (Anomala cuprea Hope), ricewater weevil (Lissorhoptrus oryzophilus Kuschel), sweetpotato weevil (Cylas formicarius Fabricius), cucurbit leaf beetle (Aulacophora femoralis Motschulsky), striped flea beetle (Phyllotreta striolata Fablicius), white-spotted longicorn beetle (Anoplophora malasiaca Thomson), pine sawyers (Monochamus alternatus Hope), corn rootworms (Diabrotica spp.), rice weevil (Sitophilus zeamais Motschulsky), lesser rice weevil (Sitophilus oryzae Linne), granary weevils (Sitophilus granarius Linnee), red four beetle (Tribolium castaneum Herbst), etc.;

Insect pests of Diptera, such as legume leafminer (Liriomyza trifolii Burgess), seedcorn maggot (Delia platura Meigen), Hessia fly (Mayetiola destructor Say), melon fly (Dacus (Zengodacus) cucurbitae Coquillett), Mediterranear fruit fly (Ceratitis capitata Wiedemann), house flies (Musca domestica Linne), stable fly (Stomoxys calcitrans Linne), Sheep ked (Melophagus orinus), common cattle grub (Hypoderm lineatum devillers), nothern cattle grub (Hypoderma boris Linnee), sheep botfly (Oestrus ovis Linnee), tsetse fly (Golossina palpalis Robineau-Desvoidy), common gnat (Culex pipiens pallens Coquillett), yellow-fever mosquitoes (Aedes aegypti Linne), Anopheles culicifacies), etc.

Insect pests of Hymenoptera, such as cabbage sawfly (Athalis rosae ruficornis Jakovlev), pine sawfly (Neodiprion sertifer Geoffroy), chestnut sawfly (Apethymus kuri Takeuchi), etc.;

Insect pests of Thysanoptera, such as melon thrips (Thrips palmi Karny), onion thrips (Thrips tabaci Lindeman), western flower thrips (Frankliniella occidentalis Pergande), flower thrip (Frankliniella intonsa Trybom), yellow tea thrip (Scirtothrips dorsalis Hood), etc.;

Insect pests of Dictyoptera, such as smokybrown cockroache (Periplaneta fuliginosa Serville), Japanese cockroach (Periplaneta japonica Karny), German cockroaches (Blattella germanica Linne), etc.;

Insect pests of Orthoptera, such as oriental migratory locust (Locusta migratoria Linne), rice grasshopper (Oxya yezoensis Shiraki), desert locust (Schistocerca gregaria Forskal), etc.;

Insect pests of Isoptera, such as Formosan subterranean termit (Coptotermes formosanus Shiraki), (Reticulitermes (Leucotermes) speratus Kolbe), (Odontotermes formosanus Shirakif), etc.;

Insect pests of Siphonaptera, such as cat fleas (Ctenocephalides felis Bouche), human fleas (Pulex irritans Linne), oriental rat flea (Xenopsylla cheopis Rothschild), etc.;

Insect pests of Mallophaga, such as Chicken bodylouse (Menacanthus stramineus Nitsch), cattle biting louse (Bovicola bovis Linne), etc.;

Insect pests of Anoplura, such as short-nosed cattle louse (Haematopinus eurysternus Nitzsh), hog louse (Haematopinus suis Linne), longnosed cattle louse (Linognathus vituli Linne), little cattle louse (Solenopotes capillatus Enderlein), etc.

Pests of TETRANYCHIDAE, such as citrus red mite (Panonychus citri McGregor), European red mite (Panonychus ulmi Kock), two-spotted spider mite (Tetranychus urticae Koch), Kanzawa spinder mite (Tetranychus kanzawai Kishida), etc.;

Pests of ERIOPHYDAE, such as pink citrus rust mite (Aculops pelekassi Keifor), pear rust mite (Epitrimerus pyri Nalepa), dry bulb mite (Aceria tulipae Keiter), pink tea mite (Acaphylla theae watt), etc.;

Pests of TARSONEMIDAE, such as broad mites (Polyphagotarsonemus latus Banks), cyclamen mite, strawberry mite (Steneotarsonemus pallidus Banks), etc.;

Pests of ACARIDAE, such as mold mite, copra mite, forage mite (Tyrophagus putrescetiae Schrank), bulb mite (Rhizoglyphus robini Claparede), etc.;

Pests of VARROIDAE, such as bee brood mite (Varroa jacobsoni Oudemans), etc.;

Pests of Ixodidae, such as bull ticks (Boophilus microplus Canestrini), (Haemaphysalis longicornis Neumann), etc.;

Pests of Sarcoptidae, such as sarcoptes mange mite (Sarcaptes scabiei Linne), etc.;

Nematodes, such as southern root-knot nematode (Meloidogyne incognita Kofoid et White), northern root-knot nematode (Meloidogyne hapla Chitwood), Cobb root-lesion nematode (Pratylenchus penetraus Cobb), walnut root-lesion nematode (Pratylenchus vulnus Allen et Jensen), potato cyst nematode (Globodera rostochiensis Wollenweber), pine wood nematode (Bursaphelenchus xylophilus Steiner et Buhrer), etc.;

Mollusca, such as apple snail (Pomacea canaliculata Lamarck), (Incilaria pilineata Benson), (Acusta despecta sieboldiana Pfeiffer), (Euhadra peliomphala Pfeiffer), pillbug (Armadillidium vulgare Latreille), etc.;

Crustaceans, such as pillbug (Armadillidium vulgare Latreille), etc.

In addition, the compounds of the present invention are effective in preventing the attachment of aquatic organisms, even at extremely low concentrations. Aquatic organisms to which the invention is directed are, for example, shellfishes and algae, such as mussel, barnacle, oyster, hydrozoan, hydra, Serpula, ascidian, seamoss, Bagula, mud pond snail, sea lettuce, green laver, Ectocarpus, etc.

Specifically, the compounds of the present invention can effectively exterminate various pests and phytopathogenic microbes of, for example, Orthoptera, Hemiptera, Lepidoptera, Coleoptera, Hymenoptera, Diptera, Temitidae, and also mites and louses, even when used at low concentrations. In addition, the compounds of the invention are effective in preventing the attachment of various aquatic organisms living in sea water and fresh water to aquatic constructions, etc. On the other hand, the compounds of the present invention contains useful compounds that have few negative influences on mammals, fishes, shellfishes and useful insects.

Now, processes for producing compounds of the present invention will be described, wherein G in the above formula (1) is represented by $G^1$.

Processes

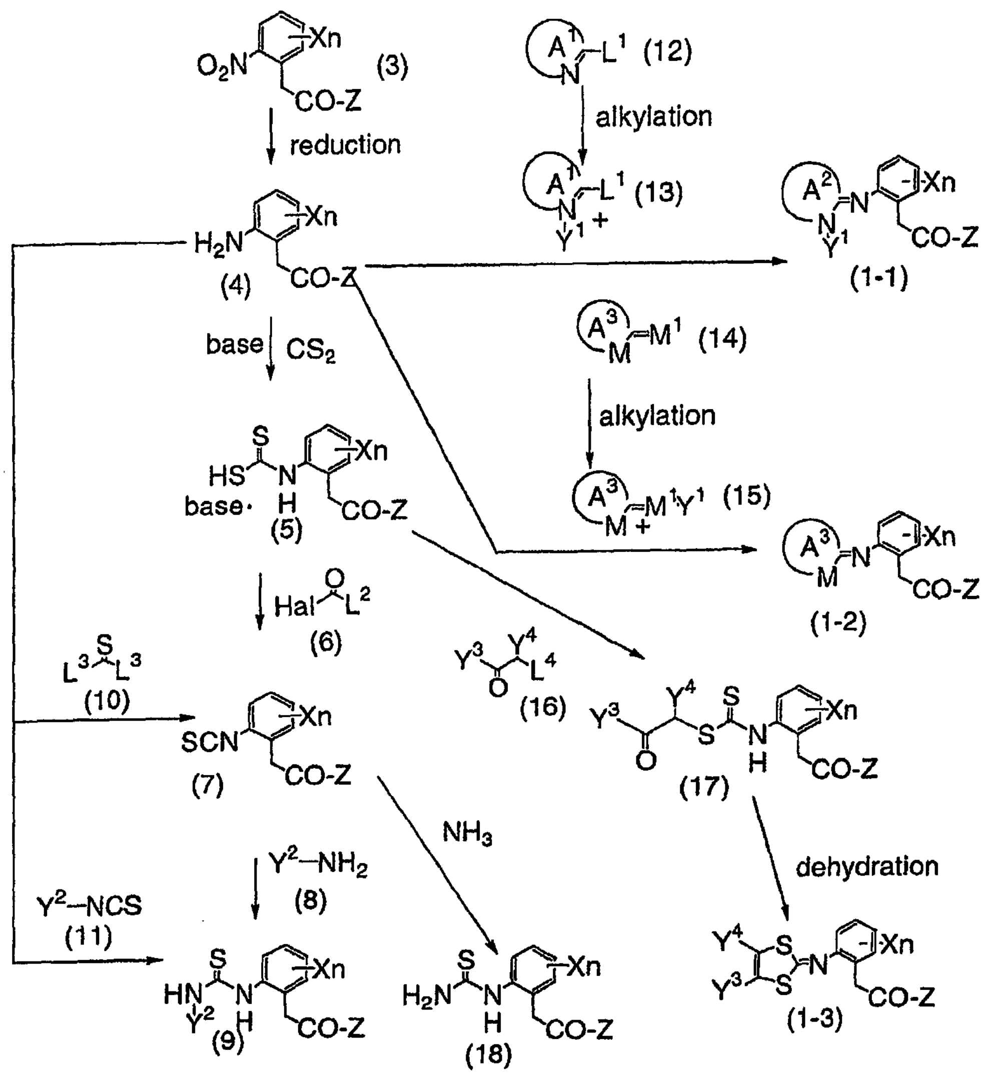

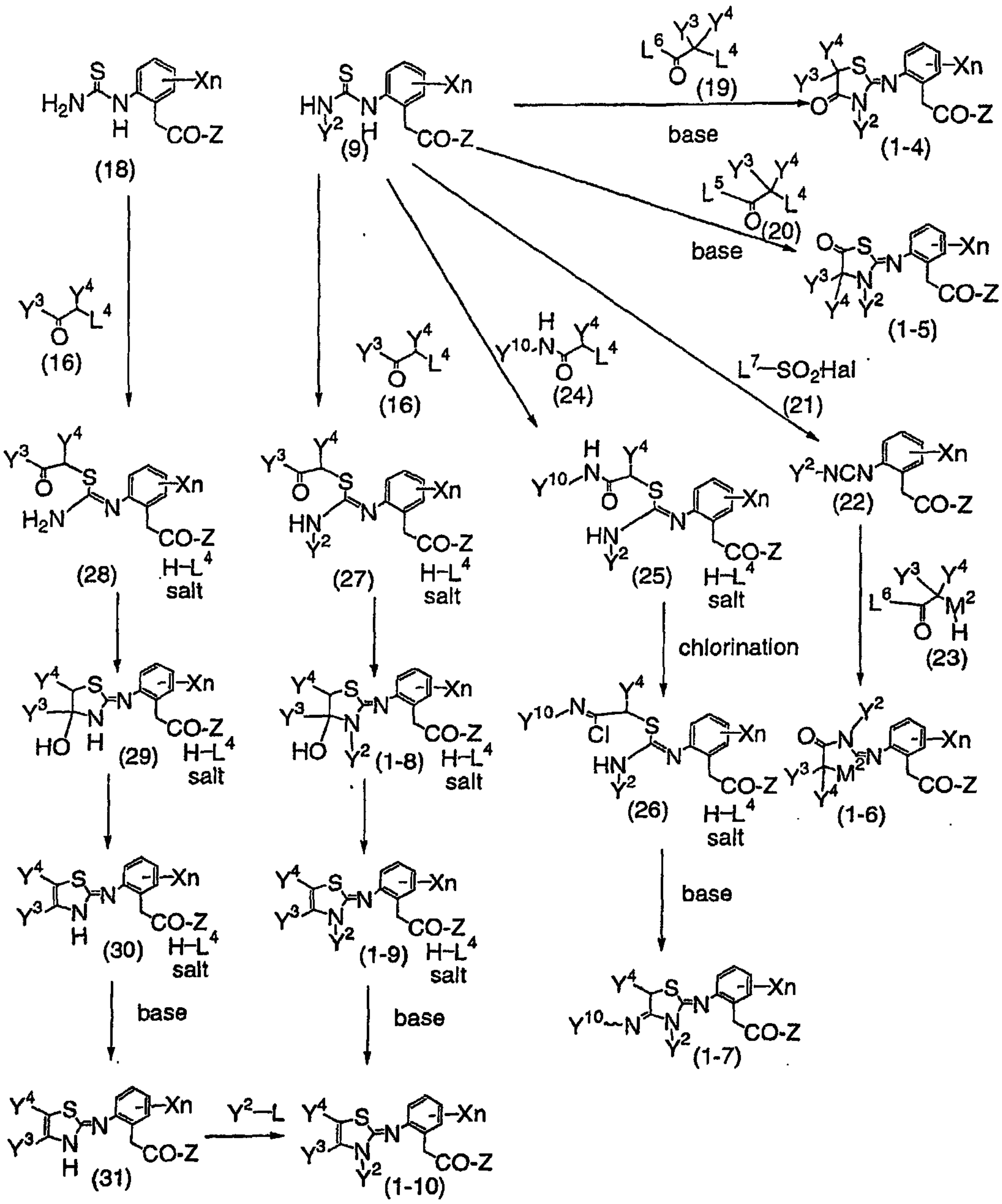
(18)
(9)
(19)
base
(1-4)
(20)
base
(1-5)
(16)
(16)
(24)
(21)
(28)
salt
(27)
salt
(25)
salt
(22)
(23)
chlorination
(29)
salt
(1-8)
salt
(26)
salt
(1-6)
(30)
salt
(1-9)
salt
base
base
base
(1-7)
(31)
(1-10)

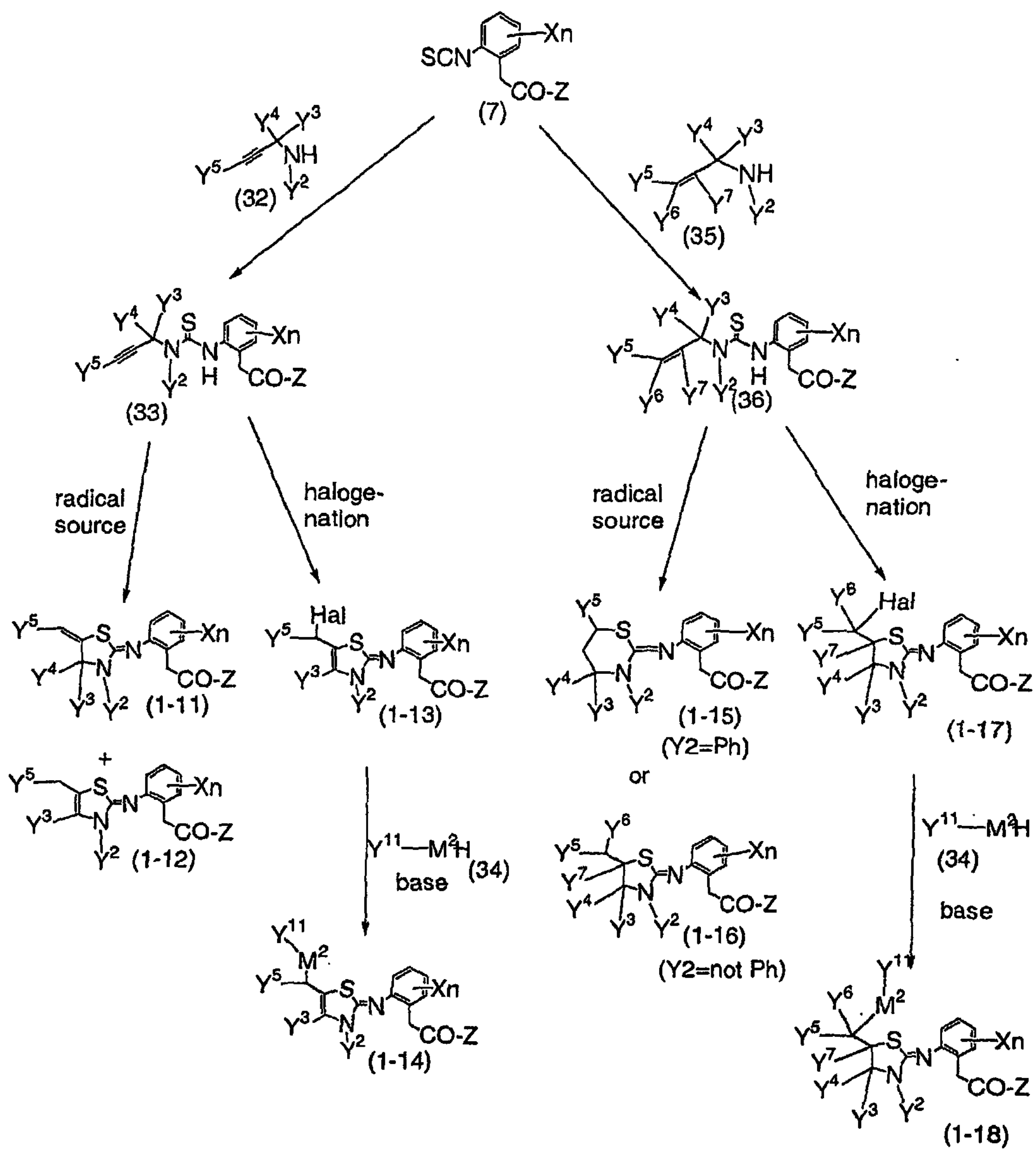
(7)
(32)
(35)
(33)
(36)
radical source
haloge-nation
radical source
haloge-nation
(1-11)
+
(1-12)
(1-13)
(34)
base
(1-14)
(1-15)
(Y2=Ph)
or
(1-16)
(Y2=not Ph)
(1-17)
(34)
base
(1-18)

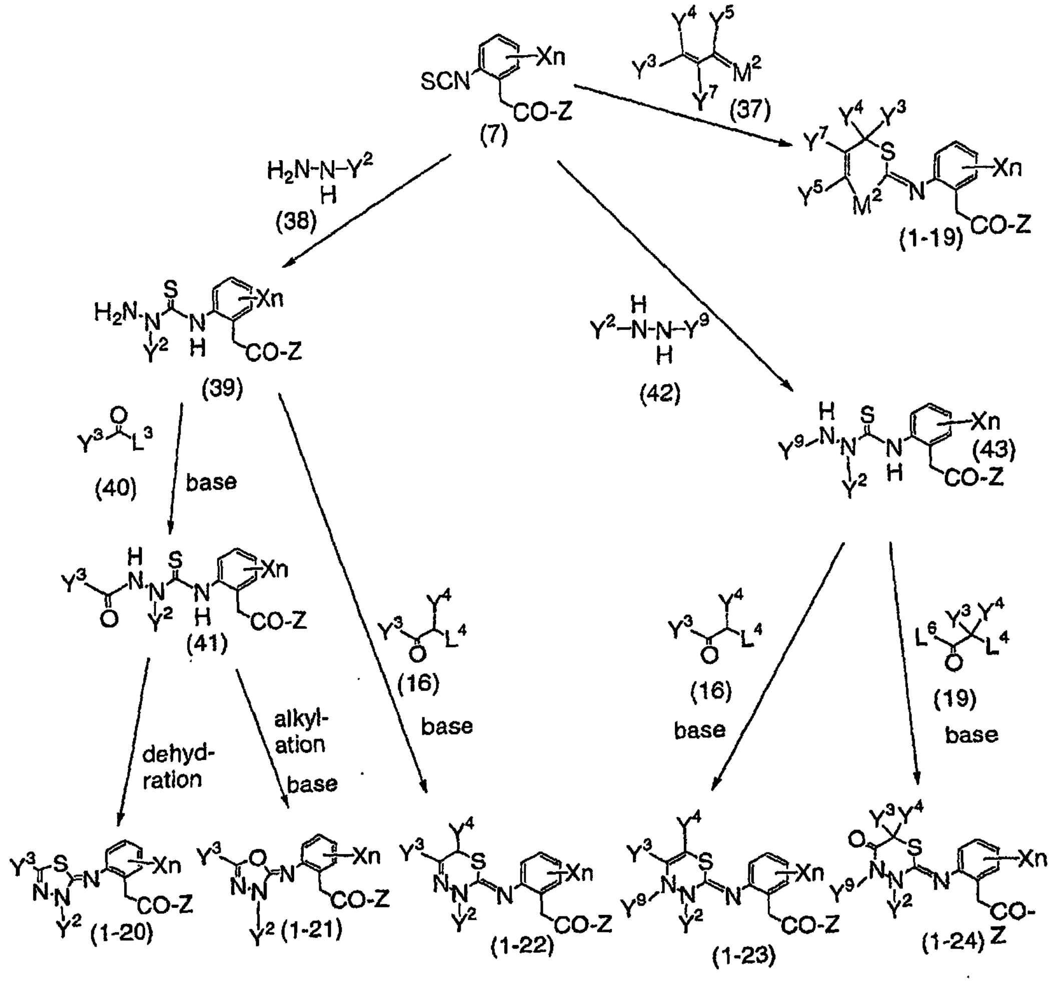
(7)
(37)
(1-19)
(38)
(39)
(42)
(43)
(40)
base
(41)
(16)
base
(16)
base
(19)
base
dehyd-
ration
alkyl-
ation
base
(1-20)
(1-21)
(1-22)
(1-23)
(1-24)

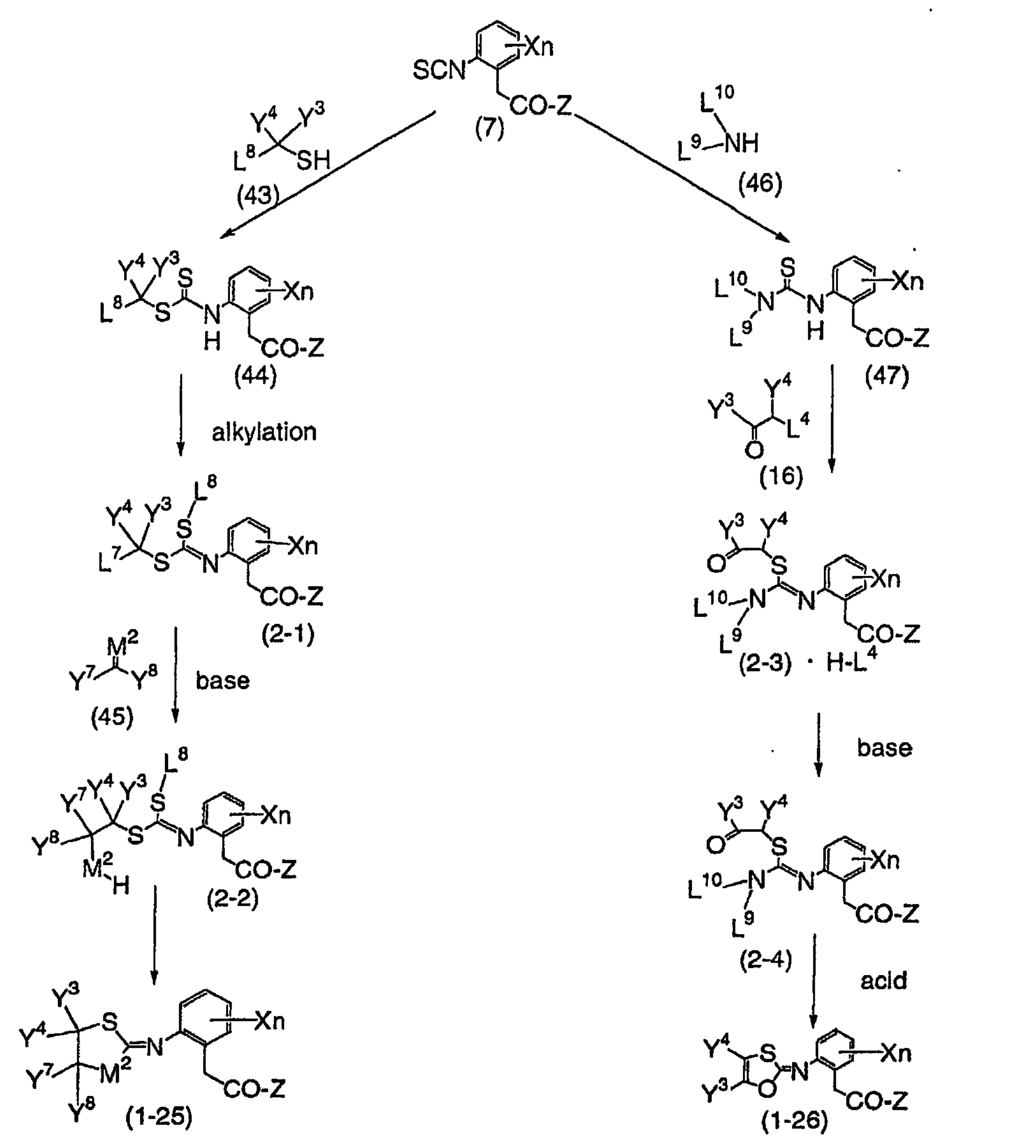


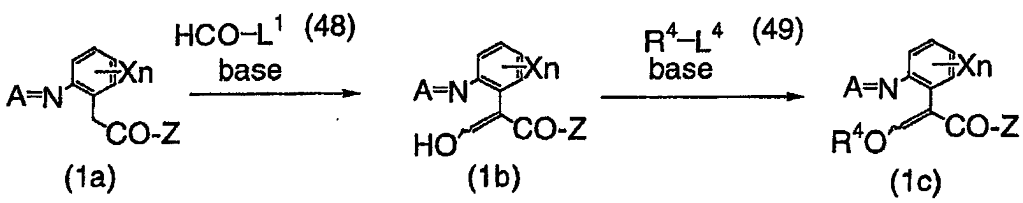


($A^1$ and $A^2$, each independently is a 3- to 13-membered, mono-, di- or tri-cyclic ring which is composed of from 3 to 13 atoms arbitrarily selected from among carbon atoms, oxygen atoms, sulfur atoms and nitrogen atoms and which may be substituted by from 1 to 13 Ys and has a nitrogen atom at the α-position to the imino bond or a leaving group L1; $A^3$ is a 3- to 13-membered, mono-, di- or tri-cyclic ring which is composed of from 3 to 13 atoms arbitrarily selected from among carbon atoms, oxygen atoms, sulfur atoms and nitrogen atoms and which may be substituted by from 1 to 13 Ys and has an oxygen atom, a sulfur atom or a nitrogen atom at the α-position to the imino bond or C=M1; X, n, Z, $R^4$ and A have the same meanings as above; $L^1$ is a good leaving group, such as a fluorine atom, a chlorine atom, a bromine atom, an iodine atom, $C_{1-4}$ alkoxy, phenoxy, $C_{1-4}$ alkylamino, $C_{1-4}$ dialkylamino, $C_{1-4}$ alkylsulfonyloxy, benzenesulfonyloxy, toluenesulfonyloxy, 1-pyrazolyl or 1-imidazolyl; each of $L^2$ and $L^6$ is a good leaving group, such as $C_{1-4}$ alkoxy, $C_{1-4}$ alkylthio, phenoxy, $C_{1-4}$ alkylamino, $C_{1-4}$ dialkylamino, 1-pyrazolyl or 1-imidazolyl; $L^3$ each independently is a good leaving group, such as a chlorine atom, a bromine atom, an iodine atom, $C_{1-4}$ alkoxy, $C_{1-4}$ alkylthio, phenoxy, $C_{1-4}$ alkylamino, $C_{1-4}$ dialkylamino, 1-pyrazolyl or 1-imidazolyl; $L^4$ is a good leaving group, such as a chlorine

atom, a bromine atom, an iodine atom, $C_{1-4}$ alkylsulfonyloxy, benzenesulfonyloxy or toluenesulfonyloxyl; $L^5$ is a good leaving group, such as a chlorine atom or a bromine atom; $L^7$ is a $C_{1-4}$ alkyl group, phenyl or a toluyl group; $L^8$ is a hydrogen atom, a trimethylsilyl group, a tertiary butyldimethylsilyl group or a tertiary butyldiphenylsilyl group; $L^9$ and $L^{10}$, each independently has the same meaning as Y, or they together represent 1-imidazolyl, 1-pyrazolyl, 1-piperidinyl or morpholino; $Y^1$ is a $C_{1-6}$ alkyl group or a benzyl group which may be substituted by $R^a$; $Y^2$, $Y^9$ and $Y^{10}$, each independently has the same meaning as Y; $Y^3$, $Y^4$, $Y^7$, $Y^8$ and $Y^{11}$, each independently is a hydrogen atom or has the same meaning as Y; $Y^5$ and $Y^6$ each independently is a hydrogen atom, a $C_{1-6}$ alkyl group or a phenyl group which may be substituted by $R^a$; M is an oxygen atom, a sulfur atom or N-$Y^2$; $M^1$ is an oxygen atom or a sulfur atom; $M^2$ is an oxygen atom, a sulfur atom or N-$Y^9$; Hal is a chlorine atom, a bromine atom, an iodine atom or a fluorine atom; and $R^a$ has the same meaning as above.)

The nitrophenyl acetic acid compound of the formula (3) as the starting material, can be produced by a known method disclosed in e.g. a published European patent application (EP-570817), Synthesis, p. 51 (1993), or J. Org. Chem., vol. 61, p.5994 (1996). As the method for producing a compound of the formula (9) from the nitrophenyl acetic acid compound (3), a method disclosed in a published European patent application (EP-447118), Organic Functional Group Preparations, published by Academic Co., vol. 1, p. 313 (1968), J. Am Chem. Soc., vol. 54, p. 781 (1932), Chem. Rev., vol. 55, p. 181 (1955), etc. Namely, by a reduction reaction of the nitrophenyl acetic acid compound (3), it is converted to an aminophenyl acetic acid compound (4), which is then reacted with carbon disulfide in the presence of a base and converted to a dithiocarbamic acid compound (5). Further, the dithiocarbamic acid compound (5) is reacted with an acid halide compound of the formula (6) and converted to an isothiocyanate compound (7). Then, the isothiocyanate compound (7) is reacted with an amine compound of the formula (8) to produce a thiourea compound (9). At that time, by using ammonia as the amine compound, a thiourea compound (18) may be produced in the same manner. Further, the isothiocyanate compound (7) may also be produced by a method of reacting an aminophenyl acetic acid compound (4) with a thiocarbonyl compound of the formula (10). Further, the thiourea compound (9) may also be produced by a method of reacting the aminophenyl acetic acid compound (4) with an isothiocyanate compound of the formula (11). Further, the thiourea compound (9) may be converted to a carbodiimide compound (22) by a reaction with a sulfonic acid halide compound (21) by means of a method disclosed in Synth. Commun., vol. 25, No. 1, p. 43 (1995).

The compounds (1-1) and (1-2) of the present invention can be produced by or in accordance with the method disclosed in Angew. Chem., vol. 80, p.799 (1968) using an aminophenyl acetic acid compound (4) as the starting material. Namely, the compound (1-1) of the present invention can be produced by preliminarily alkylating a compound of the formula (12) to obtain an ammonium salt of the formula (13) and reacting it with an aminophenyl acetic acid compound (4), if necessary in a solvent, in some cases in the presence of a catalyst. Likewise, the compound (1-2) of the present invention can be produced by preliminarily alkylating a compound of the formula (14) to obtain an oxonium salt or a thioxonium salt of the formula (15) and reacting it with an aminophenyl acetic acid compound (4), if necessary in the presence of a solvent, in some cases in the presence of a catalyst. The solvent may be any solvent so long as it is inert to the reaction, and it may, for example, be a halogenated hydrocarbon such as dichloromethane, chloroform or 1,2-dichloroethane, an aromatic hydrocarbon such as benzene, xylene or toluene, an aliphatic hydrocarbon such as pentane, hexane or cyclohexane, or a solvent mixture thereof, preferably dichloromethane, chloroform, 1,2-dichloroethane or the like. The alkylating agent may, for example, be an alkyl halide such as methyl iodide, ethyl iodide or benzyl bromide, a sulfonate such as dimethyl sulfate, diethyl sulfate, methyl trifluoromethane sulfonate, or a trialkyloxonium salt such as trimethyloxonium tetrafluoroborate or triethyloxonium tetrafluoroborate, preferably trimethyloxonium tetrafluoroborate or the like. The catalyst may, for example, be silver oxide or silver trifluoromethane sulfonate. The reaction can be carried out within a temperature range of from -80°C to the boiling point of the solvent, preferably within a range of from 0°C to the boiling point of the solvent. With respect to the reaction time, it can be carried out within a range of from 5 minutes to 300 hours, preferably within a range of from 1 hour to 168 hours. With respect to the equivalents of the alkylating agent, it may be used within a range of from 0.5 to 50 equivalents, preferably within a range of from 1 to 20 equivalents, to (12) or (14). Further, with respect to the equivalents of the substrate, (13) or (15) is used within a range of from 0.5 to 50 equivalents, preferably within a range of from 1 to 20 equivalents, to (4).

The compound (1-3) of the present invention can be produced by reacting a dithiocarbamic acid compound (5) with a ketone compound of the formula (16), if necessary in the presence of a solvent, in some cases in the presence of a base, in some cases in the presence of a catalyst, to convert it to a dithiocarbamate compound (17), and further reacting it with a dehydrating agent, if necessary in a solvent, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide, N-methylpyrrolidone or N,N'-dimethylimidazolidinone, an aliphatic hydrocarbon such as pentane, hexane or cyclohexane, dimethylsulfoxide or water, or a solvent

mixture thereof, preferably dichloromethane, chloroform, 1,2-dichloroethane or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. As the dehydrating agent, concentrated sulfuric acid, dicyclohexylcarbodiimide, phosphorus pentachloride or phosphorus oxychloride may, for example, be employed. Further, concentrated sulfuric acid may also be used as the solvent. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 0.1 to 20 equivalents, to (5). Further, with respect to the equivalents of the substrate, (16) can be used within a range of from 0.5 to 50 equivalents, preferably within a range of from 1 to 20 equivalents, to (5). With respect to the equivalents of the dehydrating agent, it can be used within a range of from 0.1 to 100 equivalents, preferably within a range of from 1 to 50 equivalents, to (17).

The compound (1-4) of the present invention can be produced by reacting a thiourea compound (9) with a carbonyl compound of the formula (19), if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably ethanol, tetrahydrofuran, chloroform, dimethylformamide or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be employed. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.1 to 50 equivalents, preferably within a range of from 1 to 20 equivalents, to (9). Further, with respect to the equivalents of the substrate, (19) can be used within a range of from 0.5 to 50 equivalents, preferably within a range of from 1 to 20 equivalents, to (9).

The compound (1-5) of the present invention can be produced by reacting a thiourea compound (9) with an acid halide compound of the formula (20), if necessary in a solvent, if necessary in the presence of a base, in some cases in the presence of a catalyst. The solvent may be any solvent so long as it is inert to the reaction, and it may, for example, be an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, chloroform, dimethylformamide or the like. The base may, for example, be an organic base such as triethylamine, tributylamine, pyridine, N-methylpiperidine or 4-dimethylamidepyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be employed. With respect to the reaction temperature, the reaction can be carried out within a range of from -80°C to the boiling point of the solvent, preferably within a range of from 0°C to the boiling point of the solvent. With respect to the reaction time, it can be carried out within a range of from 5 minutes to 100 hours, preferably within a range of from 1 hour to 48 hours. With respect to the equivalents of the base, it can be used within a range of from 0.1 to 50 equivalents, preferably within a range of from 1 to 20 equivalents, to (9). Further, with respect to the equivalents of the substrate, (20) can be used within a range of from 0.5 to 50 equivalents, preferably within a range of from 1 to 20 equivalents, to (9).

The compound (1-6) of the present invention can be produced by reacting a carbodiimide compound (22) with a carbonyl compound (23), if necessary in a solvent, if necessary in the presence of a base, in some cases in the presence of a catalyst. The solvent may be any solvent so long as it is inert to the reaction, and it may, for example, be an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as

pentane, hexane or cyclohexane, dimethylsulfoxide or water, or a solvent mixture thereof, preferably dichloromethane, chloroform, 1,2-dichloroethane or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be employed. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (22). Further, with respect to the equivalents of the substrate, (23) can be used within a range of from 0.5 to 50 equivalents, preferably within a range of from 1 to 20 equivalents, to (22).

The compound (1-7) of the present invention can be obtained by reacting a thiourea compound (9) with an amide compound of the formula (24). Namely, by reacting the thiourea compound (9) with the amide compound of the formula (24), if necessary in a solvent, in some cases in the presence of a catalyst, it can be converted to a pseudothiourea compound (25). Further, the pseudothiourea compound (25) can be converted to an imidoyl chloride compound (26) by treating it with a halogenating agent, if necessary in a solvent, in some cases in the presence of catalyst. Further, (26) can be converted to the compound (1-7) of the present invention by a reaction, if necessary in a solvent, if necessary in the presence of a base, in some cases in the presence of catalyst. Further, by using a base in the reaction of (25) with the halogenating agent, (1-7) can be obtained without isolating (26). In the reaction to obtain (25) from (9), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably ethanol, tetrahydrofuran, chloroform, 1,2-dichloroethane, ethyl acetate, acetone, acetonitrile, dimethylformamide, water or the like. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the substrate, (24) can be used within a range of from 0.5 to 50 equivalents, preferably within a range of from 1 to 20 equivalents, to (9).

In the reaction to obtain (26) from (25), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform, 1,2-dichloroethane, an ester such as ethyl acetate, a ketone such as acetone or methyl ethyl ketone, a nitrile such as acetonitrile or propionitrile, an amide such as dimethylformamide, dimethylacetamide, N-methylpyrrolidone or N,N'-dimethylimidazolidinone, an aliphatic hydrocarbon such as pentane, hexane or cyclohexane, dimethylsulfoxide or water, or a solvent mixture thereof, preferably dichloromethane, chloroform, 1,2-dichloroethane or the like. The halogenating agent may, for example, be tetrachloroethane/triphenylphosphine, phosphorus oxychloride, phosphorus pentachloride, phosphorus trichloride, oxalyl chloride, chlorine or N-chlorosuccinimide. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the chlorinating agent, it can be used within a range of from 0.01 to 50 equivalents, preferably within a range of from 0.1 to 20 equivalents, to (25). In the reaction to obtain (1-7) from (26), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide, N-methylpyrrolidone or N,N'-dimethylimidazolidinone, an aliphatic hydrocarbon such as pentane, hexane or cyclohexane, dimethylsulfoxide or water, or a solvent mixture thereof. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within

a range of from 0.1 to 20 equivalents, to (26).

The compound (1-10) of the present invention can be obtained by reacting a thiourea compound (9) with a ketone compound of the formula (16). Namely, by reacting the thiourea compound (9) with the ketone compound of the formula (16), if necessary in a solvent, in some cases in the presence of a catalyst, it can be converted to a pseudothiourea compound (27), Further, the pseudothiourea compound (27) can be converted to a hydroxythiazolidine compound (1-8) by a reaction, if necessary in a solvent, in the presence of an acidic or basic catalyst. Further, (1-8) can be converted to the compound (1-9) of the present invention by treating it with a dehydrating agent, if necessary in a solvent, if necessary in the presence of a base, in some cases in the presence of a catalyst. Further, (1-9) can be converted to a free compound (1-10) by treating it with a base, if necessary in a solvent. Further, by treatment for a long time or under heating, or treatment by means of a catalyst in the reaction of (9) with (16), (1-9) can be obtained without isolating (27) or (1-8). Further, by using a base in the reaction of (9) with (16), (1-10) can be obtained without isolating (27), (1-8) or (1-9). Further, by using a base in the reaction of (1-8) with the dehydrating agent, (1-10) can be obtained without isolating (1-9). Further, it can be obtained also by treating a thiazolidine compound (31) obtained in the same manner from a thiourea compound (18) with alkylating agent, if necessary in a solvent, if necessary in the presence of a base, in some cases in the presence of a catalyst.

In the reaction to obtain (1-8) from (9), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably ethanol, tetrahydrofuran, chloroform, 1,2-dichloroethane, ethyl acetate, acetone, acetonitrile, dimethylformamide, water or the like. The acidic catalyst may, for example, be hydrochloric acid, hydrobromic acid, hydroiodic acid, hydrofluoric acid, acetic acid or tetrafluoroboric acid. The basic catalyst may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the substrate, (16) can be used within a range of from 0.5 to 50 equivalents, preferably within a range of from 1 to 20 equivalents, to (9). In the reaction to obtain the compound (1-9) of the present invention from the hydroxythiazolidine compound (1-8), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide, N-methylpyrrolidone or N,N'-dimethylimidazolidinone, an aliphatic hydrocarbon such as pentane, hexane or cyclohexane, dimethylsulfoxide or water, or a solvent mixture thereof, preferably dichloromethane, chloroform, 1,2-dichloroethane or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. Further, pyridine or the like may also be used as the solvent. As the catalyst, tetra-N-butylammonium bromide may, for example, be employed. As the dehydrating agent, methanesulfonyl chloride, toluenesulfonyl chloride, trifluoromethanesulfonic anhydride, concentrated sulfuric acid, dicyclohexylcarbodiimide, phosphorus pentachloride or phosphorus oxychloride, may, for example, be used. Further, concentrated sulfuric acid may be used as a solvent. Further, the reaction may be carried out by azeotropic dehydration by means of a solvent such as toluene, benzene or xylene. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 0.1 to 20 equivalents, to (1-8). With respect to the equivalents of the dehydrating agent, it can be used within a range of from 0.1 to 100 equivalents, preferably within a range of from 1 to 50 equivalents, to (1-8). In the reaction to obtain (1-10) from (1-9), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide, N-methylpyrrolidone or N,N'-dimethylimidazolidinone, an aliphatic hydrocarbon such as pentane, hexane or cyclohexane, dimethylsulfoxide or water, or a solvent mixture thereof. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes 100 hours, preferably within a range of from 1 hour to 48 hours. With respect to the equivalents of the base, it can be used within a range of from 0.01 to 50 equivalents, preferably within a range of from 0.1 to 20 equivalents, to (1-9). In the reaction to obtain (1-10) from (31), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide, N-methylpyrrolidone or N,N'-dimethylimidazolidinone, an aliphatic hydrocarbon such as pentane, hexane or cyclohexane, dimethylsulfoxide or water, or a solvent mixture thereof. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. The alkylating agent may, for example, be an alkyl halide such as methyl iodide, ethyl iodide or benzyl bromide, a sulfonate such as dimethyl sulfate, diethyl sulfate, methyl trifluoromethane sulfonate, or a trialkyloxonium salt such as trimethyloxonium tetrafluoroborate or triethyloxonium tetrafluoroborate, preferably methyl trifluoromethane sulfonate. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 0.1 to 20 equivalents, to (31). With respect to the equivalents of the alkylating agent, it can be used within a range of from 0.01 to 50 equivalents, preferably within a range of from 0.1 to 20 equivalents, to (31).

The compounds (1-11) and (1-12) of the present invention can be produced by the following method. Namely, by reacting an isothiocyanate compound (7) with a propargylamine compound of the formula (32), if necessary in a solvent, if necessary in the presence of a base, in some cases in the presence of a catalyst, it can be converted to a propargylthiourea compound of the formula (33). Further, (33) is treated with a radical-forming agent, if necessary in a solvent, whereby a mixture of (1-11) and (1-12) can be obtained. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, chloroform, acetone, acetonitrile or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the solvent, tetra-N-butylammonium bromide may, for example, be used. As the radical-forming agent, trifluoroacetic acid, oxygen, air, benzoyl peroxide or azobisisobutyronitrile, may, for example, be used. Further, trifluoroacetic acid may be used as a solvent. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.05 to 150 equivalents, preferably within a range of from 1 to 20 equivalents, to (7). Further, with respect to the equivalents of the substrate, (32) can be used within a range of from 0.5 to 50 equivalents, preferably within a range of from 1 to 20 equivalents, to (7).

The compound (1-13) of the present invention can be produced by reacting the above-mentioned propargylthiourea compound (33) with a halogenating agent, if necessary, in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably dichloromethane, chloroform, 1,2-dichloroethane, acetonitrile or the like. The

base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. As the halogenating agent, iodine, bromine, N-bromosuccinimide, N-chlorosuccinimide, N-iodosuccinimide or tetrabutylammonium tribromide, may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (33). With respect to the equivalents of the halogenating agent, it can be used within a range of from 0.01 to 50 equivalents, preferably within a range of from 1 to 20 equivalents, to (33).

The compound (1-14) of the present invention can be obtained by reacting the above (1-13) with an amine compound, an alcohol compound or a mercaptan compound of the formula (34), if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, benzene, chloroform, 1,2-dichloroethane, acetonitrile or the like. The base may, for example, be an organic base such as triethylamine, tributylamine, pyridine, diazabicycloundecene, N-methylpiperidine or 4-dimethylaminopyridine, or an inorganic base such as cesium fluoride, potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (1-13). Further, with respect to the equivalents of the substrate, (34) can be used within a range of from 0.5 to 50 equivalents, preferably within a range of from 1 to 20 equivalents, to (1-13).

The compounds (1-15) and (1-16) of the present invention can be produced by the following method. Namely, by reacting an isothiocyanate compound (7) with an allylamine compound of the formula (35), if necessary in a solvent, if necessary in the presence of a base, in some cases in the presence of a catalyst, it can be converted to an allylthiourea compound of the formula (36). Further, (36) is treated with a radical-forming agent, to obtain the compound (1-15) of the present invention in the case where Y2 is phenyl, or to obtain the compound (1-16) of the present invention in the case where Y2 is other than phenyl. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, chloroform, acetone, acetonitrile or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. As the radical-forming agent, trifluoroacetic acid, oxygen, air, benzoyl peroxide or azobisisobutyronitrile, may, for example, be used. Further, trifluoroacetic acid may be used as a solvent. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.05 to 150 equivalents, preferably within a range of from 1 to 20 equivalents, to (7). Further, with respect to the equivalents of the substrate, (35) can be used within a range of from 0.5 to 50 equivalents, preferably within a range of from 1 to 20 equivalents, to (7).

The compound (1-17) of the present invention can be produced by treating the above allylthiourea compound (36) with a halogenating agent, if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane,

an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably dichloromethane, chloroform, 1,2-dichloroethane, acetonitrile or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. As the halogenating agent, iodine, bromine, N-bromosuccinimide or N-chlorosuccinimide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (36). With respect to the equivalents of the halogenating agent, it can be used within a range of from 0.01 to 50 equivalents, preferably within a range of from 1 to 20 equivalents, to (36).

The compound (1-18) of the present invention can be obtained by reacting the above (1-17) with an amine compound, an alcohol compound or a mercaptan compound of the formula (34), if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, benzene, chloroform, 1,2-dichloroethane, acetonitrile or the like. The base may, for example, be an organic base such as triethylamine, tributylamine, pyridine, diazabicycloundecene, N-methylpiperidine or 4-dimethylaminopyridine, or an inorganic base such as cesium fluoride, potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (1-17). Further, with respect to the equivalents of the substrate, (34) can be used within a range of from 0.5 to 50 equivalents, preferably within a range of from 1 to 20 equivalents, to (1-17).

The compound (1-19) of the present invention can be produced by or in accordance with the method disclosed in Synthesis, p. 896 (1981). Namely, it can be obtained by reacting an isothiocyanate compound (7) with an olefin compound of the formula (37), if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, benzene, chloroform, 1,2-dichloroethane, acetonitrile or the like. With respect to the equivalents of the base, it can be used with in a range of from 0.01 to 50 equivalents, preferably within a range of from 1 to 20 equivalents, to (7). As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (7). Further, with respect to the equivalents of the substrate, (37) can be used within a range of from 0.5 to 50 equivalents, preferably within a range of from 1 to 20 equivalents, to (7).

The compound (1-20) of the present invention can be produced by or in accordance with the method disclosed in a published West German patent application (DE-3025559). Namely, by reacting an isothiocyanate compound (7) with a hydrazine compound of the formula (38), if necessary in a solvent, in some cases in the presence of a catalyst,

it can be converted to a thiosemicarbazide compound (39). Further, by reacting it with a carbonyl compound of the formula (40), if necessary in a solvent, if necessary in the presence of a base, in some cases in the presence of a catalyst, it can be converted to an acylthiosemicarbazide compound (41). Further, the acylthiosemicarbazide compound (41) is treated with a dehydrating agent, if necessary in a solvent, in some cases in the presence of a catalyst, whereby the compound (1-20) of the present invention can be obtained.

In the reaction to obtain the thiosemicarbazide compound (39) from the isothiocyanate compound (7), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, or dimethylsulfoxide, or a solvent mixture thereof, preferably tetrahydrofuran, chloroform, 1,2-dichloroethane, acetone, acetonitrile, dimethylformamide or the like. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the substrate, (38) can be used within a range of from 0.01 to 50 equivalents, preferably within a range of from 1 to 20 equivalents, to (7).

In the reaction to convert the thiosemicarbazide compound (39) to the acylthiosemicarbazide compound (41), the solvent may be any solvent so long as it is inert to the reaction, and it may, for example, be an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, or dimethylsulfoxide, or a solvent mixture thereof, preferably tetrahydrofuran, chloroform, 1,2-dichloroethane, acetone, acetonitrile, dimethylacetamide or the like. The base may, for example, be an organic base such as triethylamine, tributylamine, pyridine, diazabicycloundecene, N-methylpiperidine or 4-dimethylaminopyridine, or an inorganic base such as cesium fluoride, potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (39). With respect to the equivalents of the substrate, (40) can be used within a range of from 0.01 to 50 equivalents, preferably within a range of from 1 to 20 equivalents, to (39).

In the reaction to obtain the compound (1-20) of the present invention from the acylthiosemicarbazide compound (41), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, or dimethylsulfoxide, or a solvent mixture thereof, preferably benzene, xylene, chloroform or the like. As the catalyst, tetra-N-butylammonium bromide may, for example, be used. As the dehydrating agent, concentrated sulfuric acid, dicyclohexylcarbodiimide, phosphorus pentachloride or phosphorus oxychloride, may, for example, be employed. Further, concentrated sulfuric acid may be used also as a solvent. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the dehydrating agent, the dehydrating agent can be used within a range of from 0.01 to 50 equivalents, preferably within a range of from 1 to 20 equivalents, to (41).

The compound (1-21) of the present invention can be produced by or in accordance with the method disclosed in a published West German patent application (DE-3025559). Namely, it can be obtained by reacting the above-mentioned acylthiosemicarbazide compound (41) with an alkylating agent, if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably ethanol, tetrahydrofuran, chloroform, 1,2-dichloroethane, dimethylacetamide or the like. The alkylating agent may, for example, be an alkyl halide such as methyl iodide, ethyl iodide or benzyl bromide, a sulfonate such as dimethyl sulfate, diethyl sulfate, methyl trifluoromethanesulfonate, or a trialkyloxonium salt such as trimethyloxonium tetrafluoroborate or triethyloxonium tetrafluoroborate. As the catalyst, tetra-N-butylammonium bromide, may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the alkylating agent, the alkylating agent can be used within a range of from 0.01 to 50 equivalents, preferably within a range of from 1 to 20 equivalents, to (41).

The compound (1-22) of the present invention can be obtained by reacting the above-mentioned thiosemicarbazide compound (39) with a ketone compound of the formula (16), if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably, ethanol, tetrahydrofuran, chloroform, 1,2-dichloroethane, acetone, acetonitrile, dimethylformamide or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.05 to 150 equivalents, preferably within a range of from 1 to 20 equivalents, to (39). Further, with respect to the equivalents of the substrate, (16) can be used within a range of from 0.5 to 50 equivalents, preferably within a range of from 1 to 20 equivalents, to (39).

The compound (1-23) of the present invention can be obtained by reacting an isothiocyanate compound (7) with a hydrazine compound of the formula (42), if necessary in a solvent, in some cases in the presence of a catalyst, to convert it to a thiosemicarbazide compound (43), and then further reacting it with a ketone compound of the formula (16), if necessary in a solvent, if necessary in the presence of a base, in some cases in the presence of a catalyst. In the reaction to obtain the thiosemicarbazide compound (43) from the isothiocyanate compound (7), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, or dimethylsulfoxide, or a solvent mixture thereof, preferably ethanol, tetrahydrofuran, chloroform, acetonitrile or the like. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the substrate, (42) can be used within a range of from 0.01 to 50 equivalents, preferably within a range of from 1 to 20 equivalents, to (7). In the reaction to obtain the compound (1-23) of the present invention from the thiosemicarbazide compound (43), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, benzene, xylene, chloroform, dimethylacetamide, N-methylpyrrolidone or the like. The base may, for example, be an organic base such as triethylamine, tributylamine, pyridine, N-methylpiperidine or 4-dimethylaminopyridine, or an inorganic base such as potassium

carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.05 to 150 equivalents, preferably within a range of from 1 to 20 equivalents, to (43). Further, with respect to the equivalents of the substrate, (16) can be used within a range of from 0.5 to 50 equivalents, preferably within a range of from 1 to 20 equivalents, to (43).

The compound (1-24) of the present invention can be produced by reacting the above-mentioned thiosemicarbazide compound (43) with a carbonyl compound of the formula (19), if necessary in a solvent, if necessary in the presence of a base, in some cases in the presence of a catalyst. The solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably ethanol, tetrahydrofuran, chloroform, dimethylformamide or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.1 to 50 equivalents, preferably within a range of from 1 to 20 equivalents, to (43). Further, with respect to the equivalents of the substrate, (19) can be used with in a range of from 0.5 to 50 equivalents, preferably within a range of from 1 to 20 equivalents, to (43).

The compound (1-25) of the present invention can be produced by or in accordance with the method disclosed in Heterocycles, vol. 50, p. 195 (1999). Namely, by reacting an isothiocyanate compound (7) with a mercaptan compound of the formula (43), if necessary in a solvent, if necessary in the presence of a base, in some cases in the presence of a catalyst, to form a dithiocarbamate compound (44), followed by treatment with an alkylating agent, if necessary in a solvent, if necessary in the presence of a base, in some cases in the presence of a catalyst, to convert it to a dithioacetal compound (2-1). Further, the dithioacetal compound (2-1)is reacted with a carbonyl compound of the formula (45), if necessary in a solvent, if necessary in the presence of a base, in some cases in the presence of a catalyst, to obtain a mixture of (2-2) and (1-25). Further, in the reaction to obtain (2-1) from (7), (2-1) can be obtained without isolating (44). Further, in the reaction to obtain (1-25) from (2-1), (1-25) can be obtained without isolating (2-2) by heating or reacting for a long time. Further, the isolated (2-2) may be reacted or heat-treated, if necessary in a solvent, if necessary in the presence of a base, in some cases in the presence of a catalyst, to convert it to (1-25). In the reaction to obtain (2-1) from (7), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably, tetrahydrofuran, benzene, toluene, dichloromethane, chloroform, 1,2-dichloroethane, acetone, acetonitrile, dimethylacetamide or the like. The base may, for example, be an organic base such as triethylamine, tributylamine, pyridine, diazabicycloundecene, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. The alkylating agent may, for example, be an alkyl halide such as methyl iodide, ethyl iodide or benzyl bromide, a sulfonate such as dimethyl sulfate, diethyl sulfate, methyl trifluoromethanesulfonate, or a trialkyloxonium salt such as trimethyloxonium tetrafluoroborate or triethyloxonium tetrafluoroborate. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (7). Further, it can be used within a range of from 0.01 to 50 equivalents, preferably within a range of from 1 to 20 equivalent, to (44). With respect to the equivalents of the substrate, (43) can be used within a range of

from 0.01 to 50 equivalents, preferably within a range of from 1 to 20 equivalents, to (7). Further, the alkylating agent can be used within a range of from 0.01 to 50 equivalents, preferably within a range of from 1 to 20 equivalents, to (44).

In the reaction to obtain (1-25) from (2-1), the solvent may be any solvent so long as it is inert to the reaction, and it may, for example, be a lower alcohol such as methanol or ethanol, an ether such as diethyl ether tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, or dimethylsulfoxide or a solvent mixture thereof, preferably tetrahydrofuran, xylene, toluene or the like. The base may, for example, be an organic base such as triethylamine, tributylamine, pyridine, diazabicycloundecene, N-methylpiperidine or 4-dimethylaminopyridine, or an inorganic base such as cesium fluoride, potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.01 to 50 equivalents, preferably within a range of from 1 to 20 equivalents, to (2-1). With respect to the equivalents of the substrate, (45) can be used within a range of from 0.01 to 50 equivalents, preferably within a range of from 1 to 20 equivalents, to (2-1).

The compound (1-26) of the present invention can be produced by or in accordance with the method disclosed in JP-A-55-108869 and Chemistry Letters, p. 1705 (1988). Namely, by reacting the isothiocyanate compound (7) with an amine compound of the formula (46), if necessary in a solvent, in some cases in the presence of a catalyst, it can be converted to a thiourea compound (47). Further, the thiourea compound (47) can be converted to a pseudothiourea compound (2-3) by reacting it with a ketone compound of the formula (16), if necessary in a solvent, if necessary in the presence of a base, in some cases in the presence of a catalyst. Further, (2-3) can be converted to a free compound (2-4) by treating it with a base, if necessary in a solvent. Further, (2-4) can be converted to the compound (1-26) of the present invention by treating it with an acid, if necessary in a solvent. Further, (2-4) can be obtained without isolating (2-3) by using the base excessively in the reaction of the thiourea compound (47) with the ketone (16). Further, (1-26) can be obtained without isolating (2-3) and (2-4) by heat treatment or reaction for a long time in the reaction of the thiourea compound (47) with the ketone (16). In the reaction to obtain the thiourea compound (47) from the isothiocyanate compound (7), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, or dimethylsulfoxide, or a solvent mixture thereof, preferably, tetrahydrofuran, chloroform, 1,2-dichloroethane, ethyl acetate, acetonitrile, dimethylacetamide or the like. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the substrate, (46) can be used within a range of from 0.01 to 50 equivalents, preferably within a range of from 1 to 20 equivalents, to (7). In the reaction to obtain the compound (1-26) of the present invention from the thiourea compound (47), the solvent may be any solvent so long as it is inert to the reaction, and it may, for example, be a lower alcohol such as methanol or ethanol, an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably, tetrahydrofuran, chloroform, 1,2-dichloroethane, acetone, acetonitrile, dimethylformamide or the like. The base may be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.05 to 150 equivalents, preferably within a range of from 1 to 20 equivalents, to (47). Further, with

respect to the equivalents of the substrate, (16) can be used within a range of from 0.5 to 50 equivalents, preferably within a range of from 1 to 20 equivalents, to (47).

The compound (1a) of the present invention can be converted to the compound (1b) of the present invention by reacting it with a formic acid halide compound, a formic acid ester compound or a formic acid amide compound of the formula (48) in a solvent in the presence of a base in some cases in the presence of a catalyst. Further, (1b) can be converted to the compound (1c) of the present invention by reacting it with an alkyl halide or an alkyl sulfate of the formula (49), if necessary in a solvent, if necessary in the presence of a base, in some cases in the presence of a catalyst. Further, (1c) can be synthesized directly without isolating (1b) by using the base excessively in the reaction to obtain (1b) from (1a). The solvent may be any solvent so long as it is inert to the reaction, and it may, for example, be an ether such as diethyl ether, tetrahydrofuran or dimethoxyethane, an aromatic hydrocarbon such as benzene, xylene or toluene, a halogenated hydrocarbon such as dichloromethane, chloroform or 1,2-dichloroethane, an ester such as ethyl acetate, a ketone such as acetone or methyl ethyl ketone, a nitrile such as acetonitrile or propionitrile, an amide such as dimethylformamide, dimethylacetamide or N-methylpyrrolidone, an aliphatic hydrocarbon such as pentane, hexane or cyclohexane, dimethylsulfoxide or water, or a solvent mixture thereof, preferably tetrahydrofuran, benzene, xylene, toluene, dichloromethane, chloroform, 1,2-dichloroethane, acetonitrile, dimethylformamide or the like. The base may, for example, be an organic base such as triethylamine, tributylamine, pyridine, N-methylpiperidine or 4-dimethylaminopyridine, or an inorganic base such as potassium carbonate, sodium carbonate, sodium hydrogencarbonate, sodium hydroxide, potassium hydroxide or sodium hydride. As the catalyst, tetra-N-butylammonium bromide may, for example, be used. With respect to the reaction temperature, the reaction can be carried out within a range of from -80°C to the boiling point of the solvent, preferably within a range of from 0°C to the boiling point of the solvent. With respect to the reaction time, the reaction can be carried out within a range of from 5 minutes to 100 hours, preferably within a range of from 1 hour to 48 hours. With respect to the equivalents of the base, it can be used within a range of from 0.05 to 150 equivalents, preferably within a range of from 1 to 20 equivalents, to (1a). Further, with respect to the equivalents of the substrate, (48) can be used within a range of from 0.5 to 50 equivalents, preferably within a range of from 1 to 20 equivalents, to (1a).

A compound of the formula (1) wherein G is one of the formulae $G^2$ to $G^{14}$, can be produced in the same manner by using, as the starting material, a compound having a group represented by one of the formulae $G^2$ to $G^{14}$ substituted at the ortho position of the nitrobenzene, instead of the phenylacetic acid compound as the starting material.

The compound of the present invention can be obtained from the reaction solution by a usual method. However, when it is required to purify the compound of the present invention, it can be separated and purified by an optional purification method such as recrystallization or column chromatography.

Now, examples of compounds covered by the present invention will be shown in Tables 1 to 8. However, the compounds of the present invention are not limited thereto.

Abbreviations in the Tables have the following meanings, respectively.

Me: methyl group, Et: ethyl group, Pr: propyl group, Bu: butyl group, Pen: pentyl group, Hex: hexyl group, Hep: heptyl group, Oct: octyl group, Non: nonyl group, Dec: decyl group, Undec: undecanyl group, Dodec: dodecyl group, Ph: phenyl group, n: normal, i: iso, s: secondary, t: tertiary and c: cyclo.

(Table 1)

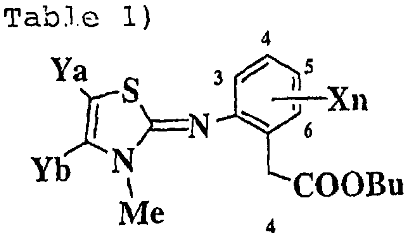 ,

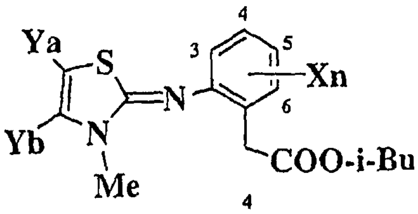 ,

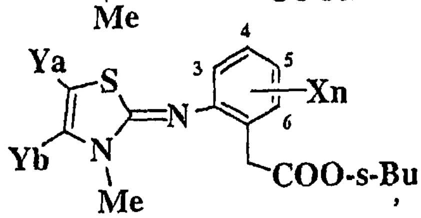 ,

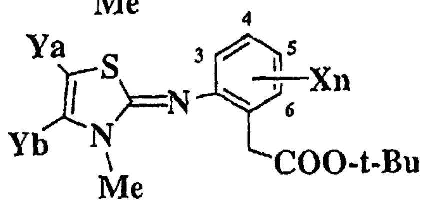 ,

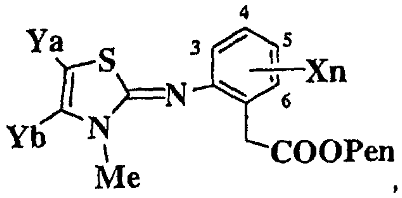 ,

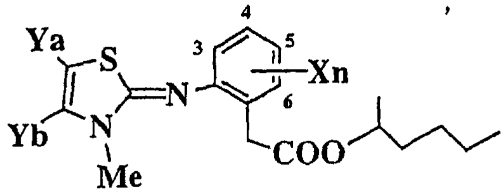 ,

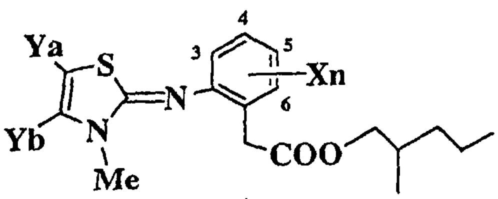 ,

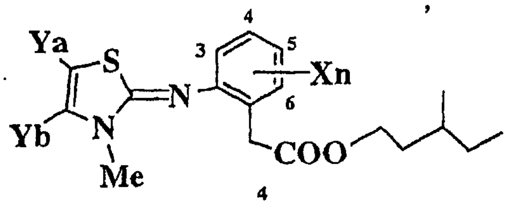 ,

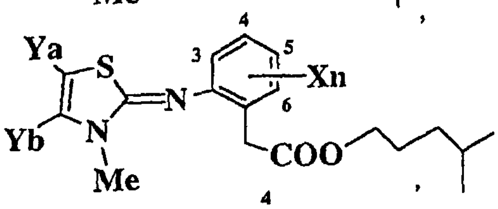 ,

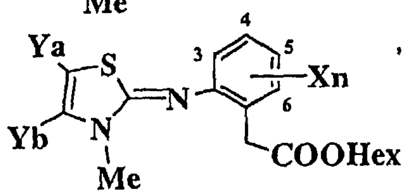 ,

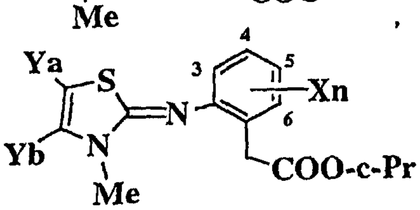 ,

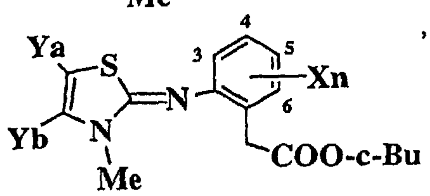 ,

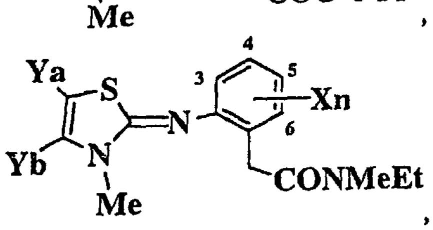 ,

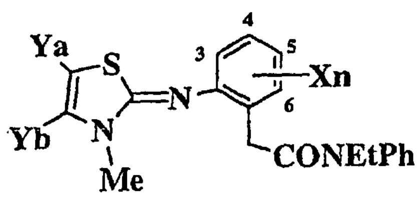 ,

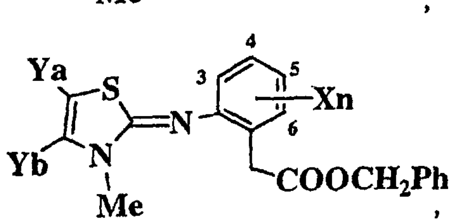 ,

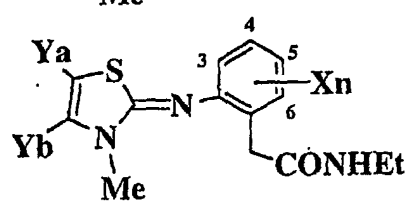 ,

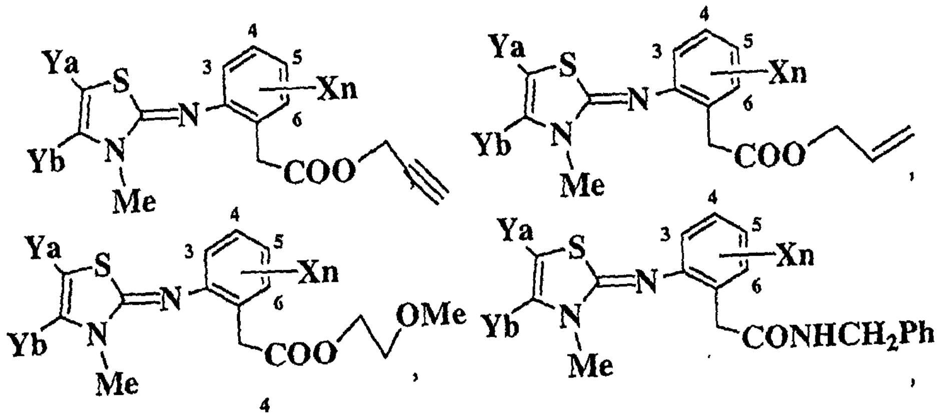

, , ,

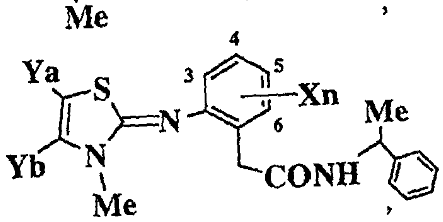

,

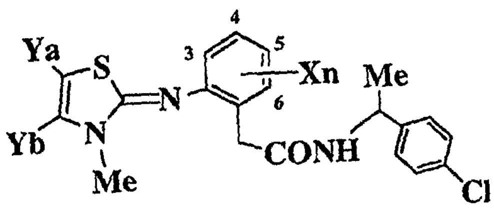

,

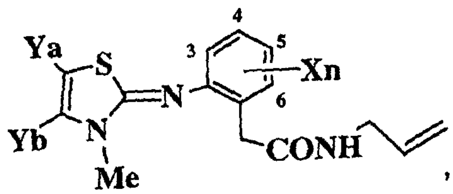

,

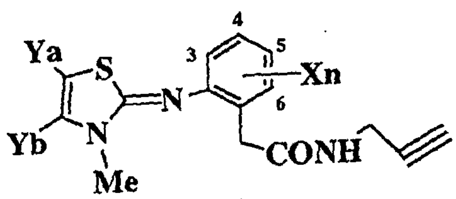

,

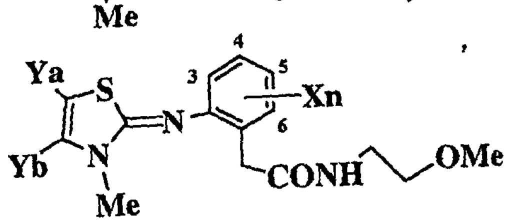

,

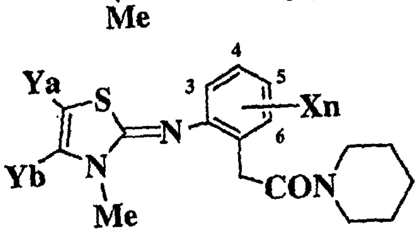

,

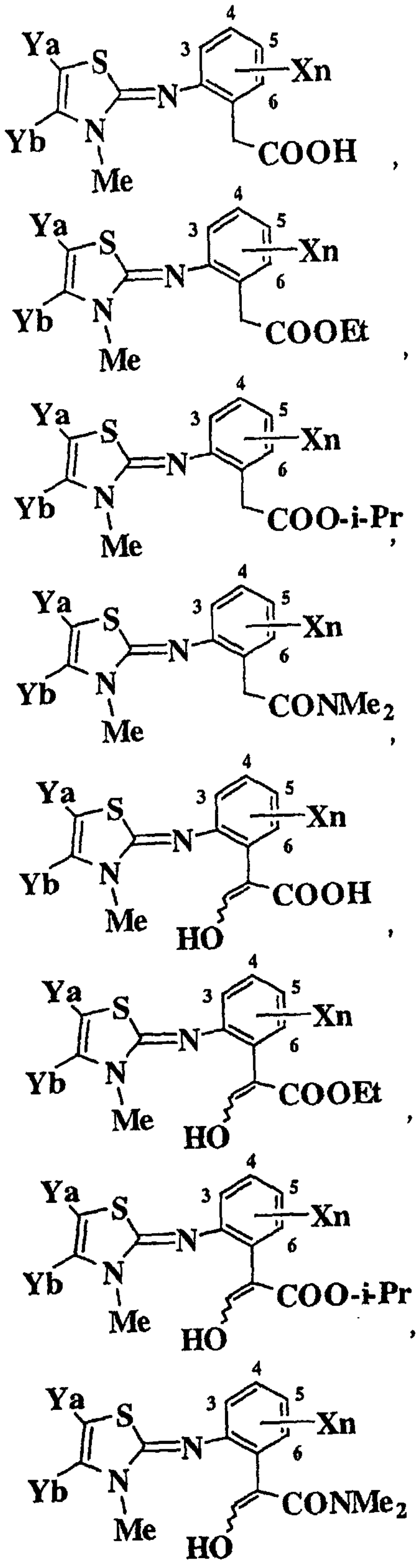

, , , , , , , ,

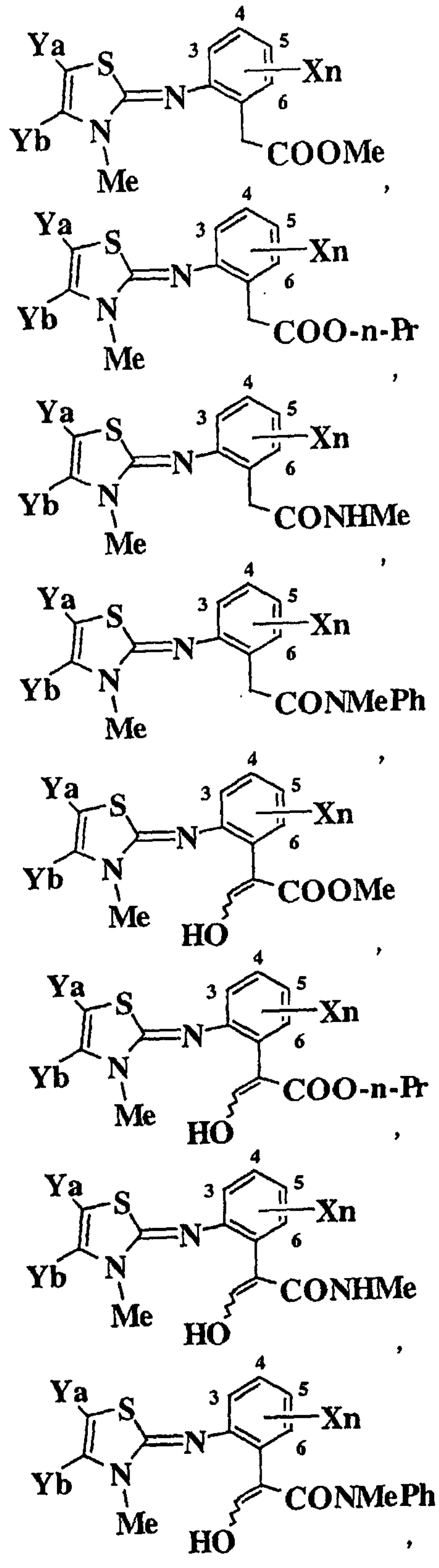

, , , , , , , ,

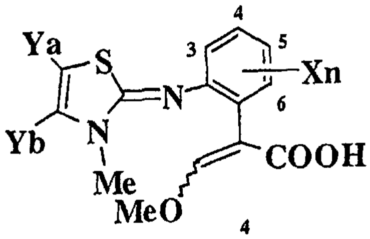
,
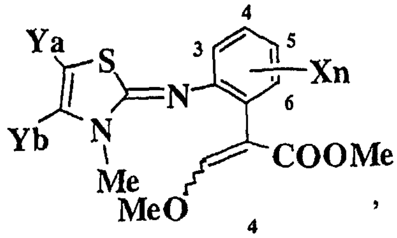
,
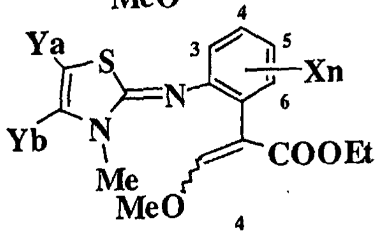
,
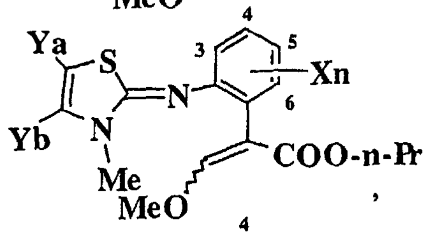
,
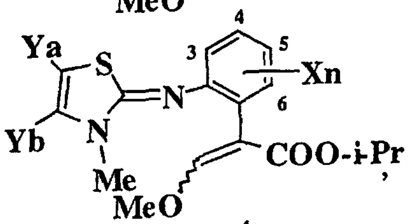
,
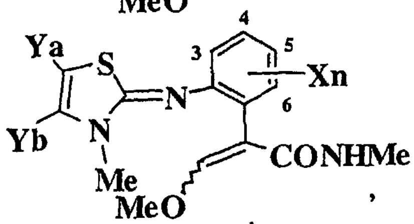
,
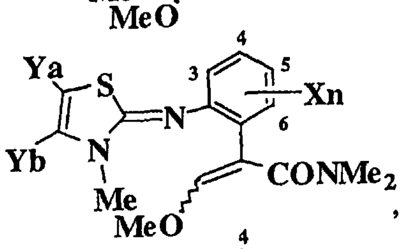
,
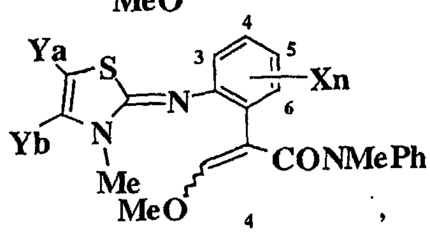
,
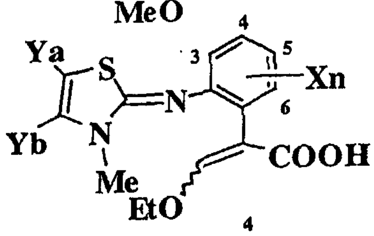
,
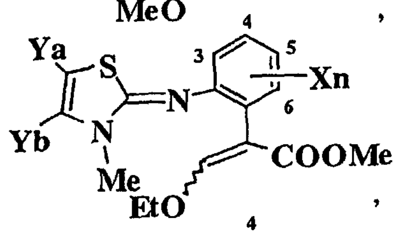
,
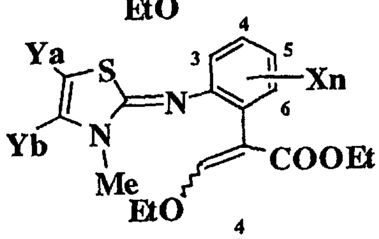
,
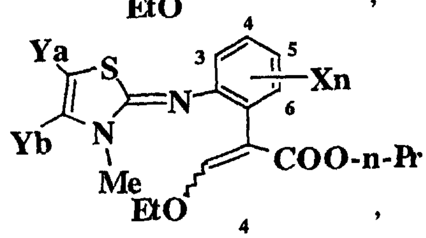
,
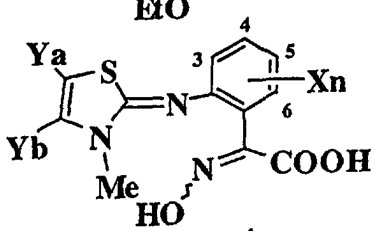
,
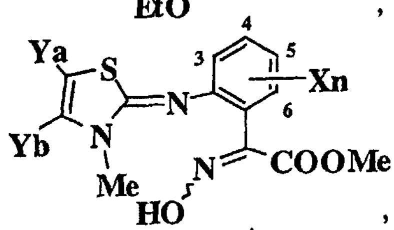
,
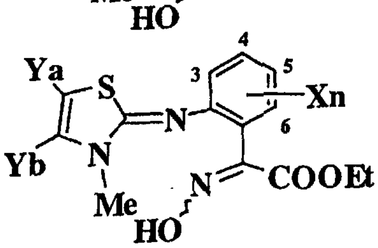
,
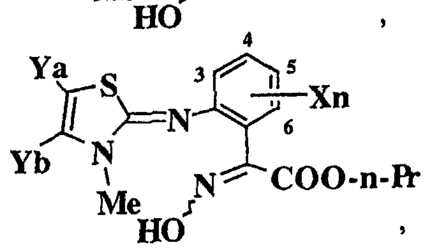
,

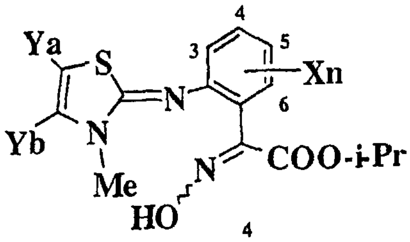
,
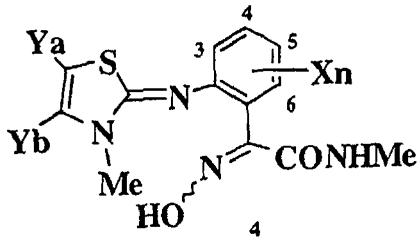
,
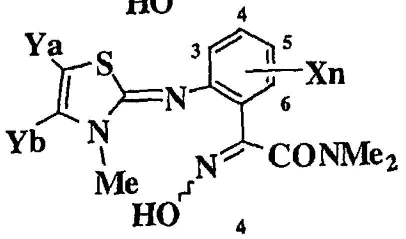
,
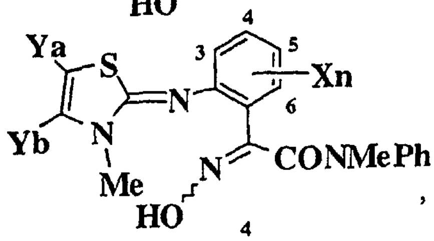
,
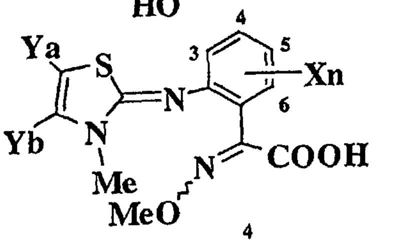
,
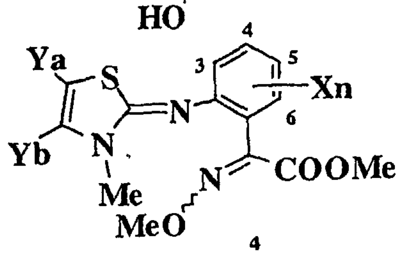
,
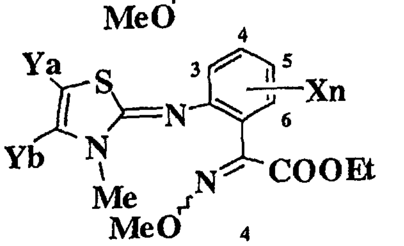
,
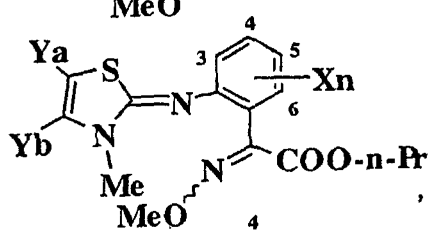
,
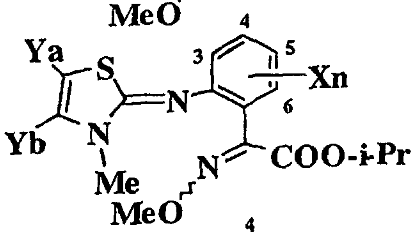
,
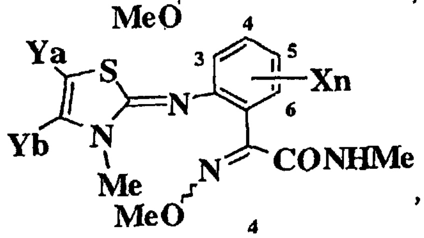
,
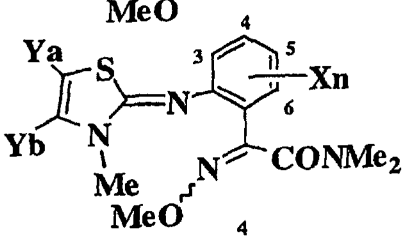
,
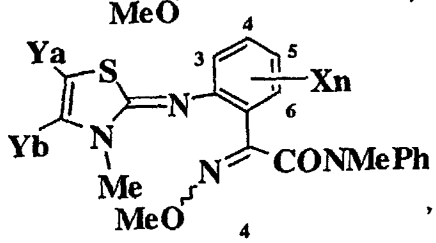
,
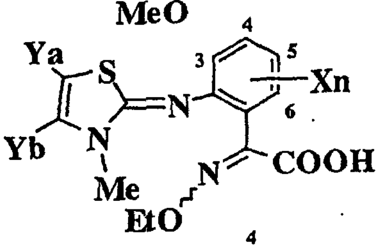
,
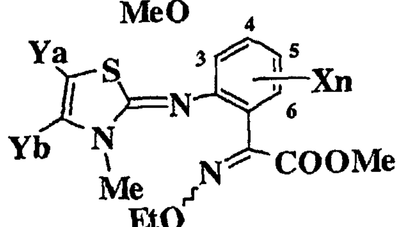
,
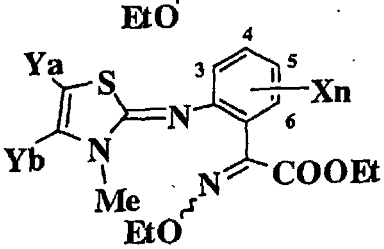
,
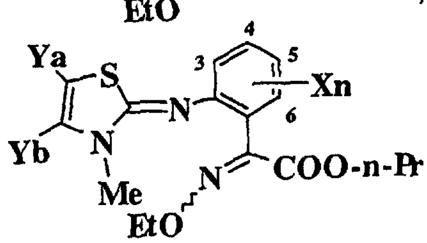

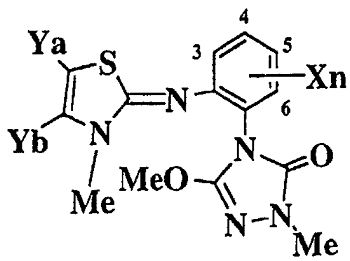
,

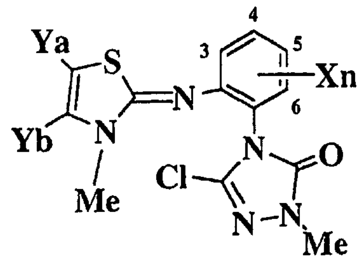
,

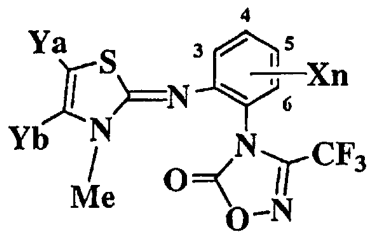
,

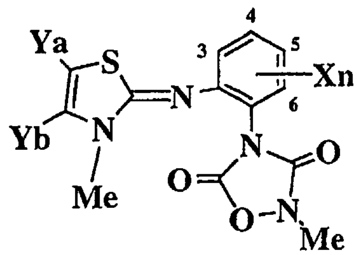
,

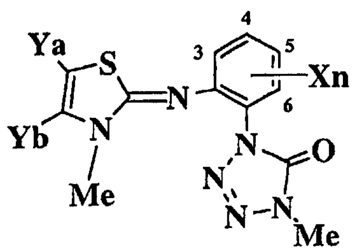
,

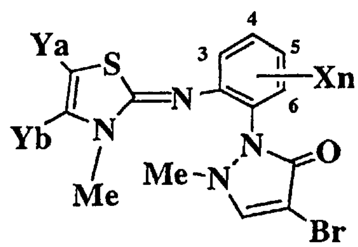
,

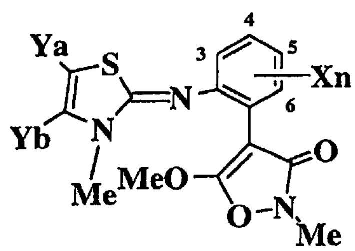
,

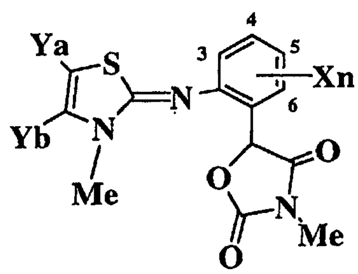
,

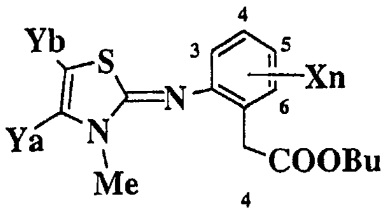 ,

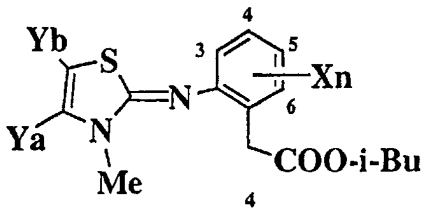 ,

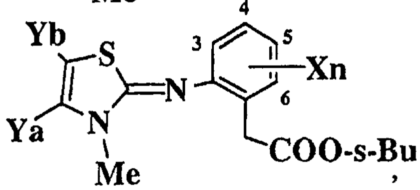 ,

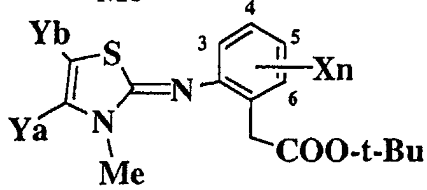 ,

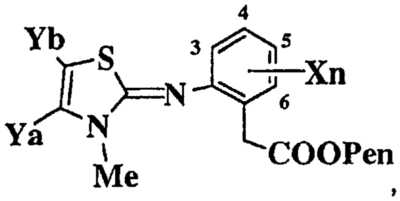 ,

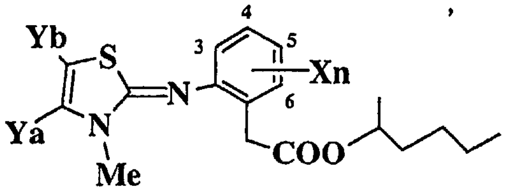 ,

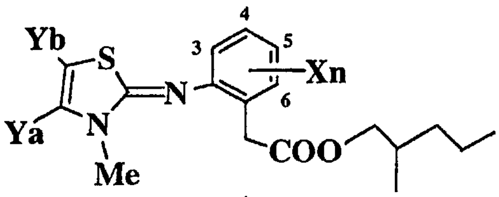 ,

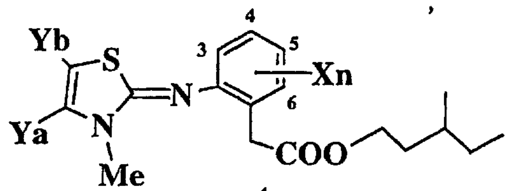 ,

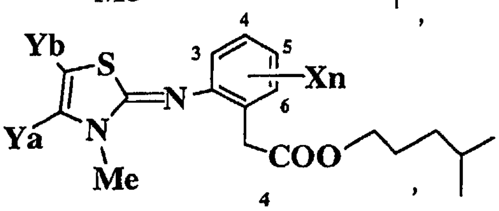 ,

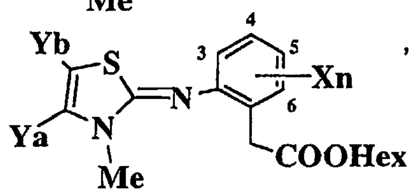 ,

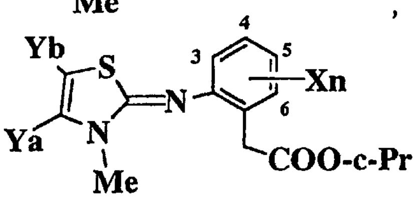 ,

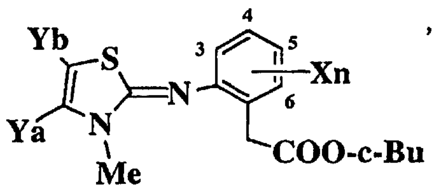 ,

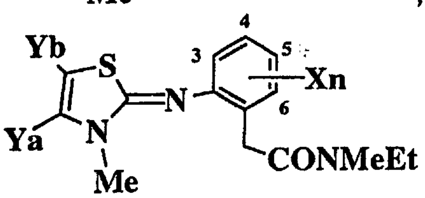 ,

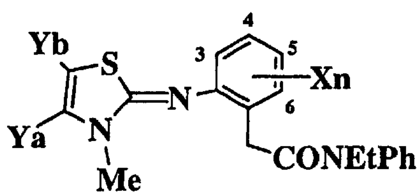 ,

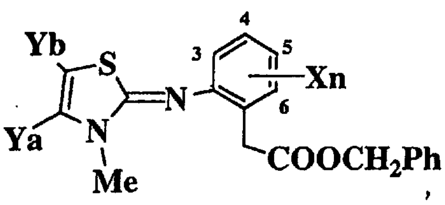 ,

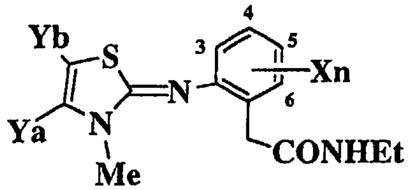 ,

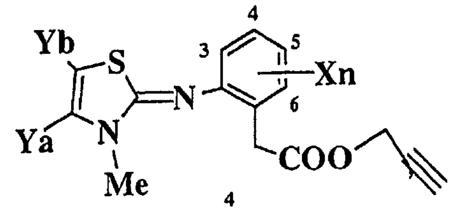

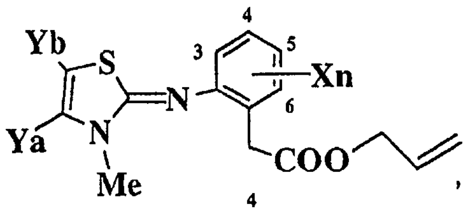

,

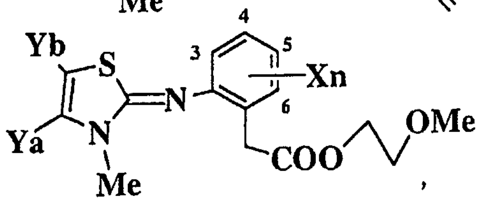

,

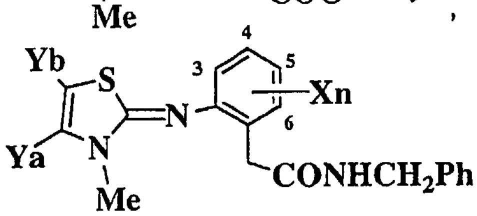

,

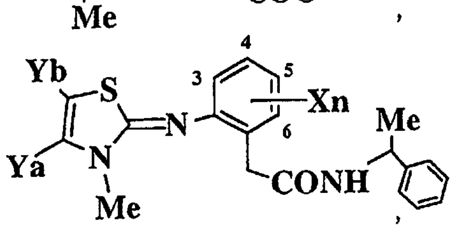

,

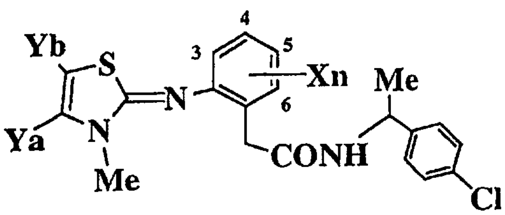

,

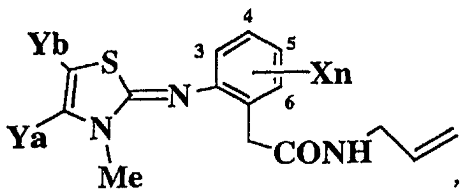

,

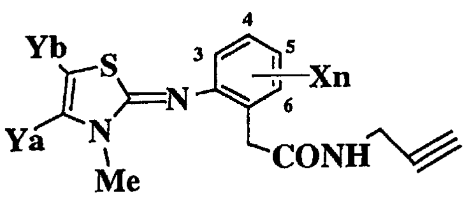

,

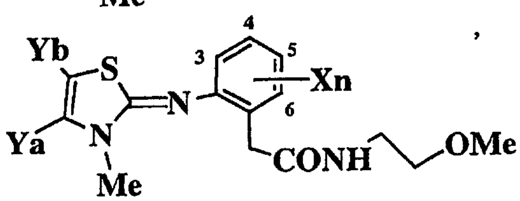

,

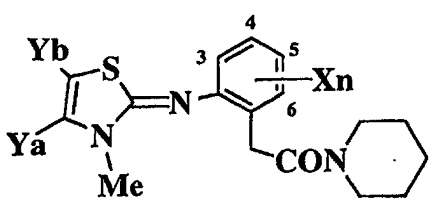

,

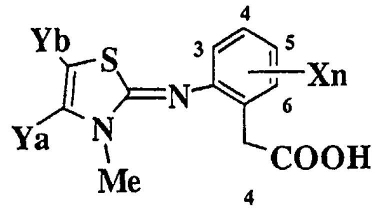 ,

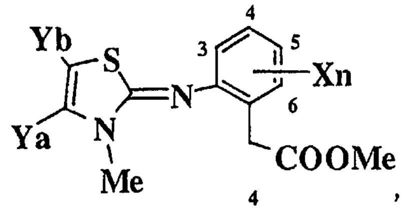 ,

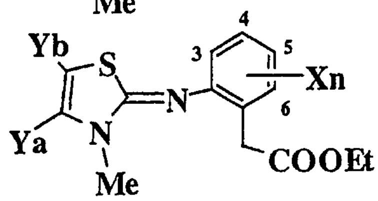 ,

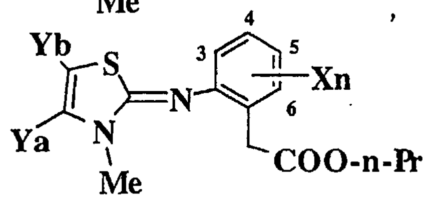 ,

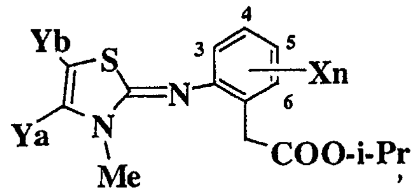 ,

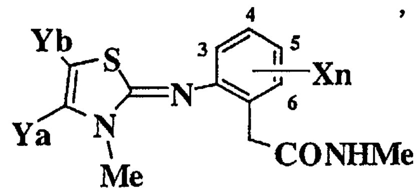 ,

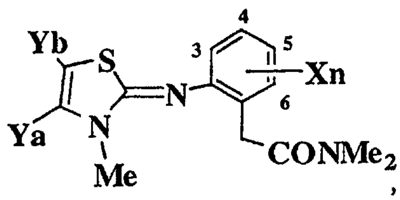 ,

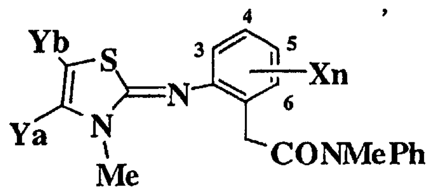 ,

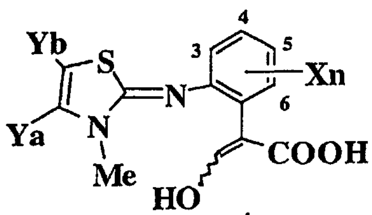 ,

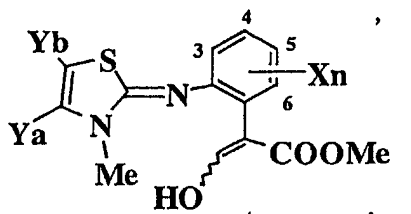 ,

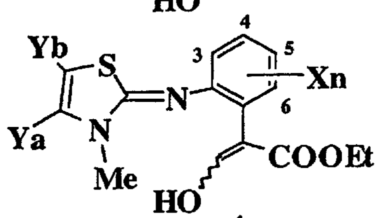 ,

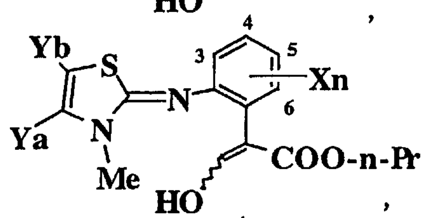 ,

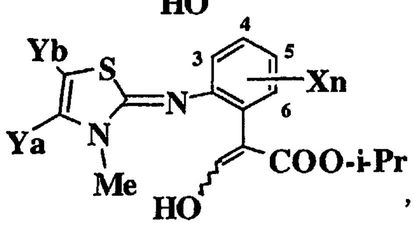 ,

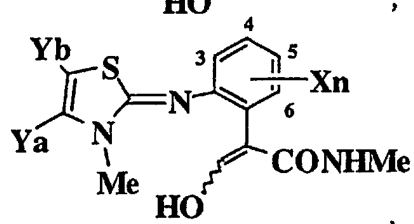 ,

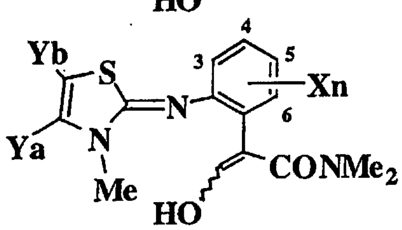 ,

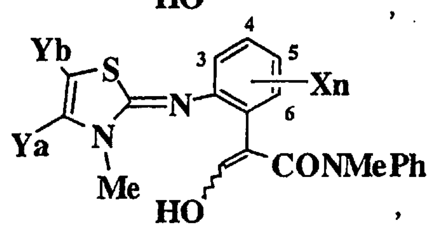 ,

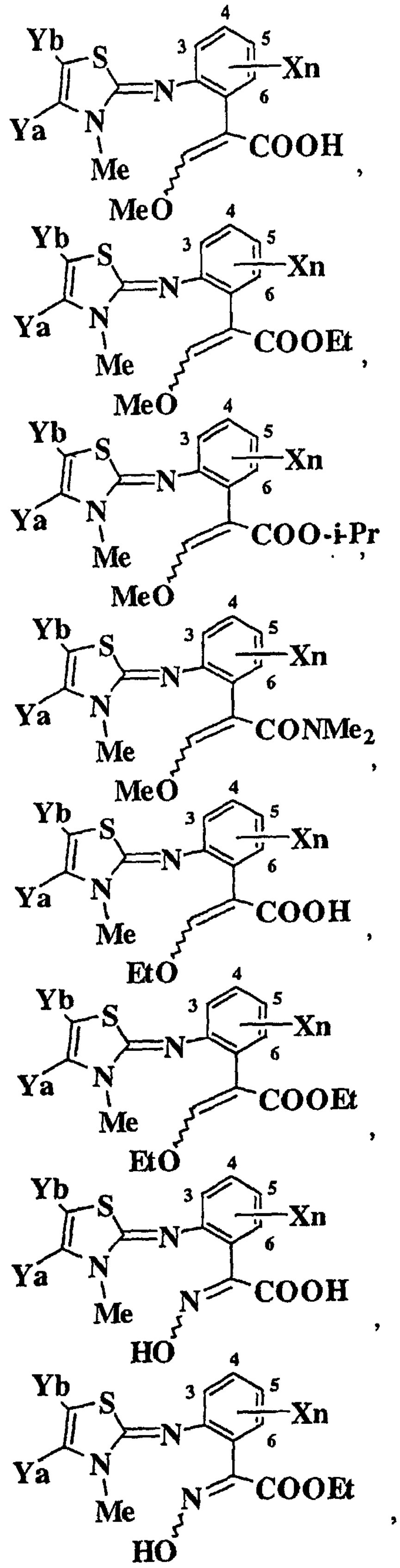

,
,
,
,
,
,
,
,

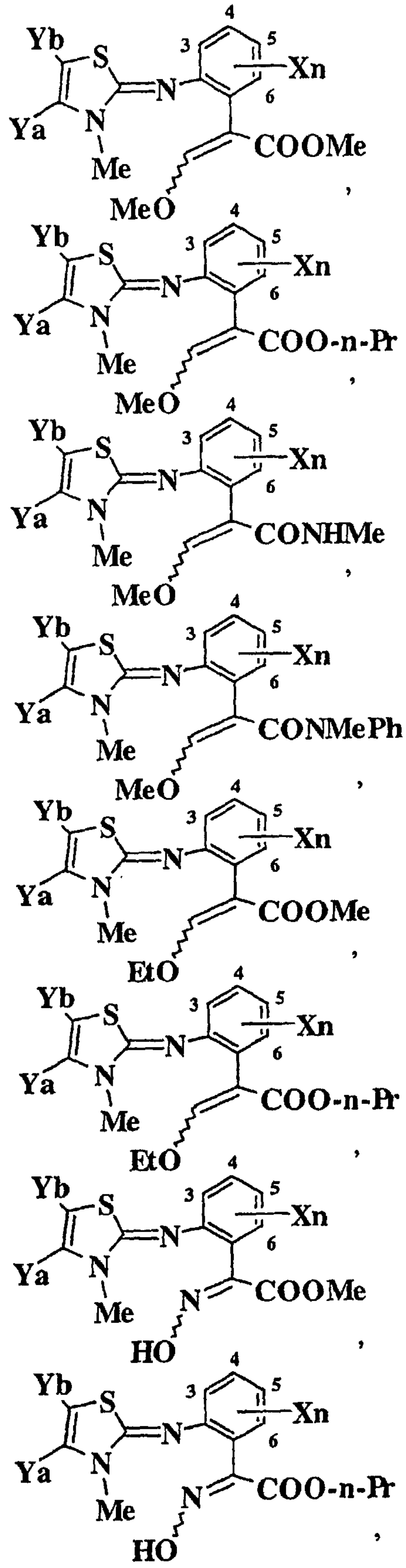

,
,
,
,
,
,
,
,

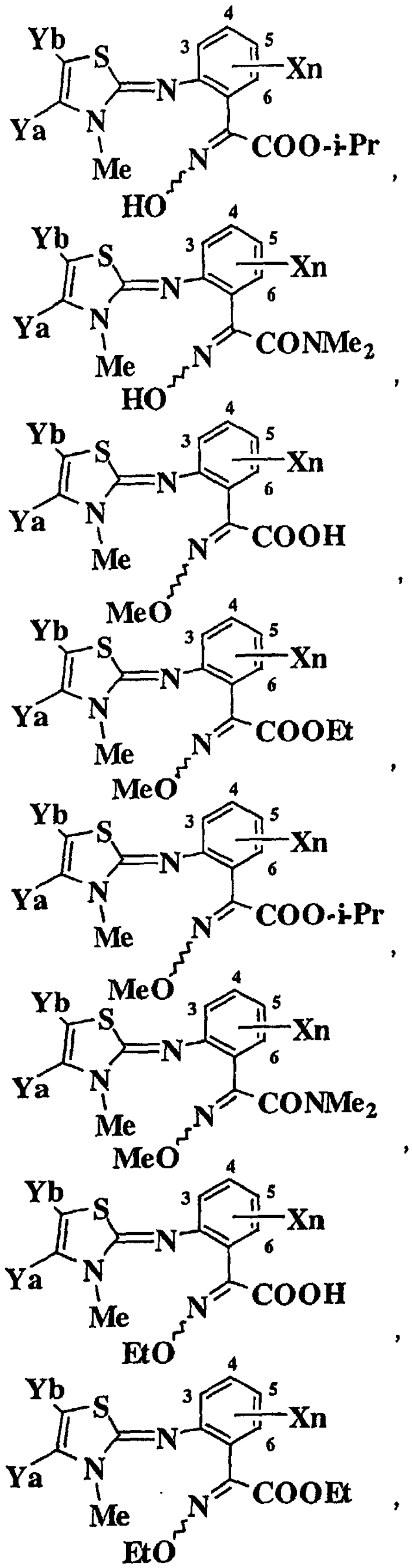

,

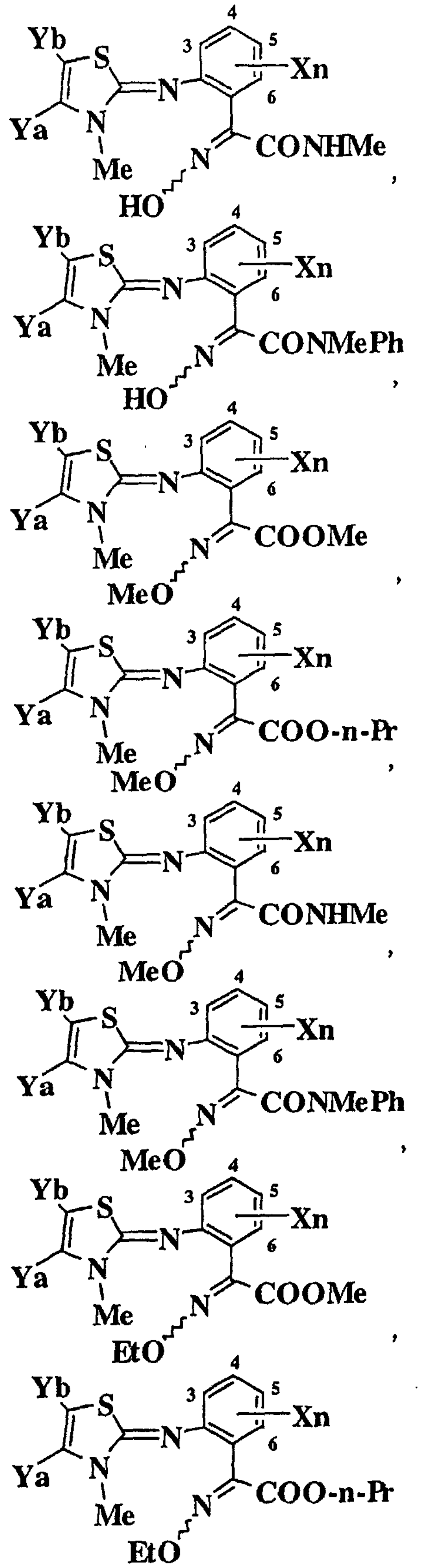

,

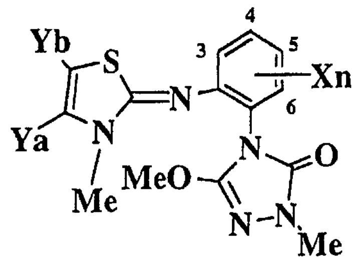

,

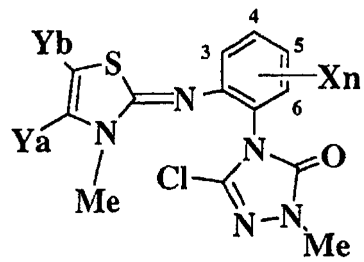

,

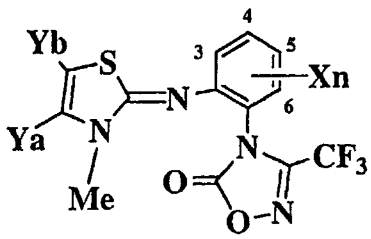

,

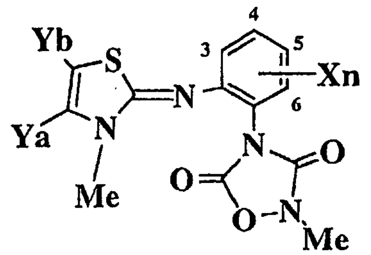

,

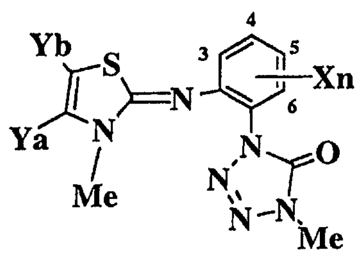

,

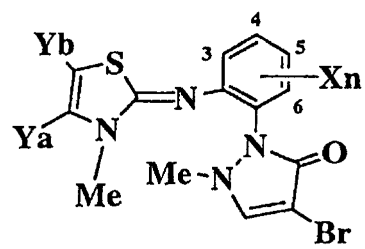

,

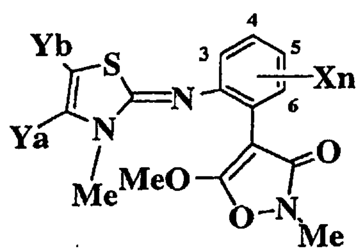

,

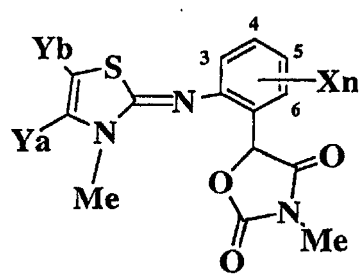

,

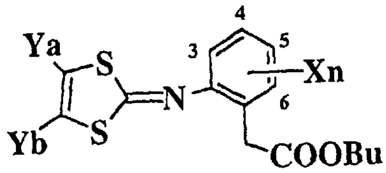 ,

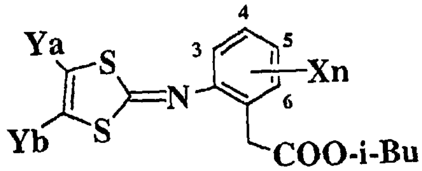 ,

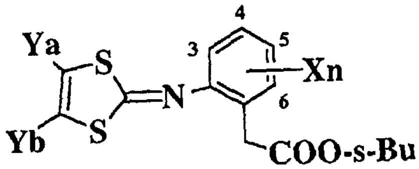 ,

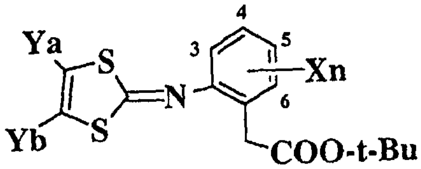 ,

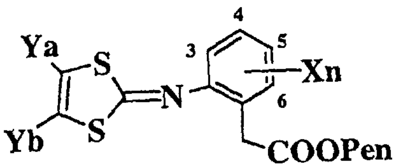 ,

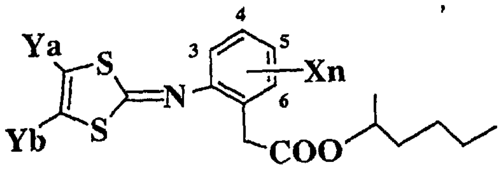 ,

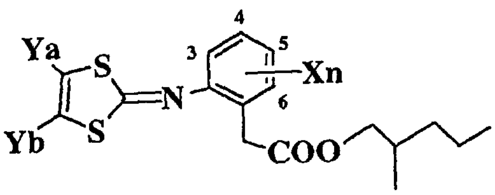 ,

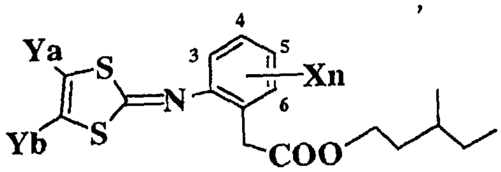 ,

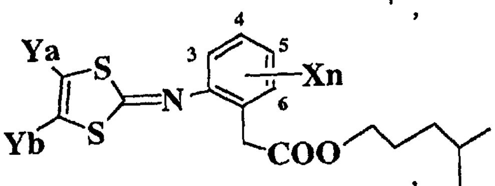 ,

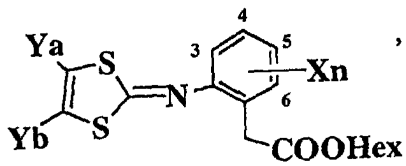 ,

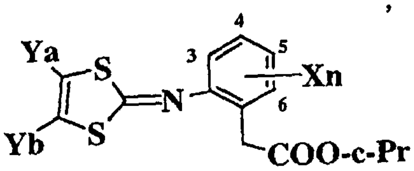 ,

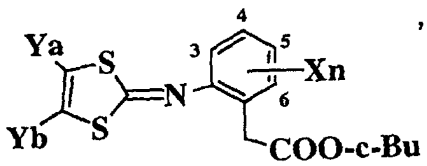 ,

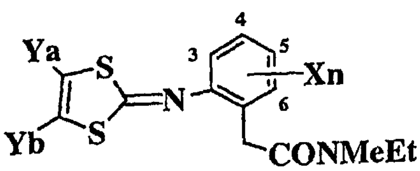 ,

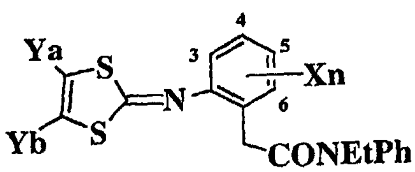 ,

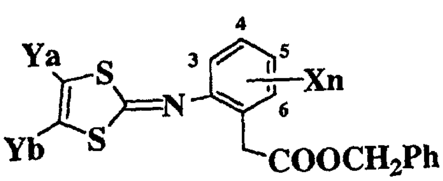 ,

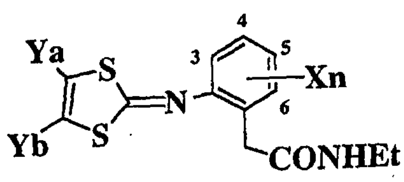 ,

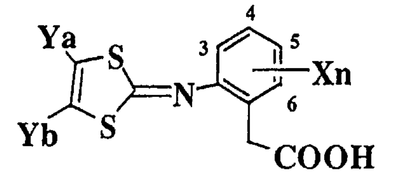,
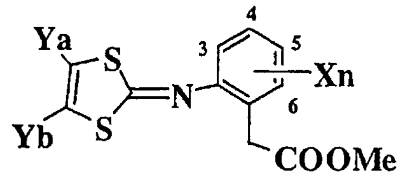,
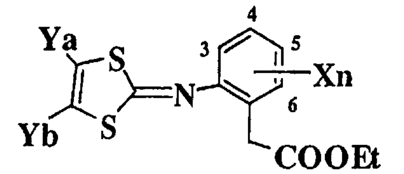,
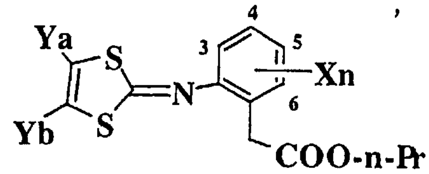,
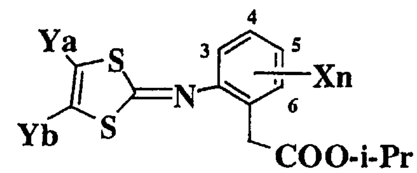,
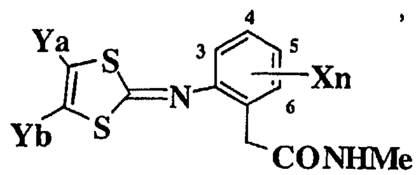,
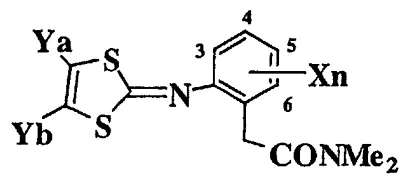,
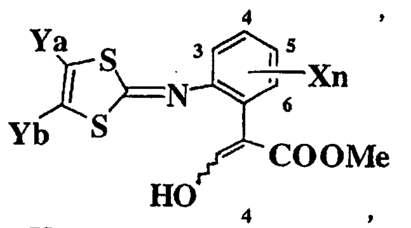,
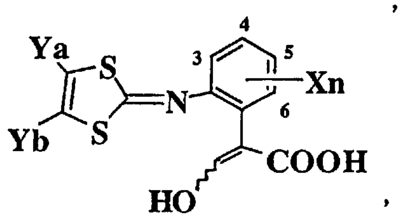,
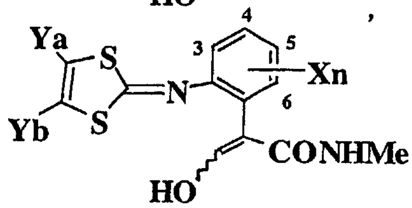,
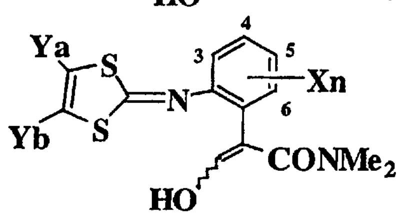,
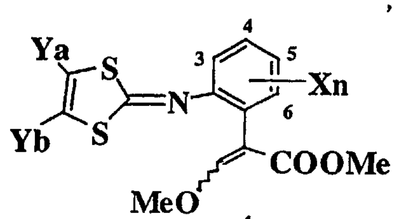,
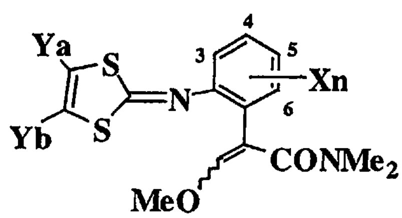,
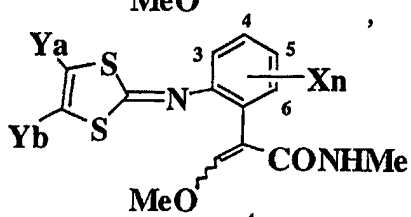,
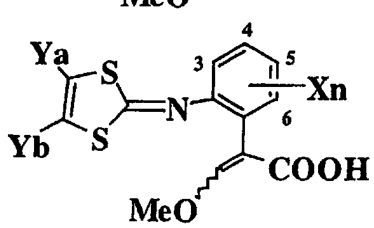,
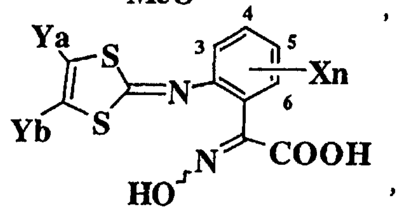,

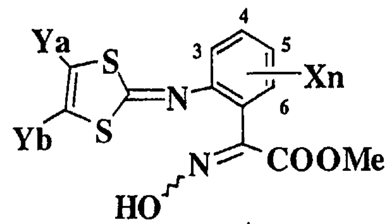

,

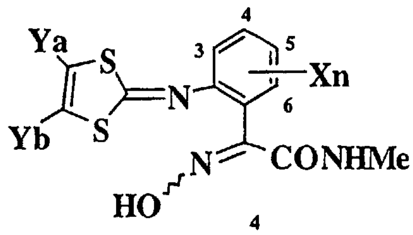

,

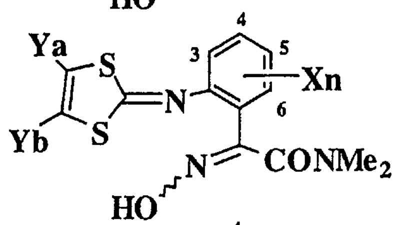

,

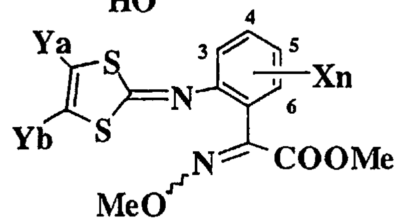

,

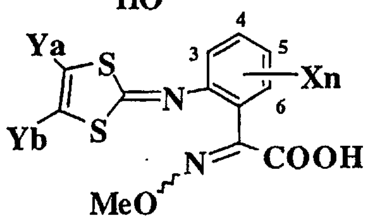

,

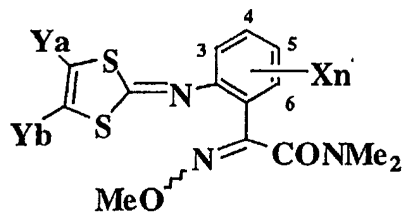

,

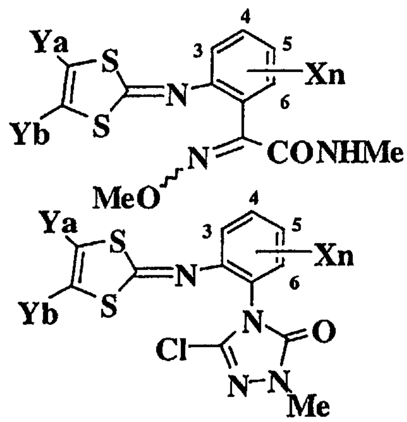

,

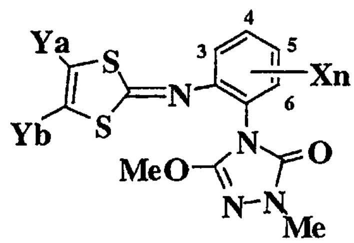

,

,

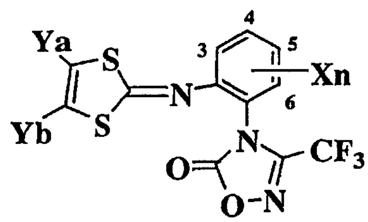

,

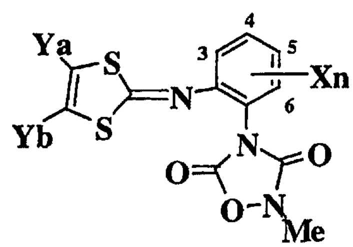

,

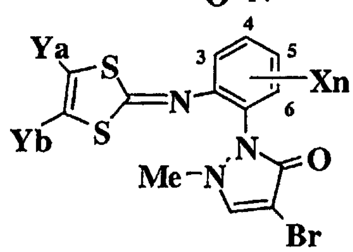

,

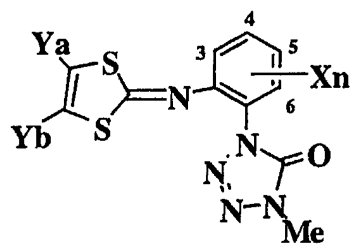

,

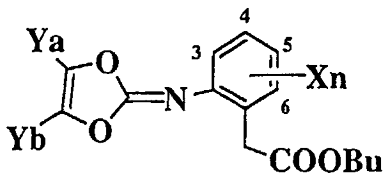
,
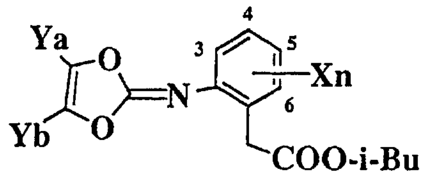
,
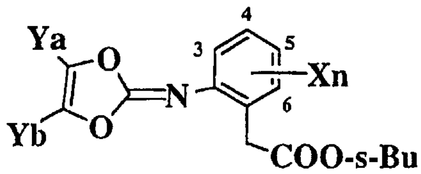
,
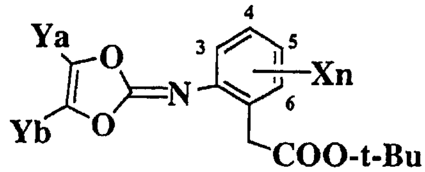
,
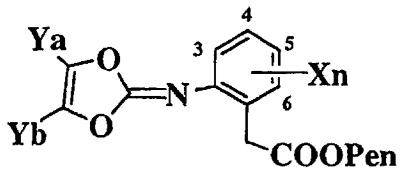
,
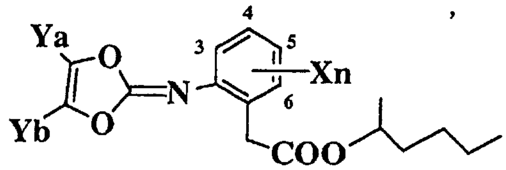
,
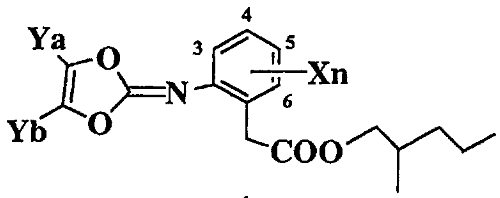
,
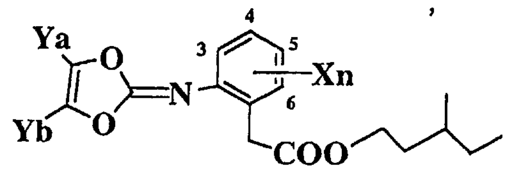
,
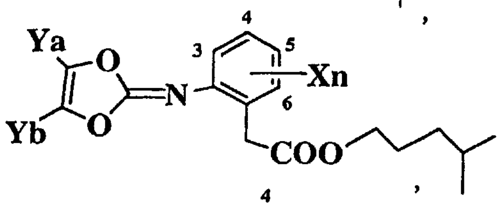
,
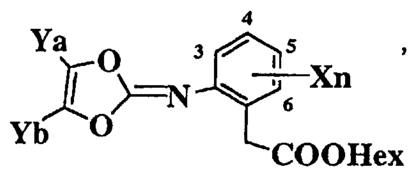
,
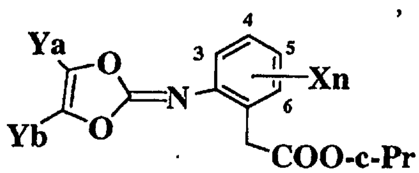
,
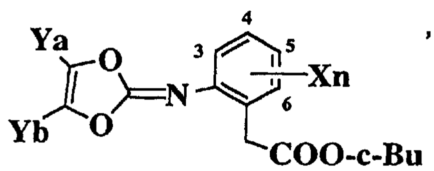
,
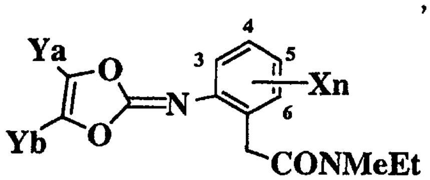
,
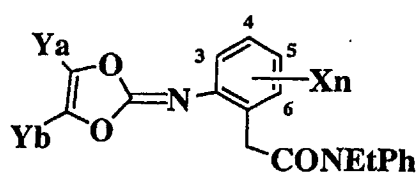
,
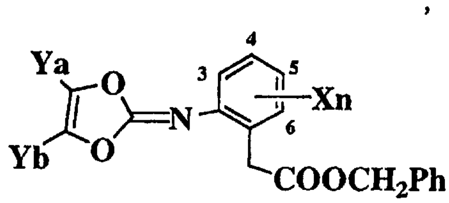
,
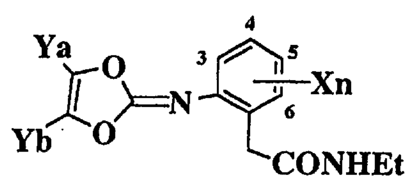
,

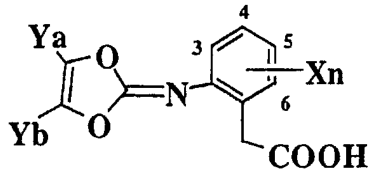

,

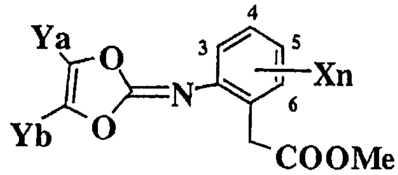

,

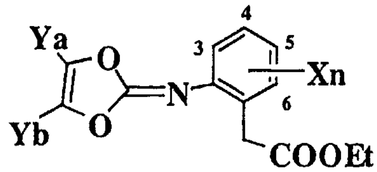

,

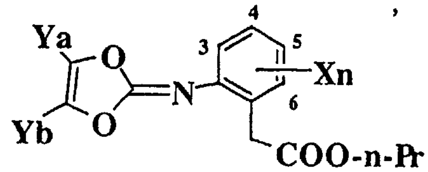

,

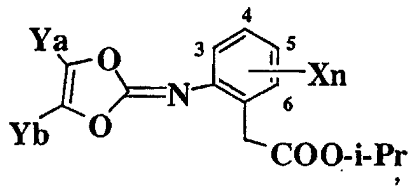

,

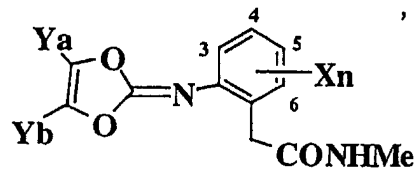

,

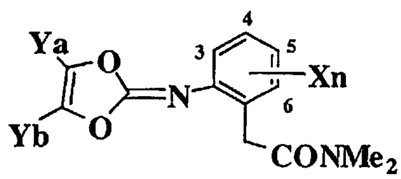

,

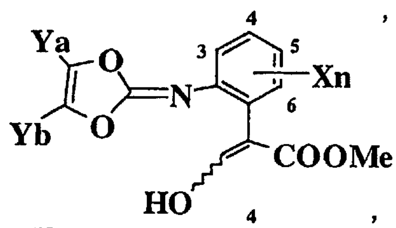

,

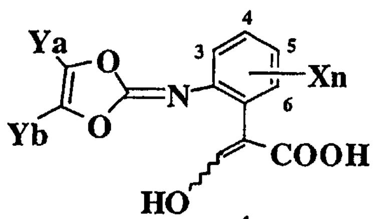

,

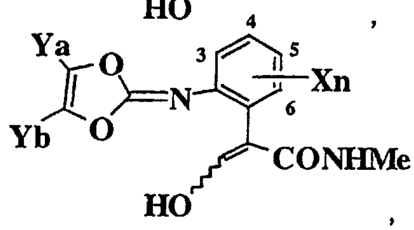

,

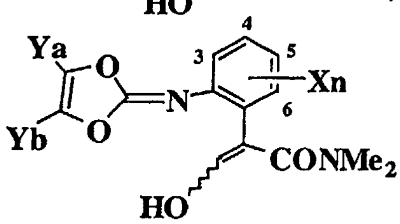

,

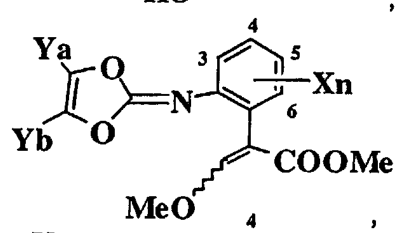

,

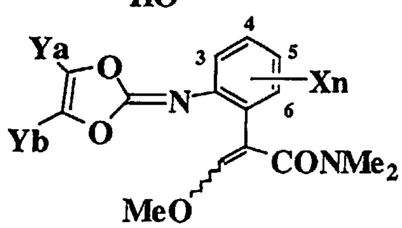

,

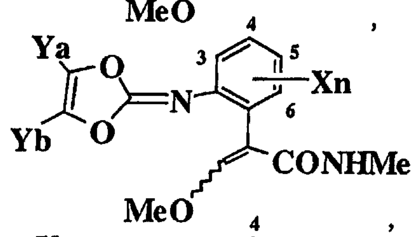

,

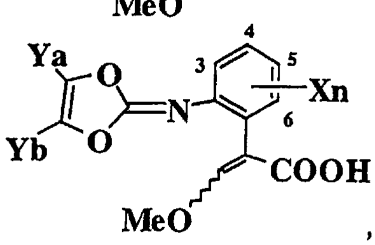

,

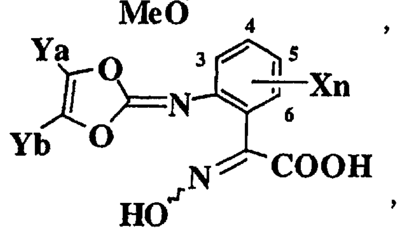

,

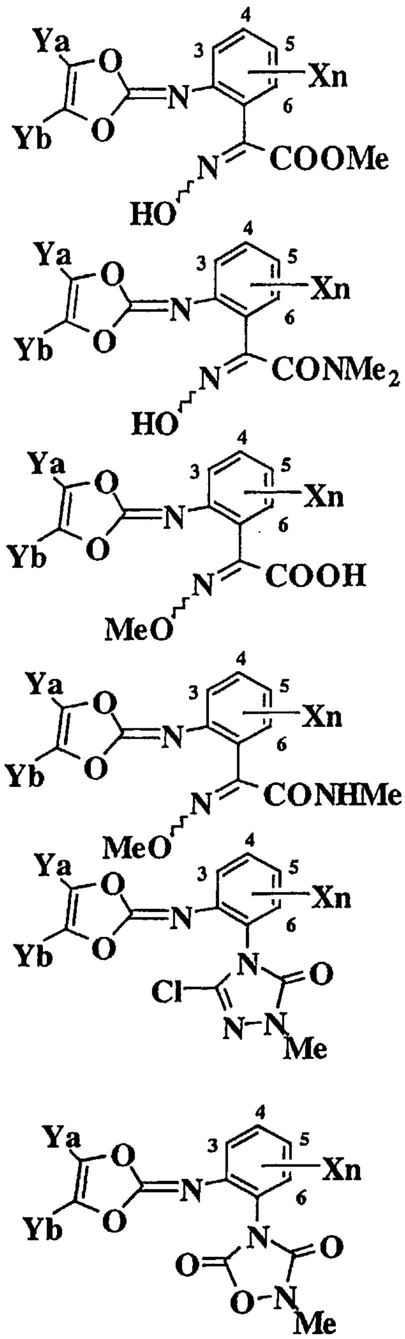

,

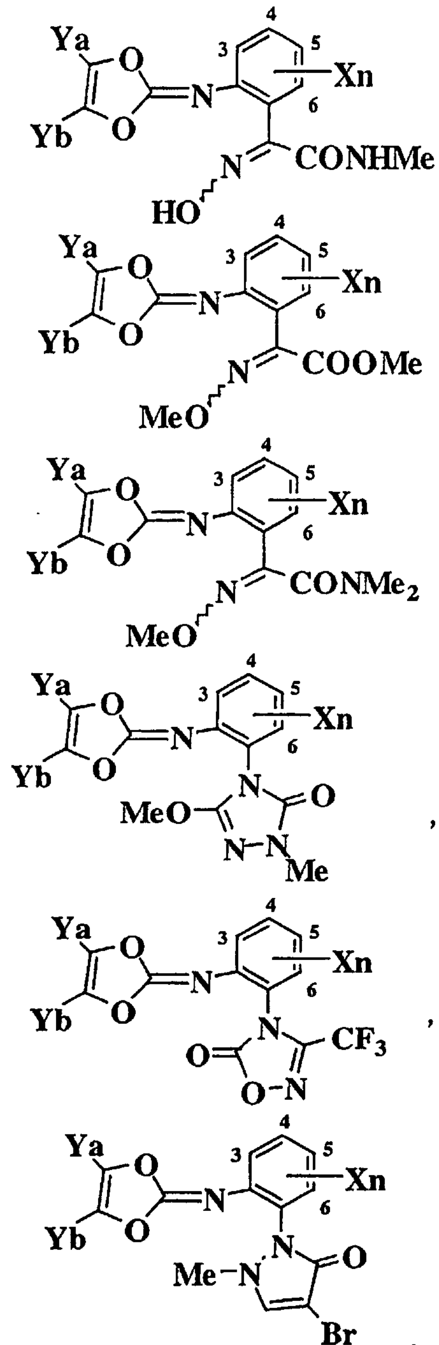

,

,

,

,

,

,

,

,

,

,

,

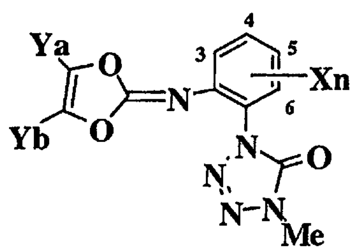

,

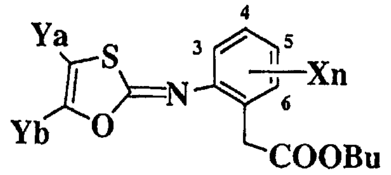
,
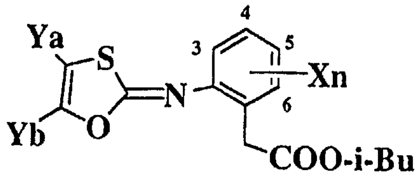
,
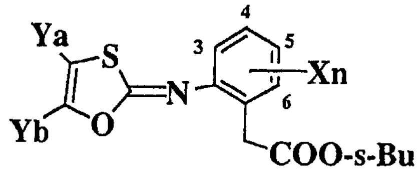
,
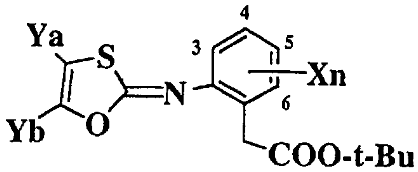
,
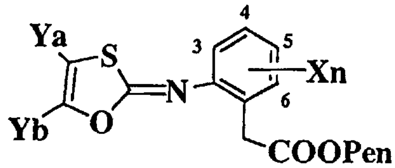
,
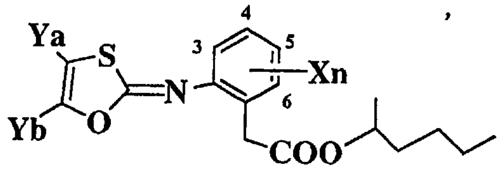
,
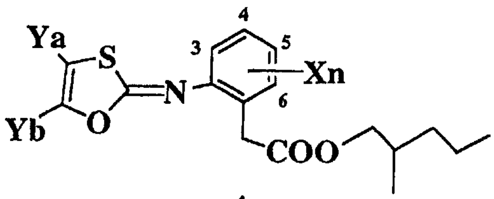
,
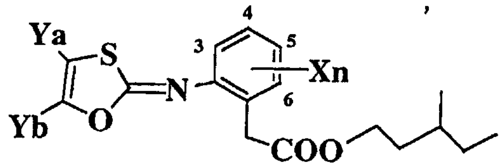
,
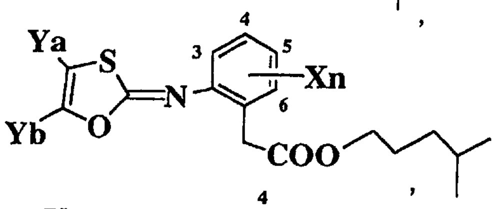
,
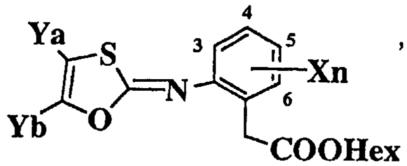
,
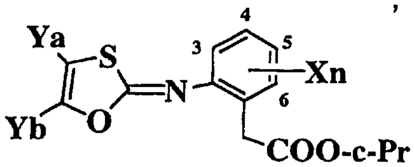
,
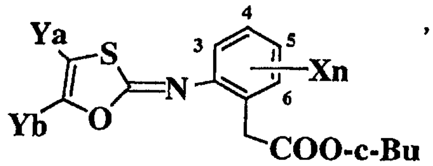
,
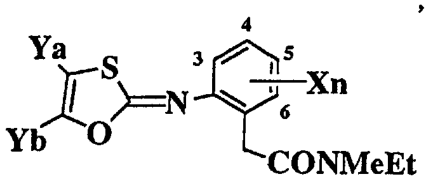
,
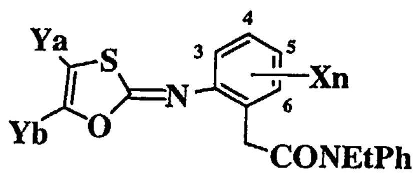
,
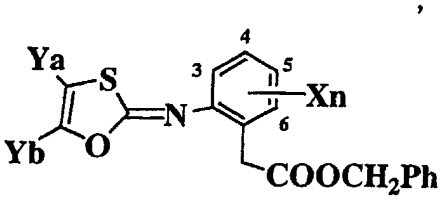
,
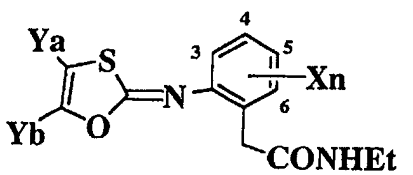
,

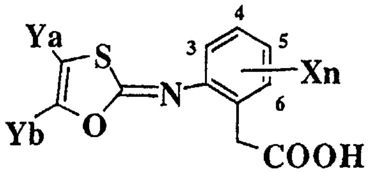
,
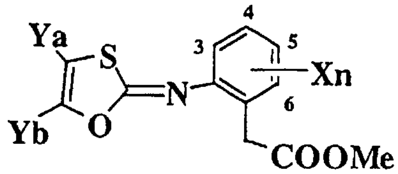
,
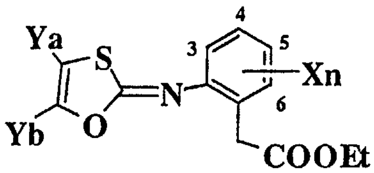
,
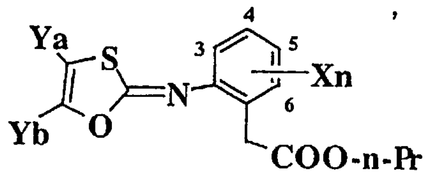
,
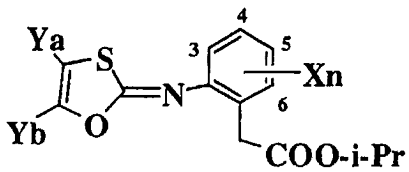
,
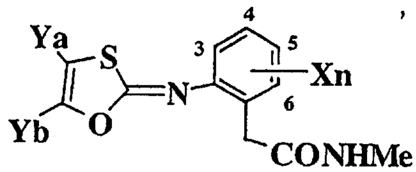
,
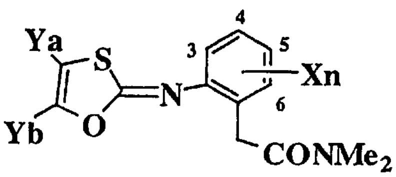
,
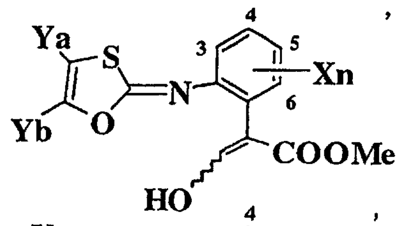
,
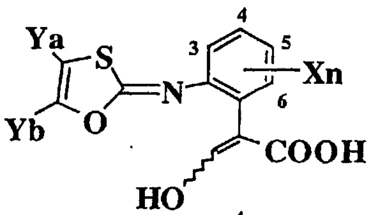
,
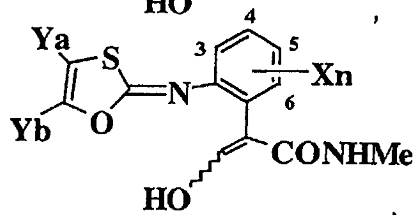
,
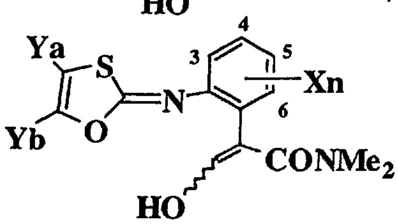
,
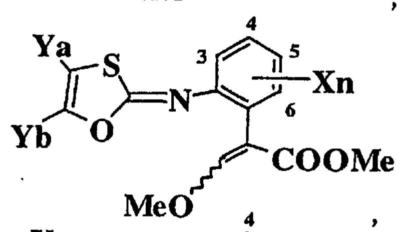
,
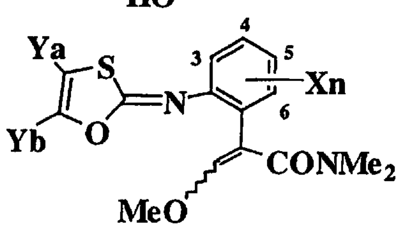
,
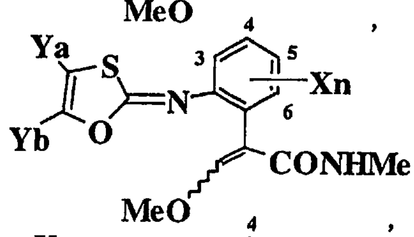
,
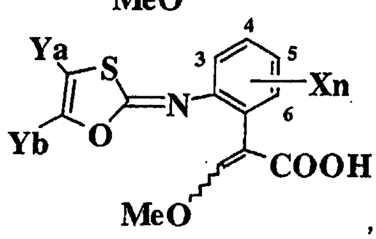
,
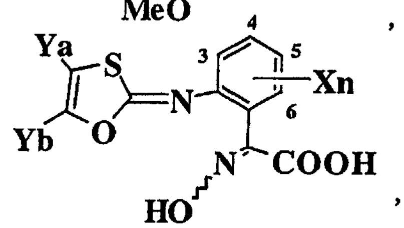
,

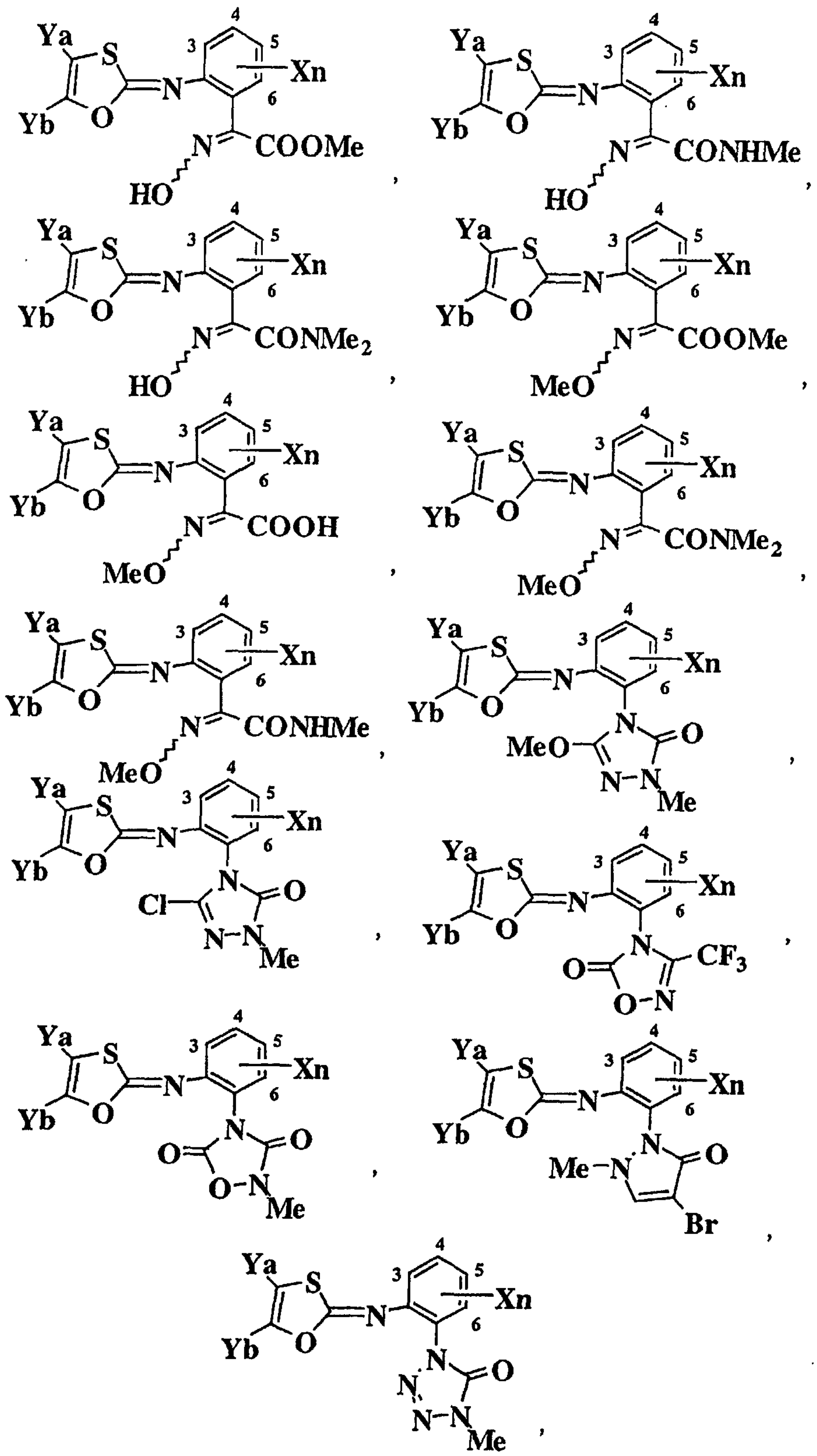

, , , , , , , , , , , , ,

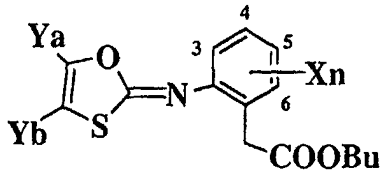
,
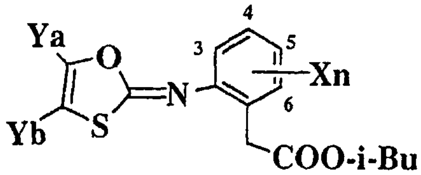
,
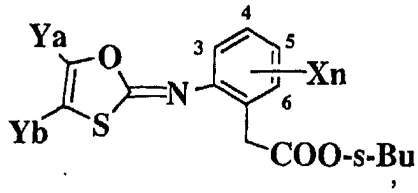
,
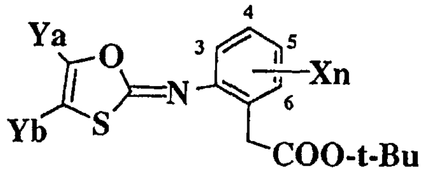
,
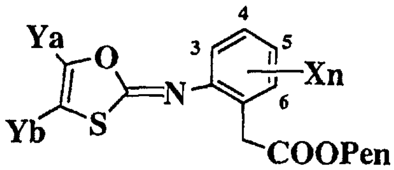
,
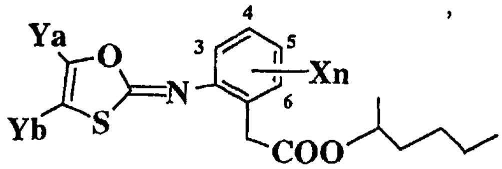
,
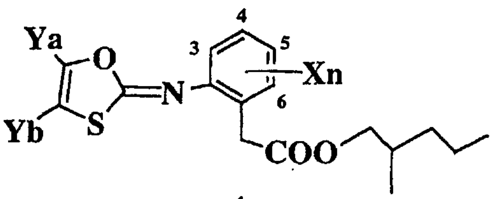
,
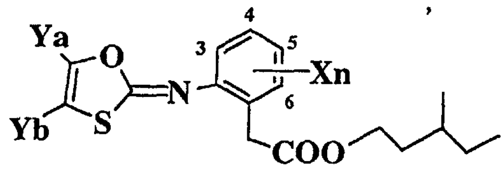
,
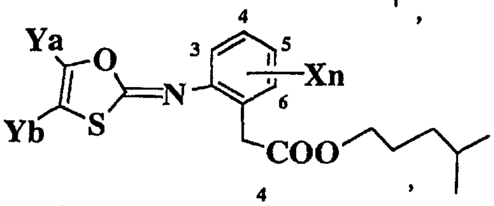
,
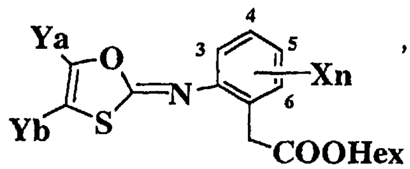
,
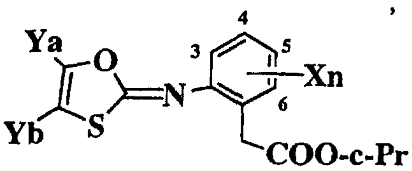
,
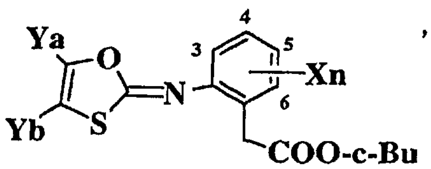
,
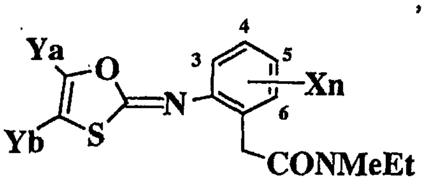
,
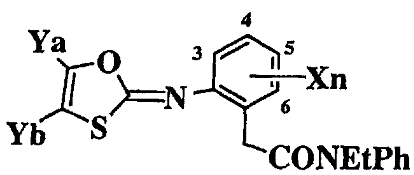
,
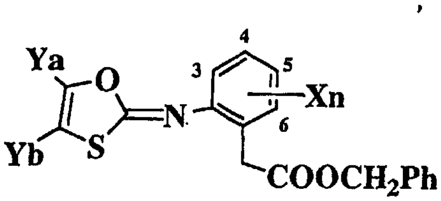
,
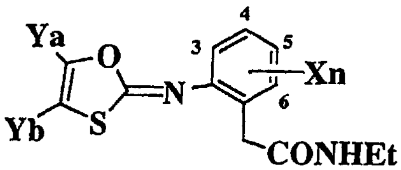
,

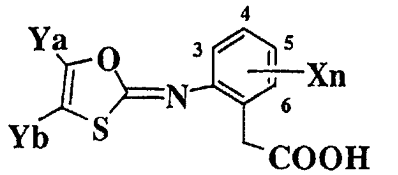

,

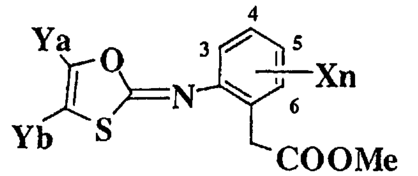

,

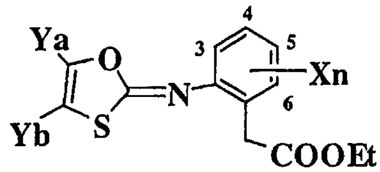

,

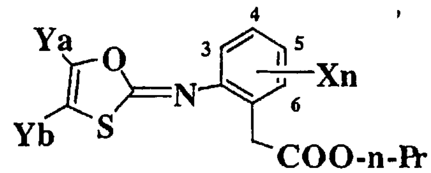

,

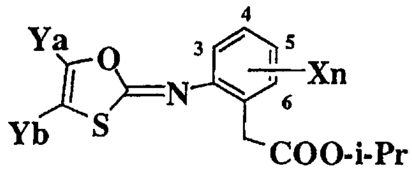

,

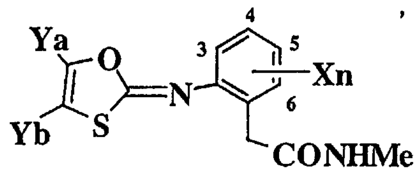

,

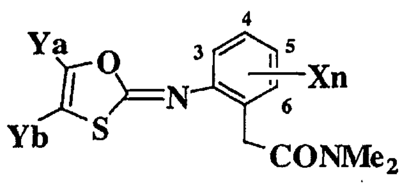

,

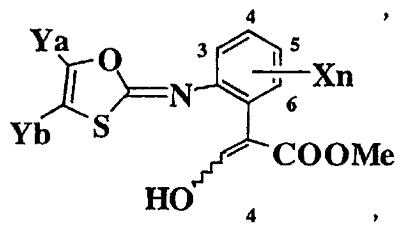

,

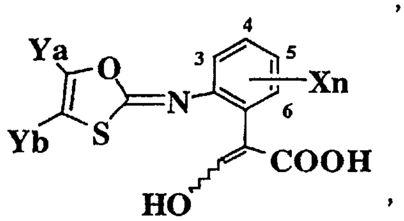

,

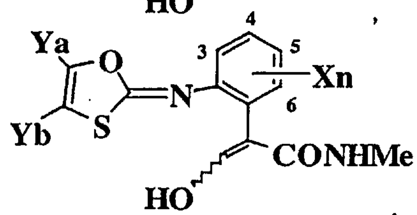

,

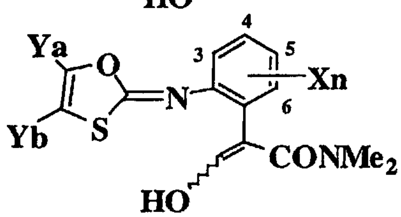

,

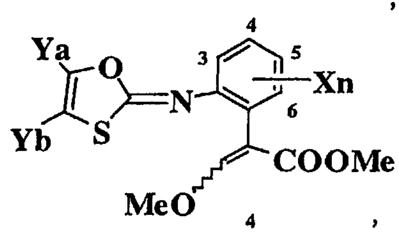

,

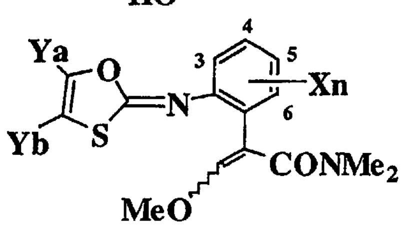

,

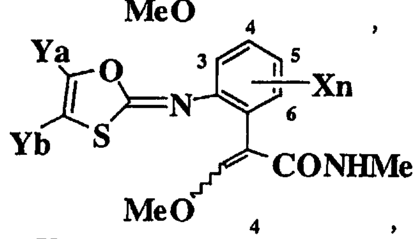

,

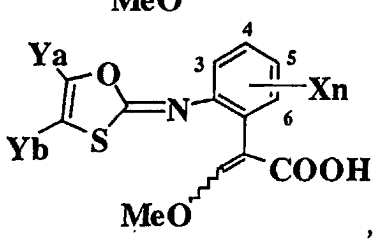

,

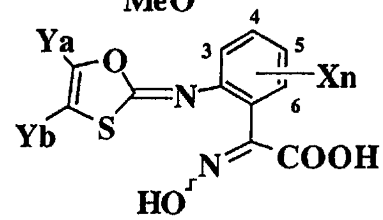

,

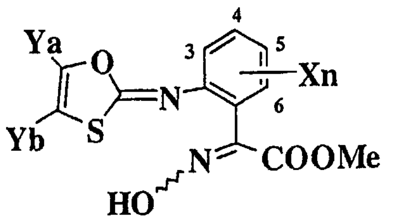 , 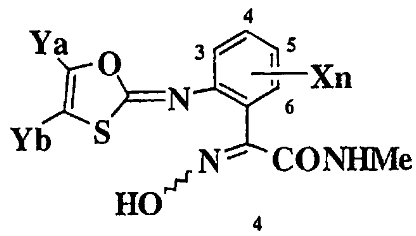 ,

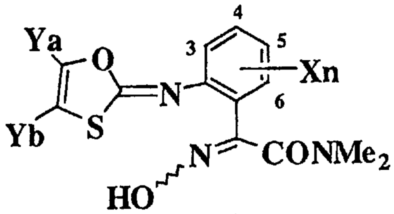 , 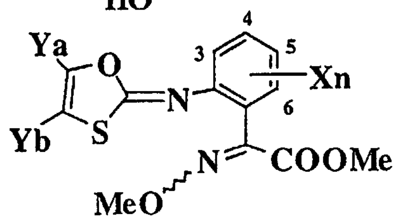 ,

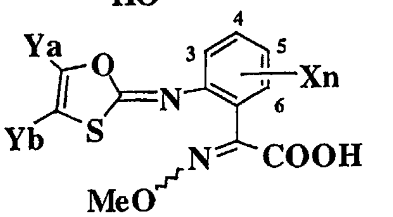 , 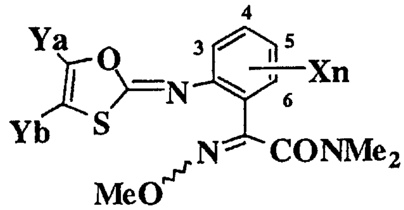 ,

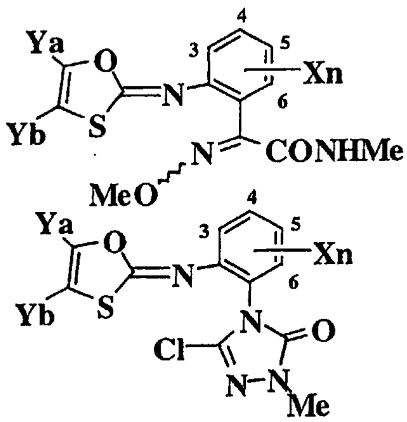 , 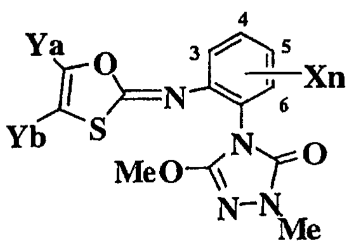 ,

, 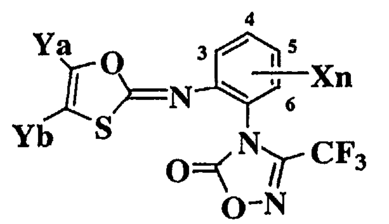 ,

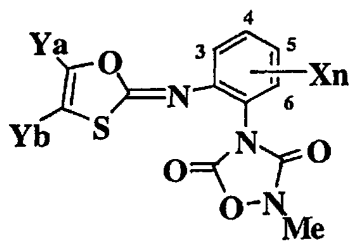 , 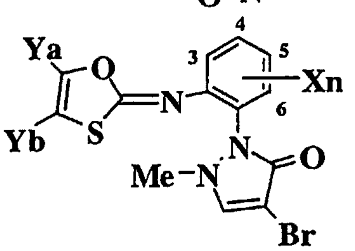 ,

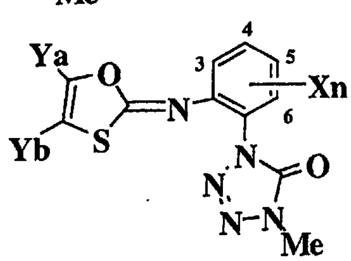 ,

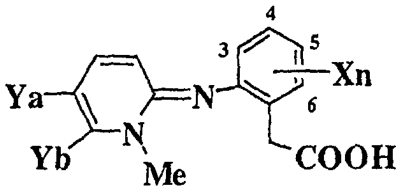
,
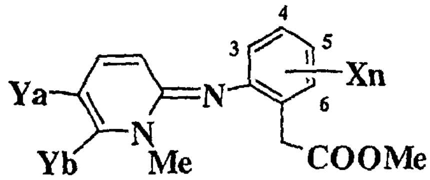
,

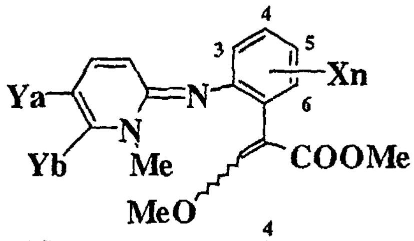
,
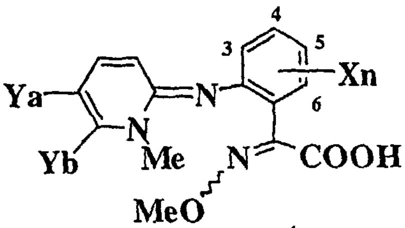
,

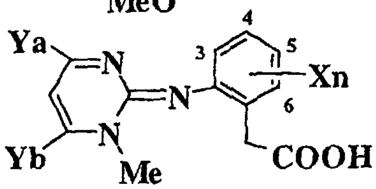
,
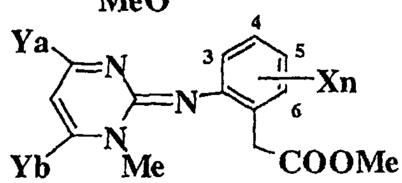
,

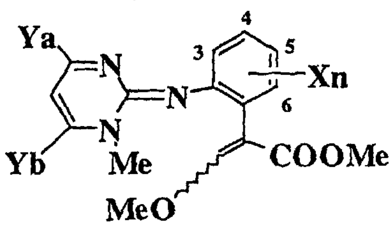
,
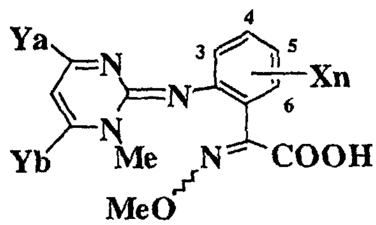
,

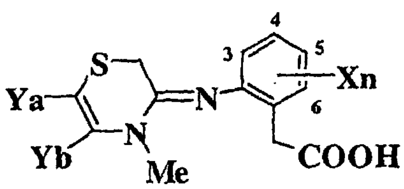
,
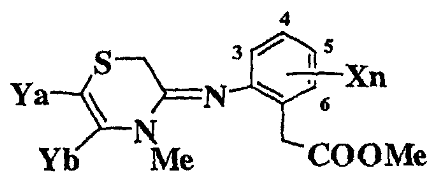
,

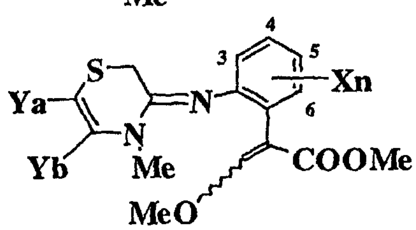
,
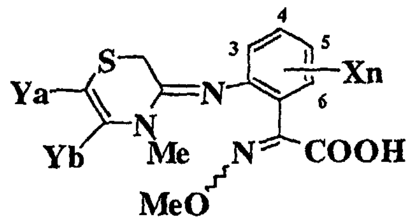
,

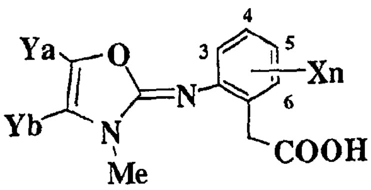 , 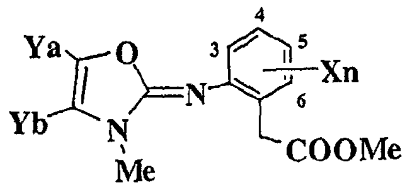 ,

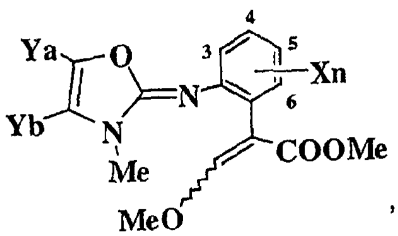 , 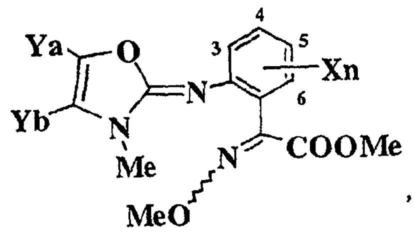 ,

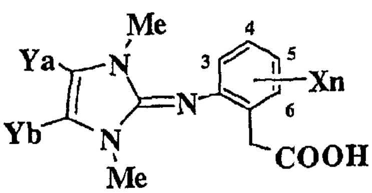 , 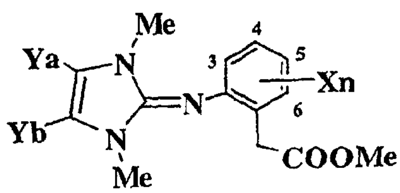 ,

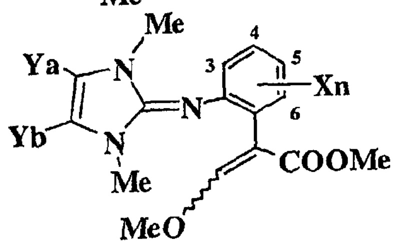 or 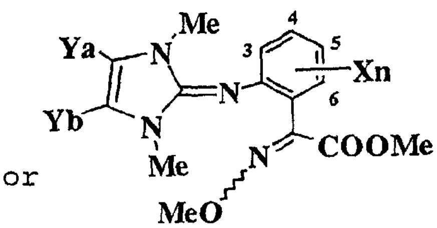

| Y a | Y b | X |
|---|---|---|
| H | H | H |
| H | Cl | H |
| H | F | H |
| H | Br | H |
| H | I | H |
| H | Me | H |
| H | Et | H |
| H | n-Pr | H |
| H | i-Pr | H |
| H | n-Bu | H |
| H | i-Bu | H |
| H | s-Bu | H |
| H | t-Bu | H |
| H | n-Pen | H |
| H | 3-Me-n-Bu | H |
| H | n-Hex | H |
| H | Ethenyl | H |
| H | 1-Propenyl | H |
| H | Ethynyl | H |
| H | $CF_3$ | H |
| H | c-Pr | H |
| H | c-Hex | H |
| H | MeO | H |
| H | t-BuO | H |

| | | |
|---|---|---|
| H | $CF_3O$ | H |
| H | MeS | H |
| H | MeSO | H |
| H | $MeSO_2$ | H |
| H | $NO_2$ | H |
| H | NC | H |
| H | CHO | H |
| H | $H_2N$ | H |
| H | HMeN | H |
| H | $Me_2N$ | H |
| H | $PhCH_2$ | H |
| H | PhCH=CH | H |
| H | 4-Cl-PHCH=CH | H |
| H | $PhCH=CHCH_2$ | H |
| H | PhCBr=CBr | H |
| H | PhCC | H |
| H | Ph | H |
| H | MeOCO | H |
| H | EtOCO | H |
| H | $MeOCH_2$ | H |
| H | MeOC(=NOMe) | H |
| H | MeSC(=NOMe) | H |
| H | MeC(=NOMe) | H |
| H | MeC(=NOMe)C(=NOMe) | H |
| H | PhC(=NOMe) | H |
| H | $MeC(=NOCH_2Ph)$ | H |
| H | COMe | H |
| H | $Me_2C=N$ | H |
| H | $CH_2SMe$ | H |
| H | $CH_2OPh$ | H |
| H | $4-Me-PhOCH_2$ | H |
| H | $2,4-Cl_2-PhOCH_2$ | H |
| H | $CH_2SCH_2Ph$ | H |
| H | $CH_2NMe_2$ | H |
| H | $CH_2N(COMe)Me$ | H |
| H | $Me_2C=N-OCH_2$ | H |
| H | $CH_2N=NCHMePh$ | H |
| H | $Morpholino=CH_2$ | H |
| H | $Pyrazol-1-ylCH_2$ | H |
| H | $Hexamethyleneimino-CH_2$ | H |
| H | $3-Ph-Pyrazol-1-yl-CH_2$ | H |
| H | $Imidazol-1-yl-CH_2$ | H |
| H | $ClCH_2$ | H |
| H | $BrCH_2$ | H |
| H | $Pyrrole-1-yl-CH_2$ | H |
| H | $3-Cl-Pyrazol-1-yl-CH_2$ | H |
| H | $PhC(Me)=N-O-CH_2$ | H |
| H | $PhMeC=NOCH_2$ | H |
| H | $CF_2Cl$ | H |
| H | $CCl_3$ | H |
| H | $FCH_2$ | H |
| H | $ICH_2$ | H |
| H | $CHF_2$ | H |
| H | CHClF | H |
| H | $HCF_2CF_2$ | H |
| H | $CF_3CF_2$ | H |
| H | $ClCF_2CF_2$ | H |
| H | $CF_3CH_2$ | H |

| | | |
|---|---|---|
| H | $ClCF_2CH_2$ | H |
| H | $CFCl_2CH_2$ | H |
| H | $FCH_2CH_2$ | H |
| H | $CF_3CH_2CH_2$ | H |
| H | $PhNHCOCH_2$ | H |
| H | PhCONH | H |
| H | $PhCONHCH_2$ | H |
| H | 1-Pyrrolidyl-$CH_2$ | H |
| H | $PhC(CF_3)=N-O-CH_2$ | H |
| H | $PhN(Me)CH_2$ | H |
| H | 4-Cl-$PhCH_2ON=C(Me)$ | H |
| H | $MeON=C(CO_2Me)$ | H |
| H | Ph(HO-N=)C | H |
| H | $Ph(PhCH_2ON=)C$ | H |
| H | 1-Naphthyl | H |
| H | 2-Naphthyl | H |
| H | 1-Me-Pyrazol-5-yl | H |
| H | 1-Me-Pyrazol-4-yl | H |
| H | 1-Me-Pyrazol-3-yl | H |
| H | 1-Ph-Pyrazol-5-yl | H |
| H | 1-Ph-Pyrazol-4-yl | H |
| H | 1-Ph-Pyrazol-3-yl | H |
| H | 1-Me-4-F-Pyrazol-5-yl | H |
| H | 1-Me-4-F-Pyrazol-3-yl | H |
| H | 1-Me-3-F-Pyrazol-4-yl | H |
| H | 1-Me-3-F-Pyrazol-5-yl | H |
| H | 1-Me-5-F-Pyrazol-3-yl | H |
| H | 1-Me-5-F-Pyrazol-4-yl | H |
| H | 1-Me-4-Cl-Pyrazol-5-yl | H |
| H | 1-Me-4-Cl-Pyrazol-3-yl | H |
| H | 1-Me-3-Cl-Pyrazol-4-yl | H |
| H | 1-Me-3-Cl-Pyrazol-5-yl | H |
| H | 1-Me-5-Cl-Pyrazol-3-yl | H |
| H | 1-Me-5-Cl-Pyrazol-4-yl | H |
| H | 1-Me-3-Br-Pyrazol-4-yl | H |
| H | 1-Me-3-Ph-Pyrazol-4-yl | H |
| H | 1-Me-5-$NO_2$-Pyrazol-4-yl | H |
| H | 1-Me-3-$CF_3$-Pyrazol-4-yl | H |
| H | 1-Me-3-$F_2ClC$-Pyrazol-4-yl | H |
| H | 1-Me-3-$CF_3$-5-MeO-Pyrazol-4-yl | H |
| H | 1-Me-5-$CF_3$-Pyrazol-3-yl | H |
| H | 1-Me-4-MeOOC-Pyrazol-5-yl | H |
| H | 1-Me-4-MeOOC-Pyrazol-3-yl | H |
| H | 1-Me-5-MeOOC-Pyrazol-3-yl | H |
| H | 1-Me-3-Cl-4-MeOOC-Pyrazol-5-yl | H |
| H | 1-Me-3-Cl-4-EtOOC-Pyrazol-5-yl | H |
| H | 1-Me-4-EtOOC-Pyrazol-3-yl | H |
| H | 1,4-$Me_2$-Pyrazol-5-yl | H |
| H | 1,4-$Me_2$-Pyrazol-3-yl | H |
| H | 1,3-$Me_2$-Pyrazol-4-yl | H |
| H | 1,3-$Me_2$-Pyrazol-5-yl | H |
| H | 1,5-$Me_2$-Pyrazol-3-yl | H |
| H | 1,5-$Me_2$-Pyrazol-4-yl | H |
| H | 1,5-$Me_2$-4-Cl-Pyrazol-3-yl | H |
| H | 1,3-$Me_2$-5-Cl-Pyrazol-4-yl | H |
| H | 1,3-$Me_2$-5-F-Pyrazol-4-yl | H |
| H | 1,3-$Me_2$-5-MeO-Pyrazol-4-yl | H |
| H | 1,3,5-$Me_3$-Pyrazol-4-yl | H |

| | | |
|---|---|---|
| H | 1,3-$Me_2$-4-Cl-Pyrazol-5-yl | H |
| H | 1,3-$Me_2$-4-F-Pyrazol-5-yl | H |
| H | 1,3-$Me_2$-4-$NO_2$-Pyrazol-5-yl | H |
| H | 1,3-$Me_2$-4-MeO-Pyrazol-5-yl | H |
| H | 1,3,5-$Me_3$-Pyrazol-5-yl | H |
| H | 1-Me-3,5-$Cl_2$-Pyrazol-4-yl | H |
| H | 1-Me-3,5-$F_2$-Pyrazol-4-yl | H |
| H | 1-Ph-3,5-$Cl_2$-Pyrazol-4-yl | H |
| H | 1-Ph-3,5-$F_2$-Pyrazol-4-yl | H |
| H | 1-(2-Pyridyl)-3,5-$Cl_2$-Pyrazol-4-yl | H |
| H | 1-Ph-5-Me-Pyrazol-4-yl | H |
| H | 1-Ph-5-$CF_3$-Pyrazol-4-yl | H |
| H | 1-Ph-5-$ClF_2C$-Pyrazol-4-yl | H |
| H | 1-t-Bu-5-Me-Pyrazol-4-yl | H |
| H | 1-Me-3-Cl-5-MeS-Pyrazol-4-yl | H |
| H | 2-F-Furan-3-yl | H |
| H | Oxazol-2-yl | H |
| H | 2-MeS-Oxazol-4-yl | H |
| H | 1,2,4-Oxadiazol-3-yl | H |
| H | 1,2,4-Thiadizazol-5-yl | H |
| H | 1,2,4-Triazol-1-yl | H |
| H | 1,2,3-Triazol-1-yl | H |
| H | 1,2,3,4-Tetrazol-1-yl | H |
| H | 6-MeO-Pyrimidin-2-yl | H |
| H | Pyridazin-3-yl | H |
| H | 1,3,5-Triazin-2-yl | H |
| H | 1,2,4-Triazin-6-yl | H |
| H | 1-Me-Pyrrol-2-yl | H |
| H | 1-Me-Pyrrol-3-yl | H |
| H | 1-Me-4-$CF_3$-Pyrrol-5-yl | H |
| H | 3-CN-Pyrrol-1-yl | H |
| H | Furan-2-yl | H |
| H | Furan-3-yl | H |
| H | 5-Me-Furan-2-yl | H |
| H | 5-Ph-Furan-2-yl | H |
| H | 2,5-$Me_2$-Furan-3-yl | H |
| H | 2,4-$Me_2$-Furan-3-yl | H |
| H | Thiophen-2-yl | H |
| H | Thiophen-3-yl | H |
| H | 5-Ph-Thiophen-2-yl | H |
| H | 5-Me-Thiophen-2-yl | H |
| H | 5-Br-Thiophen-2-yl | H |
| H | 3-Br-Thiophen-2-yl | H |
| H | 4,5-$Br_2$-Thiophen-2-yl | H |
| H | 5-I-Thiophen-2-yl | H |
| H | 5-Cl-Thiophen-2-yl | H |
| H | 5-Ph-2-Me-Thiophen-3-yl | H |
| H | 5-$NO_2$-Thiophen-3-yl | H |
| H | 3-Me-Thiophen-2-yl | H |
| H | 3-Cl-Thiophen-2-yl | H |
| H | 3-MeO-Thiophen-2-yl | H |
| H | 3-F-Thiophen-2-yl | H |
| H | 2,5-$Cl_2$-Thiophen-3-yl | H |
| H | 2,5-$Me_2$-Thiophen-3-yl | H |
| H | 4,5-$Br_2$-Thiophen-3-yl | H |
| H | Thiazol-4-yl | H |
| H | Thiazol-5-yl | H |
| H | Thiazol-2-yl | H |

| | | |
|---|---|---|
| H | 2,4-$Me_2$-Thiazol-5-yl | H |
| H | 2-Br-4-Me-Thiazol-5-yl | H |
| H | 2-Cl-4-Me-Thiazol-5-yl | H |
| H | 2-Cl-4-Et-Thiazol-5-yl | H |
| H | 2-Cl-4-$CF_3$-Thiazol-5-yl | H |
| H | 2-Me-4-$CF_3$-Thiazol-5-yl | H |
| H | 2-Me-4-Et-Thiazol-5-yl | H |
| H | 2-Br-4-Et-Thiazol-5-yl | H |
| H | 2-Et-4-Me-Thiazol-5-yl | H |
| H | 2-MeO-4-Me-Thiazol-5-yl | H |
| H | 2-Cl-4-F-Thiazol-5-yl | H |
| H | 2-Ph-4-EtOOC-Thiazol-5-yl | H |
| H | 2-Cl-Thiazol-4-yl | H |
| H | 2-Me-Thiazol-4-yl | H |
| H | 5-$CF_3$-Thiazol-2-yl | H |
| H | 1-Ph-5-Me-Oxazol-4-yl | H |
| H | 2,4-$Me_2$-Oxazol-5-yl | H |
| H | 3-Me-Isothiazol-5-yl | H |
| H | 3-$PhCH_2O$-5-Me-Isothiazol-4-yl | H |
| H | 4-Cl-5-EtOOC-Isothiazol-3-yl | H |
| H | Isoxazol-5-yl | H |
| H | 3,5-Me-Isoxazol-4-yl | H |
| H | 5-Me-Isoxazol-3-yl | H |
| H | 3-Ph-5-Me-Isoxazol-4-yl | H |
| H | 4-CN-Isoxazol-3-yl | H |
| H | 1-Me-Imidazol-5-yl | H |
| H | 1-Me-2-Iminazolyl | H |
| H | 1-Me-4,5-$Cl_2$-Imidazol-2-yl | H |
| H | 1,5-$Me_2$-2-Cl-Imidazol-4-yl | H |
| H | 1-Ph-5-Me-1,2,3-Triazol-4-yl | H |
| H | 1-Ph-5-Et-1,2,3-Triazol-4-yl | H |
| H | 1-Ph-5-$CHBr_2$-1,2,3-Triazol-4-yl | H |
| H | 4-Me-1,2,3-Thiadiazol-5-yl | H |
| H | 4-Et-1,2,3-Thiadiazol-5-yl | H |
| H | 1,2,3-Thiadiazol-5-yl | H |
| H | 1,2,3-Thiadiazol-4-yl | H |
| H | Pyridin-2-yl | H |
| H | Pyridin-3-yl | H |
| H | Pyridin-4-yl | H |
| H | 6-Me-Pyridin-3-yl | H |
| H | 6-Cl-Pyridin-2-yl | H |
| H | 6-PhO-Pyridin-2-yl | H |
| H | 2-Cl-Pyridin-4-yl | H |
| H | 2-F-Pyridin-4-yl | H |
| H | 2,6-$Cl_2$-Pyridin-4-yl | H |
| H | 2-MeO-Pyridin-4-yl | H |
| H | 3,6-$Cl_2$-Pyridin-2-yl | H |
| H | 2-Cl-6-Me-Pyridin-4-yl | H |
| H | 3-F-Pyridin-2-yl | H |
| H | 3-F-Pyridin-4-yl | H |
| H | 5-$CF_3$-6-PhO-Pyridin-2-yl | H |
| H | 2,6-$Cl_2$-Pyridin-4-yl | H |
| H | 4,6-$Cl_2$-Pyridin-2-yl | H |
| H | 1-Me-4-$CF_3$-2-Pyridon-3-yl | H |
| H | Quinoxalin-2-yl | H |
| H | 6-Cl-Quinoxalin-2-yl | H |
| H | 6-F-Quinoxalin-2-yl | H |
| H | 6-MeO-Quinoxalin-2-yl | H |

| | | |
|---|---|---|
| H | 5-Cl-Quinoxalin-2-yl | H |
| H | 5-F-Quinoxalin-2-yl | H |
| H | 5-MeO-Quinoxalin-2-yl | H |
| H | 1-Me-Indol-3-yl | H |
| H | 1-Me-2-Cl-Indol-3-yl | H |
| H | 1-Me-2-F-Indol-3-yl | H |
| H | Benzothiazol-2-yl | H |
| H | 5-F-Benzothiazol-2-yl | H |
| H | 6-F-Benzothiazol-2-yl | H |
| H | Quinolin-4-yl | H |
| H | Pyrazin-2-yl | H |
| H | 3-Cl-Pyrazin-2-yl | H |
| H | 3-Me-Pyrazin-2-yl | H |
| H | 3-Et-Pyrazin-2-yl | H |
| H | 2-Ph-4-Me-Pyrimidin-5-yl | H |
| H | 2,4-$Me_2$-Pyrimidin-5-yl | H |
| H | 4-$CF_3$-Pyrimidin-5-yl | H |
| H | 4-$CClF_2$-Pyrimidin-5-yl | H |
| H | 4-$C_2F_5$-Pyrimidin-5-yl | H |
| H | 2-Me-4-$CBrF_2$-Pyrimidin-5-yl | H |
| H | 2-Me-4-$CClF_2$-Pyrimidin-5-yl | H |
| H | Pyrimidin-2-yl | H |
| H | Pyrimidin-4-yl | H |
| H | 6-MeS-Pyrimidin-5-yl | H |
| H | 6-PhO-Pyrimidin-4-yl | H |
| H | Benzofuran-2-yl | H |
| H | Ph | H |
| H | 2-Cl-Ph | H |
| H | 3-Cl-Ph | H |
| H | 4-Cl-Ph | H |
| H | 2-F-Ph | H |
| H | 3-F-Ph | H |
| H | 4-F-Ph | H |
| H | 2-Me-Ph | H |
| H | 3-Me-Ph | H |
| H | 4-Me-Ph | H |
| H | 2-MeO-Ph | H |
| H | 3-MeO-Ph | H |
| H | 4-MeO-Ph | H |
| H | 4-Br-Ph | H |
| H | 2,4-$Cl_2$-Ph | H |
| H | 3,4-$Cl_2$-Ph | H |
| H | 2,4,6-$Cl_3$-Ph | H |
| H | 3,4-$(MeO)_2$-Ph | H |
| H | 2-Cl-4-Me-Ph | H |
| H | 2-MeO-4-Me-Ph | H |
| H | 2-Cl-4-i-PrO-Ph | H |
| H | 3-Cl-4-$PhCH_2O$-Ph | H |
| H | 2,4-$Me_2$-Ph | H |
| H | 2,5-$Me_2$-Ph | H |
| H | 2,6-$F_2$-Ph | H |
| H | 2,3,4,5,6-$F_5$-Ph | H |
| H | 4-Et-Ph | H |
| H | 4-i-Pr-Ph | H |
| H | 4-n-Bu-Ph | H |
| H | 4-s-Bu-Ph | H |
| H | 4-t-Bu-Ph | H |
| H | 4-(t-$BuCH_2$)-Ph | H |

| | | |
|---|---|---|
| H | 4-Et(Me)$_2$C-Ph | H |
| H | 4-n-Hex-Ph | H |
| H | 4-((Me)$_2$(CN)C)-Ph | H |
| H | 4-PhCH$_2$-Ph | H |
| H | 4-(4-F-Ph)(Me)$_2$C-Ph | H |
| H | 4-(MeCH=CH)-Ph | H |
| H | 4-(MeC≡C)-Ph | H |
| H | 4-CF$_3$-Ph | H |
| H | 4-CF$_3$CH$_2$-Ph | H |
| H | 4-(Cl$_2$C=CHCH$_2$)-Ph | H |
| H | 4-(BrC≡C)-Ph | H |
| H | 4-(2,2-F$_2$-c-Bu)CH$_2$-Ph | H |
| H | 4-(1-Me-c-Pr)-Ph | H |
| H | 4-i-PrO-Ph | H |
| H | 4-t-BuO-Ph | H |
| H | 4-n-HexO-Ph | H |
| H | 4-(MeC≡C-O)-Ph | H |
| H | 4-(CH$_2$=CHCH$_2$O)-Ph | H |
| H | 4-CHF$_2$O-Ph | H |
| H | 4-CBrF$_2$O-Ph | H |
| H | 4-CF$_3$O-Ph | H |
| H | 4-CF$_3$CH$_2$O-Ph | H |
| H | 4-(CF$_2$=CHCH$_2$CH$_2$O)-Ph | H |
| H | 4-CCl$_3$CH$_2$O-Ph | H |
| H | 4-MeS-Ph | H |
| H | 4-s-BuS-Ph | H |
| H | 4-EtSO-Ph | H |
| H | 4-MeSO$_2$-Ph | H |
| H | 4-EtSO$_2$-Ph | H |
| H | 4-i-PrSO$_2$-Ph | H |
| H | 4-t-BuSO$_2$-Ph | H |
| H | 4-(MeCH=CHCH$_2$S)-Ph | H |
| H | 4-(CH$_2$=CHCH$_2$SO)-Ph | H |
| H | 4-(ClCH=CHCH$_2$SO$_2$)-Ph | H |
| H | 4-(HC≡CCH$_2$S)-Ph | H |
| H | 4-(HC≡CCH$_2$SO-Ph) | H |
| H | 4-(HC≡CCH$_2$SO$_2$)-Ph | H |
| H | 4-CHF$_2$S-Ph | H |
| H | 4-CBrF$_2$S-Ph | H |
| H | 4-CF$_3$S-Ph | H |
| H | 4-CF$_3$CH$_2$S-Ph | H |
| H | 4-CHF$_2$CF$_2$S-Ph | H |
| H | 4-CHF$_2$SO-Ph | H |
| H | 4-CBrF$_2$SO-Ph | H |
| H | 4-CF$_3$SO-Ph | H |
| H | 4-CF$_3$CH$_2$SO$_2$-Ph | H |
| H | 4-CHF$_2$CF$_2$SO$_2$-Ph | H |
| H | 4-CHF$_2$SO$_2$-Ph | H |
| H | 4-CBrF$_2$SO$_2$-Ph | H |
| H | 4-CF$_3$SO$_2$-Ph | H |
| H | 4-(Cl$_2$C=CHCH$_2$S)-Ph | H |
| H | 4-(Cl$_2$C=CHCH$_2$SO)-Ph | H |
| H | 4-(Cl$_2$C=CHCH$_2$SO$_2$)-Ph | H |
| H | 4-(BrC≡CCH$_2$S)-Ph | H |
| H | 4-(BrC≡CCH$_2$SO)-Ph | H |
| H | 4-(BrC≡CCH$_2$SO$_2$)-Ph | H |
| H | 4-CHO-Ph | H |
| H | 4-NO$_2$-Ph | H |

| | | |
|---|---|---|
| H | 3-CN-Ph | H |
| H | 4-CN-Ph | H |
| H | 4-$(Me)_2$N-Ph | H |
| H | 4-Me(MeC(O))N-Ph | H |
| H | 4-PhN(Me)-Ph | H |
| H | 4-$PhCH_2$(MeCO)N-Ph | H |
| H | 4-$PhCH_2$O-Ph | H |
| H | 4-(2-Cl-Ph)$CH_2$O-Ph | H |
| H | 4-(3-Cl-Ph)$CH_2$O-Ph | H |
| H | 4-(4-Cl-Ph)$CH_2$O-Ph | H |
| H | 4-(2-Me-Ph)$CH_2$O-Ph | H |
| H | 4-(3-Me-Ph)$CH_2$O-Ph | H |
| H | 4-(4-F-Ph)$CH_2$O-Ph | H |
| H | 4-(4-Et-Ph)$CH_2$O-Ph | H |
| H | 4-(2-Cl-Ph)$CH_2$S-Ph | H |
| H | 4-(3-Cl-Ph)$CH_2$S-Ph | H |
| H | 4-(4-Cl-Ph)$CH_2$SO-Ph | H |
| H | 4-(2-Me-Ph)$CH_2$S-Ph | H |
| H | 4-(3-Me-Ph)$CH_2SO_2$-Ph | H |
| H | 4-(2,4-$F_2$-Ph)$CH_2$O-Ph | H |
| H | 3-(3,4-$Cl_2$-Ph)$CH_2$O-Ph | H |
| H | 4-(2,5-$Me_2$-Ph)$CH_2$O-Ph | H |
| H | 4-(2,3,5,6-$F_4$-Ph)$CH_2$O-Ph | H |
| H | 4-MeC(O)-Ph | H |
| H | 4-EtC(O)-Ph | H |
| H | 4-n-PrC(O)-Ph | H |
| H | 4-i-PrC(O)-Ph | H |
| H | 4-i-BuC(O)-Ph | H |
| H | 4-t-BuC(O)-Ph | H |
| H | 4-i-$BuCH_2$C(O)-Ph | H |
| H | 4-Et$(Me)_2$CC(O)-Ph | H |
| H | 4-n-HexC(O)-Ph | H |
| H | 4-PhC(O)-Ph | H |
| H | 4-(2-Cl-Ph)C(O)-Ph | H |
| H | 4-(3-Br-Ph)C(O)-Ph | H |
| H | 4-(4-Cl-Ph)C(O)-Ph | H |
| H | 4-(2-Me-Ph)C(O)-Ph | H |
| H | 4-$MeOCH_2$-Ph | H |
| H | 4-$EtOCH_2$-Ph | H |
| H | 4-i-$PrOCH_2$-Ph | H |
| H | 4-$MeSCH_2$-Ph | H |
| H | 4-$EtSCH_2$-Ph | H |
| H | 4-i-$PrSCH_2$-Ph | H |
| H | 4-$CF_3$C(O)-Ph | H |
| H | 4-$CF_3CF_2$C(O)-Ph | H |
| H | 4-MeC(O)O-Ph | H |
| H | 4-EtC(O)O-Ph | H |
| H | 4-n-PrC(O)O-Ph | H |
| H | 4-i-PrC(O)O-Ph | H |
| H | 4-i-BuC(O)O-Ph | H |
| H | 4-t-BuC(O)O-Ph | H |
| H | 4-i-$BuCH_2$C(O)O-Ph | H |
| H | 4-Et$(Me)_2$C(O)O-Ph | H |
| H | 4-n-HexC(O)O-Ph | H |
| H | 4-$CF_3$C(O)O-Ph | H |
| H | 4-$CF_3CF_2$C(O)O-Ph | H |
| H | 4-PhC(O)O-Ph | H |
| H | 3-Ph-Ph | H |

| | | |
|---|---|---|
| H | 4-Ph-Ph | H |
| H | 4-(4-Cl-Ph)-Ph | H |
| H | 4-(2,5-$Me_2$-Ph)-3-Me-Ph | H |
| H | 3-PhO-Ph | H |
| H | 4-PhO-Ph | H |
| H | 4-(4-Cl-Ph)O-Ph | H |
| H | 4-(4-Me-Ph)O-Ph | H |
| H | 4-(4-F-Ph)O-Ph | H |
| H | 4-(4-MeO-Ph)O-Ph | H |
| H | 4-(2,4-$Cl_2$-Ph)O-Ph | H |
| H | 4-(3,4-$Cl_2$-Ph)O-Ph | H |
| H | 4-(Pyridin-2-yl)-Ph | H |
| H | 4-(5-Cl-Pyridin-2-yl)-Ph | H |
| H | 4-(6-F-5-$CF_3$-Pyridin-2-yl)-Ph | H |
| H | 4-(Pyridin-2-yl)O-Ph | H |
| H | 4-(5-Cl-Pyridin-2-yl)O-Ph | H |
| H | 4-(3-Cl-5-F-Pyridin-2-yl)O-Ph | H |
| H | 4-(5-Cl-Thiophen-2-yl)O-Ph | H |
| H | 2,3-$Cl_2$-Ph | H |
| H | 3,5-$Cl_2$-Ph | H |
| H | 2,6-$Cl_2$-Ph | H |
| H | 2,5-$Cl_2$-Ph | H |
| H | 2,3-$F_2$-Ph | H |
| H | 2,5-$F_2$-Ph | H |
| H | 3,4-$F_2$-Ph | H |
| H | 3,5-$F_2$-Ph | H |
| H | 2,4-$F_2$-Ph | H |
| H | 2-$CF_3$-Ph | H |
| H | 3-(3-Cl-Ph$CH_2$O)-Ph | H |
| H | 2-F-6-$CF_3$-Ph | H |
| H | 2-F-6-Cl-Ph | H |
| H | 2-F-6-Me-Ph | H |
| H | 2-F-6-MeO-Ph | H |
| H | 2-F-6-OH-Ph | H |
| H | 2-F-6-MeS-Ph | H |
| H | 2-F-5-Cl-Ph | H |
| H | 2-F-5-$CF_3$-Ph | H |
| H | 2-F-5-Me-Ph | H |
| H | 2-F-5-MeO-Ph | H |
| H | 2-F-5-OH-Ph | H |
| H | 2-F-5-MeS-Ph | H |
| H | 2-F-4-Cl-Ph | H |
| H | 2-F-4-$CF_3$-Ph | H |
| H | 2-F-4-Me-Ph | H |
| H | 2-F-4-MeO-Ph | H |
| H | 2-F-3-Cl-Ph | H |
| H | 2-F-3-Me-Ph | H |
| H | 2-F-3-MeO-Ph | H |
| H | 3-F-2-Cl-Ph | H |
| H | 3-F-2-Me-Ph | H |
| H | 3-F-2-MeO-Ph | H |
| H | 3-F-4-Cl-Ph | H |
| H | 3-F-4-Me-Ph | H |
| H | 3-F-4-MeO-Ph | H |
| H | 3-F-5-Cl-Ph | H |
| H | 3-F-5-Me-Ph | H |
| H | 3-F-5-MeO-Ph | H |
| H | 3-F-6-Cl-Ph | H |

| | | |
|---|---|---|
| H | 3-F-6-Me-Ph | H |
| H | 3-F-6-MeO-Ph | H |
| H | 4-F-2-Cl-Ph | H |
| H | 4-F-2-Me-Ph | H |
| H | 4-F-2-MeO-Ph | H |
| H | 4-F-3-Cl-Ph | H |
| H | 4-F-3-Me-Ph | H |
| H | 4-F-3-MeO-Ph | H |
| H | 2,4,6-$F_3$-Ph | H |
| H | 2-OH-Ph | H |
| H | 4-I-Ph | H |
| H | 4-MeOC(O)-Ph | H |
| H | 4-MeNHC(O)-Ph | H |
| H | 2,6-$Me_2$-Ph | H |
| H | 2,6-$(MeO)_2$-Ph | H |
| H | 3-$CF_3$-Ph | H |
| H | 2-Br-Ph | H |
| H | 3-Br-Ph | H |
| H | 2-MeC(O)-Ph | H |
| H | 2-I-Ph | H |
| H | 3-I-Ph | H |
| H | 4-c-Pr-Ph | H |
| H | 4-(2-Cl-c-Pr)-Ph | H |
| H | 4-(2,2-$Cl_2$-c-Pr)-Ph | H |
| H | 4-Ph-CH=CH-Ph | H |
| H | 4-(Ph-C≡C)-Ph | H |
| H | 4-PhS-Ph | H |
| H | 4-HO-Ph | H |
| H | 4-EtO-Ph | H |
| H | 4-PenO-Ph | H |
| H | 2-F-3-$CF_3$-Ph | H |
| H | 2,3-$Me_2$-Ph | H |
| H | 3,4-$Me_2$-Ph | H |
| H | 3,5-$Me_2$-Ph | H |
| H | 2,3-$(MeO)_2$-Ph | H |
| H | 2,4-$(MeO)_2$-Ph | H |
| H | 2,5-$(MeO)_2$-Ph | H |
| H | 3,5-$(MeO)_2$-Ph | H |
| H | 2-F-3-I-Ph | H |
| H | 2-F-4-I-Ph | H |
| H | 2-F-5-I-Ph | H |
| H | 2-F-6-I-Ph | H |
| H | 2-F-4-EtO-Ph | H |
| H | 2-F-4-PrO-Ph | H |
| H | 2-F-4-i-PrO-Ph | H |
| H | 2-F-4-BuO-Ph | H |
| H | 2-F-4-s-BuO-Ph | H |
| H | 2-F-4-i-BuO-Ph | H |
| H | 2-F-4-t-BuO-Ph | H |
| H | 2-F-4-PenO-Ph | H |
| H | 2-F-4-(2-Me-BuO)-Ph | H |
| H | 2-F-4-(2,2-$Me_2$-PrO)-Ph | H |
| H | 2-F-4-HexO-Ph | H |
| H | 2-F-4-(2-Et-Hex)O-Ph | H |
| H | 2-F-4-Et-Ph | H |
| H | 2-F-4-Pr-Ph | H |
| H | 2-F-4-i-Pr-Ph | H |
| H | 2-F-4-Bu-Ph | H |

| | | |
|---|---|---|
| H | 2-F-4-s-Bu-Ph | H |
| H | 2-F-4-i-Bu-Ph | H |
| H | 2-F-4-t-Bu-Ph | H |
| H | 2-F-4-Pen-Ph | H |
| H | 2-F-4-(2-Me-Bu)-Ph | H |
| H | 2-F-4-(2,2-$Me_2$-Pr)-Ph | H |
| H | 2-F-4-Hex-Ph | H |
| H | 2-F-4-(2-Et-Hex)-Ph | H |
| H | 2-F-6-PhS-Ph | H |
| H | 2-F-6-$Me_2$N-Ph | H |
| H | 2-F-6-MeNH-Ph | H |
| H | 2-F-6-Ph-Ph | H |
| H | 3,4-methylenedioxy-Ph | H |
| H | 3,4-ethylenedioxy-Ph | H |
| H | 2-F-3-Br-Ph | H |
| H | 2-F-4-Br-Ph | H |
| H | 2-F-5-Br-Ph | H |
| H | 2-F-6-Br-Ph | H |
| H | 3-F-2-Br-Ph | H |
| H | 3-F-4-Br-Ph | H |
| H | 3-F-5-Br-Ph | H |
| H | 3-F-6-Br-Ph | H |
| H | 4-F-2-Br-Ph | H |
| H | 4-F-3-Br-Ph | H |
| H | 2-Cl-3-Me-Ph | H |
| H | 2-Cl-4-Me-Ph | H |
| H | 2-Cl-5-Me-Ph | H |
| H | 2-Cl-6-Me-Ph | H |
| H | 3-Cl-2-Me-Ph | H |
| H | 3-Cl-4-Me-Ph | H |
| H | 3-Cl-5-Me-Ph | H |
| H | 3-Cl-6-Me-Ph | H |
| H | 4-Cl-2-Me-Ph | H |
| H | 4-Cl-3-Me-Ph | H |
| H | 2,3-$F_2$-4-Me-Ph | H |
| H | 2,3-$F_2$-5-Me-Ph | H |
| H | 2,3-$F_2$-6-Me-Ph | H |
| H | 2,4-$F_2$-3-Me-Ph | H |
| H | 2,4-$F_2$-5-Me-Ph | H |
| H | 2,4-$F_2$-6-Me-Ph | H |
| H | 2,5-$F_2$-3-Me-Ph | H |
| H | 2,5-$F_2$-4-Me-Ph | H |
| H | 2,5-$F_2$-6-Me-Ph | H |
| H | 2,6-$F_2$-3-Me-Ph | H |
| H | 2,6-$F_2$-4-Me-Ph | H |
| H | 2,3-$F_2$-4-Cl-Ph | H |
| H | 2,3-$F_2$-5-Cl-Ph | H |
| H | 2,3-$F_2$-6-Cl-Ph | H |
| H | 2,4-$F_2$-3-Cl-Ph | H |
| H | 2,4-$F_2$-5-Cl-Ph | H |
| H | 2,4-$F_2$-6-Cl-Ph | H |
| H | 2,5-$F_2$-3-Cl-Ph | H |
| H | 2,5-$F_2$-4-Cl-Ph | H |
| H | 2,5-$F_2$-6-Cl-Ph | H |
| H | 2,6-$F_2$-3-Cl-Ph | H |
| H | 2,6-$F_2$-4-Cl-Ph | H |
| H | 2,3-$F_2$-4-MeO-Ph | H |
| H | 2,3-$F_2$-5-MeO-Ph | H |

| | | |
|---|---|---|
| H | 2,3-$F_2$-6-MeO-Ph | H |
| H | 2,4-$F_2$-3-MeO-Ph | H |
| H | 2,4-$F_2$-5-MeO-Ph | H |
| H | 2,4-$F_2$-6-MeO-Ph | H |
| H | 2,5-$F_2$-3-MeO-Ph | H |
| H | 2,5-$F_2$-4-MeO-Ph | H |
| H | 2,5-$F_2$-6-MeO-Ph | H |
| H | 2,6-$F_2$-3-MeO-Ph | H |
| H | 2,6-$F_2$-4-MeO-Ph | H |
| H | 2,3-$F_2$-4-EtO-Ph | H |
| H | 2,3-$F_2$-5-EtO-Ph | H |
| H | 2,3-$F_2$-6-EtO-Ph | H |
| H | 2,4-$F_2$-3-EtO-Ph | H |
| H | 2,4-$F_2$-5-EtO-Ph | H |
| H | 2,4-$F_2$-6-EtO-Ph | H |
| H | 2,5-$F_2$-3-EtO-Ph | H |
| H | 2,5-$F_2$-4-EtO-Ph | H |
| H | 2,5-$F_2$-6-EtO-Ph | H |
| H | 2,6-$F_2$-3-EtO-Ph | H |
| H | 2,6-$F_2$-4-EtO-Ph | H |
| H | 2,3-$F_2$-4-Et-Ph | H |
| H | 2,3-$F_2$-5-Et-Ph | H |
| H | 2,3-$F_2$-6-Et-Ph | H |
| H | 2,4-$F_2$-3-Et-Ph | H |
| H | 2,4-$F_2$-5-Et-Ph | H |
| H | 2,4-$F_2$-6-Et-Ph | H |
| H | 2,5-$F_2$-3-Et-Ph | H |
| H | 2,5-$F_2$-4-Et-Ph | H |
| H | 2,5-$F_2$-6-Et-Ph | H |
| H | 2,6-$F_2$-3-Et-Ph | H |
| H | 2,6-$F_2$-4-Et-Ph | H |
| H | 2,3-$F_2$-4-Br-Ph | H |
| H | 2,3-$F_2$-5-Br-Ph | H |
| H | 2,3-$F_2$-6-Br-Ph | H |
| H | 2,4-$F_2$-3-Br-Ph | H |
| H | 2,4-$F_2$-5-Br-Ph | H |
| H | 2,4-$F_2$-6-Br-Ph | H |
| H | 2,5-$F_2$-3-Br-Ph | H |
| H | 2,5-$F_2$-4-Br-Ph | H |
| H | 2,5-$F_2$-6-Br-Ph | H |
| H | 2,6-$F_2$-3-Br-Ph | H |
| H | 2,6-$F_2$-4-Br-Ph | H |
| H | 2,6-$F_2$-4-Pr-Ph | H |
| H | 2,6-$F_2$-4-i-Pr-Ph | H |
| H | 2,6-$F_2$-4-c-Pr-Ph | H |
| H | 2,6-$F_2$-4-Bu-Ph | H |
| H | 2,6-$F_2$-4-i-Bu-Ph | H |
| H | 2,6-$F_2$-4-s-Bu-Ph | H |
| H | 2,6-$F_2$-4-t-Bu-Ph | H |
| H | 2,6-$F_2$-4-Pen-Ph | H |
| H | 2,6-$F_2$-4-Hex-Ph | H |
| H | 2,6-$F_2$-4-Ph-Ph | H |
| H | 2,6-$F_2$-4-PhC$H_2$-Ph | H |
| H | 2,6-$F_2$-4-PrO-Ph | H |
| H | 2,6-$F_2$-4-i-PrO-Ph | H |
| H | 2,6-$F_2$-4-c-PrO-Ph | H |
| H | 2,6-$F_2$-4-BuO-Ph | H |
| H | 2,6-$F_2$-4-i-BuO-Ph | H |

| | | |
|---|---|---|
| H | 2,6-$F_2$-4-s-BuO-Ph | H |
| H | 2,6-$F_2$-4-t-BuO-Ph | H |
| H | 2,6-$F_2$-4-PenO-Ph | H |
| H | 2,6-$F_2$-4-HexO-Ph | H |
| H | 2,6-$F_2$-4-PhO-Ph | H |
| H | 2,6-$F_2$-4-PhCH$_2$O-Ph | H |
| H | 2-F-6-Cl-3-MeO-Ph | H |
| H | 2-F-6-Cl-4-MeO-Ph | H |
| H | 2-F-6-Cl-5-MeO-Ph | H |
| H | 2-F-6-Cl-3-Me-Ph | H |
| H | 2-F-6-Cl-4-Me-Ph | H |
| H | 2-F-6-Cl-5-Me-Ph | H |
| H | 2-F-6-MeO-3-Cl-Ph | H |
| H | 2-F-6-MeO-4-Cl-Ph | H |
| H | 2-F-6-MeO-5-Cl-Ph | H |
| H | 2-F-6-MeO-3-Me-Ph | H |
| H | 2-F-6-MeO-4-Me-Ph | H |
| H | 2-F-6-MeO-5-Me-Ph | H |
| H | 2,4,6-Me$_3$-Ph | H |
| H | 4-HepO-Ph | H |
| H | 4-OctO-Ph | H |
| H | 4-NonO-Ph | H |
| H | 4-DecO-Ph | H |
| H | 4-UndecO-Ph | H |
| H | 4-DodecO-Ph | H |
| H | 4-Hep-Ph | H |
| H | 4-Oct-Ph | H |
| H | 4-Non-Ph | H |
| H | 4-Dec-Ph | H |
| H | 4-Undec-Ph | H |
| H | 4-Dodec-Ph | H |
| H | 2-Cl-4-HepO-Ph | H |
| H | 2-Cl-4-OctO-Ph | H |
| H | 2-Cl-4-NonO-Ph | H |
| H | 2-Cl-4-DecO-Ph | H |
| H | 2-Cl-4-UndecO-Ph | H |
| H | 2-Cl-4-DodecO-Ph | H |
| H | 2-Cl-4-Hep-Ph | H |
| H | 2-Cl-4-Oct-Ph | H |
| H | 2-Cl-4-Non-Ph | H |
| H | 2-Cl-4-Dec-Ph | H |
| H | 2-Cl-4-Undec-Ph | H |
| H | 2-Cl-4-Dodec-Ph | H |
| H | 3-Cl-4-HepO-Ph | H |
| H | 3-Cl-4-OctO-Ph | H |
| H | 3-Cl-4-NonO-Ph | H |
| H | 3-Cl-4-DecO-Ph | H |
| H | 3-Cl-4-UndecO-Ph | H |
| H | 3-Cl-4-DodecO-Ph | H |
| H | 3-Cl-4-Hep-Ph | H |
| H | 3-Cl-4-Oct-Ph | H |
| H | 3-Cl-4-Non-Ph | H |
| H | 3-Cl-4-Dec-Ph | H |
| H | 3-Cl-4-Undec-Ph | H |
| H | 3-Cl-4-Dodec-Ph | H |
| H | 2-F-4-HepO-Ph | H |
| H | 2-F-4-OctO-Ph | H |
| H | 2-F-4-NonO-Ph | H |

| | | |
|---|---|---|
| H | 2-F-4-DecO-Ph | H |
| H | 2-F-4-UndecO-Ph | H |
| H | 2-F-4-DodecO-Ph | H |
| H | 2-F-4-Hep-Ph | H |
| H | 2-F-4-Oct-Ph | H |
| H | 2-F-4-Non-Ph | H |
| H | 2-F-4-Dec-Ph | H |
| H | 2-F-4-Undec-Ph | H |
| H | 2-F-4-Dodec-Ph | H |
| H | 3-F-4-HepO-Ph | H |
| H | 3-F-4-OctO-Ph | H |
| H | 3-F-4-NonO-Ph | H |
| H | 3-F-4-DecO-Ph | H |
| H | 3-F-4-UndecO-Ph | H |
| H | 3-F-4-DodecO-Ph | H |
| H | 3-F-4-Hep-Ph | H |
| H | 3-F-4-Oct-Ph | H |
| H | 3-F-4-Non-Ph | H |
| H | 3-F-4-Dec-Ph | H |
| H | 3-F-4-Undec-Ph | H |
| H | 3-F-4-Dodec-Ph | H |
| H | 2-Cl-3-OMe-Ph | H |
| H | 2-Cl-4-OMe-Ph | H |
| H | 2-Cl-5-OMe-Ph | H |
| H | 2-Cl-6-OMe-Ph | H |
| H | 3-Cl-2-OMe-Ph | H |
| H | 3-Cl-4-OMe-Ph | H |
| H | 3-Cl-5-OMe-Ph | H |
| H | 3-Cl-6-OMe-Ph | H |
| H | 4-Cl-2-OMe-Ph | H |
| H | 4-Cl-3-OMe-Ph | H |
| H | 2-Me-3-OMe-Ph | H |
| H | 2-Me-4-OMe-Ph | H |
| H | 2-Me-5-OMe-Ph | H |
| H | 2-Me-6-OMe-Ph | H |
| H | 3-Me-2-OMe-Ph | H |
| H | 3-Me-4-OMe-Ph | H |
| H | 3-Me-5-OMe-Ph | H |
| H | 3-Me-6-OMe-Ph | H |
| H | 4-Me-3-OMe-Ph | H |
| H | 2-$NO_2$-Ph | H |
| H | 2,5-$F_2$-4-Cl-Ph | H |
| H | 2-($PhCH_2O$)Ph | H |
| H | 3-Br-4-$Me_2N$-Ph | H |
| H | 2-OH-3,4-$Cl_2$-Ph | H |
| H | 3-Cl-4-$Me_2N$-Ph | H |
| H | 3-Br-4-$Et_2N$-Ph | H |
| H | 3-Cl-4-(Ph)MeN-Ph | H |
| H | Ph | 3-Cl |
| H | Ph | 4-Cl |
| H | Ph | 5-Cl |
| H | Ph | 6-Cl |
| H | Ph | 3-$CF_3$ |
| H | Ph | 4-$CF_3$ |
| H | Ph | 5-$CF_3$ |
| H | Ph | 3-Me |
| H | Ph | 4-Me |
| H | Ph | 5-Me |

| | | |
|---|---|---|
| H | Ph | 6-Me |
| H | Ph | 3-F |
| H | Ph | 4-F |
| H | Ph | 5-F |
| H | Ph | 6-F |
| H | Ph | 3-MeO |
| H | Ph | 4-MeO |
| H | Ph | 5-MeO |
| H | Ph | 6-MeO |
| H | Ph | $3,4-Me_2$ |
| H | Ph | $3,5-Me_2$ |
| H | Ph | $3,6-Me_2$ |
| H | Ph | $4,5-Me_2$ |
| H | Ph | $4,6-Me_2$ |
| H | Ph | $5,6-Me_2$ |
| H | Ph | $3,4-(MeO)_2$ |
| H | Ph | $3,5-(MeO)_2$ |
| H | Ph | $3,6-(MeO)_2$ |
| H | Ph | $4,5-(MeO)_2$ |
| H | Ph | $4,6-(MeO)_2$ |
| H | Ph | $5,6-(MeO)_2$ |
| H | Ph | $3,4-Cl_2$ |
| H | Ph | $3,5-Cl_2$ |
| H | Ph | $3,6-Cl_2$ |
| H | Ph | $4,5-Cl_2$ |
| H | Ph | $4,6-Cl_2$ |
| H | Ph | $5,6-Cl_2$ |
| H | Ph | $3,4-F_2$ |
| H | Ph | $3,5-F_2$ |
| H | Ph | $3,6-F_2$ |
| H | Ph | $4,5-F_2$ |
| H | Ph | $4,6-F_2$ |
| H | Ph | $5,6-F_2$ |
| H | Ph | 4-t-Bu |
| H | Ph | 4-CN |
| H | Ph | 4-Et |
| H | Ph | 4-COOMe |
| H | Ph | 4-COOEt |
| H | Ph | 4-COMe |
| H | Ph | 4-COPh |
| H | Ph | 4-F-5-Cl |
| H | Ph | 4-F-5-Me |
| H | Ph | 4-Me-5-Cl |
| H | Ph | 5-Cl-6-Me |
| H | 2-Cl-Ph | 3-Cl |
| H | 2-Cl-Ph | 4-Cl |
| H | 2-Cl-Ph | 5-Cl |
| H | 2-Cl-Ph | 6-Cl |
| H | 2-Cl-Ph | $3-CF_3$ |
| H | 2-Cl-Ph | $4-CF_3$ |
| H | 2-Cl-Ph | $5-CF_3$ |
| H | 2-Cl-Ph | 3-Me |
| H | 2-Cl-Ph | 4-Me |
| H | 2-Cl-Ph | 5-Me |
| H | 2-Cl-Ph | 6-Me |
| H | 2-Cl-Ph | 3-F |
| H | 2-Cl-Ph | 4-F |
| H | 2-Cl-Ph | 5-F |

| | | |
|---|---|---|
| H | 2-Cl-Ph | 6-F |
| H | 2-Cl-Ph | 3-MeO |
| H | 2-Cl-Ph | 4-MeO |
| H | 2-Cl-Ph | 5-MeO |
| H | 2-Cl-Ph | 6-MeO |
| H | 2-Cl-Ph | 4,5-$Me_2$ |
| H | 2-Cl-Ph | 4,5-$(MeO)_2$ |
| H | 2-Cl-Ph | 4,5-$Cl_2$ |
| H | 2-Cl-Ph | 5,6-$Cl_2$ |
| H | 2-Cl-Ph | 3,5-$F_2$ |
| H | 2-Cl-Ph | 4,5-$F_2$ |
| H | 2-Cl-Ph | 5,6-$F_2$ |
| H | 2-Cl-Ph | 4-t-Bu |
| H | 2-Cl-Ph | 4-CN |
| H | 2-Cl-Ph | 4-Et |
| H | 2-Cl-Ph | 4-COOMe |
| H | 2-Cl-Ph | 4-COMe |
| H | 2-Cl-Ph | 4-COPh |
| H | 2-Cl-Ph | 4-F-5-Cl |
| H | 2-Cl-Ph | 4-F-5-Me |
| H | 2-Cl-Ph | 4-Me-5-Cl |
| H | 2-Cl-Ph | 5-Cl-6-Me |
| H | 3-Cl-Ph | 4-Cl |
| H | 3-Cl-Ph | 5-Cl |
| H | 3-Cl-Ph | 4-$CF_3$ |
| H | 3-Cl-Ph | 5-$CF_3$ |
| H | 3-Cl-Ph | 4-Me |
| H | 3-Cl-Ph | 5-Me |
| H | 3-Cl-Ph | 4-F |
| H | 3-Cl-Ph | 5-F |
| H | 3-Cl-Ph | 4-MeO |
| H | 3-Cl-Ph | 5-MeO |
| H | 4-Cl-Ph | 3-Cl |
| H | 4-Cl-Ph | 4-Cl |
| H | 4-Cl-Ph | 5-Cl |
| H | 4-Cl-Ph | 6-Cl |
| H | 4-Cl-Ph | 3-$CF_3$ |
| H | 4-Cl-Ph | 4-$CF_3$ |
| H | 4-Cl-Ph | 5-$CF_3$ |
| H | 4-Cl-Ph | 3-Me |
| H | 4-Cl-Ph | 4-Me |
| H | 4-Cl-Ph | 5-Me |
| H | 4-Cl-Ph | 6-Me |
| H | 4-Cl-Ph | 3-F |
| H | 4-Cl-Ph | 4-F |
| H | 4-Cl-Ph | 5-F |
| H | 4-Cl-Ph | 6-F |
| H | 4-Cl-Ph | 3-MeO |
| H | 4-Cl-Ph | 4-MeO |
| H | 4-Cl-Ph | 5-MeO |
| H | 4-Cl-Ph | 6-MeO |
| H | 4-Cl-Ph | 4,5-$Me_2$ |
| H | 4-Cl-Ph | 4,5-$(MeO)_2$ |
| H | 4-Cl-Ph | 4,5-$Cl_2$ |
| H | 4-Cl-Ph | 5,6-$Cl_2$ |
| H | 4-Cl-Ph | 3,5-$F_2$ |
| H | 4-Cl-Ph | 4,5-$F_2$ |
| H | 4-Cl-Ph | 5,6-$F_2$ |

| | | |
|---|---|---|
| H | 4-Cl-Ph | 4-t-Bu |
| H | 4-Cl-Ph | 4-CN |
| H | 4-Cl-Ph | 4-Et |
| H | 4-Cl-Ph | 4-COOMe |
| H | 4-Cl-Ph | 4-COMe |
| H | 4-Cl-Ph | 4-COPh |
| H | 4-Cl-Ph | 4-F-5-Cl |
| H | 4-Cl-Ph | 4-F-5-Me |
| H | 4-Cl-Ph | 4-Me-5-Cl |
| H | 4-Cl-Ph | 5-Cl-6-Me |
| H | 2-Me-Ph | 4-Cl |
| H | 2-Me-Ph | 5-Cl |
| H | 2-Me-Ph | 4-$CF_3$ |
| H | 2-Me-Ph | 5-$CF_3$ |
| H | 2-Me-Ph | 4-Me |
| H | 2-Me-Ph | 5-Me |
| H | 2-Me-Ph | 4-F |
| H | 2-Me-Ph | 5-F |
| H | 2-Me-Ph | 4-MeO |
| H | 2-Me-Ph | 5-MeO |
| H | 3-Me-Ph | 4-Cl |
| H | 3-Me-Ph | 5-Cl |
| H | 3-Me-Ph | 4-$CF_3$ |
| H | 3-Me-Ph | 5-$CF_3$ |
| H | 3-Me-Ph | 4-Me |
| H | 3-Me-Ph | 5-Me |
| H | 3-Me-Ph | 4-F |
| H | 3-Me-Ph | 5-F |
| H | 3-Me-Ph | 4-MeO |
| H | 3-Me-Ph | 5-MeO |
| H | 4-Me-Ph | 3-Cl |
| H | 4-Me-Ph | 4-Cl |
| H | 4-Me-Ph | 5-Cl |
| H | 4-Me-Ph | 6-Cl |
| H | 4-Me-Ph | 3-$CF_3$ |
| H | 4-Me-Ph | 4-$CF_3$ |
| H | 4-Me-Ph | 5-$CF_3$ |
| H | 4-Me-Ph | 3-Me |
| H | 4-Me-Ph | 4-Me |
| H | 4-Me-Ph | 5-Me |
| H | 4-Me-Ph | 6-Me |
| H | 4-Me-Ph | 3-F |
| H | 4-Me-Ph | 4-F |
| H | 4-Me-Ph | 5-F |
| H | 4-Me-Ph | 6-F |
| H | 4-Me-Ph | 3-MeO |
| H | 4-Me-Ph | 4-MeO |
| H | 4-Me-Ph | 5-MeO |
| H | 4-Me-Ph | 6-MeO |
| H | 4-Me-Ph | 4,5-$Me_2$ |
| H | 4-Me-Ph | 4,5-$(MeO)_2$ |
| H | 4-Me-Ph | 4,5-$Cl_2$ |
| H | 4-Me-Ph | 5,6-$Cl_2$ |
| H | 4-Me-Ph | 3,5-$F_2$ |
| H | 4-Me-Ph | 4,5-$F_2$ |
| H | 4-Me-Ph | 5,6-$F_2$ |
| H | 4-Me-Ph | 4-t-Bu |
| H | 4-Me-Ph | 4-CN |

| | | |
|---|---|---|
| H | 4-Me-Ph | 4-Et |
| H | 4-Me-Ph | 4-COOMe |
| H | 4-Me-Ph | 4-COMe |
| H | 4-Me-Ph | 4-COPh |
| H | 4-Me-Ph | 4-F-5-Cl |
| H | 4-Me-Ph | 4-F-5-Me |
| H | 4-Me-Ph | 4-Me-5-Cl |
| H | 4-Me-Ph | 5-Cl-6-Me |
| H | 2-F-Ph | 3-Cl |
| H | 2-F-Ph | 4-Cl |
| H | 2-F-Ph | 5-Cl |
| H | 2-F-Ph | 6-Cl |
| H | 2-F-Ph | 3-$CF_3$ |
| H | 2-F-Ph | 4-$CF_3$ |
| H | 2-F-Ph | 5-$CF_3$ |
| H | 2-F-Ph | 3-Me |
| H | 2-F-Ph | 4-Me |
| H | 2-F-Ph | 5-Me |
| H | 2-F-Ph | 6-Me |
| H | 2-F-Ph | 3-F |
| H | 2-F-Ph | 4-F |
| H | 2-F-Ph | 5-F |
| H | 2-F-Ph | 6-F |
| H | 2-F-Ph | 3-MeO |
| H | 2-F-Ph | 4-MeO |
| H | 2-F-Ph | 5-MeO |
| H | 2-F-Ph | 6-MeO |
| H | 2-F-Ph | 4,5-$Me_2$ |
| H | 2-F-Ph | 4,5-$(MeO)_2$ |
| H | 2-F-Ph | 4,5-$Cl_2$ |
| H | 2-F-Ph | 5,6-$Cl_2$ |
| H | 2-F-Ph | 3,5-$F_2$ |
| H | 2-F-Ph | 4,5-$F_2$ |
| H | 2-F-Ph | 5,6-$F_2$ |
| H | 2-F-Ph | 4-t-Bu |
| H | 2-F-Ph | 4-CN |
| H | 2-F-Ph | 4-Et |
| H | 2-F-Ph | 4-COOMe |
| H | 2-F-Ph | 4-COMe |
| H | 2-F-Ph | 4-COPh |
| H | 2-F-Ph | 4-F-5-Cl |
| H | 2-F-Ph | 4-F-5-Me |
| H | 2-F-Ph | 4-Me-5-Cl |
| H | 2-F-Ph | 5-Cl-6-Me |
| H | 3-F-Ph | 4-Cl |
| H | 3-F-Ph | 5-Cl |
| H | 3-F-Ph | 4-$CF_3$ |
| H | 3-F-Ph | 5-$CF_3$ |
| H | 3-F-Ph | 4-Me |
| H | 3-F-Ph | 5-Me |
| H | 3-F-Ph | 4-F |
| H | 3-F-Ph | 5-F |
| H | 3-F-Ph | 4-MeO |
| H | 3-F-Ph | 5-MeO |
| H | 4-F-Ph | 3-Cl |
| H | 4-F-Ph | 4-Cl |
| H | 4-F-Ph | 5-Cl |
| H | 4-F-Ph | 6-Cl |

| | | |
|---|---|---|
| H | 4-F-Ph | 3-$CF_3$ |
| H | 4-F-Ph | 4-$CF_3$ |
| H | 4-F-Ph | 5-$CF_3$ |
| H | 4-F-Ph | 3-Me |
| H | 4-F-Ph | 4-Me |
| H | 4-F-Ph | 5-Me |
| H | 4-F-Ph | 6-Me |
| H | 4-F-Ph | 3-F |
| H | 4-F-Ph | 4-F |
| H | 4-F-Ph | 5-F |
| H | 4-F-Ph | 6-F |
| H | 4-F-Ph | 3-MeO |
| H | 4-F-Ph | 4-MeO |
| H | 4-F-Ph | 5-MeO |
| H | 4-F-Ph | 6-MeO |
| H | 4-F-Ph | 4,5-$Me_2$ |
| H | 4-F-Ph | 4,5-$(MeO)_2$ |
| H | 4-F-Ph | 4,5-$Cl_2$ |
| H | 4-F-Ph | 5,6-$Cl_2$ |
| H | 4-F-Ph | 3,5-$F_2$ |
| H | 4-F-Ph | 4,5-$F_2$ |
| H | 4-F-Ph | 5,6-$F_2$ |
| H | 4-F-Ph | 4-t-Bu |
| H | 4-F-Ph | 4-CN |
| H | 4-F-Ph | 4-Et |
| H | 4-F-Ph | 4-COOMe |
| H | 4-F-Ph | 4-COMe |
| H | 4-F-Ph | 4-COPh |
| H | 4-F-Ph | 4-F-5-Cl |
| H | 4-F-Ph | 4-F-5-Me |
| H | 4-F-Ph | 4-Me-5-Cl |
| H | 4-F-Ph | 5-Cl-6-Me |
| H | 2-MeO-Ph | 4-Cl |
| H | 2-MeO-Ph | 5-Cl |
| H | 2-MeO-Ph | 4-$CF_3$ |
| H | 2-MeO-Ph | 5-$CF_3$ |
| H | 2-MeO-Ph | 4-Me |
| H | 2-MeO-Ph | 5-Me |
| H | 2-MeO-Ph | 4-F |
| H | 2-MeO-Ph | 5-F |
| H | 2-MeO-Ph | 4-MeO |
| H | 2-MeO-Ph | 5-MeO |
| H | 3-MeO-Ph | 4-Cl |
| H | 3-MeO-Ph | 5-Cl |
| H | 3-MeO-Ph | 4-$CF_3$ |
| H | 3-MeO-Ph | 5-$CF_3$ |
| H | 3-MeO-Ph | 4-Me |
| H | 3-MeO-Ph | 5-Me |
| H | 3-MeO-Ph | 4-F |
| H | 3-MeO-Ph | 5-F |
| H | 3-MeO-Ph | 4-MeO |
| H | 3-MeO-Ph | 5-MeO |
| H | 4-MeO-Ph | 4-Cl |
| H | 4-MeO-Ph | 5-Cl |
| H | 4-MeO-Ph | 4-$CF_3$ |
| H | 4-MeO-Ph | 5-$CF_3$ |
| H | 4-MeO-Ph | 4-Me |
| H | 4-MeO-Ph | 5-Me |

| | | |
|---|---|---|
| H | 4-MeO-Ph | 4-F |
| H | 4-MeO-Ph | 5-F |
| H | 4-MeO-Ph | 4-MeO |
| H | 4-MeO-Ph | 5-MeO |
| H | 2-Br-Ph | 4-Cl |
| H | 2-Br-Ph | 5-Cl |
| H | 2-Br-Ph | 4-$CF_3$ |
| H | 2-Br-Ph | 5-$CF_3$ |
| H | 2-Br-Ph | 4-Me |
| H | 2-Br-Ph | 5-Me |
| H | 2-Br-Ph | 4-F |
| H | 2-Br-Ph | 5-F |
| H | 2-Br-Ph | 4-MeO |
| H | 2-Br-Ph | 5-MeO |
| H | 3-Br-Ph | 4-Cl |
| H | 3-Br-Ph | 5-Cl |
| H | 3-Br-Ph | 4-$CF_3$ |
| H | 3-Br-Ph | 5-$CF_3$ |
| H | 3-Br-Ph | 4-Me |
| H | 3-Br-Ph | 5-Me |
| H | 3-Br-Ph | 4-F |
| H | 3-Br-Ph | 5-F |
| H | 3-Br-Ph | 4-MeO |
| H | 3-Br-Ph | 5-MeO |
| H | 4-Br-Ph | 4-Cl |
| H | 4-Br-Ph | 5-Cl |
| H | 4-Br-Ph | 4-$CF_3$ |
| H | 4-Br-Ph | 5-$CF_3$ |
| H | 4-Br-Ph | 4-Me |
| H | 4-Br-Ph | 5-Me |
| H | 4-Br-Ph | 4-F |
| H | 4-Br-Ph | 5-F |
| H | 4-Br-Ph | 4-MeO |
| H | 4-Br-Ph | 5-MeO |
| H | 4-Et-Ph | 4-Cl |
| H | 4-Et-Ph | 5-Cl |
| H | 4-Et-Ph | 4-$CF_3$ |
| H | 4-Et-Ph | 5-$CF_3$ |
| H | 4-Et-Ph | 4-Me |
| H | 4-Et-Ph | 5-Me |
| H | 4-Et-Ph | 4-F |
| H | 4-Et-Ph | 5-F |
| H | 4-Et-Ph | 4-MeO |
| H | 4-Et-Ph | 5-MeO |
| H | 4-Pr-Ph | 4-Cl |
| H | 4-Pr-Ph | 5-Cl |
| H | 4-Pr-Ph | 4-Me |
| H | 4-Pr-Ph | 5-Me |
| H | 4-Pr-Ph | 4-F |
| H | 4-Pr-Ph | 5-F |
| H | 4-t-Bu-Ph | 4-Cl |
| H | 4-t-Bu-Ph | 5-Cl |
| H | 4-t-Bu-Ph | 4-Me |
| H | 4-t-Bu-Ph | 5-Me |
| H | 4-t-Bu-Ph | 4-F |
| H | 4-t-Bu-Ph | 5-F |
| H | 4-n-Bu-Ph | 4-Cl |
| H | 4-n-Bu-Ph | 5-Cl |

| | | |
|---|---|---|
| H | 4-n-Bu-Ph | 4-Me |
| H | 4-n-Bu-Ph | 5-Me |
| H | 4-n-Bu-Ph | 4-F |
| H | 4-n-Bu-Ph | 5-F |
| H | 4-n-Pen-Ph | 4-Cl |
| H | 4-n-Pen-Ph | 5-Cl |
| H | 4-n-Pen-Ph | 4-Me |
| H | 4-n-Pen-Ph | 5-Me |
| H | 4-n-Pen-Ph | 4-F |
| H | 4-n-Pen-Ph | 5-F |
| H | 4-n-Hex-Ph | 4-Cl |
| H | 4-n-Hex-Ph | 5-Cl |
| H | 4-n-Hex-Ph | 4-Me |
| H | 4-n-Hex-Ph | 5-Me |
| H | 4-n-Hex-Ph | 4-F |
| H | 4-n-Hex-Ph | 5-F |
| H | 2,6-$F_2$-Ph | 3-Cl |
| H | 2,6-$F_2$-Ph | 4-Cl |
| H | 2,6-$F_2$-Ph | 5-Cl |
| H | 2,6-$F_2$-Ph | 6-Cl |
| H | 2,6-$F_2$-Ph | 3-$CF_3$ |
| H | 2,6-$F_2$-Ph | 4-$CF_3$ |
| H | 2,6-$F_2$-Ph | 5-$CF_3$ |
| H | 2,6-$F_2$-Ph | 3-Me |
| H | 2,6-$F_2$-Ph | 4-Me |
| H | 2,6-$F_2$-Ph | 5-Me |
| H | 2,6-$F_2$-Ph | 6-Me |
| H | 2,6-$F_2$-Ph | 3-F |
| H | 2,6-$F_2$-Ph | 4-F |
| H | 2,6-$F_2$-Ph | 5-F |
| H | 2,6-$F_2$-Ph | 6-F |
| H | 2,6-$F_2$-Ph | 3-MeO |
| H | 2,6-$F_2$-Ph | 4-MeO |
| H | 2,6-$F_2$-Ph | 5-MeO |
| H | 2,6-$F_2$-Ph | 6-MeO |
| H | 2,6-$F_2$-Ph | 3,4-$Me_2$ |
| H | 2,6-$F_2$-Ph | 3,5-$Me_2$ |
| H | 2,6-$F_2$-Ph | 3,6-$Me_2$ |
| H | 2,6-$F_2$-Ph | 4,5-$Me_2$ |
| H | 2,6-$F_2$-Ph | 4,6-$Me_2$ |
| H | 2,6-$F_2$-Ph | 5,6-$Me_2$ |
| H | 2,6-$F_2$-Ph | 3,4-$(MeO)_2$ |
| H | 2,6-$F_2$-Ph | 3,5-$(MeO)_2$ |
| H | 2,6-$F_2$-Ph | 3,6-$(MeO)_2$ |
| H | 2,6-$F_2$-Ph | 4,5-$(MeO)_2$ |
| H | 2,6-$F_2$-Ph | 4,6-$(MeO)_2$ |
| H | 2,6-$F_2$-Ph | 5,6-$(MeO)_2$ |
| H | 2,6-$F_2$-Ph | 3,4-$Cl_2$ |
| H | 2,6-$F_2$-Ph | 3,5-$Cl_2$ |
| H | 2,6-$F_2$-Ph | 3,6-$Cl_2$ |
| H | 2,6-$F_2$-Ph | 4,5-$Cl_2$ |
| H | 2,6-$F_2$-Ph | 4,6-$Cl_2$ |
| H | 2,6-$F_2$-Ph | 5,6-$Cl_2$ |
| H | 2,6-$F_2$-Ph | 3,4-$F_2$ |
| H | 2,6-$F_2$-Ph | 3,5-$F_2$ |
| H | 2,6-$F_2$-Ph | 3,6-$F_2$ |
| H | 2,6-$F_2$-Ph | 4,5-$F_2$ |
| H | 2,6-$F_2$-Ph | 4,6-$F_2$ |

| | | |
|---|---|---|
| H | 2,6-$F_2$-Ph | 5,6-$F_2$ |
| H | 2,6-$F_2$-Ph | 4-t-Bu |
| H | 2,6-$F_2$-Ph | 4-CN |
| H | 2,6-$F_2$-Ph | 4-Et |
| H | 2,6-$F_2$-Ph | 4-COOMe |
| H | 2,6-$F_2$-Ph | 4-COOEt |
| H | 2,6-$F_2$-Ph | 4-COMe |
| H | 2,6-$F_2$-Ph | 4-COPh |
| H | 2,6-$F_2$-Ph | 4-F-5-Cl |
| H | 2,6-$F_2$-Ph | 4-F-5-Me |
| H | 2,6-$F_2$-Ph | 4-Me-5-Cl |
| H | 2,6-$F_2$-Ph | 5-Cl-6-Me |
| H | 2,5-$F_2$-Ph | 3-Cl |
| H | 2,5-$F_2$-Ph | 4-Cl |
| H | 2,5-$F_2$-Ph | 5-Cl |
| H | 2,5-$F_2$-Ph | 6-Cl |
| H | 2,5-$F_2$-Ph | 3-$CF_3$ |
| H | 2,5-$F_2$-Ph | 4-$CF_3$ |
| H | 2,5-$F_2$-Ph | 5-$CF_3$ |
| H | 2,5-$F_2$-Ph | 3-Me |
| H | 2,5-$F_2$-Ph | 4-Me |
| H | 2,5-$F_2$-Ph | 5-Me |
| H | 2,5-$F_2$-Ph | 6-Me |
| H | 2,5-$F_2$-Ph | 3-F |
| H | 2,5-$F_2$-Ph | 4-F |
| H | 2,5-$F_2$-Ph | 5-F |
| H | 2,5-$F_2$-Ph | 6-F |
| H | 2,5-$F_2$-Ph | 3-MeO |
| H | 2,5-$F_2$-Ph | 4-MeO |
| H | 2,5-$F_2$-Ph | 5-MeO |
| H | 2,5-$F_2$-Ph | 6-MeO |
| H | 2,5-$F_2$-Ph | 3,4-$Me_2$ |
| H | 2,5-$F_2$-Ph | 3,5-$Me_2$ |
| H | 2,5-$F_2$-Ph | 3,6-$Me_2$ |
| H | 2,5-$F_2$-Ph | 4,5-$Me_2$ |
| H | 2,5-$F_2$-Ph | 4,6-$Me_2$ |
| H | 2,5-$F_2$-Ph | 5,6-$Me_2$ |
| H | 2,5-$F_2$-Ph | 3,4-$(MeO)_2$ |
| H | 2,5-$F_2$-Ph | 3,5-$(MeO)_2$ |
| H | 2,5-$F_2$-Ph | 3,6-$(MeO)_2$ |
| H | 2,5-$F_2$-Ph | 4,5-$(MeO)_2$ |
| H | 2,5-$F_2$-Ph | 4,6-$(MeO)_2$ |
| H | 2,5-$F_2$-Ph | 5,6-$(MeO)_2$ |
| H | 2,5-$F_2$-Ph | 3,4-$Cl_2$ |
| H | 2,5-$F_2$-Ph | 3,5-$Cl_2$ |
| H | 2,5-$F_2$-Ph | 3,6-$Cl_2$ |
| H | 2,5-$F_2$-Ph | 4,5-$Cl_2$ |
| H | 2,5-$F_2$-Ph | 4,6-$Cl_2$ |
| H | 2,5-$F_2$-Ph | 5,6-$Cl_2$ |
| H | 2,5-$F_2$-Ph | 3,4-$F_2$ |
| H | 2,5-$F_2$-Ph | 3,5-$F_2$ |
| H | 2,5-$F_2$-Ph | 3,6-$F_2$ |
| H | 2,5-$F_2$-Ph | 4,5-$F_2$ |
| H | 2,5-$F_2$-Ph | 4,6-$F_2$ |
| H | 2,5-$F_2$-Ph | 5,6-$F_2$ |
| H | 2,5-$F_2$-Ph | 4-t-Bu |
| H | 2,5-$F_2$-Ph | 4-CN |
| H | 2,5-$F_2$-Ph | 4-Et |

| | | |
|---|---|---|
| H | 2,5-$F_2$-Ph | 4-COOMe |
| H | 2,5-$F_2$-Ph | 4-COOEt |
| H | 2,5-$F_2$-Ph | 4-COMe |
| H | 2,5-$F_2$-Ph | 4-COPh |
| H | 2,5-$F_2$-Ph | 4-F-5-Cl |
| H | 2,5-$F_2$-Ph | 4-F-5-Me |
| H | 2,5-$F_2$-Ph | 4-Me-5-Cl |
| H | 2,5-$F_2$-Ph | 5-Cl-6-Me |
| H | 2,4-$F_2$-Ph | 3-Cl |
| H | 2,4-$F_2$-Ph | 4-Cl |
| H | 2,4-$F_2$-Ph | 5-Cl |
| H | 2,4-$F_2$-Ph | 6-Cl |
| H | 2,4-$F_2$-Ph | 3-$CF_3$ |
| H | 2,4-$F_2$-Ph | 4-$CF_3$ |
| H | 2,4-$F_2$-Ph | 5-$CF_3$ |
| H | 2,4-$F_2$-Ph | 3-Me |
| H | 2,4-$F_2$-Ph | 4-Me |
| H | 2,4-$F_2$-Ph | 5-Me |
| H | 2,4-$F_2$-Ph | 6-Me |
| H | 2,4-$F_2$-Ph | 3-F |
| H | 2,4-$F_2$-Ph | 4-F |
| H | 2,4-$F_2$-Ph | 5-F |
| H | 2,4-$F_2$-Ph | 6-F |
| H | 2,4-$F_2$-Ph | 3-MeO |
| H | 2,4-$F_2$-Ph | 4-MeO |
| H | 2,4-$F_2$-Ph | 5-MeO |
| H | 2,4-$F_2$-Ph | 6-MeO |
| H | 2,4-$F_2$-Ph | 3,4-$Me_2$ |
| H | 2,4-$F_2$-Ph | 3,5-$Me_2$ |
| H | 2,4-$F_2$-Ph | 3,6-$Me_2$ |
| H | 2,4-$F_2$-Ph | 4,5-$Me_2$ |
| H | 2,4-$F_2$-Ph | 4,6-$Me_2$ |
| H | 2,4-$F_2$-Ph | 5,6-$Me_2$ |
| H | 2,4-$F_2$-Ph | 3,4-$(MeO)_2$ |
| H | 2,4-$F_2$-Ph | 3,5-$(MeO)_2$ |
| H | 2,4-$F_2$-Ph | 3,6-$(MeO)_2$ |
| H | 2,4-$F_2$-Ph | 4,5-$(MeO)_2$ |
| H | 2,4-$F_2$-Ph | 4,6-$(MeO)_2$ |
| H | 2,4-$F_2$-Ph | 5,6-$(MeO)_2$ |
| H | 2,4-$F_2$-Ph | 3,4-$Cl_2$ |
| H | 2,4-$F_2$-Ph | 3,5-$Cl_2$ |
| H | 2,4-$F_2$-Ph | 3,6-$Cl_2$ |
| H | 2,4-$F_2$-Ph | 4,5-$Cl_2$ |
| H | 2,4-$F_2$-Ph | 4,6-$Cl_2$ |
| H | 2,4-$F_2$-Ph | 5,6-$Cl_2$ |
| H | 2,4-$F_2$-Ph | 3,4-$F_2$ |
| H | 2,4-$F_2$-Ph | 3,5-$F_2$ |
| H | 2,4-$F_2$-Ph | 3,6-$F_2$ |
| H | 2,4-$F_2$-Ph | 4,5-$F_2$ |
| H | 2,4-$F_2$-Ph | 4,6-$F_2$ |
| H | 2,4-$F_2$-Ph | 5,6-$F_2$ |
| H | 2,4-$F_2$-Ph | 4-t-Bu |
| H | 2,4-$F_2$-Ph | 4-CN |
| H | 2,4-$F_2$-Ph | 4-Et |
| H | 2,4-$F_2$-Ph | 4-COOMe |
| H | 2,4-$F_2$-Ph | 4-COOEt |
| H | 2,4-$F_2$-Ph | 4-COMe |
| H | 2,4-$F_2$-Ph | 4-COPh |

| | | |
|---|---|---|
| H | 2,4-$F_2$-Ph | 4-F-5-Cl |
| H | 2,4-$F_2$-Ph | 4-F-5-Me |
| H | 2,4-$F_2$-Ph | 4-Me-5-Cl |
| H | 2,4-$F_2$-Ph | 5-Cl-6-Me |
| H | 2,3-$F_2$-Ph | 3-Cl |
| H | 2,3-$F_2$-Ph | 4-Cl |
| H | 2,3-$F_2$-Ph | 5-Cl |
| H | 2,3-$F_2$-Ph | 6-Cl |
| H | 2,3-$F_2$-Ph | 3-$CF_3$ |
| H | 2,3-$F_2$-Ph | 4-$CF_3$ |
| H | 2,3-$F_2$-Ph | 5-$CF_3$ |
| H | 2,3-$F_2$-Ph | 3-Me |
| H | 2,3-$F_2$-Ph | 4-Me |
| H | 2,3-$F_2$-Ph | 5-Me |
| H | 2,3-$F_2$-Ph | 6-Me |
| H | 2,3-$F_2$-Ph | 3-F |
| H | 2,3-$F_2$-Ph | 4-F |
| H | 2,3-$F_2$-Ph | 5-F |
| H | 2,3-$F_2$-Ph | 6-F |
| H | 2,3-$F_2$-Ph | 3-MeO |
| H | 2,3-$F_2$-Ph | 4-MeO |
| H | 2,3-$F_2$-Ph | 5-MeO |
| H | 2,3-$F_2$-Ph | 6-MeO |
| H | 2,3-$F_2$-Ph | 3,4-$Me_2$ |
| H | 2,3-$F_2$-Ph | 3,5-$Me_2$ |
| H | 2,3-$F_2$-Ph | 3,6-$Me_2$ |
| H | 2,3-$F_2$-Ph | 4,5-$Me_2$ |
| H | 2,3-$F_2$-Ph | 4,6-$Me_2$ |
| H | 2,3-$F_2$-Ph | 5,6-$Me_2$ |
| H | 2,3-$F_2$-Ph | 3,4-$(MeO)_2$ |
| H | 2,3-$F_2$-Ph | 3,5-$(MeO)_2$ |
| H | 2,3-$F_2$-Ph | 3,6-$(MeO)_2$ |
| H | 2,3-$F_2$-Ph | 4,5-$(MeO)_2$ |
| H | 2,3-$F_2$-Ph | 4,6-$(MeO)_2$ |
| H | 2,3-$F_2$-Ph | 5,6-$(MeO)_2$ |
| H | 2,3-$F_2$-Ph | 3,4-$Cl_2$ |
| H | 2,3-$F_2$-Ph | 3,5-$Cl_2$ |
| H | 2,3-$F_2$-Ph | 3,6-$Cl_2$ |
| H | 2,3-$F_2$-Ph | 4,5-$Cl_2$ |
| H | 2,3-$F_2$-Ph | 4,6-$Cl_2$ |
| H | 2,3-$F_2$-Ph | 5,6-$Cl_2$ |
| H | 2,3-$F_2$-Ph | 3,4-$F_2$ |
| H | 2,3-$F_2$-Ph | 3,5-$F_2$ |
| H | 2,3-$F_2$-Ph | 3,6-$F_2$ |
| H | 2,3-$F_2$-Ph | 4,5-$F_2$ |
| H | 2,3-$F_2$-Ph | 4,6-$F_2$ |
| H | 2,3-$F_2$-Ph | 5,6-$F_2$ |
| H | 2,3-$F_2$-Ph | 4-t-Bu |
| H | 2,3-$F_2$-Ph | 4-CN |
| H | 2,3-$F_2$-Ph | 4-Et |
| H | 2,3-$F_2$-Ph | 4-COOMe |
| H | 2,3-$F_2$-Ph | 4-COOEt |
| H | 2,3-$F_2$-Ph | 4-COMe |
| H | 2,3-$F_2$-Ph | 4-COPh |
| H | 2,3-$F_2$-Ph | 4-F-5-Cl |
| H | 2,3-$F_2$-Ph | 4-F-5-Me |
| H | 2,3-$F_2$-Ph | 4-Me-5-Cl |
| H | 2,3-$F_2$-Ph | 5-Cl-6-Me |

| | | |
|---|---|---|
| H | 3,4-$F_2$-Ph | 3-Cl |
| H | 3,4-$F_2$-Ph | 4-Cl |
| H | 3,4-$F_2$-Ph | 5-Cl |
| H | 3,4-$F_2$-Ph | 6-Cl |
| H | 3,4-$F_2$-Ph | 3-$CF_3$ |
| H | 3,4-$F_2$-Ph | 4-$CF_3$ |
| H | 3,4-$F_2$-Ph | 5-$CF_3$ |
| H | 3,4-$F_2$-Ph | 3-Me |
| H | 3,4-$F_2$-Ph | 4-Me |
| H | 3,4-$F_2$-Ph | 5-Me |
| H | 3,4-$F_2$-Ph | 6-Me |
| H | 3,4-$F_2$-Ph | 3-F |
| H | 3,4-$F_2$-Ph | 4-F |
| H | 3,4-$F_2$-Ph | 5-F |
| H | 3,4-$F_2$-Ph | 6-F |
| H | 3,4-$F_2$-Ph | 3-MeO |
| H | 3,4-$F_2$-Ph | 4-MeO |
| H | 3,4-$F_2$-Ph | 5-MeO |
| H | 3,4-$F_2$-Ph | 6-MeO |
| H | 3,4-$F_2$-Ph | 3,4-$Me_2$ |
| H | 3,4-$F_2$-Ph | 3,5-$Me_2$ |
| H | 3,4-$F_2$-Ph | 3,6-$Me_2$ |
| H | 3,4-$F_2$-Ph | 4,5-$Me_2$ |
| H | 3,4-$F_2$-Ph | 4,6-$Me_2$ |
| H | 3,4-$F_2$-Ph | 5,6-$Me_2$ |
| H | 3,4-$F_2$-Ph | 3,4-$(MeO)_2$ |
| H | 3,4-$F_2$-Ph | 3,5-$(MeO)_2$ |
| H | 3,4-$F_2$-Ph | 3,6-$(MeO)_2$ |
| H | 3,4-$F_2$-Ph | 4,5-$(MeO)_2$ |
| H | 3,4-$F_2$-Ph | 4,6-$(MeO)_2$ |
| H | 3,4-$F_2$-Ph | 5,6-$(MeO)_2$ |
| H | 3,4-$F_2$-Ph | 3,4-$Cl_2$ |
| H | 3,4-$F_2$-Ph | 3,5-$Cl_2$ |
| H | 3,4-$F_2$-Ph | 3,6-$Cl_2$ |
| H | 3,4-$F_2$-Ph | 4,5-$Cl_2$ |
| H | 3,4-$F_2$-Ph | 4,6-$Cl_2$ |
| H | 3,4-$F_2$-Ph | 5,6-$Cl_2$ |
| H | 3,4-$F_2$-Ph | 3,4-$F_2$ |
| H | 3,4-$F_2$-Ph | 3,5-$F_2$ |
| H | 3,4-$F_2$-Ph | 3,6-$F_2$ |
| H | 3,4-$F_2$-Ph | 4,5-$F_2$ |
| H | 3,4-$F_2$-Ph | 4,6-$F_2$ |
| H | 3,4-$F_2$-Ph | 5,6-$F_2$ |
| H | 3,4-$F_2$-Ph | 4-t-Bu |
| H | 3,4-$F_2$-Ph | 4-CN |
| H | 3,4-$F_2$-Ph | 4-Et |
| H | 3,4-$F_2$-Ph | 4-COOMe |
| H | 3,4-$F_2$-Ph | 4-COOEt |
| H | 3,4-$F_2$-Ph | 4-COMe |
| H | 3,4-$F_2$-Ph | 4-COPh |
| H | 3,4-$F_2$-Ph | 4-F-5-Cl |
| H | 3,4-$F_2$-Ph | 4-F-5-Me |
| H | 3,4-$F_2$-Ph | 4-Me-5-Cl |
| H | 3,4-$F_2$-Ph | 5-Cl-6-Me |
| H | 3,5-$F_2$-Ph | 3-Cl |
| H | 3,5-$F_2$-Ph | 4-Cl |
| H | 3,5-$F_2$-Ph | 5-Cl |
| H | 3,5-$F_2$-Ph | 6-Cl |

| | | |
|---|---|---|
| H | 3,5-$F_2$-Ph | 3-$CF_3$ |
| H | 3,5-$F_2$-Ph | 4-$CF_3$ |
| H | 3,5-$F_2$-Ph | 5-$CF_3$ |
| H | 3,5-$F_2$-Ph | 3-Me |
| H | 3,5-$F_2$-Ph | 4-Me |
| H | 3,5-$F_2$-Ph | 5-Me |
| H | 3,5-$F_2$-Ph | 6-Me |
| H | 3,5-$F_2$-Ph | 3-F |
| H | 3,5-$F_2$-Ph | 4-F |
| H | 3,5-$F_2$-Ph | 5-F |
| H | 3,5-$F_2$-Ph | 6-F |
| H | 3,5-$F_2$-Ph | 3-MeO |
| H | 3,5-$F_2$-Ph | 4-MeO |
| H | 3,5-$F_2$-Ph | 5-MeO |
| H | 3,5-$F_2$-Ph | 6-MeO |
| H | 3,5-$F_2$-Ph | 3,4-$Me_2$ |
| H | 3,5-$F_2$-Ph | 3,5-$Me_2$ |
| H | 3,5-$F_2$-Ph | 3,6-$Me_2$ |
| H | 3,5-$F_2$-Ph | 4,5-$Me_2$ |
| H | 3,5-$F_2$-Ph | 4,6-$Me_2$ |
| H | 3,5-$F_2$-Ph | 5,6-$Me_2$ |
| H | 3,5-$F_2$-Ph | 3,4-$(MeO)_2$ |
| H | 3,5-$F_2$-Ph | 3,5-$(MeO)_2$ |
| H | 3,5-$F_2$-Ph | 3,6-$(MeO)_2$ |
| H | 3,5-$F_2$-Ph | 4,5-$(MeO)_2$ |
| H | 3,5-$F_2$-Ph | 4,6-$(MeO)_2$ |
| H | 3,5-$F_2$-Ph | 5,6-$(MeO)_2$ |
| H | 3,5-$F_2$-Ph | 3,4-$Cl_2$ |
| H | 3,5-$F_2$-Ph | 3,5-$Cl_2$ |
| H | 3,5-$F_2$-Ph | 3,6-$Cl_2$ |
| H | 3,5-$F_2$-Ph | 4,5-$Cl_2$ |
| H | 3,5-$F_2$-Ph | 4,6-$Cl_2$ |
| H | 3,5-$F_2$-Ph | 5,6-$Cl_2$ |
| H | 3,5-$F_2$-Ph | 3,4-$F_2$ |
| H | 3,5-$F_2$-Ph | 3,5-$F_2$ |
| H | 3,5-$F_2$-Ph | 3,6-$F_2$ |
| H | 3,5-$F_2$-Ph | 4,5-$F_2$ |
| H | 3,5-$F_2$-Ph | 4,6-$F_2$ |
| H | 3,5-$F_2$-Ph | 5,6-$F_2$ |
| H | 3,5-$F_2$-Ph | 4-t-Bu |
| H | 3,5-$F_2$-Ph | 4-CN |
| H | 3,5-$F_2$-Ph | 4-Et |
| H | 3,5-$F_2$-Ph | 4-COOMe |
| H | 3,5-$F_2$-Ph | 4-COOEt |
| H | 3,5-$F_2$-Ph | 4-COMe |
| H | 3,5-$F_2$-Ph | 4-COPh |
| H | 3,5-$F_2$-Ph | 4-F-5-Cl |
| H | 3,5-$F_2$-Ph | 4-F-5-Me |
| H | 3,5-$F_2$-Ph | 4-Me-5-Cl |
| H | 3,5-$F_2$-Ph | 5-Cl-6-Me |
| H | 2-F-4-Me-Ph | 3-Cl |
| H | 2-F-4-Me-Ph | 4-Cl |
| H | 2-F-4-Me-Ph | 5-Cl |
| H | 2-F-4-Me-Ph | 6-Cl |
| H | 2-F-4-Me-Ph | 3-$CF_3$ |
| H | 2-F-4-Me-Ph | 4-$CF_3$ |
| H | 2-F-4-Me-Ph | 5-$CF_3$ |
| H | 2-F-4-Me-Ph | 3-Me |

| | | |
|---|---|---|
| H | 2-F-4-Me-Ph | 4-Me |
| H | 2-F-4-Me-Ph | 5-Me |
| H | 2-F-4-Me-Ph | 6-Me |
| H | 2-F-4-Me-Ph | 3-F |
| H | 2-F-4-Me-Ph | 4-F |
| H | 2-F-4-Me-Ph | 5-F |
| H | 2-F-4-Me-Ph | 6-F |
| H | 2-F-4-Me-Ph | 3-MeO |
| H | 2-F-4-Me-Ph | 4-MeO |
| H | 2-F-4-Me-Ph | 5-MeO |
| H | 2-F-4-Me-Ph | 6-MeO |
| H | 2-F-4-Me-Ph | 3,4-$Me_2$ |
| H | 2-F-4-Me-Ph | 3,5-$Me_2$ |
| H | 2-F-4-Me-Ph | 3,6-$Me_2$ |
| H | 2-F-4-Me-Ph | 4,5-$Me_2$ |
| H | 2-F-4-Me-Ph | 4,6-$Me_2$ |
| H | 2-F-4-Me-Ph | 5,6-$Me_2$ |
| H | 2-F-4-Me-Ph | 3,4-$(MeO)_2$ |
| H | 2-F-4-Me-Ph | 3,5-$(MeO)_2$ |
| H | 2-F-4-Me-Ph | 3,6-$(MeO)_2$ |
| H | 2-F-4-Me-Ph | 4,5-$(MeO)_2$ |
| H | 2-F-4-Me-Ph | 4,6-$(MeO)_2$ |
| H | 2-F-4-Me-Ph | 5,6-$(MeO)_2$ |
| H | 2-F-4-Me-Ph | 3,4-$Cl_2$ |
| H | 2-F-4-Me-Ph | 3,5-$Cl_2$ |
| H | 2-F-4-Me-Ph | 3,6-$Cl_2$ |
| H | 2-F-4-Me-Ph | 4,5-$Cl_2$ |
| H | 2-F-4-Me-Ph | 4,6-$Cl_2$ |
| H | 2-F-4-Me-Ph | 5,6-$Cl_2$ |
| H | 2-F-4-Me-Ph | 3,4-$F_2$ |
| H | 2-F-4-Me-Ph | 3,5-$F_2$ |
| H | 2-F-4-Me-Ph | 3,6-$F_2$ |
| H | 2-F-4-Me-Ph | 4,5-$F_2$ |
| H | 2-F-4-Me-Ph | 4,6-$F_2$ |
| H | 2-F-4-Me-Ph | 5,6-$F_2$ |
| H | 2-F-4-Me-Ph | 4-t-Bu |
| H | 2-F-4-Me-Ph | 4-CN |
| H | 2-F-4-Me-Ph | 4-Et |
| H | 2-F-4-Me-Ph | 4-COOMe |
| H | 2-F-4-Me-Ph | 4-COOEt |
| H | 2-F-4-Me-Ph | 4-COMe |
| H | 2-F-4-Me-Ph | 4-COPh |
| H | 2-F-4-Me-Ph | 4-F-5-Cl |
| H | 2-F-4-Me-Ph | 4-F-5-Me |
| H | 2-F-4-Me-Ph | 4-Me-5-Cl |
| H | 2-F-4-Me-Ph | 5-Cl-6-Me |
| H | 2-F-4-Et-Ph | 3-Cl |
| H | 2-F-4-Et-Ph | 4-Cl |
| H | 2-F-4-Et-Ph | 5-Cl |
| H | 2-F-4-Et-Ph | 6-Cl |
| H | 2-F-4-Et-Ph | 3-$CF_3$ |
| H | 2-F-4-Et-Ph | 4-$CF_3$ |
| H | 2-F-4-Et-Ph | 5-$CF_3$ |
| H | 2-F-4-Et-Ph | 3-Me |
| H | 2-F-4-Et-Ph | 4-Me |
| H | 2-F-4-Et-Ph | 5-Me |
| H | 2-F-4-Et-Ph | 6-Me |
| H | 2-F-4-Et-Ph | 3-F |

| | | |
|---|---|---|
| H | 2-F-4-Et-Ph | 4-F |
| H | 2-F-4-Et-Ph | 5-F |
| H | 2-F-4-Et-Ph | 6-F |
| H | 2-F-4-Et-Ph | 3-MeO |
| H | 2-F-4-Et-Ph | 4-MeO |
| H | 2-F-4-Et-Ph | 5-MeO |
| H | 2-F-4-Et-Ph | 6-MeO |
| H | 2-F-4-Et-Ph | 3,4-$Me_2$ |
| H | 2-F-4-Et-Ph | 3,5-$Me_2$ |
| H | 2-F-4-Et-Ph | 3,6-$Me_2$ |
| H | 2-F-4-Et-Ph | 4,5-$Me_2$ |
| H | 2-F-4-Et-Ph | 4,6-$Me_2$ |
| H | 2-F-4-Et-Ph | 5,6-$Me_2$ |
| H | 2-F-4-Et-Ph | 3,4-$(MeO)_2$ |
| H | 2-F-4-Et-Ph | 3,5-$(MeO)_2$ |
| H | 2-F-4-Et-Ph | 3,6-$(MeO)_2$ |
| H | 2-F-4-Et-Ph | 4,5-$(MeO)_2$ |
| H | 2-F-4-Et-Ph | 4,6-$(MeO)_2$ |
| H | 2-F-4-Et-Ph | 5,6-$(MeO)_2$ |
| H | 2-F-4-Et-Ph | 3,4-$Cl_2$ |
| H | 2-F-4-Et-Ph | 3,5-$Cl_2$ |
| H | 2-F-4-Et-Ph | 3,6-$Cl_2$ |
| H | 2-F-4-Et-Ph | 4,5-$Cl_2$ |
| H | 2-F-4-Et-Ph | 4,6-$Cl_2$ |
| H | 2-F-4-Et-Ph | 5,6-$Cl_2$ |
| H | 2-F-4-Et-Ph | 3,4-$F_2$ |
| H | 2-F-4-Et-Ph | 3,5-$F_2$ |
| H | 2-F-4-Et-Ph | 3,6-$F_2$ |
| H | 2-F-4-Et-Ph | 4,5-$F_2$ |
| H | 2-F-4-Et-Ph | 4,6-$F_2$ |
| H | 2-F-4-Et-Ph | 5,6-$F_2$ |
| H | 2-F-4-Et-Ph | 4-t-Bu |
| H | 2-F-4-Et-Ph | 4-CN |
| H | 2-F-4-Et-Ph | 4-Et |
| H | 2-F-4-Et-Ph | 4-COOMe |
| H | 2-F-4-Et-Ph | 4-COOEt |
| H | 2-F-4-Et-Ph | 4-COMe |
| H | 2-F-4-Et-Ph | 4-COPh |
| H | 2-F-4-Et-Ph | 4-F-5-Cl |
| H | 2-F-4-Et-Ph | 4-F-5-Me |
| H | 2-F-4-Et-Ph | 4-Me-5-Cl |
| H | 2-F-4-Et-Ph | 5-Cl-6-Me |
| H | 2-F-6-MeO-Ph | 3-Cl |
| H | 2-F-6-MeO-Ph | 4-Cl |
| H | 2-F-6-MeO-Ph | 5-Cl |
| H | 2-F-6-MeO-Ph | 6-Cl |
| H | 2-F-6-MeO-Ph | 3-$CF_3$ |
| H | 2-F-6-MeO-Ph | 4-$CF_3$ |
| H | 2-F-6-MeO-Ph | 5-$CF_3$ |
| H | 2-F-6-MeO-Ph | 3-Me |
| H | 2-F-6-MeO-Ph | 4-Me |
| H | 2-F-6-MeO-Ph | 5-Me |
| H | 2-F-6-MeO-Ph | 6-Me |
| H | 2-F-6-MeO-Ph | 3-F |
| H | 2-F-6-MeO-Ph | 4-F |
| H | 2-F-6-MeO-Ph | 5-F |
| H | 2-F-6-MeO-Ph | 6-F |
| H | 2-F-6-MeO-Ph | 3-MeO |

| | | |
|---|---|---|
| H | 2-F-6-MeO-Ph | 4-MeO |
| H | 2-F-6-MeO-Ph | 5-MeO |
| H | 2-F-6-MeO-Ph | 6-MeO |
| H | 2-F-6-MeO-Ph | 3,4-$Me_2$ |
| H | 2-F-6-MeO-Ph | 3,5-$Me_2$ |
| H | 2-F-6-MeO-Ph | 3,6-$Me_2$ |
| H | 2-F-6-MeO-Ph | 4,5-$Me_2$ |
| H | 2-F-6-MeO-Ph | 4,6-$Me_2$ |
| H | 2-F-6-MeO-Ph | 5,6-$Me_2$ |
| H | 2-F-6-MeO-Ph | 3,4-$(MeO)_2$ |
| H | 2-F-6-MeO-Ph | 3,5-$(MeO)_2$ |
| H | 2-F-6-MeO-Ph | 3,6-$(MeO)_2$ |
| H | 2-F-6-MeO-Ph | 4,5-$(MeO)_2$ |
| H | 2-F-6-MeO-Ph | 4,6-$(MeO)_2$ |
| H | 2-F-6-MeO-Ph | 5,6-$(MeO)_2$ |
| H | 2-F-6-MeO-Ph | 3,4-$Cl_2$ |
| H | 2-F-6-MeO-Ph | 3,5-$Cl_2$ |
| H | 2-F-6-MeO-Ph | 3,6-$Cl_2$ |
| H | 2-F-6-MeO-Ph | 4,5-$Cl_2$ |
| H | 2-F-6-MeO-Ph | 4,6-$Cl_2$ |
| H | 2-F-6-MeO-Ph | 5,6-$Cl_2$ |
| H | 2-F-6-MeO-Ph | 3,4-$F_2$ |
| H | 2-F-6-MeO-Ph | 3,5-$F_2$ |
| H | 2-F-6-MeO-Ph | 3,6-$F_2$ |
| H | 2-F-6-MeO-Ph | 4,5-$F_2$ |
| H | 2-F-6-MeO-Ph | 4,6-$F_2$ |
| H | 2-F-6-MeO-Ph | 5,6-$F_2$ |
| H | 2-F-6-MeO-Ph | 4-t-Bu |
| H | 2-F-6-MeO-Ph | 4-CN |
| H | 2-F-6-MeO-Ph | 4-Et |
| H | 2-F-6-MeO-Ph | 4-COOMe |
| H | 2-F-6-MeO-Ph | 4-COOEt |
| H | 2-F-6-MeO-Ph | 4-COMe |
| H | 2-F-6-MeO-Ph | 4-COPh |
| H | 2-F-6-MeO-Ph | 4-F-5-Cl |
| H | 2-F-6-MeO-Ph | 4-F-5-Me |
| H | 2-F-6-MeO-Ph | 4-Me-5-Cl |
| H | 2-F-6-MeO-Ph | 5-Cl-6-Me |
| H | 2,6-$Cl_2$-Ph | 3-Cl |
| H | 2,6-$Cl_2$-Ph | 4-Cl |
| H | 2,6-$Cl_2$-Ph | 5-Cl |
| H | 2,6-$Cl_2$-Ph | 6-Cl |
| H | 2,6-$Cl_2$-Ph | 3-$CF_3$ |
| H | 2,6-$Cl_2$-Ph | 4-$CF_3$ |
| H | 2,6-$Cl_2$-Ph | 5-$CF_3$ |
| H | 2,6-$Cl_2$-Ph | 3-Me |
| H | 2,6-$Cl_2$-Ph | 4-Me |
| H | 2,6-$Cl_2$-Ph | 5-Me |
| H | 2,6-$Cl_2$-Ph | 6-Me |
| H | 2,6-$Cl_2$-Ph | 3-F |
| H | 2,6-$Cl_2$-Ph | 4-F |
| H | 2,6-$Cl_2$-Ph | 5-F |
| H | 2,6-$Cl_2$-Ph | 6-F |
| H | 2,6-$Cl_2$-Ph | 3-MeO |
| H | 2,6-$Cl_2$-Ph | 4-MeO |
| H | 2,6-$Cl_2$-Ph | 5-MeO |
| H | 2,6-$Cl_2$-Ph | 6-MeO |
| H | 2,6-$Cl_2$-Ph | 4,5-$Me_2$ |

| | | |
|---|---|---|
| H | 2,6-$Cl_2$-Ph | 4,5-$(MeO)_2$ |
| H | 2,6-$Cl_2$-Ph | 4,5-$Cl_2$ |
| H | 2,6-$Cl_2$-Ph | 4,5-$F_2$ |
| H | 2,6-$Cl_2$-Ph | 4-t-Bu |
| H | 2,6-$Cl_2$-Ph | 4-CN |
| H | 2,6-$Cl_2$-Ph | 4-Et |
| H | 2,6-$Cl_2$-Ph | 4-COOMe |
| H | 2,6-$Cl_2$-Ph | 4-COMe |
| H | 2,6-$Cl_2$-Ph | 4-COPh |
| H | 2,5-$Cl_2$-Ph | 4-Cl |
| H | 2,5-$Cl_2$-Ph | 5-Cl |
| H | 2,5-$Cl_2$-Ph | 4-$CF_3$ |
| H | 2,5-$Cl_2$-Ph | 5-$CF_3$ |
| H | 2,5-$Cl_2$-Ph | 4-Me |
| H | 2,5-$Cl_2$-Ph | 5-Me |
| H | 2,5-$Cl_2$-Ph | 4-F |
| H | 2,5-$Cl_2$-Ph | 5-F |
| H | 2,5-$Cl_2$-Ph | 4-MeO |
| H | 2,4-$Cl_2$-Ph | 3-Cl |
| H | 2,4-$Cl_2$-Ph | 4-Cl |
| H | 2,4-$Cl_2$-Ph | 5-Cl |
| H | 2,4-$Cl_2$-Ph | 6-Cl |
| H | 2,4-$Cl_2$-Ph | 3-$CF_3$ |
| H | 2,4-$Cl_2$-Ph | 4-$CF_3$ |
| H | 2,4-$Cl_2$-Ph | 5-$CF_3$ |
| H | 2,4-$Cl_2$-Ph | 3-Me |
| H | 2,4-$Cl_2$-Ph | 4-Me |
| H | 2,4-$Cl_2$-Ph | 5-Me |
| H | 2,4-$Cl_2$-Ph | 6-Me |
| H | 2,4-$Cl_2$-Ph | 3-F |
| H | 2,4-$Cl_2$-Ph | 4-F |
| H | 2,4-$Cl_2$-Ph | 5-F |
| H | 2,4-$Cl_2$-Ph | 6-F |
| H | 2,4-$Cl_2$-Ph | 3-MeO |
| H | 2,4-$Cl_2$-Ph | 4-MeO |
| H | 2,4-$Cl_2$-Ph | 5-MeO |
| H | 2,4-$Cl_2$-Ph | 6-MeO |
| H | 2,4-$Cl_2$-Ph | 4,5-$Me_2$ |
| H | 2,4-$Cl_2$-Ph | 4,5-$(MeO)_2$ |
| H | 2,4-$Cl_2$-Ph | 4,5-$Cl_2$ |
| H | 2,4-$Cl_2$-Ph | 4,5-$F_2$ |
| H | 2,4-$Cl_2$-Ph | 4-t-Bu |
| H | 2,4-$Cl_2$-Ph | 4-CN |
| H | 2,4-$Cl_2$-Ph | 4-Et |
| H | 2,4-$Cl_2$-Ph | 4-COOMe |
| H | 2,4-$Cl_2$-Ph | 4-COMe |
| H | 2,4-$Cl_2$-Ph | 4-COPh |
| H | 2,3-$Cl_2$-Ph | 3-Cl |
| H | 2,3-$Cl_2$-Ph | 4-Cl |
| H | 2,3-$Cl_2$-Ph | 5-Cl |
| H | 2,3-$Cl_2$-Ph | 6-Cl |
| H | 2,3-$Cl_2$-Ph | 3-$CF_3$ |
| H | 2,3-$Cl_2$-Ph | 4-$CF_3$ |
| H | 2,3-$Cl_2$-Ph | 5-$CF_3$ |
| H | 2,3-$Cl_2$-Ph | 3-Me |
| H | 2,3-$Cl_2$-Ph | 4-Me |
| H | 2,3-$Cl_2$-Ph | 5-Me |
| H | 2,3-$Cl_2$-Ph | 6-Me |

| | | |
|---|---|---|
| H | 2,3-$Cl_2$-Ph | 3-F |
| H | 2,3-$Cl_2$-Ph | 4-F |
| H | 2,3-$Cl_2$-Ph | 5-F |
| H | 2,3-$Cl_2$-Ph | 6-F |
| H | 2,3-$Cl_2$-Ph | 3-MeO |
| H | 2,3-$Cl_2$-Ph | 4-MeO |
| H | 2,3-$Cl_2$-Ph | 5-MeO |
| H | 2,3-$Cl_2$-Ph | 6-MeO |
| H | 2,3-$Cl_2$-Ph | 4,5-$Me_2$ |
| H | 2,3-$Cl_2$-Ph | 4,5-$(MeO)_2$ |
| H | 2,3-$Cl_2$-Ph | 4,5-$Cl_2$ |
| H | 2,3-$Cl_2$-Ph | 4,5-$F_2$ |
| H | 2,3-$Cl_2$-Ph | 4-t-Bu |
| H | 2,3-$Cl_2$-Ph | 4-CN |
| H | 2,3-$Cl_2$-Ph | 4-Et |
| H | 2,3-$Cl_2$-Ph | 4-COOMe |
| H | 2,3-$Cl_2$-Ph | 4-COMe |
| H | 2,3-$Cl_2$-Ph | 4-COPh |
| H | 3,4-$Cl_2$-Ph | 4-Cl |
| H | 3,4-$Cl_2$-Ph | 5-Cl |
| H | 3,4-$Cl_2$-Ph | 4-$CF_3$ |
| H | 3,4-$Cl_2$-Ph | 5-$CF_3$ |
| H | 3,4-$Cl_2$-Ph | 4-Me |
| H | 3,4-$Cl_2$-Ph | 5-Me |
| H | 3,4-$Cl_2$-Ph | 4-F |
| H | 3,4-$Cl_2$-Ph | 5-F |
| H | 3,4-$Cl_2$-Ph | 4-MeO |
| H | 3,5-$Cl_2$-Ph | 3-Cl |
| H | 3,5-$Cl_2$-Ph | 4-Cl |
| H | 3,5-$Cl_2$-Ph | 5-Cl |
| H | 3,5-$Cl_2$-Ph | 6-Cl |
| H | 3,5-$Cl_2$-Ph | 3-$CF_3$ |
| H | 3,5-$Cl_2$-Ph | 4-$CF_3$ |
| H | 3,5-$Cl_2$-Ph | 5-$CF_3$ |
| H | 3,5-$Cl_2$-Ph | 3-Me |
| H | 3,5-$Cl_2$-Ph | 4-Me |
| H | 3,5-$Cl_2$-Ph | 5-Me |
| H | 3,5-$Cl_2$-Ph | 6-Me |
| H | 3,5-$Cl_2$-Ph | 3-F |
| H | 3,5-$Cl_2$-Ph | 4-F |
| H | 3,5-$Cl_2$-Ph | 5-F |
| H | 3,5-$Cl_2$-Ph | 6-F |
| H | 3,5-$Cl_2$-Ph | 3-MeO |
| H | 3,5-$Cl_2$-Ph | 4-MeO |
| H | 3,5-$Cl_2$-Ph | 5-MeO |
| H | 3,5-$Cl_2$-Ph | 6-MeO |
| H | 3,5-$Cl_2$-Ph | 4,5-$Me_2$ |
| H | 3,5-$Cl_2$-Ph | 4,5-$(MeO)_2$ |
| H | 3,5-$Cl_2$-Ph | 4,5-$Cl_2$ |
| H | 3,5-$Cl_2$-Ph | 4,5-$F_2$ |
| H | 3,5-$Cl_2$-Ph | 4-t-Bu |
| H | 3,5-$Cl_2$-Ph | 4-CN |
| H | 3,5-$Cl_2$-Ph | 4-Et |
| H | 3,5-$Cl_2$-Ph | 4-COOMe |
| H | 3,5-$Cl_2$-Ph | 4-COMe |
| H | 3,5-$Cl_2$-Ph | 4-COPh |
| H | 2,6-$Me_2$-Ph | 4-Cl |
| H | 2,6-$Me_2$-Ph | 5-Cl |

| | | |
|---|---|---|
| H | 2,6-$Me_2$-Ph | 4-$CF_3$ |
| H | 2,6-$Me_2$-Ph | 5-$CF_3$ |
| H | 2,6-$Me_2$-Ph | 4-Me |
| H | 2,6-$Me_2$-Ph | 5-Me |
| H | 2,6-$Me_2$-Ph | 4-F |
| H | 2,6-$Me_2$-Ph | 5-F |
| H | 2,6-$Me_2$-Ph | 4-MeO |
| H | 2,5-$Me_2$-Ph | 4-Cl |
| H | 2,5-$Me_2$-Ph | 5-Cl |
| H | 2,5-$Me_2$-Ph | 4-$CF_3$ |
| H | 2,5-$Me_2$-Ph | 5-$CF_3$ |
| H | 2,5-$Me_2$-Ph | 4-Me |
| H | 2,5-$Me_2$-Ph | 5-Me |
| H | 2,5-$Me_2$-Ph | 4-F |
| H | 2,5-$Me_2$-Ph | 5-F |
| H | 2,5-$Me_2$-Ph | 4-MeO |
| H | 2,4-$Me_2$-Ph | 4-Cl |
| H | 2,4-$Me_2$-Ph | 5-Cl |
| H | 2,4-$Me_2$-Ph | 4-$CF_3$ |
| H | 2,4-$Me_2$-Ph | 5-$CF_3$ |
| H | 2,4-$Me_2$-Ph | 4-Me |
| H | 2,4-$Me_2$-Ph | 5-Me |
| H | 2,4-$Me_2$-Ph | 4-F |
| H | 2,4-$Me_2$-Ph | 5-F |
| H | 2,4-$Me_2$-Ph | 4-MeO |
| H | 2,3-$Me_2$-Ph | 4-Cl |
| H | 2,3-$Me_2$-Ph | 5-Cl |
| H | 2,3-$Me_2$-Ph | 4-$CF_3$ |
| H | 2,3-$Me_2$-Ph | 5-$CF_3$ |
| H | 2,3-$Me_2$-Ph | 4-Me |
| H | 2,3-$Me_2$-Ph | 5-Me |
| H | 2,3-$Me_2$-Ph | 4-F |
| H | 2,3-$Me_2$-Ph | 5-F |
| H | 2,3-$Me_2$-Ph | 4-MeO |
| H | 3,4-$Me_2$-Ph | 4-Cl |
| H | 3,4-$Me_2$-Ph | 5-Cl |
| H | 3,4-$Me_2$-Ph | 4-$CF_3$ |
| H | 3,4-$Me_2$-Ph | 5-$CF_3$ |
| H | 3,4-$Me_2$-Ph | 4-Me |
| H | 3,4-$Me_2$-Ph | 5-Me |
| H | 3,4-$Me_2$-Ph | 4-F |
| H | 3,4-$Me_2$-Ph | 5-F |
| H | 3,4-$Me_2$-Ph | 4-MeO |
| H | 3,5-$Me_2$-Ph | 4-Cl |
| H | 3,5-$Me_2$-Ph | 5-Cl |
| H | 3,5-$Me_2$-Ph | 4-$CF_3$ |
| H | 3,5-$Me_2$-Ph | 5-$CF_3$ |
| H | 3,5-$Me_2$-Ph | 4-Me |
| H | 3,5-$Me_2$-Ph | 5-Me |
| H | 3,5-$Me_2$-Ph | 4-F |
| H | 3,5-$Me_2$-Ph | 5-F |
| H | 3,5-$Me_2$-Ph | 4-MeO |
| H | 2,6-$F_2$-4-Me-Ph | 4-Cl |
| H | 2,6-$F_2$-4-Me-Ph | 5-Cl |
| H | 2,6-$F_2$-4-Me-Ph | 4-$CF_3$ |
| H | 2,6-$F_2$-4-Me-Ph | 5-$CF_3$ |
| H | 2,6-$F_2$-4-Me-Ph | 4-Me |
| H | 2,6-$F_2$-4-Me-Ph | 5-Me |

| | | |
|---|---|---|
| H | 2,6-$F_2$-4-Me-Ph | 4-F |
| H | 2,6-$F_2$-4-Me-Ph | 5-F |
| H | 2,6-$F_2$-4-Me-Ph | 4-MeO |
| H | 2,6-$F_2$-4-Et-Ph | 4-Cl |
| H | 2,6-$F_2$-4-Et-Ph | 5-Cl |
| H | 2,6-$F_2$-4-Et-Ph | 4-$CF_3$ |
| H | 2,6-$F_2$-4-Et-Ph | 5-$CF_3$ |
| H | 2,6-$F_2$-4-Et-Ph | 4-Me |
| H | 2,6-$F_2$-4-Et-Ph | 5-Me |
| H | 2,6-$F_2$-4-Et-Ph | 4-F |
| H | 2,6-$F_2$-4-Et-Ph | 5-F |
| H | 2,6-$F_2$-4-Et-Ph | 4-MeO |
| | -CH=CH-CH=CH- | H |
| | -CH=CH-CCl=CH- | H |
| | -CH=CH-CH=CCl- | H |
| | -CH=CH-C(OMe)=CH- | H |
| | -CH=CH-CF=CH- | H |
| | -CH=CH-C(Me)=CH- | H |
| | -CH=N-CH=CH- | H |
| | -N=CH-CH=CH- | H |
| | $-(CH_2)_3-$ | H |
| | $-(CH_2)_4-$ | H |
| | $-(CH_2)_5-$ | H |
| | $-CH_2-O-CH_2-$ | H |
| | $-CH_2-CH_2-O-CH_2-$ | H |
| | $-CO-(CH_2)_3-$ | H |
| | $-CH_2-CH(CH_2Ph)-CH_2-$ | H |
| | $-CH_2-CH_2-CH(Me)-CH_2-$ | H |
| | $-CH_2-CH_2-CH(Ph)-CH_2-$ | H |
| | -CH=CH-CH=C(OMe)- | H |
| Me | Cl | H |
| Me | Me | H |
| Me | Et | H |
| Me | n-Pr | H |
| Me | i-Pr | H |
| Me | n-Bu | H |
| Me | i-Bu | H |
| Me | s-Bu | H |
| Me | t-Bu | H |
| Me | n-Pen | H |
| Me | 3-Me-n-Bu | H |
| Me | n-Hex | H |
| Me | $CF_3$ | H |
| Me | c-Pr | H |
| Me | c-Hex | H |
| Me | $CH_2OMe$ | H |
| Me | C(=NOMe)OMe | H |
| Me | C(=NOMe)Me | H |
| Me | C(=NOMe)C(=NOMe)Me | H |
| Me | C(=NOMe)Ph | H |
| Me | $C(=NOCH_2Ph)Me$ | H |
| Me | $CH_2SMe$ | H |
| Me | $CH_2OPh$ | H |
| Me | $CH_2NMe_2$ | H |
| Me | $ClCH_2$ | H |
| Me | $BrCH_2$ | H |
| Me | $CF_2Cl$ | H |
| Me | $CCl_3$ | H |

| | | |
|---|---|---|
| Me | $FCH_2$ | H |
| Me | $ICH_2$ | H |
| Me | $CF_2CF_3$ | H |
| Me | $MeO_2C-(MeON=)C$ | H |
| Me | $Ph-(PhCH_2ON=)C$ | H |
| Me | 1-Naphthyl | H |
| Me | 2-Naphthyl | H |
| Me | 1-Me-Pyrazol-5-yl | H |
| Me | 1-Me-Pyrazol-4-yl | H |
| Me | 1-Me-Pyrazol-3-yl | H |
| Me | 1-Me-4-Cl-Pyrazol-5-yl | H |
| Me | 1-Me-4-Cl-Pyrazol-3-yl | H |
| Me | 1-Me-3-Cl-Pyrazol-4-yl | H |
| Me | 1-Me-3-Cl-Pyrazol-5-yl | H |
| Me | 1-Me-5-Cl-Pyrazol-3-yl | H |
| Me | 1-Me-5-Cl-Pyrazol-4-yl | H |
| Me | 1-Me-3-$CF_3$-Pyrazol-4-yl | H |
| Me | 1-Me-5-$CF_3$-Pyrazol-3-yl | H |
| Me | 1-Me-4-MeOOC-Pyrazol-5-yl | H |
| Me | 1-Me-3-Cl-4-MeOOC-Pyrazol-5-yl | H |
| Me | 1-Me-3-Cl-4-EtOOC-Pyrazol-5-yl | H |
| Me | 1-Me-4-EtOOC-Pyrazol-3-yl | H |
| Me | 1,3-$Me_2$-Pyrazol-4-yl | H |
| Me | 1,3-$Me_2$-Pyrazol-5-yl | H |
| Me | 1,3-$Me_2$-5-Cl-Pyrazol-4-yl | H |
| Me | 1,3-$Me_2$-5-F-Pyrazol-4-yl | H |
| Me | 1,3,5-$Me_3$-Pyrazol-4-yl | H |
| Me | 1,3-$Me_2$-4-Cl-Pyrazol-5-yl | H |
| Me | 1-Me-3,5-$Cl_2$-Pyrazol-4-yl | H |
| Me | 1-Me-3,5-$F_2$-Pyrazol-4-yl | H |
| Me | 1-Ph-3,5-$Cl_2$-Pyrazol-4-yl | H |
| Me | 1-Ph-3,5-$F_2$-Pyrazol-4-yl | H |
| Me | Oxazol-2-yl | H |
| Me | 1,2,4-Oxazol-3-yl | H |
| Me | 6-MeO-Pyrimidin-2-yl | H |
| Me | Pyridazin-3-yl | H |
| Me | 1,3,5-Triazin-2-yl | H |
| Me | 1,2,4-Triazin-6-yl | H |
| Me | 1-Me-Pyrrole-2-yl | H |
| Me | 1-Me-Pyrrole-3-yl | H |
| Me | Furan-2-yl | H |
| Me | Furan-3-yl | H |
| Me | 5-Me-Furan-2-yl | H |
| Me | 2,5-$Me_2$-Furan-3-yl | H |
| Me | Thiophen-2-yl | H |
| Me | Thiophen-3-yl | H |
| Me | 5-Me-Thiophen-2-yl | H |
| Me | 5-Br-Thiophen-2-yl | H |
| Me | 3-Br-Thiophen-2-yl | H |
| Me | 4,5-$Br_2$-Thiophen-2-yl | H |
| Me | 5-Cl-Thiophen-2-yl | H |
| Me | 3-Me-Thiophen-2-yl | H |
| Me | 3-Cl-Thiophen-2-yl | H |
| Me | 3-F-Thiophen-2-yl | H |
| Me | 2,5-$Cl_2$-Thiophen-3-yl | H |
| Me | 2,5-$Me_2$-Thiophen-3-yl | H |
| Me | 4,5-$Br_2$-Thiophen-3-yl | H |
| Me | Thiazol-4-yl | H |

| | | |
|---|---|---|
| Me | Thiazol-5-yl | H |
| Me | Thiazol-2-yl | H |
| Me | 2,4-$Me_2$-Thiazol-5-yl | H |
| Me | 2-Br-4-Me-Thiazol-5-yl | H |
| Me | 2-Cl-4-Me-Thiazol-5-yl | H |
| Me | 2-Cl-4-$CF_3$-Thiazol-5-yl | H |
| Me | 2-Me-4-$CF_3$-Thiazol-5-yl | H |
| Me | 2-Cl-4-F-Thiazol-5-yl | H |
| Me | 2-Cl-Thiazol-4-yl | H |
| Me | 2-Me-Thiazol-4-yl | H |
| Me | 5-$CF_3$-Thiazol-2-yl | H |
| Me | 1,3-$Me_2$-Oxazol-5-yl | H |
| Me | 3-Me-Isothiazol-5-yl | H |
| Me | Isoxazol-5-yl | H |
| Me | 3,5-Me-Isoxazol-2-yl | H |
| Me | 5-Me-Isoxazol-3-yl | H |
| Me | 1-Me-Imidazol-5-yl | H |
| Me | 1-Me-2-Imidazolyl | H |
| Me | 1-Me-4,5-$Cl_2$-Imidazol-2-yl | H |
| Me | 1,5-$Me_2$-2-Cl-Imidazol-4-yl | H |
| Me | 1-Ph-5-Me-1,2,3-Triazol-4-yl | H |
| Me | 4-Me-1,2,3-Thiadiazol-5-yl | H |
| Me | 4-Et-1,2,3-Thiadiazol-5-yl | H |
| Me | 1,2,3-Thiadiazol-5-yl | H |
| Me | 1,2,3-Thiadiazol-4-yl | H |
| Me | Pyridin-2-yl | H |
| Me | Pyridin-3-yl | H |
| Me | Pyridin-4-yl | H |
| Me | 6-Me-Pyridin-3-yl | H |
| Me | 6-Cl-Pyridin-2-yl | H |
| Me | 6-PhO-Pyridin-2-yl | H |
| Me | 2-Cl-Pyridin-4-yl | H |
| Me | 2-F-Pyridin-4-yl | H |
| Me | 2,6-$Cl_2$-Pyridin-4-yl | H |
| Me | 2-MeO-Pyridin-4-yl | H |
| Me | 3,6-$Cl_2$-Pyridin-2-yl | H |
| Me | 2-Cl-6-Me-Pyridin-4-yl | H |
| Me | 3-F-Pyridin-2-yl | H |
| Me | 3-F-Pyridin-4-yl | H |
| Me | 5-$CF_3$-6-PhO-Pyridin-2-yl | H |
| Me | 2,6-$Cl_2$-Pyridin-4-yl | H |
| Me | 4,6-$Cl_2$-Pyridin-2-yl | H |
| Me | 1-Me-4-$CF_3$-2-pyridon-3-yl | H |
| Me | Quinoxalin-2-yl | H |
| Me | 6-Cl-Quinoxalin-2-yl | H |
| Me | 6-F-Quinoxalin-2-yl | H |
| Me | 5-Cl-Quinoxalin-2-yl | H |
| Me | 5-F-Quinoxalin-2-yl | H |
| Me | 1-Me-Indol-3-yl | H |
| Me | Benzothiazol-2-yl | H |
| Me | Quinolin-4-yl | H |
| Me | Pyradin-2-yl | H |
| Me | 3-Cl-Pyradin-2-yl | H |
| Me | 2,4-$Me_2$-Pyrimidin-5-yl | H |
| Me | 4-$CClF_2$-Pyrimidin-5-yl | H |
| Me | Pyrimidin-2-yl | H |
| Me | Pyrimidin-4-yl | H |
| Me | 6-MeS-Pyrimidin-5-yl | H |

| | | |
|---|---|---|
| Me | 6-PhO-Pyrimidin-4-yl | H |
| Me | Benzofuran-2-yl | H |
| Me | Ph | H |
| Me | 2-Cl-Ph | H |
| Me | 3-Cl-Ph | H |
| Me | 4-Cl-Ph | H |
| Me | 2-F-Ph | H |
| Me | 3-F-Ph | H |
| Me | 4-F-Ph | H |
| Me | 2-Me-Ph | H |
| Me | 3-Me-Ph | H |
| Me | 4-Me-Ph | H |
| Me | 2-MeO-Ph | H |
| Me | 3-MeO-Ph | H |
| Me | 4-MeO-Ph | H |
| Me | 4-Br-Ph | H |
| Me | 2,4-$Cl_2$-Ph | H |
| Me | 3,4-$Cl_2$-Ph | H |
| Me | 2,4,6-$Cl_3$-Ph | H |
| Me | 3,4-$(MeO)_2$-Ph | H |
| Me | 2-Cl-4-Me-Ph | H |
| Me | 2-MeO-4-Me-Ph | H |
| Me | 2-Cl-4-i-PrO-Ph | H |
| Me | 3-Cl-4-$PhCH_2O$-Ph | H |
| Me | 2,4-$Me_2$-Ph | H |
| Me | 2,5-$Me_2$-Ph | H |
| Me | 2,6-$F_2$-Ph | H |
| Me | 2,3,4,5,6-$F_5$-Ph | H |
| Me | 4-Et-Ph | H |
| Me | 4-i-Pr-Ph | H |
| Me | 4-n-Bu-Ph | H |
| Me | 4-s-Bu-Ph | H |
| Me | 4-t-Bu-Ph | H |
| Me | 4-(t-$BuCH_2$)-Ph | H |
| Me | 4-Et$(Me)_2$C-Ph | H |
| Me | 4-n-Hex-Ph | H |
| Me | 4-$PhCH_2$-Ph | H |
| Me | 4-(4-F-Ph)$(Me)_2$C-Ph | H |
| Me | 4-$CF_3$-Ph | H |
| Me | 4-i-PrO-Ph | H |
| Me | 4-t-BuO-Ph | H |
| Me | 4-n-HexO-Ph | H |
| Me | 4-$CHF_2O$-Ph | H |
| Me | 4-$CF_3O$-Ph | H |
| Me | 4-MeS-Ph | H |
| Me | 4-s-BuS-Ph | H |
| Me | 4-EtSO-Ph | H |
| Me | 4-$MeSO_2$-Ph | H |
| Me | 4-$EtSO_2$-Ph | H |
| Me | 4-$CHF_2S$-Ph | H |
| Me | 4-$CF_3S$-Ph | H |
| Me | 4-$CF_3SO$-Ph | H |
| Me | 4-$CHF_2SO_2$-Ph | H |
| Me | 4-$CF_3SO_2$-Ph | H |
| Me | 4-CHO-Ph | H |
| Me | 4-$NO_2$-Ph | H |
| Me | 3-CN-Ph | H |
| Me | 4-CN-Ph | H |

| | | |
|---|---|---|
| Me | 4-(Me)$_2$N-Ph | H |
| Me | 4-Me(MeC(O))N-Ph | H |
| Me | 4-PhN(Me)-Ph | H |
| Me | 4-PhCH$_2$(MeCO)N-Ph | H |
| Me | 4-PhCH$_2$O-Ph | H |
| Me | 4-(2-Cl-Ph)CH$_2$O-Ph | H |
| Me | 4-(3-Cl-Ph)CH$_2$O-Ph | H |
| Me | 4-(4-Cl-Ph)CH$_2$O-Ph | H |
| Me | 4-(2-Me-Ph)CH$_2$O-Ph | H |
| Me | 4-(3-Me-Ph)CH$_2$O-Ph | H |
| Me | 4-(4-F-Ph)CH$_2$O-Ph | H |
| Me | 4-(4-Et-Ph)CH$_2$O-Ph | H |
| Me | 4-(2,4-F$_2$-Ph)CH$_2$O-Ph | H |
| Me | 3-(3,4-Cl$_2$-Ph)CH$_2$O-Ph | H |
| Me | 4-(2,5-Me$_2$-Ph)CH$_2$O-Ph | H |
| Me | 4-MeC(O)-Ph | H |
| Me | 4-EtC(O)-Ph | H |
| Me | 4-n-PrC(O)-Ph | H |
| Me | 4-PhC(O)-Ph | H |
| Me | 4-(2-Cl-Ph)C(O)-Ph | H |
| Me | 4-CF$_3$C(O)-Ph | H |
| Me | 4-MeC(O)O-Ph | H |
| Me | 4-EtC(O)O-Ph | H |
| Me | 4-CF$_3$C(O)O-Ph | H |
| Me | 4-PhC(O)O-Ph | H |
| Me | 4-Ph-Ph | H |
| Me | 4-PhO-Ph | H |
| Me | 4-(4-Cl-Ph)O-Ph | H |
| Me | 4-(Pyridin-2-yl)O-Ph | H |
| Me | 2,3-Cl$_2$-Ph | H |
| Me | 3,5-Cl$_2$-Ph | H |
| Me | 2,6-Cl$_2$-Ph | H |
| Me | 2,5-Cl$_2$-Ph | H |
| Me | 2,3-F$_2$-Ph | H |
| Me | 2,5-F$_2$-Ph | H |
| Me | 3,4-F$_2$-Ph | H |
| Me | 3,5-F$_2$-Ph | H |
| Me | 2,4-F$_2$-Ph | H |
| Me | 2-CF$_3$-Ph | H |
| Me | 3-(3-Cl-PhCH$_2$O)-Ph | H |
| Me | 2-F-6-CF$_3$-Ph | H |
| Me | 2-F-6-Cl-Ph | H |
| Me | 2-F-6-Me-Ph | H |
| Me | 2-F-6-MeO-Ph | H |
| Me | 2-F-6-OH-Ph | H |
| Me | 2-F-6-MeS-Ph | H |
| Me | 2-F-5-Cl-Ph | H |
| Me | 2-F-5-CF$_3$-Ph | H |
| Me | 2-F-5-Me-Ph | H |
| Me | 2-F-5-MeO-Ph | H |
| Me | 2-F-5-OH-Ph | H |
| Me | 2-F-5-MeS-Ph | H |
| Me | 2-F-4-Cl-Ph | H |
| Me | 2-F-4-CF$_3$-Ph | H |
| Me | 2-F-4-Me-Ph | H |
| Me | 2-F-4-MeO-Ph | H |
| Me | 2-F-3-Cl-Ph | H |
| Me | 2-F-3-Me-Ph | H |

| | | |
|---|---|---|
| Me | 2-F-3-MeO-Ph | H |
| Me | 3-F-2-Cl-Ph | H |
| Me | 3-F-2-Me-Ph | H |
| Me | 3-F-2-MeO-Ph | H |
| Me | 3-F-4-Cl-Ph | H |
| Me | 3-F-4-Me-Ph | H |
| Me | 3-F-4-MeO-Ph | H |
| Me | 3-F-5-Cl-Ph | H |
| Me | 3-F-5-Me-Ph | H |
| Me | 3-F-5-MeO-Ph | H |
| Me | 3-F-6-Cl-Ph | H |
| Me | 3-F-6-Me-Ph | H |
| Me | 3-F-6-MeO-Ph | H |
| Me | 4-F-2-Cl-Ph | H |
| Me | 4-F-2-Me-Ph | H |
| Me | 4-F-2-MeO-Ph | H |
| Me | 4-F-3-Cl-Ph | H |
| Me | 4-F-3-Me-Ph | H |
| Me | 4-F-3-MeO-Ph | H |
| Me | 4-I-Ph | H |
| Me | 4-MeOC(O)-Ph | H |
| Me | 4-MeNHC(O)-Ph | H |
| Me | 2,6-$Me_2$-Ph | H |
| Me | 2,6-$(MeO)_2$-Ph | H |
| Me | 3-$CF_3$-Ph | H |
| Me | 2-Br-Ph | H |
| Me | 3-Br-Ph | H |
| Me | 4-EtO-Ph | H |
| Me | 2-F-3-$CF_3$-Ph | H |
| Me | 2,3-$Me_2$-Ph | H |
| Me | 3,4-$Me_2$-Ph | H |
| Me | 3,5-$Me_2$-Ph | H |
| Me | 2,3-$(MeO)_2$-Ph | H |
| Me | 2,4-$(MeO)_2$-Ph | H |
| Me | 2,5-$(MeO)_2$-Ph | H |
| Me | 3,5-$(MeO)_2$-Ph | H |
| Me | 2-F-4-EtO-Ph | H |
| Me | 2-F-4-Et-Ph | H |
| Me | 2-F-6-PhS-Ph | H |
| Me | 2-F-6-Ph-Ph | H |
| Me | 3,4-methylenedioxy-Ph | H |
| Me | 3,4-ethylenedioxy-Ph | H |
| Me | 2-F-3-Br-Ph | H |
| Me | 2-F-4-Br-Ph | H |
| Me | 2-F-5-Br-Ph | H |
| Me | 2-F-6-Br-Ph | H |
| Me | 3-F-2-Br-Ph | H |
| Me | 3-F-4-Br-Ph | H |
| Me | 3-F-5-Br-Ph | H |
| Me | 3-F-6-Br-Ph | H |
| Me | 4-F-2-Br-Ph | H |
| Me | 4-F-3-Br-Ph | H |
| Me | 2-Cl-3-Me-Ph | H |
| Me | 2-Cl-4-Me-Ph | H |
| Me | 2-Cl-5-Me-Ph | H |
| Me | 2-Cl-6-Me-Ph | H |
| Me | 3-Cl-2-Me-Ph | H |
| Me | 3-Cl-4-Me-Ph | H |

| | | |
|---|---|---|
| Me | 3-Cl-5-Me-Ph | H |
| Me | 3-Cl-6-Me-Ph | H |
| Me | 4-Cl-2-Me-Ph | H |
| Me | 4-Cl-3-Me-Ph | H |
| Me | 2,6-$F_2$-4-Me-Ph | H |
| Me | 2,6-$F_2$-4-MeO-Ph | H |
| Me | 2,6-$F_2$-4-EtO-Ph | H |
| Me | 2,6-$F_2$-4-Et-Ph | H |
| Me | 2,6-$F_2$-4-Br-Ph | H |
| Me | 2,6-$F_2$-4-Ph-Ph | H |
| Me | 2,6-$F_2$-4-Ph$CH_2$-Ph | H |
| Me | 2,4,6-$Me_3$-Ph | H |
| Me | 4-Hep-Ph | H |
| Me | 4-Oct-Ph | H |
| Me | 4-Non-Ph | H |
| Me | 4-Dec-Ph | H |
| Me | 4-Undec-Ph | H |
| Me | 4-Dodec-Ph | H |
| Me | 2-Cl-4-Hep-Ph | H |
| Me | 2-Cl-4-Oct-Ph | H |
| Me | 2-Cl-4-Non-Ph | H |
| Me | 2-Cl-4-Dec-Ph | H |
| Me | 2-Cl-4-Undec-Ph | H |
| Me | 2-Cl-4-Dodec-Ph | H |
| Me | 3-Cl-4-Hep-Ph | H |
| Me | 3-Cl-4-Oct-Ph | H |
| Me | 3-Cl-4-Non-Ph | H |
| Me | 3-Cl-4-Dec-Ph | H |
| Me | 3-Cl-4-Undec-Ph | H |
| Me | 3-Cl-4-Dodec-Ph | H |
| Me | 2-F-4-Hep-Ph | H |
| Me | 2-F-4-Oct-Ph | H |
| Me | 2-F-4-Non-Ph | H |
| Me | 2-F-4-Dec-Ph | H |
| Me | 2-F-4-Undec-Ph | H |
| Me | 2-F-4-Dodec-Ph | H |
| Me | 3-F-4-Hep-Ph | H |
| Me | 3-F-4-Oct-Ph | H |
| Me | 3-F-4-Non-Ph | H |
| Me | 3-F-4-Dec-Ph | H |
| Me | 3-F-4-Undec-Ph | H |
| Me | 3-F-4-Dodec-Ph | H |
| Me | 2-Cl-3-OMe-Ph | H |
| Me | 2-Cl-4-OMe-Ph | H |
| Me | 2-Cl-5-OMe-Ph | H |
| Me | 2-Cl-6-OMe-Ph | H |
| Me | 3-Cl-2-OMe-Ph | H |
| Me | 3-Cl-4-OMe-Ph | H |
| Me | 3-Cl-5-OMe-Ph | H |
| Me | 3-Cl-6-OMe-Ph | H |
| Me | 4-Cl-2-OMe-Ph | H |
| Me | 4-Cl-3-OMe-Ph | H |
| Me | 2-Me-3-OMe-Ph | H |
| Me | 2-Me-4-OMe-Ph | H |
| Me | 2-Me-5-OMe-Ph | H |
| Me | 2-Me-6-OMe-Ph | H |
| Me | 3-Me-2-OMe-Ph | H |
| Me | 3-Me-4-OMe-Ph | H |

| | | |
|---|---|---|
| Me | 3-Me-5-OMe-Ph | H |
| Me | 3-Me-6-OMe-Ph | H |
| Me | 4-Me-3-OMe-Ph | H |
| Me | 2-$NO_2$-Ph | H |
| Me | Ph | 4-Cl |
| Me | Ph | 5-Cl |
| Me | Ph | 4-$CF_3$ |
| Me | Ph | 4-Me |
| Me | Ph | 5-Me |
| Me | Ph | 6-Me |
| Me | Ph | 4-F |
| Me | Ph | 5-F |
| Me | Ph | 4-MeO |
| Me | 2-Cl-Ph | 4-Cl |
| Me | 2-Cl-Ph | 5-Cl |
| Me | 2-Cl-Ph | 4-$CF_3$ |
| Me | 2-Cl-Ph | 4-Me |
| Me | 2-Cl-Ph | 5-Me |
| Me | 2-Cl-Ph | 4-F |
| Me | 2-Cl-Ph | 5-F |
| Me | 3-Cl-Ph | 4-Cl |
| Me | 3-Cl-Ph | 5-Cl |
| Me | 3-Cl-Ph | 4-$CF_3$ |
| Me | 3-Cl-Ph | 4-Me |
| Me | 3-Cl-Ph | 5-Me |
| Me | 3-Cl-Ph | 4-F |
| Me | 3-Cl-Ph | 5-F |
| Me | 4-Cl-Ph | 4-Cl |
| Me | 4-Cl-Ph | 5-Cl |
| Me | 4-Cl-Ph | 4-$CF_3$ |
| Me | 4-Cl-Ph | 4-Me |
| Me | 4-Cl-Ph | 5-Me |
| Me | 4-Cl-Ph | 4-F |
| Me | 4-Cl-Ph | 5-F |
| Me | 2-Me-Ph | 4-Cl |
| Me | 2-Me-Ph | 5-Cl |
| Me | 2-Me-Ph | 4-$CF_3$ |
| Me | 2-Me-Ph | 4-Me |
| Me | 2-Me-Ph | 5-Me |
| Me | 2-Me-Ph | 4-F |
| Me | 2-Me-Ph | 5-F |
| Me | 3-Me-Ph | 4-Cl |
| Me | 3-Me-Ph | 4-Me |
| Me | 3-Me-Ph | 4-F |
| Me | 3-Me-Ph | 5-F |
| Me | 4-Me-Ph | 4-Cl |
| Me | 4-Me-Ph | 5-Cl |
| Me | 4-Me-Ph | 4-$CF_3$ |
| Me | 4-Me-Ph | 3-Me |
| Me | 4-Me-Ph | 4-Me |
| Me | 4-Me-Ph | 5-Me |
| Me | 4-Me-Ph | 6-Me |
| Me | 4-Me-Ph | 3-F |
| Me | 4-Me-Ph | 4-F |
| Me | 4-Me-Ph | 5-F |
| Me | 4-Me-Ph | 6-F |
| Me | 4-Me-Ph | 3-MeO |
| Me | 4-Me-Ph | 4-MeO |

| | | |
|---|---|---|
| Me | 4-Me-Ph | 5-MeO |
| Me | 4-Me-Ph | 6-MeO |
| Me | 4-Me-Ph | 4,5-$Me_2$ |
| Me | 4-Me-Ph | 4,5-$(MeO)_2$ |
| Me | 4-Me-Ph | 4,5-$Cl_2$ |
| Me | 4-Me-Ph | 5,6-$Cl_2$ |
| Me | 4-Me-Ph | 3,5-$F_2$ |
| Me | 4-Me-Ph | 4,5-$F_2$ |
| Me | 4-Me-Ph | 5,6-$F_2$ |
| Me | 4-Me-Ph | 4-t-Bu |
| Me | 4-Me-Ph | 4-CN |
| Me | 4-Me-Ph | 4-Et |
| Me | 4-Me-Ph | 4-COOMe |
| Me | 4-Me-Ph | 4-COMe |
| Me | 4-Me-Ph | 4-COPh |
| Me | 4-Me-Ph | 4-F-5-Cl |
| Me | 4-Me-Ph | 4-F-5-Me |
| Me | 4-Me-Ph | 4-Me-5-Cl |
| Me | 4-Me-Ph | 5-Cl-6-Me |
| Me | 2-F-Ph | 4-Cl |
| Me | 2-F-Ph | 5-Cl |
| Me | 2-F-Ph | 4-$CF_3$ |
| Me | 2-F-Ph | 4-Me |
| Me | 2-F-Ph | 5-Me |
| Me | 2-F-Ph | 4-F |
| Me | 2-F-Ph | 5-F |
| Me | 2-F-Ph | 4-MeO |
| Me | 3-F-Ph | 4-Cl |
| Me | 3-F-Ph | 4-Me |
| Me | 3-F-Ph | 5-Me |
| Me | 3-F-Ph | 4-F |
| Me | 4-F-Ph | 4-Cl |
| Me | 4-F-Ph | 5-Cl |
| Me | 4-F-Ph | 4-$CF_3$ |
| Me | 4-F-Ph | 4-Me |
| Me | 4-F-Ph | 5-Me |
| Me | 4-F-Ph | 4-F |
| Me | 4-F-Ph | 5-F |
| Me | 4-F-Ph | 4-MeO |
| Me | 2-MeO-Ph | 4-Cl |
| Me | 2-MeO-Ph | 4-$CF_3$ |
| Me | 2-MeO-Ph | 4-Me |
| Me | 2-MeO-Ph | 4-F |
| Me | 3-MeO-Ph | 4-Cl |
| Me | 3-MeO-Ph | 4-$CF_3$ |
| Me | 3-MeO-Ph | 4-Me |
| Me | 3-MeO-Ph | 4-F |
| Me | 4-MeO-Ph | 4-Cl |
| Me | 4-MeO-Ph | 4-$CF_3$ |
| Me | 4-MeO-Ph | 4-Me |
| Me | 4-MeO-Ph | 4-F |
| Me | 4-MeO-Ph | 4-MeO |
| Me | 2-Br-Ph | 4-Cl |
| Me | 2-Br-Ph | 4-$CF_3$ |
| Me | 2-Br-Ph | 4-Me |
| Me | 2-Br-Ph | 5-Me |
| Me | 2-Br-Ph | 4-F |
| Me | 3-Br-Ph | 4-Cl |

| | | |
|---|---|---|
| Me | 3-Br-Ph | 4-$CF_3$ |
| Me | 3-Br-Ph | 4-Me |
| Me | 3-Br-Ph | 4-F |
| Me | 4-Br-Ph | 4-Cl |
| Me | 4-Br-Ph | 4-Me |
| Me | 4-Br-Ph | 5-Me |
| Me | 4-Br-Ph | 4-F |
| Me | 4-Br-Ph | 5-F |
| Me | 4-Br-Ph | 4-MeO |
| Me | 4-Et-Ph | 4-Cl |
| Me | 4-Et-Ph | 4-$CF_3$ |
| Me | 4-Et-Ph | 4-Me |
| Me | 4-Et-Ph | 4-F |
| Me | 4-Pr-Ph | 4-Cl |
| Me | 4-Pr-Ph | 4-Me |
| Me | 4-Pr-Ph | 4-F |
| Me | 4-t-Bu-Ph | 4-Cl |
| Me | 4-t-Bu-Ph | 4-Me |
| Me | 4-t-Bu-Ph | 4-F |
| Me | 4-n-Bu-Ph | 4-Cl |
| Me | 4-n-Bu-Ph | 4-Me |
| Me | 4-n-Bu-Ph | 4-F |
| Me | 4-n-Pen-Ph | 4-Cl |
| Me | 4-n-Pen-Ph | 4-Me |
| Me | 4-n-Pen-Ph | 4-F |
| Me | 4-n-Hex-Ph | 4-Cl |
| Me | 4-n-Hex-Ph | 4-Me |
| Me | 4-n-Hex-Ph | 4-F |
| Me | 2,6-$F_2$-Ph | 4-Cl |
| Me | 2,6-$F_2$-Ph | 5-Cl |
| Me | 2,6-$F_2$-Ph | 4-$CF_3$ |
| Me | 2,6-$F_2$-Ph | 4-Me |
| Me | 2,6-$F_2$-Ph | 5-Me |
| Me | 2,6-$F_2$-Ph | 6-Me |
| Me | 2,6-$F_2$-Ph | 4-F |
| Me | 2,6-$F_2$-Ph | 5-F |
| Me | 2,6-$F_2$-Ph | 4-MeO |
| Me | 2,6-$F_2$-Ph | 3,4-$Me_2$ |
| Me | 2,6-$F_2$-Ph | 3,5-$Me_2$ |
| Me | 2,6-$F_2$-Ph | 3,6-$Me_2$ |
| Me | 2,6-$F_2$-Ph | 4,5-$Me_2$ |
| Me | 2,6-$F_2$-Ph | 4,6-$Me_2$ |
| Me | 2,6-$F_2$-Ph | 5,6-$Me_2$ |
| Me | 2,6-$F_2$-Ph | 3,4-$(MeO)_2$ |
| Me | 2,6-$F_2$-Ph | 3,5-$(MeO)_2$ |
| Me | 2,6-$F_2$-Ph | 3,6-$(MeO)_2$ |
| Me | 2,6-$F_2$-Ph | 4,5-$(MeO)_2$ |
| Me | 2,6-$F_2$-Ph | 4,6-$(MeO)_2$ |
| Me | 2,6-$F_2$-Ph | 5,6-$(MeO)_2$ |
| Me | 2,6-$F_2$-Ph | 3,4-$Cl_2$ |
| Me | 2,6-$F_2$-Ph | 3,5-$Cl_2$ |
| Me | 2,6-$F_2$-Ph | 3,6-$Cl_2$ |
| Me | 2,6-$F_2$-Ph | 4,5-$Cl_2$ |
| Me | 2,6-$F_2$-Ph | 4,6-$Cl_2$ |
| Me | 2,6-$F_2$-Ph | 5,6-$Cl_2$ |
| Me | 2,6-$F_2$-Ph | 3,4-$F_2$ |
| Me | 2,6-$F_2$-Ph | 3,5-$F_2$ |
| Me | 2,6-$F_2$-Ph | 3,6-$F_2$ |

| | | |
|---|---|---|
| Me | 2,6-$F_2$-Ph | 4,5-$F_2$ |
| Me | 2,6-$F_2$-Ph | 4,6-$F_2$ |
| Me | 2,6-$F_2$-Ph | 5,6-$F_2$ |
| Me | 2,6-$F_2$-Ph | 4-t-Bu |
| Me | 2,6-$F_2$-Ph | 4-CN |
| Me | 2,6-$F_2$-Ph | 4-Et |
| Me | 2,6-$F_2$-Ph | 4-COOMe |
| Me | 2,6-$F_2$-Ph | 4-COOEt |
| Me | 2,6-$F_2$-Ph | 4-COMe |
| Me | 2,6-$F_2$-Ph | 4-COPh |
| Me | 2,6-$F_2$-Ph | 4-F-5-Cl |
| Me | 2,6-$F_2$-Ph | 4-F-5-Me |
| Me | 2,6-$F_2$-Ph | 4-Me-5-Cl |
| Me | 2,6-$F_2$-Ph | 5-Cl-6-Me |
| Me | 2,5-$F_2$-Ph | 4-Cl |
| Me | 2,5-$F_2$-Ph | 5-Cl |
| Me | 2,5-$F_2$-Ph | 4-$CF_3$ |
| Me | 2,5-$F_2$-Ph | 4-Me |
| Me | 2,5-$F_2$-Ph | 5-Me |
| Me | 2,5-$F_2$-Ph | 4-F |
| Me | 2,5-$F_2$-Ph | 5-F |
| Me | 2,5-$F_2$-Ph | 4-MeO |
| Me | 2,4-$F_2$-Ph | 3-Cl |
| Me | 2,4-$F_2$-Ph | 4-Cl |
| Me | 2,4-$F_2$-Ph | 5-Cl |
| Me | 2,4-$F_2$-Ph | 6-Cl |
| Me | 2,4-$F_2$-Ph | 3-$CF_3$ |
| Me | 2,4-$F_2$-Ph | 4-$CF_3$ |
| Me | 2,4-$F_2$-Ph | 5-$CF_3$ |
| Me | 2,4-$F_2$-Ph | 3-Me |
| Me | 2,4-$F_2$-Ph | 4-Me |
| Me | 2,4-$F_2$-Ph | 5-Me |
| Me | 2,4-$F_2$-Ph | 6-Me |
| Me | 2,4-$F_2$-Ph | 3-F |
| Me | 2,4-$F_2$-Ph | 4-F |
| Me | 2,4-$F_2$-Ph | 5-F |
| Me | 2,4-$F_2$-Ph | 6-F |
| Me | 2,4-$F_2$-Ph | 3-MeO |
| Me | 2,4-$F_2$-Ph | 4-MeO |
| Me | 2,4-$F_2$-Ph | 5-MeO |
| Me | 2,4-$F_2$-Ph | 6-MeO |
| Me | 2,3-$F_2$-Ph | 3-Cl |
| Me | 2,3-$F_2$-Ph | 4-Cl |
| Me | 2,3-$F_2$-Ph | 5-Cl |
| Me | 2,3-$F_2$-Ph | 6-Cl |
| Me | 2,3-$F_2$-Ph | 3-$CF_3$ |
| Me | 2,3-$F_2$-Ph | 4-$CF_3$ |
| Me | 2,3-$F_2$-Ph | 5-$CF_3$ |
| Me | 2,3-$F_2$-Ph | 3-Me |
| Me | 2,3-$F_2$-Ph | 4-Me |
| Me | 2,3-$F_2$-Ph | 5-Me |
| Me | 2,3-$F_2$-Ph | 6-Me |
| Me | 2,3-$F_2$-Ph | 3-F |
| Me | 2,3-$F_2$-Ph | 4-F |
| Me | 2,3-$F_2$-Ph | 5-F |
| Me | 2,3-$F_2$-Ph | 6-F |
| Me | 2,3-$F_2$-Ph | 3-MeO |
| Me | 2,3-$F_2$-Ph | 4-MeO |

| | | |
|---|---|---|
| Me | 2,3-$F_2$-Ph | 5-MeO |
| Me | 2,3-$F_2$-Ph | 6-MeO |
| Me | 3,4-$F_2$-Ph | 3-Cl |
| Me | 3,4-$F_2$-Ph | 4-Cl |
| Me | 3,4-$F_2$-Ph | 5-Cl |
| Me | 3,4-$F_2$-Ph | 6-Cl |
| Me | 3,4-$F_2$-Ph | 3-$CF_3$ |
| Me | 3,4-$F_2$-Ph | 4-$CF_3$ |
| Me | 3,4-$F_2$-Ph | 5-$CF_3$ |
| Me | 3,4-$F_2$-Ph | 3-Me |
| Me | 3,4-$F_2$-Ph | 4-Me |
| Me | 3,4-$F_2$-Ph | 5-Me |
| Me | 3,4-$F_2$-Ph | 6-Me |
| Me | 3,4-$F_2$-Ph | 3-F |
| Me | 3,4-$F_2$-Ph | 4-F |
| Me | 3,4-$F_2$-Ph | 5-F |
| Me | 3,4-$F_2$-Ph | 6-F |
| Me | 3,4-$F_2$-Ph | 3-MeO |
| Me | 3,4-$F_2$-Ph | 4-MeO |
| Me | 3,4-$F_2$-Ph | 5-MeO |
| Me | 3,4-$F_2$-Ph | 6-MeO |
| Me | 3,5-$F_2$-Ph | 3-Cl |
| Me | 3,5-$F_2$-Ph | 4-Cl |
| Me | 3,5-$F_2$-Ph | 5-Cl |
| Me | 3,5-$F_2$-Ph | 6-Cl |
| Me | 3,5-$F_2$-Ph | 3-$CF_3$ |
| Me | 3,5-$F_2$-Ph | 4-$CF_3$ |
| Me | 3,5-$F_2$-Ph | 5-$CF_3$ |
| Me | 3,5-$F_2$-Ph | 3-Me |
| Me | 3,5-$F_2$-Ph | 4-Me |
| Me | 3,5-$F_2$-Ph | 5-Me |
| Me | 3,5-$F_2$-Ph | 6-Me |
| Me | 3,5-$F_2$-Ph | 3-F |
| Me | 3,5-$F_2$-Ph | 4-F |
| Me | 3,5-$F_2$-Ph | 5-F |
| Me | 3,5-$F_2$-Ph | 6-F |
| Me | 3,5-$F_2$-Ph | 3-MeO |
| Me | 3,5-$F_2$-Ph | 4-MeO |
| Me | 3,5-$F_2$-Ph | 5-MeO |
| Me | 3,5-$F_2$-Ph | 6-MeO |
| Me | 2-F-4-Me-Ph | 3-Cl |
| Me | 2-F-4-Me-Ph | 4-Cl |
| Me | 2-F-4-Me-Ph | 5-Cl |
| Me | 2-F-4-Me-Ph | 6-Cl |
| Me | 2-F-4-Me-Ph | 3-$CF_3$ |
| Me | 2-F-4-Me-Ph | 4-$CF_3$ |
| Me | 2-F-4-Me-Ph | 5-$CF_3$ |
| Me | 2-F-4-Me-Ph | 3-Me |
| Me | 2-F-4-Me-Ph | 4-Me |
| Me | 2-F-4-Me-Ph | 5-Me |
| Me | 2-F-4-Me-Ph | 6-Me |
| Me | 2-F-4-Me-Ph | 3-F |
| Me | 2-F-4-Me-Ph | 4-F |
| Me | 2-F-4-Me-Ph | 5-F |
| Me | 2-F-4-Me-Ph | 6-F |
| Me | 2-F-4-Me-Ph | 3-MeO |
| Me | 2-F-4-Me-Ph | 4-MeO |
| Me | 2-F-4-Me-Ph | 5-MeO |

| | | |
|---|---|---|
| Me | 2-F-4-Me-Ph | 6-MeO |
| Me | 2-F-4-Me-Ph | 3,4-$Me_2$ |
| Me | 2-F-4-Me-Ph | 3,5-$Me_2$ |
| Me | 2-F-4-Me-Ph | 3,6-$Me_2$ |
| Me | 2-F-4-Me-Ph | 4,5-$Me_2$ |
| Me | 2-F-4-Me-Ph | 4,6-$Me_2$ |
| Me | 2-F-4-Me-Ph | 5,6-$Me_2$ |
| Me | 2-F-4-Me-Ph | 3,4-$(MeO)_2$ |
| Me | 2-F-4-Me-Ph | 3,5-$(MeO)_2$ |
| Me | 2-F-4-Me-Ph | 3,6-$(MeO)_2$ |
| Me | 2-F-4-Me-Ph | 4,5-$(MeO)_2$ |
| Me | 2-F-4-Me-Ph | 4,6-$(MeO)_2$ |
| Me | 2-F-4-Me-Ph | 5,6-$(MeO)_2$ |
| Me | 2-F-4-Me-Ph | 3,4-$Cl_2$ |
| Me | 2-F-4-Me-Ph | 3,5-$Cl_2$ |
| Me | 2-F-4-Me-Ph | 3,6-$Cl_2$ |
| Me | 2-F-4-Me-Ph | 4,5-$Cl_2$ |
| Me | 2-F-4-Me-Ph | 4,6-$Cl_2$ |
| Me | 2-F-4-Me-Ph | 5,6-$Cl_2$ |
| Me | 2-F-4-Me-Ph | 3,4-$F_2$ |
| Me | 2-F-4-Me-Ph | 3,5-$F_2$ |
| Me | 2-F-4-Me-Ph | 3,6-$F_2$ |
| Me | 2-F-4-Me-Ph | 4,5-$F_2$ |
| Me | 2-F-4-Me-Ph | 4,6-$F_2$ |
| Me | 2-F-4-Me-Ph | 5,6-$F_2$ |
| Me | 2-F-4-Me-Ph | 4-t-Bu |
| Me | 2-F-4-Me-Ph | 4-CN |
| Me | 2-F-4-Me-Ph | 4-Et |
| Me | 2-F-4-Me-Ph | 4-COOMe |
| Me | 2-F-4-Me-Ph | 4-COOEt |
| Me | 2-F-4-Me-Ph | 4-COMe |
| Me | 2-F-4-Me-Ph | 4-COPh |
| Me | 2-F-4-Me-Ph | 4-F-5-Cl |
| Me | 2-F-4-Me-Ph | 4-F-5-Me |
| Me | 2-F-4-Me-Ph | 4-Me-5-Cl |
| Me | 2-F-4-Me-Ph | 5-Cl-6-Me |
| Me | 2-F-4-Et-Ph | 4-Cl |
| Me | 2-F-4-Et-Ph | 5-Cl |
| Me | 2-F-4-Et-Ph | 4-$CF_3$ |
| Me | 2-F-4-Et-Ph | 4-Me |
| Me | 2-F-4-Et-Ph | 5-Me |
| Me | 2-F-4-Et-Ph | 6-Me |
| Me | 2-F-4-Et-Ph | 4-F |
| Me | 2-F-4-Et-Ph | 5-F |
| Me | 2-F-4-Et-Ph | 6-F |
| Me | 2-F-4-Et-Ph | 4-MeO |
| Me | 2-F-6-MeO-Ph | 3-Cl |
| Me | 2-F-6-MeO-Ph | 4-Cl |
| Me | 2-F-6-MeO-Ph | 5-Cl |
| Me | 2-F-6-MeO-Ph | 4-Me |
| Me | 2-F-6-MeO-Ph | 5-Me |
| Me | 2-F-6-MeO-Ph | 4-F |
| Me | 2,6-$Cl_2$-Ph | 4-Cl |
| Me | 2,6-$Cl_2$-Ph | 4-Me |
| Me | 2,6-$Cl_2$-Ph | 5-Me |
| Me | 2,6-$Cl_2$-Ph | 4-F |
| Me | 2,6-$Cl_2$-Ph | 5-F |
| Me | 2,5-$Cl_2$-Ph | 4-Cl |

| | | |
|---|---|---|
| Me | 2,5-$Cl_2$-Ph | 4-Me |
| Me | 2,5-$Cl_2$-Ph | 5-Me |
| Me | 2,5-$Cl_2$-Ph | 4-F |
| Me | 2,5-$Cl_2$-Ph | 5-F |
| Me | 2,4-$Cl_2$-Ph | 4-Cl |
| Me | 2,4-$Cl_2$-Ph | 4-Me |
| Me | 2,4-$Cl_2$-Ph | 5-Me |
| Me | 2,4-$Cl_2$-Ph | 4-F |
| Me | 2,4-$Cl_2$-Ph | 5-F |
| Me | 2,3-$Cl_2$-Ph | 4-Cl |
| Me | 2,3-$Cl_2$-Ph | 4-Me |
| Me | 2,3-$Cl_2$-Ph | 5-Me |
| Me | 2,3-$Cl_2$-Ph | 4-F |
| Me | 2,3-$Cl_2$-Ph | 5-F |
| Me | 3,4-$Cl_2$-Ph | 4-Cl |
| Me | 3,4-$Cl_2$-Ph | 4-Me |
| Me | 3,4-$Cl_2$-Ph | 5-Me |
| Me | 3,4-$Cl_2$-Ph | 4-F |
| Me | 3,4-$Cl_2$-Ph | 5-F |
| Me | 3,5-$Cl_2$-Ph | 4-Cl |
| Me | 3,5-$Cl_2$-Ph | 4-Me |
| Me | 3,5-$Cl_2$-Ph | 5-Me |
| Me | 3,5-$Cl_2$-Ph | 4-F |
| Me | 3,5-$Cl_2$-Ph | 5-F |
| Me | 2,6-$Me_2$-Ph | 4-Cl |
| Me | 2,6-$Me_2$-Ph | 4-Me |
| Me | 2,6-$Me_2$-Ph | 5-Me |
| Me | 2,6-$Me_2$-Ph | 4-F |
| Me | 2,6-$Me_2$-Ph | 5-F |
| Me | 2,5-$Me_2$-Ph | 4-Cl |
| Me | 2,5-$Me_2$-Ph | 4-Me |
| Me | 2,5-$Me_2$-Ph | 4-F |
| Me | 2,5-$Me_2$-Ph | 5-F |
| Me | 2,4-$Me_2$-Ph | 4-Cl |
| Me | 2,4-$Me_2$-Ph | 4-Me |
| Me | 2,4-$Me_2$-Ph | 5-Me |
| Me | 2,4-$Me_2$-Ph | 4-F |
| Me | 2,4-$Me_2$-Ph | 5-F |
| Me | 2,3-$Me_2$-Ph | 4-Cl |
| Me | 2,3-$Me_2$-Ph | 4-Me |
| Me | 2,3-$Me_2$-Ph | 5-Me |
| Me | 2,3-$Me_2$-Ph | 4-F |
| Me | 2,3-$Me_2$-Ph | 5-F |
| Me | 3,4-$Me_2$-Ph | 4-Cl |
| Me | 3,4-$Me_2$-Ph | 4-Me |
| Me | 3,4-$Me_2$-Ph | 5-Me |
| Me | 3,4-$Me_2$-Ph | 4-F |
| Me | 3,5-$Me_2$-Ph | 4-Cl |
| Me | 3,5-$Me_2$-Ph | 4-Me |
| Me | 3,5-$Me_2$-Ph | 4-F |
| Me | 2,6-$F_2$-4-Me-Ph | 4-Cl |
| Me | 2,6-$F_2$-4-Me-Ph | 5-Cl |
| Me | 2,6-$F_2$-4-Me-Ph | 4-$CF_3$ |
| Me | 2,6-$F_2$-4-Me-Ph | 5-$CF_3$ |
| Me | 2,6-$F_2$-4-Me-Ph | 4-Me |
| Me | 2,6-$F_2$-4-Me-Ph | 5-Me |
| Me | 2,6-$F_2$-4-Me-Ph | 4-F |
| Me | 2,6-$F_2$-4-Me-Ph | 5-F |

| | | |
|---|---|---|
| Me | 2,6-$F_2$-4-Me-Ph | 4-MeO |
| Me | 2,6-$F_2$-4-Et-Ph | 4-Me |
| Et | Et | H |
| Et | n-Pr | H |
| Et | i-Pr | H |
| Et | n-Bu | H |
| Et | s-Bu | H |
| Et | t-Bu | H |
| Et | $CF_3$ | H |
| Et | $CF_2CF_3$ | H |
| Et | 1-Naphthyl | H |
| Et | 2-Naphthyl | H |
| Et | 1-Me-3-Cl-Pyrazol-4-yl | H |
| Et | 1-Me-3-Cl-Pyrazol-5-yl | H |
| Et | 1-Me-5-Cl-Pyrazol-3-yl | H |
| Et | 1-Me-5-Cl-Pyrazol-4-yl | H |
| Et | 1-Me-4-MeOOC-Pyrazol-5-yl | H |
| Et | 6-Cl-Pyridin-2-yl | H |
| Et | 6-PhO-Pyridin-2-yl | H |
| Et | 6-Cl-Quinoxalin-2-yl | H |
| Et | 6-F-Quinoxalin-2-yl | H |
| Et | 4-$CClF_2$-Pyrimidin-5-yl | H |
| Et | Ph | H |
| Et | 2-Cl-Ph | H |
| Et | 4-Cl-Ph | H |
| Et | 2-F-Ph | H |
| Et | 4-F-Ph | H |
| Et | 2-Me-Ph | H |
| Et | 3-Me-Ph | H |
| Et | 4-Me-Ph | H |
| Et | 2-MeO-Ph | H |
| Et | 3-MeO-Ph | H |
| Et | 4-MeO-Ph | H |
| Et | 4-Br-Ph | H |
| Et | 2,6-$F_2$-Ph | H |
| Et | 4-$CF_3$-Ph | H |
| Et | 4-Ph-Ph | H |
| Et | 4-PhO-Ph | H |
| Et | 2,3-$F_2$-Ph | H |
| Et | 2,5-$F_2$-Ph | H |
| Et | 3,4-$F_2$-Ph | H |
| Et | 3,5-$F_2$-Ph | H |
| Et | 2,4-$F_2$-Ph | H |
| Et | 2-F-6-Cl-Ph | H |
| Et | 2-F-6-MeO-Ph | H |
| Et | 2-F-5-Cl-Ph | H |
| Et | 2-F-5-Me-Ph | H |
| Et | 2-F-5-MeO-Ph | H |
| Et | 2-F-5-OH-Ph | H |
| Et | 2-F-5-MeS-Ph | H |
| Et | 2-F-4-Cl-Ph | H |
| Et | 2-F-4-Me-Ph | H |
| Et | 2-F-4-MeO-Ph | H |
| Et | 2-F-3-Cl-Ph | H |
| Et | 2-F-3-Me-Ph | H |
| Et | 2-F-3-MeO-Ph | H |
| Et | 3-F-2-Cl-Ph | H |
| Et | 3-F-2-Me-Ph | H |

| | | |
|---|---|---|
| Et | 3-F-2-MeO-Ph | H |
| Et | 3-F-4-Cl-Ph | H |
| Et | 3-F-4-Me-Ph | H |
| Et | 3-F-4-MeO-Ph | H |
| Et | 3-F-5-Cl-Ph | H |
| Et | 3-F-5-Me-Ph | H |
| Et | 3-F-5-MeO-Ph | H |
| Et | 3-F-6-Cl-Ph | H |
| Et | 3-F-6-Me-Ph | H |
| Et | 3-F-6-MeO-Ph | H |
| Et | 4-F-2-Cl-Ph | H |
| Et | 4-F-2-Me-Ph | H |
| Et | 4-F-2-MeO-Ph | H |
| Et | 4-F-3-Cl-Ph | H |
| Et | 4-F-3-Me-Ph | H |
| Et | 4-F-3-MeO-Ph | H |
| Et | 2,6-$(MeO)_2$-Ph | H |
| Et | 2-Br-Ph | H |
| Et | 3-Br-Ph | H |
| Et | 4-EtO-Ph | H |
| Et | 2,3-$Me_2$-Ph | H |
| Et | 3,4-$Me_2$-Ph | H |
| Et | 3,5-$Me_2$-Ph | H |
| Et | 2-Cl-3-Me-Ph | H |
| Et | 2-Cl-4-Me-Ph | H |
| Et | 2-Cl-5-Me-Ph | H |
| Et | 2-Cl-6-Me-Ph | H |
| Et | 3-Cl-2-Me-Ph | H |
| Et | 3-Cl-4-Me-Ph | H |
| Et | 3-Cl-5-Me-Ph | H |
| Et | 3-Cl-6-Me-Ph | H |
| Et | 4-Cl-2-Me-Ph | H |
| Et | 4-Cl-3-Me-Ph | H |
| Et | 2,4,6-$Me_3$-Ph | H |
| Et | Ph | 4-Cl |
| Et | Ph | 4-$CF_3$ |
| Et | Ph | 4-Me |
| Et | Ph | 6-Me |
| Et | Ph | 4-F |
| Et | 2-Cl-Ph | 4-Cl |
| Et | 2-Cl-Ph | 4-$CF_3$ |
| Et | 2-Cl-Ph | 4-Me |
| Et | 2-Cl-Ph | 5-Me |
| Et | 2-Cl-Ph | 4-F |
| Et | 2-Cl-Ph | 5-F |
| Et | 4-Cl-Ph | 4-Cl |
| Et | 4-Cl-Ph | 5-Cl |
| Et | 4-Cl-Ph | 4-$CF_3$ |
| Et | 4-Cl-Ph | 4-Me |
| Et | 4-Cl-Ph | 5-Me |
| Et | 4-Cl-Ph | 4-F |
| Et | 4-Cl-Ph | 5-F |
| Et | 2-Me-Ph | 4-Cl |
| Et | 2-Me-Ph | 5-Cl |
| Et | 2-Me-Ph | 4-Me |
| Et | 2-Me-Ph | 5-Me |
| Et | 2-Me-Ph | 4-F |
| Et | 3-Me-Ph | 4-Cl |

| | | |
|---|---|---|
| Et | 3-Me-Ph | 4-Me |
| Et | 3-Me-Ph | 4-F |
| Et | 3-Me-Ph | 5-F |
| Et | 4-Me-Ph | 4-Cl |
| Et | 4-Me-Ph | 5-Cl |
| Et | 4-Me-Ph | 4-$CF_3$ |
| Et | 4-Me-Ph | 3-Me |
| Et | 4-Me-Ph | 4-Me |
| Et | 4-Me-Ph | 5-Me |
| Et | 4-Me-Ph | 6-Me |
| Et | 4-Me-Ph | 3-F |
| Et | 4-Me-Ph | 4-F |
| Et | 4-Me-Ph | 5-F |
| Et | 4-Me-Ph | 6-F |
| Et | 2-F-Ph | 4-Cl |
| Et | 2-F-Ph | 5-Cl |
| Et | 2-F-Ph | 4-$CF_3$ |
| Et | 2-F-Ph | 4-Me |
| Et | 2-F-Ph | 5-Me |
| Et | 2-F-Ph | 4-F |
| Et | 2-F-Ph | 5-F |
| Et | 3-F-Ph | 4-Cl |
| Et | 3-F-Ph | 4-Me |
| Et | 3-F-Ph | 5-Me |
| Et | 3-F-Ph | 4-F |
| Et | 4-F-Ph | 4-Cl |
| Et | 4-F-Ph | 5-Cl |
| Et | 4-F-Ph | 4-$CF_3$ |
| Et | 4-F-Ph | 4-Me |
| Et | 4-F-Ph | 5-Me |
| Et | 4-F-Ph | 4-F |
| Et | 4-F-Ph | 5-F |
| Et | 2-MeO-Ph | 4-Me |
| Et | 2-MeO-Ph | 4-F |
| Et | 3-MeO-Ph | 4-Me |
| Et | 3-MeO-Ph | 4-F |
| Et | 4-MeO-Ph | 4-Me |
| Et | 4-MeO-Ph | 4-F |
| Et | 2-Br-Ph | 4-Cl |
| Et | 2-Br-Ph | 4-$CF_3$ |
| Et | 2-Br-Ph | 4-Me |
| Et | 2-Br-Ph | 5-Me |
| Et | 2-Br-Ph | 4-F |
| Et | 3-Br-Ph | 4-Me |
| Et | 3-Br-Ph | 4-F |
| Et | 4-Br-Ph | 4-Cl |
| Et | 4-Br-Ph | 4-Me |
| Et | 4-Br-Ph | 5-Me |
| Et | 4-Br-Ph | 4-F |
| Et | 4-Br-Ph | 5-F |
| Et | 4-Br-Ph | 4-MeO |
| Et | 4-Et-Ph | 4-Me |
| Et | 4-Et-Ph | 4-F |
| Et | 4-Pr-Ph | 4-Cl |
| Et | 4-Pr-Ph | 4-Me |
| Et | 4-Pr-Ph | 4-F |
| Et | 4-t-Bu-Ph | 4-Cl |
| Et | 4-t-Bu-Ph | 4-Me |

| | | |
|---|---|---|
| Et | 4-t-Bu-Ph | 4-F |
| Et | 2,6-$F_2$-Ph | 4-Cl |
| Et | 2,6-$F_2$-Ph | 5-Cl |
| Et | 2,6-$F_2$-Ph | 4-$CF_3$ |
| Et | 2,6-$F_2$-Ph | 4-Me |
| Et | 2,6-$F_2$-Ph | 5-Me |
| Et | 2,6-$F_2$-Ph | 6-Me |
| Et | 2,6-$F_2$-Ph | 4-F |
| Et | 2,6-$F_2$-Ph | 5-F |
| Et | 2,6-$F_2$-Ph | 4-MeO |
| Et | 2,5-$F_2$-Ph | 4-Cl |
| Et | 2,5-$F_2$-Ph | 5-Cl |
| Et | 2,5-$F_2$-Ph | 4-Me |
| Et | 2,5-$F_2$-Ph | 5-Me |
| Et | 2,5-$F_2$-Ph | 4-F |
| Et | 2,5-$F_2$-Ph | 5-F |
| Et | 2,4-$F_2$-Ph | 4-Cl |
| Et | 2,4-$F_2$-Ph | 5-Cl |
| Et | 2,4-$F_2$-Ph | 4-Me |
| Et | 2,4-$F_2$-Ph | 5-Me |
| Et | 2,4-$F_2$-Ph | 6-Me |
| Et | 2,4-$F_2$-Ph | 4-F |
| Et | 2,4-$F_2$-Ph | 5-F |
| Et | 2,4-$F_2$-Ph | 6-F |
| Et | 2,3-$F_2$-Ph | 4-Cl |
| Et | 2,3-$F_2$-Ph | 5-Cl |
| Et | 2,3-$F_2$-Ph | 4-Me |
| Et | 2,3-$F_2$-Ph | 5-Me |
| Et | 2,3-$F_2$-Ph | 6-Me |
| Et | 2,3-$F_2$-Ph | 4-F |
| Et | 2,3-$F_2$-Ph | 5-F |
| Et | 2,3-$F_2$-Ph | 6-F |
| Et | 3,4-$F_2$-Ph | 4-Cl |
| Et | 3,4-$F_2$-Ph | 5-Cl |
| Et | 3,4-$F_2$-Ph | 4-Me |
| Et | 3,4-$F_2$-Ph | 5-Me |
| Et | 3,4-$F_2$-Ph | 6-Me |
| Et | 3,4-$F_2$-Ph | 4-F |
| Et | 3,4-$F_2$-Ph | 5-F |
| Et | 3,5-$F_2$-Ph | 4-Cl |
| Et | 3,5-$F_2$-Ph | 5-Cl |
| Et | 3,5-$F_2$-Ph | 4-Me |
| Et | 3,5-$F_2$-Ph | 5-Me |
| Et | 3,5-$F_2$-Ph | 4-F |
| Et | 3,5-$F_2$-Ph | 5-F |
| Et | 2-F-4-Me-Ph | 4-Cl |
| Et | 2-F-4-Me-Ph | 5-Cl |
| Et | 2-F-4-Me-Ph | 6-Cl |
| Et | 2-F-4-Me-Ph | 4-Me |
| Et | 2-F-4-Me-Ph | 5-Me |
| Et | 2-F-4-Me-Ph | 6-Me |
| Et | 2-F-4-Me-Ph | 4-F |
| Et | 2-F-4-Me-Ph | 5-F |
| Et | 2-F-6-MeO-Ph | 4-Cl |
| Et | 2-F-6-MeO-Ph | 5-Cl |
| Et | 2-F-6-MeO-Ph | 4-Me |
| Et | 2-F-6-MeO-Ph | 5-Me |
| Et | 2-F-6-MeO-Ph | 4-F |

| | | |
|---|---|---|
| Et | 2,6-$Cl_2$-Ph | 4-Cl |
| Et | 2,6-$Cl_2$-Ph | 4-Me |
| Et | 2,6-$Cl_2$-Ph | 4-F |
| Et | 2,5-$Cl_2$-Ph | 4-Cl |
| Et | 2,5-$Cl_2$-Ph | 4-Me |
| Et | 2,5-$Cl_2$-Ph | 4-F |
| Et | 2,4-$Cl_2$-Ph | 4-Cl |
| Et | 2,4-$Cl_2$-Ph | 4-Me |
| Et | 2,4-$Cl_2$-Ph | 4-F |
| Et | 2,4-$Me_2$-Ph | 4-Cl |
| Et | 2,4-$Me_2$-Ph | 4-Me |
| Et | 2,4-$Me_2$-Ph | 5-Me |
| Et | 2,4-$Me_2$-Ph | 4-F |
| Et | 2,4-$Me_2$-Ph | 5-F |
| Et | 3,4-$Me_2$-Ph | 4-Cl |
| Et | 3,4-$Me_2$-Ph | 4-Me |
| Et | 3,4-$Me_2$-Ph | 5-Me |
| Et | 3,4-$Me_2$-Ph | 4-F |
| Et | 2,6-$F_2$-4-Me-Ph | 4-Cl |
| Et | 2,6-$F_2$-4-Me-Ph | 5-Cl |
| Et | 2,6-$F_2$-4-Me-Ph | 4-Me |
| Et | 2,6-$F_2$-4-Me-Ph | 5-Me |
| Et | 2,6-$F_2$-4-Me-Ph | 4-F |
| Et | 2,6-$F_2$-4-Me-Ph | 5-F |
| Pr | Et | H |
| Pr | n-Pr | H |
| Pr | i-Pr | H |
| Pr | s-Bu | H |
| Pr | t-Bu | H |
| Pr | $CF_3$ | H |
| Pr | $CF_2CF_3$ | H |
| Pr | 1-Me-3-Cl-Pyrazol-4-yl | H |
| Pr | 1-Me-5-Cl-Pyrazol-3-yl | H |
| Pr | 1-Me-5-Cl-Pyrazol-4-yl | H |
| Pr | Ph | H |
| Pr | 2-Cl-Ph | H |
| Pr | 4-Cl-Ph | H |
| Pr | 2-F-Ph | H |
| Pr | 4-F-Ph | H |
| Pr | 2-Me-Ph | H |
| Pr | 4-Me-Ph | H |
| Pr | 4-Br-Ph | H |
| Pr | 2,6-$F_2$-Ph | H |
| Pr | 2,3-$F_2$-Ph | H |
| Pr | 2,5-$F_2$-Ph | H |
| Pr | 3,4-$F_2$-Ph | H |
| Pr | 3,5-$F_2$-Ph | H |
| Pr | 2,4-$F_2$-Ph | H |
| Pr | 2-Br-Ph | H |
| Pr | Ph | 4-Cl |
| Pr | Ph | 4-$CF_3$ |
| Pr | Ph | 4-Me |
| Pr | Ph | 6-Me |
| Pr | Ph | 4-F |
| Pr | 2-Cl-Ph | 4-Cl |
| Pr | 2-Cl-Ph | 4-$CF_3$ |
| Pr | 2-Cl-Ph | 4-Me |
| Pr | 2-Cl-Ph | 5-Me |

| | | |
|---|---|---|
| Pr | 2-Cl-Ph | 4-F |
| Pr | 4-Cl-Ph | 4-Cl |
| Pr | 4-Cl-Ph | 4-$CF_3$ |
| Pr | 4-Cl-Ph | 4-Me |
| Pr | 4-Cl-Ph | 5-Me |
| Pr | 4-Cl-Ph | 4-F |
| Pr | 2-Me-Ph | 4-Cl |
| Pr | 2-Me-Ph | 4-Me |
| Pr | 2-Me-Ph | 5-Me |
| Pr | 2-Me-Ph | 4-F |
| Pr | 4-Me-Ph | 4-Cl |
| Pr | 4-Me-Ph | 4-Me |
| Pr | 4-Me-Ph | 5-Me |
| Pr | 4-Me-Ph | 4-F |
| Pr | 4-Me-Ph | 5-F |
| Pr | 2-F-Ph | 4-Cl |
| Pr | 2-F-Ph | 5-Cl |
| Pr | 2-F-Ph | 4-Me |
| Pr | 2-F-Ph | 4-F |
| Pr | 4-F-Ph | 4-Cl |
| Pr | 4-F-Ph | 4-Me |
| Pr | 4-F-Ph | 5-Me |
| Pr | 4-F-Ph | 4-F |
| Pr | 2,6-$F_2$-Ph | 4-Cl |
| Pr | 2,6-$F_2$-Ph | 4-Me |
| Pr | 2,6-$F_2$-Ph | 5-Me |
| Pr | 2,6-$F_2$-Ph | 4-F |
| Pr | 2,5-$F_2$-Ph | 4-Cl |
| Pr | 2,5-$F_2$-Ph | 5-Cl |
| Pr | 2,5-$F_2$-Ph | 4-Me |
| Pr | 2,5-$F_2$-Ph | 5-Me |
| Pr | 2,5-$F_2$-Ph | 4-F |
| Pr | 2,5-$F_2$-Ph | 5-F |
| Pr | 3,4-$F_2$-Ph | 4-Cl |
| Pr | 3,4-$F_2$-Ph | 5-Cl |
| Pr | 2-F-4-Me-Ph | 4-Cl |
| Pr | 2-F-4-Me-Ph | 4-Me |
| Pr | 2-F-4-Me-Ph | 4-F |
| Br | n-Pr | H |
| Br | i-Pr | H |
| Br | s-Bu | H |
| Br | t-Bu | H |
| Br | $CF_3$ | H |
| Br | $CF_2CF_3$ | H |
| Br | 1-Me-3-Cl-Pyrazol-4-yl | H |
| Br | 1-Me-5-Cl-Pyrazol-3-yl | H |
| Br | 1-Me-5-Cl-Pyrazol-4-yl | H |
| Br | Ph | H |
| Br | 2-Cl-Ph | H |
| Br | 4-Cl-Ph | H |
| Br | 2-F-Ph | H |
| Br | 4-F-Ph | H |
| Br | 2-Me-Ph | H |
| Br | 4-Me-Ph | H |
| Br | 4-Br-Ph | H |
| Br | 2,6-$F_2$-Ph | H |
| Br | 2,3-$F_2$-Ph | H |
| Br | 2,5-$F_2$-Ph | H |

| | | |
|---|---|---|
| Br | 3,4-$F_2$-Ph | H |
| Br | 3,5-$F_2$-Ph | H |
| Br | 2,4-$F_2$-Ph | H |
| Br | 2-Br-Ph | H |
| Br | Ph | 4-Cl |
| Br | Ph | 4-$CF_3$ |
| Br | Ph | 4-Me |
| Br | Ph | 6-Me |
| Br | Ph | 4-F |
| Br | 2-Cl-Ph | 4-Cl |
| Br | 2-Cl-Ph | 4-$CF_3$ |
| Br | 2-Cl-Ph | 4-Me |
| Br | 2-Cl-Ph | 5-Me |
| Br | 2-Cl-Ph | 4-F |
| Br | 4-Cl-Ph | 4-Cl |
| Br | 4-Cl-Ph | 4-$CF_3$ |
| Br | 4-Cl-Ph | 4-Me |
| Br | 4-Cl-Ph | 5-Me |
| Br | 4-Cl-Ph | 4-F |
| Br | 2-Me-Ph | 4-Cl |
| Br | 2-Me-Ph | 4-Me |
| Br | 2-Me-Ph | 5-Me |
| Br | 2-Me-Ph | 4-F |
| Br | 4-Me-Ph | 4-Cl |
| Br | 4-Me-Ph | 4-Me |
| Br | 4-Me-Ph | 5-Me |
| Br | 4-Me-Ph | 4-F |
| Br | 4-Me-Ph | 5-F |
| Br | 2-F-Ph | 4-Cl |
| Br | 2-F-Ph | 5-Cl |
| Br | 2-F-Ph | 4-Me |
| Br | 2-F-Ph | 4-F |
| Br | 4-F-Ph | 4-Cl |
| Br | 4-F-Ph | 4-Me |
| Br | 4-F-Ph | 5-Me |
| Br | 4-F-Ph | 4-F |
| Br | 2,6-$F_2$-Ph | 4-Cl |
| Br | 2,6-$F_2$-Ph | 4-Me |
| Br | 2,6-$F_2$-Ph | 5-Me |
| Br | 2,6-$F_2$-Ph | 4-F |
| Br | 2,5-$F_2$-Ph | 4-Cl |
| Br | 2,5-$F_2$-Ph | 5-Cl |
| Br | 2,5-$F_2$-Ph | 4-Me |
| Br | 2,5-$F_2$-Ph | 5-Me |
| Br | 2,5-$F_2$-Ph | 4-F |
| Br | 2,5-$F_2$-Ph | 5-F |
| Br | 3,4-$F_2$-Ph | 4-Cl |
| Br | 3,4-$F_2$-Ph | 5-Cl |
| Br | 2-F-4-Me-Ph | 4-Cl |
| Br | 2-F-4-Me-Ph | 4-Me |
| Br | 2-F-4-Me-Ph | 4-F |
| Ph | i-Pr | H |
| Ph | s-Bu | H |
| Ph | t-Bu | H |
| Ph | $CF_3$ | H |
| Ph | $CF_2CF_3$ | H |
| Ph | Ph | H |
| Ph | 2-Cl-Ph | H |

| | | |
|---|---|---|
| Ph | 4-Cl-Ph | H |
| Ph | 2-F-Ph | H |
| Ph | 4-F-Ph | H |
| Ph | 2-Me-Ph | H |
| Ph | 4-Me-Ph | H |
| Ph | 4-Br-Ph | H |
| Ph | 2-Br-Ph | H |
| Ph | Ph | 4-Cl |
| Ph | Ph | 4-$CF_3$ |
| Ph | Ph | 4-Me |
| Ph | Ph | 6-Me |
| Ph | Ph | 4-F |
| Ph | 2-Cl-Ph | 4-Me |
| Ph | 4-Cl-Ph | 4-Me |
| Ph | 2-Me-Ph | 4-Me |
| Ph | 4-Me-Ph | 4-Cl |
| Ph | 4-Me-Ph | 4-Me |
| Ph | 4-Me-Ph | 4-F |
| Ph | 2-F-Ph | 4-Cl |
| Ph | 2-F-Ph | 4-Me |
| Ph | 2-F-Ph | 4-F |
| Ph | 4-F-Ph | 4-Me |
| Ph | 4-F-Ph | 4-F |
| Ph | 2,6-$F_2$-Ph | 4-Me |
| Ph | 2,6-$F_2$-Ph | 5-Me |
| Ph | 2,6-$F_2$-Ph | 4-F |
| i-Pr | i-Pr | H |
| i-Pr | s-Bu | H |
| i-Pr | t-Bu | H |
| i-Pr | $CF_3$ | H |
| i-Pr | $CF_2CF_3$ | H |
| i-Pr | Ph | H |
| i-Pr | 2-Cl-Ph | H |
| i-Pr | 4-Cl-Ph | H |
| i-Pr | 2-F-Ph | H |
| i-Pr | 4-F-Ph | H |
| i-Pr | 2-Me-Ph | H |
| i-Pr | 4-Me-Ph | H |
| i-Pr | 4-Br-Ph | H |
| i-Pr | 2-Br-Ph | H |
| i-Pr | Ph | 4-Cl |
| i-Pr | Ph | 4-$CF_3$ |
| i-Pr | Ph | 4-Me |
| i-Pr | Ph | 6-Me |
| i-Pr | Ph | 4-F |
| MeO | i-Pr | H |
| MeO | s-Bu | H |
| MeO | t-Bu | H |
| MeO | $CF_3$ | H |
| MeO | Ph | H |
| $Me_2N$ | i-Pr | H |
| $Me_2N$ | s-Bu | H |
| $Me_2N$ | t-Bu | H |
| $Me_2N$ | $CF_3$ | H |
| $Me_2N$ | Ph | H |
| Cl | i-Pr | H |
| Cl | s-Bu | H |
| Cl | t-Bu | H |

| | | |
|---|---|---|
| Cl | $CF_3$ | H |
| Cl | Ph | H |
| Cl | 2-Cl-Ph | H |
| Cl | 4-Cl-Ph | H |
| Cl | 2-F-Ph | H |
| Cl | 4-F-Ph | H |
| Cl | 2-Me-Ph | H |
| Cl | 4-Me-Ph | H |
| F | i-Pr | H |
| F | s-Bu | H |
| F | t-Bu | H |
| F | $CF_3$ | H |
| F | Ph | H |
| F | 2-Cl-Ph | H |
| F | 4-Cl-Ph | H |
| F | 2-F-Ph | H |
| F | 4-F-Ph | H |
| F | 2-Me-Ph | H |
| F | 4-Me-Ph | H |
| 2-Cl-Ph | i-Pr | H |
| 2-Cl-Ph | s-Bu | H |
| 2-Cl-Ph | t-Bu | H |
| 2-Cl-Ph | $CF_3$ | H |
| 2-Cl-Ph | 2-Cl-Ph | H |
| 2-Cl-Ph | 4-Cl-Ph | H |
| 2-Cl-Ph | 2-F-Ph | H |
| 2-Cl-Ph | 4-F-Ph | H |
| 2-Cl-Ph | 2-Me-Ph | H |
| 2-Cl-Ph | 4-Me-Ph | H |
| 2,6-$F_2$-Ph | i-Pr | H |
| 2,6-$F_2$-Ph | s-Bu | H |
| 2,6-$F_2$-Ph | t-Bu | H |
| 2,6-$F_2$-Ph | $CF_3$ | H |
| 2,6-$F_2$-Ph | 2-Cl-Ph | H |
| 2,6-$F_2$-Ph | 4-Cl-Ph | H |
| 2,6-$F_2$-Ph | 2-F-Ph | H |
| 2,6-$F_2$-Ph | 4-F-Ph | H |
| 2,6-$F_2$-Ph | 2-Me-Ph | H |
| 2,6-$F_2$-Ph | 4-Me-Ph | H |
| 4-Cl-Ph | s-Bu | H |
| 4-Cl-Ph | t-Bu | H |
| 4-Cl-Ph | $CF_3$ | H |
| 4-Cl-Ph | 4-Cl-Ph | H |
| 4-Cl-Ph | 2-F-Ph | H |
| 4-Cl-Ph | 4-F-Ph | H |
| 4-Cl-Ph | 2-Me-Ph | H |
| 4-Cl-Ph | 4-Me-Ph | H |
| H | H | 3-Cl |
| H | H | 3-$CF_3$ |
| H | H | 3-Me |
| H | H | 6-F |
| H | Me | 5-Cl |
| H | Me | 3-$CF_3$ |
| H | Me | 6-Me |
| H | Me | 3-F |
| H | Me | 4-Cl |
| H | Me | 4-$CF_3$ |
| H | Me | 4-Me |

| | | |
|---|---|---|
| H | Me | 4-F |
| H | Et | 4-Cl |
| H | Et | 4-$CF_3$ |
| H | Et | 4-Me |
| H | Et | 4-F |

(Table 2)

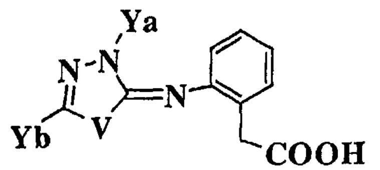 ,

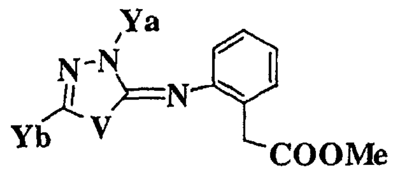 ,

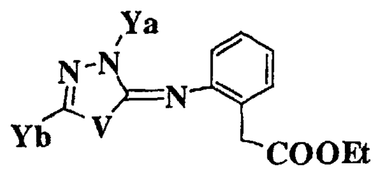 ,

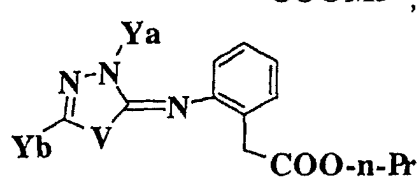 ,

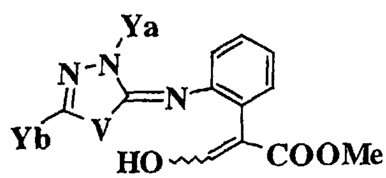 ,

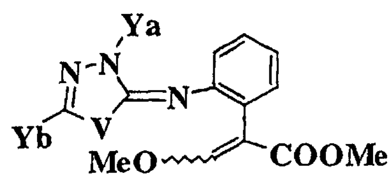 ,

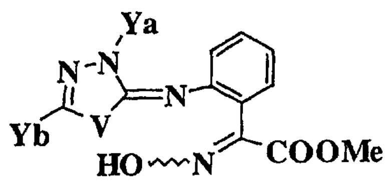 ,

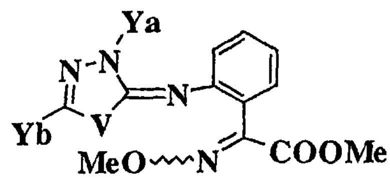 ,

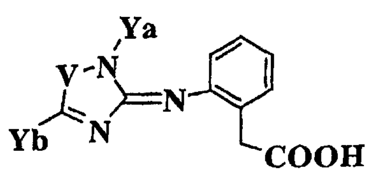 ,

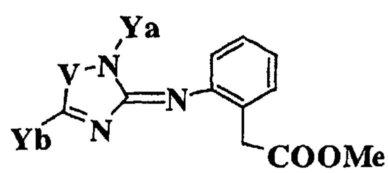 ,

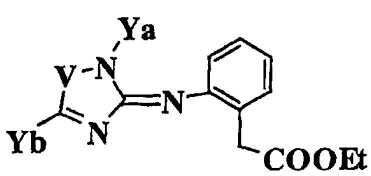 ,

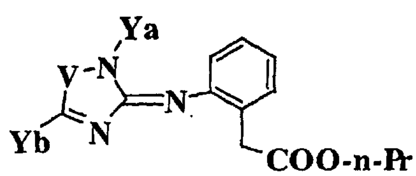 ,

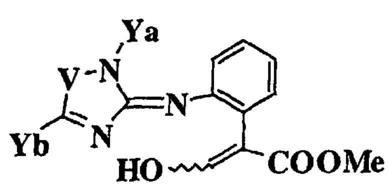 ,

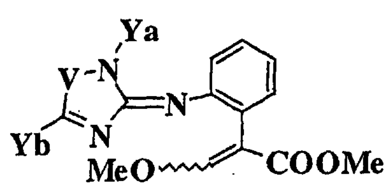 ,

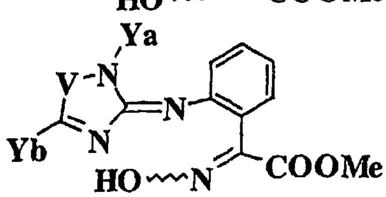 ,

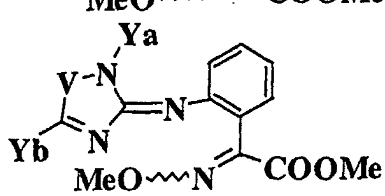 ,

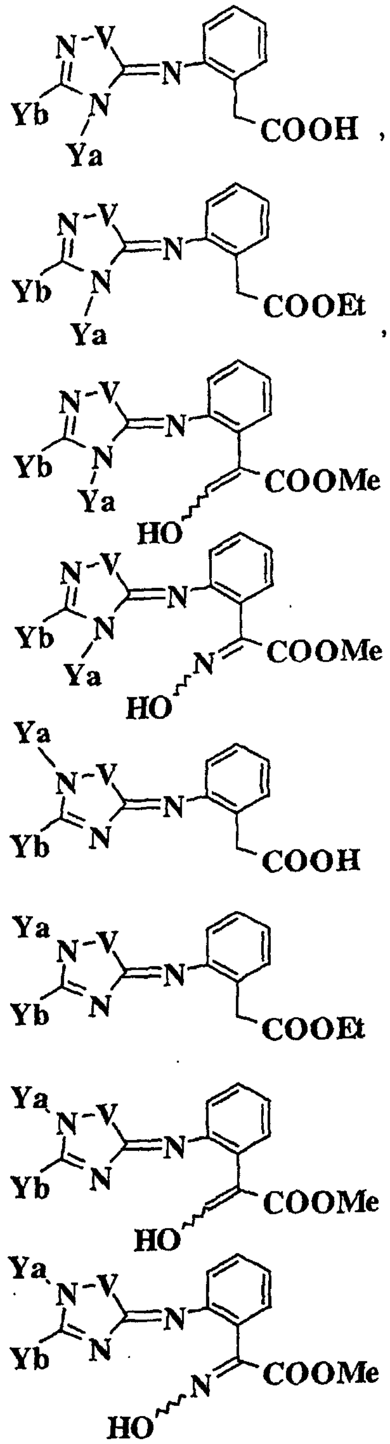

,

,

,

,

,

,

,

or

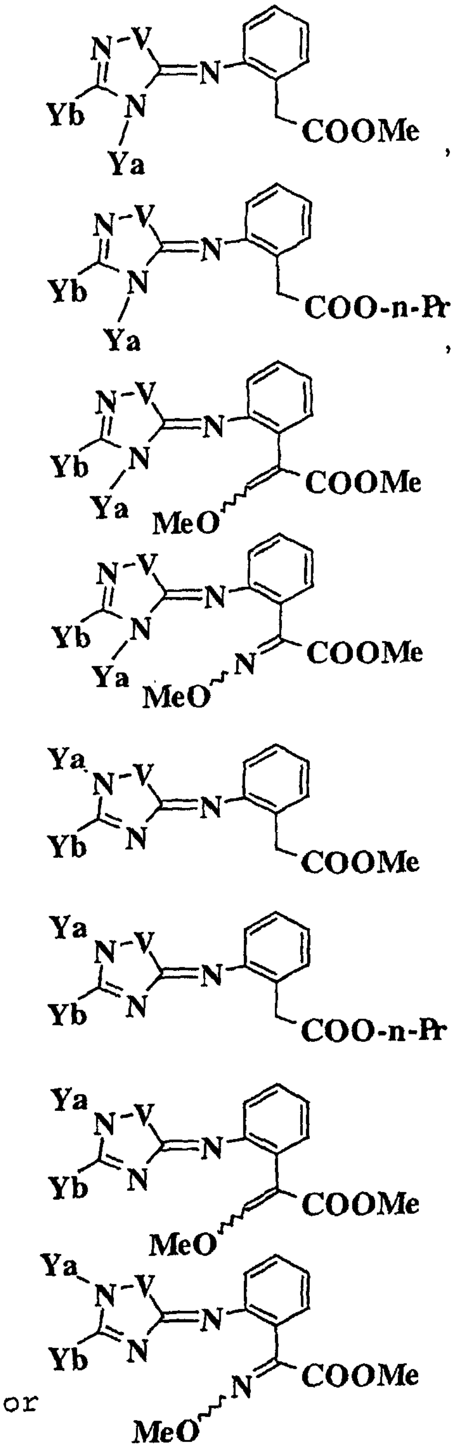

,

,

,

,

,

,

,

| Y a | Y b | V |
|---|---|---|
| Me | H | S |
| Me | Cl | S |
| Me | Me | S |

| | | |
|---|---|---|
| Me | Et | S |
| Me | n-Pr | S |
| Me | i-Pr | S |
| Me | n-Bu | S |
| Me | n-Hex | S |
| Me | Ethenyl | S |
| Me | 1-Propynyl | S |
| Me | $CF_3$ | S |
| Me | c-Pr | S |
| Me | MeO | S |
| Me | MeS | S |
| Me | MeSO | S |
| Me | $MeSO_2$ | S |
| Me | $NO_2$ | S |
| Me | CN | S |
| Me | CHO | S |
| Me | $Me_2N$ | S |
| Me | $PhCH_2$ | S |
| Me | PhO | S |
| Me | $CO_2Me$ | S |
| Me | $MeOCH_2$ | S |
| Me | COMe | S |
| Me | $CH_2SMe$ | S |
| Me | $CH_2OPh$ | S |
| Me | (4-Me-Ph)$OCH_2$ | S |
| Me | (2,4-$Cl_2$-Ph)$OCH_2$ | S |
| Me | $CH_2SCH_2Ph$ | S |
| Me | $CF_2Cl$ | S |
| Me | 1-Naphthyl | S |
| Me | 2-Naphthyl | S |
| Me | Thiophen-2-yl | S |
| Me | Furan-2-yl | S |
| Me | 1-Me-Pyrrole-2-yl | S |
| Me | Pyridin-4-yl | S |
| Me | 6-Cl-Pyridin-2-yl | S |
| Me | 2-Pyrazyl | S |
| Me | Pyrimidin-2-yl | S |
| Me | Thiazol-2-yl | S |
| Me | Thiazol-5-yl | S |
| Me | 5-$CF_3$-Thiazol-2-yl | S |
| Me | 1-Me-3-Cl-Pyrazol-5-yl | S |
| Me | 1-Me-Imidazol-2-yl | S |
| Me | Ph | S |
| Me | 2-Cl-Ph | S |
| Me | 3-Cl-Ph | S |
| Me | 4-F-Ph | S |
| Me | 2-Me-Ph | S |
| Me | 3-Me-Ph | S |
| Me | 4-Me-Ph | S |
| Me | 2-MeO-Ph | S |
| Me | 3-MeO-Ph | S |
| Me | 4-MeO-Ph | S |
| Me | 2,4-$Cl_2$-Ph | S |
| Me | 2-Cl-4-Me-Ph | S |
| Me | 2,5-$Me_2$-Ph | S |
| Me | 2,6-$F_2$-Ph | S |
| Me | 4-Et-Ph | S |
| Me | 4-$PhCH_2$-Ph | S |

| | | |
|---|---|---|
| Me | 4-$CF_3$-Ph | S |
| Me | 4-MeS-Ph | S |
| Me | 4-EtSO-Ph | S |
| Me | 4-$MeSO_2$-Ph | S |
| Me | 4-$EtSO_2$-Ph | S |
| Me | 4-CHO-Ph | S |
| Me | 4-$NO_2$-Ph | S |
| Me | 3-CN-Ph | S |
| Me | 4-CN-Ph | S |
| Me | 4-$PhCH_2$(MeCO)N-Ph | S |
| Me | 4-(2,4-$F_2$-Ph)$CH_2$O-Ph | S |
| Me | 4-MeC(O)-Ph | S |
| Me | 4-(4-Cl-Ph)C(O)-Ph | S |
| Me | 4-$MeOCH_2$-Ph | S |
| Me | 4-$EtOCH_2$-Ph | S |
| Me | 4-$MeSCH_2$-Ph | S |
| Me | 4-$EtSCH_2$-Ph | S |
| Me | 4-$CF_3$C(O)-Ph | S |
| Me | 4-MeC(O)O-Ph | S |
| Me | 4-t-BuC(O)O-Ph | S |
| Me | 4-$CF_3$C(O)O-Ph | S |
| Me | 4-PhC(O)O-Ph | S |
| Me | 4-Ph-Ph | S |
| Me | 4-(4-Cl-Ph)-Ph | S |
| Me | 4-(4-MeO-Ph)O-Ph | S |
| Me | 4-(2,4-$Cl_2$-Ph)O-Ph | S |
| Me | 4-(Pyridin-2-yl)O-Ph | S |
| Me | 4-(5-Cl-Pyridin-2-yl)O-Ph | S |
| Et | H | S |
| Et | Cl | S |
| Et | Me | S |
| Et | Et | S |
| Et | MeO | S |
| Et | MeS | S |
| Et | $Me_2N$ | S |
| Et | $PhCH_2$ | S |
| Et | PhO | S |
| Et | $CO_2Me$ | S |
| Et | $MeOCH_2$ | S |
| Et | COMe | S |
| Et | $CH_2SMe$ | S |
| Et | $CH_2OPh$ | S |
| Et | $CH_2SCH_2Ph$ | S |
| Et | 2-Naphthyl | S |
| Et | Thiophen-2-yl | S |
| Et | Furan-3-yl | S |
| Et | 1-Me-Pyrrole-3-yl | S |
| Et | Pyridin-2-yl | S |
| Et | Pyrazin-2-yl | S |
| Et | Pyrimidin-2-yl | S |
| Et | Thiazol-5-yl | S |
| Et | 1-Me-3-Cl-Pyrazol-5-yl | S |
| Et | 1-Me-Imidazol-2-yl | S |
| Et | Ph | S |
| Et | 4-Cl-Ph | S |
| Et | 4-F-Ph | S |
| Et | 4-Me-Ph | S |
| Et | 4-MeO-Ph | S |

| | | |
|---|---|---|
| Et | 4-Br-Ph | S |
| Et | 4-$CF_3$-Ph | S |
| Et | 4-$CF_3O$-Ph | S |
| Et | 4-MeS-Ph | S |
| Et | 4-$MeSO_2$-Ph | S |
| Et | 4-CHO-Ph | S |
| Et | 4-$NO_2$-Ph | S |
| Et | 3-CN-Ph | S |
| Et | 4-$(Me)_2N$-Ph | S |
| Et | 4-$PhCH_2O$-Ph | S |
| Et | 4-MeC(O)-Ph | S |
| Et | 4-(4-Cl-Ph)C(O)-Ph | S |
| Et | 4-$MeOCH_2$-Ph | S |
| Et | 4-$EtSCH_2$-Ph | S |
| Et | 4-$CF_3C(O)$-Ph | S |
| Et | 4-MeC(O)O-Ph | S |
| Et | 4-$CF_3C(O)O$-Ph | S |
| Et | 4-PhC(O)O-Ph | S |
| Et | 4-Ph-Ph | S |
| Et | 4-(4-Me-Ph)O-Ph | S |
| Et | 4-(Pyridin-2-yl)O-Ph | S |
| Ph | H | S |
| Ph | Cl | S |
| Ph | Me | S |
| Ph | Et | S |
| Ph | $CO_2Me$ | S |
| Ph | COMe | S |
| Ph | 2-Naphthyl | S |
| Ph | Thiophen-2-yl | S |
| Ph | Furan-3-yl | S |
| Ph | 1-Me-Pyrrole-2-yl | S |
| Ph | Pyridin-4-yl | S |
| Ph | Pyrazin-2-yl | S |
| Ph | Pyrimidin-4-yl | S |
| Ph | Thiazol-2-yl | S |
| Ph | 1-Me-3-Cl-Pyrazol-5-yl | S |
| Ph | 1-Me-Imidazol-2-yl | S |
| Ph | Ph | S |
| Ph | 4-Cl-Ph | S |
| Ph | 2-F-Ph | S |
| Ph | 3-F-Ph | S |
| Ph | 4-F-Ph | S |
| Ph | 2-Me-Ph | S |
| Ph | 3-Me-Ph | S |
| Ph | 4-Me-Ph | S |
| Ph | 2-MeO-Ph | S |
| Ph | 3-MeO-Ph | S |
| Ph | 4-MeO-Ph | S |
| Ph | 2,4-$Cl_2$-Ph | S |
| Ph | 4-$PhCH_2$-Ph | S |
| Ph | 4-$CF_3$-Ph | S |
| Ph | 4-$CF_3O$-Ph | S |
| Ph | 4-MeS-Ph | S |
| Ph | 4-EtSO-Ph | S |
| Ph | 4-$MeSO_2$-Ph | S |
| Ph | 4-CHO-Ph | S |
| Ph | 4-$NO_2$-Ph | S |
| Ph | 3-CN-Ph | S |

| | | |
|---|---|---|
| Ph | 4-CN-Ph | S |
| Ph | 4-(Me)$_2$N-Ph | S |
| Ph | 4-Me(MeC(O))N-Ph | S |
| Ph | 4-PhN(Me)-Ph | S |
| Ph | PhCH$_2$O-Ph | S |
| Ph | 4-MeC(O)-Ph | S |
| Ph | 4-(4-Cl-Ph)C(O)-Ph | S |
| Ph | 4-MeOCH$_2$-Ph | S |
| Ph | 4-EtSCH$_2$-Ph | S |
| Ph | 4-CF$_3$C(O)-Ph | S |
| Ph | 4-MeC(O)O-Ph | S |
| Ph | 4-CF$_3$C(O)O-Ph | S |
| Ph | 4-PhC(O)O-Ph | S |
| Ph | 4-Ph-Ph | S |
| Ph | 3-PhO-Ph | S |
| CO$_2$Me | H | S |
| CO$_2$Me | Cl | S |
| CO$_2$Me | Me | S |
| CO$_2$Me | Et | S |
| CO$_2$Me | CO$_2$Me | S |
| CO$_2$Me | COMe | S |
| CO$_2$Me | 2-Naphthyl | S |
| CO$_2$Me | Thiophen-2-yl | S |
| CO$_2$Me | Furan-3-yl | S |
| CO$_2$Me | 1-Me-Pyrrole-2-yl | S |
| CO$_2$Me | Pyridin-4-yl | S |
| CO$_2$Me | Pyrazin-2-yl | S |
| CO$_2$Me | Pyrimidin-4-yl | S |
| CO$_2$Me | Thiazol-2-yl | S |
| CO$_2$Me | 1-Me-3-Cl-Pyrazol-5-yl | S |
| CO$_2$Me | 1-Me-Imidazol-2-yl | S |
| CO$_2$Me | Ph | S |
| CO$_2$Me | 4-Cl-Ph | S |
| CO$_2$Me | 4-F-Ph | S |
| CO$_2$Me | 4-Me-Ph | S |
| CO$_2$Me | 4-MeO-Ph | S |
| CO$_2$Me | 4-CF$_3$-Ph | S |
| CO$_2$Me | 4-CF$_3$O-Ph | S |
| CO$_2$Me | 4-MeS-Ph | S |
| CO$_2$Me | 4-EtSO-Ph | S |
| CO$_2$Me | 4-MeSO$_2$-Ph | S |
| CO$_2$Me | 4-CHO-Ph | S |
| CO$_2$Me | 4-NO$_2$-Ph | S |
| CO$_2$Me | 3-CN-Ph | S |
| CO$_2$Me | 4-PhN(Me)-Ph | S |
| CO$_2$Me | PhCH$_2$O-Ph | S |
| CO$_2$Me | 4-MeC(O)-Ph | S |
| CO$_2$Me | 4-(4-Cl-Ph)C(O)-Ph | S |
| CO$_2$Me | 4-MeOCH$_2$-Ph | S |
| CO$_2$Me | 4-EtSCH$_2$-Ph | S |
| CO$_2$Me | 4-CF$_3$C(O)-Ph | S |
| CO$_2$Me | 4-MeC(O)O-Ph | S |
| CO$_2$Me | 4-CF$_3$C(O)O-Ph | S |
| CO$_2$Me | 4-PhC(O)O-Ph | S |
| CO$_2$Me | 4-Ph-Ph | S |
| CO$_2$Me | 3-PhO-Ph | S |
| Me | H | O |
| Me | Cl | O |

| | | |
|---|---|---|
| Me | Me | 0 |
| Me | Et | 0 |
| Me | n-Pr | 0 |
| Me | i-Pr | 0 |
| Me | n-Bu | 0 |
| Me | n-Hex | 0 |
| Me | Ethenyl | 0 |
| Me | 1-Propynyl | 0 |
| Me | $CF_3$ | 0 |
| Me | c-Pr | 0 |
| Me | MeO | 0 |
| Me | MeS | 0 |
| Me | MeSO | 0 |
| Me | $MeSO_2$ | 0 |
| Me | $NO_2$ | 0 |
| Me | CN | 0 |
| Me | CHO | 0 |
| Me | $Me_2N$ | 0 |
| Me | $PhCH_2$ | 0 |
| Me | PhO | 0 |
| Me | $CO_2Me$ | 0 |
| Me | $MeOCH_2$ | 0 |
| Me | COMe | 0 |
| Me | $CH_2SMe$ | 0 |
| Me | $CH_2OPh$ | 0 |
| Me | $(4\text{-}Me\text{-}Ph)OCH_2$ | 0 |
| Me | $(2,4\text{-}Cl_2\text{-}Ph)OCH_2$ | 0 |
| Me | $CH_2SCH_2Ph$ | 0 |
| Me | $CF_2Cl$ | 0 |
| Me | 1-Naphthyl | 0 |
| Me | 2-Naphthyl | 0 |
| Me | Thiophen-2-yl | 0 |
| Me | Furan-2-yl | 0 |
| Me | 1-Me-Pyrrole-2-yl | 0 |
| Me | Pyridin-4-yl | 0 |
| Me | 6-Cl-Pyridin-2-yl | 0 |
| Me | Pyrazin-2-yl | 0 |
| Me | Pyrimidin-2-yl | 0 |
| Me | Thiazol-2-yl | 0 |
| Me | Thiazol-5-yl | 0 |
| Me | $5\text{-}CF_3\text{-}Thiazol\text{-}2\text{-}yl$ | 0 |
| Me | 1-Me-3-Cl-Pyrazol-5-yl | 0 |
| Me | 1-Me-Imidazol-2-yl | 0 |
| Me | Ph | 0 |
| Me | 2-Cl-Ph | 0 |
| Me | 3-Cl-Ph | 0 |
| Me | 4-F-Ph | 0 |
| Me | 2-Me-Ph | 0 |
| Me | 3-Me-Ph | 0 |
| Me | 4-Me-Ph | 0 |
| Me | 2-MeO-Ph | 0 |
| Me | 3-MeO-Ph | 0 |
| Me | 4-MeO-Ph | 0 |
| Me | $2,4\text{-}Cl_2\text{-}Ph$ | 0 |
| Me | 2-Cl-4-Me-Ph | 0 |
| Me | $2,5\text{-}Me_2\text{-}Ph$ | 0 |
| Me | $2,6\text{-}F_2\text{-}Ph$ | 0 |
| Me | 4-Et-Ph | 0 |

| | | |
|---|---|---|
| Me | 4-$PhCH_2$-Ph | 0 |
| Me | 4-$CF_3$-Ph | 0 |
| Me | 4-MeS-Ph | 0 |
| Me | 4-EtSO-Ph | 0 |
| Me | 4-$MeSO_2$-Ph | 0 |
| Me | 4-$EtSO_2$-Ph | 0 |
| Me | 4-CHO-Ph | 0 |
| Me | 4-$NO_2$-Ph | 0 |
| Me | 3-CN-Ph | 0 |
| Me | 4-CN-Ph | 0 |
| Me | 4-$PhCH_2$(MeCO)N-Ph | 0 |
| Me | 4-(2,4-$F_2$-Ph)$CH_2$O-Ph | 0 |
| Me | 4-MeC(O)-Ph | 0 |
| Me | 4-(4-Cl-Ph)C(O)-Ph | 0 |
| Me | 4-$MeOCH_2$-Ph | 0 |
| Me | 4-$EtOCH_2$-Ph | 0 |
| Me | 4-$MeSCH_2$-Ph | 0 |
| Me | 4-$EtSCH_2$-Ph | 0 |
| Me | 4-$CF_3$C(O)-Ph | 0 |
| Me | 4-MeC(O)O-Ph | 0 |
| Me | 4-t-BuC(O)O-Ph | 0 |
| Me | 4-$CF_3$C(O)O-Ph | 0 |
| Me | 4-PhC(O)O-Ph | 0 |
| Me | 4-Ph-Ph | 0 |
| Me | 4-(4-Cl-Ph)-Ph | 0 |
| Me | 4-(4-MeO-Ph)O-Ph | 0 |
| Me | 4-(2,4-$Cl_2$-Ph)O-Ph | 0 |
| Me | 4-(Pyridin-2-yl)O-Ph | 0 |
| Me | 4-(5-Cl-Pyridin-2-yl)O-Ph | 0 |
| Et | H | 0 |
| Et | Cl | 0 |
| Et | Me | 0 |
| Et | Et | 0 |
| Et | MeO | 0 |
| Et | MeS | 0 |
| Et | $Me_2N$ | 0 |
| Et | $PhCH_2$ | 0 |
| Et | PhO | 0 |
| Et | $CO_2Me$ | 0 |
| Et | $MeOCH_2$ | 0 |
| Et | COMe | 0 |
| Et | $CH_2SMe$ | 0 |
| Et | $CH_2OPh$ | 0 |
| Et | $CH_2SCH_2Ph$ | 0 |
| Et | 2-Naphthyl | 0 |
| Et | Thiophen-2-yl | 0 |
| Et | Furan-3-yl | 0 |
| Et | 1-Me-Pyrrole-3-yl | 0 |
| Et | Pyridin-2-yl | 0 |
| Et | Pyrazin-2-yl | 0 |
| Et | Pyrimidin-2-yl | 0 |
| Et | Thiazol-5-yl | 0 |
| Et | 1-Me-3-Cl-Pyrazol-5-yl | 0 |
| Et | 1-Me-Imidazol-2-yl | 0 |
| Et | Ph | 0 |
| Et | 4-Cl-Ph | 0 |
| Et | 4-F-Ph | 0 |
| Et | 4-Me-Ph | 0 |

| | | |
|---|---|---|
| Et | 4-MeO-Ph | 0 |
| Et | 4-Br-Ph | 0 |
| Et | 4-$CF_3$-Ph | 0 |
| Et | 4-$CF_3$O-Ph | 0 |
| Et | 4-MeS-Ph | 0 |
| Et | 4-$MeSO_2$-Ph | 0 |
| Et | 4-CHO-Ph | 0 |
| Et | 4-$NO_2$-Ph | 0 |
| Et | 3-CN-Ph | 0 |
| Et | 4-$(Me)_2$N-Ph | 0 |
| Et | 4-$PhCH_2$O-Ph | 0 |
| Et | 4-MeC(O)-Ph | 0 |
| Et | 4-(4-Cl-Ph)C(O)-Ph | 0 |
| Et | 4-$MeOCH_2$-Ph | 0 |
| Et | 4-$EtSCH_2$-Ph | 0 |
| Et | 4-$CF_3$C(O)-Ph | 0 |
| Et | 4-MeC(O)O-Ph | 0 |
| Et | 4-$CF_3$C(O)O-Ph | 0 |
| Et | 4-PhC(O)O-Ph | 0 |
| Et | 4-Ph-Ph | 0 |
| Et | 4-(4-Me-Ph)O-Ph | 0 |
| Et | 4-(Pyridin-2-yl)O-Ph | 0 |
| Ph | H | 0 |
| Ph | Cl | 0 |
| Ph | Me | 0 |
| Ph | Et | 0 |
| Ph | $CO_2$Me | 0 |
| Ph | COMe | 0 |
| Ph | 2-Naphthyl | 0 |
| Ph | Thiophen-2-yl | 0 |
| Ph | Furan-3-yl | 0 |
| Ph | 1-Me-Pyrrole-2-yl | 0 |
| Ph | Pyridin-4-yl | 0 |
| Ph | Pyrazin-2-yl | 0 |
| Ph | Pyrimidin-4-yl | 0 |
| Ph | Thiazol-2-yl | 0 |
| Ph | 1-Me-3-Cl-Pyrazol-5-yl | 0 |
| Ph | 1-Me-Imidazol-2-yl | 0 |
| Ph | Ph | 0 |
| Ph | 4-Cl-Ph | 0 |
| Ph | 2-F-Ph | 0 |
| Ph | 3-F-Ph | 0 |
| Ph | 4-F-Ph | 0 |
| Ph | 2-Me-Ph | 0 |
| Ph | 3-Me-Ph | 0 |
| Ph | 4-Me-Ph | 0 |
| Ph | 2-MeO-Ph | 0 |
| Ph | 3-MeO-Ph | 0 |
| Ph | 4-MeO-Ph | 0 |
| Ph | 2,4-$Cl_2$-Ph | 0 |
| Ph | 4-$PhCH_2$-Ph | 0 |
| Ph | 4-$CF_3$-Ph | 0 |
| Ph | 4-$CF_3$O-Ph | 0 |
| Ph | 4-MeS-Ph | 0 |
| Ph | 4-EtSO-Ph | 0 |
| Ph | 4-$MeSO_2$-Ph | 0 |
| Ph | 4-CHO-Ph | 0 |
| Ph | 4-$NO_2$-Ph | 0 |

| | | |
|---|---|---|
| Ph | 3-CN-Ph | O |
| Ph | 4-CN-Ph | O |
| Ph | 4-(Me)$_2$N-Ph | O |
| Ph | 4-Me(MeC(O))N-Ph | O |
| Ph | 4-PhN(Me)-Ph | O |
| Ph | PhCH$_2$O-Ph | O |
| Ph | 4-MeC(O)-Ph | O |
| Ph | 4-(4-Cl-Ph)C(O)-Ph | O |
| Ph | 4-MeOCH$_2$-Ph | O |
| Ph | 4-EtSCH$_2$-Ph | O |
| Ph | 4-CF$_3$C(O)-Ph | O |
| Ph | 4-MeC(O)O-Ph | O |
| Ph | 4-CF$_3$C(O)O-Ph | O |
| Ph | 4-PhC(O)O-Ph | O |
| Ph | 4-Ph-Ph | O |
| Ph | 3-PhO-Ph | O |
| CO$_2$Me | H | O |
| CO$_2$Me | Cl | O |
| CO$_2$Me | Me | O |
| CO$_2$Me | Et | O |
| CO$_2$Me | CO$_2$Me | O |
| CO$_2$Me | COMe | O |
| CO$_2$Me | 2-Naphthyl | O |
| CO$_2$Me | Thiophen-2-yl | O |
| CO$_2$Me | Furan-3-yl | O |
| CO$_2$Me | 1-Me-Pyrrol-2-yl | O |
| CO$_2$Me | Pyridin-4-yl | O |
| CO$_2$Me | Pyrazin-2-yl | O |
| CO$_2$Me | Pyrimidin-4-yl | O |
| CO$_2$Me | Thiazol-2-yl | O |
| CO$_2$Me | 1-Me-3-Cl-Pyrazol-5-yl | O |
| CO$_2$Me | 1-Me-Imidazol-2-yl | O |
| CO$_2$Me | Ph | O |
| CO$_2$Me | 4-Cl-Ph | O |
| CO$_2$Me | 4-F-Ph | O |
| CO$_2$Me | 4-Me-Ph | O |
| CO$_2$Me | 4-MeO-Ph | O |
| CO$_2$Me | 4-CF$_3$-Ph | O |
| CO$_2$Me | 4-CF$_3$O-Ph | O |
| CO$_2$Me | 4-MeS-Ph | O |
| CO$_2$Me | 4-EtSO-Ph | O |
| CO$_2$Me | 4-MeSO$_2$-Ph | O |
| CO$_2$Me | 4-CHO-Ph | O |
| CO$_2$Me | 4-NO$_2$-Ph | O |
| CO$_2$Me | 3-CN-Ph | O |
| CO$_2$Me | 4-PhN(Me)-Ph | O |
| CO$_2$Me | PhCH$_2$O-Ph | O |
| CO$_2$Me | 4-MeC(O)-Ph | O |
| CO$_2$Me | 4-(4-Cl-Ph)C(O)-Ph | O |
| CO$_2$Me | 4-MeOCH$_2$-Ph | O |
| CO$_2$Me | 4-EtSCH$_2$-Ph | O |
| CO$_2$Me | 4-CF$_3$C(O)-Ph | O |
| CO$_2$Me | 4-MeC(O)O-Ph | O |
| CO$_2$Me | 4-CF$_3$C(O)O-Ph | O |
| CO$_2$Me | 4-PhC(O)O-Ph | O |
| CO$_2$Me | 4-Ph-Ph | O |
| CO$_2$Me | 3-PhO-Ph | O |
| Me | H | N-Me |

| | | |
|---|---|---|
| Me | Cl | N-Me |
| Me | Me | N-Me |
| Me | Et | N-Me |
| Me | n-Pr | N-Me |
| Me | i-Pr | N-Me |
| Me | n-Bu | N-Me |
| Me | n-Hex | N-Me |
| Me | Ethenyl | N-Me |
| Me | 1-Propynyl | N-Me |
| Me | $CF_3$ | N-Me |
| Me | c-Pr | N-Me |
| Me | MeO | N-Me |
| Me | MeS | N-Me |
| Me | MeSO | N-Me |
| Me | $MeSO_2$ | N-Me |
| Me | $NO_2$ | N-Me |
| Me | CN | N-Me |
| Me | CHO | N-Me |
| Me | $Me_2N$ | N-Me |
| Me | $PhCH_2$ | N-Me |
| Me | PhO | N-Me |
| Me | $CO_2Me$ | N-Me |
| Me | $MeOCH_2$ | N-Me |
| Me | COMe | N-Me |
| Me | $CH_2SMe$ | N-Me |
| Me | $CH_2OPh$ | N-Me |
| Me | (4-Me-Ph)$OCH_2$ | N-Me |
| Me | (2,4-$Cl_2$-Ph)$OCH_2$ | N-Me |
| Me | $CH_2SCH_2Ph$ | N-Me |
| Me | $CF_2Cl$ | N-Me |
| Me | 1-Naphthyl | N-Me |
| Me | 2-Naphthyl | N-Me |
| Me | Thiophen-2-yl | N-Me |
| Me | Furan-2-yl | N-Me |
| Me | 1-Me-Pyrrol-2-yl | N-Me |
| Me | Pyridin-4-yl | N-Me |
| Me | 6-Cl-Pyridin-2-yl | N-Me |
| Me | Pyrazin-2-yl | N-Me |
| Me | Pyrimidin-2-yl | N-Me |
| Me | Thiazol-2-yl | N-Me |
| Me | Thiazol-5-yl | N-Me |
| Me | 5-$CF_3$-Thiazol-2-yl | N-Me |
| Me | 1-Me-3-Cl-Pyrazol-5-yl | N-Me |
| Me | 1-Me-Imidazol-2-yl | N-Me |
| Me | Ph | N-Me |
| Me | 2-Cl-Ph | N-Me |
| Me | 3-Cl-Ph | N-Me |
| Me | 4-F-Ph | N-Me |
| Me | 2-Me-Ph | N-Me |
| Me | 3-Me-Ph | N-Me |
| Me | 4-Me-Ph | N-Me |
| Me | 2-MeO-Ph | N-Me |
| Me | 3-MeO-Ph | N-Me |
| Me | 4-MeO-Ph | N-Me |
| Me | 2,4-$Cl_2$-Ph | N-Me |
| Me | 2-Cl-4-Me-Ph | N-Me |
| Me | 2,5-$Me_2$-Ph | N-Me |
| Me | 2,6-$F_2$-Ph | N-Me |

| | | |
|---|---|---|
| Me | 4-Et-Ph | N-Me |
| Me | 4-PhCH$_2$-Ph | N-Me |
| Me | 4-CF$_3$-Ph | N-Me |
| Me | 4-MeS-Ph | N-Me |
| Me | 4-EtSO-Ph | N-Me |
| Me | 4-MeSO$_2$-Ph | N-Me |
| Me | 4-EtSO$_2$-Ph | N-Me |
| Me | 4-CHO-Ph | N-Me |
| Me | 4-NO$_2$-Ph | N-Me |
| Me | 3-CN-Ph | N-Me |
| Me | 4-CN-Ph | N-Me |
| Me | 4-PhCH$_2$(MeCO)N-Ph | N-Me |
| Me | 4-(2,4-F$_2$-Ph)CH$_2$O-Ph | N-Me |
| Me | 4-MeC(O)-Ph | N-Me |
| Me | 4-(4-Cl-Ph)C(O)-Ph | N-Me |
| Me | 4-MeOCH$_2$-Ph | N-Me |
| Me | 4-EtOCH$_2$-Ph | N-Me |
| Me | 4-MeSCH$_2$-Ph | N-Me |
| Me | 4-EtSCH$_2$-Ph | N-Me |
| Me | 4-CF$_3$C(O)-Ph | N-Me |
| Me | 4-MeC(O)O-Ph | N-Me |
| Me | 4-t-BuC(O)O-Ph | N-Me |
| Me | 4-CF$_3$C(O)O-Ph | N-Me |
| Me | 4-PhC(O)O-Ph | N-Me |
| Me | 4-Ph-Ph | N-Me |
| Me | 4-(4-Cl-Ph)-Ph | N-Me |
| Me | 4-(4-MeO-Ph)O-Ph | N-Me |
| Me | 4-(2,4-Cl$_2$-Ph)O-Ph | N-Me |
| Me | 4-(Pyridin-2-yl)O-Ph | N-Me |
| Me | 4-(5-Cl-Pyridin-2-yl)O-Ph | N-Me |
| Et | H | N-Me |
| Et | Cl | N-Me |
| Et | Me | N-Me |
| Et | Et | N-Me |
| Et | MeO | N-Me |
| Et | MeS | N-Me |
| Et | Me$_2$N | N-Me |
| Et | PhCH$_2$ | N-Me |
| Et | PhO | N-Me |
| Et | CO$_2$Me | N-Me |
| Et | MeOCH$_2$ | N-Me |
| Et | COMe | N-Me |
| Et | CH$_2$SMe | N-Me |
| Et | CH$_2$OPh | N-Me |
| Et | CH$_2$SCH$_2$Ph | N-Me |
| Et | 2-Naphthyl | N-Me |
| Et | Thiophen-2-yl | N-Me |
| Et | Furan-3-yl | N-Me |
| Et | 1-Me-Pyrrole-3-yl | N-Me |
| Et | Pyridin-2-yl | N-Me |
| Et | Pyrazin-2-yl | N-Me |
| Et | Pyrimidin-2-yl | N-Me |
| Et | Thiazol-5-yl | N-Me |
| Et | 1-Me-3-Cl-Pyrazol-5-yl | N-Me |
| Et | 1-Me-Imidazol-2-yl | N-Me |
| Et | Ph | N-Me |
| Et | 4-Cl-Ph | N-Me |
| Et | 4-F-Ph | N-Me |

| | | |
|---|---|---|
| Et | 4-Me-Ph | N-Me |
| Et | 4-MeO-Ph | N-Me |
| Et | 4-Br-Ph | N-Me |
| Et | 4-$CF_3$-Ph | N-Me |
| Et | 4-$CF_3$O-Ph | N-Me |
| Et | 4-MeS-Ph | N-Me |
| Et | 4-$MeSO_2$-Ph | N-Me |
| Et | 4-CHO-Ph | N-Me |
| Et | 4-$NO_2$-Ph | N-Me |
| Et | 3-CN-Ph | N-Me |
| Et | 4-$(Me)_2$N-Ph | N-Me |
| Et | 4-$PhCH_2$O-Ph | N-Me |
| Et | 4-MeC(O)-Ph | N-Me |
| Et | 4-(4-Cl-Ph)C(O)-Ph | N-Me |
| Et | 4-$MeOCH_2$-Ph | N-Me |
| Et | 4-$EtSCH_2$-Ph | N-Me |
| Et | 4-$CF_3$C(O)-Ph | N-Me |
| Et | 4-MeC(O)O-Ph | N-Me |
| Et | 4-$CF_3$C(O)O-Ph | N-Me |
| Et | 4-PhC(O)O-Ph | N-Me |
| Et | 4-Ph-Ph | N-Me |
| Et | 4-(4-Me-Ph)O-Ph | N-Me |
| Et | 4-(Pyridin-2-yl)O-Ph | N-Me |
| Ph | H | N-Me |
| Ph | Cl | N-Me |
| Ph | Me | N-Me |
| Ph | Et | N-Me |
| Ph | $CO_2$Me | N-Me |
| Ph | COMe | N-Me |
| Ph | 2-Naphthyl | N-Me |
| Ph | Thiophen-2-yl | N-Me |
| Ph | Furan-3-yl | N-Me |
| Ph | 1-Me-Pyrrol-2-yl | N-Me |
| Ph | Pyridin-4-yl | N-Me |
| Ph | Pyrazin-2-yl | N-Me |
| Ph | Pyrimidin-4-yl | N-Me |
| Ph | Thiazol-2-yl | N-Me |
| Ph | 1-Me-3-Cl-Pyrazol-5-yl | N-Me |
| Ph | 1-Me-Imidazol-2-yl | N-Me |
| Ph | Ph | N-Me |
| Ph | 4-Cl-Ph | N-Me |
| Ph | 2-F-Ph | N-Me |
| Ph | 3-F-Ph | N-Me |
| Ph | 4-F-Ph | N-Me |
| Ph | 2-Me-Ph | N-Me |
| Ph | 3-Me-Ph | N-Me |
| Ph | 4-Me-Ph | N-Me |
| Ph | 2-MeO-Ph | N-Me |
| Ph | 3-MeO-Ph | N-Me |
| Ph | 4-MeO-Ph | N-Me |
| Ph | 2,4-$Cl_2$-Ph | N-Me |
| Ph | 4-$PhCH_2$-Ph | N-Me |
| Ph | 4-$CF_3$-Ph | N-Me |
| Ph | 4-$CF_3$O-Ph | N-Me |
| Ph | 4-MeS-Ph | N-Me |
| Ph | 4-EtSO-Ph | N-Me |
| Ph | 4-$MeSO_2$-Ph | N-Me |
| Ph | 4-CHO-Ph | N-Me |

| | | |
|---|---|---|
| Ph | 4-$NO_2$-Ph | N-Me |
| Ph | 3-CN-Ph | N-Me |
| Ph | 4-CN-Ph | N-Me |
| Ph | 4-$(Me)_2$N-Ph | N-Me |
| Ph | 4-Me(MeC(O))N-Ph | N-Me |
| Ph | 4-PhN(Me)-Ph | N-Me |
| Ph | $PhCH_2O$-Ph | N-Me |
| Ph | 4-MeC(O)-Ph | N-Me |
| Ph | 4-(4-Cl-Ph)C(O)-Ph | N-Me |
| Ph | 4-$MeOCH_2$-Ph | N-Me |
| Ph | 4-$EtSCH_2$-Ph | N-Me |
| Ph | 4-$CF_3C$(O)-Ph | N-Me |
| Ph | 4-MeC(O)O-Ph | N-Me |
| Ph | 4-$CF_3C$(O)O-Ph | N-Me |
| Ph | 4-PhC(O)O-Ph | N-Me |
| Ph | 4-Ph-Ph | N-Me |
| Ph | 3-PhO-Ph | N-Me |
| $CO_2Me$ | H | N-Me |
| $CO_2Me$ | Cl | N-Me |
| $CO_2Me$ | Me | N-Me |
| $CO_2Me$ | Et | N-Me |
| $CO_2Me$ | $CO_2Me$ | N-Me |
| $CO_2Me$ | COMe | N-Me |
| $CO_2Me$ | 2-Naphthyl | N-Me |
| $CO_2Me$ | Thiophen-2-yl | N-Me |
| $CO_2Me$ | Furan-3-yl | N-Me |
| $CO_2Me$ | 1-Me-Pyrrol-2-yl | N-Me |
| $CO_2Me$ | Pyridin-4-yl | N-Me |
| $CO_2Me$ | Pyrazin-2-yl | N-Me |
| $CO_2Me$ | Pyrimidin-4-yl | N-Me |
| $CO_2Me$ | Thiazol-2-yl | N-Me |
| $CO_2Me$ | 1-Me-3-Cl-Pyrazol-5-yl | N-Me |
| $CO_2Me$ | 1-Me-Imidazol-2-yl | N-Me |
| $CO_2Me$ | Ph | N-Me |
| $CO_2Me$ | 4-Cl-Ph | N-Me |
| $CO_2Me$ | 4-F-Ph | N-Me |
| $CO_2Me$ | 4-Me-Ph | N-Me |
| $CO_2Me$ | 4-MeO-Ph | N-Me |
| $CO_2Me$ | 4-$CF_3$-Ph | N-Me |
| $CO_2Me$ | 4-$CF_3O$-Ph | N-Me |
| $CO_2Me$ | 4-MeS-Ph | N-Me |
| $CO_2Me$ | 4-EtSO-Ph | N-Me |
| $CO_2Me$ | 4-$MeSO_2$-Ph | N-Me |
| $CO_2Me$ | 4-CHO-Ph | N-Me |
| $CO_2Me$ | 4-$NO_2$-Ph | N-Me |
| $CO_2Me$ | 3-CN-Ph | N-Me |
| $CO_2Me$ | 4-PhN(Me)-Ph | N-Me |
| $CO_2Me$ | $PhCH_2O$-Ph | N-Me |
| $CO_2Me$ | 4-MeC(O)-Ph | N-Me |
| $CO_2Me$ | 4-(4-Cl-Ph)C(O)-Ph | N-Me |
| $CO_2Me$ | 4-$MeOCH_2$-Ph | N-Me |
| $CO_2Me$ | 4-$EtSCH_2$-Ph | N-Me |
| $CO_2Me$ | 4-$CF_3C$(O)-Ph | N-Me |
| $CO_2Me$ | 4-MeC(O)O-Ph | N-Me |
| $CO_2Me$ | 4-$CF_3C$(O)O-Ph | N-Me |
| $CO_2Me$ | 4-PhC(O)O-Ph | N-Me |
| $CO_2Me$ | 4-Ph-Ph | N-Me |
| $CO_2Me$ | 3-PhO-Ph | N-Me |

| | | |
|---|---|---|
| Me | H | N-$CO_2$Me |
| Me | Cl | N-$CO_2$Me |
| Me | Me | N-$CO_2$Me |
| Me | Et | N-$CO_2$Me |
| Me | n-Pr | N-$CO_2$Me |
| Me | i-Pr | N-$CO_2$Me |
| Me | n-Bu | N-$CO_2$Me |
| Me | n-Hex | N-$CO_2$Me |
| Me | Ethenyl | N-$CO_2$Me |
| Me | 1-Propynyl | N-$CO_2$Me |
| Me | $CF_3$ | N-$CO_2$Me |
| Me | c-Pr | N-$CO_2$Me |
| Me | MeO | N-$CO_2$Me |
| Me | MeS | N-$CO_2$Me |
| Me | MeSO | N-$CO_2$Me |
| Me | $MeSO_2$ | N-$CO_2$Me |
| Me | $NO_2$ | N-$CO_2$Me |
| Me | CN | N-$CO_2$Me |
| Me | CHO | N-$CO_2$Me |
| Me | $Me_2N$ | N-$CO_2$Me |
| Me | $PhCH_2$ | N-$CO_2$Me |
| Me | PhO | N-$CO_2$Me |
| Me | $CO_2Me$ | N-$CO_2$Me |
| Me | $MeOCH_2$ | N-$CO_2$Me |
| Me | COMe | N-$CO_2$Me |
| Me | $CH_2SMe$ | N-$CO_2$Me |
| Me | $CH_2OPh$ | N-$CO_2$Me |
| Me | (4-Me-Ph)$OCH_2$ | N-$CO_2$Me |
| Me | (2,4-$Cl_2$-Ph)$OCH_2$ | N-$CO_2$Me |
| Me | $CH_2SCH_2Ph$ | N-$CO_2$Me |
| Me | $CF_2Cl$ | N-$CO_2$Me |
| Me | 1-Naphthyl | N-$CO_2$Me |
| Me | 2-Naphthyl | N-$CO_2$Me |
| Me | Thiophen-2-yl | N-$CO_2$Me |
| Me | Furan-2-yl | N-$CO_2$Me |
| Me | 1-Me-Pyrrol-2-yl | N-$CO_2$Me |
| Me | Pyridin-4-yl | N-$CO_2$Me |
| Me | 6-Cl-Pyridin-2-yl | N-$CO_2$Me |
| Me | Pyrazin-2-yl | N-$CO_2$Me |
| Me | Pyrimidin-2-yl | N-$CO_2$Me |
| Me | Thiazol-2-yl | N-$CO_2$Me |
| Me | Thiazol-5-yl | N-$CO_2$Me |
| Me | 5-$CF_3$-Thiazol-2-yl | N-$CO_2$Me |
| Me | 1-Me-3-Cl-Pyrazol-5-yl | N-$CO_2$Me |
| Me | 1-Me-Imidazol-2-yl | N-$CO_2$Me |
| Me | Ph | N-$CO_2$Me |
| Me | 2-Cl-Ph | N-$CO_2$Me |
| Me | 3-Cl-Ph | N-$CO_2$Me |
| Me | 4-F-Ph | N-$CO_2$Me |
| Me | 2-Me-Ph | N-$CO_2$Me |
| Me | 3-Me-Ph | N-$CO_2$Me |
| Me | 4-Me-Ph | N-$CO_2$Me |
| Me | 2-MeO-Ph | N-$CO_2$Me |
| Me | 3-MeO-Ph | N-$CO_2$Me |
| Me | 4-MeO-Ph | N-$CO_2$Me |
| Me | 2,4-$Cl_2$-Ph | N-$CO_2$Me |
| Me | 2-Cl-4-Me-Ph | N-$CO_2$Me |
| Me | 2,5-$Me_2$-Ph | N-$CO_2$Me |

| | | |
|---|---|---|
| Me | 2,6-$F_2$-Ph | N-$CO_2$Me |
| Me | 4-Et-Ph | N-$CO_2$Me |
| Me | 4-Ph$CH_2$-Ph | N-$CO_2$Me |
| Me | 4-$CF_3$-Ph | N-$CO_2$Me |
| Me | 4-MeS-Ph | N-$CO_2$Me |
| Me | 4-EtSO-Ph | N-$CO_2$Me |
| Me | 4-Me$SO_2$-Ph | N-$CO_2$Me |
| Me | 4-Et$SO_2$-Ph | N-$CO_2$Me |
| Me | 4-CHO-Ph | N-$CO_2$Me |
| Me | 4-$NO_2$-Ph | N-$CO_2$Me |
| Me | 3-CN-Ph | N-$CO_2$Me |
| Me | 4-CN-Ph | N-$CO_2$Me |
| Me | 4-Ph$CH_2$(MeCO)N-Ph | N-$CO_2$Me |
| Me | 4-(2,4-$F_2$-Ph)$CH_2$O-Ph | N-$CO_2$Me |
| Me | 4-MeC(O)-Ph | N-$CO_2$Me |
| Me | 4-(4-Cl-Ph)C(O)-Ph | N-$CO_2$Me |
| Me | 4-MeO$CH_2$-Ph | N-$CO_2$Me |
| Me | 4-EtO$CH_2$-Ph | N-$CO_2$Me |
| Me | 4-MeS$CH_2$-Ph | N-$CO_2$Me |
| Me | 4-EtS$CH_2$-Ph | N-$CO_2$Me |
| Me | 4-$CF_3$C(O)-Ph | N-$CO_2$Me |
| Me | 4-MeC(O)O-Ph | N-$CO_2$Me |
| Me | 4-t-BuC(O)O-Ph | N-$CO_2$Me |
| Me | 4-$CF_3$C(O)O-Ph | N-$CO_2$Me |
| Me | 4-PhC(O)O-Ph | N-$CO_2$Me |
| Me | 4-Ph-Ph | N-$CO_2$Me |
| Me | 4-(4-Cl-Ph)-Ph | N-$CO_2$Me |
| Me | 4-(4-MeO-Ph)O-Ph | N-$CO_2$Me |
| Me | 4-(2,4-$Cl_2$-Ph)O-Ph | N-$CO_2$Me |
| Me | 4-(Pyridin-2-yl)O-Ph | N-$CO_2$Me |
| Me | 4-(5-Cl-Pyridin-2-yl)O-Ph | N-$CO_2$Me |
| Et | H | N-$CO_2$Me |
| Et | Cl | N-$CO_2$Me |
| Et | Me | N-$CO_2$Me |
| Et | Et | N-$CO_2$Me |
| Et | MeO | N-$CO_2$Me |
| Et | MeS | N-$CO_2$Me |
| Et | $Me_2$N | N-$CO_2$Me |
| Et | Ph$CH_2$ | N-$CO_2$Me |
| Et | PhO | N-$CO_2$Me |
| Et | $CO_2$Me | N-$CO_2$Me |
| Et | MeO$CH_2$ | N-$CO_2$Me |
| Et | COMe | N-$CO_2$Me |
| Et | $CH_2$SMe | N-$CO_2$Me |
| Et | $CH_2$OPh | N-$CO_2$Me |
| Et | $CH_2$S$CH_2$Ph | N-$CO_2$Me |
| Et | 2-Naphthyl | N-$CO_2$Me |
| Et | Thiophen-2-yl | N-$CO_2$Me |
| Et | Furan-3-yl | N-$CO_2$Me |
| Et | 1-Me-Pyrrole-3-yl | N-$CO_2$Me |
| Et | Pyridin-2-yl | N-$CO_2$Me |
| Et | Pyrazin-2-yl | N-$CO_2$Me |
| Et | Pyrimidin-2-yl | N-$CO_2$Me |
| Et | Thiazol-5-yl | N-$CO_2$Me |
| Et | 1-Me-3-Cl-Pyrazol-5-yl | N-$CO_2$Me |
| Et | 1-Me-Imidazol-2-yl | N-$CO_2$Me |
| Et | Ph | N-$CO_2$Me |
| Et | 4-Cl-Ph | N-$CO_2$Me |

| | | |
|---|---|---|
| Et | 4-F-Ph | N-$CO_2$Me |
| Et | 4-Me-Ph | N-$CO_2$Me |
| Et | 4-MeO-Ph | N-$CO_2$Me |
| Et | 4-Br-Ph | N-$CO_2$Me |
| Et | 4-$CF_3$-Ph | N-$CO_2$Me |
| Et | 4-$CF_3$O-Ph | N-$CO_2$Me |
| Et | 4-MeS-Ph | N-$CO_2$Me |
| Et | 4-Me$SO_2$-Ph | N-$CO_2$Me |
| Et | 4-CHO-Ph | N-$CO_2$Me |
| Et | 4-$NO_2$-Ph | N-$CO_2$Me |
| Et | 3-CN-Ph | N-$CO_2$Me |
| Et | 4-$(Me)_2$N-Ph | N-$CO_2$Me |
| Et | 4-Ph$CH_2$O-Ph | N-$CO_2$Me |
| Et | 4-MeC(O)-Ph | N-$CO_2$Me |
| Et | 4-(4-Cl-Ph)C(O)-Ph | N-$CO_2$Me |
| Et | 4-MeO$CH_2$-Ph | N-$CO_2$Me |
| Et | 4-EtS$CH_2$-Ph | N-$CO_2$Me |
| Et | 4-$CF_3$C(O)-Ph | N-$CO_2$Me |
| Et | 4-MeC(O)O-Ph | N-$CO_2$Me |
| Et | 4-$CF_3$C(O)O-Ph | N-$CO_2$Me |
| Et | 4-PhC(O)O-Ph | N-$CO_2$Me |
| Et | 4-Ph-Ph | N-$CO_2$Me |
| Et | 4-(4-Me-Ph)O-Ph | N-$CO_2$Me |
| Et | 4-(Pyridin-2-yl)O-Ph | N-$CO_2$Me |
| Ph | H | N-$CO_2$Me |
| Ph | Cl | N-$CO_2$Me |
| Ph | Me | N-$CO_2$Me |
| Ph | Et | N-$CO_2$Me |
| Ph | $CO_2$Me | N-$CO_2$Me |
| Ph | COMe | N-$CO_2$Me |
| Ph | 2-Naphthyl | N-$CO_2$Me |
| Ph | Thiophen-2-yl | N-$CO_2$Me |
| Ph | Furan-3-yl | N-$CO_2$Me |
| Ph | 1-Me-Pyrrol-2-yl | N-$CO_2$Me |
| Ph | Pyridin-4-yl | N-$CO_2$Me |
| Ph | Pyrazin-2-yl | N-$CO_2$Me |
| Ph | Pyrimidin-4-yl | N-$CO_2$Me |
| Ph | Thiazol-2-yl | N-$CO_2$Me |
| Ph | 1-Me-3-Cl-Pyrazol-5-yl | N-$CO_2$Me |
| Ph | 1-Me-Imidazol-2-yl | N-$CO_2$Me |
| Ph | Ph | N-$CO_2$Me |
| Ph | 4-Cl-Ph | N-$CO_2$Me |
| Ph | 2-F-Ph | N-$CO_2$Me |
| Ph | 3-F-Ph | N-$CO_2$Me |
| Ph | 4-F-Ph | N-$CO_2$Me |
| Ph | 2-Me-Ph | N-$CO_2$Me |
| Ph | 3-Me-Ph | N-$CO_2$Me |
| Ph | 4-Me-Ph | N-$CO_2$Me |
| Ph | 2-MeO-Ph | N-$CO_2$Me |
| Ph | 3-MeO-Ph | N-$CO_2$Me |
| Ph | 4-MeO-Ph | N-$CO_2$Me |
| Ph | 2,4-$Cl_2$-Ph | N-$CO_2$Me |
| Ph | 4-Ph$CH_2$-Ph | N-$CO_2$Me |
| Ph | 4-$CF_3$-Ph | N-$CO_2$Me |
| Ph | 4-$CF_3$O-Ph | N-$CO_2$Me |
| Ph | 4-MeS-Ph | N-$CO_2$Me |
| Ph | 4-EtSO-Ph | N-$CO_2$Me |
| Ph | 4-Me$SO_2$-Ph | N-$CO_2$Me |

| | | |
|---|---|---|
| Ph | 4-CHO-Ph | N-$CO_2$Me |
| Ph | 4-$NO_2$-Ph | N-$CO_2$Me |
| Ph | 3-CN-Ph | N-$CO_2$Me |
| Ph | 4-CN-Ph | N-$CO_2$Me |
| Ph | 4-$(Me)_2$N-Ph | N-$CO_2$Me |
| Ph | 4-Me(MeC(O))N-Ph | N-$CO_2$Me |
| Ph | 4-PhN(Me)-Ph | N-$CO_2$Me |
| Ph | $PhCH_2$O-Ph | N-$CO_2$Me |
| Ph | 4-MeC(O)-Ph | N-$CO_2$Me |
| Ph | 4-(4-Cl-Ph)C(O)-Ph | N-$CO_2$Me |
| Ph | 4-$MeOCH_2$-Ph | N-$CO_2$Me |
| Ph | 4-$EtSCH_2$-Ph | N-$CO_2$Me |
| Ph | 4-$CF_3$C(O)-Ph | N-$CO_2$Me |
| Ph | 4-MeC(O)O-Ph | N-$CO_2$Me |
| Ph | 4-$CF_3$C(O)O-Ph | N-$CO_2$Me |
| Ph | 4-PhC(O)O-Ph | N-$CO_2$Me |
| Ph | 4-Ph-Ph | N-$CO_2$Me |
| Ph | 3-PhO-Ph | N-$CO_2$Me |
| $CO_2$Me | H | N-$CO_2$Me |
| $CO_2$Me | Cl | N-$CO_2$Me |
| $CO_2$Me | Me | N-$CO_2$Me |
| $CO_2$Me | Et | N-$CO_2$Me |
| $CO_2$Me | $CO_2$Me | N-$CO_2$Me |
| $CO_2$Me | COMe | N-$CO_2$Me |
| $CO_2$Me | 2-Naphthyl | N-$CO_2$Me |
| $CO_2$Me | Thiophen-2-yl | N-$CO_2$Me |
| $CO_2$Me | Furan-3-yl | N-$CO_2$Me |
| $CO_2$Me | 1-Me-Pyrrol-2-yl | N-$CO_2$Me |
| $CO_2$Me | Pyridin-4-yl | N-$CO_2$Me |
| $CO_2$Me | Pyrazin-2-yl | N-$CO_2$Me |
| $CO_2$Me | Pyrimidin-4-yl | N-$CO_2$Me |
| $CO_2$Me | Thiazol-2-yl | N-$CO_2$Me |
| $CO_2$Me | 1-Me-3-Cl-Pyrazol-5-yl | N-$CO_2$Me |
| $CO_2$Me | 1-Me-Imidazol-2-yl | N-$CO_2$Me |
| $CO_2$Me | Ph | N-$CO_2$Me |
| $CO_2$Me | 4-Cl-Ph | N-$CO_2$Me |
| $CO_2$Me | 4-F-Ph | N-$CO_2$Me |
| $CO_2$Me | 4-Me-Ph | N-$CO_2$Me |
| $CO_2$Me | 4-MeO-Ph | N-$CO_2$Me |
| $CO_2$Me | 4-$CF_3$-Ph | N-$CO_2$Me |
| $CO_2$Me | 4-$CF_3$O-Ph | N-$CO_2$Me |
| $CO_2$Me | 4-MeS-Ph | N-$CO_2$Me |
| $CO_2$Me | 4-EtSO-Ph | N-$CO_2$Me |
| $CO_2$Me | 4-$MeSO_2$-Ph | N-$CO_2$Me |
| $CO_2$Me | 4-CHO-Ph | N-$CO_2$Me |
| $CO_2$Me | 4-$NO_2$-Ph | N-$CO_2$Me |
| $CO_2$Me | 3-CN-Ph | N-$CO_2$Me |
| $CO_2$Me | 4-PhN(Me)-Ph | N-$CO_2$Me |
| $CO_2$Me | $PhCH_2$O-Ph | N-$CO_2$Me |
| $CO_2$Me | 4-MeC(O)-Ph | N-$CO_2$Me |
| $CO_2$Me | 4-(4-Cl-Ph)C(O)-Ph | N-$CO_2$Me |
| $CO_2$Me | 4-$MeOCH_2$-Ph | N-$CO_2$Me |
| $CO_2$Me | 4-$EtSCH_2$-Ph | N-$CO_2$Me |
| $CO_2$Me | 4-$CF_3$C(O)-Ph | N-$CO_2$Me |
| $CO_2$Me | 4-MeC(O)O-Ph | N-$CO_2$Me |
| $CO_2$Me | 4-$CF_3$C(O)O-Ph | N-$CO_2$Me |
| $CO_2$Me | 4-PhC(O)O-Ph | N-$CO_2$Me |
| $CO_2$Me | 4-Ph-Ph | N-$CO_2$Me |

| | | |
|---|---|---|
| $CO_2Me$ | 3-PhO-Ph | N-$CO_2Me$ |

(Table 3)

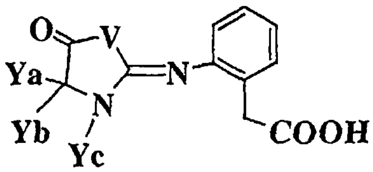、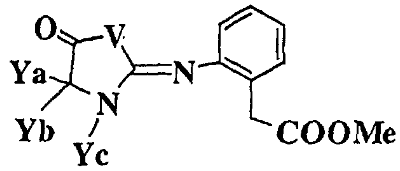、

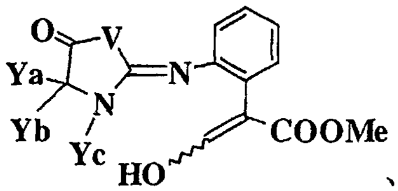、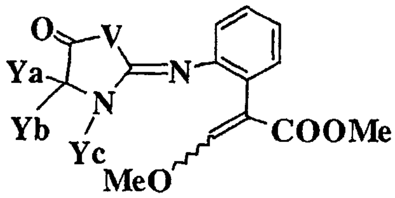、

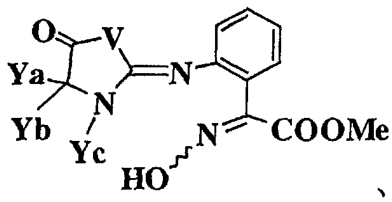、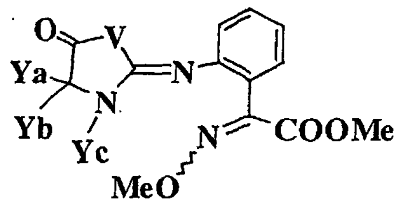、

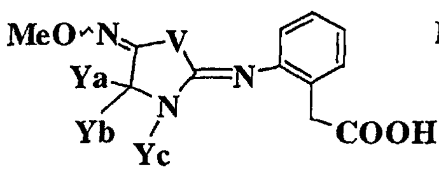、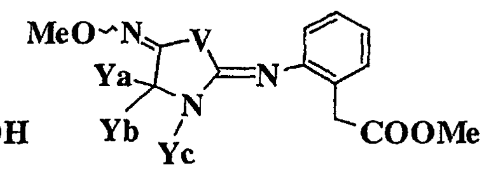、

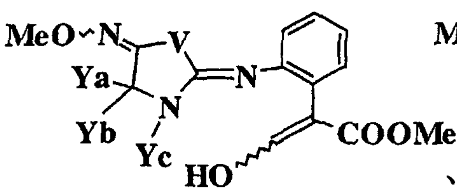、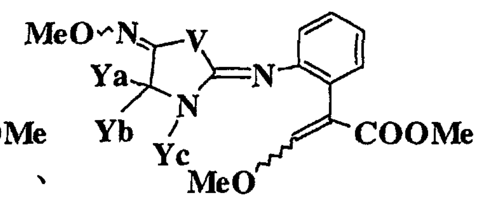、

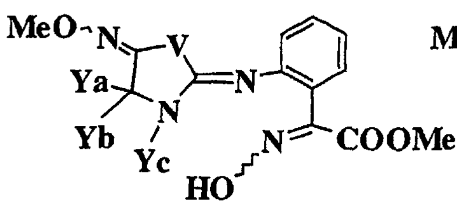、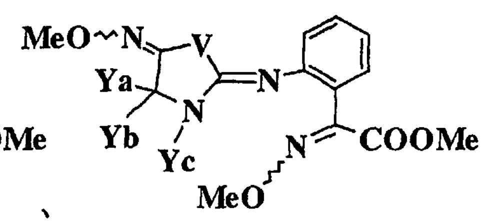、

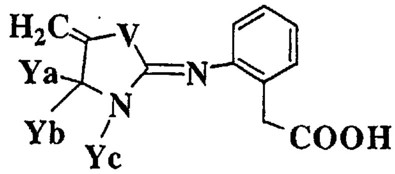 、 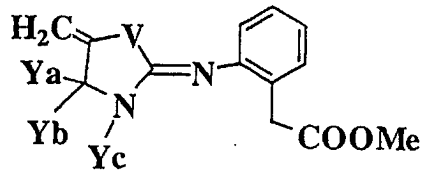 、

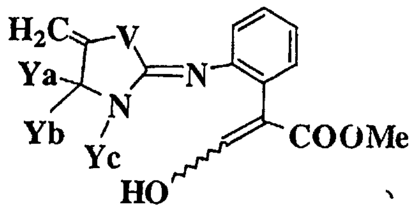 、 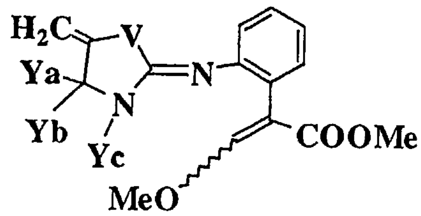 、

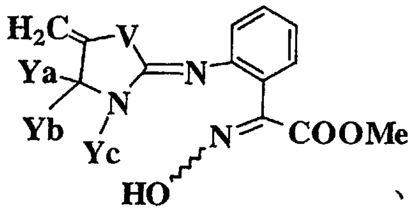 、 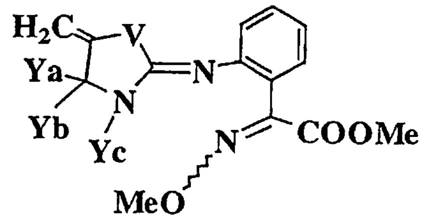 、

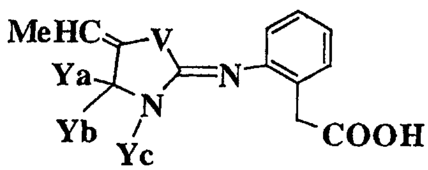 、 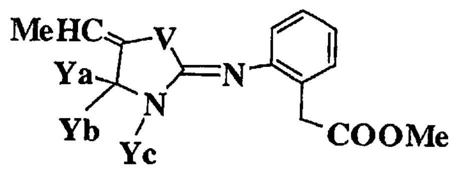 、

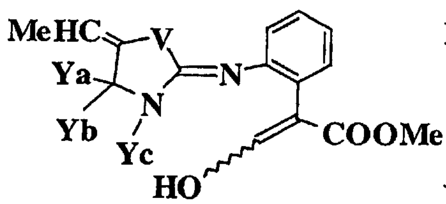 、 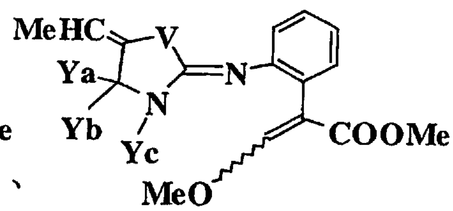 、

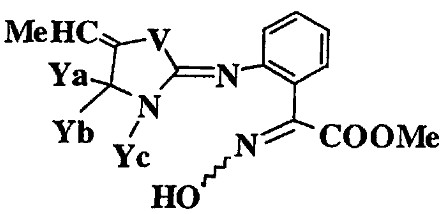 、 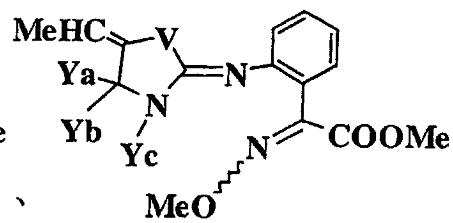 、

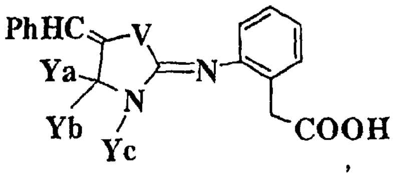
,
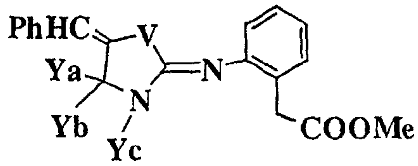
,

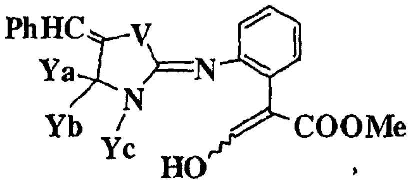
,
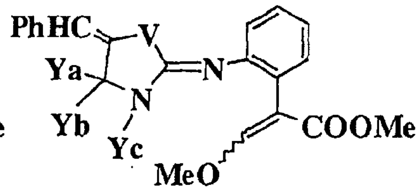
,

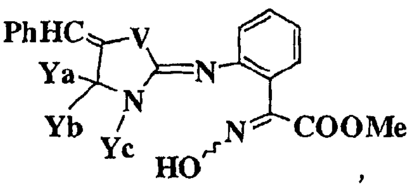
,
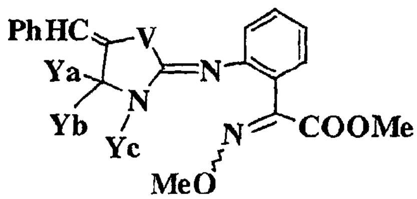
,

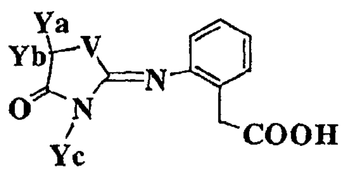
,
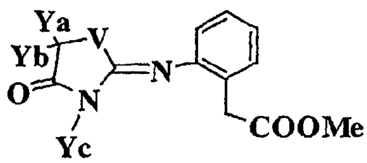
,

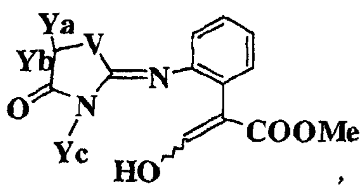
,
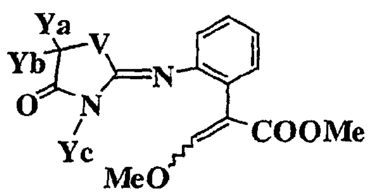
,

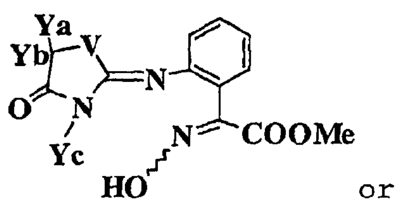
or
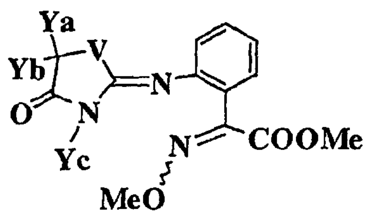

| Ya | Yb | Yc | V |
|---|---|---|---|
| H | H | Me | S |
| H | Cl | Me | S |
| H | Me | Me | S |
| H | Et | Me | S |
| H | n-Pr | Me | S |
| H | i-Pr | Me | S |
| H | n-Bu | Me | S |
| H | i-Bu | Me | S |
| H | s-Bu | Me | S |
| H | t-Bu | Me | S |
| H | n-Pen | Me | S |

| | | | |
|---|---|---|---|
| H | 3-Me-n-Bu | Me | S |
| H | n-Hex | Me | S |
| H | 1-Propenyl | Me | S |
| H | $CF_3$ | Me | S |
| H | c-Pr | Me | S |
| H | c-Hex | Me | S |
| H | MeO | Me | S |
| H | t-BuO | Me | S |
| H | MeS | Me | S |
| H | MeSO | Me | S |
| H | $MeSO_2$ | Me | S |
| H | $Me_2N$ | Me | S |
| H | $PhCH_2$ | Me | S |
| H | PhCH=CH | Me | S |
| H | PhO | Me | S |
| H | $CO_2Me$ | Me | S |
| H | $CO_2Et$ | Me | S |
| H | COMe | Me | S |
| H | $CH_2SMe$ | Me | S |
| H | $CH_2OPh$ | Me | S |
| H | $CH_2SCH_2Ph$ | Me | S |
| H | $CH_2NMe_2$ | Me | S |
| H | $Me_2C$=N-$OCH_2$ | Me | S |
| H | $CH_2N$=NCHPhMe | Me | S |
| H | Morpholino-$CH_2$ | Me | S |
| H | $CF_2Cl$ | Me | S |
| H | $ClCH_2$ | Me | S |
| H | 2-Naphthyl | Me | S |
| H | Thiophen-2-yl | Me | S |
| H | Furan-2-yl | Me | S |
| H | 1-Me-Pyrrole-3-yl | Me | S |
| H | Pyridin-2-yl | Me | S |
| H | Pyrazin-2-yl | Me | S |
| H | Pyrimidin-4-yl | Me | S |
| H | Thiazol-2-yl | Me | S |
| H | 1-Me-Pyrazol-5-yl | Me | S |
| H | 1-Me-Imidazol-2-yl | Me | S |
| H | Ph | Me | S |
| H | 4-Cl-Ph | Me | S |
| H | 4-F-Ph | Me | S |
| H | 4-Me-Ph | Me | S |
| H | 4-Br-Ph | Me | S |
| H | 2,4-$Cl_2$-Ph | Me | S |
| H | 3,4-$Cl_2$-Ph | Me | S |
| H | 2,6-$F_2$-Ph | Me | S |
| H | 4-$CF_3$-Ph | Me | S |
| H | 4-$CF_3O$-Ph | Me | S |
| H | 4-CHO-Ph | Me | S |
| H | 4-$NO_2$-Ph | Me | S |
| H | 3-CN-Ph | Me | S |
| H | 4-MeC(O)-Ph | Me | S |
| H | 4-$MeOCH_2$-Ph | Me | S |
| H | 4-$EtOCH_2$-Ph | Me | S |
| H | 4-$CF_3C$(O)-Ph | Me | S |
| H | 4-MeC(O)O-Ph | Me | S |
| H | 4-PhC(O)O-Ph | Me | S |
| H | 4-Ph-Ph | Me | S |
| Me | H | Me | S |

| | | | |
|---|---|---|---|
| H | Cl | Me | S |
| Cl | Cl | Me | S |
| Me | Cl | Me | S |
| Me | Me | Me | S |
| Me | Et | Me | S |
| Me | n-Pr | Me | S |
| Me | i-Pr | Me | S |
| Me | n-Bu | Me | S |
| Me | i-Bu | Me | S |
| Me | s-Bu | Me | S |
| Me | t-Bu | Me | S |
| Me | n-Pen | Me | S |
| Me | 3-Me-n-Bu | Me | S |
| Me | n-Hex | Me | S |
| Me | 1-Propenyl | Me | S |
| Me | $CF_3$ | Me | S |
| Me | c-Hex | Me | S |
| Me | $CO_2Me$ | Me | S |
| Me | COMe | Me | S |
| Me | $CH_2SMe$ | Me | S |
| Me | $PhOCH_2$ | Me | S |
| Me | $CH_2SCH_2Ph$ | Me | S |
| Me | $CH_2NMe_2$ | Me | S |
| Me | $Me_2C$=N-$OCH_2$ | Me | S |
| Me | $CH_2N$=NCHMePh | Me | S |
| Me | Morpholino=$CH_2$ | Me | S |
| Me | Ph | Me | S |
| Me | 4-Cl-Ph | Me | S |
| Me | 4-F-Ph | Me | S |
| Me | 4-Me-Ph | Me | S |
| Me | 4-Br-Ph | Me | S |
| Et | H | Me | S |
| Et | Cl | Me | S |
| Et | Me | Me | S |
| Et | Et | Me | S |
| Et | n-Pr | Me | S |
| Et | i-Pr | Me | S |
| Et | n-Bu | Me | S |
| Et | i-Bu | Me | S |
| Et | s-Bu | Me | S |
| Et | t-Bu | Me | S |
| Et | n-Pen | Me | S |
| Et | 3-Me-n-Bu | Me | S |
| Et | n-Hex | Me | S |
| Et | 1-Propenyl | Me | S |
| Et | $CF_3$ | Me | S |
| Et | c-Hex | Me | S |
| Et | $CO_2Me$ | Me | S |
| Et | COMe | Me | S |
| Et | $CH_2SMe$ | Me | S |
| Et | $PhOCH_2$ | Me | S |
| Et | $CH_2SCH_2Ph$ | Me | S |
| Et | $CH_2NMe_2$ | Me | S |
| Et | $Me_2C$=N-$OCH_2$ | Me | S |
| Et | $CH_2N$=NCHMePh | Me | S |
| Et | Morpholino-$CH_2$ | Me | S |
| Et | Ph | Me | S |
| Et | 4-Cl-Ph | Me | S |

| | | | | |
|---|---|---|---|---|
| Et | | 4-F-Ph | Me | S |
| Et | | 4-Me-Ph | Me | S |
| Et | | 4-Br-Ph | Me | S |
| | $-CH_2CH_2-$ | | Me | S |
| | $-CCl_2CH_2-$ | | Me | S |
| | $-CBr_2CH_2-$ | | Me | S |
| | $-OCH_2-$ | | Me | S |
| H | | H | Et | S |
| H | | Cl | Et | S |
| H | | Me | Et | S |
| H | | Et | Et | S |
| H | | n-Pr | Et | S |
| H | | i-Pr | Et | S |
| H | | 1-Propenyl | Et | S |
| H | | $CF_3$ | Et | S |
| H | | c-Pr | Et | S |
| H | | c-Hex | Et | S |
| H | | $PhCH_2$ | Et | S |
| H | | $CO_2Me$ | Et | S |
| H | | COMe | Et | S |
| H | | $CH_2SMe$ | Et | S |
| H | | $PhOCH_2$ | Et | S |
| H | | Ph | Et | S |
| H | | 4-Ph-Ph | Et | S |
| Me | | H | Et | S |
| Me | | Cl | Et | S |
| Me | | Me | Et | S |
| Me | | Et | Et | S |
| Me | | 1-Propenyl | Et | S |
| Me | | $CF_3$ | Et | S |
| Me | | $CO_2Me$ | Et | S |
| Me | | COMe | Et | S |
| Me | | $CH_2SMe$ | Et | S |
| Me | | $CH_2OPh$ | Et | S |
| Me | | $CH_2SCH_2Ph$ | Et | S |
| Me | | $CH_2NMe_2$ | Et | S |
| Me | | Ph | Et | S |
| | $-CH_2CH_2-$ | | Et | S |
| | $-CCl_2CH_2-$ | | Et | S |
| | $-CBr_2CH_2-$ | | Et | S |
| | $-OCH_2-$ | | Et | S |
| H | | H | $CO_2Me$ | S |
| H | | Cl | $CO_2Me$ | S |
| H | | Me | $CO_2Me$ | S |
| H | | Et | $CO_2Me$ | S |
| H | | n-Pr | $CO_2Me$ | S |
| H | | i-Pr | $CO_2Me$ | S |
| H | | $CF_3$ | $CO_2Me$ | S |
| H | | $CO_2Me$ | $CO_2Me$ | S |
| H | | COMe | $CO_2Me$ | S |
| H | | Ph | $CO_2Me$ | S |
| Me | | H | $CO_2Me$ | S |
| Me | | Cl | $CO_2Me$ | S |
| Me | | Me | $CO_2Me$ | S |
| Me | | Et | $CO_2Me$ | S |
| Me | | 1-Propenyl | $CO_2Me$ | S |
| Me | | $CF_3$ | $CO_2Me$ | S |
| Me | | $CO_2Me$ | $CO_2Me$ | S |

| | | | | |
|---|---|---|---|---|
| Me | | COMe | $CO_2Me$ | S |
| Me | | Ph | $CO_2Me$ | S |
| | $-CH_2CH_2-$ | | $CO_2Me$ | S |
| | $-CCl_2CH_2-$ | | $CO_2Me$ | S |
| | $-CBr_2CH_2-$ | | $CO_2Me$ | S |
| | $-OCH_2-$ | | $CO_2Me$ | S |
| H | | H | Ph | S |
| H | | Cl | Ph | S |
| H | | Me | Ph | S |
| H | | Et | Ph | S |
| H | | n-Pr | Ph | S |
| H | | i-Pr | Ph | S |
| H | | $CF_3$ | Ph | S |
| H | | $CO_2Me$ | Ph | S |
| H | | COMe | Ph | S |
| H | | Ph | Ph | S |
| Me | | H | Ph | S |
| Me | | Cl | Ph | S |
| Me | | Me | Ph | S |
| Me | | Et | Ph | S |
| Me | | 1-Propenyl | Ph | S |
| Me | | $CF_3$ | Ph | S |
| Me | | $CO_2Me$ | Ph | S |
| Me | | COMe | Ph | S |
| Me | | Ph | Ph | S |
| | $-CH_2CH_2-$ | | Ph | S |
| | $-CCl_2CH_2-$ | | Ph | S |
| | $-CBr_2CH_2-$ | | Ph | S |
| | $-OCH_2-$ | | Ph | S |
| H | | H | Me | O |
| H | | Cl | Me | O |
| H | | Me | Me | O |
| H | | Et | Me | O |
| H | | n-Pr | Me | O |
| H | | i-Pr | Me | O |
| H | | n-Bu | Me | O |
| H | | i-Bu | Me | O |
| H | | s-Bu | Me | O |
| H | | t-Bu | Me | O |
| H | | n-Pen | Me | O |
| H | | 3-Me-n-Bu | Me | O |
| H | | n-Hex | Me | O |
| H | | 1-Propenyl | Me | O |
| H | | $CF_3$ | Me | O |
| H | | c-Pr | Me | O |
| H | | c-Hex | Me | O |
| H | | MeO | Me | O |
| H | | t-BuO | Me | O |
| H | | MeS | Me | O |
| H | | MeSO | Me | O |
| H | | $MeSO_2$ | Me | O |
| H | | $Me_2N$ | Me | O |
| H | | $PhCH_2$ | Me | O |
| H | | PhCH=CH | Me | O |
| H | | PhO | Me | O |
| H | | $CO_2Me$ | Me | O |
| H | | $CO_2Et$ | Me | O |
| H | | COMe | Me | O |

| | | | |
|---|---|---|---|
| H | $CH_2SMe$ | Me | 0 |
| H | $CH_2OPh$ | Me | 0 |
| H | $CH_2SCH_2Ph$ | Me | 0 |
| H | $CH_2NMe_2$ | Me | 0 |
| H | $Me_2C=NOCH_2$ | Me | 0 |
| H | $CH_2N=NCHMePh$ | Me | 0 |
| H | Morpholino-$CH_2$ | Me | 0 |
| H | $CF_2Cl$ | Me | 0 |
| H | $ClCH_2$ | Me | 0 |
| H | 2-Naphthyl | Me | 0 |
| H | Thiophen-2-yl | Me | 0 |
| | Furan-2-yl | Me | 0 |
| | 1-Me-Pyrrole-3-yl | Me | 0 |
| | Pyridin-2-yl | Me | 0 |
| | Pyrazin-2-yl | Me | 0 |
| | Pyrimidin-4-yl | Me | 0 |
| | Thiazol-2-yl | Me | 0 |
| | 1-Me-Pyrazol-5-yl | Me | 0 |
| | 1-Me-Imidazol-2-yl | Me | 0 |
| | Ph | Me | 0 |
| | 4-Cl-Ph | Me | 0 |
| | 4-F-Ph | Me | 0 |
| H | 4-Me-Ph | Me | 0 |
| H | 4-Br-Ph | Me | 0 |
| H | 2,4-$Cl_2$-Ph | Me | 0 |
| H | 3,4-$Cl_2$-Ph | Me | 0 |
| H | 2,6-$F_2$-Ph | Me | 0 |
| H | 4-$CF_3$-Ph | Me | 0 |
| H | 4-$CF_3O$-Ph | Me | 0 |
| H | 4-CHO-Ph | Me | 0 |
| H | 4-$NO_2$-Ph | Me | 0 |
| H | 3-CN-Ph | Me | 0 |
| H | 4-MeC(O)-Ph | Me | 0 |
| H | 4-$MeOCH_2$-Ph | Me | 0 |
| H | 4-$EtOCH_2$-Ph | Me | 0 |
| H | 4-$CF_3C(O)$-Ph | Me | 0 |
| H | 4-MeC(O)O-Ph | Me | 0 |
| H | 4-PhC(O)O-Ph | Me | 0 |
| H | 4-Ph-Ph | Me | 0 |
| Me | H | Me | 0 |
| Me | Cl | Me | 0 |
| Me | Me | Me | 0 |
| Me | Et | Me | 0 |
| Me | n-Pr | Me | 0 |
| Me | i-Pr | Me | 0 |
| Me | n-Bu | Me | 0 |
| Me | i-Bu | Me | 0 |
| Me | s-Bu | Me | 0 |
| Me | t-Bu | Me | 0 |
| Me | n-Pen | Me | 0 |
| Me | 3-Me-n-Bu | Me | 0 |
| Me | n-Hex | Me | 0 |
| Me | 1-Propenyl | Me | 0 |
| Me | $CF_3$ | Me | 0 |
| Me | c-Hex | Me | 0 |
| Me | $CO_2Me$ | Me | 0 |
| Me | COMe | Me | 0 |
| Me | $CH_2SMe$ | Me | 0 |

| | | | | |
|---|---|---|---|---|
| Me | | $CH_2OPh$ | Me | 0 |
| Me | | $CH_2SCH_2Ph$ | Me | 0 |
| Me | | $CH_2NMe_2$ | Me | 0 |
| Me | | $Me_2C{=}N{-}OCH_2$ | Me | 0 |
| Me | | $CH_2N{=}NCHMePh$ | Me | 0 |
| Me | | Morpholino-$CH_2$ | Me | 0 |
| Me | | Ph | Me | 0 |
| Me | | 4-Cl-Ph | Me | 0 |
| Me | | 4-F-Ph | Me | 0 |
| Me | | 4-Me-Ph | Me | 0 |
| Me | | 4-Br-Ph | Me | 0 |
| Et | | H | Me | 0 |
| Et | | Cl | Me | 0 |
| Et | | Me | Me | 0 |
| Et | | Et | Me | 0 |
| Et | | n-Pr | Me | 0 |
| Et | | i-Pr | Me | 0 |
| Et | | n-Bu | Me | 0 |
| Et | | i-Bu | Me | 0 |
| Et | | s-Bu | Me | 0 |
| Et | | t-Bu | Me | 0 |
| Et | | n-Pen | Me | 0 |
| Et | | 3-Me-n-Bu | Me | 0 |
| Et | | n-Hex | Me | 0 |
| Et | | 1-Propenyl | Me | 0 |
| Et | | $CF_3$ | Me | 0 |
| Et | | c-Hex | Me | 0 |
| Et | | $CO_2Me$ | Me | 0 |
| Et | | COMe | Me | 0 |
| Et | | $CH_2SMe$ | Me | 0 |
| Et | | $CH_2OPh$ | Me | 0 |
| Et | | $CH_2SCH_2Ph$ | Me | 0 |
| Et | | $CH_2NMe_2$ | Me | 0 |
| Et | | $CH_2ON{=}CMe_2$ | Me | 0 |
| Et | | $CH_2N{=}NCHMePh$ | Me | 0 |
| Et | | $CH_2$(Morpholino) | Me | 0 |
| Et | | Ph | Me | 0 |
| Et | | 4-Cl-Ph | Me | 0 |
| Et | | 4-F-Ph | Me | 0 |
| Et | | 4-Me-Ph | Me | 0 |
| Et | | 4-Br-Ph | Me | 0 |
| | $-CH_2CH_2-$ | | Me | 0 |
| | $-CCl_2CH_2-$ | | Me | 0 |
| | $-CBr_2CH_2-$ | | Me | 0 |
| | $-OCH_2-$ | | Me | 0 |
| H | | H | Et | 0 |
| H | | Cl | Et | 0 |
| H | | Me | Et | 0 |
| H | | Et | Et | 0 |
| H | | n-Pr | Et | 0 |
| H | | i-Pr | Et | 0 |
| H | | 1-Propenyl | Et | 0 |
| H | | $CF_3$ | Et | 0 |
| H | | c-Pr | Et | 0 |
| H | | c-Hex | Et | 0 |
| H | | $CH_2Ph$ | Et | 0 |
| H | | $CO_2Me$ | Et | 0 |
| H | | COMe | Et | 0 |

| | | | | |
|---|---|---|---|---|
| H | | $CH_2SMe$ | Et | 0 |
| H | | $CH_2OPh$ | Et | 0 |
| H | | Ph | Et | 0 |
| H | | 4-Ph-Ph | Et | 0 |
| Me | | H | Et | 0 |
| Me | | Cl | Et | 0 |
| Me | | Me | Et | 0 |
| Me | | Et | Et | 0 |
| Me | | 1-Propenyl | Et | 0 |
| Me | | $CF_3$ | Et | 0 |
| Me | | $CO_2Me$ | Et | 0 |
| Me | | COMe | Et | 0 |
| Me | | $CH_2SMe$ | Et | 0 |
| Me | | $CH_2OPh$ | Et | 0 |
| Me | | $CH_2SCH_2Ph$ | Et | 0 |
| Me | | $CH_2NMe_2$ | Et | 0 |
| Me | | Ph | Et | 0 |
| | $-CH_2CH_2-$ | | Et | 0 |
| | $-CCl_2CH_2-$ | | Et | 0 |
| | $-CBr_2CH_2-$ | | Et | 0 |
| | $-OCH_2-$ | | Et | 0 |
| H | | H | $CO_2Me$ | 0 |
| H | | Cl | $CO_2Me$ | 0 |
| H | | Me | $CO_2Me$ | 0 |
| H | | Et | $CO_2Me$ | 0 |
| H | | n-Pr | $CO_2Me$ | 0 |
| H | | i-Pr | $CO_2Me$ | 0 |
| H | | $CF_3$ | $CO_2Me$ | 0 |
| H | | $CO_2Me$ | $CO_2Me$ | 0 |
| H | | COMe | $CO_2Me$ | 0 |
| H | | Ph | $CO_2Me$ | 0 |
| Me | | H | $CO_2Me$ | 0 |
| Me | | Cl | $CO_2Me$ | 0 |
| Me | | Me | $CO_2Me$ | 0 |
| Me | | Et | $CO_2Me$ | 0 |
| Me | | 1-Propenyl | $CO_2Me$ | 0 |
| Me | | $CF_3$ | $CO_2Me$ | 0 |
| Me | | $CO_2Me$ | $CO_2Me$ | 0 |
| Me | | COMe | $CO_2Me$ | 0 |
| Me | | Ph | $CO_2Me$ | 0 |
| | $-CH_2CH_2-$ | | $CO_2Me$ | 0 |
| | $-CCl_2CH_2-$ | | $CO_2Me$ | 0 |
| | $-CBr_2CH_2-$ | | $CO_2Me$ | 0 |
| | $-OCH_2-$ | | $CO_2Me$ | 0 |
| H | | H | Ph | 0 |
| H | | Cl | Ph | 0 |
| H | | Me | Ph | 0 |
| H | | Et | Ph | 0 |
| H | | n-Pr | Ph | 0 |
| H | | i-Pr | Ph | 0 |
| H | | $CF_3$ | Ph | 0 |
| H | | $CO_2Me$ | Ph | 0 |
| H | | COMe | Ph | 0 |
| H | | Ph | Ph | 0 |
| Me | | H | Ph | 0 |
| Me | | Cl | Ph | 0 |
| Me | | Me | Ph | 0 |
| Me | | Et | Ph | 0 |

| | | | | |
|---|---|---|---|---|
| Me | | 1-Propenyl | Ph | O |
| Me | | $CF_3$ | Ph | O |
| Me | | $CO_2Me$ | Ph | O |
| Me | | COMe | Ph | O |
| Me | | Ph | Ph | O |
| | $-CH_2CH_2-$ | | Ph | O |
| | $-CCl_2CH_2-$ | | Ph | O |
| | $-CBr_2CH_2-$ | | Ph | O |
| | $-OCH_2-$ | | Ph | O |
| H | | H | Me | N-Me |
| H | | Cl | Me | N-Me |
| H | | Me | Me | N-Me |
| H | | Et | Me | N-Me |
| H | | n-Pr | Me | N-Me |
| H | | i-Pr | Me | N-Me |
| H | | n-Bu | Me | N-Me |
| H | | i-Bu | Me | N-Me |
| H | | s-Bu | Me | N-Me |
| H | | t-Bu | Me | N-Me |
| H | | n-Pen | Me | N-Me |
| H | | 3-Me-n-Bu | Me | N-Me |
| H | | n-Hex | Me | N-Me |
| H | | 1-Propenyl | Me | N-Me |
| H | | $CF_3$ | Me | N-Me |
| H | | c-Pr | Me | N-Me |
| H | | c-Hex | Me | N-Me |
| H | | OMe | Me | N-Me |
| H | | O(t-Bu) | Me | N-Me |
| H | | SMe | Me | N-Me |
| H | | SOMe | Me | N-Me |
| H | | $SO_2Me$ | Me | N-Me |
| H | | $NMe_2$ | Me | N-Me |
| H | | $CH_2Ph$ | Me | N-Me |
| H | | CH=CHPh | Me | N-Me |
| H | | OPh | Me | N-Me |
| H | | $CO_2Me$ | Me | N-Me |
| H | | $CO_2Et$ | Me | N-Me |
| H | | COMe | Me | N-Me |
| H | | $CH_2SMe$ | Me | N-Me |
| H | | $CH_2OPh$ | Me | N-Me |
| H | | $CH_2SCH_2Ph$ | Me | N-Me |
| H | | $CH_2NMe_2$ | Me | N-Me |
| H | | $CH_2ON=CMe_2$ | Me | N-Me |
| H | | $CH_2N=NCHMePh$ | Me | N-Me |
| H | | $CH_2$(Morpholino) | Me | N-Me |
| H | | $CF_2Cl$ | Me | N-Me |
| H | | $ClCH_2$ | Me | N-Me |
| H | | 2-Naphthyl | Me | N-Me |
| H | | Thiophen-2-yl | Me | N-Me |
| H | | Furan-2-yl | Me | N-Me |
| H | | 1-Me-Pyrrole-3-yl | Me | N-Me |
| H | | Pyridin-2-yl | Me | N-Me |
| H | | Pyrazin-2-yl | Me | N-Me |
| H | | Pyrimidin-4-yl | Me | N-Me |
| H | | Thiazol-2-yl | Me | N-Me |
| H | | 1-Me-Pyrazol-5-yl | Me | N-Me |
| H | | 1-Me-Imidazol-2-yl | Me | N-Me |
| H | | Ph | Me | N-Me |

| | | | |
|---|---|---|---|
| H | 4-Cl-Ph | Me | N-Me |
| H | 4-F-Ph | Me | N-Me |
| H | 4-Me-Ph | Me | N-Me |
| H | 4-Br-Ph | Me | N-Me |
| H | 2,4-$Cl_2$-Ph | Me | N-Me |
| H | 3,4-$Cl_2$-Ph | Me | N-Me |
| H | 2,6-$F_2$-Ph | Me | N-Me |
| H | 4-$CF_3$-Ph | Me | N-Me |
| H | 4-$CF_3O$-Ph | Me | N-Me |
| H | 4-CHO-Ph | Me | N-Me |
| H | 4-$NO_2$-Ph | Me | N-Me |
| H | 3-CN-Ph | Me | N-Me |
| H | 4-MeC(O)-Ph | Me | N-Me |
| H | 4-$MeOCH_2$-Ph | Me | N-Me |
| H | 4-$EtOCH_2$-Ph | Me | N-Me |
| H | 4-$CF_3C(O)$-Ph | Me | N-Me |
| H | 4-MeC(O)O-Ph | Me | N-Me |
| H | 4-PhC(O)O-Ph | Me | N-Me |
| H | 4-Ph-Ph | Me | N-Me |
| Me | H | Me | N-Me |
| Me | Cl | Me | N-Me |
| Me | Me | Me | N-Me |
| Me | Et | Me | N-Me |
| Me | n-Pr | Me | N-Me |
| Me | i-Pr | Me | N-Me |
| Me | n-Bu | Me | N-Me |
| Me | i-Bu | Me | N-Me |
| Me | s-Bu | Me | N-Me |
| Me | t-Bu | Me | N-Me |
| Me | n-Pen | Me | N-Me |
| Me | 3-Me-n-Bu | Me | N-Me |
| Me | n-Hex | Me | N-Me |
| Me | 1-Propenyl | Me | N-Me |
| Me | $CF_3$ | Me | N-Me |
| Me | c-Hex | Me | N-Me |
| Me | $CO_2Me$ | Me | N-Me |
| Me | COMe | Me | N-Me |
| Me | $CH_2SMe$ | Me | N-Me |
| Me | $CH_2OPh$ | Me | N-Me |
| Me | $CH_2SCH_2Ph$ | Me | N-Me |
| Me | $CH_2NMe_2$ | Me | N-Me |
| Me | $CH_2ON=CMe_2$ | Me | N-Me |
| Me | $CH_2N=NCHMePh$ | Me | N-Me |
| Me | $CH_2$(Morpholino) | Me | N-Me |
| Me | Ph | Me | N-Me |
| Me | 4-Cl-Ph | Me | N-Me |
| Me | 4-F-Ph | Me | N-Me |
| Me | 4-Me-Ph | Me | N-Me |
| Me | 4-Br-Ph | Me | N-Me |
| Et | H | Me | N-Me |
| Et | Cl | Me | N-Me |
| Et | Me | Me | N-Me |
| Et | Et | Me | N-Me |
| Et | n-Pr | Me | N-Me |
| Et | i-Pr | Me | N-Me |
| Et | n-Bu | Me | N-Me |
| Et | i-Bu | Me | N-Me |
| Et | s-Bu | Me | N-Me |

| | | | | |
|---|---|---|---|---|
| Et | | t-Bu | Me | N-Me |
| Et | | n-Pen | Me | N-Me |
| Et | | 3-Me-n-Bu | Me | N-Me |
| Et | | n-Hex | Me | N-Me |
| Et | | 1-Propenyl | Me | N-Me |
| Et | | $CF_3$ | Me | N-Me |
| Et | | c-Hex | Me | N-Me |
| Et | | $CO_2Me$ | Me | N-Me |
| Et | | COMe | Me | N-Me |
| Et | | $CH_2SMe$ | Me | N-Me |
| Et | | $CH_2OPh$ | Me | N-Me |
| Et | | $CH_2SCH_2Ph$ | Me | N-Me |
| Et | | $CH_2NMe_2$ | Me | N-Me |
| Et | | $CH_2ON{=}CMe_2$ | Me | N-Me |
| Et | | $CH_2N{=}NCHMePh$ | Me | N-Me |
| Et | | $CH_2$(Morpholino) | Me | N-Me |
| Et | | Ph | Me | N-Me |
| Et | | 4-Cl-Ph | Me | N-Me |
| Et | | 4-F-Ph | Me | N-Me |
| Et | | 4-Me-Ph | Me | N-Me |
| Et | | 4-Br-Ph | Me | N-Me |
| | $-CH_2CH_2-$ | | Me | N-Me |
| | $-CCl_2CH_2-$ | | Me | N-Me |
| | $-CBr_2CH_2-$ | | Me | N-Me |
| | $-OCH_2-$ | | Me | N-Me |
| H | | H | Et | N-Me |
| H | | Cl | Et | N-Me |
| H | | Me | Et | N-Me |
| H | | Et | Et | N-Me |
| H | | n-Pr | Et | N-Me |
| H | | i-Pr | Et | N-Me |
| H | | 1-Propenyl | Et | N-Me |
| H | | $CF_3$ | Et | N-Me |
| H | | c-Pr | Et | N-Me |
| H | | c-Hex | Et | N-Me |
| H | | $CH_2Ph$ | Et | N-Me |
| H | | $CO_2Me$ | Et | N-Me |
| H | | COMe | Et | N-Me |
| H | | $CH_2SMe$ | Et | N-Me |
| H | | $CH_2OPh$ | Et | N-Me |
| H | | Ph | Et | N-Me |
| H | | 4-Ph-Ph | Et | N-Me |
| Me | | H | Et | N-Me |
| Me | | Cl | Et | N-Me |
| Me | | Me | Et | N-Me |
| Me | | Et | Et | N-Me |
| Me | | 1-Propenyl | Et | N-Me |
| Me | | $CF_3$ | Et | N-Me |
| Me | | $CO_2Me$ | Et | N-Me |
| Me | | COMe | Et | N-Me |
| Me | | $CH_2SMe$ | Et | N-Me |
| Me | | $CH_2OPh$ | Et | N-Me |
| Me | | $CH_2SCH_2Ph$ | Et | N-Me |
| Me | | $CH_2NMe_2$ | Et | N-Me |
| Me | | Ph | Et | N-Me |
| | $-CH_2CH_2-$ | | Et | N-Me |
| | $-CCl_2CH_2-$ | | Et | N-Me |
| | $-CBr_2CH_2-$ | | Et | N-Me |

| | | | | |
|---|---|---|---|---|
| | $-OCH_2-$ | | Et | N-Me |
| H | | H | $CO_2Me$ | N-Me |
| H | | Cl | $CO_2Me$ | N-Me |
| H | | Me | $CO_2Me$ | N-Me |
| H | | Et | $CO_2Me$ | N-Me |
| H | | n-Pr | $CO_2Me$ | N-Me |
| H | | i-Pr | $CO_2Me$ | N-Me |
| H | | $CF_3$ | $CO_2Me$ | N-Me |
| H | | $CO_2Me$ | $CO_2Me$ | N-Me |
| H | | COMe | $CO_2Me$ | N-Me |
| H | | Ph | $CO_2Me$ | N-Me |
| Me | | H | $CO_2Me$ | N-Me |
| Me | | Cl | $CO_2Me$ | N-Me |
| Me | | Me | $CO_2Me$ | N-Me |
| Me | | Et | $CO_2Me$ | N-Me |
| Me | | 1-Propenyl | $CO_2Me$ | N-Me |
| Me | | $CF_3$ | $CO_2Me$ | N-Me |
| Me | | $CO_2Me$ | $CO_2Me$ | N-Me |
| Me | | COMe | $CO_2Me$ | N-Me |
| Me | | Ph | $CO_2Me$ | N-Me |
| | $-CH_2CH_2-$ | | $CO_2Me$ | N-Me |
| | $-CCl_2CH_2-$ | | $CO_2Me$ | N-Me |
| | $-CBr_2CH_2-$ | | $CO_2Me$ | N-Me |
| | $-OCH_2-$ | | $CO_2Me$ | N-Me |
| H | | H | Ph | N-Me |
| H | | Cl | Ph | N-Me |
| H | | Me | Ph | N-Me |
| H | | Et | Ph | N-Me |
| H | | n-Pr | Ph | N-Me |
| H | | i-Pr | Ph | N-Me |
| H | | $CF_3$ | Ph | N-Me |
| H | | $CO_2Me$ | Ph | N-Me |
| H | | COMe | Ph | N-Me |
| H | | Ph | Ph | N-Me |
| Me | | H | Ph | N-Me |
| Me | | Cl | Ph | N-Me |
| Me | | Me | Ph | N-Me |
| Me | | Et | Ph | N-Me |
| Me | | 1-Propenyl | Ph | N-Me |
| Me | | $CF_3$ | Ph | N-Me |
| Me | | $CO_2Me$ | Ph | N-Me |
| Me | | COMe | Ph | N-Me |
| Me | | Ph | Ph | N-Me |
| | $-CH_2CH_2-$ | | Ph | N-Me |
| | $-CCl_2CH_2-$ | | Ph | N-Me |
| | $-CBr_2CH_2-$ | | Ph | N-Me |
| | $-OCH_2-$ | | Ph | N-Me |
| H | | H | Me | $N-CO_2Me$ |
| H | | Cl | Me | $N-CO_2Me$ |
| H | | Me | Me | $N-CO_2Me$ |
| H | | Et | Me | $N-CO_2Me$ |
| H | | n-Pr | Me | $N-CO_2Me$ |
| H | | i-Pr | Me | $N-CO_2Me$ |
| H | | n-Bu | Me | $N-CO_2Me$ |
| H | | i-Bu | Me | $N-CO_2Me$ |
| H | | s-Bu | Me | $N-CO_2Me$ |
| H | | t-Bu | Me | $N-CO_2Me$ |
| H | | n-Pen | Me | $N-CO_2Me$ |

| | | | |
|---|---|---|---|
| H | 3-Me-n-Bu | Me | N-$CO_2$Me |
| H | n-Hex | Me | N-$CO_2$Me |
| H | 1-Propenyl | Me | N-$CO_2$Me |
| H | $CF_3$ | Me | N-$CO_2$Me |
| H | c-Pr | Me | N-$CO_2$Me |
| H | c-Hex | Me | N-$CO_2$Me |
| H | OMe | Me | N-$CO_2$Me |
| H | O(t-Bu) | Me | N-$CO_2$Me |
| H | SMe | Me | N-$CO_2$Me |
| H | SOMe | Me | N-$CO_2$Me |
| H | $SO_2$Me | Me | N-$CO_2$Me |
| H | $NMe_2$ | Me | N-$CO_2$Me |
| H | $CH_2$Ph | Me | N-$CO_2$Me |
| H | CH=CHPh | Me | N-$CO_2$Me |
| H | OPh | Me | N-$CO_2$Me |
| H | $CO_2$Me | Me | N-$CO_2$Me |
| H | $CO_2$Et | Me | N-$CO_2$Me |
| H | COMe | Me | N-$CO_2$Me |
| H | $CH_2$SMe | Me | N-$CO_2$Me |
| H | $CH_2$OPh | Me | N-$CO_2$Me |
| H | $CH_2SCH_2$Ph | Me | N-$CO_2$Me |
| H | $CH_2NMe_2$ | Me | N-$CO_2$Me |
| H | $CH_2$ON=$CMe_2$ | Me | N-$CO_2$Me |
| H | $CH_2$N=NCHMePh | Me | N-$CO_2$Me |
| H | $CH_2$(Morpholino) | Me | N-$CO_2$Me |
| H | $CF_2$Cl | Me | N-$CO_2$Me |
| H | Cl$CH_2$ | Me | N-$CO_2$Me |
| H | 2-Naphthyl | Me | N-$CO_2$Me |
| H | Thiophen-2-yl | Me | N-$CO_2$Me |
| H | Furan-2-yl | Me | N-$CO_2$Me |
| H | 1-Me-Pyrrole-3-yl | Me | N-$CO_2$Me |
| H | Pyridin-2-yl | Me | N-$CO_2$Me |
| H | Pyrazin-2-yl | Me | N-$CO_2$Me |
| H | Pyrimidin-4-yl | Me | N-$CO_2$Me |
| H | Thiazol-2-yl | Me | N-$CO_2$Me |
| H | 1-Me-Pyrazol-5-yl | Me | N-$CO_2$Me |
| H | 1-Me-Imidazol-2-yl | Me | N-$CO_2$Me |
| H | Ph | Me | N-$CO_2$Me |
| H | 4-Cl-Ph | Me | N-$CO_2$Me |
| H | 4-F-Ph | Me | N-$CO_2$Me |
| H | 4-Me-Ph | Me | N-$CO_2$Me |
| H | 4-Br-Ph | Me | N-$CO_2$Me |
| H | 2,4-$Cl_2$-Ph | Me | N-$CO_2$Me |
| H | 3,4-$Cl_2$-Ph | Me | N-$CO_2$Me |
| H | 2,6-$F_2$-Ph | Me | N-$CO_2$Me |
| H | 4-$CF_3$-Ph | Me | N-$CO_2$Me |
| H | 4-$CF_3$O-Ph | Me | N-$CO_2$Me |
| H | 4-CHO-Ph | Me | N-$CO_2$Me |
| H | 4-$NO_2$-Ph | Me | N-$CO_2$Me |
| H | 3-CN-Ph | Me | N-$CO_2$Me |
| H | 4-MeC(O)-Ph | Me | N-$CO_2$Me |
| H | 4-MeO$CH_2$-Ph | Me | N-$CO_2$Me |
| H | 4-EtO$CH_2$-Ph | Me | N-$CO_2$Me |
| H | 4-$CF_3$C(O)-Ph | Me | N-$CO_2$Me |
| H | 4-MeC(O)O-Ph | Me | N-$CO_2$Me |
| H | 4-PhC(O)O-Ph | Me | N-$CO_2$Me |
| H | 4-Ph-Ph | Me | N-$CO_2$Me |
| Me | H | Me | N-$CO_2$Me |

| | | | |
|---|---|---|---|
| Me | Cl | Me | N-$CO_2$Me |
| Me | Me | Me | N-$CO_2$Me |
| Me | Et | Me | N-$CO_2$Me |
| Me | n-Pr | Me | N-$CO_2$Me |
| Me | i-Pr | Me | N-$CO_2$Me |
| Me | n-Bu | Me | N-$CO_2$Me |
| Me | i-Bu | Me | N-$CO_2$Me |
| Me | s-Bu | Me | N-$CO_2$Me |
| Me | t-Bu | Me | N-$CO_2$Me |
| Me | n-Pen | Me | N-$CO_2$Me |
| Me | 3-Me-n-Bu | Me | N-$CO_2$Me |
| Me | n-Hex | Me | N-$CO_2$Me |
| Me | 1-Propenyl | Me | N-$CO_2$Me |
| Me | $CF_3$ | Me | N-$CO_2$Me |
| Me | c-Hex | Me | N-$CO_2$Me |
| Me | $CO_2$Me | Me | N-$CO_2$Me |
| Me | COMe | Me | N-$CO_2$Me |
| Me | $CH_2$SMe | Me | N-$CO_2$Me |
| Me | $CH_2$OPh | Me | N-$CO_2$Me |
| Me | $CH_2SCH_2$Ph | Me | N-$CO_2$Me |
| Me | $CH_2NMe_2$ | Me | N-$CO_2$Me |
| Me | $CH_2ON=CMe_2$ | Me | N-$CO_2$Me |
| Me | $CH_2$N=NCHMePh | Me | N-$CO_2$Me |
| Me | $CH_2$(Morpholino) | Me | N-$CO_2$Me |
| Me | Ph | Me | N-$CO_2$Me |
| Me | 4-Cl-Ph | Me | N-$CO_2$Me |
| Me | 4-F-Ph | Me | N-$CO_2$Me |
| Me | 4-Me-Ph | Me | N-$CO_2$Me |
| Me | 4-Br-Ph | Me | N-$CO_2$Me |
| Et | H | Me | N-$CO_2$Me |
| Et | Cl | Me | N-$CO_2$Me |
| Et | Me | Me | N-$CO_2$Me |
| Et | Et | Me | N-$CO_2$Me |
| Et | n-Pr | Me | N-$CO_2$Me |
| Et | i-Pr | Me | N-$CO_2$Me |
| Et | n-Bu | Me | N-$CO_2$Me |
| Et | i-Bu | Me | N-$CO_2$Me |
| Et | s-Bu | Me | N-$CO_2$Me |
| Et | t-Bu | Me | N-$CO_2$Me |
| Et | n-Pen | Me | N-$CO_2$Me |
| Et | 3-Me-n-Bu | Me | N-$CO_2$Me |
| Et | n-Hex | Me | N-$CO_2$Me |
| Et | 1-Propenyl | Me | N-$CO_2$Me |
| Et | $CF_3$ | Me | N-$CO_2$Me |
| Et | c-Hex | Me | N-$CO_2$Me |
| Et | $CO_2$Me | Me | N-$CO_2$Me |
| Et | COMe | Me | N-$CO_2$Me |
| Et | $CH_2$SMe | Me | N-$CO_2$Me |
| Et | $CH_2$OPh | Me | N-$CO_2$Me |
| Et | $CH_2SCH_2$Ph | Me | N-$CO_2$Me |
| Et | $CH_2NMe_2$ | Me | N-$CO_2$Me |
| Et | $CH_2ON=CMe_2$ | Me | N-$CO_2$Me |
| Et | $CH_2$N=NCHMePh | Me | N-$CO_2$Me |
| Et | $CH_2$(Morpholino) | Me | N-$CO_2$Me |
| Et | Ph | Me | N-$CO_2$Me |
| Et | 4-Cl-Ph | Me | N-$CO_2$Me |
| Et | 4-F-Ph | Me | N-$CO_2$Me |
| Et | 4-Me-Ph | Me | N-$CO_2$Me |

| | | | | |
|---|---|---|---|---|
| Et | | 4-Br-Ph | Me | $N-CO_2Me$ |
| | $-CH_2CH_2-$ | | Me | $N-CO_2Me$ |
| | $-CCl_2CH_2-$ | | Me | $N-CO_2Me$ |
| | $-CBr_2CH_2-$ | | Me | $N-CO_2Me$ |
| | $-OCH_2-$ | | Me | $N-CO_2Me$ |
| H | | H | Et | $N-CO_2Me$ |
| H | | Cl | Et | $N-CO_2Me$ |
| H | | Me | Et | $N-CO_2Me$ |
| H | | Et | Et | $N-CO_2Me$ |
| H | | n-Pr | Et | $N-CO_2Me$ |
| H | | i-Pr | Et | $N-CO_2Me$ |
| H | | 1-Propenyl | Et | $N-CO_2Me$ |
| H | | $CF_3$ | Et | $N-CO_2Me$ |
| H | | c-Pr | Et | $N-CO_2Me$ |
| H | | c-Hex | Et | $N-CO_2Me$ |
| H | | $CH_2Ph$ | Et | $N-CO_2Me$ |
| H | | $CO_2Me$ | Et | $N-CO_2Me$ |
| H | | COMe | Et | $N-CO_2Me$ |
| H | | $CH_2SMe$ | Et | $N-CO_2Me$ |
| H | | $CH_2OPh$ | Et | $N-CO_2Me$ |
| H | | Ph | Et | $N-CO_2Me$ |
| H | | 4-Ph-Ph | Et | $N-CO_2Me$ |
| Me | | H | Et | $N-CO_2Me$ |
| Me | | Cl | Et | $N-CO_2Me$ |
| Me | | Me | Et | $N-CO_2Me$ |
| Me | | Et | Et | $N-CO_2Me$ |
| Me | | 1-Propenyl | Et | $N-CO_2Me$ |
| Me | | $CF_3$ | Et | $N-CO_2Me$ |
| Me | | $CO_2Me$ | Et | $N-CO_2Me$ |
| Me | | COMe | Et | $N-CO_2Me$ |
| Me | | $CH_2SMe$ | Et | $N-CO_2Me$ |
| Me | | $CH_2OPh$ | Et | $N-CO_2Me$ |
| Me | | $CH_2SCH_2Ph$ | Et | $N-CO_2Me$ |
| Me | | $CH_2NMe_2$ | Et | $N-CO_2Me$ |
| Me | | Ph | Et | $N-CO_2Me$ |
| | $-CH_2CH_2-$ | | Et | $N-CO_2Me$ |
| | $-CCl_2CH_2-$ | | Et | $N-CO_2Me$ |
| | $-CBr_2CH_2-$ | | Et | $N-CO_2Me$ |
| | $-OCH_2-$ | | Et | $N-CO_2Me$ |
| H | | H | $CO_2Me$ | $N-CO_2Me$ |
| H | | Cl | $CO_2Me$ | $N-CO_2Me$ |
| H | | Me | $CO_2Me$ | $N-CO_2Me$ |
| H | | Et | $CO_2Me$ | $N-CO_2Me$ |
| H | | n-Pr | $CO_2Me$ | $N-CO_2Me$ |
| H | | i-Pr | $CO_2Me$ | $N-CO_2Me$ |
| H | | $CF_3$ | $CO_2Me$ | $N-CO_2Me$ |
| H | | $CO_2Me$ | $CO_2Me$ | $N-CO_2Me$ |
| H | | COMe | $CO_2Me$ | $N-CO_2Me$ |
| H | | Ph | $CO_2Me$ | $N-CO_2Me$ |
| Me | | H | $CO_2Me$ | $N-CO_2Me$ |
| Me | | Cl | $CO_2Me$ | $N-CO_2Me$ |
| Me | | Me | $CO_2Me$ | $N-CO_2Me$ |
| Me | | Et | $CO_2Me$ | $N-CO_2Me$ |
| Me | | 1-Propenyl | $CO_2Me$ | $N-CO_2Me$ |
| Me | | $CF_3$ | $CO_2Me$ | $N-CO_2Me$ |
| Me | | $CO_2Me$ | $CO_2Me$ | $N-CO_2Me$ |
| Me | | COMe | $CO_2Me$ | $N-CO_2Me$ |
| Me | | Ph | $CO_2Me$ | $N-CO_2Me$ |

| | | | | |
|---|---|---|---|---|
| | $-CH_2CH_2-$ | | $CO_2Me$ | $N-CO_2Me$ |
| | $-CCl_2CH_2-$ | | $CO_2Me$ | $N-CO_2Me$ |
| | $-CBr_2CH_2-$ | | $CO_2Me$ | $N-CO_2Me$ |
| | $-OCH_2-$ | | $CO_2Me$ | $N-CO_2Me$ |
| H | | H | Ph | $N-CO_2Me$ |
| H | | Cl | Ph | $N-CO_2Me$ |
| H | | Me | Ph | $N-CO_2Me$ |
| H | | Et | Ph | $N-CO_2Me$ |
| H | | n-Pr | Ph | $N-CO_2Me$ |
| H | | i-Pr | Ph | $N-CO_2Me$ |
| H | | $CF_3$ | Ph | $N-CO_2Me$ |
| H | | $CO_2Me$ | Ph | $N-CO_2Me$ |
| H | | COMe | Ph | $N-CO_2Me$ |
| H | | Ph | Ph | $N-CO_2Me$ |
| Me | | H | Ph | $N-CO_2Me$ |
| Me | | Cl | Ph | $N-CO_2Me$ |
| Me | | Me | Ph | $N-CO_2Me$ |
| Me | | Et | Ph | $N-CO_2Me$ |
| Me | | 1-Propenyl | Ph | $N-CO_2Me$ |
| Me | | $CF_3$ | Ph | $N-CO_2Me$ |
| Me | | $CO_2Me$ | Ph | $N-CO_2Me$ |
| Me | | COMe | Ph | $N-CO_2Me$ |
| Me | | Ph | Ph | $N-CO_2Me$ |
| | $-CH_2CH_2-$ | | Ph | $N-CO_2Me$ |
| | $-CCl_2CH_2-$ | | Ph | $N-CO_2Me$ |
| | $-CBr_2CH_2-$ | | Ph | $N-CO_2Me$ |
| | $-OCH_2-$ | | Ph | $N-CO_2Me$ |

(Table 4)

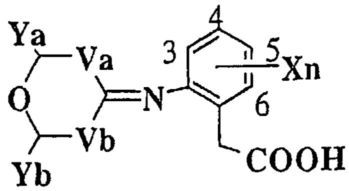,
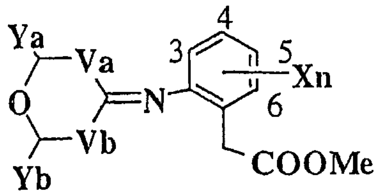,
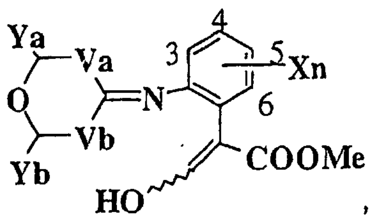,
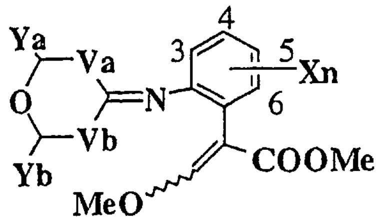,
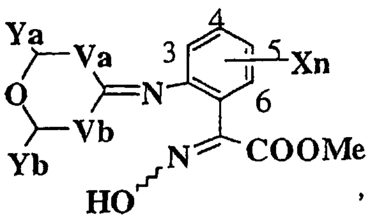,
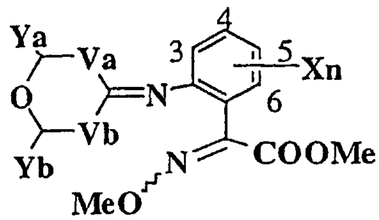,
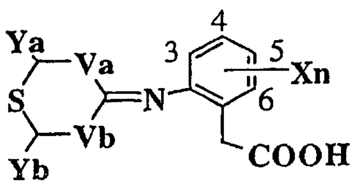,
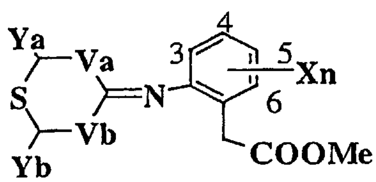,
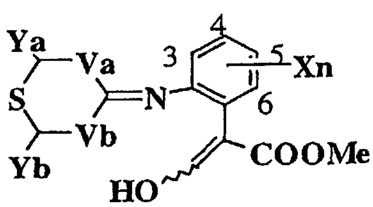,
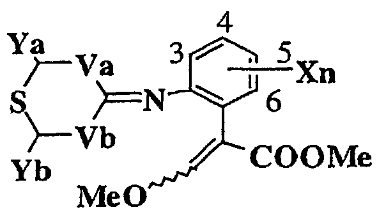,
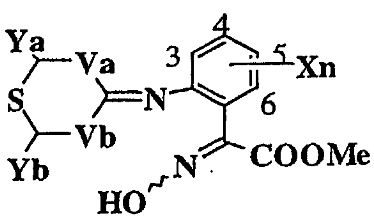,
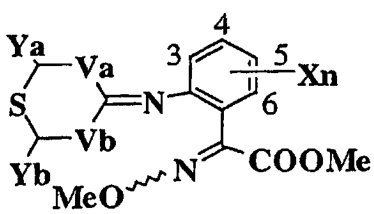,

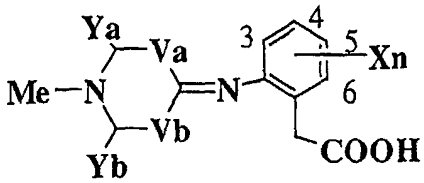

,

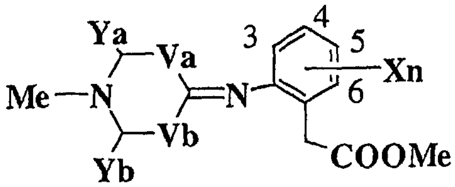

,

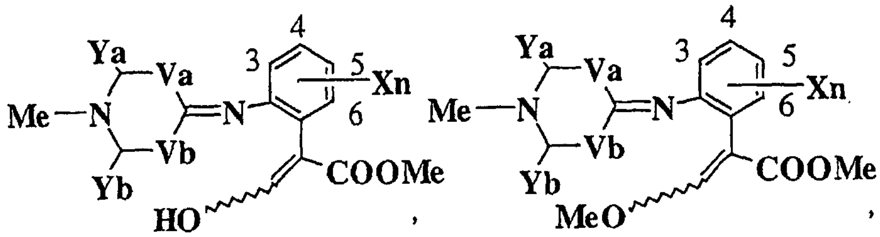

,

,

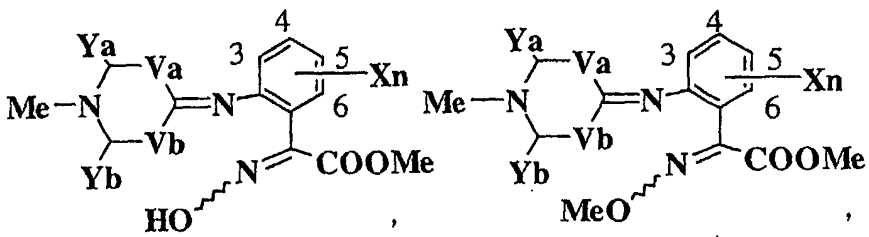

,

,

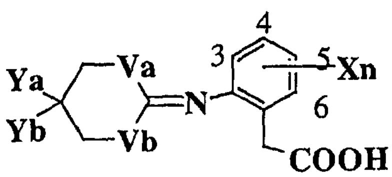

,

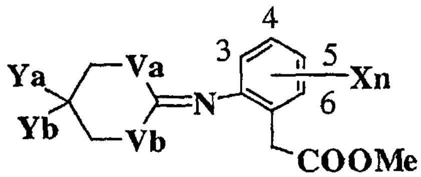

,

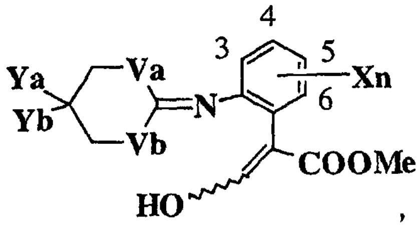

,

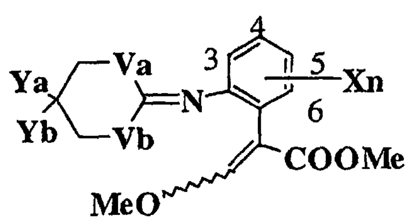

,

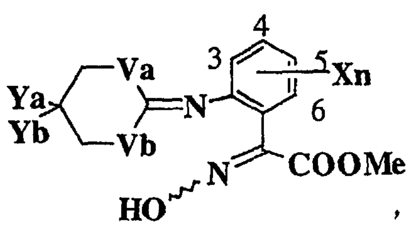

,

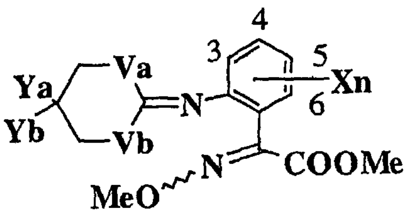

,

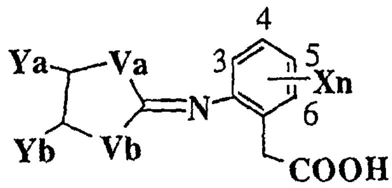 ,

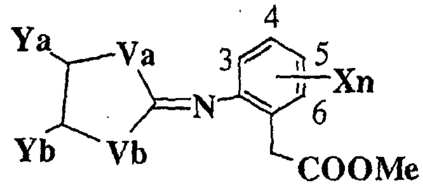 ,

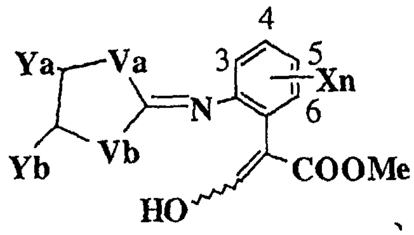 ,

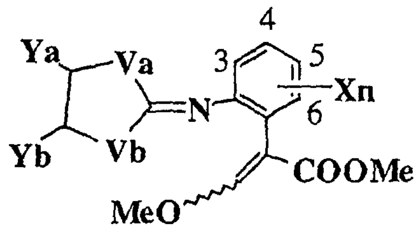 ,

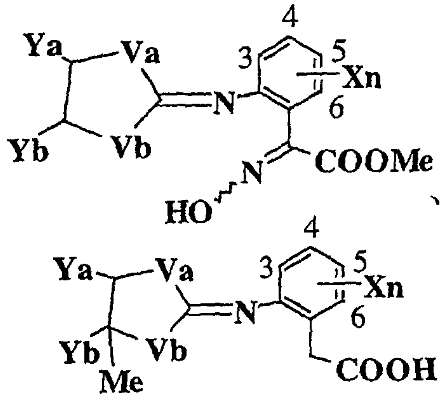 , ,

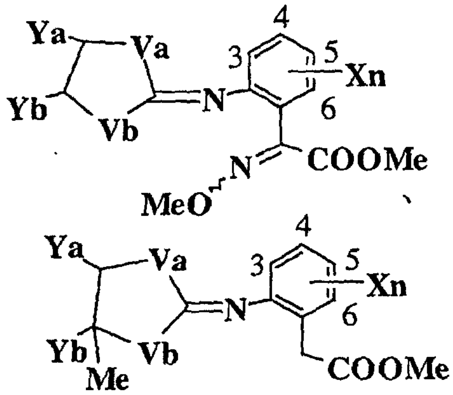 , ,

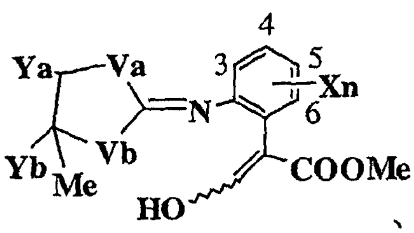 ,

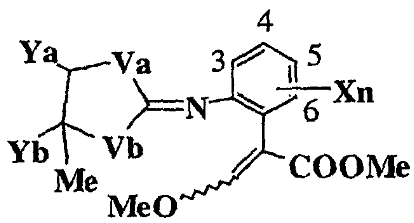 ,

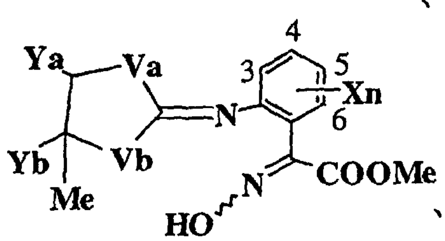 ,

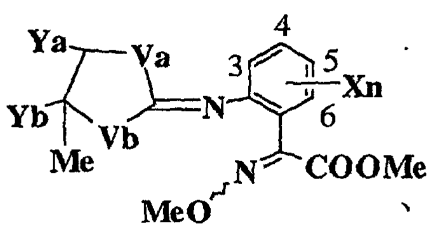 ,

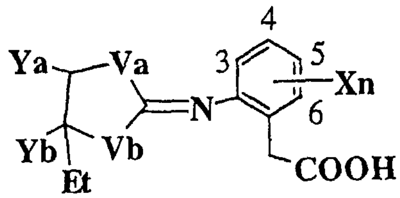,
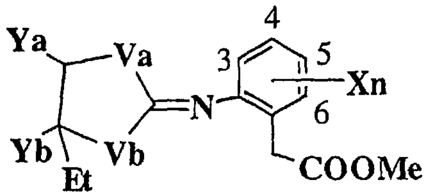,
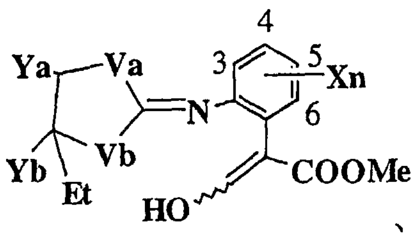,
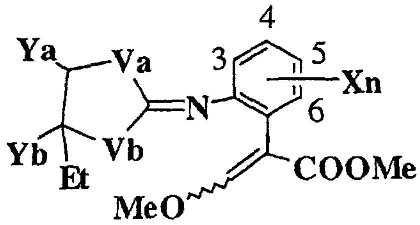,
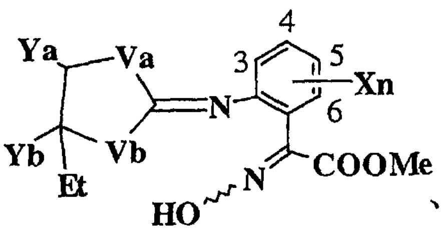,
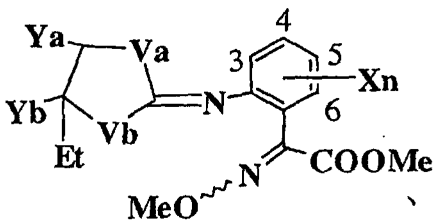,
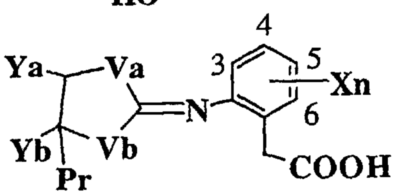,
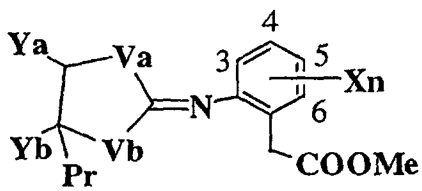,
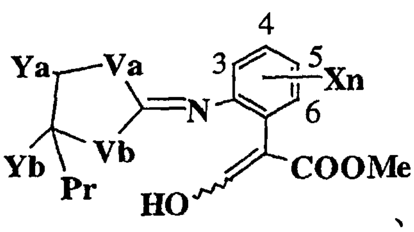,
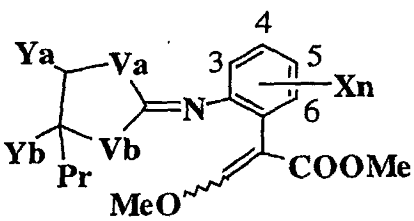,
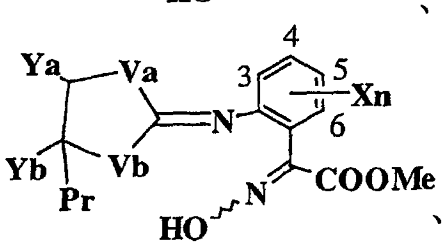,
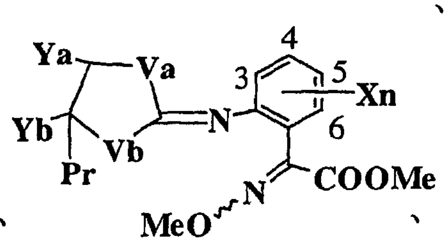,

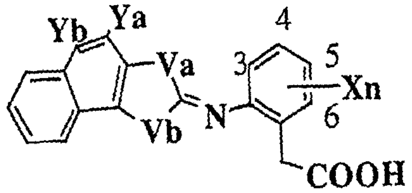
、
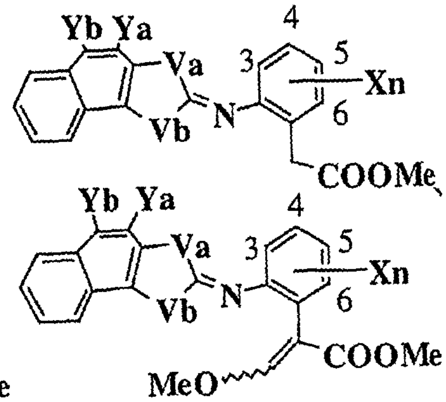
、
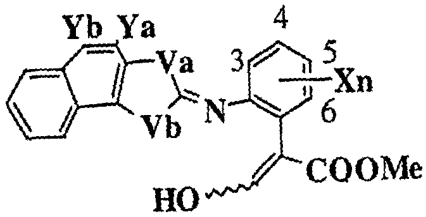
、
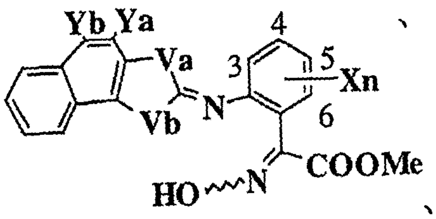
、
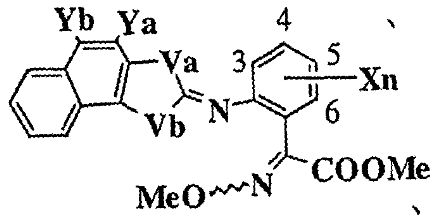
、
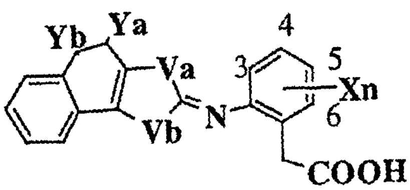
、
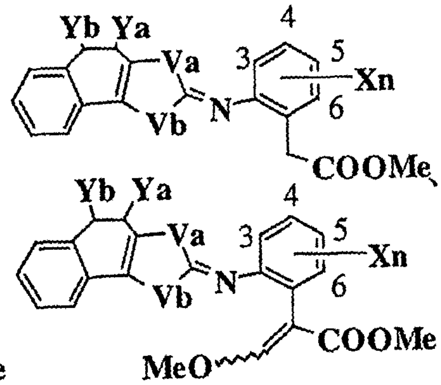
、
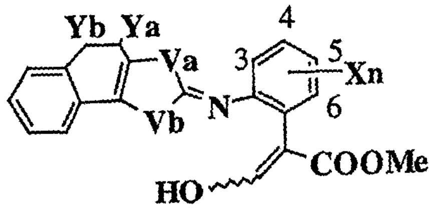
、
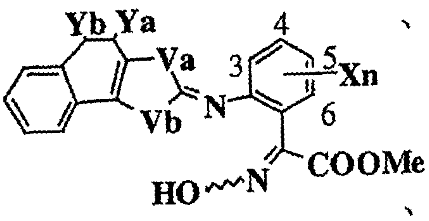
、
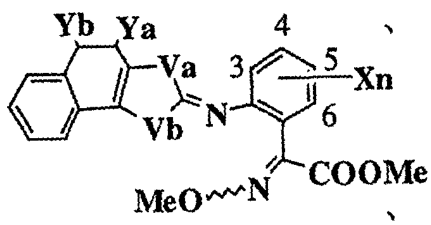
、

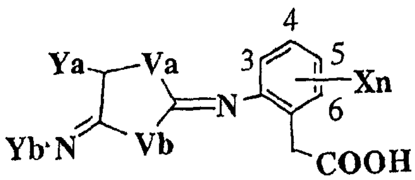
,
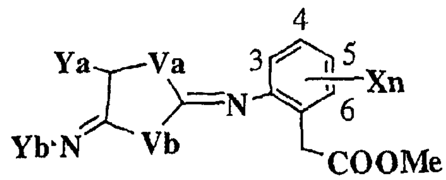
,
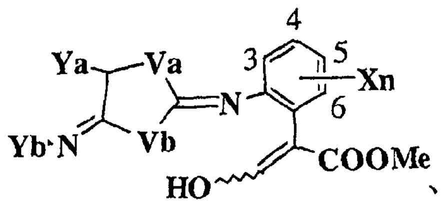
,
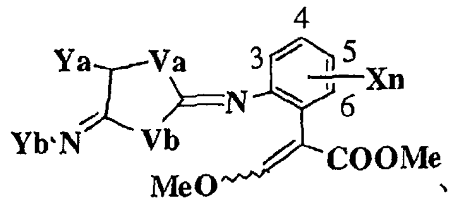
,
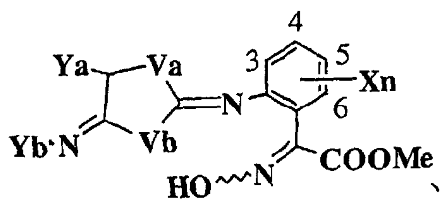
,
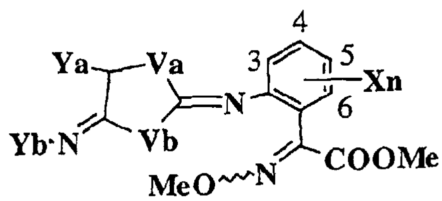

or

| Y a | Y b | V a | V b | X n |
|---|---|---|---|---|
| H | H | N-Me | S | H |
| H | H | N-Et | S | H |
| H | H | N-n-Pr | S | H |
| H | H | N-i-Pr | S | H |
| H | H | N-1-Propenyl | S | H |
| H | H | N-c-Pr | S | H |
| H | H | N-OMe | S | H |
| H | H | N-$SO_2$Me | S | H |
| H | H | N-$NMe_2$ | S | H |
| H | H | N-$CH_2$Ph | S | H |
| H | H | N-$CO_2$Me | S | H |
| H | H | N-$CO_2$Et | S | H |
| H | H | N-COMe | S | H |
| H | H | N-$COCF_3$ | S | H |
| H | H | N-$CH_2$SMe | S | H |
| H | H | N-$CH_2$OPh | S | H |
| H | H | N-$CH_2$-Thiophen-2-yl | S | H |
| H | H | N-$CH_2$-Furan-2-yl | S | H |
| H | H | N-$CH_2$-Pyridin-2-yl | S | H |
| H | H | N-$CH_2$-Pyrazin-2-yl | S | H |
| H | H | N-$CH_2$-Pyrimidin-4-yl | S | H |
| H | H | N-$CH_2$-Thiazol-2-yl | S | H |
| H | H | N-$CH_2$-1-Me-5-Pyrazolyl | S | H |
| H | H | N-Ph | S | H |
| H | H | N-Me | N-Me | H |
| H | H | N-Et | N-Me | H |
| H | H | N-n-Pr | N-Me | H |
| H | H | N-i-Pr | N-Me | H |
| H | H | N-1-Propenyl | N-Me | H |
| H | H | N-c-Pr | N-Me | H |

| | | | | |
|---|---|---|---|---|
| H | H | N-OMe | N-Me | H |
| H | H | N-$SO_2$Me | N-Me | H |
| H | H | N-$NMe_2$ | N-Me | H |
| H | H | N-$CH_2$Ph | N-Me | H |
| H | H | N-$CO_2$Me | N-Me | H |
| H | H | N-$CO_2$Et | N-Me | H |
| H | H | N-COMe | N-Me | H |
| H | H | N-$COCF_3$ | N-Me | H |
| H | H | N-$CH_2$SMe | N-Me | H |
| H | H | N-$CH_2$OPh | N-Me | H |
| H | H | N-$CH_2$-Thiophen-2-yl | N-Me | H |
| H | H | N-$CH_2$-Furan-2-yl | N-Me | H |
| H | H | N-$CH_2$-Pyridin-2-yl | N-Me | H |
| H | H | N-$CH_2$-Pyrazin-2-yl | N-Me | H |
| H | H | N-$CH_2$-Pyrimidin-4-yl | N-Me | H |
| H | H | N-$CH_2$-Thiazol-2-yl | N-Me | H |
| H | H | N-$CH_2$-1-Me-5-Pyrazolyl | N-Me | H |
| H | H | N-Ph | N-Me | H |
| H | H | N-Me | O | H |
| H | H | N-Et | O | H |
| H | H | N-n-Pr | O | H |
| H | H | N-i-Pr | O | H |
| H | H | N-1-Propenyl | O | H |
| H | H | N-c-Pr | O | H |
| H | H | N-OMe | O | H |
| H | H | N-$SO_2$Me | O | H |
| H | H | N-$NMe_2$ | O | H |
| H | H | N-$CH_2$Ph | O | H |
| H | H | N-$CO_2$Me | O | H |
| H | H | N-$CO_2$Et | O | H |
| H | H | N-COMe | O | H |
| H | H | N-$COCF_3$ | O | H |
| H | H | N-$CH_2$SMe | O | H |
| H | H | N-$CH_2$OPh | O | H |
| H | H | N-$CH_2$-Thiophen-2-yl | O | H |
| H | H | N-$CH_2$-Furan-2-yl | O | H |
| H | H | N-$CH_2$-Pyridin-2-yl | O | H |
| H | H | N-$CH_2$-Pyrazin-2-yl | O | H |
| H | H | N-$CH_2$-Pyrimidin-4-yl | O | H |
| H | H | N-$CH_2$-Thiazol-2-yl | O | H |
| H | H | N-$CH_2$-1-Me-5-Pyrazolyl | O | H |
| H | H | N-Ph | O | H |
| H | H | S | O | H |
| H | Cl | S | O | H |
| H | Me | S | O | H |
| H | Et | S | O | H |
| H | n-Pr | S | O | H |
| H | i-Pr | S | O | H |
| H | n-Bu | S | O | H |
| H | i-Bu | S | O | H |
| H | s-Bu | S | O | H |
| H | t-Bu | S | O | H |
| H | n-Pen | S | O | H |
| H | 3-Me-n-Bu | S | O | H |
| H | n-Hex | S | O | H |
| H | Ethenyl | S | O | H |
| H | 1-Propenyl | S | O | H |
| H | Ethynyl | S | O | H |

| | | | | |
|---|---|---|---|---|
| H | $CF_3$ | S | O | H |
| H | c-Pr | S | O | H |
| H | c-Hex | S | O | H |
| H | CN | S | O | H |
| H | CHO | S | O | H |
| H | $CH_2Ph$ | S | O | H |
| H | CH=CHPh | S | O | H |
| H | CH=CH-(4-Ph)Ph | S | O | H |
| H | $CH_2CH$=CHPh | S | O | H |
| H | OPh | S | O | H |
| H | $CO_2Me$ | S | O | H |
| H | $CO_2Et$ | S | O | H |
| H | $CH_2OMe$ | S | O | H |
| H | C(=NOMe)OMe | S | O | H |
| H | C(=NOMe)OSMe | S | O | H |
| H | C(=NOMe)Me | S | O | H |
| H | C(=NOMe)C(=NOMe)Me | S | O | H |
| H | C(=NOMe)Ph | S | O | H |
| H | C(=NOCH$_2$Ph)Me | S | O | H |
| H | COMe | S | O | H |
| H | $CH_2SMe$ | S | O | H |
| H | $CH_2OPh$ | S | O | H |
| H | $CH_2O$(4-Me-Ph) | S | O | H |
| H | $CH_2O$(2,4-$Cl_2$-Ph) | S | O | H |
| H | $CH_2SCH_2Ph$ | S | O | H |
| H | $CH_2NMe_2$ | S | O | H |
| H | $CH_2N$(COMe)Me | S | O | H |
| H | $CH_2ON$=$CMe_2$ | S | O | H |
| H | $CH_2N$=NCHMePh | S | O | H |
| H | $CH_2$(Morpholino) | S | O | H |
| H | $CH_2$(1-Pyrazolyl) | S | O | H |
| H | $CH_2$(Hexamethyleneimino) | S | O | H |
| H | $CH_2$(3-Ph-1-Pyrazolyl) | S | O | H |
| H | $CH_2$(1-Imidazolyl) | S | O | H |
| H | $CF_2Cl$ | S | O | H |
| H | $CCl_3$ | S | O | H |
| H | $ClCH_2$ | S | O | H |
| H | $BrCH_2$ | S | O | H |
| H | $FCH_2$ | S | O | H |
| H | $ICH_2$ | S | O | H |
| H | 1-Naphthyl | S | O | H |
| H | 2-Naphthyl | S | O | H |
| H | Thiophen-2-yl | S | O | H |
| H | Thiophen-3-yl | S | O | H |
| H | 5-Cl-Thiophen-2-yl | S | O | H |
| H | 2,5-$Cl_2$-Thiophen-3-yl | S | O | H |
| H | 2,5-$Me_2$-Thiophen-3-yl | S | O | H |
| H | 5-Cl-Thiophen-2-yl | S | O | H |
| H | 3-Me-Thiophen-2-yl | S | O | H |
| H | 4,5-$Br_2$-Thiophen-3-yl | S | O | H |
| H | Furan-2-yl | S | O | H |
| H | Furan-3-yl | S | O | H |
| H | 2,5-$Me_2$-Furan-3-yl | S | O | H |
| H | 1-Me-Pyrrol-2-yl | S | O | H |
| H | 1-Me-Pyrrole-3-yl | S | O | H |
| H | Pyridin-2-yl | S | O | H |
| H | Pyridin-3-yl | S | O | H |
| H | Pyridin-4-yl | S | O | H |

| | | | | |
|---|---|---|---|---|
| H | 6-Cl-Pyridin-2-yl | S | O | H |
| H | 5-$CF_3$-6-PhO-2-Pyridyl | S | O | H |
| H | Pyrazin-2-yl | S | O | H |
| H | Pyrimidin-2-yl | S | O | H |
| H | Pyrimidin-4-yl | S | O | H |
| H | 6-MeS-5-Pyrimidyl | S | O | H |
| H | 6-PhO-Pyrimidin-4-yl | S | O | H |
| H | Thiazol-2-yl | S | O | H |
| H | Thiazol-5-yl | S | O | H |
| H | 5-$CF_3$-Thiazol-2-yl | S | O | H |
| H | Pyrazol-3-yl | S | O | H |
| H | 1-Me-Pyrazol-5-yl | S | O | H |
| H | 1-Ph-Pyrazol-3-yl | S | O | H |
| H | Pyrazol-5-yl | S | O | H |
| H | 1-Me-3-Cl-Pyrazol-5-yl | S | O | H |
| H | 1-Me-Imidazol-2-yl | S | O | H |
| H | Benzothiazol-2-yl | S | O | H |
| H | Benzofuran-2-yl | S | O | H |
| H | Ph | S | O | H |
| H | 2-Cl-Ph | S | O | H |
| H | 2-Cl-Ph | S | O | 4-Cl |
| H | 2-Cl-Ph | S | O | 5-Cl |
| H | 2-Cl-Ph | S | O | 4-$CF_3$ |
| H | 2-Cl-Ph | S | O | 5-$CF_3$ |
| H | 2-Cl-Ph | S | O | 4-Me |
| H | 2-Cl-Ph | S | O | 5-Me |
| H | 2-Cl-Ph | S | O | 4-F |
| H | 2-Cl-Ph | S | O | 5-F |
| H | 2-Cl-Ph | S | O | 4-MeO |
| H | 3-Cl-Ph | S | O | H |
| H | 4-Cl-Ph | S | O | H |
| H | 4-Cl-Ph | S | O | 4-Cl |
| H | 4-Cl-Ph | S | O | 5-Cl |
| H | 4-Cl-Ph | S | O | 4-$CF_3$ |
| H | 4-Cl-Ph | S | O | 5-$CF_3$ |
| H | 4-Cl-Ph | S | O | 4-Me |
| H | 4-Cl-Ph | S | O | 5-Me |
| H | 4-Cl-Ph | S | O | 4-F |
| H | 4-Cl-Ph | S | O | 5-F |
| H | 4-Cl-Ph | S | O | 4-MeO |
| H | 2-F-Ph | S | O | H |
| H | 2-F-Ph | S | O | 4-Cl |
| H | 2-F-Ph | S | O | 5-Cl |
| H | 2-F-Ph | S | O | 4-$CF_3$ |
| H | 2-F-Ph | S | O | 5-$CF_3$ |
| H | 2-F-Ph | S | O | 4-Me |
| H | 2-F-Ph | S | O | 5-Me |
| H | 2-F-Ph | S | O | 4-F |
| H | 2-F-Ph | S | O | 5-F |
| H | 2-F-Ph | S | O | 4-MeO |
| H | 3-F-Ph | S | O | H |
| H | 4-F-Ph | S | O | H |
| H | 4-F-Ph | S | O | 4-Cl |
| H | 4-F-Ph | S | O | 5-Cl |
| H | 4-F-Ph | S | O | 4-$CF_3$ |
| H | 4-F-Ph | S | O | 5-$CF_3$ |
| H | 4-F-Ph | S | O | 4-Me |
| H | 4-F-Ph | S | O | 5-Me |

| | | | | |
|---|---|---|---|---|
| H | 4-F-Ph | S | 0 | 4-F |
| H | 4-F-Ph | S | 0 | 5-F |
| H | 4-F-Ph | S | 0 | 4-MeO |
| H | 2-Me-Ph | S | 0 | H |
| H | 2-Me-Ph | S | 0 | 4-Cl |
| H | 2-Me-Ph | S | 0 | 5-Cl |
| H | 2-Me-Ph | S | 0 | 4-$CF_3$ |
| H | 2-Me-Ph | S | 0 | 5-$CF_3$ |
| H | 2-Me-Ph | S | 0 | 4-Me |
| H | 2-Me-Ph | S | 0 | 5-Me |
| H | 2-Me-Ph | S | 0 | 4-F |
| H | 2-Me-Ph | S | 0 | 5-F |
| H | 2-Me-Ph | S | 0 | 4-MeO |
| H | 3-Me-Ph | S | 0 | H |
| H | 4-Me-Ph | S | 0 | H |
| H | 4-Me-Ph | S | 0 | 4-Cl |
| H | 4-Me-Ph | S | 0 | 5-Cl |
| H | 4-Me-Ph | S | 0 | 4-$CF_3$ |
| H | 4-Me-Ph | S | 0 | 5-$CF_3$ |
| H | 4-Me-Ph | S | 0 | 4-Me |
| H | 4-Me-Ph | S | 0 | 5-Me |
| H | 4-Me-Ph | S | 0 | 4-F |
| H | 4-Me-Ph | S | 0 | 5-F |
| H | 4-Me-Ph | S | 0 | 4-MeO |
| H | 2-MeO-Ph | S | 0 | H |
| H | 4-MeO-Ph | S | 0 | H |
| H | 4-Br-Ph | S | 0 | H |
| H | 2,4-$Cl_2$-Ph | S | 0 | H |
| H | 3,4-$Cl_2$-Ph | S | 0 | H |
| H | 2,4,6-$Cl_3$-Ph | S | 0 | H |
| H | 3,4-$(MeO)_2$-Ph | S | 0 | H |
| H | 2-Cl-4-Me-Ph | S | 0 | H |
| H | 2-MeO-4-Me-Ph | S | 0 | H |
| H | 2-Cl-4-i-PrO-Ph | S | 0 | H |
| H | 3-Cl-4-$PhCH_2O$-Ph | S | 0 | H |
| H | 2,4-$Me_2$-Ph | S | 0 | H |
| H | 2,5-$Me_2$-Ph | S | 0 | H |
| H | 2,6-$F_2$-Ph | S | 0 | 6-Cl |
| H | 2,6-$F_2$-Ph | S | 0 | 5-Cl |
| H | 2,6-$F_2$-Ph | S | 0 | 3-Cl |
| H | 2,6-$F_2$-Ph | S | 0 | 4,5-$Cl_2$ |
| H | 2,6-$F_2$-Ph | S | 0 | 4-$CF_3$ |
| H | 2,6-$F_2$-Ph | S | 0 | 4,5-$Me_2$ |
| H | 2,6-$F_2$-Ph | S | 0 | 4-Me |
| H | 2,6-$F_2$-Ph | S | 0 | 5-Me |
| H | 2,6-$F_2$-Ph | S | 0 | 4-F |
| H | 2,6-$F_2$-Ph | S | 0 | 5-F |
| H | 2,6-$F_2$-Ph | S | 0 | 4-MeO |
| H | 2,6-$F_2$-Ph | S | 0 | H |
| H | 2,5-$F_2$-Ph | S | 0 | H |
| H | 2,4-$F_2$-Ph | S | 0 | H |
| H | 2,3-$F_2$-Ph | S | 0 | H |
| H | 3,5-$F_2$-Ph | S | 0 | H |
| H | 3,4-$F_2$-Ph | S | 0 | H |
| H | 2,3,4,5,6-$F_5$-Ph | S | 0 | H |
| H | 4-Et-Ph | S | 0 | H |
| H | 4-i-Pr-Ph | S | 0 | H |
| H | 4-n-Bu-Ph | S | 0 | H |

| | | | | |
|---|---|---|---|---|
| H | 4-s-Bu-Ph | S | O | H |
| H | 4-t-Bu-Ph | S | O | H |
| H | 4-(t-$BuCH_2$)-Ph | S | O | H |
| H | 4-Et$(Me)_2$-Ph | S | O | H |
| H | 4-n-Hex-Ph | S | O | H |
| H | 4-($(Me)_2$(CN)C)-Ph | S | O | H |
| H | 4-$PhCH_2$-Ph | S | O | H |
| H | 4-(4-F-Ph)$(Me)_2$-Ph | S | O | H |
| H | 4-(MeCH=CH)-Ph | S | O | H |
| H | 4-(MeC≡C)-Ph | S | O | H |
| H | 4-$CF_3$-Ph | S | O | H |
| H | 4-$CF_3CH_2$-Ph | S | O | H |
| H | 4-($Cl_2C$=$CHCH_2$)-Ph | S | O | H |
| H | 4-(BrC≡C)-Ph | S | O | H |
| H | 4-(2,2-$F_2$-c-Bu)$CH_2$-Ph | S | O | H |
| H | 4-(1-Me-c-Pr)-Ph | S | O | H |
| H | 4-i-PrO-Ph | S | O | H |
| H | 4-t-BuO-Ph | S | O | H |
| H | 4-n-HexO-Ph | S | O | H |
| H | 4-(MeC≡C-O)-Ph | S | O | H |
| H | 4-($CH_2$=$CHCH_2O$)-Ph | S | O | H |
| H | 4-$CHF_2O$-Ph | S | O | H |
| H | 4-$CBrF_2O$-Ph | S | O | H |
| H | 4-$CF_3O$-Ph | S | O | H |
| H | 4-$CF_3CH_2O$-Ph | S | O | H |
| H | 4-($CF_2$=$CHCH_2CH_2O$)-Ph | S | O | H |
| H | 4-$CCl_3CH_2O$-Ph | S | O | H |
| H | 4-MeS-Ph | S | O | H |
| H | 4-s-BuS-Ph | S | O | H |
| H | 4-EtSO-Ph | S | O | H |
| H | 4-$MeSO_2$-Ph | S | O | H |
| H | 4-$EtSO_2$-Ph | S | O | H |
| H | 4-i-$PrSO_2$-Ph | S | O | H |
| H | 4-t-$BuSO_2$-Ph | S | O | H |
| H | 4-(MeCH=$CHCH_2S$)-Ph | S | O | H |
| H | 4-($CH_2$=$CHCH_2SO$)-Ph | S | O | H |
| H | 4-(ClCH=$CHCH_2SO_2$)-Ph | S | O | H |
| H | 4-(HC≡$CCH_2S$)-Ph | S | O | H |
| H | 4-(HC≡$CCH_2SO$)-Ph | S | O | H |
| H | 4-(HC≡$CCH_2SO_2$)-Ph | S | O | H |
| H | 4-$CHF_2S$-Ph | S | O | H |
| H | 4-$CBrF_2S$-Ph | S | O | H |
| H | 4-$CF_3S$-Ph | S | O | H |
| H | 4-$CF_3CH_2S$-Ph | S | O | H |
| H | 4-$CHF_2CF_2S$-Ph | S | O | H |
| H | 4-$CHF_2SO$-Ph | S | O | H |
| H | 4-$CBrF_2SO$-Ph | S | O | H |
| H | 4-$CF_3SO$-Ph | S | O | H |
| H | 4-$CF_3CH_2SO_2$-Ph | S | O | H |
| H | 4-$CHF_2CF_2SO_2$-Ph | S | O | H |
| H | 4-$CHF_2SO_2$-Ph | S | O | H |
| H | 4-$CBrF_2SO_2$-Ph | S | O | H |
| H | 4-$CF_3SO_2$-Ph | S | O | H |
| H | 4-($Cl_2C$=$CHCH_2S$)-Ph | S | O | H |
| H | 4-($Cl_2C$=$CHCH_2SO$)-Ph | S | O | H |
| H | 4-($Cl_2C$=$CHCH_2SO_2$)-Ph | S | O | H |
| H | 4-(BrC≡$CCH_2S$)-Ph | S | O | H |
| H | 4-(BrC≡$CCH_2SO$)-Ph | S | O | H |

| | | | | |
|---|---|---|---|---|
| H | 4-(BrC≡CCH$_2$SO$_2$)-Ph | S | O | H |
| H | 4-CHO-Ph | S | O | H |
| H | 4-NO$_2$-Ph | S | O | H |
| H | 3-CN-Ph | S | O | H |
| H | 4-CN-Ph | S | O | H |
| H | 4-(Me)$_2$N-Ph | S | O | H |
| H | 4-Me(MeC(O))N-Ph | S | O | H |
| H | 4-PhN(Me)-Ph | S | O | H |
| H | 4-PhCH$_2$(MeCO)N-Ph | S | O | H |
| H | 4-PhCH$_2$O-Ph | S | O | H |
| H | 4-(2-Cl-Ph)CH$_2$O-Ph | S | O | H |
| H | 4-(3-Cl-Ph)CH$_2$O-Ph | S | O | H |
| H | 4-(4-Cl-Ph)CH$_2$O-Ph | S | O | H |
| H | 4-(2-Me-Ph)CH$_2$O-Ph | S | O | H |
| H | 4-(3-Me-Ph)CH$_2$O-Ph | S | O | H |
| H | 4-(4-F-Ph)CH$_2$O-Ph | S | O | H |
| H | 4-(4-Et-Ph)CH$_2$O-Ph | S | O | H |
| H | 4-(2-Cl-Ph)CH$_2$S-Ph | S | O | H |
| H | 4-(3-Cl-Ph)CH$_2$S-Ph | S | O | H |
| H | 4-(4-Cl-Ph)CH$_2$SO-Ph | S | O | H |
| H | 4-(2-Me-Ph)CH$_2$S-Ph | S | O | H |
| H | 4-(3-Me-Ph)CH$_2$SO$_2$-Ph | S | O | H |
| H | 4-(2,4-F$_2$-Ph)CH$_2$O-Ph | S | O | H |
| H | 3-(3,4-Cl$_2$-Ph)CH$_2$O-Ph | S | O | H |
| H | 4-(2,5-Me$_2$-Ph)CH$_2$O-Ph | S | O | H |
| H | 4-(2,3,5,6-F$_4$-Ph)CH$_2$O-Ph | S | O | H |
| H | 4-MeC(O)-Ph | S | O | H |
| H | 4-EtC(O)-Ph | S | O | H |
| H | 4-n-PrC(O)-Ph | S | O | H |
| H | 4-i-PrC(O)-Ph | S | O | H |
| H | 4-i-BuC(O)-Ph | S | O | H |
| H | 4-t-BuC(O)-Ph | S | O | H |
| H | 4-i-BuCH$_2$C(O)-Ph | S | O | H |
| H | 4-Et(Me)$_2$C(O)-Ph | S | O | H |
| H | 4-n-HexC(O)-Ph | S | O | H |
| H | 4-PhC(O)-Ph | S | O | H |
| H | 4-(2-Cl-Ph)C(O)-Ph | S | O | H |
| H | 4-(3-Br-Ph)C(O)-Ph | S | O | H |
| H | 4-(4-Cl-Ph)C(O)-Ph | S | O | H |
| H | 4-(2-Me-Ph)C(O)-Ph | S | O | H |
| H | 4-MeOCH$_2$-Ph | S | O | H |
| H | 4-EtOCH$_2$-Ph | S | O | H |
| H | 4-i-PrOCH$_2$-Ph | S | O | H |
| H | 4-MeSCH$_2$-Ph | S | O | H |
| H | 4-EtSCH$_2$-Ph | S | O | H |
| H | 4-i-PrSCH$_2$-Ph | S | O | H |
| H | 4-CF$_3$C(O)-Ph | S | O | H |
| H | 4-CF$_3$CF$_2$C(O)-Ph | S | O | H |
| H | 4-MeC(O)O-Ph | S | O | H |
| H | 4-EtC(O)O-Ph | S | O | H |
| H | 4-n-PrC(O)O-Ph | S | O | H |
| H | 4-i-PrC(O)O-Ph | S | O | H |
| H | 4-i-BuC(O)O-Ph | S | O | H |
| H | 4-t-BuC(O)O-Ph | S | O | H |
| H | 4-i-BuCH$_2$C(O)O-Ph | S | O | H |
| H | 4-Et(Me)$_2$CC(O)O-Ph | S | O | H |
| H | 4-n-HexC(O)O-Ph | S | O | H |
| H | 4-CF$_3$C(O)O-Ph | S | O | H |

| | | | | |
|---|---|---|---|---|
| H | 4-$CF_3CF_2C(O)O$-Ph | S | O | H |
| H | 4-PhC(O)O-Ph | S | O | H |
| H | 3-Ph-Ph | S | O | H |
| H | 4-Ph-Ph | S | O | H |
| H | 4-(4-Cl-Ph)-Ph | S | O | H |
| H | 4-(2,5-$Me_2$-Ph)-3-Me-Ph | S | O | H |
| H | 3-PhO-Ph | S | O | H |
| H | 4-PhO-Ph | S | O | H |
| H | 4-(4-Cl-Ph)O-Ph | S | O | H |
| H | 4-(4-Me-Ph)O-Ph | S | O | H |
| H | 4-(4-F-Ph)O-Ph | S | O | H |
| H | 4-(4-MeO-Ph)O-Ph | S | O | H |
| H | 4-(2,4-$Cl_2$-Ph)O-Ph | S | O | H |
| H | 4-(3,4-$Cl_2$-Ph)O-Ph | S | O | H |
| H | 4-(Pyridin-2-yl)-Ph | S | O | H |
| H | 4-(5-Cl-Pyridin-2-yl)-Ph | S | O | H |
| H | 4-(6-F-5-$CF_3$-Pyridin-2-yl)-Ph | S | O | H |
| H | 4-(Pyridin-2-yl)O-Ph | S | O | H |
| H | 4-(5-Cl-Pyridin-2-yl)O-Ph | S | O | H |
| H | 4-(3-Cl-5-F-Pyridin-2-yl)O-Ph | S | O | H |
| H | 4-(5-Cl-Thiophen-2-yl)O-Ph | S | O | H |
| -CH=CH-CH=CH- | | S | O | H |
| -CCl=CH-CH=CH- | | S | O | H |
| -CH=CCl-CH=CH- | | S | O | H |
| -CH=CH-CCl=CH- | | S | O | H |
| -CH=CH-CH=CCl- | | S | O | H |
| -CH=CH-C(OMe)=CH- | | S | O | H |
| -CH=CH-CF=CH- | | S | O | H |
| -CH=CH-CMe=CH- | | S | O | H |
| -CH=CH-CH=N- | | S | O | H |
| -CH=CH-N=CH- | | S | O | H |
| -CH=N-CH=CH- | | S | O | H |
| -N=CH-CH=CH- | | S | O | H |
| $-(CH_2)_3-$ | | S | O | H |
| $-(CH_2)_4-$ | | S | O | H |
| $-(CH_2)_5-$ | | S | O | H |
| $-CH_2-O-CH_2-$ | | S | O | H |
| $-CH_2-CH_2-O-CH_2-$ | | S | O | H |
| $-CO-(CH_2)_3-$ | | S | O | H |
| $-CH_2-CH(CH_2Ph)CH_2-$ | | S | O | H |
| $-CH_2-CH_2-CH(Me)-CH_2-$ | | S | O | H |
| $-CH_2-CH_2-CH(Ph)-CH_2-$ | | S | O | H |
| Me | Me | S | O | H |
| Me | Et | S | O | H |
| Me | n-Pr | S | O | H |
| Me | i-Pr | S | O | H |
| Me | n-Bu | S | O | H |
| Me | n-Hex | S | O | H |
| Me | Ethenyl | S | O | H |
| Me | 1-Propynyl | S | O | H |
| Me | $CF_3$ | S | O | H |
| Me | c-Pr | S | O | H |
| Me | CN | S | O | H |
| Me | CHO | S | O | H |
| Me | $CH_2Ph$ | S | O | H |
| Me | $CO_2Me$ | S | O | H |
| Me | $CH_2OMe$ | S | O | H |
| Me | COMe | S | O | H |

| | | | | |
|---|---|---|---|---|
| Me | $CH_2SMe$ | S | O | H |
| Me | $CH_2OPh$ | S | O | H |
| Me | $CH_2O$(4-Me-Ph) | S | O | H |
| Me | $CH_2O$(2,4-$Cl_2$-Ph) | S | O | H |
| Me | $CH_2SCH_2Ph$ | S | O | H |
| Me | $CF_2Cl$ | S | O | H |
| Me | 1-Naphthyl | S | O | H |
| Me | 2-Naphthyl | S | O | H |
| Me | Thiophen-2-yl | S | O | H |
| Me | Furan-2-yl | S | O | H |
| Me | 1-Me-Pyrrol-2-yl | S | O | H |
| Me | Pyridin-4-yl | S | O | H |
| Me | 6-Cl-Pyridin-2-yl | S | O | H |
| Me | Pyrazin-2-yl | S | O | H |
| Me | Pyrimidin-2-yl | S | O | H |
| Me | Thiazol-2-yl | S | O | H |
| Me | Thiazol-5-yl | S | O | H |
| Me | 5-$CF_3$-Thiazol-2-yl | S | O | H |
| Me | 1-Me-3-Cl-Pyrazol-5-yl | S | O | H |
| Me | 1-Me-Imidazol-2-yl | S | O | H |
| Me | Ph | S | O | H |
| Me | 2-Cl-Ph | S | O | H |
| Me | 3-Cl-Ph | S | O | H |
| Me | 4-F-Ph | S | O | H |
| Me | 2-Me-Ph | S | O | H |
| Me | 3-Me-Ph | S | O | H |
| Me | 4-Me-Ph | S | O | H |
| Me | 2-MeO-Ph | S | O | H |
| Me | 3-MeO-Ph | S | O | H |
| Me | 4-MeO-Ph | S | O | H |
| Me | 2,4-$Cl_2$-Ph | S | O | H |
| Me | 2-Cl-4-Me-Ph | S | O | H |
| Me | 2,5-$Me_2$-Ph | S | O | H |
| Me | 2,6-$F_2$-Ph | S | O | H |
| Me | 4-Et-Ph | S | O | H |
| Me | 4-$PhCH_2$-Ph | S | O | H |
| Me | 4-$CF_3$-Ph | S | O | H |
| Me | 4-MeS-Ph | S | O | H |
| Me | 4-EtSO-Ph | S | O | H |
| Me | 4-$MeSO_2$-Ph | S | O | H |
| Me | 4-$EtSO_2$-Ph | S | O | H |
| Me | 4-CHO-Ph | S | O | H |
| Me | 4-$NO_2$-Ph | S | O | H |
| Me | 3-CN-Ph | S | O | H |
| Me | 4-CN-Ph | S | O | H |
| Me | 4-$PhCH_2$(MeCO)N-Ph | S | O | H |
| Me | 4-(2,4-$F_2$-Ph)$CH_2O$-Ph | S | O | H |
| Me | 4-MeC(O)-Ph | S | O | H |
| Me | 4-(4-Cl-Ph)C(O)-Ph | S | O | H |
| Me | 4-$MeOCH_2$-Ph | S | O | H |
| Me | 4-$EtOCH_2$-Ph | S | O | H |
| Me | 4-$MeSCH_2$-Ph | S | O | H |
| Me | 4-$EtSCH_2$-Ph | S | O | H |
| Me | 4-$CF_3C$(O)-Ph | S | O | H |
| Me | 4-MeC(O)O-Ph | S | O | H |
| Me | 4-t-BuC(O)O-Ph | S | O | H |
| Me | 4-$CF_3C$(O)O-Ph | S | O | H |
| Me | 4-PhC(O)O-Ph | S | O | H |

| | | | | |
|---|---|---|---|---|
| Me | 4-Ph-Ph | S | 0 | H |
| Me | 4-(4-Cl-Ph)-Ph | S | 0 | H |
| Me | 4-(4-MeO-Ph)O-Ph | S | 0 | H |
| Me | 4-(2,4-$Cl_2$-Ph)O-Ph | S | 0 | H |
| Me | 4-(Pyridin-2-yl)O-Ph | S | 0 | H |
| Me | 4-(5-Cl-Pyridin-2-yl)O-Ph | S | 0 | H |
| Et | Et | S | 0 | H |
| Et | c-Pr | S | 0 | H |
| Et | $CH_2Ph$ | S | 0 | H |
| Et | $CO_2Me$ | S | 0 | H |
| Et | $CH_2OMe$ | S | 0 | H |
| Et | COMe | S | 0 | H |
| Et | $CH_2SMe$ | S | 0 | H |
| Et | $CH_2OPh$ | S | 0 | H |
| Et | $CH_2O$(4-Me-Ph) | S | 0 | H |
| Et | $CH_2SCH_2Ph$ | S | 0 | H |
| Et | 2-Naphthyl | S | 0 | H |
| Et | Thiophen-2-yl | S | 0 | H |
| Et | Furan-3-yl | S | 0 | H |
| Et | 1-Me-Pyrrole-3-yl | S | 0 | H |
| Et | Pyridin-2-yl | S | 0 | H |
| Et | Pyrazin-2-yl | S | 0 | H |
| Et | Pyrimidin-2-yl | S | 0 | H |
| Et | Thiazol-5-yl | S | 0 | H |
| Et | 1-Me-3-Cl-Pyrazol-5-yl | S | 0 | H |
| Et | 1-Me-Imidazol-2-yl | S | 0 | H |
| Et | Ph | S | 0 | H |
| Et | 4-Cl-Ph | S | 0 | H |
| Et | 2-F-Ph | S | 0 | H |
| Et | 3-F-Ph | S | 0 | H |
| Et | 4-F-Ph | S | 0 | H |
| Et | 2-Me-Ph | S | 0 | H |
| Et | 3-Me-Ph | S | 0 | H |
| Et | 4-Me-Ph | S | 0 | H |
| Et | 2-MeO-Ph | S | 0 | H |
| Et | 3-MeO-Ph | S | 0 | H |
| Et | 4-MeO-Ph | S | 0 | H |
| Et | 4-Br-Ph | S | 0 | H |
| Et | 2,4-$Cl_2$-Ph | S | 0 | H |
| Et | 3,4-$Cl_2$-Ph | S | 0 | H |
| Et | 4-n-Hex-Ph | S | 0 | H |
| Et | 4-$PhCH_2$-Ph | S | 0 | H |
| Et | 4-$CF_3$-Ph | S | 0 | H |
| Et | 4-$CF_3O$-Ph | S | 0 | H |
| Et | 4-MeS-Ph | S | 0 | H |
| Et | 4-$MeSO_2$-Ph | S | 0 | H |
| Et | 4-CHO-Ph | S | 0 | H |
| Et | 4-$NO_2$-Ph | S | 0 | H |
| Et | 3-CN-Ph | S | 0 | H |
| Et | 4-CN-Ph | S | 0 | H |
| Et | 4-$(Me)_2N$-Ph | S | 0 | H |
| Et | 4-$PhCH_2O$-Ph | S | 0 | H |
| Et | 4-MeC(O)-Ph | S | 0 | H |
| Et | 4-(4-Cl-Ph)C(O)-Ph | S | 0 | H |
| Et | 4-$MeOCH_2$-Ph | S | 0 | H |
| Et | 4-$EtSCH_2$-Ph | S | 0 | H |
| Et | 4-$CF_3C$(O)-Ph | S | 0 | H |
| Et | 4-MeC(O)O-Ph | S | 0 | H |

| | | | | |
|---|---|---|---|---|
| Et | 4-$CF_3$C(O)O-Ph | S | O | H |
| Et | 4-PhC(O)O-Ph | S | O | H |
| Et | 4-Ph-Ph | S | O | H |
| Et | 4-(4-Me-Ph)O-Ph | S | O | H |
| Et | 4-(Pyridin-2-yl)O-Ph | S | O | H |
| Ph | $CO_2$Me | S | O | H |
| Ph | COMe | S | O | H |
| Ph | 2-Naphthyl | S | O | H |
| Ph | Thiophen-2-yl | S | O | H |
| Ph | Furan-3-yl | S | O | H |
| Ph | 1-Me-Pyrrol-2-yl | S | O | H |
| Ph | Pyridin-4-yl | S | O | H |
| Ph | Pyrazin-2-yl | S | O | H |
| Ph | Pyrimidin-4-yl | S | O | H |
| Ph | Thiazol-2-yl | S | O | H |
| Ph | 1-Me-3-Cl-Pyrazol-5-yl | S | O | H |
| Ph | 1-Me-Imidazol-2-yl | S | O | H |
| Ph | Ph | S | O | H |
| Ph | 4-Cl-Ph | S | O | H |
| Ph | 2-F-Ph | S | O | H |
| Ph | 3-F-Ph | S | O | H |
| Ph | 4-F-Ph | S | O | H |
| Ph | 2-Me-Ph | S | O | H |
| Ph | 3-Me-Ph | S | O | H |
| Ph | 4-Me-Ph | S | O | H |
| Ph | 2-MeO-Ph | S | O | H |
| Ph | 3-MeO-Ph | S | O | H |
| Ph | 4-MeO-Ph | S | O | H |
| Ph | 2,4-$Cl_2$-Ph | S | O | H |
| Ph | 4-Ph$CH_2$-Ph | S | O | H |
| Ph | 4-$CF_3$-Ph | S | O | H |
| Ph | 4-$CF_3$O-Ph | S | O | H |
| Ph | 4-MeS-Ph | S | O | H |
| Ph | 4-EtSO-Ph | S | O | H |
| Ph | 4-Me$SO_2$-Ph | S | O | H |
| Ph | 4-CHO-Ph | S | O | H |
| Ph | 4-$NO_2$-Ph | S | O | H |
| Ph | 3-CN-Ph | S | O | H |
| Ph | 4-CN-Ph | S | O | H |
| Ph | 4-$(Me)_2$N-Ph | S | O | H |
| Ph | 4-Me(MeC(O))N-Ph | S | O | H |
| Ph | 4-PhN(Me)-Ph | S | O | H |
| Ph | Ph$CH_2$O-Ph | S | O | H |
| Ph | 4-MeC(O)-Ph | S | O | H |
| Ph | 4-(4-Cl-Ph)C(O)-Ph | S | O | H |
| Ph | 4-MeO$CH_2$-Ph | S | O | H |
| Ph | 4-EtS$CH_2$-Ph | S | O | H |
| Ph | 4-$CF_3$C(O)-Ph | S | O | H |
| Ph | 4-MeC(O)O-Ph | S | O | H |
| Ph | 4-$CF_3$C(O)O-Ph | S | O | H |
| Ph | 4-PhC(O)O-Ph | S | O | H |
| Ph | 4-Ph-Ph | S | O | H |
| Ph | 3-PhO-Ph | S | O | H |
| Cl | $CH_2$Ph | S | O | H |
| Cl | $CO_2$Me | S | O | H |
| Br | $CO_2$Me | S | O | H |
| Cl | $CH_2$OMe | S | O | H |
| Cl | COMe | S | O | H |

| | | | | |
|---|---|---|---|---|
| Cl | $CH_2OPh$ | S | O | H |
| Cl | $ClCH_2$ | S | O | H |
| Cl | 2-Naphthyl | S | O | H |
| Cl | Thiophen-2-yl | S | O | H |
| Cl | Furan-2-yl | S | O | H |
| Cl | Pyridin-4-yl | S | O | H |
| Br | Pyridin-4-yl | S | O | H |
| Cl | Thiazol-2-yl | S | O | H |
| Cl | 1-Me-3-Cl-Pyrazol-5-yl | S | O | H |
| Cl | 1-Me-Imidazol-2-yl | S | O | H |
| Cl | Ph | S | O | H |
| Br | Ph | S | O | H |
| Cl | 4-Cl-Ph | S | O | H |
| Cl | 4-Me-Ph | S | O | H |
| Cl | 4-MeO-Ph | S | O | H |
| Cl | 4-$CF_3$-Ph | S | O | H |
| Cl | 4-CHO-Ph | S | O | H |
| Cl | 4-$NO_2$-Ph | S | O | H |
| Cl | 4-CN-Ph | S | O | H |
| Cl | 4-Ph-Ph | S | O | H |
| H | H | S | O | 4-Cl |
| H | H | S | O | 4-$CF_3$ |
| H | H | S | O | 4-Me |
| H | H | S | O | 4-F |
| H | Me | S | O | 4-Cl |
| H | Me | S | O | 4-$CF_3$ |
| H | Me | S | O | 5-Me |
| H | Me | S | O | 4-F |
| Me | H | S | O | 4-Cl |
| Me | H | S | O | 4-$CF_3$ |
| Me | H | S | O | 5-Me |
| Me | H | S | O | 4-F |
| H | Ph | S | O | 4-Cl |
| H | Ph | S | O | 6-F |
| H | Ph | S | O | 5-Cl |
| H | Ph | S | O | 3-F |
| H | Ph | S | O | 4,5-$Cl_2$ |
| H | Ph | S | O | 4-$CF_3$ |
| H | Ph | S | O | 4,5-$Me_2$ |
| H | Ph | S | O | 4-Me |
| H | Ph | S | O | 5-Me |
| H | Ph | S | O | 4-F |
| H | Ph | S | O | 5-F |
| H | Ph | S | O | 4-MeO |
| Me | Ph | S | O | 5-Cl |
| Me | Ph | S | O | 4-$CF_3$ |
| Me | Ph | S | O | 5-Me |
| Me | Ph | S | O | 4-F |
| Me | Ph | S | O | 4-MeO |
| H | H | S | N-Me | H |
| H | Cl | S | N-Me | H |
| H | Me | S | N-Me | H |
| H | Et | S | N-Me | H |
| H | n-Pr | S | N-Me | H |
| H | i-Pr | S | N-Me | H |
| H | n-Bu | S | N-Me | H |
| H | i-Bu | S | N-Me | H |
| H | s-Bu | S | N-Me | H |

| | | | | |
|---|---|---|---|---|
| H | t-Bu | S | N-Me | H |
| H | n-Pen | S | N-Me | H |
| H | 3-Me-n-Bu | S | N-Me | H |
| H | n-Hex | S | N-Me | H |
| H | Ethenyl | S | N-Me | H |
| H | 1-Propenyl | S | N-Me | H |
| H | Ethynyl | S | N-Me | H |
| H | $CF_3$ | S | N-Me | H |
| H | c-Pr | S | N-Me | H |
| H | c-Hex | S | N-Me | H |
| H | CN | S | N-Me | H |
| H | CHO | S | N-Me | H |
| H | $CH_2Ph$ | S | N-Me | H |
| H | CH=CHPh | S | N-Me | H |
| H | CH=CH-(4-Ph)Ph | S | N-Me | H |
| H | $CH_2CH$=CHPh | S | N-Me | H |
| H | OPh | S | N-Me | H |
| H | $CO_2Me$ | S | N-Me | H |
| H | $CO_2Et$ | S | N-Me | H |
| H | $CH_2OMe$ | S | N-Me | H |
| H | C(=NOMe)OMe | S | N-Me | H |
| H | C(=NOMe)OSMe | S | N-Me | H |
| H | C(=NOMe)Me | S | N-Me | H |
| H | C(=NOMe)C(=NOMe)Me | S | N-Me | H |
| H | C(=NOMe)Ph | S | N-Me | H |
| H | C(=$NOCH_2Ph$)Me | S | N-Me | H |
| H | COMe | S | N-Me | H |
| H | $CH_2SMe$ | S | N-Me | H |
| H | $CH_2OPh$ | S | N-Me | H |
| H | $CH_2O$(4-Me-Ph) | S | N-Me | H |
| H | $CH_2O$(2,4-$Cl_2$-Ph) | S | N-Me | H |
| H | $CH_2SCH_2Ph$ | S | N-Me | H |
| H | $CH_2NMe_2$ | S | N-Me | H |
| H | $CH_2N$(COMe)Me | S | N-Me | H |
| H | $CH_2ON$=$CMe_2$ | S | N-Me | H |
| H | $CH_2N$=NCHMePh | S | N-Me | H |
| H | $CH_2$(Morpholino) | S | N-Me | H |
| H | $CH_2$(1-Pyrazolyl) | S | N-Me | H |
| H | $CH_2$(Hexamethyleneimino) | S | N-Me | H |
| H | $CH_2$(3-Ph-1-Pyrazolyl) | S | N-Me | H |
| H | $CH_2$(1-Imidazolyl) | S | N-Me | H |
| H | $CF_2Cl$ | S | N-Me | H |
| H | $CCl_3$ | S | N-Me | H |
| H | $ClCH_2$ | S | N-Me | H |
| H | $BrCH_2$ | S | N-Me | H |
| H | $FCH_2$ | S | N-Me | H |
| H | $ICH_2$ | S | N-Me | H |
| H | 1-Naphthyl | S | N-Me | H |
| H | 2-Naphthyl | S | N-Me | H |
| H | Thiophen-2-yl | S | N-Me | H |
| H | Thiophen-3-yl | S | N-Me | H |
| H | 5-Cl-Thiophen-2-yl | S | N-Me | H |
| H | 2,5-$Cl_2$-Thiophen-3-yl | S | N-Me | H |
| H | 2,5-$Me_2$-Thiophen-3-yl | S | N-Me | H |
| H | 5-Cl-Thiophen-2-yl | S | N-Me | H |
| H | 3-Me-Thiophen-2-yl | S | N-Me | H |
| H | 4,5-$Br_2$-Thiophen-3-yl | S | N-Me | H |
| H | Furan-2-yl | S | N-Me | H |

| | | | | |
|---|---|---|---|---|
| H | Furan-3-yl | S | N-Me | H |
| H | 2,5-$Me_2$-Furan-3-yl | S | N-Me | H |
| H | 1-Me-Pyrrol-2-yl | S | N-Me | H |
| H | 1-Me-Pyrrole-3-yl | S | N-Me | H |
| H | Pyridin-2-yl | S | N-Me | H |
| H | Pyridin-3-yl | S | N-Me | H |
| H | Pyridin-4-yl | S | N-Me | H |
| H | 6-Cl-Pyridin-2-yl | S | N-Me | H |
| H | 5-$CF_3$-6-PhO-Pyridin-2-yl | S | N-Me | H |
| H | Pyrazin-2-yl | S | N-Me | H |
| H | Pyrimidin-2-yl | S | N-Me | H |
| H | Pyrimidin-4-yl | S | N-Me | H |
| H | 6-MeS-Pyrimidin-5-yl | S | N-Me | H |
| H | 6-PhO-Pyrimidin-4-yl | S | N-Me | H |
| H | Thiazol-2-yl | S | N-Me | H |
| H | Thiazol-5-yl | S | N-Me | H |
| H | 5-$CF_3$-Thiazol-2-yl | S | N-Me | H |
| H | Pyrazol-3-yl | S | N-Me | H |
| H | 1-Me-Pyrazol-5-yl | S | N-Me | H |
| H | 1-Ph-Pyrazol-3-yl | S | N-Me | H |
| H | Pyrazol-5-yl | S | N-Me | H |
| H | 1-Me-3-Cl-Pyrazol-5-yl | S | N-Me | H |
| H | 1-Me-Imidazol-2-yl | S | N-Me | H |
| H | Benzothiazol-2-yl | S | N-Me | H |
| H | Benzofuran-2-yl | S | N-Me | H |
| H | Ph | S | N-Me | H |
| H | 2-Cl-Ph | S | N-Me | H |
| H | 2-Cl-Ph | S | N-Me | 4-Cl |
| H | 2-Cl-Ph | S | N-Me | 5-Cl |
| H | 2-Cl-Ph | S | N-Me | 4-$CF_3$ |
| H | 2-Cl-Ph | S | N-Me | 5-$CF_3$ |
| H | 2-Cl-Ph | S | N-Me | 4-Me |
| H | 2-Cl-Ph | S | N-Me | 5-Me |
| H | 2-Cl-Ph | S | N-Me | 4-F |
| H | 2-Cl-Ph | S | N-Me | 5-F |
| H | 2-Cl-Ph | S | N-Me | 4-MeO |
| H | 3-Cl-Ph | S | N-Me | H |
| H | 4-Cl-Ph | S | N-Me | H |
| H | 4-Cl-Ph | S | N-Me | 4-Cl |
| H | 4-Cl-Ph | S | N-Me | 5-Cl |
| H | 4-Cl-Ph | S | N-Me | 4-$CF_3$ |
| H | 4-Cl-Ph | S | N-Me | 5-$CF_3$ |
| H | 4-Cl-Ph | S | N-Me | 4-Me |
| H | 4-Cl-Ph | S | N-Me | 5-Me |
| H | 4-Cl-Ph | S | N-Me | 4-F |
| H | 4-Cl-Ph | S | N-Me | 5-F |
| H | 4-Cl-Ph | S | N-Me | 4-MeO |
| H | 2-F-Ph | S | N-Me | H |
| H | 2-F-Ph | S | N-Me | 4-Cl |
| H | 2-F-Ph | S | N-Me | 5-Cl |
| H | 2-F-Ph | S | N-Me | 4-$CF_3$ |
| H | 2-F-Ph | S | N-Me | 5-$CF_3$ |
| H | 2-F-Ph | S | N-Me | 4-Me |
| H | 2-F-Ph | S | N-Me | 5-Me |
| H | 2-F-Ph | S | N-Me | 4-F |
| H | 2-F-Ph | S | N-Me | 5-F |
| H | 2-F-Ph | S | N-Me | 4-MeO |
| H | 3-F-Ph | S | N-Me | H |

| | | | | |
|---|---|---|---|---|
| H | 4-F-Ph | S | N-Me | H |
| H | 4-F-Ph | S | N-Me | 4-Cl |
| H | 4-F-Ph | S | N-Me | 5-Cl |
| H | 4-F-Ph | S | N-Me | 4-$CF_3$ |
| H | 4-F-Ph | S | N-Me | 5-$CF_3$ |
| H | 4-F-Ph | S | N-Me | 4-Me |
| H | 4-F-Ph | S | N-Me | 5-Me |
| H | 4-F-Ph | S | N-Me | 4-F |
| H | 4-F-Ph | S | N-Me | 5-F |
| H | 4-F-Ph | S | N-Me | 4-MeO |
| H | 2-Me-Ph | S | N-Me | H |
| H | 2-Me-Ph | S | N-Me | 4-Cl |
| H | 2-Me-Ph | S | N-Me | 5-Cl |
| H | 2-Me-Ph | S | N-Me | 4-$CF_3$ |
| H | 2-Me-Ph | S | N-Me | 5-$CF_3$ |
| H | 2-Me-Ph | S | N-Me | 4-Me |
| H | 2-Me-Ph | S | N-Me | 5-Me |
| H | 2-Me-Ph | S | N-Me | 4-F |
| H | 2-Me-Ph | S | N-Me | 5-F |
| H | 2-Me-Ph | S | N-Me | 4-MeO |
| H | 3-Me-Ph | S | N-Me | H |
| H | 4-Me-Ph | S | N-Me | H |
| H | 4-Me-Ph | S | N-Me | 4-Cl |
| H | 4-Me-Ph | S | N-Me | 5-Cl |
| H | 4-Me-Ph | S | N-Me | 4-$CF_3$ |
| H | 4-Me-Ph | S | N-Me | 5-$CF_3$ |
| H | 4-Me-Ph | S | N-Me | 4-Me |
| H | 4-Me-Ph | S | N-Me | 5-Me |
| H | 4-Me-Ph | S | N-Me | 4-F |
| H | 4-Me-Ph | S | N-Me | 5-F |
| H | 4-Me-Ph | S | N-Me | 4-MeO |
| H | 2-MeO-Ph | S | N-Me | H |
| H | 4-MeO-Ph | S | N-Me | H |
| H | 4-Br-Ph | S | N-Me | H |
| H | 2,4-$Cl_2$-Ph | S | N-Me | H |
| H | 3,4-$Cl_2$-Ph | S | N-Me | H |
| H | 2,4,6-$Cl_3$-Ph | S | N-Me | H |
| H | 3,4-$(MeO)_2$-Ph | S | N-Me | H |
| H | 2-Cl-4-Me-Ph | S | N-Me | H |
| H | 2-MeO-4-Me-Ph | S | N-Me | H |
| H | 2-Cl-4-i-PrO-Ph | S | N-Me | H |
| H | 3-Cl-4-$PhCH_2O$-Ph | S | N-Me | H |
| H | 2,4-$Me_2$-Ph | S | N-Me | H |
| H | 2,5-$Me_2$-Ph | S | N-Me | H |
| H | 2,6-$F_2$-Ph | S | N-Me | 6-Cl |
| H | 2,6-$F_2$-Ph | S | N-Me | 5-Cl |
| H | 2,6-$F_2$-Ph | S | N-Me | 3-Cl |
| H | 2,6-$F_2$-Ph | S | N-Me | 4,5-$Cl_2$ |
| H | 2,6-$F_2$-Ph | S | N-Me | 4-$CF_3$ |
| H | 2,6-$F_2$-Ph | S | N-Me | 4,5-$Me_2$ |
| H | 2,6-$F_2$-Ph | S | N-Me | 4-Me |
| H | 2,6-$F_2$-Ph | S | N-Me | 5-Me |
| H | 2,6-$F_2$-Ph | S | N-Me | 4-F |
| H | 2,6-$F_2$-Ph | S | N-Me | 5-F |
| H | 2,6-$F_2$-Ph | S | N-Me | 4-MeO |
| H | 2,6-$F_2$-Ph | S | N-Me | H |
| H | 2,5-$F_2$-Ph | S | N-Me | H |
| H | 2,4-$F_2$-Ph | S | N-Me | H |

| | | | | |
|---|---|---|---|---|
| H | 2,3-$F_2$-Ph | S | N-Me | H |
| H | 3,5-$F_2$-Ph | S | N-Me | H |
| H | 3,4-$F_2$-Ph | S | N-Me | H |
| H | 2,3,4,5,6-$F_5$-Ph | S | N-Me | H |
| H | 4-Et-Ph | S | N-Me | H |
| H | 4-i-Pr-Ph | S | N-Me | H |
| H | 4-n-Bu-Ph | S | N-Me | H |
| H | 4-s-Bu-Ph | S | N-Me | H |
| H | 4-t-Bu-Ph | S | N-Me | H |
| H | 4-(t-$BuCH_2$)-Ph | S | N-Me | H |
| H | 4-Et$(Me)_2$C-Ph | S | N-Me | H |
| H | 4-n-Hex-Ph | S | N-Me | H |
| H | 4-($(Me)_2$(CN)C)-Ph | S | N-Me | H |
| H | 4-$PhCH_2$-Ph | S | N-Me | H |
| H | 4-(4-F-Ph)$(Me)_2$C-Ph | S | N-Me | H |
| H | 4-(MeCH=CH)-Ph | S | N-Me | H |
| H | 4-(MeC≡C)-Ph | S | N-Me | H |
| H | 4-$CF_3$-Ph | S | N-Me | H |
| H | 4-$CF_3CH_2$-Ph | S | N-Me | H |
| H | 4-($Cl_2C$=$CHCH_2$)-Ph | S | N-Me | H |
| H | 4-(BrC≡C)-Ph | S | N-Me | H |
| H | 4-(2,2-$F_2$-c-Bu)$CH_2$-Ph | S | N-Me | H |
| H | 4-(1-Me-c-Pr)-Ph | S | N-Me | H |
| H | 4-i-PrO-Ph | S | N-Me | H |
| H | 4-t-BuO-Ph | S | N-Me | H |
| H | 4-n-HexO-Ph | S | N-Me | H |
| H | 4-(MeC≡C-O)-Ph | S | N-Me | H |
| H | 4-($CH_2$=$CHCH_2O$)-Ph | S | N-Me | H |
| H | 4-$CHF_2O$-Ph | S | N-Me | H |
| H | 4-$CBrF_2O$-Ph | S | N-Me | H |
| H | 4-$CF_3O$-Ph | S | N-Me | H |
| H | 4-$CF_3CH_2O$-Ph | S | N-Me | H |
| H | 4-($CF_2$=$CHCH_2CH_2O$)-Ph | S | N-Me | H |
| H | 4-$CCl_3CH_2O$-Ph | S | N-Me | H |
| H | 4-MeS-Ph | S | N-Me | H |
| H | 4-s-BuS-Ph | S | N-Me | H |
| H | 4-EtSO-Ph | S | N-Me | H |
| H | 4-$MeSO_2$-Ph | S | N-Me | H |
| H | 4-$EtSO_2$-Ph | S | N-Me | H |
| H | 4-i-$PrSO_2$-Ph | S | N-Me | H |
| H | 4-t-$BuSO_2$-Ph | S | N-Me | H |
| H | 4-(MeCH=$CHCH_2S$)-Ph | S | N-Me | H |
| H | 4-($CH_2$=$CHCH_2SO$)-Ph | S | N-Me | H |
| H | 4-(ClCH=$CHCH_2SO_2$)-Ph | S | N-Me | H |
| H | 4-(HC≡$CCH_2S$)-Ph | S | N-Me | H |
| H | 4-(HC≡$CCH_2SO$-Ph) | S | N-Me | H |
| H | 4-(HC≡$CCH_2SO_2$)-Ph | S | N-Me | H |
| H | 4-$CHF_2S$-Ph | S | N-Me | H |
| H | 4-$CBrF_2S$-Ph | S | N-Me | H |
| H | 4-$CF_3S$-Ph | S | N-Me | H |
| H | 4-$CF_3CH_2S$-Ph | S | N-Me | H |
| H | 4-$CHF_2CF_2S$-Ph | S | N-Me | H |
| H | 4-$CHF_2SO$-Ph | S | N-Me | H |
| H | 4-$CBrF_2SO$-Ph | S | N-Me | H |
| H | 4-$CF_3SO$-Ph | S | N-Me | H |
| H | 4-$CF_3CH_2SO_2$-Ph | S | N-Me | H |
| H | 4-$CHF_2CF_2SO_2$-Ph | S | N-Me | H |
| H | 4-$CHF_2SO_2$-Ph | S | N-Me | H |

| | | | | |
|---|---|---|---|---|
| H | 4-$CBrF_2SO_2$-Ph | S | N-Me | H |
| H | 4-$CF_3SO_2$-Ph | S | N-Me | H |
| H | 4-($Cl_2C$=$CHCH_2S$)-Ph | S | N-Me | H |
| H | 4-($Cl_2C$=$CHCH_2SO$)-Ph | S | N-Me | H |
| H | 4-($Cl_2C$=$CHCH_2SO_2$)-Ph | S | N-Me | H |
| H | 4-(BrC≡$CCH_2S$)-Ph | S | N-Me | H |
| H | 4-(BrC≡$CCH_2SO$)-Ph | S | N-Me | H |
| H | 4-(BrC≡$CCH_2SO_2$)-Ph | S | N-Me | H |
| H | 4-CHO-Ph | S | N-Me | H |
| H | 4-$NO_2$-Ph | S | N-Me | H |
| H | 3-CN-Ph | S | N-Me | H |
| H | 4-CN-Ph | S | N-Me | H |
| H | 4-$(Me)_2N$-Ph | S | N-Me | H |
| H | 4-Me(MeC(O))N-Ph | S | N-Me | H |
| H | 4-PhN(Me)-Ph | S | N-Me | H |
| H | 4-$PhCH_2$(MeCO)N-Ph | S | N-Me | H |
| H | 4-$PhCH_2O$-Ph | S | N-Me | H |
| H | 4-(2-Cl-Ph)$CH_2O$-Ph | S | N-Me | H |
| H | 4-(3-Cl-Ph)$CH_2O$-Ph | S | N-Me | H |
| H | 4-(4-Cl-Ph)$CH_2O$-Ph | S | N-Me | H |
| H | 4-(2-Me-Ph)$CH_2O$-Ph | S | N-Me | H |
| H | 4-(3-Me-Ph)$CH_2O$-Ph | S | N-Me | H |
| H | 4-(4-F-Ph)$CH_2O$-Ph | S | N-Me | H |
| H | 4-(4-Et-Ph)$CH_2O$-Ph | S | N-Me | H |
| H | 4-(2-Cl-Ph)$CH_2S$-Ph | S | N-Me | H |
| H | 4-(3-Cl-Ph)$CH_2S$-Ph | S | N-Me | H |
| H | 4-(4-Cl-Ph)$CH_2SO$-Ph | S | N-Me | H |
| H | 4-(2-Me-Ph)$CH_2S$-Ph | S | N-Me | H |
| H | 4-(3-Me-Ph)$CH_2SO_2$-Ph | S | N-Me | H |
| H | 4-(2,4-$F_2$-Ph)$CH_2O$-Ph | S | N-Me | H |
| H | 3-(3,4-$Cl_2$-Ph)$CH_2O$-Ph | S | N-Me | H |
| H | 4-(2,5-$Me_2$-Ph)$CH_2O$-Ph | S | N-Me | H |
| H | 4-(2,3,5,6-$F_4$-Ph)$CH_2O$-Ph | S | N-Me | H |
| H | 4-MeC(O)-Ph | S | N-Me | H |
| H | 4-EtC(O)-Ph | S | N-Me | H |
| H | 4-n-PrC(O)-Ph | S | N-Me | H |
| H | 4-i-PrC(O)-Ph | S | N-Me | H |
| H | 4-i-BuC(O)-Ph | S | N-Me | H |
| H | 4-t-BuC(O)-Ph | S | N-Me | H |
| H | 4-i-$BuCH_2C$(O)-Ph | S | N-Me | H |
| H | 4-Et$(Me)_2$CC(O)-Ph | S | N-Me | H |
| H | 4-n-HexC(O)-Ph | S | N-Me | H |
| H | 4-PhC(O)-Ph | S | N-Me | H |
| H | 4-(2-Cl-Ph)C(O)-Ph | S | N-Me | H |
| H | 4-(3-Br-Ph)C(O)-Ph | S | N-Me | H |
| H | 4-(4-Cl-Ph)C(O)-Ph | S | N-Me | H |
| H | 4-(2-Me-Ph)C(O)-Ph | S | N-Me | H |
| H | 4-$MeOCH_2$-Ph | S | N-Me | H |
| H | 4-$EtOCH_2$-Ph | S | N-Me | H |
| H | 4-i-$PrOCH_2$-Ph | S | N-Me | H |
| H | 4-$MeSCH_2$-Ph | S | N-Me | H |
| H | 4-$EtSCH_2$-Ph | S | N-Me | H |
| H | 4-i-$PrSCH_2$-Ph | S | N-Me | H |
| H | 4-$CF_3C$(O)-Ph | S | N-Me | H |
| H | 4-$CF_3CF_2C$(O)-Ph | S | N-Me | H |
| H | 4-MeC(O)O-Ph | S | N-Me | H |
| H | 4-EtC(O)O-Ph | S | N-Me | H |
| H | 4-n-PrC(O)O-Ph | S | N-Me | H |

| | | | | |
|---|---|---|---|---|
| H | 4-i-PrC(0)0-Ph | S | N-Me | H |
| H | 4-i-BuC(0)0-Ph | S | N-Me | H |
| H | 4-t-BuC(0)0-Ph | S | N-Me | H |
| H | 4-i-BuCH$_2$C(0)0-Ph | S | N-Me | H |
| H | 4-Et(Me)$_2$CC(0)0-Ph | S | N-Me | H |
| H | 4-n-HexC(0)0-Ph | S | N-Me | H |
| H | 4-CF$_3$C(0)0-Ph | S | N-Me | H |
| H | 4-CF$_3$CF$_2$C(0)0-Ph | S | N-Me | H |
| H | 4-PhC(0)0-Ph | S | N-Me | H |
| H | 3-Ph-Ph | S | N-Me | H |
| H | 4-Ph-Ph | S | N-Me | H |
| H | 4-(4-Cl-Ph)-Ph | S | N-Me | H |
| H | 4-(2,5-Me$_2$-Ph)-3-Me-Ph | S | N-Me | H |
| H | 3-PhO-Ph | S | N-Me | H |
| H | 4-PhO-Ph | S | N-Me | H |
| H | 4-(4-Cl-Ph)O-Ph | S | N-Me | H |
| H | 4-(4-Me-Ph)O-Ph | S | N-Me | H |
| H | 4-(4-F-Ph)O-Ph | S | N-Me | H |
| H | 4-(4-MeO-Ph)O-Ph | S | N-Me | H |
| H | 4-(2,4-Cl$_2$-Ph)O-Ph | S | N-Me | H |
| H | 4-(3,4-Cl$_2$-Ph)O-Ph | S | N-Me | H |
| H | 4-(Pyridin-2-yl)-Ph | S | N-Me | H |
| H | 4-(5-Cl-Pyridin-2-yl)-Ph | S | N-Me | H |
| H | 4-(6-F-5-CF$_3$-Pyridin-2-yl)-Ph | S | N-Me | H |
| H | 4-(Pyridin-2-yl)O-Ph | S | N-Me | H |
| H | 4-(5-Cl-Pyridin-2-yl)O-Ph | S | N-Me | H |
| H | 4-(3-Cl-5-F-Pyridin-2-yl)O-Ph | S | N-Me | H |
| H | 4-(5-Cl-Thiophen-2-yl)O-Ph | S | N-Me | H |
| -CH=CH-CH=CH- | | S | N-Me | H |
| -CCl=CH-CH=CH- | | S | N-Me | H |
| -CH=CCl-CH=CH- | | S | N-Me | H |
| -CH=CH-CCl=CH- | | S | N-Me | H |
| -CH=CH-CH=CCl- | | S | N-Me | H |
| -CH=CH-C(OMe)=CH- | | S | N-Me | H |
| -CH=CH-CF=CH- | | S | N-Me | H |
| -CH=CH-CMe=CH- | | S | N-Me | H |
| -CH=CH-CH=N- | | S | N-Me | H |
| -CH=CH-N=CH- | | S | N-Me | H |
| -CH=N-CH=CH- | | S | N-Me | H |
| -N=CH-CH=CH- | | S | N-Me | H |
| -(CH$_2$)$_3$- | | S | N-Me | H |
| -(CH$_2$)$_4$- | | S | N-Me | H |
| -(CH$_2$)$_5$- | | S | N-Me | H |
| -CH$_2$-O-CH$_2$- | | S | N-Me | H |
| -CH$_2$-CH$_2$-O-CH$_2$- | | S | N-Me | H |
| -CO-(CH$_2$)$_3$- | | S | N-Me | H |
| -CH$_2$-CH(CH$_2$Ph)CH$_2$- | | S | N-Me | H |
| -CH$_2$-CH$_2$-CH(Me)-CH$_2$- | | S | N-Me | H |
| -CH$_2$-CH$_2$-CH(Ph)-CH$_2$- | | S | N-Me | H |
| Me | Me | S | N-Me | H |
| Me | Et | S | N-Me | H |
| Me | n-Pr | S | N-Me | H |
| Me | i-Pr | S | N-Me | H |
| Me | n-Bu | S | N-Me | H |
| Me | n-Hex | S | N-Me | H |
| Me | Ethenyl | S | N-Me | H |
| Me | 1-Propynyl | S | N-Me | H |
| Me | CF$_3$ | S | N-Me | H |

| | | | | |
|---|---|---|---|---|
| Me | c-Pr | S | N-Me | H |
| Me | CN | S | N-Me | H |
| Me | CHO | S | N-Me | H |
| Me | $CH_2Ph$ | S | N-Me | H |
| Me | $CO_2Me$ | S | N-Me | H |
| Me | $CH_2OMe$ | S | N-Me | H |
| Me | COMe | S | N-Me | H |
| Me | $CH_2SMe$ | S | N-Me | H |
| Me | $CH_2OPh$ | S | N-Me | H |
| Me | $CH_2O$(4-Me-Ph) | S | N-Me | H |
| Me | $CH_2O$(2,4-$Cl_2$-Ph) | S | N-Me | H |
| Me | $CH_2SCH_2Ph$ | S | N-Me | H |
| Me | $CF_2Cl$ | S | N-Me | H |
| Me | 1-Naphthyl | S | N-Me | H |
| Me | 2-Naphthyl | S | N-Me | H |
| Me | Thiophen-2-yl | S | N-Me | H |
| Me | Furan-2-yl | S | N-Me | H |
| Me | 1-Me-Pyrrol-2-yl | S | N-Me | H |
| Me | Pyridin-4-yl | S | N-Me | H |
| Me | 6-Cl-Pyridin-2-yl | S | N-Me | H |
| Me | Pyrazin-2-yl | S | N-Me | H |
| Me | Pyrimidin-2-yl | S | N-Me | H |
| Me | Thiazol-2-yl | S | N-Me | H |
| Me | Thiazol-5-yl | S | N-Me | H |
| Me | 5-$CF_3$-Thiazol-2-yl | S | N-Me | H |
| Me | 1-Me-3-Cl-Pyrazol-5-yl | S | N-Me | H |
| Me | 1-Me-Imidazol-2-yl | S | N-Me | H |
| Me | Ph | S | N-Me | H |
| Me | 2-Cl-Ph | S | N-Me | H |
| Me | 3-Cl-Ph | S | N-Me | H |
| Me | 4-F-Ph | S | N-Me | H |
| Me | 2-Me-Ph | S | N-Me | H |
| Me | 3-Me-Ph | S | N-Me | H |
| Me | 4-Me-Ph | S | N-Me | H |
| Me | 2-MeO-Ph | S | N-Me | H |
| Me | 3-MeO-Ph | S | N-Me | H |
| Me | 4-MeO-Ph | S | N-Me | H |
| Me | 2,4-$Cl_2$-Ph | S | N-Me | H |
| Me | 2-Cl-4-Me-Ph | S | N-Me | H |
| Me | 2,5-$Me_2$-Ph | S | N-Me | H |
| Me | 2,6-$F_2$-Ph | S | N-Me | H |
| Me | 4-Et-Ph | S | N-Me | H |
| Me | 4-$PhCH_2$-Ph | S | N-Me | H |
| Me | 4-$CF_3$-Ph | S | N-Me | H |
| Me | 4-MeS-Ph | S | N-Me | H |
| Me | 4-EtSO-Ph | S | N-Me | H |
| Me | 4-$MeSO_2$-Ph | S | N-Me | H |
| Me | 4-$EtSO_2$-Ph | S | N-Me | H |
| Me | 4-CHO-Ph | S | N-Me | H |
| Me | 4-$NO_2$-Ph | S | N-Me | H |
| Me | 3-CN-Ph | S | N-Me | H |
| Me | 4-CN-Ph | S | N-Me | H |
| Me | 4-$PhCH_2$(MeCO)N-Ph | S | N-Me | H |
| Me | 4-(2,4-$F_2$-Ph)$CH_2O$-Ph | S | N-Me | H |
| Me | 4-MeC(O)-Ph | S | N-Me | H |
| Me | 4-(4-Cl-Ph)C(O)-Ph | S | N-Me | H |
| Me | 4-$MeOCH_2$-Ph | S | N-Me | H |
| Me | 4-$EtOCH_2$-Ph | S | N-Me | H |

| | | | | |
|---|---|---|---|---|
| Me | 4-$MeSCH_2$-Ph | S | N-Me | H |
| Me | 4-$EtSCH_2$-Ph | S | N-Me | H |
| Me | 4-$CF_3C(O)$-Ph | S | N-Me | H |
| Me | 4-MeC(O)O-Ph | S | N-Me | H |
| Me | 4-t-BuC(O)O-Ph | S | N-Me | H |
| Me | 4-$CF_3C(O)O$-Ph | S | N-Me | H |
| Me | 4-PhC(O)O-Ph | S | N-Me | H |
| Me | 4-Ph-Ph | S | N-Me | H |
| Me | 4-(4-Cl-Ph)-Ph | S | N-Me | H |
| Me | 4-(4-MeO-Ph)O-Ph | S | N-Me | H |
| Me | 4-(2,4-$Cl_2$-Ph)O-Ph | S | N-Me | H |
| Me | 4-(Pyridin-2-yl)O-Ph | S | N-Me | H |
| Me | 4-(5-Cl-Pyridin-2-yl)O-Ph | S | N-Me | H |
| Et | Et | S | N-Me | H |
| Et | c-Pr | S | N-Me | H |
| Et | $CH_2Ph$ | S | N-Me | H |
| Et | $CO_2Me$ | S | N-Me | H |
| Et | $CH_2OMe$ | S | N-Me | H |
| Et | COMe | S | N-Me | H |
| Et | $CH_2SMe$ | S | N-Me | H |
| Et | $CH_2OPh$ | S | N-Me | H |
| Et | $CH_2O$(4-Me-Ph) | S | N-Me | H |
| Et | $CH_2SCH_2Ph$ | S | N-Me | H |
| Et | 2-Naphthyl | S | N-Me | H |
| Et | Thiophen-2-yl | S | N-Me | H |
| Et | Furan-3-yl | S | N-Me | H |
| Et | 1-Me-Pyrrole-3-yl | S | N-Me | H |
| Et | Pyridin-2-yl | S | N-Me | H |
| Et | Pyrazin-2-yl | S | N-Me | H |
| Et | Pyrimidin-2-yl | S | N-Me | H |
| Et | Thiazol-5-yl | S | N-Me | H |
| Et | 1-Me-3-Cl-Pyrazol-5-yl | S | N-Me | H |
| Et | 1-Me-Imidazol-2-yl | S | N-Me | H |
| Et | Ph | S | N-Me | H |
| Et | 4-Cl-Ph | S | N-Me | H |
| Et | 2-F-Ph | S | N-Me | H |
| Et | 3-F-Ph | S | N-Me | H |
| Et | 4-F-Ph | S | N-Me | H |
| Et | 2-Me-Ph | S | N-Me | H |
| Et | 3-Me-Ph | S | N-Me | H |
| Et | 4-Me-Ph | S | N-Me | H |
| Et | 2-MeO-Ph | S | N-Me | H |
| Et | 3-MeO-Ph | S | N-Me | H |
| Et | 4-MeO-Ph | S | N-Me | H |
| Et | 4-Br-Ph | S | N-Me | H |
| Et | 2,4-$Cl_2$-Ph | S | N-Me | H |
| Et | 3,4-$Cl_2$-Ph | S | N-Me | H |
| Et | 4-n-Hex-Ph | S | N-Me | H |
| Et | 4-$PhCH_2$-Ph | S | N-Me | H |
| Et | 4-$CF_3$-Ph | S | N-Me | H |
| Et | 4-$CF_3O$-Ph | S | N-Me | H |
| Et | 4-MeS-Ph | S | N-Me | H |
| Et | 4-$MeSO_2$-Ph | S | N-Me | H |
| Et | 4-CHO-Ph | S | N-Me | H |
| Et | 4-$NO_2$-Ph | S | N-Me | H |
| Et | 3-CN-Ph | S | N-Me | H |
| Et | 4-CN-Ph | S | N-Me | H |
| Et | 4-$(Me)_2N$-Ph | S | N-Me | H |

| | | | | |
|---|---|---|---|---|
| Et | 4-$PhCH_2O$-Ph | S | N-Me | H |
| Et | 4-MeC(O)-Ph | S | N-Me | H |
| Et | 4-(4-Cl-Ph)C(O)-Ph | S | N-Me | H |
| Et | 4-$MeOCH_2$-Ph | S | N-Me | H |
| Et | 4-$EtSCH_2$-Ph | S | N-Me | H |
| Et | 4-$CF_3C$(O)-Ph | S | N-Me | H |
| Et | 4-MeC(O)O-Ph | S | N-Me | H |
| Et | 4-$CF_3C$(O)O-Ph | S | N-Me | H |
| Et | 4-PhC(O)O-Ph | S | N-Me | H |
| Et | 4-Ph-Ph | S | N-Me | H |
| Et | 4-(4-Me-Ph)O-Ph | S | N-Me | H |
| Et | 4-(Pyridin-2-yl)O-Ph | S | N-Me | H |
| Ph | $CO_2Me$ | S | N-Me | H |
| Ph | COMe | S | N-Me | H |
| Ph | 2-Naphthyl | S | N-Me | H |
| Ph | Thiophen-2-yl | S | N-Me | H |
| Ph | Furan-3-yl | S | N-Me | H |
| Ph | 1-Me-Pyrrol-2-yl | S | N-Me | H |
| Ph | Pyridin-4-yl | S | N-Me | H |
| Ph | Pyrazin-2-yl | S | N-Me | H |
| Ph | Pyrimidin-4-yl | S | N-Me | H |
| Ph | Thiazol-2-yl | S | N-Me | H |
| Ph | 1-Me-3-Cl-Pyrazol-5-yl | S | N-Me | H |
| Ph | 1-Me-Imidazol-2-yl | S | N-Me | H |
| Ph | Ph | S | N-Me | H |
| Ph | 4-Cl-Ph | S | N-Me | H |
| Ph | 2-F-Ph | S | N-Me | H |
| Ph | 3-F-Ph | S | N-Me | H |
| Ph | 4-F-Ph | S | N-Me | H |
| Ph | 2-Me-Ph | S | N-Me | H |
| Ph | 3-Me-Ph | S | N-Me | H |
| Ph | 4-Me-Ph | S | N-Me | H |
| Ph | 2-MeO-Ph | S | N-Me | H |
| Ph | 3-MeO-Ph | S | N-Me | H |
| Ph | 4-MeO-Ph | S | N-Me | H |
| Ph | 2,4-$Cl_2$-Ph | S | N-Me | H |
| Ph | 4-$PhCH_2$-Ph | S | N-Me | H |
| Ph | 4-$CF_3$-Ph | S | N-Me | H |
| Ph | 4-$CF_3O$-Ph | S | N-Me | H |
| Ph | 4-MeS-Ph | S | N-Me | H |
| Ph | 4-EtSO-Ph | S | N-Me | H |
| Ph | 4-$MeSO_2$-Ph | S | N-Me | H |
| Ph | 4-CHO-Ph | S | N-Me | H |
| Ph | 4-$NO_2$-Ph | S | N-Me | H |
| Ph | 3-CN-Ph | S | N-Me | H |
| Ph | 4-CN-Ph | S | N-Me | H |
| Ph | 4-$(Me)_2N$-Ph | S | N-Me | H |
| Ph | 4-Me(MeC(O))N-Ph | S | N-Me | H |
| Ph | 4-PhN(Me)-Ph | S | N-Me | H |
| Ph | $PhCH_2O$-Ph | S | N-Me | H |
| Ph | 4-MeC(O)-Ph | S | N-Me | H |
| Ph | 4-(4-Cl-Ph)C(O)-Ph | S | N-Me | H |
| Ph | 4-$MeOCH_2$-Ph | S | N-Me | H |
| Ph | 4-$EtSCH_2$-Ph | S | N-Me | H |
| Ph | 4-$CF_3C$(O)-Ph | S | N-Me | H |
| Ph | 4-MeC(O)O-Ph | S | N-Me | H |
| Ph | 4-$CF_3C$(O)O-Ph | S | N-Me | H |
| Ph | 4-PhC(O)O-Ph | S | N-Me | H |

| | | | | |
|---|---|---|---|---|
| Ph | 4-Ph-Ph | S | N-Me | H |
| Ph | 3-PhO-Ph | S | N-Me | H |
| Cl | $CH_2Ph$ | S | N-Me | H |
| Cl | $CO_2Me$ | S | N-Me | H |
| Br | $CO_2Me$ | S | N-Me | H |
| Cl | $CH_2OMe$ | S | N-Me | H |
| Cl | COMe | S | N-Me | H |
| Cl | $CH_2OPh$ | S | N-Me | H |
| Cl | $ClCH_2$ | S | N-Me | H |
| Cl | 2-Naphthyl | S | N-Me | H |
| Cl | Thiophen-2-yl | S | N-Me | H |
| Cl | Furan-2-yl | S | N-Me | H |
| Cl | Pyridin-4-yl | S | N-Me | H |
| Br | Pyridin-4-yl | S | N-Me | H |
| Cl | Thiazol-2-yl | S | N-Me | H |
| Cl | 1-Me-3-Cl-Pyrazol-5-yl | S | N-Me | H |
| Cl | 1-Me-Imidazol-2-yl | S | N-Me | H |
| Cl | Ph | S | N-Me | H |
| Br | Ph | S | N-Me | H |
| Cl | 4-Cl-Ph | S | N-Me | H |
| Cl | 4-Me-Ph | S | N-Me | H |
| Cl | 4-MeO-Ph | S | N-Me | H |
| Cl | $4-CF_3-Ph$ | S | N-Me | H |
| Cl | 4-CHO-Ph | S | N-Me | H |
| Cl | $4-NO_2-Ph$ | S | N-Me | H |
| Cl | 4-CN-Ph | S | N-Me | H |
| Cl | 4-Ph-Ph | S | N-Me | H |
| H | Ph | S | N-Me | 4-Cl |
| H | Ph | S | N-Me | 6-F |
| H | Ph | S | N-Me | 5-Cl |
| H | Ph | S | N-Me | 3-F |
| H | Ph | S | N-Me | $4,5-Cl_2$ |
| H | Ph | S | N-Me | $4-CF_3$ |
| H | Ph | S | N-Me | $4,5-Me_2$ |
| H | Ph | S | N-Me | 4-Me |
| H | Ph | S | N-Me | 5-Me |
| H | Ph | S | N-Me | 4-F |
| H | Ph | S | N-Me | 5-F |
| H | Ph | S | N-Me | 4-MeO |
| Me | Ph | S | N-Me | 5-Cl |
| Me | Ph | S | N-Me | $4-CF_3$ |
| Me | Ph | S | N-Me | 5-Me |
| Me | Ph | S | N-Me | 4-F |
| Me | Ph | S | N-Me | 4-MeO |
| H | H | S | S | H |
| H | Cl | S | S | H |
| H | Me | S | S | H |
| H | Et | S | S | H |
| H | n-Pr | S | S | H |
| H | i-Pr | S | S | H |
| H | n-Bu | S | S | H |
| H | i-Bu | S | S | H |
| H | s-Bu | S | S | H |
| H | t-Bu | S | S | H |
| H | n-Pen | S | S | H |
| H | 3-Me-n-Bu | S | S | H |
| H | n-Hex | S | S | H |
| H | Ethenyl | S | S | H |

| | | | | |
|---|---|---|---|---|
| H | 1-Propenyl | S | S | H |
| H | Ethynyl | S | S | H |
| H | $CF_3$ | S | S | H |
| H | c-Pr | S | S | H |
| H | c-Hex | S | S | H |
| H | CN | S | S | H |
| H | CHO | S | S | H |
| H | $CH_2Ph$ | S | S | H |
| H | CH=CHPh | S | S | H |
| H | CH=CH-(4-Ph)Ph | S | S | H |
| H | $CH_2CH$=CHPh | S | S | H |
| H | OPh | S | S | H |
| H | $CO_2Me$ | S | S | H |
| H | $CO_2Et$ | S | S | H |
| H | $CH_2OMe$ | S | S | H |
| H | C(=NOMe)OMe | S | S | H |
| H | C(=NOMe)OSMe | S | S | H |
| H | C(=NOMe)Me | S | S | H |
| H | C(=NOMe)C(=NOMe)Me | S | S | H |
| H | C(=NOMe)Ph | S | S | H |
| H | C(=$NOCH_2Ph$)Me | S | S | H |
| H | COMe | S | S | H |
| H | $CH_2SMe$ | S | S | H |
| H | $CH_2OPh$ | S | S | H |
| H | $CH_2O$(4-Me-Ph) | S | S | H |
| H | $CH_2O$(2,4-$Cl_2$-Ph) | S | S | H |
| H | $CH_2SCH_2Ph$ | S | S | H |
| H | $CH_2NMe_2$ | S | S | H |
| H | $CH_2N$(COMe)Me | S | S | H |
| H | $CH_2ON$=$CMe_2$ | S | S | H |
| H | $CH_2N$=NCHMePh | S | S | H |
| H | $CH_2$(Morpholino) | S | S | H |
| H | $CH_2$(1-Pyrazolyl) | S | S | H |
| H | $CH_2$(Hexamethyleneimino) | S | S | H |
| H | $CH_2$(3-Ph-1-Pyrazolyl) | S | S | H |
| H | $CH_2$(1-Imidazolyl) | S | S | H |
| H | $CF_2Cl$ | S | S | H |
| H | $CCl_3$ | S | S | H |
| H | $ClCH_2$ | S | S | H |
| H | $BrCH_2$ | S | S | H |
| H | $FCH_2$ | S | S | H |
| H | $ICH_2$ | S | S | H |
| H | 1-Naphthyl | S | S | H |
| H | 2-Naphthyl | S | S | H |
| H | Thiophen-2-yl | S | S | H |
| H | Thiophen-3-yl | S | S | H |
| H | 5-Cl-Thiophen-2-yl | S | S | H |
| H | 2,5-$Cl_2$-Thiophen-3-yl | S | S | H |
| H | 2,5-$Me_2$-Thiophen-3-yl | S | S | H |
| H | 5-Cl-Thiophen-2-yl | S | S | H |
| H | 3-Me-Thiophen-2-yl | S | S | H |
| H | 4,5-$Br_2$-Thiophen-3-yl | S | S | H |
| H | Furan-2-yl | S | S | H |
| H | Furan-3-yl | S | S | H |
| H | 2,5-$Me_2$-Furan-3-yl | S | S | H |
| H | 1-Me-Pyrrol-2-yl | S | S | H |
| H | 1-Me-Pyrrole-3-yl | S | S | H |
| H | Pyridin-2-yl | S | S | H |

| | | | | |
|---|---|---|---|---|
| H | Pyridin-3-yl | S | S | H |
| H | Pyridin-4-yl | S | S | H |
| H | 6-Cl-Pyridin-2-yl | S | S | H |
| H | 5-$CF_3$-6-PhO-Pyridin-2-yl | S | S | H |
| H | Pyrazin-2-yl | S | S | H |
| H | Pyrimidin-2-yl | S | S | H |
| H | Pyrimidin-4-yl | S | S | H |
| H | 6-MeS-Pyrimidin-5-yl | S | S | H |
| H | 6-PhO-Pyrimidin-4-yl | S | S | H |
| H | Thiazol-2-yl | S | S | H |
| H | Thiazol-5-yl | S | S | H |
| H | 5-$CF_3$-Thiazol-2-yl | S | S | H |
| H | Pyrazol-3-yl | S | S | H |
| H | 1-Me-Pyrazol-5-yl | S | S | H |
| H | 1-Ph-Pyrazol-3-yl | S | S | H |
| H | Pyrazol-5-yl | S | S | H |
| H | 1-Me-3-Cl-Pyrazol-5-yl | S | S | H |
| H | 1-Me-Imidazol-2-yl | S | S | H |
| H | Benzothiazol-2-yl | S | S | H |
| H | Benzofuran-2-yl | S | S | H |
| H | Ph | S | S | H |
| H | 2-Cl-Ph | S | S | H |
| H | 3-Cl-Ph | S | S | H |
| H | 4-Cl-Ph | S | S | H |
| H | 2-F-Ph | S | S | H |
| H | 3-F-Ph | S | S | H |
| H | 4-F-Ph | S | S | H |
| H | 2-Me-Ph | S | S | H |
| H | 3-Me-Ph | S | S | H |
| H | 4-Me-Ph | S | S | H |
| H | 2-MeO-Ph | S | S | H |
| H | 3-MeO-Ph | S | S | H |
| H | 4-MeO-Ph | S | S | H |
| H | 4-Br-Ph | S | S | H |
| H | 2,4-$Cl_2$-Ph | S | S | H |
| H | 3,4-$Cl_2$-Ph | S | S | H |
| H | 2,4,6-$Cl_3$-Ph | S | S | H |
| H | 3,4-$(MeO)_2$-Ph | S | S | H |
| H | 2-Cl-4-Me-Ph | S | S | H |
| H | 2-MeO-4-Me-Ph | S | S | H |
| H | 2-Cl-4-i-PrO-Ph | S | S | H |
| H | 3-Cl-4-$PhCH_2O$-Ph | S | S | H |
| H | 2,4-$Me_2$-Ph | S | S | H |
| H | 2,5-$Me_2$-Ph | S | S | H |
| H | 2,6-$F_2$-Ph | S | S | H |
| H | 2,5-$F_2$-Ph | S | S | H |
| H | 2,4-$F_2$-Ph | S | S | H |
| H | 2,3-$F_2$-Ph | S | S | H |
| H | 3,5-$F_2$-Ph | S | S | H |
| H | 3,4-$F_2$-Ph | S | S | H |
| H | 2,3,4,5,6-$F_5$-Ph | S | S | H |
| H | 4-Et-Ph | S | S | H |
| H | 4-i-Pr-Ph | S | S | H |
| H | 4-n-Bu-Ph | S | S | H |
| H | 4-s-Bu-Ph | S | S | H |
| H | 4-t-Bu-Ph | S | S | H |
| H | 4-(t-$BuCH_2$)-Ph | S | S | H |
| H | 4-Et$(Me)_2$C-Ph | S | S | H |

| | | | | |
|---|---|---|---|---|
| H | 4-n-Hex-Ph | S | S | H |
| H | 4-((Me)$_2$(CN)C)-Ph | S | S | H |
| H | 4-PhCH$_2$-Ph | S | S | H |
| H | 4-(4-F-Ph)(Me)$_2$C-Ph | S | S | H |
| H | 4-(MeCH=CH)-Ph | S | S | H |
| H | 4-(MeC≡C)-Ph | S | S | H |
| H | 4-CF$_3$-Ph | S | S | H |
| H | 4-CF$_3$CH$_2$-Ph | S | S | H |
| H | 4-(Cl$_2$C=CHCH$_2$)-Ph | S | S | H |
| H | 4-(BrC≡C)-Ph | S | S | H |
| H | 4-(2,2-F$_2$-c-Bu)CH$_2$-Ph | S | S | H |
| H | 4-(1-Me-c-Pr)-Ph | S | S | H |
| H | 4-i-PrO-Ph | S | S | H |
| H | 4-t-BuO-Ph | S | S | H |
| H | 4-n-HexO-Ph | S | S | H |
| H | 4-(MeC≡C-O)-Ph | S | S | H |
| H | 4-(CH$_2$=CHCH$_2$O)-Ph | S | S | H |
| H | 4-CHF$_2$O-Ph | S | S | H |
| H | 4-CBrF$_2$O-Ph | S | S | H |
| H | 4-CF$_3$O-Ph | S | S | H |
| H | 4-CF$_3$CH$_2$O-Ph | S | S | H |
| H | 4-(CF$_2$=CHCH$_2$CH$_2$O)-Ph | S | S | H |
| H | 4-CCl$_3$CH$_2$O-Ph | S | S | H |
| H | 4-MeS-Ph | S | S | H |
| H | 4-s-BuS-Ph | S | S | H |
| H | 4-EtSO-Ph | S | S | H |
| H | 4-MeSO$_2$-Ph | S | S | H |
| H | 4-EtSO$_2$-Ph | S | S | H |
| H | 4-i-PrSO$_2$-Ph | S | S | H |
| H | 4-t-BuSO$_2$-Ph | S | S | H |
| H | 4-(MeCH=CHCH$_2$S)-Ph | S | S | H |
| H | 4-(CH$_2$=CHCH$_2$SO)-Ph | S | S | H |
| H | 4-(ClCH=CHCH$_2$SO$_2$)-Ph | S | S | H |
| H | 4-(HC≡CCH$_2$S)-Ph | S | S | H |
| H | 4-(HC≡CCH$_2$SO-Ph) | S | S | H |
| H | 4-(HC≡CCH$_2$SO$_2$)-Ph | S | S | H |
| H | 4-CHF$_2$S-Ph | S | S | H |
| H | 4-CBrF$_2$S-Ph | S | S | H |
| H | 4-CF$_3$S-Ph | S | S | H |
| H | 4-CF$_3$CH$_2$S-Ph | S | S | H |
| H | 4-CHF$_2$CF$_2$S-Ph | S | S | H |
| H | 4-CHF$_2$SO-Ph | S | S | H |
| H | 4-CBrF$_2$SO-Ph | S | S | H |
| H | 4-CF$_3$SO-Ph | S | S | H |
| H | 4-CF$_3$CH$_2$SO$_2$-Ph | S | S | H |
| H | 4-CHF$_2$CF$_2$SO$_2$-Ph | S | S | H |
| H | 4-CHF$_2$SO$_2$-Ph | S | S | H |
| H | 4-CBrF$_2$SO$_2$-Ph | S | S | H |
| H | 4-CF$_3$SO$_2$-Ph | S | S | H |
| H | 4-(Cl$_2$C=CHCH$_2$S)-Ph | S | S | H |
| H | 4-(Cl$_2$C=CHCH$_2$SO)-Ph | S | S | H |
| H | 4-(Cl$_2$C=CHCH$_2$SO$_2$)-Ph | S | S | H |
| H | 4-(BrC≡CCH$_2$S)-Ph | S | S | H |
| H | 4-(BrC≡CCH$_2$SO)-Ph | S | S | H |
| H | 4-(BrC≡CCH$_2$SO$_2$)-Ph | S | S | H |
| H | 4-CHO-Ph | S | S | H |
| H | 4-NO$_2$-Ph | S | S | H |
| H | 3-CN-Ph | S | S | H |

| | | | | |
|---|---|---|---|---|
| H | 4-CN-Ph | S | S | H |
| H | 4-$(Me)_2$N-Ph | S | S | H |
| H | 4-Me(MeC(O))N-Ph | S | S | H |
| H | 4-PhN(Me)-Ph | S | S | H |
| H | 4-$PhCH_2$(MeCO)N-Ph | S | S | H |
| H | 4-$PhCH_2$O-Ph | S | S | H |
| H | 4-(2-Cl-Ph)$CH_2$O-Ph | S | S | H |
| H | 4-(3-Cl-Ph)$CH_2$O-Ph | S | S | H |
| H | 4-(4-Cl-Ph)$CH_2$O-Ph | S | S | H |
| H | 4-(2-Me-Ph)$CH_2$O-Ph | S | S | H |
| H | 4-(3-Me-Ph)$CH_2$O-Ph | S | S | H |
| H | 4-(4-F-Ph)$CH_2$O-Ph | S | S | H |
| H | 4-(4-Et-Ph)$CH_2$O-Ph | S | S | H |
| H | 4-(2-Cl-Ph)$CH_2$S-Ph | S | S | H |
| H | 4-(3-Cl-Ph)$CH_2$S-Ph | S | S | H |
| H | 4-(4-Cl-Ph)$CH_2$SO-Ph | S | S | H |
| H | 4-(2-Me-Ph)$CH_2$S-Ph | S | S | H |
| H | 4-(3-Me-Ph)$CH_2SO_2$-Ph | S | S | H |
| H | 4-(2,4-$F_2$-Ph)$CH_2$O-Ph | S | S | H |
| H | 3-(3,4-$Cl_2$-Ph)$CH_2$O-Ph | S | S | H |
| H | 4-(2,5-$Me_2$-Ph)$CH_2$O-Ph | S | S | H |
| H | 4-(2,3,5,6-$F_4$-Ph)$CH_2$O-Ph | S | S | H |
| H | 4-MeC(O)-Ph | S | S | H |
| H | 4-EtC(O)-Ph | S | S | H |
| H | 4-n-PrC(O)-Ph | S | S | H |
| H | 4-i-PrC(O)-Ph | S | S | H |
| H | 4-i-BuC(O)-Ph | S | S | H |
| H | 4-t-BuC(O)-Ph | S | S | H |
| H | 4-i-$BuCH_2$C(O)-Ph | S | S | H |
| H | 4-Et$(Me)_2$CC(O)-Ph | S | S | H |
| H | 4-n-HexC(O)-Ph | S | S | H |
| H | 4-PhC(O)-Ph | S | S | H |
| H | 4-(2-Cl-Ph)C(O)-Ph | S | S | H |
| H | 4-(3-Br-Ph)C(O)-Ph | S | S | H |
| H | 4-(4-Cl-Ph)C(O)-Ph | S | S | H |
| H | 4-(2-Me-Ph)C(O)-Ph | S | S | H |
| H | 4-$MeOCH_2$-Ph | S | S | H |
| H | 4-$EtOCH_2$-Ph | S | S | H |
| H | 4-i-$PrOCH_2$-Ph | S | S | H |
| H | 4-$MeSCH_2$-Ph | S | S | H |
| H | 4-$EtSCH_2$-Ph | S | S | H |
| H | 4-i-$PrSCH_2$-Ph | S | S | H |
| H | 4-$CF_3$C(O)-Ph | S | S | H |
| H | 4-$CF_3CF_2$C(O)-Ph | S | S | H |
| H | 4-MeC(O)O-Ph | S | S | H |
| H | 4-EtC(O)O-Ph | S | S | H |
| H | 4-n-PrC(O)O-Ph | S | S | H |
| H | 4-i-PrC(O)O-Ph | S | S | H |
| H | 4-i-BuC(O)O-Ph | S | S | H |
| H | 4-t-BuC(O)O-Ph | S | S | H |
| H | 4-i-$BuCH_2$C(O)O-Ph | S | S | H |
| H | 4-Et$(Me)_2$C-C(O)O-Ph | S | S | H |
| H | 4-n-HexC(O)O-Ph | S | S | H |
| H | 4-$CF_3$C(O)O-Ph | S | S | H |
| H | 4-$CF_3CF_2$C(O)O-Ph | S | S | H |
| H | 4-PhC(O)O-Ph | S | S | H |
| H | 3-Ph-Ph | S | S | H |
| H | 4-Ph-Ph | S | S | H |

| | | | | |
|---|---|---|---|---|
| H | 4-(4-Cl-Ph)-Ph | S | S | H |
| H | 4-(2,5-$Me_2$-Ph)-3-Me-Ph | S | S | H |
| H | 3-PhO-Ph | S | S | H |
| H | 4-PhO-Ph | S | S | H |
| H | 4-(4-Cl-Ph)O-Ph | S | S | H |
| H | 4-(4-Me-Ph)O-Ph | S | S | H |
| H | 4-(4-F-Ph)O-Ph | S | S | H |
| H | 4-(4-MeO-Ph)O-Ph | S | S | H |
| H | 4-(2,4-$Cl_2$-Ph)O-Ph | S | S | H |
| H | 4-(3,4-$Cl_2$-Ph)O-Ph | S | S | H |
| H | 4-(Pyridin-2-yl)-Ph | S | S | H |
| H | 4-(5-Cl-Pyridin-2-yl)-Ph | S | S | H |
| H | 4-(6-F-5-$CF_3$-Pyridin-2-yl)-Ph | S | S | H |
| H | 4-(Pyridin-2-yl)O-Ph | S | S | H |
| H | 4-(5-Cl-Pyridin-2-yl)O-Ph | S | S | H |
| H | 4-(3-Cl-5-F-Pyridin-2-yl)O-Ph | S | S | H |
| H | 4-(5-Cl-Thiophen-2-yl)O-Ph | S | S | H |
| -CH=CH-CH=CH- | | S | S | H |
| -CCl=CH-CH=CH- | | S | S | H |
| -CH=CCl-CH=CH- | | S | S | H |
| -CH=CH-CCl=CH- | | S | S | H |
| -CH=CH-CH=CCl- | | S | S | H |
| -CH=CH-C(OMe)=CH- | | S | S | H |
| -CH=CH-CF=CH- | | S | S | H |
| -CH=CH-CMe=CH- | | S | S | H |
| -CH=CH-CH=N- | | S | S | H |
| -CH=CH-N=CH- | | S | S | H |
| -CH=N-CH=CH- | | S | S | H |
| -N=CH-CH=CH- | | S | S | H |
| $-(CH_2)_3-$ | | S | S | H |
| $-(CH_2)_4-$ | | S | S | H |
| $-(CH_2)_5-$ | | S | S | H |
| $-CH_2-O-CH_2-$ | | S | S | H |
| $-CH_2-CH_2-O-CH_2-$ | | S | S | H |
| $-CO-(CH_2)_3-$ | | S | S | H |
| $-CH_2-CH(CH_2Ph)CH_2-$ | | S | S | H |
| $-CH_2-CH_2-CH(Me)-CH_2-$ | | S | S | H |
| $-CH_2-CH_2-CH(Ph)-CH_2-$ | | S | S | H |
| Me | Me | S | S | H |
| Me | Et | S | S | H |
| Me | n-Pr | S | S | H |
| Me | i-Pr | S | S | H |
| Me | n-Bu | S | S | H |
| Me | n-Hex | S | S | H |
| Me | Ethenyl | S | S | H |
| Me | 1-Propynyl | S | S | H |
| Me | $CF_3$ | S | S | H |
| Me | c-Pr | S | S | H |
| Me | CN | S | S | H |
| Me | CHO | S | S | H |
| Me | $CH_2Ph$ | S | S | H |
| Me | $CO_2Me$ | S | S | H |
| Me | $CH_2OMe$ | S | S | H |
| Me | COMe | S | S | H |
| Me | $CH_2SMe$ | S | S | H |
| Me | $CH_2OPh$ | S | S | H |
| Me | $CH_2O$(4-Me-Ph) | S | S | H |
| Me | $CH_2O$(2,4-$Cl_2$-Ph) | S | S | H |

| | | | | |
|---|---|---|---|---|
| Me | $CH_2SCH_2Ph$ | S | S | H |
| Me | $CF_2Cl$ | S | S | H |
| Me | 1-Naphthyl | S | S | H |
| Me | 2-Naphthyl | S | S | H |
| Me | Thiophen-2-yl | S | S | H |
| Me | Furan-2-yl | S | S | H |
| Me | 1-Me-Pyrrol-2-yl | S | S | H |
| Me | Pyridin-4-yl | S | S | H |
| Me | 6-Cl-Pyridin-2-yl | S | S | H |
| Me | Pyrazin-2-yl | S | S | H |
| Me | Pyrimidin-2-yl | S | S | H |
| Me | Thiazol-2-yl | S | S | H |
| Me | Thiazol-5-yl | S | S | H |
| Me | 5-$CF_3$-Thiazol-2-yl | S | S | H |
| Me | 1-Me-3-Cl-Pyrazol-5-yl | S | S | H |
| Me | 1-Me-Imidazol-2-yl | S | S | H |
| Me | Ph | S | S | H |
| Me | 2-Cl-Ph | S | S | H |
| Me | 3-Cl-Ph | S | S | H |
| Me | 4-F-Ph | S | S | H |
| Me | 2-Me-Ph | S | S | H |
| Me | 3-Me-Ph | S | S | H |
| Me | 4-Me-Ph | S | S | H |
| Me | 2-MeO-Ph | S | S | H |
| Me | 3-MeO-Ph | S | S | H |
| Me | 4-MeO-Ph | S | S | H |
| Me | 2,4-$Cl_2$-Ph | S | S | H |
| Me | 2-Cl-4-Me-Ph | S | S | H |
| Me | 2,5-$Me_2$-Ph | S | S | H |
| Me | 2,6-$F_2$-Ph | S | S | H |
| Me | 4-Et-Ph | S | S | H |
| Me | 4-$PhCH_2$-Ph | S | S | H |
| Me | 4-$CF_3$-Ph | S | S | H |
| Me | 4-MeS-Ph | S | S | H |
| Me | 4-EtSO-Ph | S | S | H |
| Me | 4-$MeSO_2$-Ph | S | S | H |
| Me | 4-$EtSO_2$-Ph | S | S | H |
| Me | 4-CHO-Ph | S | S | H |
| Me | 4-$NO_2$-Ph | S | S | H |
| Me | 3-CN-Ph | S | S | H |
| Me | 4-CN-Ph | S | S | H |
| Me | 4-$PhCH_2$(MeCO)N-Ph | S | S | H |
| Me | 4-(2,4-$F_2$-Ph)$CH_2$O-Ph | S | S | H |
| Me | 4-MeC(O)-Ph | S | S | H |
| Me | 4-(4-Cl-Ph)C(O)-Ph | S | S | H |
| Me | 4-$MeOCH_2$-Ph | S | S | H |
| Me | 4-$EtOCH_2$-Ph | S | S | H |
| Me | 4-$MeSCH_2$-Ph | S | S | H |
| Me | 4-$EtSCH_2$-Ph | S | S | H |
| Me | 4-$CF_3$C(O)-Ph | S | S | H |
| Me | 4-MeC(O)O-Ph | S | S | H |
| Me | 4-t-BuC(O)O-Ph | S | S | H |
| Me | 4-$CF_3$C(O)O-Ph | S | S | H |
| Me | 4-PhC(O)O-Ph | S | S | H |
| Me | 4-Ph-Ph | S | S | H |
| Me | 4-(4-Cl-Ph)-Ph | S | S | H |
| Me | 4-(4-MeO-Ph)O-Ph | S | S | H |
| Me | 4-(2,4-$Cl_2$-Ph)O-Ph | S | S | H |

| | | | | |
|---|---|---|---|---|
| Me | 4-(Pyridin-2-yl)O-Ph | S | S | H |
| Me | 4-(5-Cl-Pyridin-2-yl)O-Ph | S | O | H |
| Et | Et | S | S | H |
| Et | c-Pr | S | S | H |
| Et | $CH_2Ph$ | S | S | H |
| Et | $CO_2Me$ | S | S | H |
| Et | $CH_2OMe$ | S | S | H |
| Et | COMe | S | S | H |
| Et | $CH_2SMe$ | S | S | H |
| Et | $CH_2OPh$ | S | S | H |
| Et | $CH_2O$(4-Me-Ph) | S | S | H |
| Et | $CH_2SCH_2Ph$ | S | S | H |
| Et | 2-Naphthyl | S | S | H |
| Et | Thiophen-2-yl | S | S | H |
| Et | Furan-3-yl | S | S | H |
| Et | 1-Me-Pyrrole-3-yl | S | S | H |
| Et | Pyridin-2-yl | S | S | H |
| Et | Pyrazin-2-yl | S | S | H |
| Et | Pyrimidin-2-yl | S | S | H |
| Et | Thiazol-5-yl | S | S | H |
| Et | 1-Me-3-Cl-Pyrazol-5-yl | S | S | H |
| Et | 1-Me-Imidazol-2-yl | S | S | H |
| Et | Ph | S | S | H |
| Et | 4-Cl-Ph | S | S | H |
| Et | 2-F-Ph | S | S | H |
| Et | 3-F-Ph | S | S | H |
| Et | 4-F-Ph | S | S | H |
| Et | 2-Me-Ph | S | S | H |
| Et | 3-Me-Ph | S | S | H |
| Et | 4-Me-Ph | S | S | H |
| Et | 2-MeO-Ph | S | S | H |
| Et | 3-MeO-Ph | S | S | H |
| Et | 4-MeO-Ph | S | S | H |
| Et | 4-Br-Ph | S | S | H |
| Et | 2,4-$Cl_2$-Ph | S | S | H |
| Et | 3,4-$Cl_2$-Ph | S | S | H |
| Et | 4-n-Hex-Ph | S | S | H |
| Et | 4-$PhCH_2$-Ph | S | S | H |
| Et | 4-$CF_3$-Ph | S | S | H |
| Et | 4-$CF_3O$-Ph | S | S | H |
| Et | 4-MeS-Ph | S | S | H |
| Et | 4-$MeSO_2$-Ph | S | S | H |
| Et | 4-CHO-Ph | S | S | H |
| Et | 4-$NO_2$-Ph | S | S | H |
| Et | 3-CN-Ph | S | S | H |
| Et | 4-CN-Ph | S | S | H |
| Et | 4-$(Me)_2N$-Ph | S | S | H |
| Et | 4-$PhCH_2O$-Ph | S | S | H |
| Et | 4-MeC(O)-Ph | S | S | H |
| Et | 4-(4-Cl-Ph)C(O)-Ph | S | S | H |
| Et | 4-$MeOCH_2$-Ph | S | S | H |
| Et | 4-$EtSCH_2$-Ph | S | S | H |
| Et | 4-$CF_3C$(O)-Ph | S | S | H |
| Et | 4-MeC(O)O-Ph | S | S | H |
| Et | 4-$CF_3C$(O)O-Ph | S | S | H |
| Et | 4-PhC(O)O-Ph | S | S | H |
| Et | 4-Ph-Ph | S | S | H |
| Et | 4-(4-Me-Ph)O-Ph | S | S | H |

| | | | | |
|---|---|---|---|---|
| Et | 4-(Pyridin-2-yl)O-Ph | S | S | H |
| Ph | $CO_2Me$ | S | S | H |
| Ph | COMe | S | S | H |
| Ph | 2-Naphthyl | S | S | H |
| Ph | Thiophen-2-yl | S | S | H |
| Ph | Furan-3-yl | S | S | H |
| Ph | 1-Me-Pyrrol-2-yl | S | S | H |
| Ph | Pyridin-4-yl | S | S | H |
| Ph | Pyrazin-2-yl | S | S | H |
| Ph | Pyrimidin-4-yl | S | S | H |
| Ph | Thiazol-2-yl | S | S | H |
| Ph | 1-Me-3-Cl-Pyrazol-5-yl | S | S | H |
| Ph | 1-Me-Imidazol-2-yl | S | S | H |
| Ph | Ph | S | S | H |
| Ph | 4-Cl-Ph | S | S | H |
| Ph | 2-F-Ph | S | S | H |
| Ph | 3-F-Ph | S | S | H |
| Ph | 4-F-Ph | S | S | H |
| Ph | 2-Me-Ph | S | S | H |
| Ph | 3-Me-Ph | S | S | H |
| Ph | 4-Me-Ph | S | S | H |
| Ph | 2-MeO-Ph | S | S | H |
| Ph | 3-MeO-Ph | S | S | H |
| Ph | 4-MeO-Ph | S | S | H |
| Ph | 2,4-$Cl_2$-Ph | S | S | H |
| Ph | 4-$PhCH_2$-Ph | S | S | H |
| Ph | 4-$CF_3$-Ph | S | S | H |
| Ph | 4-$CF_3O$-Ph | S | S | H |
| Ph | 4-MeS-Ph | S | S | H |
| Ph | 4-EtSO-Ph | S | S | H |
| Ph | 4-$MeSO_2$-Ph | S | S | H |
| Ph | 4-CHO-Ph | S | S | H |
| Ph | 4-$NO_2$-Ph | S | S | H |
| Ph | 3-CN-Ph | S | S | H |
| Ph | 4-CN-Ph | S | S | H |
| Ph | 4-$(Me)_2N$-Ph | S | S | H |
| Ph | 4-Me(MeC(O))N-Ph | S | S | H |
| Ph | 4-PhN(Me)-Ph | S. | S | H |
| Ph | $PhCH_2O$-Ph | S | S | H |
| Ph | 4-MeC(O)-Ph | S | S | H |
| Ph | 4-(4-Cl-Ph)C(O)-Ph | S | S | H |
| Ph | 4-$MeOCH_2$-Ph | S | S | H |
| Ph | 4-$EtSCH_2$-Ph | S | S | H |
| Ph | 4-$CF_3C(O)$-Ph | S | S | H |
| Ph | 4-MeC(O)O-Ph | S | S | H |
| Ph | 4-$CF_3C(O)O$-Ph | S | S | H |
| Ph | 4-PhC(O)O-Ph | S | S | H |
| Ph | 4-Ph-Ph | S | S | H |
| Ph | 3-PhO-Ph | S | S | H |
| Cl | $CH_2Ph$ | S | S | H |
| Cl | $CO_2Me$ | S | S | H |
| Br | $CO_2Me$ | S | S | H |
| Cl | $CH_2OMe$ | S | S | H |
| Cl | COMe | S | S | H |
| Cl | $CH_2OPh$ | S | S | H |
| Cl | $ClCH_2$ | S | S | H |
| Cl | 2-Naphthyl | S | S | H |
| Cl | Thiophen-2-yl | S | S | H |

| | | | | |
|---|---|---|---|---|
| Cl | Furan-2-yl | S | S | H |
| Cl | Pyridin-4-yl | S | S | H |
| Br | Pyridin-4-yl | S | S | H |
| Cl | Thiazol-2-yl | S | S | H |
| Cl | 1-Me-3-Cl-Pyrazol-5-yl | S | S | H |
| Cl | 1-Me-Imidazol-2-yl | S | S | H |
| Cl | Ph | S | S | H |
| Br | Ph | S | S | H |
| Cl | 4-Cl-Ph | S | S | H |
| Cl | 4-Me-Ph | S | S | H |
| Cl | 4-MeO-Ph | S | S | H |
| Cl | 4-$CF_3$-Ph | S | S | H |
| Cl | 4-CHO-Ph | S | S | H |
| Cl | 4-$NO_2$-Ph | S | S | H |
| Cl | 4-CN-Ph | S | S | H |
| Cl | 4-Ph-Ph | S | S | H |
| H | H | S | S | 4-Cl |
| H | H | S | S | 4-$CF_3$ |
| H | H | S | S | 4-Me |
| H | H | S | S | 4-F |
| H | Me | S | S | 4-Cl |
| H | Me | S | S | 4-$CF_3$ |
| H | Me | S | S | 5-Me |
| H | Me | S | S | 4-F |
| Me | H | S | S | 4-Cl |
| Me | H | S | S | 4-$CF_3$ |
| Me | H | S | S | 5-Me |
| Me | H | S | S | 4-F |
| H | Ph | S | S | 4-Cl |
| H | Ph | S | S | 6-F |
| H | Ph | S | S | 5-Cl |
| H | Ph | S | S | 3-F |
| H | Ph | S | S | 4,5-$Cl_2$ |
| H | Ph | S | S | 4-$CF_3$ |
| H | Ph | S | S | 4,5-$Me_2$ |
| H | Ph | S | S | 4-Me |
| H | Ph | S | S | 5-Me |
| H | Ph | S | S | 4-F |
| H | Ph | S | S | 5-F |
| H | Ph | S | S | 4-MeO |
| H | 2,6-$F_2$-Ph | S | S | 4-Cl |
| H | 2,6-$F_2$-Ph | S | S | 6-Cl |
| H | 2,6-$F_2$-Ph | S | S | 5-Cl |
| H | 2,6-$F_2$-Ph | S | S | 3-Cl |
| H | 2,6-$F_2$-Ph | S | S | 4,5-$Cl_2$ |
| H | 2,6-$F_2$-Ph | S | S | 4-$CF_3$ |
| H | 2,6-$F_2$-Ph | S | S | 4,5-$Me_2$ |
| H | 2,6-$F_2$-Ph | S | S | 4-Me |
| H | 2,6-$F_2$-Ph | S | S | 5-Me |
| H | 2,6-$F_2$-Ph | S | S | 4-F |
| H | 2,6-$F_2$-Ph | S | S | 5-F |
| H | 2,6-$F_2$-Ph | S | S | 4-MeO |
| Me | Ph | S | S | 5-Cl |
| Me | Ph | S | S | 4-$CF_3$ |
| Me | Ph | S | S | 5-Me |
| Me | Ph | S | S | 4-F |
| Me | Ph | S | S | 4-MeO |
| H | H | O | O | H |

| | | | | |
|---|---|---|---|---|
| H | Cl | 0 | 0 | H |
| H | Me | 0 | 0 | H |
| H | Et | 0 | 0 | H |
| H | n-Pr | 0 | 0 | H |
| H | i-Pr | 0 | 0 | H |
| H | n-Bu | 0 | 0 | H |
| H | i-Bu | 0 | 0 | H |
| H | s-Bu | 0 | 0 | H |
| H | t-Bu | 0 | 0 | H |
| H | n-Pen | 0 | 0 | H |
| H | 3-Me-n-Bu | 0 | 0 | H |
| H | n-Hex | 0 | 0 | H |
| H | Ethenyl | 0 | 0 | H |
| H | 1-Propenyl | 0 | 0 | H |
| H | Ethynyl | 0 | 0 | H |
| H | $CF_3$ | 0 | 0 | H |
| H | c-Pr | 0 | 0 | H |
| H | c-Hex | 0 | 0 | H |
| H | CN | 0 | 0 | H |
| H | CHO | 0 | 0 | H |
| H | $CH_2Ph$ | 0 | 0 | H |
| H | CH=CHPh | 0 | 0 | H |
| H | CH=CH-(4-Ph)Ph | 0 | 0 | H |
| H | $CH_2CH$=CHPh | 0 | 0 | H |
| H | OPh | 0 | 0 | H |
| H | $CO_2Me$ | 0 | 0 | H |
| H | $CO_2Et$ | 0 | 0 | H |
| H | $CH_2OMe$ | 0 | 0 | H |
| H | C(=NOMe)OMe | 0 | 0 | H |
| H | C(=NOMe)OSMe | 0 | 0 | H |
| H | C(=NOMe)Me | 0 | 0 | H |
| H | C(=NOMe)C(=NOMe)Me | 0 | 0 | H |
| H | C(=NOMe)Ph | 0 | 0 | H |
| H | C(=$NOCH_2Ph$)Me | 0 | 0 | H |
| H | COMe | 0 | 0 | H |
| H | $CH_2SMe$ | 0 | 0 | H |
| H | $CH_2OPh$ | 0 | 0 | H |
| H | $CH_2O$(4-Me-Ph) | 0 | 0 | H |
| H | $CH_2O$(2,4-$Cl_2$-Ph) | 0 | 0 | H |
| H | $CH_2SCH_2Ph$ | 0 | 0 | H |
| H | $CH_2NMe_2$ | 0 | 0 | H |
| H | $CH_2N$(COMe)Me | 0 | 0 | H |
| H | $CH_2ON$=$CMe_2$ | 0 | 0 | H |
| H | $CH_2N$=NCHMePh | 0 | 0 | H |
| H | $CH_2$(Morpholino) | 0 | 0 | H |
| H | $CH_2$(1-Pyrazolyl) | 0 | 0 | H |
| H | $CH_2$(Hexamethyleneimino) | 0 | 0 | H |
| H | $CH_2$(3-Ph-1-Pyrazolyl) | 0 | 0 | H |
| H | $CH_2$(1-Imidazolyl) | 0 | 0 | H |
| H | $CF_2Cl$ | 0 | 0 | H |
| H | $CCl_3$ | 0 | 0 | H |
| H | $ClCH_2$ | 0 | 0 | H |
| H | $BrCH_2$ | 0 | 0 | H |
| H | $FCH_2$ | 0 | 0 | H |
| H | $ICH_2$ | 0 | 0 | H |
| H | 1-Naphthyl | 0 | 0 | H |
| H | 2-Naphthyl | 0 | 0 | H |
| H | Thiophen-2-yl | 0 | 0 | H |

| | | | | |
|---|---|---|---|---|
| H | Thiophen-3-yl | O | O | H |
| H | 5-Cl-Thiophen-2-yl | O | O | H |
| H | 2,5-$Cl_2$-Thiophen-3-yl | O | O | H |
| H | 2,5-$Me_2$-Thiophen-3-yl | O | O | H |
| H | 5-Cl-Thiophen-2-yl | O | O | H |
| H | 3-Me-Thiophen-2-yl | O | O | H |
| H | 4,5-$Br_2$-Thiophen-3-yl | O | O | H |
| H | Furan-2-yl | O | O | H |
| H | Furan-3-yl | O | O | H |
| H | 2,5-$Me_2$-Furan-3-yl | O | O | H |
| H | 1-Me-Pyrrol-2-yl | O | O | H |
| H | 1-Me-Pyrrole-3-yl | O | O | H |
| H | Pyridin-2-yl | O | O | H |
| H | Pyridin-3-yl | O | O | H |
| H | Pyridin-4-yl | O | O | H |
| H | 6-Cl-Pyridin-2-yl | O | O | H |
| H | 5-$CF_3$-6-PhO-Pyridin-2-yl | O | O | H |
| H | Pyrazin-2-yl | O | O | H |
| H | Pyrimidin-2-yl | O | O | H |
| H | Pyrimidin-4-yl | O | O | H |
| H | 6-MeS-5-Pyrimidyl | O | O | H |
| H | 6-PhO-Pyrimidin-4-yl | O | O | H |
| H | Thiazol-2-yl | O | O | H |
| H | Thiazol-5-yl | O | O | H |
| H | 5-$CF_3$-Thiazol-2-yl | O | O | H |
| H | Pyrazol-3-yl | O | O | H |
| H | 1-Me-Pyrazol-5-yl | O | O | H |
| H | 1-Ph-Pyrazol-3-yl | O | O | H |
| H | Pyrazol-5-yl | O | O | H |
| H | 1-Me-3-Cl-Pyrazol-5-yl | O | O | H |
| H | 1-Me-Imidazol-2-yl | O | O | H |
| H | Benzothiazol-2-yl | O | O | H |
| H | Benzofuran-2-yl | O | O | H |
| H | Ph | O | O | H |
| H | 2-Cl-Ph | O | O | H |
| H | 3-Cl-Ph | O | O | H |
| H | 4-Cl-Ph | O | O | H |
| H | 2-F-Ph | O | O | H |
| H | 3-F-Ph | O | O | H |
| H | 4-F-Ph | O | O | H |
| H | 2-Me-Ph | O | O | H |
| H | 3-Me-Ph | O | O | H |
| H | 4-Me-Ph | O | O | H |
| H | 2-MeO-Ph | O | O | H |
| H | 3-MeO-Ph | O | O | H |
| H | 4-MeO-Ph | O | O | H |
| H | 4-Br-Ph | O | O | H |
| H | 2,4-$Cl_2$-Ph | O | O | H |
| H | 3,4-$Cl_2$-Ph | O | O | H |
| H | 2,4,6-$Cl_3$-Ph | O | O | H |
| H | 3,4-$(MeO)_2$-Ph | O | O | H |
| H | 2-Cl-4-Me-Ph | O | O | H |
| H | 2-MeO-4-Me-Ph | O | O | H |
| H | 2-Cl-4-i-PrO-Ph | O | O | H |
| H | 3-Cl-4-$PhCH_2O$-Ph | O | O | H |
| H | 2,4-$Me_2$-Ph | O | O | H |
| H | 2,5-$Me_2$-Ph | O | O | H |
| H | 2,6-$F_2$-Ph | O | O | H |

| | | | | |
|---|---|---|---|---|
| H | 2,5-$F_2$-Ph | O | O | H |
| H | 2,4-$F_2$-Ph | O | O | H |
| H | 2,3-$F_2$-Ph | O | O | H |
| H | 3,5-$F_2$-Ph | O | O | H |
| H | 3,4-$F_2$-Ph | O | O | H |
| H | 2,3,4,5,6-$F_5$-Ph | O | O | H |
| H | 4-Et-Ph | O | O | H |
| H | 4-i-Pr-Ph | O | O | H |
| H | 4-n-Bu-Ph | O | O | H |
| H | 4-s-Bu-Ph | O | O | H |
| H | 4-t-Bu-Ph | O | O | H |
| H | 4-(t-$BuCH_2$)-Ph | O | O | H |
| H | 4-Et(Me)$_2$C-Ph | O | O | H |
| H | 4-n-Hex-Ph | O | O | H |
| H | 4-((Me)$_2$(CN)C)-Ph | O | O | H |
| H | 4-$PhCH_2$-Ph | O | O | H |
| H | 4-(4-F-Ph)(Me)$_2$C-Ph | O | O | H |
| H | 4-(MeCH=CH)-Ph | O | O | H |
| H | 4-(MeC≡C)-Ph | O | O | H |
| H | 4-$CF_3$-Ph | O | O | H |
| H | 4-$CF_3CH_2$-Ph | O | O | H |
| H | 4-($Cl_2C$=$CHCH_2$)-Ph | O | O | H |
| H | 4-(BrC≡C)-Ph | O | O | H |
| H | 4-(2,2-$F_2$-c-$BuCH_2$)-Ph | O | O | H |
| H | 4-(1-Me-c-Pr)-Ph | O | O | H |
| H | 4-i-PrO-Ph | O | O | H |
| H | 4-t-BuO-Ph | O | O | H |
| H | 4-n-HexO-Ph | O | O | H |
| H | 4-(MeC≡C-O)-Ph | O | O | H |
| H | 4-($CH_2$=$CHCH_2O$)-Ph | O | O | H |
| H | 4-$CHF_2O$-Ph | O | O | H |
| H | 4-$CBrF_2O$-Ph | O | O | H |
| H | 4-$CF_3O$-Ph | O | O | H |
| H | 4-$CF_3CH_2O$-Ph | O | O | H |
| H | 4-($CF_2$=$CHCH_2CH_2O$)-Ph | O | O | H |
| H | 4-$CCl_3CH_2O$-Ph | O | O | H |
| H | 4-MeS-Ph | O | O | H |
| H | 4-s-BuS-Ph | O | O | H |
| H | 4-EtSO-Ph | O | O | H |
| H | 4-$MeSO_2$-Ph | O | O | H |
| H | 4-$EtSO_2$-Ph | O | O | H |
| H | 4-i-$PrSO_2$-Ph | O | O | H |
| H | 4-t-$BuSO_2$-Ph | O | O | H |
| H | 4-(MeCH=$CHCH_2S$)-Ph | O | O | H |
| H | 4-($CH_2$=$CHCH_2SO$)-Ph | O | O | H |
| H | 4-(ClCH=$CHCH_2SO_2$)-Ph | O | O | H |
| H | 4-(HC≡$CCH_2S$)-Ph | O | O | H |
| H | 4-(HC≡$CCH_2SO$)-Ph | O | O | H |
| H | 4-(HC≡$CCH_2SO_2$)-Ph | O | O | H |
| H | 4-$CHF_2S$-Ph | O | O | H |
| H | 4-$CBrF_2S$-Ph | O | O | H |
| H | 4-$CF_3S$-Ph | O | O | H |
| H | 4-$CF_3CH_2S$-Ph | O | O | H |
| H | 4-$CHF_2CF_2S$-Ph | O | O | H |
| H | 4-$CHF_2SO$-Ph | O | O | H |
| H | 4-$CBrF_2SO$-Ph | O | O | H |
| H | 4-$CF_3SO$-Ph | O | O | H |
| H | 4-$CF_3CH_2SO_2$-Ph | O | O | H |

| | | | | |
|---|---|---|---|---|
| H | 4-$CHF_2CF_2SO_2$-Ph | 0 | 0 | H |
| H | 4-$CHF_2SO_2$-Ph | 0 | 0 | H |
| H | 4-$CBrF_2SO_2$-Ph | 0 | 0 | H |
| H | 4-$CF_3SO_2$-Ph | 0 | 0 | H |
| H | 4-($Cl_2C$=$CHCH_2S$)-Ph | 0 | 0 | H |
| H | 4-($Cl_2C$=$CHCH_2SO$)-Ph | 0 | 0 | H |
| H | 4-$Cl_2C$=$CHCH_2SO_2$-Ph | 0 | 0 | H |
| H | 4-(BrC≡$CCH_2S$)-Ph | 0 | 0 | H |
| H | 4-(BrC≡$CCH_2SO$)-Ph | 0 | 0 | H |
| H | 4-(BrC≡$CCH_2SO_2$)-Ph | 0 | 0 | H |
| H | 4-CHO-Ph | 0 | 0 | H |
| H | 4-$NO_2$-Ph | 0 | 0 | H |
| H | 3-CN-Ph | 0 | 0 | H |
| H | 4-CN-Ph | 0 | 0 | H |
| H | 4-$(Me)_2N$-Ph | 0 | 0 | H |
| H | 4-Me(MeC(O))N-Ph | 0 | 0 | H |
| H | 4-PhN(Me)-Ph | 0 | 0 | H |
| H | 4-$PhCH_2$(MeCO)N-Ph | 0 | 0 | H |
| H | 4-$PhCH_2O$-Ph | 0 | 0 | H |
| H | 4-(2-Cl-Ph)$CH_2O$-Ph | 0 | 0 | H |
| H | 4-(3-Cl-Ph)$CH_2O$-Ph | 0 | 0 | H |
| H | 4-(4-Cl-Ph)$CH_2O$-Ph | 0 | 0 | H |
| H | 4-(2-Me-Ph)$CH_2O$-Ph | 0 | 0 | H |
| H | 4-(3-Me-Ph)$CH_2O$-Ph | 0 | 0 | H |
| H | 4-(4-F-Ph)$CH_2O$-Ph | 0 | 0 | H |
| H | 4-(4-Et-Ph)$CH_2O$-Ph | 0 | 0 | H |
| H | 4-(2-Cl-Ph)$CH_2S$-Ph | 0 | 0 | H |
| H | 4-(3-Cl-Ph)$CH_2S$-Ph | 0 | 0 | H |
| H | 4-(4-Cl-Ph)$CH_2SO$-Ph | 0 | 0 | H |
| H | 4-(2-Me-Ph)$CH_2S$-Ph | 0 | 0 | H |
| H | 4-(3-Me-Ph)$CH_2SO_2$-Ph | 0 | 0 | H |
| H | 4-(2,4-$F_2$-Ph)$CH_2O$-Ph | 0 | 0 | H |
| H | 3-(3,4-$Cl_2$-Ph)$CH_2O$-Ph | 0 | 0 | H |
| H | 4-(2,5-$Me_2$-Ph)$CH_2O$-Ph | 0 | 0 | H |
| H | 4-(2,3,5,6-$F_4$-Ph)$CH_2O$-Ph | 0 | 0 | H |
| H | 4-MeC(O)-Ph | 0 | 0 | H |
| H | 4-EtC(O)-Ph | 0 | 0 | H |
| H | 4-n-PrC(O)-Ph | 0 | 0 | H |
| H | 4-i-PrC(O)-Ph | 0 | 0 | H |
| H | 4-i-BuC(O)-Ph | 0 | 0 | H |
| H | 4-t-BuC(O)-Ph | 0 | 0 | H |
| H | 4-i-$BuCH_2C$(O)-Ph | 0 | 0 | H |
| H | 4-Et$(Me)_2$CC(O)-Ph | 0 | 0 | H |
| H | 4-n-HexC(O)-Ph | 0 | 0 | H |
| H | 4-PhC(O)-Ph | 0 | 0 | H |
| H | 4-(2-Cl-Ph)C(O)-Ph | 0 | 0 | H |
| H | 4-(3-Br-Ph)C(O)-Ph | 0 | 0 | H |
| H | 4-(4-Cl-Ph)C(O)-Ph | 0 | 0 | H |
| H | 4-(2-Me-Ph)C(O)-Ph | 0 | 0 | H |
| H | 4-$MeOCH_2$-Ph | 0 | 0 | H |
| H | 4-$EtOCH_2$-Ph | 0 | 0 | H |
| H | 4-i-$PrOCH_2$-Ph | 0 | 0 | H |
| H | 4-$MeSCH_2$-Ph | 0 | 0 | H |
| H | 4-$EtSCH_2$-Ph | 0 | 0 | H |
| H | 4-i-$PrSCH_2$-Ph | 0 | 0 | H |
| H | 4-$CF_3C$(O)-Ph | 0 | 0 | H |
| H | 4-$CF_3CF_2C$(O)-Ph | 0 | 0 | H |
| H | 4-MeC(O)O-Ph | 0 | 0 | H |

| | | | | |
|---|---|---|---|---|
| H | 4-EtC(0)0-Ph | 0 | 0 | H |
| H | 4-n-PrC(0)0-Ph | 0 | 0 | H |
| H | 4-i-PrC(0)0-Ph | 0 | 0 | H |
| H | 4-i-BuC(0)0-Ph | 0 | 0 | H |
| H | 4-t-BuC(0)0-Ph | 0 | 0 | H |
| H | 4-i-Bu$CH_2$C(0)0-Ph | 0 | 0 | H |
| H | 4-Et$(Me)_2$CC(0)0-Ph | 0 | 0 | H |
| H | 4-n-HexC(0)0-Ph | 0 | 0 | H |
| H | 4-$CF_3$C(0)0-Ph | 0 | 0 | H |
| H | 4-$CF_3CF_2$C(0)0-Ph | 0 | 0 | H |
| H | 4-PhC(0)0-Ph | 0 | 0 | H |
| H | 3-Ph-Ph | 0 | 0 | H |
| H | 4-Ph-Ph | 0 | 0 | H |
| H | 4-(4-Cl-Ph)-Ph | 0 | 0 | H |
| H | 4-(2,5-$Me_2$-Ph)-3-Me-Ph | 0 | 0 | H |
| H | 3-Ph0-Ph | 0 | 0 | H |
| H | 4-Ph0-Ph | 0 | 0 | H |
| H | 4-(4-Cl-Ph)0-Ph | 0 | 0 | H |
| H | 4-(4-Me-Ph)0-Ph | 0 | 0 | H |
| H | 4-(4-F-Ph)0-Ph | 0 | 0 | H |
| H | 4-(4-Me0-Ph)0-Ph | 0 | 0 | H |
| H | 4-(2,4-$Cl_2$-Ph)0-Ph | 0 | 0 | H |
| H | 4-(3,4-$Cl_2$-Ph)0-Ph | 0 | 0 | H |
| H | 4-(Pyridin-2-yl)-Ph | 0 | 0 | H |
| H | 4-(5-Cl-Pyridin-2-yl)-Ph | 0 | 0 | H |
| H | 4-(6-F-5-$CF_3$-Pyridin-2-yl)-Ph | 0 | 0 | H |
| H | 4-(Pyridin-2-yl)0-Ph | 0 | 0 | H |
| H | 4-(5-Cl-Pyridin-2-yl)0-Ph | 0 | 0 | H |
| H | 4-(3-Cl-5-F-Pyridin-2-yl)0-Ph | 0 | 0 | H |
| H | 4-(5-Cl-Thiophen-2-yl)0-Ph | 0 | 0 | H |
| -CH=CH-CH=CH- | | 0 | 0 | H |
| -C(Cl)=CH-CH=CH- | | 0 | 0 | H |
| -CH=C(Cl)-CH=CH- | | 0 | 0 | H |
| -CH=CH-C(Cl)=CH- | | 0 | 0 | H |
| -CH=CH-CH=C(Cl)- | | 0 | 0 | H |
| -CH=CH-C(OMe)=CH- | | 0 | 0 | H |
| -CH=CH-CF=CH- | | 0 | 0 | H |
| -CH=CH-C(Me)=CH- | | 0 | 0 | H |
| -CH=CH-CH=N- | | 0 | 0 | H |
| -CH=CH-N=CH- | | 0 | 0 | H |
| -CH=N-CH=CH- | | 0 | 0 | H |
| -N=CH-CH=CH- | | 0 | 0 | H |
| $-(CH_2)_3-$ | | 0 | 0 | H |
| $-(CH_2)_4-$ | | 0 | 0 | H |
| $-(CH_2)_5-$ | | 0 | 0 | H |
| $-CH_2-0-CH_2-$ | | 0 | 0 | H |
| $-CH_2-CH_2-0-CH_2-$ | | 0 | 0 | H |
| $-CO-(CH_2)_3-$ | | 0 | 0 | H |
| $-CH_2-CH(CH_2Ph)CH_2-$ | | 0 | 0 | H |
| $-CH_2-CH_2-CH(Me)-CH_2-$ | | 0 | 0 | H |
| $-CH_2-CH_2-CH(Ph)-CH_2-$ | | 0 | 0 | H |
| Me | Me | 0 | 0 | H |
| Me | Et | 0 | 0 | H |
| Me | n-Pr | 0 | 0 | H |
| Me | i-Pr | 0 | 0 | H |
| Me | n-Bu | 0 | 0 | H |
| Me | n-Hex | 0 | 0 | H |
| Me | Ethenyl | 0 | 0 | H |

| | | | | |
|---|---|---|---|---|
| Me | 1-Propynyl | O | O | H |
| Me | $CF_3$ | O | O | H |
| Me | c-Pr | O | O | H |
| Me | CN | O | O | H |
| Me | CHO | O | O | H |
| Me | $CH_2Ph$ | O | O | H |
| Me | $CO_2Me$ | O | O | H |
| Me | $CH_2OMe$ | O | O | H |
| Me | COMe | O | O | H |
| Me | $CH_2SMe$ | O | O | H |
| Me | $CH_2OPh$ | O | O | H |
| Me | $CH_2O$(4-Me-Ph) | O | O | H |
| Me | $CH_2O$(2,4-$Cl_2$-Ph) | O | O | H |
| Me | $CH_2SCH_2Ph$ | O | O | H |
| Me | $CF_2Cl$ | O | O | H |
| Me | 1-Naphthyl | O | O | H |
| Me | 2-Naphthyl | O | O | H |
| Me | Thiophen-2-yl | O | O | H |
| Me | Furan-2-yl | O | O | H |
| Me | 1-Me-Pyrrol-2-yl | O | O | H |
| Me | Pyridin-4-yl | O | O | H |
| Me | 6-Cl-Pyridin-2-yl | O | O | H |
| Me | Pyrazin-2-yl | O | O | H |
| Me | Pyrimidin-2-yl | O | O | H |
| Me | Thiazol-2-yl | O | O | H |
| Me | Thiazol-5-yl | O | O | H |
| Me | 5-$CF_3$-Thiazol-2-yl | O | O | H |
| Me | 1-Me-3-Cl-Pyrazol-5-yl | O | O | H |
| Me | 1-Me-Imidazol-2-yl | O | O | H |
| Me | Ph | O | O | H |
| Me | 2-Cl-Ph | O | O | H |
| Me | 3-Cl-Ph | O | O | H |
| Me | 4-F-Ph | O | O | H |
| Me | 2-Me-Ph | O | O | H |
| Me | 3-Me-Ph | O | O | H |
| Me | 4-Me-Ph | O | O | H |
| Me | 2-MeO-Ph | O | O | H |
| Me | 3-MeO-Ph | O | O | H |
| Me | 4-MeO-Ph | O | O | H |
| Me | 2,4-$Cl_2$-Ph | O | O | H |
| Me | 2-Cl-4-Me-Ph | O | O | H |
| Me | 2,5-$Me_2$-Ph | O | O | H |
| Me | 2,6-$F_2$-Ph | O | O | H |
| Me | 4-Et-Ph | O | O | H |
| Me | 4-$PhCH_2$-Ph | O | O | H |
| Me | 4-$CF_3$-Ph | O | O | H |
| Me | 4-MeS-Ph | O | O | H |
| Me | 4-EtSO-Ph | O | O | H |
| Me | 4-$MeSO_2$-Ph | O | O | H |
| Me | 4-$EtSO_2$-Ph | O | O | H |
| Me | 4-CHO-Ph | O | O | H |
| Me | 4-$NO_2$-Ph | O | O | H |
| Me | 3-CN-Ph | O | O | H |
| Me | 4-CN-Ph | O | O | H |
| Me | 4-$PhCH_2$(MeCO)N-Ph | O | O | H |
| Me | 4-(2,4-$F_2$-Ph)$CH_2O$-Ph | O | O | H |
| Me | 4-MeC(O)-Ph | O | O | H |
| Me | 4-(4-Cl-Ph)C(O)-Ph | O | O | H |

| | | | | |
|---|---|---|---|---|
| Me | 4-MeOCH₂-Ph | 0 | 0 | H |
| Me | 4-EtOCH₂-Ph | 0 | 0 | H |
| Me | 4-MeSCH₂-Ph | 0 | 0 | H |
| Me | 4-EtSCH₂-Ph | 0 | 0 | H |
| Me | 4-CF₃C(O)-Ph | 0 | 0 | H |
| Me | 4-MeC(O)O-Ph | 0 | 0 | H |
| Me | 4-t-BuC(O)O-Ph | 0 | 0 | H |
| Me | 4-CF₃C(O)O-Ph | 0 | 0 | H |
| Me | 4-PhC(O)O-Ph | 0 | 0 | H |
| Me | 4-Ph-Ph | 0 | 0 | H |
| Me | 4-(4-Cl-Ph)-Ph | 0 | 0 | H |
| Me | 4-(4-MeO-Ph)O-Ph | 0 | 0 | H |
| Me | 4-(2,4-Cl₂-Ph)O-Ph | 0 | 0 | H |
| Me | 4-(Pyridin-2-yl)O-Ph | 0 | 0 | H |
| Me | 4-(5-Cl-Pyridin-2-yl)O-Ph | 0 | 0 | H |
| Et | Et | 0 | 0 | H |
| Et | c-Pr | 0 | 0 | H |
| Et | CH₂Ph | 0 | 0 | H |
| Et | CO₂Me | 0 | 0 | H |
| Et | CH₂OMe | 0 | 0 | H |
| Et | COMe | 0 | 0 | H |
| Et | CH₂SMe | 0 | 0 | H |
| Et | CH₂OPh | 0 | 0 | H |
| Et | CH₂O(4-Me-Ph) | 0 | 0 | H |
| Et | CH₂SCH₂Ph | 0 | 0 | H |
| Et | 2-Naphthyl | 0 | 0 | H |
| Et | Thiophen-2-yl | 0 | 0 | H |
| Et | Furan-3-yl | 0 | 0 | H |
| Et | 1-Me-Pyrrole-3-yl | 0 | 0 | H |
| Et | Pyridin-2-yl | 0 | 0 | H |
| Et | Pyrazin-2-yl | 0 | 0 | H |
| Et | Pyrimidin-2-yl | 0 | 0 | H |
| Et | Thiazol-5-yl | 0 | 0 | H |
| Et | 1-Me-3-Cl-Pyrazol-5-yl | 0 | 0 | H |
| Et | 1-Me-Imidazol-2-yl | 0 | 0 | H |
| Et | Ph | 0 | 0 | H |
| Et | 4-Cl-Ph | 0 | 0 | H |
| Et | 2-F-Ph | 0 | 0 | H |
| Et | 3-F-Ph | 0 | 0 | H |
| Et | 4-F-Ph | 0 | 0 | H |
| Et | 2-Me-Ph | 0 | 0 | H |
| Et | 3-Me-Ph | 0 | 0 | H |
| Et | 4-Me-Ph | 0 | 0 | H |
| Et | 2-MeO-Ph | 0 | 0 | H |
| Et | 3-MeO-Ph | 0 | 0 | H |
| Et | 4-MeO-Ph | 0 | 0 | H |
| Et | 4-Br-Ph | 0 | 0 | H |
| Et | 2,4-Cl₂-Ph | 0 | 0 | H |
| Et | 3,4-Cl₂-Ph | 0 | 0 | H |
| Et | 4-n-Hex-Ph | 0 | 0 | H |
| Et | 4-PhCH₂-Ph | 0 | 0 | H |
| Et | 4-CF₃-Ph | 0 | 0 | H |
| Et | 4-CF₃O-Ph | 0 | 0 | H |
| Et | 4-MeS-Ph | 0 | 0 | H |
| Et | 4-MeSO₂-Ph | 0 | 0 | H |
| Et | 4-CHO-Ph | 0 | 0 | H |
| Et | 4-NO₂-Ph | 0 | 0 | H |
| Et | 3-CN-Ph | 0 | 0 | H |

| | | | | |
|---|---|---|---|---|
| Et | 4-CN-Ph | O | O | H |
| Et | 4-(Me)$_2$N-Ph | O | O | H |
| Et | 4-PhCH$_2$O-Ph | O | O | H |
| Et | 4-MeC(O)-Ph | O | O | H |
| Et | 4-(4-Cl-Ph)C(O)-Ph | O | O | H |
| Et | 4-MeOCH$_2$-Ph | O | O | H |
| Et | 4-EtSCH$_2$-Ph | O | O | H |
| Et | 4-CF$_3$C(O)-Ph | O | O | H |
| Et | 4-MeC(O)O-Ph | O | O | H |
| Et | 4-CF$_3$C(O)O-Ph | O | O | H |
| Et | 4-PhC(O)O-Ph | O | O | H |
| Et | 4-Ph-Ph | O | O | H |
| Et | 4-(4-Me-Ph)O-Ph | O | O | H |
| Et | 4-(Pyridin-2-yl)O-Ph | O | O | H |
| Ph | CO$_2$Me | O | O | H |
| Ph | COMe | O | O | H |
| Ph | 2-Naphthyl | O | O | H |
| Ph | Thiophen-2-yl | O | O | H |
| Ph | Furan-3-yl | O | O | H |
| Ph | 1-Me-Pyrrol-2-yl | O | O | H |
| Ph | Pyridin-4-yl | O | O | H |
| Ph | Pyrazin-2-yl | O | O | H |
| Ph | Pyrimidin-4-yl | O | O | H |
| Ph | Thiazol-2-yl | O | O | H |
| Ph | 1-Me-3-Cl-Pyrazol-5-yl | O | O | H |
| Ph | 1-Me-Imidazol-2-yl | O | O | H |
| Ph | Ph | O | O | H |
| Ph | 4-Cl-Ph | O | O | H |
| Ph | 2-F-Ph | O | O | H |
| Ph | 3-F-Ph | O | O | H |
| Ph | 4-F-Ph | O | O | H |
| Ph | 2-Me-Ph | O | O | H |
| Ph | 3-Me-Ph | O | O | H |
| Ph | 4-Me-Ph | O | O | H |
| Ph | 2-MeO-Ph | O | O | H |
| Ph | 3-MeO-Ph | O | O | H |
| Ph | 4-MeO-Ph | O | O | H |
| Ph | 2,4-Cl$_2$-Ph | O | O | H |
| Ph | 4-PhCH$_2$-Ph | O | O | H |
| Ph | 4-CF$_3$-Ph | O | O | H |
| Ph | 4-CF$_3$O-Ph | O | O | H |
| Ph | 4-MeS-Ph | O | O | H |
| Ph | 4-EtSO-Ph | O | O | H |
| Ph | 4-MeSO$_2$-Ph | O | O | H |
| Ph | 4-CHO-Ph | O | O | H |
| Ph | 4-NO$_2$-Ph | O | O | H |
| Ph | 3-CN-Ph | O | O | H |
| Ph | 4-CN-Ph | O | O | H |
| Ph | 4-(Me)$_2$N-Ph | O | O | H |
| Ph | 4-Me(MeC(O))N-Ph | O | O | H |
| Ph | 4-PhN(Me)-Ph | O | O | H |
| Ph | PhCH$_2$O-Ph | O | O | H |
| Ph | 4-MeC(O)-Ph | O | O | H |
| Ph | 4-(4-Cl-Ph)C(O)-Ph | O | O | H |
| Ph | 4-MeOCH$_2$-Ph | O | O | H |
| Ph | 4-EtSCH$_2$-Ph | O | O | H |
| Ph | 4-CF$_3$C(O)-Ph | O | O | H |
| Ph | 4-MeC(O)O-Ph | O | O | H |

| | | | | |
|---|---|---|---|---|
| Ph | 4-$CF_3C(O)O$-Ph | O | O | H |
| Ph | 4-PhC(O)O-Ph | O | O | H |
| Ph | 4-Ph-Ph | O | O | H |
| Ph | 3-PhO-Ph | O | O | H |
| Cl | $CH_2Ph$ | O | O | H |
| Cl | $CO_2Me$ | O | O | H |
| Br | $CO_2Me$ | O | O | H |
| Cl | $CH_2OMe$ | O | O | H |
| Cl | COMe | O | O | H |
| Cl | $CH_2OPh$ | O | O | H |
| Cl | $ClCH_2$ | O | O | H |
| Cl | 2-Naphthyl | O | O | H |
| Cl | Thiophen-2-yl | O | O | H |
| Cl | Furan-2-yl | O | O | H |
| Cl | Pyridin-4-yl | O | O | H |
| Br | Pyridin-4-yl | O | O | H |
| Cl | Thiazol-2-yl | O | O | H |
| Cl | 1-Me-3-Cl-Pyrazol-5-yl | O | O | H |
| Cl | 1-Me-Imidazol-2-yl | O | O | H |
| Cl | Ph | O | O | H |
| Br | Ph | O | O | H |
| Cl | 4-Cl-Ph | O | O | H |
| Cl | 4-Me-Ph | O | O | H |
| Cl | 4-MeO-Ph | O | O | H |
| Cl | 4-$CF_3$-Ph | O | O | H |
| Cl | 4-CHO-Ph | O | O | H |
| Cl | 4-$NO_2$-Ph | O | O | H |
| Cl | 4-CN-Ph | O | O | H |
| Cl | 4-Ph-Ph | O | O | H |
| H | H | O | O | 4-Cl |
| H | H | O | O | 4-$CF_3$ |
| H | H | O | O | 4-Me |
| H | H | O | O | 4-F |
| H | Me | O | O | 4-Cl |
| H | Me | O | O | 4-$CF_3$ |
| H | Me | O | O | 5-Me |
| H | Me | O | O | 4-F |
| Me | H | O | O | 4-Cl |
| Me | H | O | O | 4-$CF_3$ |
| Me | H | O | O | 5-Me |
| Me | H | O | O | 4-F |
| H | Ph | O | O | 4-Cl |
| H | Ph | O | O | 6-F |
| H | Ph | O | O | 5-Cl |
| H | Ph | O | O | 3-F |
| H | Ph | O | O | 4,5-$Cl_2$ |
| H | Ph | O | O | 4-$CF_3$ |
| H | Ph | O | O | 4,5-$Me_2$ |
| H | Ph | O | O | 4-Me |
| H | Ph | O | O | 5-Me |
| H | Ph | O | O | 4-F |
| H | Ph | O | O | 5-F |
| H | Ph | O | O | 4-MeO |
| H | 2,6-$F_2$-Ph | O | O | 4-Cl |
| H | 2,6-$F_2$-Ph | O | O | 6-Cl |
| H | 2,6-$F_2$-Ph | O | O | 5-Cl |
| H | 2,6-$F_2$-Ph | O | O | 3-Cl |
| H | 2,6-$F_2$-Ph | O | O | 4,5-$Cl_2$ |

| | | | | |
|---|---|---|---|---|
| H | 2,6-$F_2$-Ph | O | O | 4-$CF_3$ |
| H | 2,6-$F_2$-Ph | O | O | 4,5-$Me_2$ |
| H | 2,6-$F_2$-Ph | O | O | 4-Me |
| H | 2,6-$F_2$-Ph | O | O | 5-Me |
| H | 2,6-$F_2$-Ph | O | O | 4-F |
| H | 2,6-$F_2$-Ph | O | O | 5-F |
| H | 2,6-$F_2$-Ph | O | O | 4-MeO |
| Me | Ph | O | O | 5-Cl |
| Me | Ph | O | O | 4-$CF_3$ |
| Me | Ph | O | O | 5-Me |
| Me | Ph | O | O | 4-F |
| Me | Ph | O | O | 4-MeO |
| H | H | S | S | H |
| H | H | O | S | H |
| H | H | S | O | H |
| H | Me | N-Me | S | H |
| H | Me | N-Et | S | H |
| H | Me | N-n-Pr | S | H |
| H | Me | N-i-Pr | S | H |
| H | Me | N-1-Propenyl | S | H |
| H | Me | N-c-Pr | S | H |
| H | Me | N-OMe | S | H |
| H | Me | N-$SO_2$Me | S | H |
| H | Me | N-$NMe_2$ | S | H |
| H | Me | N-$CH_2$Ph | S | H |
| H | Me | N-$CO_2$Me | S | H |
| H | Me | N-$CO_2$Et | S | H |
| H | Me | N-COMe | S | H |
| H | Me | N-$COCF_3$ | S | H |
| H | Me | N-$CH_2$SMe | S | H |
| H | Me | N-$CH_2$OPh | S | H |
| H | Me | N-$CH_2$-Thiophen-2-yl | S | H |
| H | Me | N-$CH_2$-Furan-2-yl | S | H |
| H | Me | N-$CH_2$-Pyridin-2-yl | S | H |
| H | Me | N-$CH_2$-Pyrazin-2-yl | S | H |
| H | Me | N-$CH_2$-Pyrimidin-4-yl | S | H |
| H | Me | N-$CH_2$-Thiazol-2-yl | S | H |
| H | Me | N-$CH_2$-1-Me-Pyrazol-5-yl | S | H |
| H | Me | N-Ph | S | H |
| H | Me | N-Me | N-Me | H |
| H | Me | N-Et | N-Me | H |
| H | Me | N-n-Pr | N-Me | H |
| H | Me | N-i-Pr | N-Me | H |
| H | Me | N-1-Propenyl | N-Me | H |
| H | Me | N-c-Pr | N-Me | H |
| H | Me | N-OMe | N-Me | H |
| H | Me | N-$SO_2$Me | N-Me | H |
| H | Me | N-$NMe_2$ | N-Me | H |
| H | Me | N-$CH_2$Ph | N-Me | H |
| H | Me | N-$CO_2$Me | N-Me | H |
| H | Me | N-$CO_2$Et | N-Me | H |
| H | Me | N-COMe | N-Me | H |
| H | Me | N-$COCF_3$ | N-Me | H |
| H | Me | N-$CH_2$SMe | N-Me | H |
| H | Me | N-$CH_2$OPh | N-Me | H |
| H | Me | N-$CH_2$-Thiophen-2-yl | N-Me | H |
| H | Me | N-$CH_2$-Furan-2-yl | N-Me | H |
| H | Me | N-$CH_2$-Pyridin-2-yl | N-Me | H |

| | | | | |
|---|---|---|---|---|
| H | Me | N-$CH_2$-Pyrazin-2-yl | N-Me | H |
| H | Me | N-$CH_2$-Pyrimidin-4-yl | N-Me | H |
| H | Me | N-$CH_2$-Thiazol-2-yl | N-Me | H |
| H | Me | N-$CH_2$-1-Me-Pyrazol-5-yl | N-Me | H |
| H | Me | N-Ph | N-Me | H |
| H | Me | N-Me | O | H |
| H | Me | N-Et | O | H |
| H | Me | N-n-Pr | O | H |
| H | Me | N-i-Pr | O | H |
| H | Me | N-1-Propenyl | O | H |
| H | Me | N-c-Pr | O | H |
| H | Me | N-OMe | O | H |
| H | Me | N-$SO_2$Me | O | H |
| H | Me | N-$NMe_2$ | O | H |
| H | Me | N-$CH_2$Ph | O | H |
| H | Me | N-$CO_2$Me | O | H |
| H | Me | N-$CO_2$Et | O | H |
| H | Me | N-COMe | O | H |
| H | Me | N-$COCF_3$ | O | H |
| H | Me | N-$CH_2$SMe | O | H |
| H | Me | N-$CH_2$OPh | O | H |
| H | Me | N-$CH_2$-Thiophen-2-yl | O | H |
| H | Me | N-$CH_2$-Furan-2-yl | O | H |
| H | Me | N-$CH_2$-Pyridin-2-yl | O | H |
| H | Me | N-$CH_2$-Pyrazin-2-yl | O | H |
| H | Me | N-$CH_2$-Pyrimidin-4-yl | O | H |
| H | Me | N-$CH_2$-Thiazol-2-yl | O | H |
| H | Me | N-$CH_2$-1-Me-Pyrazol-5-yl | O | H |
| H | Me | N-Ph | O | H |
| Me | Me | N-Me | S | H |
| Me | Me | N-Et | S | H |
| Me | Me | N-n-Pr | S | H |
| Me | Me | N-i-Pr | S | H |
| Me | Me | N-1-Propenyl | S | H |
| Me | Me | N-c-Pr | S | H |
| Me | Me | N-OMe | S | H |
| Me | Me | N-$SO_2$Me | S | H |
| Me | Me | N-$NMe_2$ | S | H |
| Me | Me | N-$CH_2$Ph | S | H |
| Me | Me | N-$CO_2$Me | S | H |
| Me | Me | N-$CO_2$Et | S | H |
| Me | Me | N-COMe | S | H |
| Me | Me | N-$COCF_3$ | S | H |
| Me | Me | N-$CH_2$SMe | S | H |
| Me | Me | N-$CH_2$OPh | S | H |
| Me | Me | N-$CH_2$-Thiophen-2-yl | S | H |
| Me | Me | N-$CH_2$-Furan-2-yl | S | H |
| Me | Me | N-$CH_2$-Pyridin-2-yl | S | H |
| Me | Me | N-$CH_2$-Pyrazin-2-yl | S | H |
| Me | Me | N-$CH_2$-Pyrimidin-4-yl | S | H |
| Me | Me | N-$CH_2$-Thiazol-2-yl | S | H |
| Me | Me | N-$CH_2$-1-Me-Pyrazol-5-yl | S | H |
| Me | Me | N-Ph | S | H |
| Me | Me | N-Me | N-Me | H |
| Me | Me | N-Et | N-Me | H |
| Me | Me | N-n-Pr | N-Me | H |
| Me | Me | N-i-Pr | N-Me | H |
| Me | Me | N-1-Propenyl | N-Me | H |

| | | | | |
|---|---|---|---|---|
| Me | Me | N-c-Pr | N-Me | H |
| Me | Me | N-OMe | N-Me | H |
| Me | Me | N-$SO_2$Me | N-Me | H |
| Me | Me | N-$NMe_2$ | N-Me | H |
| Me | Me | N-$CH_2$Ph | N-Me | H |
| Me | Me | N-$CO_2$Me | N-Me | H |
| Me | Me | N-$CO_2$Et | N-Me | H |
| Me | Me | N-COMe | N-Me | H |
| Me | Me | N-$COCF_3$ | N-Me | H |
| Me | Me | N-$CH_2$SMe | N-Me | H |
| Me | Me | N-$CH_2$OPh | N-Me | H |
| Me | Me | N-$CH_2$-Thiophen-2-yl | N-Me | H |
| Me | Me | N-$CH_2$-Furan-2-yl | N-Me | H |
| Me | Me | N-$CH_2$-Pyridin-2-yl | N-Me | H |
| Me | Me | N-$CH_2$-Pyrazin-2-yl | N-Me | H |
| Me | Me | N-$CH_2$-Pyrimidin-4-yl | N-Me | H |
| Me | Me | N-$CH_2$-Thiazol-2-yl | N-Me | H |
| Me | Me | N-$CH_2$-1-Me-Pyrazol-5-yl | N-Me | H |
| Me | Me | N-Ph | N-Me | H |
| Me | Me | N-Me | O | H |
| Me | Me | N-Et | O | H |
| Me | Me | N-n-Pr | O | H |
| Me | Me | N-i-Pr | O | H |
| Me | Me | N-1-Propenyl | O | H |
| Me | Me | N-c-Pr | O | H |
| Me | Me | N-OMe | O | H |
| Me | Me | N-$SO_2$Me | O | H |
| Me | Me | N-$NMe_2$ | O | H |
| Me | Me | N-$CH_2$Ph | O | H |
| Me | Me | N-$CO_2$Me | O | H |
| Me | Me | N-$CO_2$Et | O | H |
| Me | Me | N-COMe | O | H |
| Me | Me | N-$COCF_3$ | O | H |
| Me | Me | N-$CH_2$SMe | O | H |
| Me | Me | N-$CH_2$OPh | O | H |
| Me | Me | N-$CH_2$-Thiophen-2-yl | O | H |
| Me | Me | N-$CH_2$-Furan-2-yl | O | H |
| Me | Me | N-$CH_2$-Pyridin-2-yl | O | H |
| Me | Me | N-$CH_2$-Pyrazin-2-yl | O | H |
| Me | Me | N-$CH_2$-Pyrimidin-4-yl | O | H |
| Me | Me | N-$CH_2$-Thiazol-2-yl | O | H |
| Me | Me | N-$CH_2$-1-Me-Pyrazol-5-yl | O | H |
| Me | Me | N-Ph | O | H |
| H | Cl | N-Me | S | H |
| H | Et | N-Me | S | H |
| H | n-Pr | N-Me | S | H |
| H | i-Pr | N-Me | S | H |
| H | t-Bu | N-Me | S | H |
| H | 1-Propenyl | N-Me | S | H |
| H | $CF_3$ | N-Me | S | H |
| H | $CO_2$Me | N-Me | S | H |
| H | COMe | N-Me | S | H |
| H | $CH_2$SMe | N-Me | S | H |
| H | $CH_2$OPh | N-Me | S | H |
| H | $CH_2SCH_2$Ph | N-Me | S | H |
| H | $CH_2NMe_2$ | N-Me | S | H |
| H | Ph | N-Me | S | H |
| H | 4-Cl-Ph | N-Me | S | H |

| | | | | |
|---|---|---|---|---|
| H | 4-Me-Ph | N-Me | S | H |
| H | Me | N-Et | S | H |
| H | Ph | N-Et | S | H |
| H | Me | N-OMe | S | H |
| H | Ph | N-OMe | S | H |
| H | Me | $N-CH_2Ph$ | S | H |
| H | Ph | $N-CH_2Ph$ | S | H |
| H | Me | $N-CO_2Me$ | S | H |
| H | Ph | $N-CO_2Me$ | S | H |
| H | Me | N-Ph | S | H |
| H | Ph | N-Ph | S | H |
| Cl | H | N-Me | S | H |
| Me | Ph | N-Me | S | H |
| Et | H | N-Me | S | H |
| n-Pr | H | N-Me | S | H |
| i-Pr | H | N-Me | S | H |
| t-Bu | H | N-Me | S | H |
| 1-Propenyl | H | N-Me | S | H |
| $CF_3$ | H | N-Me | S | H |
| $CO_2Me$ | H | N-Me | S | H |
| COMe | H | N-Me | S | H |
| $CH_2SMe$ | H | N-Me | S | H |
| $CH_2OPh$ | H | N-Me | S | H |
| $CH_2SCH_2Ph$ | H | N-Me | S | H |
| $CH_2NMe_2$ | H | N-Me | S | H |
| Ph | H | N-Me | S | H |
| Ph | Me | N-Me | S | H |
| Ph | Ph | N-Me | S | H |
| 4-Cl-Ph | H | N-Me | S | H |
| 4-Me-Ph | H | N-Me | S | H |
| Me | H | N-Et | S | H |
| Ph | H | N-Et | S | H |
| Me | H | N-OMe | S | H |
| Ph | H | N-OMe | S | H |
| Me | H | $N-CH_2Ph$ | S | H |
| Ph | H | $N-CH_2Ph$ | S | H |
| Me | H | $N-CO_2Me$ | S | H |
| Ph | H | $N-CO_2Me$ | S | H |
| Me | H | N-Ph | S | H |
| Ph | H | N-Ph | S | H |
| H | Cl | N-Me | O | H |
| H | Et | N-Me | O | H |
| H | n-Pr | N-Me | O | H |
| H | i-Pr | N-Me | O | H |
| H | t-Bu | N-Me | O | H |
| H | 1-Propenyl | N-Me | O | H |
| H | $CF_3$ | N-Me | O | H |
| H | $CO_2Me$ | N-Me | O | H |
| H | COMe | N-Me | O | H |
| H | $CH_2SMe$ | N-Me | O | H |
| H | $CH_2OPh$ | N-Me | O | H |
| H | $CH_2SCH_2Ph$ | N-Me | O | H |
| H | $CH_2NMe_2$ | N-Me | O | H |
| H | Ph | N-Me | O | H |
| H | 4-Cl-Ph | N-Me | O | H |
| H | 4-Me-Ph | N-Me | O | H |
| H | Me | N-Et | O | H |
| H | Ph | N-Et | O | H |

| | | | | |
|---|---|---|---|---|
| H | Me | N-OMe | O | H |
| H | Ph | N-OMe | O | H |
| H | Me | $N-CH_2Ph$ | O | H |
| H | Ph | $N-CH_2Ph$ | O | H |
| H | Me | $N-CO_2Me$ | O | H |
| H | Ph | $N-CO_2Me$ | O | H |
| H | Me | N-Ph | O | H |
| H | Ph | N-Ph | O | H |
| Cl | H | N-Me | O | H |
| Me | Ph | N-Me | O | H |
| Et | H | N-Me | O | H |
| n-Pr | H | N-Me | O | H |
| i-Pr | H | N-Me | O | H |
| t-Bu | H | N-Me | O | H |
| 1-Propenyl | H | N-Me | O | H |
| $CF_3$ | H | N-Me | O | H |
| $CO_2Me$ | H | N-Me | O | H |
| COMe | H | N-Me | O | H |
| $CH_2SMe$ | H | N-Me | O | H |
| $CH_2OPh$ | H | N-Me | O | H |
| $CH_2SCH_2Ph$ | H | N-Me | O | H |
| $CH_2NMe_2$ | H | N-Me | O | H |
| Ph | H | N-Me | O | H |
| Ph | Me | N-Me | O | H |
| Ph | Ph | N-Me | O | H |
| 4-Cl-Ph | H | N-Me | O | H |
| 4-Me-Ph | H | N-Me | O | H |
| Me | H | N-Et | O | H |
| Ph | H | N-Et | O | H |
| Me | H | N-OMe | O | H |
| Ph | H | N-OMe | O | H |
| Me | H | $N-CH_2Ph$ | O | H |
| Ph | H | $N-CH_2Ph$ | O | H |
| Me | H | $N-CO_2Me$ | O | H |
| Ph | H | $N-CO_2Me$ | O | H |
| Me | H | N-Ph | O | H |
| Ph | H | N-Ph | O | H |
| H | Et | N-Me | N-Me | H |
| H | n-Pr | N-Me | N-Me | H |
| H | i-Pr | N-Me | N-Me | H |
| H | t-Bu | N-Me | N-Me | H |
| H | 1-Propenyl | N-Me | N-Me | H |
| H | $CF_3$ | N-Me | N-Me | H |
| H | $CO_2Me$ | N-Me | N-Me | H |
| H | COMe | N-Me | N-Me | H |
| H | $CH_2SMe$ | N-Me | N-Me | H |
| H | $CH_2OPh$ | N-Me | N-Me | H |
| H | $CH_2SCH_2Ph$ | N-Me | N-Me | H |
| H | $CH_2NMe_2$ | N-Me | N-Me | H |
| H | Ph | N-Me | N-Me | H |
| H | 4-Cl-Ph | N-Me | N-Me | H |
| H | 4-Me-Ph | N-Me | N-Me | H |
| H | Me | N-Et | N-Me | H |
| H | Ph | N-Et | N-Me | H |
| H | Me | N-OMe | N-Me | H |
| H | Ph | N-OMe | N-Me | H |
| H | Me | $N-CH_2Ph$ | N-Me | H |
| H | Ph | $N-CH_2Ph$ | N-Me | H |

| | | | | |
|---|---|---|---|---|
| H | Me | N-$CO_2$Me | N-Me | H |
| H | Ph | N-$CO_2$Me | N-Me | H |
| H | Me | N-Ph | N-Me | H |
| H | Ph | N-Ph | N-Me | H |
| Ph | Me | N-Me | N-Me | H |
| Ph | Ph | N-Me | N-Me | H |
| H | Et | N-Me | N-Ph | H |
| H | n-Pr | N-Me | N-Ph | H |
| H | i-Pr | N-Me | N-Ph | H |
| H | t-Bu | N-Me | N-Ph | H |
| H | 1-Propenyl | N-Me | N-Ph | H |
| H | $CF_3$ | N-Me | N-Ph | H |
| H | $CO_2$Me | N-Me | N-Ph | H |
| H | COMe | N-Me | N-Ph | H |
| H | $CH_2$SMe | N-Me | N-Ph | H |
| H | $CH_2$OPh | N-Me | N-Ph | H |
| H | $CH_2SCH_2$Ph | N-Me | N-Ph | H |
| H | $CH_2NMe_2$ | N-Me | N-Ph | H |
| H | Ph | N-Me | N-Ph | H |
| H | 4-Cl-Ph | N-Me | N-Ph | H |
| H | 4-Me-Ph | N-Me | N-Ph | H |
| H | Me | N-Et | N-Ph | H |
| H | Ph | N-Et | N-Ph | H |
| H | Me | N-OMe | N-Ph | H |
| H | Ph | N-OMe | N-Ph | H |
| H | Me | N-$CH_2$Ph | N-Ph | H |
| H | Ph | N-$CH_2$Ph | N-Ph | H |
| H | Me | N-$CO_2$Me | N-Ph | H |
| H | Ph | N-$CO_2$Me | N-Ph | H |
| H | Me | N-Ph | N-Ph | H |
| H | Ph | N-Ph | N-Ph | H |
| Ph | Me | N-Me | N-Ph | H |
| Ph | Ph | N-Me | N-Ph | H |

(Table 5)

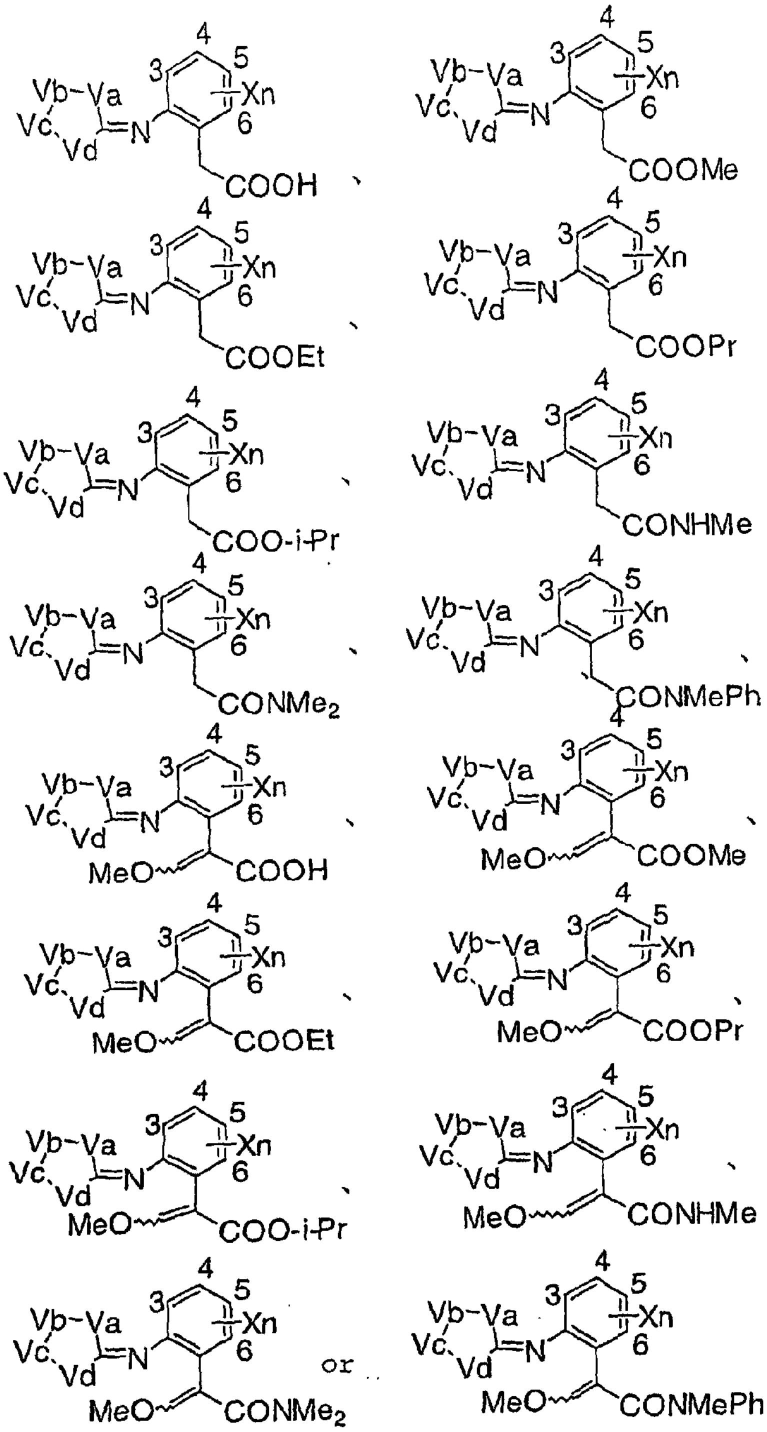

or

| -Va-Vb-Vc-Vd- | X |
|---|---|
| $-S-CH_2-C(OH)(C_2F_5)-N(Me)-$ | H |
| $-S-CH(CH_2Br)-CH_2-N(Me)-$ | H |
| $-S-S-CH=N-$ | H |

| | |
|---|---|
| -S-S-C(Ph)=N- | H |
| -S-S-C($CF_3$)=N- | H |
| -S-N(Me)-$CH_2$-S- | H |
| -S-N(Ph)-$CH_2$-S- | H |
| -S-N(Me)-CH(Ph)-S- | H |
| -N=N-C(=O)-O- | H |
| -N=N-CH(Me)-O- | H |
| -N=N-CH(Ph)-O- | H |
| -S-O-CH=N- | H |
| -S-O-C(Me)=N- | H |
| -S-O-C(Ph)=N- | H |
| -CH=CH-N(Me)-S- | H |
| -CH=CH-N(Ph)-S- | H |
| -C(Me)=CH-N(Ph)-S- | H |
| -CH=C(Me)-N(Ph)-S- | H |
| -C(Me)=C(Me)-N(Ph)-S- | H |
| -CH=CH-N(Me)-O- | H |
| -CH=CH-N(Ph)-O- | H |
| -C(Me)=CH-N(Ph)-O- | H |
| -CH=C(Me)-N(Ph)-O- | H |
| -C(Me)=C(Me)-N(Ph)-O- | H |
| -$CH_2$-$CH_2$-N(Me)-O- | H |
| -$CH_2$-$CH_2$-N(Ph)-O- | H |
| -$CH_2$-C(=O)-N(Ph)-O- | H |
| -CH=N-N(Me)-$CH_2$- | H |
| -CH=N-N(Ph)-$CH_2$- | H |
| -CH=N-N(Ph)-C(=O)- | H |
| -C(Me)=N-N(Ph)-C(=O)- | H |
| -N(Me)-$CH_2$-N(Ph)-O- | H |
| -N(Me)-C(=O)-N(Ph)-O- | H |
| -N(Me)-$CH_2$-N(Ph)-S- | H |
| -N(Me)-C(=O)-N(Ph)-S- | H |
| -S-$CH_2$-C(Ph)=N- | H |
| -S-$CH_2$-N(Ph)-N(Me)- | H |
| -S-C(=O)-N(Ph)-N(Me)- | H |
| -O-$CH_2$-C(Ph)=N- | H |
| -O-$CH_2$-N(Ph)-N(Me)- | H |
| -O-C(=O)-N(Ph)-N(Me)- | H |
| -S-CH(Ph)-N(Me)-N(Me)- | H |
| -O-CH(Ph)-N(Me)-N(Me)- | H |
| -N=C(Me)-N=N- | H |
| -N=C(Ph)-N=N- | H |
| -N=CH-CH=N- | H |
| -N=C(Ph)-CH=N- | H |
| -S-N(Me)-$CH_2$-N(Me)- | H |
| -S-N(Ph)-$CH_2$-N(Me)- | H |
| -O-N(Me)-$CH_2$-N(Me)- | H |
| -O-N(Ph)-$CH_2$-N(Me)- | H |
| -$CH_2$-$CH_2$-N(Me)-N(Me)- | H |
| -$CH_2$-$CH_2$-N(Ph)-N(Me)- | H |
| -$CH_2$-C(Ph)=N-N(Me)- | H |
| -CH=C(Ph)-N(Me)-N(Me)- | H |
| -CH=CH-N(Ph)-N(Me)- | H |
| -CH=C(Ph)-N=N- | H |
| -CH=N-C(Ph)=N- | H |
| -$CH_2$-$CH_2$-N=N- | H |
| -N(Me)-O-CH(Ph)-N(Me)- | H |
| -O-$CH_2$-C(Ph)=N- | H |

| | |
|---|---|
| $-CH_2-CH_2-CH_2-O-$ | H |
| $-CH_2-CH_2-CH_2-S-$ | H |
| $-CH_2-CH_2-CH_2-N(Me)-$ | H |
| $-CH=CH-CH_2-O-$ | H |
| $-CH=CH-CH_2-S-$ | H |
| $-CH=CH-CH_2-N(Me)-$ | H |
| $-CH_2-S-C(=N-Ph)-N(Me)-$ | H |

(Table 6)

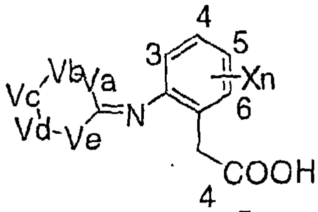 ,
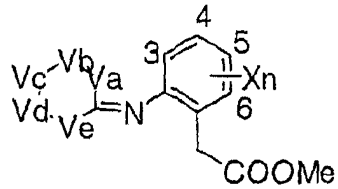 ,
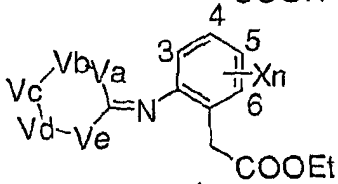 ,
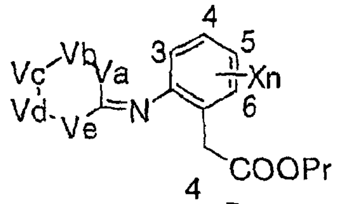 ,
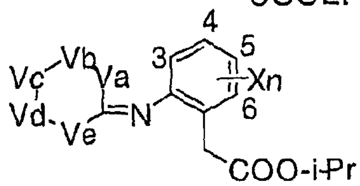 ,
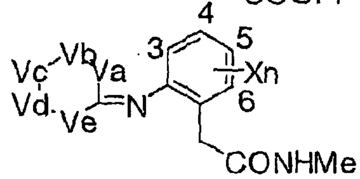 ,
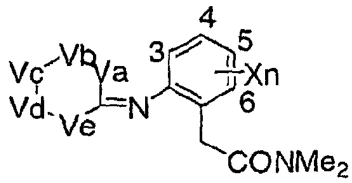 ,
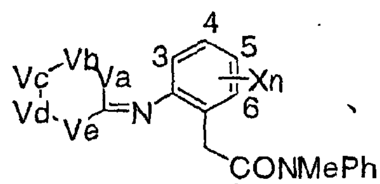 ,
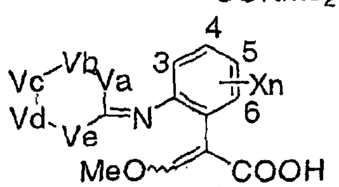 ,
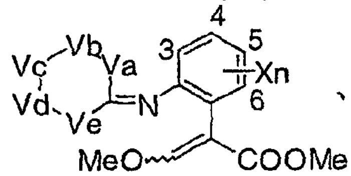 ,
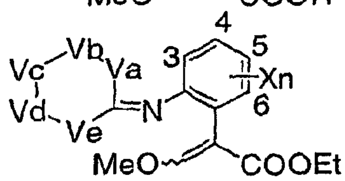 ,
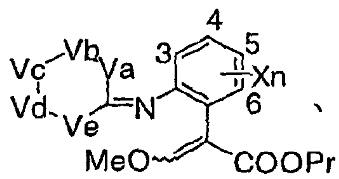 ,
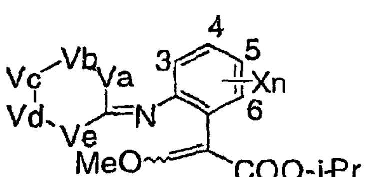 ,
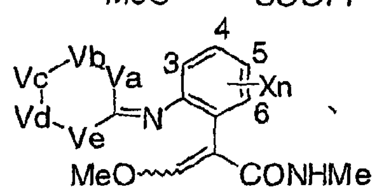 ,
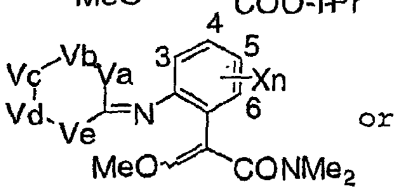 or
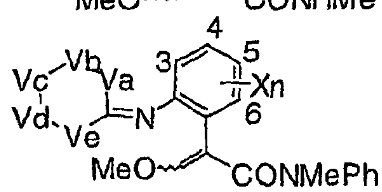

| -Va-Vb-Vc-Vd-Ve- | X |
|---|---|
| -S-$CH_2$-CH=N-N(Me)- | H |
| -S-$CH_2$-CH=N-N(Ph)- | H |
| -S-$CH_2$-C(Me)=N-N(Me)- | H |
| -S-$CH_2$-C(Ph)=N-N(Me)- | H |

| | |
|---|---|
| $-S-C(=O)-CH_2-N(Me)-N(Me)-$ | H |
| $-S-C(=O)-CH_2-N(Ph)-N(Me)-$ | H |
| $-S-CH=C(Me)-N(Ph)-N(Me)-$ | H |
| $-S-C(=O)-C(Me)=N-N(Me)-$ | H |
| $-S-C(=O)-C(Ph)=N-N(Me)-$ | H |
| $-CH_2-S-CH=CH-N(Me)-$ | H |
| $-N=CH-N=C(F)-N(Me)-$ | H |
| $-N=CH-N=C(Cl)-N(Me)-$ | H |
| $-N=CH-N=C(Br)-N(Me)-$ | H |
| $-N=CH-N=C(Ph)-N(Me)-$ | H |
| $-N=CH-N=C(OPh)-N(Me)-$ | H |
| $-N=CH-N=CH-N(Et)-$ | H |
| $-N=CH-N=CH-N(Pr)-$ | H |
| $-N=CH-N=C(CF_3)-N(Me)-$ | H |
| $-N=C(Cl)-N=CH-N(Me)-$ | H |
| $-N=CH-N=CH-N(Me)-$ | H |
| $-CH_2-CH_2-O-CH_2-CH_2-$ | H |
| $-CH_2-CH_2-N(Me)-CH_2-CH_2-$ | H |
| $-N(Me)-CH_2-CH=CH-S-$ | H |
| $-N(Me)-CH_2-C(Ph)=CH-S-$ | H |
| $-N(Me)-CH_2-CH=C(Ph)-S-$ | H |
| $-N(Me)-CH_2-CH=CH-O-$ | H |
| $-N(Me)-CH_2-C(Ph)=CH-O-$ | H |
| $-N(Me)-CH_2-CH=C(Ph)-O-$ | H |
| $-N=CH-CH=CH-S-$ | H |
| $-N=CH-C(Ph)=CH-S-$ | H |
| $-N=CH-CH=C(Ph)-S-$ | H |
| $-N=CH-CH=CH-O-$ | H |
| $-N=CH-C(Ph)=CH-O-$ | H |
| $-N=CH-CH=C(Ph)-O-$ | H |
| $-S-CH_2-C(=O)-N(Me)-N(Me)-$ | H |
| $-S-CH_2-C(=O)-NPh-N(Me)-$ | H |
| $-S-CH=CH-CH(Ph)-N(Me)-$ | H |
| $-CH=CH-CH=C(Ph)-N(-OMe)-$ | H |
| $-CH=CH-CH=C(F)-N(-OMe)-$ | H |
| $-CH=CH-CH=C(Cl)-N(-OMe)-$ | H |
| $-CH=CH-CH=C(-OPh)-N(-OMe)-$ | H |

(Table 7)

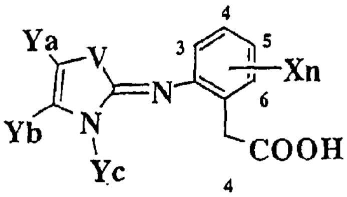
,
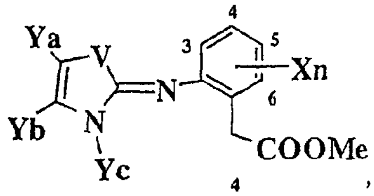
,
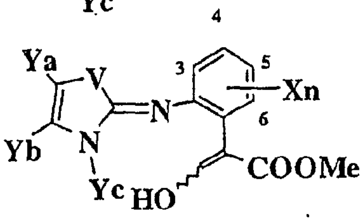
,
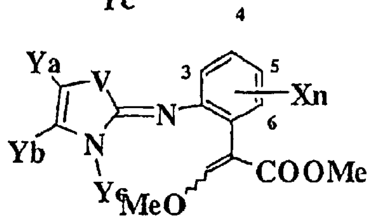
,
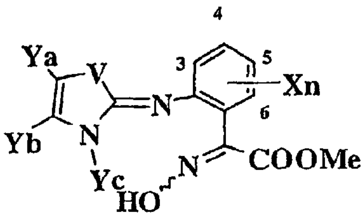
or
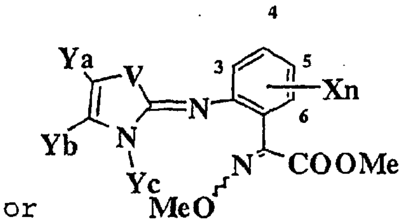

| Y a | Y b | Y c | V | X |
|---|---|---|---|---|
| H | H | Et | S | H |
| H | H | Ph | S | H |
| H | Me | Et | S | H |
| Me | H | Ph | S | H |
| H | Ph | Et | S | H |
| H | Ph | n-Pr | S | H |
| H | Ph | i-Pr | S | H |
| H | Ph | $CH_2CH=CH_2$ | S | H |
| H | Ph | $CH_2Ph$ | S | H |
| H | Ph | OMe | S | H |
| H | Ph | $NMe_2$ | S | H |
| H | Ph | $NH_2$ | S | H |
| H | Ph | NHPh | S | H |
| H | Ph | $CO_2Me$ | S | H |
| H | Ph | $CO_2Ph$ | S | H |
| H | Ph | Ph | S | H |
| Me | Ph | Et | S | H |
| Me | $CH_2OMe$ | OMe | S | H |
| Me | $CH_2OMe$ | $NMe_2$ | S | H |
| Me | COMe | $CO_2Me$ | S | H |
| Me | COMe | Ph | S | H |
| H | $CH_2OPh$ | OMe | S | H |
| H | $CH_2OPh$ | $NMe_2$ | S | H |
| H | $ClCH_2$ | Ph | S | H |
| Me | $ClCH_2$ | OMe | S | H |
| Me | 2-Naphthyl | $NMe_2$ | S | H |
| Me | 2-Naphthyl | $CO_2Me$ | S | H |
| Me | Thiophen-2-yl | OMe | S | H |
| H | Thiophen-2-yl | $NMe_2$ | S | H |
| H | Furan-2-yl | $CO_2Me$ | S | H |
| H | Furan-2-yl | Ph | S | H |
| H | Pyridin-2-yl | OMe | S | H |

| | | | | |
|---|---|---|---|---|
| Me | Pyridin-3-yl | $NMe_2$ | S | H |
| Me | Pyridin-4-yl | $CO_2Me$ | S | H |
| Me | Pyridin-4-yl | Ph | S | H |
| Me | Pyridin-4-yl | OMe | S | H |
| H | Pyridin-4-yl | $NMe_2$ | S | H |
| H | 4-Cl-Ph | OMe | S | H |
| H | 4-Cl-Ph | $NMe_2$ | S | H |
| H | 4-Cl-Ph | $CO_2Me$ | S | H |
| H | 4-Cl-Ph | Ph | S | H |
| Me | 4-F-Ph | OMe | S | H |
| Me | 4-F-Ph | $NMe_2$ | S | H |
| Me | 4-F-Ph | $CO_2Me$ | S | H |
| Me | 4-F-Ph | Ph | S | H |
| H | 4-Me-Ph | OMe | S | H |
| H | 4-Me-Ph | $NMe_2$ | S | H |
| H | 4-MeO-Ph | $CO_2Me$ | S | H |
| H | 4-MeO-Ph | Ph | S | H |
| Me | 4-$CF_3$-Ph | OMe | S | H |
| Me | 4-$CF_3$-Ph | $NMe_2$ | S | H |
| Me | $PhCH_2O$-Ph | $CO_2Me$ | S | H |
| Me | $PhCH_2O$-Ph | Ph | S | H |
| H | 4-Ph-Ph | OMe | S | H |
| H | 4-Ph-Ph | $NMe_2$ | S | H |
| H | 3-PhO-Ph | $CO_2Me$ | S | H |
| H | 3-PhO-Ph | Ph | S | H |
| H | Ph | NMePh | S | H |
| H | Ph | $NH_2$ | S | H |
| H | Ph | NHPh | S | H |
| H | H | Et | O | H |
| H | H | Ph | O | H |
| H | Me | Et | O | H |
| H | Me | Ph | O | H |
| Me | H | Et | O | H |
| Me | H | Ph | O | H |
| H | Ph | Et | O | H |
| H | Ph | n-Pr | O | H |
| H | Ph | i-Pr | O | H |
| H | Ph | $CH_2CH=CH_2$ | O | H |
| H | Ph | $CH_2Ph$ | O | H |
| H | Ph | OMe | O | H |
| H | Ph | $NMe_2$ | O | H |
| H | Ph | $CO_2Me$ | O | H |
| H | Ph | $CO_2Ph$ | O | H |
| H | Ph | Ph | O | H |
| Me | Ph | Et | O | H |
| H | $CO_2Me$ | OMe | O | H |
| H | $CO_2Me$ | $NMe_2$ | O | H |
| H | $CO_2Me$ | $CO_2Me$ | O | H |
| H | $CO_2Me$ | Ph | O | H |
| Me | $CH_2OMe$ | OMe | O | H |
| Me | $CH_2OMe$ | $NMe_2$ | O | H |
| Me | COMe | $CO_2Me$ | O | H |
| Me | COMe | Ph | O | H |
| H | $CH_2OPh$ | OMe | O | H |
| H | $CH_2OPh$ | $NMe_2$ | O | H |
| Me | 2-Naphthyl | $NMe_2$ | O | H |
| Me | Pyridin-4-yl | $CO_2Me$ | O | H |
| Me | Pyridin-4-yl | Ph | O | H |

| | | | | |
|---|---|---|---|---|
| Me | Pyridin-4-yl | OMe | O | H |
| H | Pyridin-4-yl | $NMe_2$ | O | H |
| H | 4-Cl-Ph | OMe | O | H |
| Me | 4-F-Ph | $NMe_2$ | O | H |
| H | 4-MeO-Ph | $CO_2Me$ | O | H |
| Me | $PhCH_2O$-Ph | Ph | O | H |
| H | 4-Ph-Ph | OMe | O | H |
| H | 4-Ph-Ph | $NMe_2$ | O | H |
| H | 3-PhO-Ph | $CO_2Me$ | O | H |
| H | 3-PhO-Ph | Ph | O | H |
| H | H | Et | N-Me | H |
| H | H | Ph | N-Me | H |
| H | Me | Et | N-Me | H |
| H | Me | Ph | N-Me | H |
| Me | H | Et | N-Me | H |
| H | Ph | Et | N-Me | H |
| H | Ph | n-Pr | N-Me | H |
| H | Ph | i-Pr | N-Me | H |
| H | Ph | $CH_2CH=CH_2$ | N-Me | H |
| H | Ph | $CH_2Ph$ | N-Me | H |
| H | Ph | OMe | N-Me | H |
| H | Ph | $NMe_2$ | N-Me | H |
| H | Ph | $CO_2Me$ | N-Me | H |
| H | Ph | $CO_2Ph$ | N-Me | H |
| H | Ph | Ph | N-Me | H |
| Me | Ph | Et | N-Me | H |
| Me | Ph | Ph | N-Me | H |
| H | $CO_2Me$ | OMe | N-Me | H |
| H | $CO_2Me$ | Ph | N-Me | H |
| Me | COMe | $CO_2Me$ | N-Me | H |
| Me | COMe | Ph | N-Me | H |
| Me | 2-Naphthyl | $CO_2Me$ | N-Me | H |
| Me | Pyridin-4-yl | Ph | N-Me | H |
| H | 4-Ph-Ph | $NMe_2$ | N-Me | H |

(Table 8)

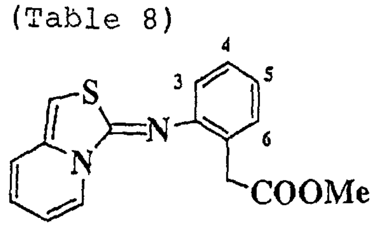
,
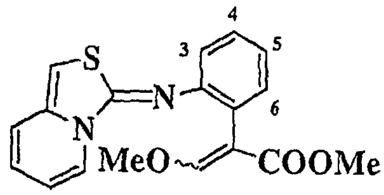
,
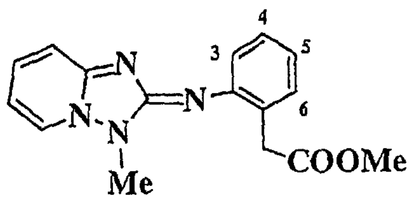
,
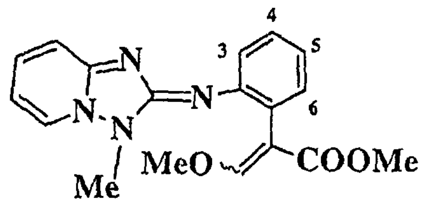
,
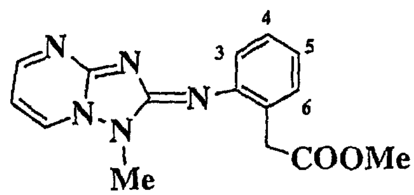
,
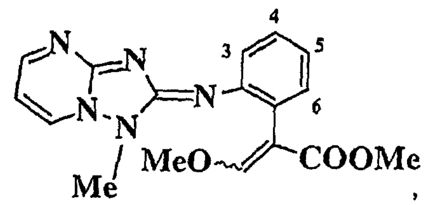
,
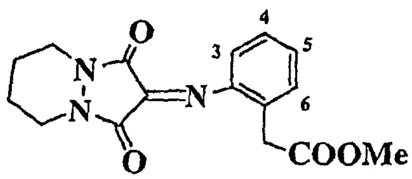
,
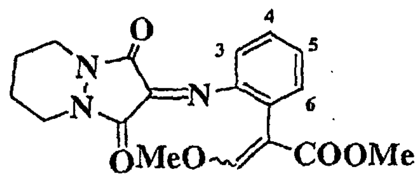
,
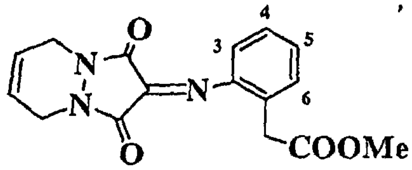
,
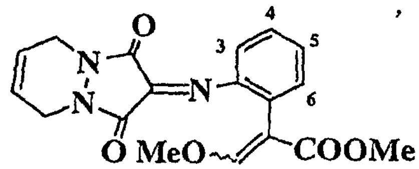
,
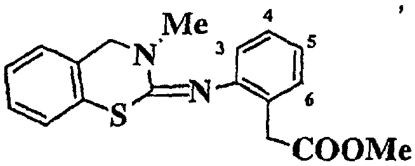
,
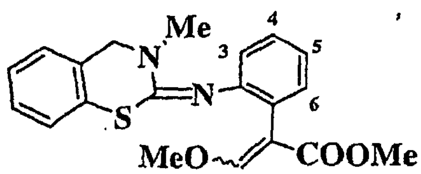
,
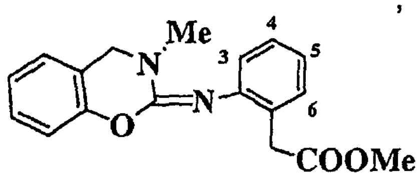
,
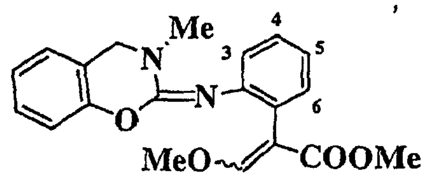
,
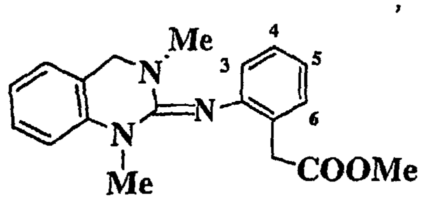
,
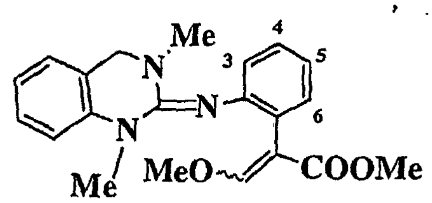
,

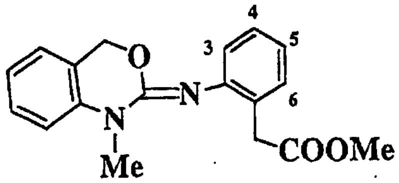

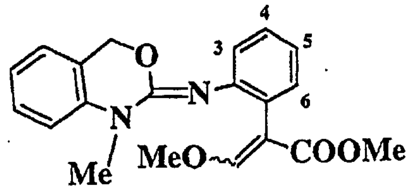

,

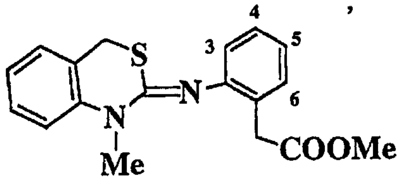

,

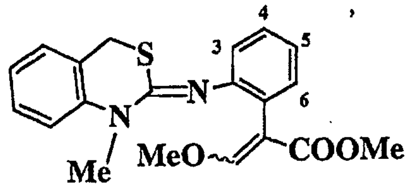

,

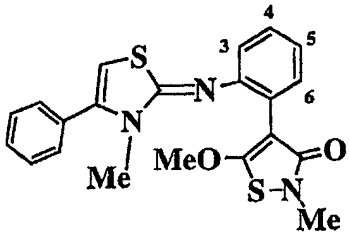

,

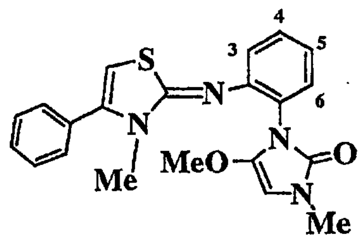

,

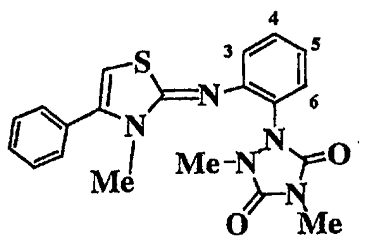

,

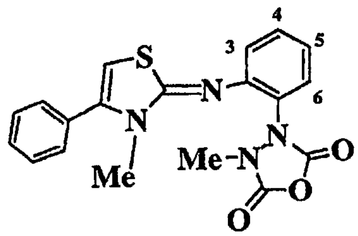

or

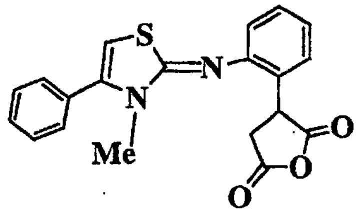

.

In the Tables, "Furan" represents furan, "Pyrrole" pyrrole, "Oxazol" oxazole, "Oxadiazol" oxadiazole, "Thiadiazol" thiadiazole, "Triazole" triazole, "Tetrazol" tetrazole, "Pyrimidin" pyrimidine, "Pyridazin" pyridazine, "Triazin" triazine, "Pyrazol" pyrazole, "Pyrrol" pyrrole, "Thiophen" thiophene, "Thiazol" thiazole, "Oxazol" oxazole, "Isotihazol" isthiazole, "Isoxazol" isoxazole, "Imidazol" imidazole, "Pyridin" pyridine, "Quinoxalin" quinoxaline, "Indol" indole, "Benzothiazol" benzothiazole, "Beisofuran" benzofuran, "Quinolin" quinoline, "Pyrazin" pyrazine, "y1" yl, "Ethenyl" ethenyl, "Ethynyl" ethynyl, "Propenyl" propenyl, "Propynyl" propynyl, "Naphthyl" naphthyl, "Hexamethyleneimino" hexamethyleneimino, "Morphorino" morpholin-1-yl, "Piperidino" pipridin-1-yl, "Pyrroridyl" pyrrolidyl, "methylenedioxy" methylenedioxy, and "ethylenedioxy" ethylenedioxy.

When the compounds of the present invention are used as plant disease and plant insect pest controlling agents, they are usually mixed with a suitable solid or liquid carrier, and if desired, a surfactant, a penetrating agent, a spreader, a thickener, an antifreezing agent, a binder, an anticaking agent, a disintegrator or a stabilizer may be added to prepare an optional formulation, such as a solution, an emulsifiable concentrate, a wettable powder, a water-soluble powder, a dry flowable, a water-soluble granule, a flowable, an emulsion, a suspoemulsion, a microemulsion, a dust, a granule or a gel. Further, any of the above-mentioned formulations may be encapsulated in water-soluble capsules for use with a view to saving labor and improving the safety.

As the solid carrier, for example, natural minerals such as quartz, kaolinate, pyrophyllite, sericite, talc, bentonite, acid clay, attapulgite, zeolite and diatomaceous earth, inorganic salts such as calcium carbonate, ammonium sulfate, sodium sulfate and potassium chloride, synthetic silicic acid and synthetic silicates, may be mentioned.

As the liquid carrier, for example, alcohols such as ethylene glycol, propylene glycol and isopropanol, aromatic

hydrocarbons such as xylene, alkylbenzenes and alkylnaphthalenes, ethers such as butyl cellosolve, ketones such as cyclohexanone, esters such as γ-butyrolactone, acid amides such as N-methylpyrrolidone and N-octylpyrrolidone, vegetable oils such as soybean oil, rapeseed oil, cottonseed oil and castor oil, and water, may be mentioned.

These solid and liquid carriers may be used singly or in combination.

As the surfactant, for example, nonionic surfactants such as polyoxyethylene alkyl ether, polyoxyethylene alkyl aryl ether, polyoxyethylene styryl phenyl ether, polyoxyethylene polyoxypropylene block copolymer, polyoxyethylene fatty acid ester, sorbitan fatty acid ester and polyoxyethylene sorbitan fatty acid ester, anionic surfactants such as alkylsulfates, alkylbenzenesulfonates, lignin sulfonate, alkylsulfosuccinates, naphthalenesulfonates, alkylnaphthalenesulfonates, salts of naphthalenesulfonic acid-formalin condensate, salts of alkylnaphthalenesulfonic acid-formalin condensate, sulfate and phosphate of polyoxyethylene alkyl aryl ether, sulfate and phosphate of polyoxyethylene styryl phenyl ether, polycarboxylates and polystyrenesulfonates, cationic surfactants such as alkylamine salts and quaternary alkylammonium salts and amphoteric surfactants of the amino acid type and the betaine type, may be mentioned.

The amount of such a surfactant is not particularly limited, but it is usually preferred to be within a range of from 0.05 to 20 parts by weight per 100 parts by weight of the formulation of the present invention. These surfactants may be used singly or in combination.

Further, when the compounds of the present invention are to be used as agricultural chemicals, they may be combined with other herbicides, various insecticides, miticides, nematocides, fungicides, plant growth regulators, synergists, fertilizers or soil conditioning materials at the time of formulating them or at the time of application, as the case requires.

Particularly, their combined use with other agricultural chemicals or plant hormones, is expected to bring about cost reduction due to a reduction in the dose and broadening of the fungicidal and insecticidal spectra or higher pesticidal effects, due to synergistic effects of the combined chemicals. In such a case, the compounds of the present invention can be combined with plural known agricultural chemicals simultaneously. The agricultural chemicals which may be used in combination with the compounds of the present invention, may, for example, be compounds disclosed in Farm Chemicals Handbook (1999). Their common names may specifically be exemplified as follows, but it should be understood that useful agricultural chemicals are not limited thereto.

Fungicides: acibenzolar, ampropyfos, anilazine, azaconazole, azoxystrobin, benalaxyl, benodanil, benomyl, benzamacril, binapacryl, biphenyl, bitertanol, bethoxazine, bordeaux mixture, blasticidin-S, bromoconazole,

bupirimate, buthiobate, calcium polysulfide, captafol, captan, copper oxychloride, carpropamid, carbendazim, carboxin, chinomethionat, chlobenthiazone, chlorfenazol, chloroneb, chlorothalonil, chlozolinate, cufraneb,

cymoxanil, cyproconazol, cyprodinil, cyprofuram, debacarb, dichlorophen, diclobutrazol, dichlofluanid, diclomedine, dicloran, diethofencarb, diclocymet, difenoconazole, diflumetorim, dimethirimol,

dimethomorph, diniconazole, diniconazole-M, dinocap, diphenylamine, dipyrithione, ditalimfos, dithianon, dodemorph, dodine, drazoxolon, edifenphos, epoxiconazole, etaconazole, ethirimol, etridiazole, famoxadone, fenarimol, febuconazole, fenfuram,

fenpiclonil, fenpropidin, fenpropimorph, fentin, ferbam, ferimzone, fluazinam, fludioxonil, fluoroimide, fluquinconazole, flusilazole, flusulfamide, flutolanil, flutriafol, folpet, fosetyl-aluminium, fuberidazole, furalaxyl, fenamidone, fenhexamid,

guazatine, hexachlorobenzene, hexaconazole, hymexazol, imazalil, imibenconazole, iminoctadine, ipconazole, iprobenfos, iprodione, isoprothiolane, iprovalicarb,

kasugamycin, kresoxim-methyl, mancopper, mancozeb, maneb, mepanipyrim, mepronil, metalaxyl, metconazole, metiram, metominostrobin, myclobutanil, nabam, nickel bis(dimethyldithiocarbamate), nitrothal-isopropyl, nuarimol, octhilinone, ofurace, oxadixyl, oxycarboxin, oxpoconazole fumarate,

pefurzoate, penconazole, pencycuron, phthalide, piperalin, polyoxins, probenazole, prochloraz, procymidone, propamocarb hydrochloride, propiconazole, propineb, pyrazophos, pyrifenox, pyrimethanil, pyroquilon, quinoxyfen, quintozene,

sulfur, spiroxamine, tebuconazole, tecnazene, tetraconazole, thiabendazole, thifluzamide, thiophanate-methyl, thiram, tolclofos-methyl, tolylfluanid,

triadimefon, triadimenol, triazoxide, tricyclazole, tridemorph, triflumizole, triforine, triticonazole, validamycin, vinclozolin, zineb, ziram, etc.

Bactericides: streptomycin, oxytetracycline, oxolinic acid, etc.

Nematocides: aldoxycarb, fosthiazate, fosthietan, oxamyl, fenamiphos, etc.

Miticides: amitraz, bromopropylate, chinomethionat, chlorobezilate, clofentezine, cyhexatine, dicofol, dienochlor, etoxazole, fenazaquin, fenbutatin oxide, fenpropathrin, fenproximate, halfenprox, hexythiazox, milbemectin, propargite, pyridaben, pyrimidifen, tebufenpyrad, etc.

Insecticides: abamectin, acephate, acetamipirid, azinphos-methyl, bendiocarb, benfuracarb, bensultap, bifenthrin, buprofezin, butocarboxim, carbaryl, carbofuran, carbosulfan, cartap, chlorfenapyr, chlorpyrifos, chlorfenvinphos, chlorfluazuron, clothianidin, chromafenozide, chlorpyrifos-methyl, cyfluthrin, beta-cyfluthrin, cypermethrin, cyromazine,

cyhalothrin, lambda-cyhalothrin, deltamethrin, diafenthiuron, diazinon, diacloden, diflubenzuron, dimethylvinphos, diofenolan, disulfoton, dimethoate, EPN, esfenvalerate, ethiofencarb, ethiprole, etofenprox, etrimfos, fenitrothion, fenobucarb, fenoxycarb, fenpropathrin, fenvalerate, fipronil, flucythrinate, flufenoxuron, flufenprox, taufluvalinate, fonophos, formetanate, formothion, furathiocarb,

halofenozide, hexaflumuron, hydramethylnon, imidacloprid, isofenphos, indoxacarb, isoprocarb, isoxathion, lufenuron, malathion, metaldehyde, methamidophos, methidathion, methacrifos, metalcarb, methomyl, methoprene, methoxychlor, methoxyfenozide, monocrotophos, muscalure, nitenpyram, omethoate, oxydemeton-methyl, oxamyl,

parathion, parathion-methyl, permethrin, phenthoate, phoxim, phorate, phosalone, phosmet, phosphamidon, pirimicarb, pirimiphos-methyl, profenofos, pymetrozine, pyraclofos, pyriproxyfen, rotenone, sulprofos, silafluofen, spinosad, sulfotep, tebfenozide, teflubenzuron, tefluthorin, terbufos, tetrachlorvinphos, thiodicarb, thiamethoxam, thiofanox, thiometon, tolfenpyrad, tralomethrin, trichlorfon, triazuron, triflumuron, vamidothion, etc.

The dose of the compounds of the present invention varies depending upon the application site, the season for application, the manner of application, the type of crop plants or the like. However, it is usually within a range of from 0.005 to 50 kg per hectare (ha) as the amount of the active ingredient.

Now, Examples of formulations in which the compounds of the present invention are employed, will be given. However, the formulation examples of the present invention are by no means limited thereto. In the following Formulation Examples, "parts" means "parts by weight".

| [Wettable powder] | |
|---|---|
| Compound of the present invention | 0.1 - 80 parts |
| Solid carrier | 10 - 98.9 parts |
| Surfactant | 1 - 10 parts |
| Others | 0 - 5 parts |

As the others, for example, an anticaking agent, a stabilizer and the like may be mentioned.

| [Emulsifiable concentrate] | |
|---|---|
| Compound of the present invention | 0.1 - 30 parts |
| Liquid carrier | 45 - 95 parts |
| Surfactant | 4.9 - 15 parts |
| Others | 0 - 10 parts |

As the others, for example, a spreader, a stabilizer and the like may be mentioned.

| [Flowable] | |
|---|---|
| Compound of the present invention | 0.1 - 70 parts |
| Liquid carrier | 15- 98.89 parts |
| Surfactant | 1 - 12 parts |
| Others | 0.01 - 30 parts |

As the others, for example, an antifreezing agent, a thickener and the like may be mentioned.

| [Dry flowable] | |
|---|---|
| Compound of the present invention | 0.1 - 90 parts |
| Solid carrier | 0 - 98.9 parts |
| Surfactant | 1 - 20 parts |
| Others | 0 - 10 parts |

As the others, for example, a binder, a stabilizer and the like may be mentioned.

| [Liquid formulation] | |
|---|---|
| Compound of the present invention<br>Liquid carrier<br>Others | 0.01 - 70 parts<br>20 - 99.99 parts<br>0 - 10 parts |

As the others, for example, an antifreezing agent, a spreader and the like may be mentioned.

| [Granule] | |
|---|---|
| Compound of the present invention<br>Solid carrier<br>Others | 0.01 - 80 parts<br>10 - 99.99 parts<br>0 - 10 parts |

As the others, for example, a binder, a stabilizer and the like may be mentioned.

| [Dust] | |
|---|---|
| Compound of the present invention<br>Solid carrier<br>Others | 0.01 - 30 parts<br>65 - 99.99 parts<br>0 - 5 parts |

As the others, for example, an anti-drifting agent, a stabilizer and the like may be mentioned.
In use, the above formulations may be applied by diluting it with water by from 1 to 10000 times or without dilution.
The application method of the compound of the present invention may, for example, be foliage treatment, soil treatment or seed disinfection, but any method commonly used by those skilled in the art may effectively be employed.
Now, Preparation Examples of the compounds of the present invention will be given below as Working Examples, but it should be understood that the present invention is by no means restricted thereto.

EXAMPLE 1

Preparation of methyl 2-(2-(aza(5-methyl-4-phenyl-2,5-thiazolinylidene)methyl)phenyl)acetate (compound I-1 of the present invention)

4.76 g (20 mmol) of methyl 2-(2-(((methylamino)thioxomethyl)amino)phenyl)acetate was dissolved in 23 ml of N,N-dimethylformamide, and 3.98 g (20 mmol) of phenacyl bromide was added thereto. After heating and stirring the mixture at 115°C for 3 hours, 50 ml of water and 25 ml of a 1N sodium hydroxide aqueous solution were added to the reaction mixture, followed by extraction with ethyl acetate. The organic layer was dried over anhydrous magnesium sulfate, followed by filtration, and the solvent was distilled off under reduced pressure. The obtained residue was purified by silica gel column chromatography (chloroform) to obtain 6.6 g of the desired methyl 2-(2-(aza(5-methyl-4-phenyl-2,5-thiazolinylidene)methyl)phenyl acetate, as a colorless oil.

Refractive index: $n_{D21.5}$1.5600

EXAMPLE 2

Preparation of methyl 2-(2-(aza(5-methyl-4-phenyl-2,5-thiazolinylidene)methyl)phenyl)-3-methoxy-2-propenoate (compound I-37 of the present invention) and (compound I-38 of the present invention)

0.61 g (14 mmol) of 55% sodium hydride was suspended in 10 ml of N,N-dimethylformamide, and a solution having 2 g (6 mmol) of methyl 2-(2-(aza(5-methyl-4-phenyl-2,5-thiazolinylidene)methyl)phenyl)acetate and 3.6 g (60 mmol) of methyl formate dissolved in 40 ml of N,N-dimethylformamide, was added thereto at room temperature, followed by stirring for 12 hours. To this mixture, 8.28 g (60 mmol) of anhydrous potassium carbonate was added, and then 1.51 g (12 mmol) of dimethyl sulfate was dropwise added thereto. After stirring the mixture for further 3 hours at room temperature, 150 ml of water was added to the reaction mixture, followed by extraction with ethyl acetate. Then, the organic layer was dried over anhydrous magnesium sulfate. After filtration, the solvent was distilled off, and the obtained residue was purified by silica gel column chromatography (n-hexane: ethyl acetate=1:1), whereby as a low

polarity component, 0.84 g of E-isomer methyl 2-(2-(aza(5-methyl-4-phenyl-2,5-thiazolinylidene)methyl)phenyl)-3-methoxy-2-propenoate and as a high polarity component, 0.24 g of Z-isomer methyl 2-(2-(aza(5-methyl-4-phenyl-2,5-thiazolinylidene)methyl)phenyl)-3-methoxy-2-propenoate were obtained, respectively, as colorless crystals.

E-isomer, melting point: 118-120°C

Z-isomer, melting point: 141-143°C

EXAMPLE 3

Preparation of methyl 2-(2-(aza(5-(dimethylamino)-4-phenyl-(2,5-thiazolinylidene))methyl)phenyl)acetate (compound II-11 of the present invention)

0.44 g (7.4 mmol) of 1,1-dimethylhydrazine was dissolved in 100 ml of tetrahydrofuran, and 1.53 g (7.4 mmol) of methyl 2-(2-isothiocyanatephenyl)acetate was added thereto. After stirring it at room temperature for 1 hour, the solvent was distilled off under reduced pressure. The obtained residue was washed with diisopropyl ether to obtain 1.43 g of methyl 2-(2-((((dimethylamino)amino)thioxomethyl)amino)phenyl)acetate as colorless crystals.

Melting point: 130-131°C

Then, 1.2 g (4.5 mmol) of the obtained methyl 2-(2-((((dimethylamino)amino)thioxomethyl)amino)phenyl)acetate was dissolved in 5 ml of N,N-dimethylformamide, and 0.9 g (4.5 mmol) of phenacyl bromide was added thereto. After stirring the mixture at room temperature for 1 hour, 20 ml of water and 5 ml of a 1N sodium hydroxide aqueous solution were added to the reaction mixture, followed by extraction with ethyl acetate. The organic layer was dried over anhydrous magnesium sulfate, followed by filtration, and the solvent was distilled off under reduced pressure. The obtained residue was washed with diisopropyl ether to obtain 1.1 g of the desired methyl 2-(2-(aza(5-(dimethylamino)-4-phenyl-(2,5-thiazolinylidene))methyl)phenyl)acetate as colorless crystals.

Melting point: 88-89°C

EXAMPLE 4

Preparation of methyl 2-(2-(aza(6-phenyl-2,6-thiazaperhydroynylidene))methyl)phenyl)acetate (compound V-1 of the present invention)

0.27 (2 mmol) of N-phenyl-N-allylamine was dissolved in 2 ml of tetrahydrofuran, and 0.41 g (2 mmol) of methyl 2-(2-isothiocyanatephenyl)acetate was added thereto. After stirring it at room temperature for 16 hours, the solvent was distilled off under reduced pressure. The obtained residue was washed with diethyl ether to obtain 0.5 g of methyl 2-(2-(((methyl-2-propenylamino)thioxomethyl)amino)phenyl)acetate as colorless crystals.

Melting point: 72-73°C

Then, 0.4 g of methyl 2-(2-(((methyl-2-propenylamino)thioxomethyl)amino)phenyl)acetate was dissolved in 2 ml of trifluoroacetic acid, followed by stirring at room temperature for 16 hours. Then, the solvent was distilled off under reduced pressure to obtain 0.33 g of the desired methyl 2-(2-(aza(6-phenyl(2,6-thiazaperhydroynylidene))methyl)phenyl)acetate as a slightly yellow oil.

Refractive index: $n_{D21.1}$1.5728

EXAMPLE 5

Preparation of methyl 2-(2-(aza(5-methyl-3-methylene(2,5-thiazolinylidene)methyl)phenyl)acetate (compound IV-1 of the present invention)

1.52 g (22 mmol) of N-methyl-N-propargylamine was dissolved in 200 ml of tetrahydrofuran, and 4.14 g (20 mmol) of methyl 2-(2-isothiocyanatephenyl)acetate was added thereto. After stirring the mixture at room temperature for 2 hours, the solvent was distilled off under reduced pressure. The obtained residue was washed with diisopropyl ether to obtain 4.3 g of methyl 2-(2-(((methyl-2-propynylamino)thioxomethyl)amino)phenyl)-acetate as colorless crystals.

Melting point: 83-84°C

Then, the obtained methyl 2-(2-(((methylpropynylamino)thioxomethyl)amino)phenyl)acetate was left to stand at room temperature for 2 weeks to obtain the desired methyl 2-(2-(aza(5-methyl-3-methylene(2,5-thiazolinylidene) methyl)phenyl)acetate as a slightly yellow oil.

Refractive index: $n_{D21.0}$1.5078

EXAMPLE 6

Preparation of methyl 2-(2-(aza(5-methyl-3-(bromomethyl)-2,5-thiazolinylidene)methyl)phenyl)acetate hydrobromide (compound I-202 of the present invention)

2.7 g (10 mmol) of methyl 2-(2-(((methylpropynylamino)thioxomethyl)amino)phenyl)acetate obtained in Example 5, was dissolved in 50 ml of chloroform, and 1.6 g (10 mmol) of bromine was added thereto. This mixture was stirred at room temperature for 1 hour, and then, the solvent was distilled off under reduced pressure. The residue was washed with ethyl acetate to obtain 2.6 g of the desired methyl 2-(2-(aza(5-methyl-3-(bromomethyl)-2,5-thiazolinylidene)methyl)phenyl)acetate hydrobromide as colorless crystals.

Melting point: 180-183°C

EXAMPLE 7

Preparation of methyl 2-(2-(aza(5-methyl-3-(bromomethyl)-2,5-thiazolidinylidene)methyl)phenyl)acetate hydrobromide (compound IV-13 of the present invention)

1.56 g (22 mmol) of N-methyl-N-allylamine was dissolved in 200 ml of tetrahydrofuran, and 4.14 g (20 mmol) of methyl 2-(2-isothiocyanatephenyl)acetate was added thereto. After stirring the mixture at room temperature for 2 hours, the solvent was distilled off under reduced pressure. The obtained residue was washed with diisopropyl ether, to obtain 3.9 g of methyl 2-(2-(((methyl-2-propenylamino)thioxomethyl)amino)phenyl)-acetate as colorless crystals.

Melting point: 46-47°C

Then, 3.5 g (12.6 mmol) of the obtained methyl 2-(2-(((methyl-2-propenylamino) thioxomethyl)amino) phenyl)-acetate was dissolved in 130 ml of chloroform, and 2 g (12.6 mmol) of bromine was added thereto. The mixture was stirred at room temperature for 3 hours, and then, the solvent was distilled off under reduced pressure. The residue was washed with diethyl ether to obtain 5.4 g of the desired methyl 2-(2-(aza(5-methyl-3-(bromomethyl)-2,5-thiazolidinylidene)methyl)phenyl)acetate hydrobromide as colorless crystals.

Melting point: 177-179°C

EXAMPLE 8

Preparation of methyl 2-(2-(aza(5-methyl-4-(trifluoromethyl)(2,5-thiazolinylidene))methyl)phenyl)-acetate (compound I-29 of the present invention)

A solution having 1.5 g (6.3 mmol) of methyl 2-(2-(((methylamino)thioxomethyl)amino)phenyl)acetate and 1.2 g (6.3 mmol) of 3-bromo-1,1,1-trifluoro-2-propanone dissolved in 5 ml of N,N-dimethylformamide, was stirred at room temperature for 2 hours. Water and a 1N sodium hydroxide aqueous solution were added to the reaction solution, followed by extraction with ethyl acetate. The organic layer was washed with water and a saturated sodium chloride aqueous solution and then dried over anhydrous magnesium sulfate, followed by filtration. The solvent was distilled off under reduced pressure. The obtained residue was dissolved in 30 ml of dichloromethane, and 3.13 g (39.6 mmol) of pyridine was added thereto. Then, 1.67 g (8.0 mmol) of trifluoroacetic anhydride was dropwise added thereto, followed by stirring at room temperature for 1 hour. After removing a precipitated salt by filtration, the solvent was distilled off under reduced pressure. The residue was purified by silica gel column chromatography (n-hexane:ethyl acetate=2:1) to obtain 1.8 g of the desired methyl 2-(2-(aza-(5-methyl-4-(trifluoromethyl)(2,5-thiazolinylidene))methyl)phenyl)-acetate as a colorless oil.

Refractive index: $n_{D21.5}$1.5528

EXAMPLE 9

Preparation of methyl 2-(2-(aza(3-methyl-3-hydrobenzothiazol-2-ylidene)methyl)phenyl)acetate (compound VI-1 of the present invention)

0.45 g (3.03 mmol) of trimethyloxonium tetrafluoroborate was suspended in 15 ml of 1,2-dichloroethane, and 0.51 g (3.03 mmol) of 2-chlorobenzothiazole was added thereto. This mixture was heated and stirred at 60°C for 1.5 hours and then cooled to room temperature. Further, 0.5 g (3.03 mmol) of methyl 2-aminophenyl acetate dissolved in 2 ml of 1,2-dichloroethane, was added, followed by stirring at room temperature for 2 hours. To the reaction mixture, 50 ml of a saturated sodium hydrogencarbonate aqueous solution was added, followed by extraction with 1,2-dichloroethane. Then, the organic layer was dried over anhydrous sodium sulfate. After filtration, the solvent was distilled

off, and the obtained residue was purified by high performance liquid chromatography (acetonitrile:water=4:1), followed by thin layer chromatography (chloroform), to obtain 0.52 g of methyl 2-(2-(aza(3-methyl-3-hydrobenzothiazol-2-ylidene)methyl)phenyl)acetate as a colorless oil.

Refractive index: $n_{D21.5}$1.5512

EXAMPLE 10

Preparation of methyl 2-(2-(azabenzo[c]2,5-dithiolen-2-ylidenemethyl)phenyl)acetate (compound VI-3 of the present invention)

0.45 g (3.03 mmol) of trimethyloxonium tetrafluoroborate was suspended in 15 ml of 1,2-dichloroethane, and 0.56 g (3.03 mmol) of 1,3-benzodithiol-2-thion was added thereto. This mixture was heated and stirred at 60°C for 30 minutes and then, cooled to room temperature. Further, 0.5 g (3.03 mmol) of methyl 2-aminophenyl acetate dissolved in 2 ml of 1,2-dichloroethane, was added, followed by stirring at room temperature for 2.5 hours. To the reaction mixture, 50 ml of a saturated sodium hydrogencarbonate aqueous solution was added, followed by extraction with chloroform. Then, the organic layer was dried over anhydrous sodium sulfate. After filtration, the solvent was distilled off, and the obtained residue was purified by high performance liquid chromatography (acetonitrile:water=4:1) to obtain 0.1 g of methyl 2-(2-(azabenzo[c]2,5-dithiolen-2-ylidenemethyl)phenyl)acetate as a slightly yellow oil.

Refractive index: $n_{D21.4}$1.5032

EXAMPLE 11

Preparation of methyl 2-(2-(aza(3-(4-bromophenyl)(2,5-dithiolenylidene))methyl)phenyl)acetate (compound IV-39 of the present invention)

2.04 g (5.96 mmol) of triethylammonium 2-(2-methoxy-2-oxoethyl)phenylcarbamodithioate was dissolved in 30 ml of chloroform, and 0.13 g (1.28 mmol) of triethylamine dissolved in 2 ml of chloroform, was added thereto. Then, 1.74 g (6.26 mmol) of 2-bromo-1-(4-bromophenyl)ethanone was added. After stirring at room temperature for 4 hours, the reaction mixture was poured into 1N hydrochloric acid and extracted with chloroform. The organic layer was washed with a saturated sodium chloride aqueous solution and dried over anhydrous sodium sulfate, followed by filtration. The solvent was distilled off under reduced pressure. The obtained residue was purified by silica gel column chromatography (ethyl acetate:hexane=1:2) to obtain 1.53 g of methyl 2-(2-(((2-oxo-2-(4-bromophenyl)ethylthio)thioxomethyl)amino)phenyl)acetate.

Then, 1.53 g of the obtained methyl 2-(2-(((2-oxo-2-(4-bromophenyl)ethylthio)thioxomethyl)amino)phenyl)-acetate was dissolved in 15 ml of sulfuric acid. After stirring at room temperature for 1 hour, the reaction mixture was poured into ice water and extracted with ethyl acetate. The organic layer was washed with a saturated sodium chloride aqueous solution and dried over anhydrous sodium sulfate, followed by filtration. The solvent was distilled off under reduced pressure. The obtained residue was purified by silica gel column chromatography (ethyl acetate:hexane=1:3) to obtain 0.4 g of the desired methyl 2-(2-(aza(3-(4-bromophenyl)(2,5-dithiolenylidene))methyl)phenyl)acetate as colorless crystals.

Melting point: 109-112°C

EXAMPLE 12

Preparation of methyl (2-((1,3-dimethyl-4-oxo-2-imidazolidinylidene)amino)phenyl)acetate (compound IV-8 of the present invention)

0.6 g (4 mmol) of sarcosineethyl ester hydrochloride was suspended in 15 ml of chloroform, and 1.0 g (10 mmol) of triethylamine was dropwise added thereto at room temperature. This suspension mixture solution was cooled with ice, and a solution of 0.8 g (4 mmol) of methyl (2-(((methylimino)methylene)amino)phenyl)acetate in chloroform (5 ml) was dropwise added thereto. The reaction solution was returned to room temperature and stirred for further 3 hours. Then, the reaction solution was washed with water and a saturated sodium chloride aqueous solution, and dried over anhydrous sodium sulfate, followed by filtration. The solvent was distilled off under reduced pressure. The obtained residue was purified by silica gel column chromatography (n-hexane:ethyl acetate=3:2) and then crystallized from diisopropyl ether, to obtain 0.4 g of the desired methyl (2-((1,3-dimethyl-4-oxo-2-imidazolidinylidene)amino)phenyl)acetate as colorless crystals.

Melting point: 57-59°C

EXAMPLE 13

Preparation of methyl (2-((3-methyl-6-phenyl(3H-2,6-thiazinylidene)amino)phenyl)acetate (compound V-3 of the present invention)

2.86 g (13.8 mmol) of methyl (2-isothiocyanatephenyl)acetate and 2 g (13.8 mmol) of N-(3-phenyl-2-propenylidene)methaneamine were dissolved in 30 ml of benzene and stirred for 2 hours under heating and refluxing. After completion of the reaction, the mixture was returned to room temperature, and the solvent was distilled off. Then, the residue was purified by silica gel column chromatography (n-hexane:ethyl acetate=2:1) to obtain 2.2 g of the desired methyl (2-((3-methyl-6-phenyl(3H-2,6-thiazinylidene)amino)phenyl)acetate as a colorless oil.

Refractive index: $n_{D21.5}$1.6292

EXAMPLE 14

Preparation of methyl 2-(2- (1-aza-2-(2,2-dimethyl-2-silapropylthio)-2-methylthiovinyl)phenyl)acetate (compound VII-8)

2.9 g (14 mmol) of methyl (2-isothiocyanatephenyl)acetate and 1.68 g (14 mmol) trimethylsilylmethanethiol were dissolved in 20 ml of ethanol, and then a catalytic amount of 1,8-diazabicycio[5.4.0]undec-7-ene was added, followed by stirring at room temperature for 1 hour. After completion of the reaction, the solvent was distilled off under reduced pressure, and water was added. Extraction was carried out with chloroform, and the organic layer was washed with a saturated sodium chloride aqueous solution and dried over anhydrous sodium sulfate, followed by filtration. The solvent was distilled off under reduced pressure to obtain 4.75 g of the desired methyl 2-(2-(((2,2-dimethyl-2-silapropylthio)-2-methylthiovinyl)phenyl)acetate as a crude product.

The obtained crude product was dissolved in 20 ml of acetone, and under cooling with ice, 2.32 g (16.8 mmol) of potassium carbonate and 4.60 g (28 mmol) of methyl trifluoromethane sulfonate, were added, followed by stirring for 2 hours under cooling with ice. After completion of the reaction, the solvent was distilled off. Then, water was added to the residue, followed by extraction with ethyl acetate. The organic layer was washed with a saturated sodium chloride aqueous solution and dried over anhydrous magnesium sulfate, followed by filtration. The solvent was distilled off under reduced pressure. The obtained residue was purified by silica gel column chromatography (n-hexane:ethyl acetate=5:1) to obtain 4.5 g of the desired methyl 2-(2-(1-aza-2-(2,2-dimethyl-2-silapropylthio)-2-methylthiovinyl)phenyl)acetate as a colorless oil.

Refractive index: $n_{D21.0}$1.5676

EXAMPLE 15

Preparation of methyl 2-(2-(1-aza-2-(2-hydroxy-2-phenylethylthio)-2-methylthiovinyl)phenyl)acetate

1.03 g (3 mmol) of methyl 2-(2-(1-aza-2-(2,2-dimethyl-2-silapropylthio)-2-methylthiovinyl)phenyl)acetate, 3.18 g (30 mmol) of benzaldehyde and 0.92 g (6.1 mmol) of cesium fluoride were dissolved in N,N-dimethylformamide (6 ml) and stirred at room temperature for 3 days in a nitrogen atmosphere. After completion of the reaction, water was added to the reaction solution, followed by extraction with ethyl acetate. The organic layer was washed with a saturated sodium chloride aqueous solution and dried over anhydrous magnesium sulfate, followed by filtration. The solvent was distilled off under reduced pressure. The obtained residue was purified by silica gel column chromatography (n-hexane: ethyl acetate=2:1) to obtain 0.3 g of methyl 2-(2-(1-aza-2-(2-hydroxy-2-phenylethylthio)-2-methylthiovinyl)phenyl)acetate.

EXAMPLE 16

Preparation of methyl 2-(2-(aza(3-phenyl(2,5-oxathioranylidene))methyl)phenyl)acetate (compound IV-28 of the present invention)

0.3 g (0.8 mmol) of methyl 2-(2-(1-aza-2-(2-hydroxy-2-phenylethylthio)-2-methylthiovinyl)phenyl)acetate was dissolved in 10 ml of xylene and stirred for 1 hour under heating and refluxing. After completion of the reaction, the mixture was returned to room temperature, and the solvent was distilled off under reduced pressure. Then, the residue was purified by silica gel column chromatography (n-hexane:ethyl acetate=2:1) to obtain 0.2 g of the desired methyl 2-(2-(aza(3-phenyl(2,5-oxathioranylidene))methyl)phenyl)acetate.

$^1$HNMR ($CDCl_3$ $\delta$ (ppm))

3.38(dd, J=9.1, 11.0Hz, 1H), 3.60-3.65(m, 1H), 3.65(s, 3H), 3.66(s, 2H), 5.63(dd, J=5.8, 9.1Hz, 1H), 6.96-7.46 (m, 9H)

EXAMPLE 17

Preparation of methyl 2-(2-(aza(6-methyl-4-phenyl(3H-2,5,6-thiadiazinylidene))methyl)phenyl)acetate (compound V-2 of the present invention)

0.34 g (7.4 mmol) of methylhydrazine was dissolved in 100 ml of tetrahydrofuran, and 1.53 g (7.4 mmol) of methyl 2-(2-isothiocyanatephenyl)acetate was added thereto. After stirring the mixture at room temperature for 1 hour, the solvent was distilled off under reduced pressure. The obtained residue was washed with diisopropyl ether to obtain 1.4 g of methyl 2-(2-(((aminomethylamino)thioxomethyl)amino)phenyl)acetate as colorless crystals.

Melting point: 149-150°C

Then, 1.1 g (4.3 mmol) of the obtained methyl 2-(2-(((aminomethylamino)thioxomethyl)amino)phenyl)acetate was dissolved in 5 ml of N,N-dimethylformamide, and 0.87g (4.3 mmol) of phenacyl bromide was added thereto. After stirring the mixture at room temperature for 2 hours, 20 ml of water and 5 ml of a 1N sodium hydroxide aqueous solution were added to the reaction mixture, followed by extraction with ethyl acetate. The organic layer was dried over anhydrous magnesium sulfate, followed by filtration. The solvent was distilled off under reduced pressure, and the obtained residue was washed with diisopropyl ether, to obtain 1.4 g of the desired methyl 2-(2-(aza(6-methyl-4-phenyl(3H-2,5,6-thiadiazinylidene)methyl)phenyl)acetate as colorless crystals.

Melting point: 85-86°C

EXAMPLE 18

Preparation of methyl 2-(2-(aza(5-methyl-3-phenyl(2,4,5-thiadiazolinylidene))methyl)phenyl)acetate (compound IV-10 of the present invention)

8 g (31.6 mmol) of methyl 2-(2-(((aminomethylamino)thioxomethyl)amino)phenyl)acetate obtained in Example 17 was dissolved in 20 ml of pyridine, and the mixture was cooled to 0°C. Then, 4.44 g (31.6 mmol) of benzoyl chloride was added. The reaction mixture was heated to room temperature and further stirred at room temperature for 2 hours. Then, the solvent was distilled off under reduced pressure. To the obtained residue, 50 ml of water and 1N hydrochloric acid were added to bring the pH to 3, followed by extraction with ethyl acetate. The organic layer was dried over anhydrous magnesium sulfate, followed by filtration. Then, the solvent was distilled off under reduced pressure, and the obtained residue was washed with diisopropyl ether to obtain 8.1 g of methyl 2-(2-(((methyl(phenylcarbonylamino) amino)thioxomethyl)amino)-phenyl)acetate as colorless crystals.

Melting point: 148-150°C

Then, 5.36 g (15.4 mmol) of the obtained methyl 2-(2-(((methyl(phenylcarbonylamino)amino)thioxomethyl)-amino)phenyl)acetate was gradually added to 25 ml of concentrated sulfuric acid. The mixture was stirred at room temperature for 2 hours and then poured into 20 ml of ice water, followed by extraction with ethyl acetate. The organic layer was dried over anhydrous magnesium sulfate, followed by filtration. The solvent was distilled off under reduced pressure, and the obtained residue was washed with diisopropyl ether to obtain 3 g of the desired methyl 2-(2-(aza (5-methyl-3-phenyl(2,4,5-thiadiazolinylidene))methyl)phenyl)acetate as colorless crystals.

Melting point: 76-77°C

EXAMPLE 19

Preparation of methyl 2-(2-(aza(5-methyl-3-phenyl(2,4,5-oxadiazolinylidene))methyl)phenyl)acetate (compound IV-9 of the present invention)

3.8 g (10.6 mmol) of methyl 2-(2-(((methyl(phenylcarbonylamino)amino)thioxomethyl)amino)-phenyl)acetate obtained in Example 18, was dissolved in acetone. Then, 35 ml of a 1N potassium carbonate aqueous solution and 2 ml of ethyl iodide were added, followed by stirring at 60°C for 2 hours. Then, the solvent was distilled off under reduced pressure. 20 ml of water was added to the residue, and precipitated crystals were collected by filtration. The obtained crystals were dissolved in ethyl acetate and dried over anhydrous magnesium sulfate, followed by filtration. The solvent was distilled off under reduced pressure. The obtained residue was washed with diisopropyl ether to obtain 2.4 g of the desired methyl 2-(2-(aza(5-methyl-3-phenyl(2,4,5-oxadiazolinylidene))methyl)phenyl)acetate as colorless crystals.

Melting point: 97-99°C

EXAMPLE 20

Preparation of methyl 2-(2-(1-aza-2-(dimethylamino)-2-(2-(2-chlorophenyl)-2-oxoethylthio)vinyl)phenyl)acetate (compound VII-15)

0.4 g (1.59 mmol) of methyl 2-(2-(((dimethylamino)thioxomethyl)amino)phenyl)acetate was dissolved in 12 ml of 1,4-dioxane, and 0.37 g (1.59 mmol) of 2-chlorophenacyl bromide was added thereto. After stirring the mixture at room temperature for 6 hours, precipitated crystals were collected by filtration and washed with diethyl ether. The obtained crystals were dissolved in 10 ml of a 1N sodium hydroxide aqueous solution, followed by extraction with ethyl acetate. The organic layer was dried over anhydrous magnesium sulfate. After filtration, the solvent was distilled off under reduced pressure, to obtain 0.38g of methyl 2-(2-(1-aza-2-(dimethylamino)-2-(2-(2-chlorophenyl)-2-oxoethylthio) vinyl)phenyl)acetate as a colorless oil.

Refractive index: $n_{D21.3}$1.5364

EXAMPLE 21

Preparation of methyl 2-(2-(aza(3-(2-methoxyphenyl)(2,5-oxathiolenylidene)methyl)phenyl)acetate (compound IV-16 of the present invention)

1 g (3.96 mmol) of methyl 2-(2-(((dimethylamino)thioxomethyl)amino)phenyl)acetate was dissolved in 20 ml of 1,4-dioxane, and 0.91 g (3.96 mmol) of 2-methoxyphenacyl bromide was added thereto. After carrying out heating and refluxing for 20 hours, 1,4-dioxane was distilled off under reduced pressure. The obtained reaction mixture was dissolved in 10 ml of a 1N sodium hydroxide aqueous solution, followed by extraction with ethyl acetate. Then, the organic layer was dried over anhydrous magnesium sulfate. After filtration, the solvent was distilled off under reduced pressure. The obtained residue was purified by silica gel chromatography (ethyl acetate:hexane=1:4) to obtain 0.9 g of the desired methyl 2-(2-(aza(3-(2-methoxyphenyl)(2,5-oxathiolenylidene)methyl)phenyl)acetate as a colorless oil.

Refractive index: $n_{D21.7}$1.5262

EXAMPLE 22

Preparation of methyl 2-(2-(aza(3-(2-methylpropyl)(2,5-oxathiolenylidene)methyl)phenyl)acetate (compound IV-29 of the present invention)

1.04 g (5 mmol) of methyl (2-isothiocyanatephenyl)acetate and 3 g (44 mmol) of imidazole were dissolved in 30 ml of dioxane, and 1.8 g (10 mmol) of 1-bromo-4-methyl-2-pentanone was added, followed by stirring at room temperature for 30 minutes. To this reaction solution, 0.9 g (5 mmol) of 1-bromo-4-methyl-2-pentanone was further added, followed by stirring for 30 minutes, and this operation was carried out twice. Then, the reaction solution was stirred for 3 hours under heating and refluxing, and then returned to room temperature. The solvent was distilled off under reduced pressure. To the obtained residue, a 1N sodium hydroxide aqueous solution was added, followed by extraction with ethyl acetate. The organic layer was washed with a saturated sodium chloride aqueous solution and then dried over anhydrous magnesium sulfate, followed by filtration. Then, the solvent was distilled off under reduced pressure. The obtained residue was purified twice with silica gel column chromatography (n-hexane:ethyl acetate=4: 1, chloroform) to obtain 0.3 g of the desired methyl 2-(2-(aza(3-(2-methylpropyl) (2,5-oxathiolenylidene)methyl)phenyl) acetate as a colorless oil.

Refractive index: $n_{D21.5}$1.5630

EXAMPLE 23

Preparation of methyl 2-(2-(aza(2-methyl-3,5-dimethoxy-2,6-pyrimidinylidene)methyl)phenyl)acetate (compound V-4 of the present invention)

0.45 g (3.03 mmol) of trimethyloxonium tetrafluoroborate was suspended in 15 ml of 1,2-dichloroethane, and 0.53 g (3.03 mmol) of 2-chloro-4,6-diemethoxypyrimidine was added thereto. This mixture was heated and stirred at 60°C for 1 hour and then cooled to room temperature. Further, 0.5 g (3.03 mmol) of methyl 2-aminophenyl acetate dissolved in 2 ml of 1,2-dichloroethane, was added, followed by stirring at room temperature for 2.5 hours. To the reaction mixture, 50 ml of a saturated sodium hydrogencarbonate aqueous solution was added, followed by extraction with chloroform. Then, the organic layer was dried over anhydrous sodium sulfate. After filtration, the solvent was distilled off, and the obtained residue was purified by high performance liquid chromatography (acetonitrile:water=4:

1) to obtain 60 mg of methyl 2-(2-(aza(2-methyl-3,5-dimethoxy-2,6-pyrimidinylidene)methyl)phenyl)acetate as colorless crystals.

Melting point: 122-123°C

EXAMPLE 24

Preparation of methyl 2-(2-(aza(2-methyl-3-fluoro-2-pyridinylidene)methyl)phenyl)acetate (compound V-5 of the present invention)

0.45 g (3.03 mmol) of trimethyloxonium tetrafluoroborate was suspended in 15 ml of 1,2-dichloroethane, and 0.35 g (3.03 mmol) of 2,6-difluoropyridine was added thereto. This mixture was heated and stirred at 60°C for 2 hours and then cooled to room temperature, and 0.5 g (3.03 mmol) of methyl 2-aminophenyl acetate dissolved in 2 ml of 1,2-dichloroethane, was added, followed by stirring at room temperature for 6 days. To the reaction mixture, 50 ml of a saturated sodium hydrogencarbonate aqueous solution was added, followed by extraction with chloroform. The organic layer was dried over anhydrous sodium sulfate. After filtration, the solvent was distilled off, and the obtained residue was purified by high performance liquid chromatography (acetonitrile:water=4:1) to obtain 80 mg of methyl 2-(2-(aza(2-methyl-3-fluoro-2-pyridinylidene)methyl)phenyl)acetate as an orange colored oil.

Refractive index: $n_{D21.7}$1.5286

EXAMPLE 25

Preparation of methyl 2-(2-(aza(6-methyl-5-phenyl(2H-3,6-thiadinylidene))methyl)phenyl)acetate (compound V-9 of the present invention)

10 g (0.11 mol) of thioglycolamide and 11.2 g (0.11 mol) of triethylamine were suspended in methyl ethyl ketone (100 ml), and 22 g (0.11 mol) of phenacyl bromide was added under cooling with ice. The reaction solution was heated and refluxed for 7 hours. The solution was left to cool to room temperature, and then, the solvent was distilled off under reduced pressure. Water was added thereto, followed by extraction with ethyl acetate. The organic layer was washed with a saturated sodium chloride aqueous solution and dried over anhydrous magnesium sulfate, followed by filtration. The solvent was distilled off under reduced pressure. The residue was crystallized from a mixed solution of diisopropyl ether and diethyl ether, followed by filtration under reduced pressure and drying, to obtain 20.6 g (0.1 mol) of phenacyl thioacetamide.

Melting point: 106-107°C

To a suspension solution of 20.4 g (0.1 mol) of the obtained phenacyl thioacetamide in 80 ml of toluene, a catalytic amount of p-toluenesulfonic acid was added, followed by heating and refluxing for 60 hours while removing water. The solution was left to cool to room temperature, and then, precipitated crystals were washed with toluene and diethyl ether, followed by drying under reduced pressure to obtain 17.4 g (91 mmol) of 5-phenyl-2H-1,4-thiazin-3(4H)-one.

Melting point: 150-155°C

Then, 13 g (68 mmol) of the obtained 5-phenyl-2H-1,4-thiazin-3(4H)-one was dissolved in 300 ml of acetonitrile, and 14.1 g (138 mmol) of potassium carbonate and 38.7 g (273 mmol) of methyl iodide were added, followed by heating and refluxing for 30 hours. The reaction mixture was cooled, and the formed precipitate was removed by filtration. The filtrate was concentrated to dryness. The obtained residue was separated to chloroform and water, and a syrup obtained by concentrating the organic layer, was crystallized from diisopropyl ether. The crystals were collected by filtration, followed by drying under reduced pressure to obtain 8 g (39 mmol) of 4-methyl-5-phenyl-2H-1,4-thiazin-3(4H)-one.

Melting point: 95-97°C

Then, 2.7 g (13.2 mmol) of the obtained 4-methyl-5-phenyl-2H-1,4-thiazin-3(4H)-one was dissolved in 50 ml of 1,4-dioxane, and 3.9 g (9.6 mmol) of a Lawesson's Reagent was added thereto, followed by stirring at 70°C for 5 hours. The mixture was left to cool to room temperature, and insolubles were filtered off. The solvent was distilled off under reduced pressure. The obtained residue was crystallized from a small amount of ethanol. The crystals were collected by filtration and dried under reduced pressure to obtain 2.3 g (10.4 mmol) of 4-methyl-5-phenyl-2H-1,4-thiazin-3(4H)-thione.

Melting point: 83-85°C

Then, 2 g (9 mmol) of the obtained 4-methyl-5-phenyl-2H-1,4-thiazin-3(4H)-thione was dissolved in acetone (20 ml), and 4 g (28 mmol) of methyl iodide was added, followed by heating and refluxing for 9 hours. The mixture was left to cool to room temperature, and the precipitate was collected by filtration and washed with diisopropyl ether, followed by drying under reduced pressure, to obtain 2.4 g (6.6 mmol) of 4-methyl-3-methylthio-5-phenyl-2H-1,4-thiazinium iodide.

Melting point: 140-145°C (decomposed)

Then, 0.34 g (2.1 mmol) of methyl 2-aminophenyl acetate was dissolved in 10 ml of 1,2-dichloroethane, and 0.5 g (1.4 mmol) of the obtained 4-methyl-3-methylthio-5-phenyl-2H-1,4-thiadinium iodide was added, followed by stirring at room temperature for 3 hours. After completion of the reaction, a 1N sodium hydroxide aqueous solution was added, followed by extraction with chloroform. The organic layer was washed with a saturated sodium chloride aqueous solution and dried over anhydrous magnesium sulfate, followed by filtration. The solvent was distilled off under reduced pressure. The residue was purified by silica gel column chromatography (n-hexane:ethyl acetate-2:1) to obtain 0.36 g (1 mmol) of the desired methyl 2-(2-(aza(6-methyl-5-phenyl(2H-3,6-thiadinylidene))methyl)phenyl)acetate.

Refractive index: $n_{D20.4}$1.6251

EXAMPLE 26

Preparation of methyl 2-(2-(aza(4-(azaphenylmethylene)-5-methyl(2,5-thiazolidinylidene))methyl)phenyl)acetate (compound IV-70 of the present invention)

1.40 g (15.0 mmol) of aniline was dissolved in 40 ml of tetrahydrofuran, and a solution having 2.50 g (15.9 mmol) of bromoacetyl chloride dissolved in 1 ml of tetrahydrofuran, and a solution having 1.72 g (17.0 mmol) of triethylamine dissolved in 2 ml of tetrahydrofuran, were added thereto at room temperature, followed by stirring for 4 hours. The reaction mixture was poured into 1N hydrochloric acid, and extracted with ethyl acetate. The organic layer was washed with a 1N sodium hydroxide aqueous solution and then with a saturated sodium chloride aqueous solution, and then dried over anhydrous sodium sulfate, followed by filtration. The solvent was distilled off under reduced pressure, to obtain 3.01 g of 2-bromo-N-phenylacetamide.

1.59 g (7.43 mmol) of the obtained 2-bromo-N-phenylacetamide, was dissolved in 30 ml of tetrahydrofuran, and 1.61 g (6.76 mmol) of methyl 2-(2-(((methylamino)thioxomethyl)amino)phenyl)acetate was added thereto at room temperature, followed by stirring for 4 hours and 30 minutes. A salt precipitated during the reaction was collected by filtration to obtain 2.62 g of methyl 2-(2-(1-aza-2-(methylamino)-2-(2-anilino-2-oxoethylthio)vinyl)phenyl)acetate hydrobromide.

Then, 2.62 g (5.79 mmol) of the obtained methyl 2-(2-(1-aza-2-(methylamino)-2-(2-anilino-2-oxoethylthio)vinyl)phenyl)acetate hydrobromide, was dissolved in 30 ml of 1,2-dichloroethane. Then, a solution having 0.66 g (6.52 mmol) of triethylamine dissolved in 1 ml of 1,2-dichloroethane, a solution having 2.00 g (13 nunol) of carbon tetrachloride dissolved in 1 ml of 1,2-dichloroethane, and 3.66 g (12.8 mmol) of triphenylphosphine, were added thereto at room temperature, followed by stirring for 24 hours. To this reaction mixture, water was added, followed by extraction with chloroform. The organic layer was washed with a saturated sodium chloride aqueous solution and dried over anhydrous sodium sulfate, followed by filtration. Then, the solvent was distilled off under reduced pressure. The obtained residue was purified by silica gel column chromatography (n-hexane:ethyl acetate=2:1), to obtain 0.10 g of the desired methyl 2-(2-(aza(4-(azaphenylmethylene)-5-methyl(2,5-thiazolidinylidene))methyl)phenyl)acetate.

Refractive index: $n_{D20.7}$1.5326

REFERENCE EXAMPLE 1

Preparation of methyl 2-(2-(((methylamino)thioxomethyl)amino)phenyl)acetate

18 g (0.1 mol) of 2-nitrophenylacetic acid was dissolved in 400 ml of methanol, and 5 ml of concentrated sulfuric acid was added thereto, followed by heating and refluxing for 3 hours. Methanol was distilled off, and then, 100 ml of ice water was added, followed by extraction with ethyl acetate. The organic layer was washed with a saturated sodium hydrogencarbonate aqueous solution and dried over anhydrous magnesium sulfate. After filtration, the solvent was distilled off to obtain 20 g of methyl 2-nitrophenyl acetate as a colorless oil.

Then, 15 g (75 mmol) of the obtained methyl 2-nitrophenyl acetate was dissolved in methanol, and 0.5 g of 5% palladium-active carbon was added thereto, followed by stirring for 3 hours at room temperature in a hydrogen atmosphere. The palladium-active carbon was filtered off, and then, methanol was distilled off under reduced pressure, to obtain 12.3 g of methyl 2-aminophenyl acetate as a slightly yellow oil.

Then, 7 g (42.4 mmol) of the obtained methyl 2-aminophenyl acetate was dissolved in 40 ml of anhydrous tetrahydrofuran, and 3.1 g (42.4 mmol) of methyl isothiocyanate, 4.3 g (42.4 mmol) of triethylamine and 0.5 g of 4-dimethylaminopyridine, were added thereto, followed by stirring at room temperature. 96 Hours later, the solvent was distilled off under reduced pressure, and 50 ml of water was added to the obtained reaction mixture, followed by extraction with ethyl acetate. The organic layer was washed with 1N hydrochloric acid, and then, dried over anhydrous magnesium sulfate. After filtration, the solvent was distilled off under reduced pressure, and the obtained residue was

washed with a solvent mixture of diethyl ether and diisopropyl ether, to obtain 6.5 g of methyl 2-(2-(((methylamino) thioxomethyl)amino)phenyl)-acetate as colorless crystals.

Melting point: 99-100°C

REFERENCE EXAMPLE 2

Preparation of triethylammonium 2-(2-methoxy-2-oxoethyl)phenylcarbamodithioate

A solution of 60.4 g (0.37 mol) of methyl (2-aminophenyl)acetate in benzene (120 ml), was cooled with ice, and 27.9 g (0.37 mol) of carbon disulfide and 37.1 g (0.37 mol) of triethylamine were added. The mixture was left to stand still for 1 week in a refrigerator. The reaction solution was returned to room temperature, and 200 ml of diethyl ether was added, followed by stirring for 30 minutes. Precipitated crystals were sedimented, and the supernatant was removed. 200 ml of diethyl ether was added again, followed by stirring for 30 minutes. Crystals were collected by filtration under reduced pressure, and subjected to drying under reduced pressure, to obtain 117 g of the desired triethylammonium 2-(2-methoxy-2-oxoethyl)phenylcarbamodithioate as slightly yellow crystals.

REFERENCE EXAMPLE 3

Preparation of methyl (2-isothiocyanate phenyl)acetate

A mixed solution comprising 90 g (0.26 mol) of triethylammonium 2-(2-methoxy-2-oxoethyl)phenylcarbamodithioate, 26.6 g (0.26 mol) of triethylamine and 200 ml of chloroform, was cooled with ice, and 28.6 g (0.26 mol) of ethyl chloroformate was added. The reaction solution was returned to room temperature, followed by stirring for further 1.5 hours. To the reaction solution, 300 ml of a 1N sodium hydroxide aqueous solution was added. The organic layer and the aqueous layer were separated. The aqueous layer was extracted with chloroform, and the extract was added to the previous organic layer. This organic layer was washed with a saturated sodium chloride aqueous solution, dried over anhydrous sodium sulfate, and then subjected to filtration. The solvent was distilled off under reduced pressure, to obtain 55.8 g of the desired methyl (2-isothiocyanate phenyl)acetate as a slightly yellow oil.

Refractive index: $n_{D21.4}$1.5032

REFERENCE EXAMPLE 4

Preparation of methyl (2-(((methylimino)methylene)amino)phenyl)acetate

2.38 g (10 mmol) of methyl 2-(2-(((methylamino)thioxomethyl)amino)phenyl)acetate, 3.03 g (30 mmol) of triethylamine and 50 mg (0.4 mmol) of 4-(dimethylamino)pyridine, were dissolved in 100 ml of chloroform, and 2.29 g (20 mmol) of methanesulfonyl chloride was dropwise added thereto at a temperature of at most 25°C. After stirring for 1 hour at room temperature, the reaction solution was washed with water, dried over anhydrous sodium sulfate and then subjected to filtration. The solvent was distilled off under reduced pressure. The obtained residue was purified by silica gel column chromatography (chloroform), to obtain 1.5 g of the desired methyl (2-(((methylimino)methylene) amino)phenyl)acetate as a slightly yellow oil.

Refractive index: $n_{D21.4}$1.5032

REFERENCE EXAMPLE 5

Preparation of methyl (2-(((dimethylamino)thioxomethyl)amino)phenyl)acetate

A solution of 5 g (24.2 mmol) of methyl (2-isothiocyanate phenyl) acetate in 50 ml of tetrahydrofuran, was cooled with ice, and 2.8 g (24.8 mmol) of dimethylamine (40% aqueous solution) was added. The reaction solution was returned to room temperature, followed by stirring for further 30 minutes. Then, the solvent was distilled off under reduced pressure, and the precipitated crystals were washed with diethyl ether and dried under reduced pressure, to obtain 4.9 g of the desired methyl (2-(((dimethylamino)thioxomethyl)amino)phenyl)acetate as colorless crystals.

Melting point: 110-113°C

Now, the physical properties, etc. of compounds of the formula (1) prepared in accordance with these methods, are shown in Tables 9 to 16. Here, abbreviations in the Tables, are as mentioned above, or T1 to T8 represent the followings.

T1: 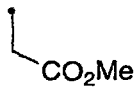

T2-1: 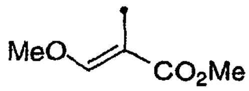

T2-2: 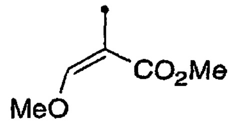

T3-1: 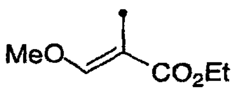

T3-2: 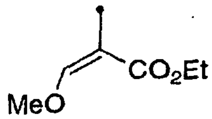

T4-1: 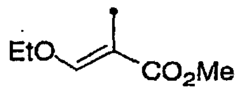

T4-2: 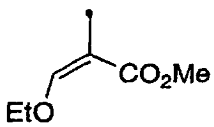

T5: 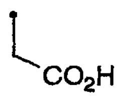

T6: 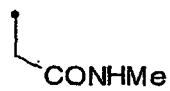

T7: 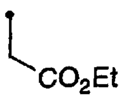

T8: 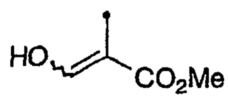

(Table 9)

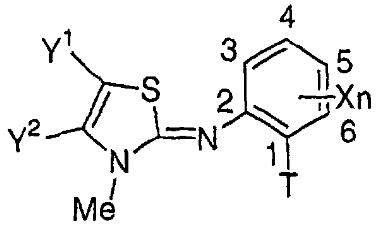

| No. | $Y^1$ | $Y^2$ | T | X | Physical properties (m.p. etc.) | Notes |
|---|---|---|---|---|---|---|
| I-1 | H | Ph | T1 | H | $n_D^{21.5}$ 1.5600 | |
| I-2 | H | 2-Cl-Ph | T1 | H | m.p.112-113℃ | |
| I-3 | H | 3-Cl-Ph | T1 | H | $n_D^{22.1}$ 1.4906 | |
| I-4 | H | 4-Cl-Ph | T1 | H | m.p.73-74℃ | |
| I-5 | H | 2-MeO-Ph | T1 | H | m.p.86-89℃ | |
| I-6 | H | 3-MeO-Ph | T1 | H | $n_D^{21.3}$ 1.4859 | |
| I-7 | H | 4-MeO-Ph | T1 | H | m.p.82-83℃ | |
| I-8 | H | 2-Me-Ph | T1 | H | m.p.110-112℃ | |
| I-9 | H | 3-Me-Ph | T1 | H | $n_D^{21.5}$ 1.5391 | |
| I-10 | H | 4-Me-Ph | T1 | H | $n_D^{21.2}$ 1.5282 | |
| I-11 | H | 2-F-Ph | T1 | H | m.p.102.5-104.5℃ | |
| I-12 | H | 3-F-Ph | T1 | H | $n_D^{21.5}$ 1.5762 | |
| I-13 | H | 4-F-Ph | T1 | H | m.p.75-76℃ | |
| I-14 | H | 4-$NO_2$-Ph | T1 | H | m.p.125-127℃ | |
| I-15 | H | 4-Ph-Ph | T1 | H | $n_D^{22.1}$ 1.5154 | |
| I-16 | H | 4-$CF_3$-Ph | T1 | H | $n_D^{21.9}$ 1.5115 | |
| I-17 | H | 3-CN-Ph | T1 | H | m.p.137-141℃ | |
| I-18 | H | 2-Naphthyl | T1 | H | m.p.95-96℃ | |
| I-19 | H | 2-Thienyl | T1 | H | $n_D^{21.8}$ 1.4598 | |
| I-20 | H | 2-Furyl | T1 | H | $n_D^{21.8}$ 1.5382 | |
| I-21 | H | 3-Pyridyl | T1 | H | m.p.85-87℃ | |
| I-22 | H | H | T1 | H | $n_D^{20.2}$ 1.5187 | |
| I-23 | H | Me | T1 | H | $n_D^{20.2}$ 1.5172 | |
| I-24 | H | MeOC(=O) | T1 | H | $n_D^{20.2}$ 1.5393 | |
| I-25 | H | t-Bu | T1 | H | $n_D^{21.8}$ 1.4629 | |
| I-26 | H | MeC(=NOMe)- | T1 | H | m.p.48-50℃ | |
| I-27 | H | Et | T1 | H | $n_D^{21.6}$ 1.6100 | |
| I-28 | H | i-Pr | T1 | H | $n_D^{21.0}$ 1.5982 | |
| I-29 | H | $CF_3$ | T1 | H | $n_D^{21.7}$ 1.5528 | |
| I-30 | H | $ClCH_2$ | T1 | H | $n_D^{21.8}$ 1.6074 | |
| I-31 | H | $MeSCH_2$ | T1 | H | $n_D^{21.1}$ 1.6315 | |
| I-32 | MeCO | Me | T1 | H | $n_D^{20.2}$ 1.5126 | |
| I-33 | Me | Me | T1 | H | $n_D^{21.7}$ 1.6084 | |
| I-34 | H | Ph | T5 | H | m.p.143-144℃ | |
| I-35 | H | Ph | T6 | H | m.p.123-125℃ | |
| I-36 | H | Ph | T7 | H | $n_D^{20.0}$ 1.4620 | |
| I-37 | H | Ph | T2-1 | H | m.p.118-120℃ | |
| I-38 | H | Ph | T2-2 | H | m.p.141-143℃ | |
| I-39 | H | Ph | T3-1 | H | $n_D^{22.0}$ 1.4908 | |
| I-40 | H | Ph | T3-2 | H | $n_D^{22.0}$ 1.5098 | |
| I-41 | H | Ph | T4-1 | H | $n_D^{22.0}$ 1.5902 | |
| I-42 | H | Ph | T4-2 | H | $n_D^{22.0}$ 1.5005 | |
| I-43 | H | 2-Cl-Ph | T2-1 | H | m.p.166-167℃ | |
| I-44 | H | 2-Cl-Ph | T2-2 | H | m.p.133-134℃ | |

| | | | | | |
|---|---|---|---|---|---|
| I-45 | H | 3-Cl-Ph | T2-1 | H | m.p.115-116℃ |
| I-46 | H | 3-Cl-Ph | T2-2 | H | m.p.133-134℃ |
| I-47 | H | 4-Cl-Ph | T2-1 | H | m.p.154-156℃ |
| I-48 | H | 4-Cl-Ph | T2-2 | H | m.p.136-138℃ |
| I-49 | H | 4-MeO-Ph | T2-1,2mixture | H | m.p.151-153℃ |
| I-50 | H | 4-Me-Ph | T2-1 | H | Viscous oil |
| I-51 | H | 4-Me-Ph | T2-2 | H | Viscous oil |
| I-52 | H | 4-F-Ph | T2-1 | H | Viscous oil |
| I-53 | H | 4-F-Ph | T2-2 | H | Viscous oil |
| I-54 | H | 4-Ph-Ph | T2-1 | H | m.p.71-75℃ |
| I-55 | H | 4-$CF_3$-Ph | T2-1 | H | m.p.169-171℃ |
| I-56 | H | 4-$CF_3$-Ph | T2-2 | H | m.p.131-132℃ |
| I-57 | H | 2-Naphthyl | T2-1 | H | m.p.136-138℃ |
| I-58 | H | 2-Naphthyl | T2-2 | H | Oil |
| I-59 | H | 2-Thienyl | T2-1 | H | Oil |
| I-60 | H | H | T2-1 | H | m.p.115-117℃ |
| I-61 | H | H | T2-2 | H | m.p.109-111℃ |
| I-62 | H | Me | T2-1 | H | m.p.122-123℃ |
| I-63 | H | Me | T2-2 | H | m.p.108-109℃ |
| I-64 | H | t-Bu | T2-1 | H | m.p.120-121℃ |
| I-65 | H | t-Bu | T2-2 | H | m.p.118-120℃ |
| I-66 | H | MeC(=NOMe)- | T2-1 | H | $n_D^{21.9}$1.4912 |
| I-67 | H | MeC(=NOMe)- | T2-2 | H | $n_D^{22.0}$1.4876 |
| I-68 | H | Et | T2-1 | H | m.p.78-80℃ |
| I-69 | H | Et | T2-2 | H | $n_D^{21.5}$1.6154 |
| I-70 | H | i-Pr | T2-1 | H | $n_D^{21.7}$1.5772 |
| I-71 | H | i-Pr | T2-2 | H | m.p.104-106℃ |
| I-72 | H | $CF_3$ | T2-1 | H | $n_D^{21.3}$1.5638 |
| I-73 | H | $CF_3$ | T2-2 | H | $n_D^{21.6}$1.5507 |
| I-74 | Me | H | T2-1 | H | m.p.100-101℃ |
| I-75 | Me | H | T2-2 | H | m.p.125-127℃ |
| I-76 | Me | Me | T2-1 | H | m.p.162-163℃ |
| I-77 | Me | Me | T2-2 | H | m.p.148-152℃ |
| I-78 | H | 2-Thiazolyl | T1 | H | m.p.93-94℃ |
| I-79 | H | 2,3-$Cl_2$-Ph | T1 | H | Viscous oil |
| I-80 | H | 3,5-$Cl_2$-Ph | T1 | H | $n_D^{21.3}$1.4874 |
| I-81 | H | 2,6-$Cl_2$-Ph | T1 | H | m.p.126-128℃ |
| I-82 | H | 2,5-$Cl_2$-Ph | T1 | H | m.p.92-93.5℃ |
| I-83 | H | 2,4-$Cl_2$-Ph | T1 | H | m.p.116.5-117.5℃ |
| I-84 | H | 3,4-$Cl_2$-Ph | T1 | H | $n_D^{21.2}$1.5676 |
| I-85 | H | 2,3-$F_2$-Ph | T1 | H | m.p.60-61℃ |
| I-86 | H | 3,4-$F_2$-Ph | T1 | H | $n_D^{21.0}$1.5595 |
| I-87 | H | 2,6-$F_2$-Ph | T1 | H | m.p.137.5-138.5℃ |
| I-88 | H | 2,5-$F_2$-Ph | T1 | H | m.p.95-96℃ |
| I-89 | H | 2,4-$F_2$-Ph | T1 | H | m.p.96-98℃ |
| I-90 | H | 2-Pyrazyl | T1 | H | m.p.84-86℃ |
| I-91 | H | 2-Benzofuranyl | T1 | H | $n_D^{20.5}$1.5170 |
| I-92 | H | 4-Br-Ph | T1 | H | m.p.120-122℃ |
| I-93 | H | 4-Et-Ph | T1 | H | $n_D^{20.7}$1.5065 |
| I-94 | H | 4-PhO-Ph | T1 | H | $n_D^{20.7}$1.4881 |
| I-95 | H | 2-$CF_3$-Ph | T1 | H | $n_D^{20.6}$1.5355 |
| I-96 | H | 4-$CF_3$O-Ph | T1 | H | $n_D^{21.3}$1.5178 |
| I-97 | H | 2-F-6-$CF_3$-Ph | T1 | H | m.p.99.5-101℃ |
| I-98 | H | 3-(3-Cl-$PhCH_2O$)-Ph | T1 | H | $n_D^{21.4}$1.5141 |
| I-99 | H | 1-Me-3-Cl-5-Pyrazolyl | T1 | H | m.p.89-91℃ |
| I-100 | Cl | Ph | T1 | H | $n_D^{21.5}$1.5748 |
| I-101 | Ph | Ph | T1 | H | m.p.116-118℃ |
| I-102 | Me | Ph | T1 | H | m.p.89-91℃ |

| | | | | | | |
|---|---|---|---|---|---|---|
| I-103 | $Me_2NCH_2$ | Ph | T1 | H | $n_D^{21.3}$ 1.4743 | |
| I-104 | H | 2-Cl-Ph | T8 | H | m.p. 143-144℃ | |
| I-105 | H | 2-MeO-Ph | T2-1 | H | m.p. 130-131℃ | |
| I-106 | H | 2-MeO-Ph | T2-2 | H | m.p. 129-131.5℃ | |
| I-107 | H | 3-MeO-Ph | T2-1 | H | m.p. 127-130℃ | |
| I-108 | H | 3-MeO-Ph | T2-2 | H | m.p. 109-113℃ | |
| I-109 | H | 2-Me-Ph | T2-1 | H | m.p. 111-113℃ | |
| I-110 | H | 2-Me-Ph | T2-2 | H | m.p. 139.5-142℃ | |
| I-111 | H | 3-Me-Ph | T2-1 | H | m.p. 117.5-119℃ | |
| I-112 | H | 3-Me-Ph | T2-2 | H | m.p. 129-131℃ | |
| I-113 | H | 2-F-Ph | T2-1 | H | m.p. 111-111.5℃ | |
| I-114 | H | 2-F-Ph | T2-2 | H | m.p. 128-129℃ | |
| I-115 | H | 3-F-Ph | T2-1 | H | m.p. 137-138℃ | |
| I-116 | H | 3-F-Ph | T2-2 | H | m.p. 136-139℃ | |
| I-117 | H | 4-PhO-Ph | T2-1 | H | m.p. 132.5-134.5℃ | |
| I-118 | H | 4-PhO-Ph | T2-2 | H | Viscous oil | |
| I-119 | H | 4-Br-Ph | T2-1 | H | m.p. 179-180.5℃ | |
| I-120 | H | 4-Et-Ph | T2-1 | H | m.p. 93-95℃ | |
| I-121 | H | 3-Pyridyl | T2-1 | H | m.p. 125-128℃ | |
| I-122 | H | 3-Pyridyl | T2-2 | H | m.p. 122-125.5℃ | |
| I-123 | H | 2-Furyl | T2-1 | H | Viscous oil | |
| I-124 | H | 2-Thiazolyl | T2-1 | H | Viscous oil | |
| I-125 | H | $2-CF_3-Ph$ | T2-1 | H | m.p. 150-151℃ | |
| I-126 | H | $3-(3-Cl-PhCH_2O)-Ph$ | T2-1 | H | m.p. 91-94℃ | |
| I-127 | H | $2,3-Cl_2-Ph$ | T2-1 | H | m.p. 176-178℃ | |
| I-128 | H | $2,3-Cl_2-Ph$ | T2-2 | H | m.p. 130-131℃ | |
| I-129 | H | $3,5-Cl_2-Ph$ | T2-1 | H | Viscous oil | |
| I-130 | H | $3,5-Cl_2-Ph$ | T2-2 | H | Viscous oil | |
| I-131 | H | $2,6-Cl_2-Ph$ | T2-1 | H | m.p. 165-169℃ | |
| I-132 | H | $2,6-Cl_2-Ph$ | T2-2 | H | Viscous oil | |
| I-133 | H | $2,5-Cl_2-Ph$ | T2-1 | H | m.p. 173-175℃ | |
| I-134 | H | $2,5-Cl_2-Ph$ | T2-2 | H | Viscous oil | |
| I-135 | H | $2,6-F_2-Ph$ | T2-1 | H | m.p. 136-139℃ | |
| I-136 | H | 2-F-6-MeO-Ph | T2-1 | H | m.p. 145-148℃ | |
| I-137 | Cl | Ph | T2-1 | H | m.p. 132-134℃ | |
| I-138 | Cl | Ph | T2-2 | H | Viscous oil | |
| I-139 | Me | Ph | T2-1 | H | m.p. 151.5-153℃ | |
| I-140 | Me | Ph | T2-2 | H | m.p. 127.5-129℃ | |
| I-141 | Ph | Ph | T2-1 | H | m.p. 134.5-136.5℃ | |
| I-142 | Ph | Ph | T2-2 | H | m.p. 180-182℃ | |
| I-143 | H | Bu | T1 | H | $n_D^{21.5}$ 1.5947 | |
| I-144 | H | i-Bu | T1 | H | $n_D^{21.6}$ 1.5882 | |
| I-145 | H | s-Bu | T1 | H | $n_D^{21.5}$ 1.5916 | |
| I-146 | H | Hex | T1 | H | $n_D^{21.3}$ 1.5799 | |
| I-147 | H | c-Hex | T1 | H | $n_D^{21.3}$ 1.5872 | |
| I-148 | H | $C_2F_5$ | T1 | H | $n_D^{21.5}$ 1.5290 | |
| I-149 | H | $(2,4-Cl_2-PhO)CH_2$ | T1 | H | m.p. 104-105℃ | |
| I-150 | H | Pyrrolidino-$CH_2$ | T1 | H | m.p. 82-84℃ | |
| I-151 | H | $PhC(CF_3)=N-OCH_2$ | T1 | H | $n_D^{20.6}$ 1.5817 | |
| I-152 | H | $PhN(Me)CH_2$ | T1 | H | Oil | |
| I-153 | H | PhCH=CH | T1 | H | Oil | |
| I-154 | H | $Ph_3PCH_2$ | T1 | H | m.p. 170℃< | Cl salt |
| I-155 | H | $Me(4-Cl-PhCH_2O-N=)C$ | T1 | H | $n_D^{20.7}$ 1.6177 | |
| I-156 | 2-F-Ph | Me | T1 | H | m.p. 66-71℃ | |
| I-157 | Ph | Me | T1 | H | m.p. 107-109℃ | |
| I-158 | Ph | Et | T1 | H | m.p. 91-94℃ | |
| I-159 | Me(MeON=)C | Me | T1 | H | m.p. 99-100℃ | |
| I-160 | H | Bu | T2-1 | H | m.p. 111-112℃ | |

| | | | | | |
|---|---|---|---|---|---|
| I-161 | H | Bu | T2-2 | H | Oil |
| I-162 | H | i-Bu | T2-1 | H | $n_{D20.3}$1.5966 |
| I-163 | H | i-Bu | T2-2 | H | m.p.105-107℃ |
| I-164 | H | s-Bu | T2-1 | H | $n_{D20.3}$1.6004 |
| I-165 | H | s-Bu | T2-2 | H | $n_{D20.5}$1.5943 |
| I-166 | H | Hex | T2-1 | H | m.p.79-82℃ |
| I-167 | H | Hex | T2-2 | H | m.p.56-58℃ |
| I-168 | H | $C_2F_5$ | T2-1 | H | Oil |
| I-169 | H | $C_2F_5$ | T2-2 | H | Oil |
| I-170 | H | 2,4-$Cl_2$-PhO$CH_2$ | T2-1 | H | m.p.191-193℃ |
| I-171 | H | 2,4-$Cl_2$-PhO$CH_2$ | T2-2 | H | Oil |
| I-172 | H | Pyrroridino-$CH_2$ | T2-1 | H | m.p.151-154℃ |
| I-173 | H | PhC($CF_3$)=N-O$CH_2$ | T2-1 | H | Oil |
| I-174 | H | PhC($CF_3$)=N-O$CH_2$ | T2-2 | H | Oil |
| I-175 | H | PhN(Me)$CH_2$ | T2-1 | H | Oil |
| I-176 | H | PhN(Me)$CH_2$ | T2-2 | H | Oil |
| I-177 | H | Me(4-Cl-Ph$CH_2$O-N=)C | T2-1 | H | Oil |
| I-178 | H | Me(4-Cl-Ph$CH_2$O-N=)C | T2-2 | H | Oil |
| I-179 | H | c-Pr | T2-1 | H | Oil |
| I-180 | H | c-Pr | T2-2 | H | m.p.101-104℃ |
| I-181 | H | MeS$CH_2$ | T2-1 | H | Oil |
| I-182 | H | MeS$CH_2$ | T2-2 | H | m.p.144-148℃ |
| I-183 | 2-F-Ph | Me | T2-1 | H | Oil |
| I-184 | 2-F-Ph | Me | T2-2 | H | Oil |
| I-185 | Ph | Et | T2-1 | H | m.p.175-177℃ |
| I-186 | Ph | Et | T2-2 | H | m.p.126-129℃ |
| I-187 | H | Ph | T1 | 4-F | m.p.74-76℃ |
| I-188 | H | Ph | T1 | 4-Me | m.p.108-110℃ |
| I-189 | H | Ph | T1 | 5-Me | m.p.105-107℃ |
| I-190 | H | Ph | T1 | 4-Cl | Oil |
| I-191 | H | Ph | T1 | 5-Cl | m.p.57-58℃ |
| I-192 | H | Ph | T1 | 4-$CF_3$ | m.p.150-160℃ HBrsalt |
| I-193 | H | 2,6-$F_2$-Ph | T1 | 4-F | m.p.128-129℃ |
| I-194 | H | Ph | T1 | 4-MeO | m.p.72-73℃ |
| I-195 | H | Ph | T1 | 4-MeO | m.p.176-184℃ HBr salt |
| I-196 | H | 2-Me-Ph | T1 | H | m.p.205-211℃ HBr salt |
| I-197 | H | 2-F-Ph | T1 | H | m.p.191-197℃ HBr salt |
| I-198 | H | i-Pr | T1 | H | m.p.212-217℃ HBr salt |
| I-199 | H | 2-$CF_3$-Ph | T1 | H | m.p.210℃<decomp HBr salt |
| I-200 | H | c-Hex | T1 | H | m.p.208-211℃ HBr salt |
| I-201 | H | c-Pr | T1 | H | m.p.172-174℃ HBr salt |
| I-202 | Br$CH_2$ | H | T1 | H | m.p.180-183℃ HBr salt |
| I-203 | H | 3,5-$F_2$-Ph | T1 | H | $n_{D21.3}$1.5882 |
| I-204 | H | 2,6-$F_2$-Ph | T5 | H | m.p.172-174℃ |
| I-205 | H | 2,6-$F_2$-Ph | T6 | H | m.p.123-126℃ |
| I-206 | H | 2-Pyridyl | T1 | H | m.p.98-99.5℃ |
| I-207 | H | 1-Naphthyl | T1 | H | m.p.121-122℃ |
| I-208 | H | 4-t-Bu-Ph | T1 | H | m.p.129-131℃ |
| I-209 | H | PhCO | T1 | H | $n_{D25.3}$1.5727 |
| I-210 | H | 2,3,4,5,6-$F_5$-Ph | T1 | H | m.p.129-131℃ |
| I-211 | H | 2-F-6-Cl-Ph | T1 | H | m.p.92-93℃ |
| I-212 | H | Ph | T1 | 3-F | m.p.69-70℃ |
| I-213 | H | Me$O_2$C-(MeON=)C | T1 | H | m.p.111-112℃ |
| I-214 | H | Ph-(HON=)C | T1 | H | $n_{D21.8}$1.5154 |
| I-215 | H | Ph-(MeON=)C | T1 | H | $n_{D21.9}$1.5239 |
| I-216 | H | Ph-(Ph$CH_2$ON=)C | T1 | H | $n_{D21.7}$1.5953 |
| I-217 | F | Ph | T1 | H | $n_{D21.7}$1.5117 |
| I-218 | H | 2-F-6-MeO-Ph | T1 | H | m.p.112-114℃ |

| | | | | | |
|---|---|---|---|---|---|
| I-219 | H | 1-Me-6-$F_3C$-2-Pyridon-3-yl | T1 | H | m.p.115-116.5℃ |
| I-220 | H | 2-F-4-$F_3C$-Ph | T1 | H | m.p.71-72℃ |
| I-221 | H | 2-$O_2N$-Ph | T1 | H | m.p.107.5-109℃ |
| I-222 | H | 2,6-$Me_2$-Ph | T1 | H | m.p.133.5-134℃ |
| I-223 | H | 2,5-$F_2$-4-Cl-Ph | T1 | H | m.p.117-119℃ |
| I-224 | H | 4,6-$Cl_2$-2-Pyridyl | T1 | H | Viscous oil |
| I-225 | H | 2,5-$Cl_2$-3-Thienyl | T1 | H | $n_D^{21.7}$1.5356 |
| I-226 | H | 2,5-$Me_2$-Ph | T1 | H | $n_D^{21.7}$1.5582 |
| I-227 | H | 2-($PhCH_2O$)-Ph | T1 | H | m.p.83-84.5℃ |
| I-228 | H | 3,4-Methlyenedioxy-Ph | T1 | H | m.p.69-70.5℃ |
| I-229 | H | Ph-C(Br)=C(Br) | T1 | H | Viscous oil |
| I-230 | H | 2-Cl-Ph | T5 | H | m.p.203-205℃ |
| I-231 | H | $FH_2C$ | T1 | H | Viscous oil |
| I-232 | H | PhC≡C | T1 | H | m.p.85-86℃ |
| I-233 | Br | Ph | T1 | H | $n_D^{21.4}$1.6418 |
| I-234 | H | 3-Br-4-$Me_2N$-Ph | T1 | H | m.p.106.5-108.5℃ |
| I-235 | H | 2-HO-3,4-$Cl_2$-Ph | T1 | H | m.p.143-145℃ |
| I-236 | H | 4-CN-Ph | T1 | H | m.p.129-131℃ |
| I-237 | H | 2,6-$Cl_2$-4-Pyridyl | T1 | H | m.p.157-159℃ |
| I-238 | H | 4-MeOC(=O)-Ph | T1 | H | m.p.104-106℃ |
| I-239 | H | 4-Cl-Ph-C(Me)$_2$- | T1 | H | $n_D^{20.8}$1.5361 |
| I-240 | H | Ph | T1 | 5-F | m.p.105-106℃ |
| I-241 | H | 4-Me-Ph | T1 | 4-Me | m.p.100-101℃ |
| I-242 | H | 4-n-Pr-Ph | T1 | H | m.p.70-71℃ |
| I-243 | H | 4-n-Bu-Ph | T1 | H | m.p.43.5-44.5℃ |
| I-244 | H | 2,3,6-$F_3$-Ph | T1 | H | m.p.83-84℃ |
| I-245 | H | 4-MeS-Ph | T1 | H | m.p.78-80℃ |
| I-246 | H | 2-Br-Ph | T1 | H | m.p.117-119℃ |
| I-247 | H | 4-Hex-Ph | T1 | H | $n_D^{20.5}$1.5134 |
| I-248 | H | 2,6-$F_2$-Ph | T1 | 6-Me | m.p.116-117℃ |
| I-249 | H | 4-Me-Ph | T1 | 6-Me | $n_D^{21.7}$1.4480 |
| I-250 | H | 3-F-4-MeO-Ph | T1 | H | m.p.103.5-104.5℃ |
| I-251 | H | 4-Me-Ph | T1 | 5-F | m.p.123-124.5℃ |
| I-252 | H | 2,6-$F_2$-Ph | T1 | 5-F | m.p.123-125℃ |
| I-253 | H | 4-$F_2HC$-O-Ph | T1 | H | $n_D^{20.4}$1.5442 |
| I-254 | H | 4-I-Ph | T1 | H | m.p.141-143℃ |
| I-255 | H | 4-($PhCH_2CH_2CH_2O$)-Ph | T1 | H | $n_D^{20.3}$1.5492 |
| I-256 | H | 2-F-4-EtO-Ph | T1 | H | $n_D^{20.4}$1.5406 |
| I-257 | H | 2-F-4-Cl-Ph | T1 | H | m.p.80.5-82℃ |
| I-258 | H | 2-F-4-Br-Ph | T1 | H | m.p.62-63℃ |
| I-259 | H | 2-F-6-I-Ph | T1 | H | m.p.94-96℃ |
| I-260 | H | 3-F-6-Me-Ph | T1 | H | m.p.85.5-86.5℃ |
| I-261 | Me | 4-F-Ph | T1 | H | m.p.90.5-91.5℃ |
| I-262 | H | 2-F-5-$CF_3$-Ph | T1 | H | m.p.132-133℃ |
| I-263 | H | 2-Me-3-F-Ph | T1 | H | m.p.123.5-125℃ |
| I-264 | H | 2,6-$F_2$-3-Me-Ph | T1 | H | m.p.108-110℃ |
| I-265 | H | 2-Cl-4-F-Ph | T1 | H | m.p.122-124℃ |
| I-266 | H | 2-F-5-Me-Ph | T1 | H | m.p.78.5-79.5℃ |
| I-267 | H | 3-Cl-4-F-Ph | T1 | H | $n_D^{21.7}$1.5478 |
| I-268 | H | 3-F-4-Me-Ph | T1 | H | $n_D^{20.9}$1.4930 |
| I-269 | H | 2,4-$Me_2$-Ph | T1 | H | $n_D^{21.8}$1.4915 |
| I-270 | H | 2,3-$F_2$-4-Me-Ph | T1 | H | m.p.70.5-71.5℃ |
| I-271 | H | 2,3-$Me_2$-Ph | T1 | H | m.p.93.5-95℃ |
| I-272 | H | 3,5-$Me_2$-Ph | T1 | H | $n_D^{20.8}$1.5326 |
| I-273 | H | 3-Me-4-F-Ph | T1 | H | m.p.83.5-84.5℃ |
| I-274 | H | 2,6-$F_2$-Ph | T1 | H | m.p.97-98℃ |
| I-275 | Me | 4-Me-Ph | T1 | 4-Me | $n_D^{21.4}$1.5495 |
| I-276 | H | 2-F-4-MeO-Ph | T1 | H | $n_D^{21.5}$1.5922 |

(Table 10)

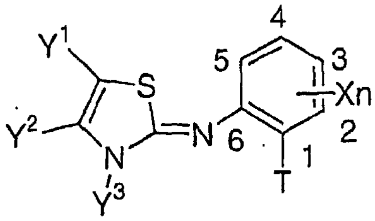

| No. | Y¹ | Y² | Y³ | T | X | Physical properties (m.p.etc.) | Notes |
|---|---|---|---|---|---|---|---|
| II-1 | H | Ph | Et | T1 | H | $n_D^{21.2}$1.5160 | |
| II-2 | H | Ph | Et | T2-1 | H | $n_D^{21.1}$1.5378 | |
| II-3 | H | Ph | Et | T2-2 | H | $n_D^{21.1}$1.4961 | |
| II-4 | H | Ph | Ph | T1 | H | $n_D^{21.1}$1.5702 | |
| II-5 | H | Ph | Ph | T2-1 | H | m.p.73-74℃ | |
| II-6 | H | Ph | Ph | T2-1 | H | $n_D^{21.1}$1.5112 | |
| II-7 | H | Ph | C(=0)OEt | T1 | H | m.p.122-123℃ | |
| II-8 | H | Ph | $PhCH_2$ | T1 | H | m.p.130-131℃ | |
| II-9 | H | Ph | $PhCH_2$ | T2-1 | H | m.p.134-136℃ | |
| II-10 | H | Ph | $PhCH_2$ | T2-2 | H | m.p.145-147℃ | |
| II-11 | H | Ph | $Me_2N$ | T1 | H | m.p.88-89℃ | |
| II-12 | H | Ph | $Me_2N$ | T2-1 | H | m.p.113-114℃ | |
| II-13 | H | Ph | $Me_2N$ | T2-2 | H | m.p.133-134℃ | |
| II-14 | H | Ph | Me | $CH_2COOPr$ | H | $n_D^{22.2}$1.5711 | |
| II-15 | H | Ph | Me | $CH_2COOBu$ | H | $n_D^{21.2}$1.5545 | |
| II-16 | H | Ph | Me | $CH_2COOPen$ | H | $n_D^{21.2}$1.5762 | |
| II-17 | H | Ph | Me | $CH_2COOCH_2CH_2OMe$ | H | $n_D^{21.2}$1.5792 | |
| II-18 | H | Ph | Me | $CH_2COOCH_2Ph$ | H | m.p.139-141℃ | |
| II-19 | H | Ph | Me | $CH_2CONH$-i-Pr | H | m.p.119-121℃ | |
| II-20 | H | Ph | Me | $CH_2CONHCHMe$(4-Cl-Ph) | H | m.p.119-121℃ | |
| II-21 | H | Ph | Me | $CH_2CONMe(CH_2C{\equiv}CH)$ | H | m.p.111-112℃ | |
| II-22 | H | H | $PhCH_2$ | T1 | H | $n_D^{21.7}$1.6236 | |
| II-23 | H | Me | $PhCH_2$ | T1 | H | $n_D^{21.7}$1.6136 | |
| II-24 | H | H | Ph | T1 | H | m.p.86-88℃ | |
| II-25 | H | Ph | Pr | T1 | H | m.p.91-92℃ | |
| II-26 | H | Ph | i-Pr | T1 | H | m.p.72-73℃ | |
| II-27 | H | Me | PhMeN | T1 | H | $n_D^{21.2}$1.6190 | |
| II-28 | H | Ph | $H_2N$ | T1 | H | m.p.200℃(decomp) | HBr salt |

(Table 11)

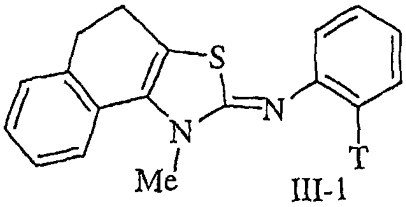

III-1

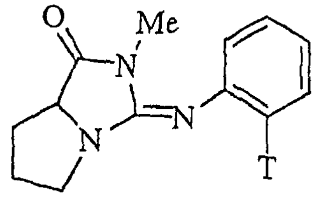

III-2

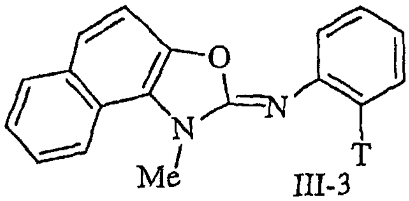

III-3

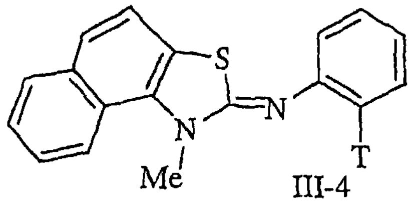

III-4

| No. | T | Physical properties (m.p. etc.) |
|---|---|---|
| III-1 | T1 | m.p. 101.5-103℃ |
| III-2 | T1 | $n_D^{21.8}$ 1.5684 |
| III-3 | T1 | m.p. 155-156℃ |
| III-4 | T1 | m.p. 133-135℃ |

(Table 12)

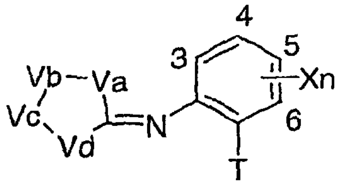

| No. | -Va-Vb-Vc-Vd- | T | X | Physical properties (m.p.etc.) | Notes |
|---|---|---|---|---|---|
| IV-1 | $-S-C(=CH_2)-CH_2-N(Me)-$ | T1 | H | $n_D^{21.0}$1.5078 | |
| IV-2 | $-S-C(=CH_2)-CH_2-N(Me)-$ | T2-1 | H | $n_D^{21.0}$1.4735 | |
| IV-3 | $-S-C(=O)-CH_2-N(Me)-$ | T1 | H | Oil | |
| IV-4 | -S-CH(Me)-C(=O)-N(Me)- | T1 | H | Oil | |
| IV-5 | -S-CH(Me)-C(=O)-N(Me)- | T2-1 | H | Oil | |
| IV-6 | $-S-CH_2-C(OH)(C_2F_5)-N(Me)-$ | T1 | H | m.p.178-180℃ | HBr salt |
| IV-7 | $-S-CH_2-C(=O)-N(Me)-$ | T1 | H | m.p.74-75℃ | |
| IV-8 | $-N(Me)-CH_2-C(=O)-N(Me)-$ | T1 | H | m.p.57-59℃ | |
| IV-9 | -O-C(Ph)=N-N(Me)- | T1 | H | m.p.97-99℃ | |
| IV-10 | -S-C(Ph)=N-N(Me)- | T1 | H | m.p.76-77℃ | |
| IV-11 | -S-C(Ph)=N-N(Me)- | T2-1 | H | m.p.127-129℃ | |
| IV-12 | -S-C(Ph)=N-N(Me)- | T2-2 | H | m.p.97-98℃ | |
| IV-13 | $-S-CH(CH_2Br)-CH_2-N(Me)-$ | T1 | H | m.p.177-179℃ | HBr salt |
| IV-14 | -S-CH=C(Ph)-O- | T1 | H | m.p.67-69℃ | |
| IV-15 | -S-CH=C(4-Br-Ph)-O- | T1 | H | Viscous oil | |
| IV-16 | -S-CH=C(2-MeO-Ph)-O- | T1 | H | $n_D^{21.7}$1.5262 | |
| IV-17 | -S-CH=C(3-MeO-Ph)-O- | T1 | H | m.p.111-112℃ | |
| IV-18 | -S-CH=C(4-MeO-Ph)-O- | T1 | H | m.p.78-79℃ | |
| IV-19 | -S-CH=C(3-Cl-Ph)-O- | T1 | H | m.p.110-111℃ | |
| IV-20 | -S-CH=C(Ph)-S- | T1 | H | Viscous oil | |

| | | | | |
|---|---|---|---|---|
| IV-21 | -S-CH=C(4-Cl-Ph)-O- | T1 | H | m.p.99-100℃ |
| IV-22 | -S-CH=C(2-F-Ph)-O- | T1 | H | m.p.62-63℃ |
| IV-23 | -S-CH=C(3-F-Ph)-O- | T1 | H | m.p.85-86℃ |
| IV-24 | -S-CH=C(4-F-Ph)-O- | T1 | H | m.p.90-91℃ |
| IV-25 | -S-CH=C(2-Me-Ph)-O- | T1 | H | m.p.78-79℃ |
| IV-26 | -S-CH=C(3-Me-Ph)-O- | T1 | H | m.p.77-79℃ |
| IV-27 | -S-CH=C(4-Me-Ph)-O- | T1 | H | m.p.59-60℃ |
| IV-28 | -S-$CH_2$-CH(Ph)-O- | T1 | H | Viscous oil |
| IV-29 | -S-CH=C(i-Bu)-O- | T1 | H | $n_D^{20.9}$ 1.5630 |
| IV-30 | -S-CH=C(1-Naphthyl)-O- | T1 | H | Viscous oil |
| IV-31 | -S-CH=C(3,4-$F_2$-Ph)-O- | T1 | H | m.p.106-107℃ |
| IV-32 | -S-CH=C(4-$NO_2$-Ph)-O- | T1 | H | m.p.123-125℃ |
| IV-33 | -S-CH=C(4-CN-Ph)-O- | T1 | H | m.p.135-137℃ |
| IV-34 | -S-CH=C(2-Cl-Ph)-O- | T1 | 4-F | $n_D^{20.9}$ 1.6218 |
| IV-35 | -S-CH=C(2-Cl-Ph)-O- | T1 | H | m.p.86-87℃ |
| IV-36 | -S-CH=C(2,6-$F_2$-Ph)-O- | T1 | H | m.p.79-81℃ |
| IV-37 | -S-CH=C(2-MeO-Ph)-O- | T1 | 4-F | $n_D^{21.3}$ 1.5304 |
| IV-38 | -S-CH=C(2,5-$F_2$-Ph)-O- | T1 | H | m.p.97-98℃ |
| IV-39 | -S-CH=C(4-Br-Ph)-S- | T1 | H | m.p.109-112℃ |
| IV-40 | -N(Me)-$CH_2$-$CH_2$-N(Me)- | T1 | H | $n_D^{21.3}$ 1.4973 |
| IV-41 | -S-CH=C(4-Me-Ph)-S- | T1 | H | $n_D^{20.9}$ 1.5053 |
| IV-42 | -S-CH=C(4-MeO-Ph)-S- | T1 | H | m.p.91-93℃ |
| IV-43 | -S-CH=C(4-Cl-Ph)-S- | T1 | H | m.p.98-101℃ |
| IV-44 | -S-CH=C(2-F-Ph)-S- | T1 | H | $n_D^{20.7}$ 1.6589 |
| IV-45 | -S-CH=C(2-Me-Ph)-S- | T1 | H | $n_D^{21.1}$ 1.6505 |
| IV-46 | -S-CH=C(2-Cl-Ph)-S- | T1 | H | $n_D^{20.1}$ 1.6604 |
| IV-47 | -S-CH=C(2-Br-Ph)-S- | T1 | H | $n_D^{20.8}$ 1.6644 |
| IV-48 | -S-CH=C(2,6-$F_2$-Ph)-S- | T1 | H | $n_D^{21.6}$ 1.6391 |
| IV-49 | -S-CH=C(2,5-$F_2$-Ph)-S- | T1 | H | $n_D^{21.1}$ 1.6465 |
| IV-50 | -S-CH=C(2,4-$F_2$-Ph)-S- | T1 | H | $n_D^{21.9}$ 1.6421 |
| IV-51 | -S-CH=C(3,4-$F_2$-Ph)-S- | T1 | H | $n_D^{22.0}$ 1.6375 |
| IV-52 | -S-C(Me)=C(Ph)-S- | T1 | H | $n_D^{21.8}$ 1.6470 |
| IV-53 | -S-CH=C(2-Br-Ph)-O- | T1 | H | m.p.74-75℃ |
| IV-54 | -S-CH=C(4-t-Bu-Ph)-O- | T1 | H | Viscous oil |
| IV-55 | -S-CH=C(2,6-$Me_2$-Ph)-O- | T1 | H | $n_D^{20.5}$ 1.5560 |
| IV-56 | -S-C(Me)=C(Ph)-O- | T1 | H | $n_D^{20.4}$ 1.5522 |
| IV-57 | -S-CH=C(4-PhO-Ph)-O- | T1 | H | Viscous oil |
| IV-58 | -S-CH=C(4-Hex-Ph)-O- | T1 | H | m.p.72-73℃ |
| IV-59 | -S-CH=C(4-Bu-Ph)-O- | T1 | H | $n_D^{20.4}$ 1.5584 |
| IV-60 | -S-CH=C(4-Pr-Ph)-O- | T1 | H | $n_D^{20.4}$ 1.5356 |
| IV-61 | -S-CH=C(4-Me-Ph)-O- | T1 | 4-Me | $n_D^{21.5}$ 1.5528 |
| IV-62 | -S-CH=C(2,4-$F_2$-Ph)-O- | T1 | H | m.p.100-101℃ |
| IV-63 | -S-CH=C(2-F-6-Cl-Ph)-O- | T1 | H | m.p.87-88℃ |
| IV-64 | -S-CH=C(4-$CF_3$O-Ph)-O- | T1 | H | m.p.79-80℃ |
| IV-65 | -S-CH=C(4-$CF_3$-Ph)-O- | T1 | H | m.p.91-92℃ |
| IV-66 | -S-C(Br)=C(Ph)-O- | T1 | H | Viscous oil |
| IV-67 | -S-CH=C(2,4-$Cl_2$-Ph)-O- | T1 | H | m.p.65-66℃ |
| IV-68 | -S-CH=C(2,5-$Cl_2$-Ph)-O- | T1 | H | m.p.128-129℃ |
| IV-69 | -S-CH=C(3,4-$Cl_2$-Ph)-O- | T1 | H | m.p.115-116℃ |
| IV-70 | -S-$CH_2$-C(=N-Ph)-N(Me)- | T1 | H | $n_D^{20.7}$ 1.5326 |
| IV-71 | -O-CH=C(Ph)-N(Me)- | T1 | H | Viscous oil |
| IV-72 | -S-C($Cl_2$)-C(=O)-N(Me)- | T1 | H | $n_D^{21.8}$ 1.6031 |
| IV-73 | -S-CH=C(2,3-$F_2$-Ph)-S- | T1 | H | m.p.52-53℃ |
| IV-74 | -S-C(Me)=C(4-F-Ph)-O- | T1 | H | m.p.60-62℃ |
| IV-75 | -S-C(Et)=C(Ph)-O- | T1 | H | m.p.68-69℃ |
| IV-76 | -S-CH=C(2,3-$F_2$-Ph)-O- | T1 | H | m.p.88-89℃ |
| IV-77 | -S-CH=C(2,5-$Me_2$-Ph)-S- | T1 | H | $n_D^{20.0}$ 1.6480 |
| IV-78 | -S-C(Ph)=C(Ph)-S- | T1 | H | Viscous oil |

| | | | | |
|---|---|---|---|---|
| IV-79 | -S-CH=C(2,5-$Me_2$-Ph)-O- | T1 | H | m.p.80-82℃ |
| IV-80 | -S-CH=C(2-F-4-Cl-Ph)-O- | T1 | H | m.p.85-87℃ |
| IV-81 | -S-C(Me)=C(2,5-$F_2$-Ph)-O- | T1 | H | $n_D^{21.2}$1.6532 |
| IV-82 | -S-CH=C(4-i-Pr-Ph)-O- | T1 | H | m.p.83-85℃ |
| IV-83 | -S-CH=C(4-F-3-Cl-Ph)-O- | T1 | H | m.p.117-118℃ |
| IV-84 | -S-CH=C(2-F-4-EtO-Ph)-O- | T1 | H | m.p.101-103℃ |
| IV-85 | -S-CH=C(4-F-Ph)-O- | T1 | H | $n_D^{20.8}$1.6054 |
| IV-86 | -S-CH=C(4-Et-Ph)-O- | T1 | H | $n_D^{20.7}$1.6091 |
| IV-87 | -S-CH=C(4-MeS-Ph)-O- | T1 | H | m.p.84-85℃ |
| IV-88 | -S-CH=C(2-Me-5-F-Ph)-O- | T1 | H | m.p.55-56℃ |
| IV-89 | -S-CH=C(3,5-$F_2$-Ph)-O- | T1 | H | m.p.138-139℃ |
| IV-90 | -S-CH=C(3,5-$Cl_2$-Ph)-O- | T1 | H | m.p.84-85℃ |
| IV-91 | -S-CH=C(3-F-4-MeO-Ph)-O- | T1 | H | $n_D^{20.2}$1.6140 |
| IV-92 | -S-CH=C(2-Me-5-F-Ph)-S- | T1 | H | $n_D^{20.4}$1.6382 |
| IV-93 | -S-C(Me)=C(4-F-Ph)-S- | T1 | H | $n_D^{20.2}$1.6353 |
| IV-94 | -S-C(4-F-Ph)=C(Me)-O- | T1 | H | $n_D^{21.3}$1.5963 |
| IV-95 | -S-C(Me)=C(2,6-$F_2$-Ph)-O- | T1 | H | $n_D^{21.2}$1.5792 |
| IV-96 | -S-C(Me)=C(2-Cl-Ph)-O- | T1 | H | $n_D^{21.1}$1.5571 |
| IV-97 | -S-CH=C(2,4-$Me_2$-Ph)-O- | T1 | H | m.p.55-57℃ |
| IV-98 | -S-CH=C(2,6-$F_2$-3-Me-Ph)-O- | T1 | H | m.p.88-89℃ |
| IV-99 | -S-CH=C(2-Cl-4-F-Ph)-O- | T1 | H | m.p.100-101℃ |
| IV-100 | -S-CH=C(2-F-5-Me-Ph)-O- | T1 | H | m.p.70-71℃ |
| IV-101 | -S-CH=C(2,4-$Cl_2$-Ph)-O- | T1 | 4-Me | m.p.109-110℃ |
| IV-102 | -S-C(Me)=C(4-Me-Ph)-O- | T1 | H | m.p.88-89℃ |
| IV-103 | -S-CH=C(3,5-$F_2$-Ph)-S- | T1 | H | $n_D^{20.4}$1.6408 |
| IV-104 | -S-CH=C(2,4-$Me_2$-Ph)-S- | T1 | H | $n_D^{20.9}$1.6401 |
| IV-105 | -S-CH=C(2-F-5-Me-Ph)-S- | T1 | H | $n_D^{20.6}$1.6442 |
| IV-106 | -S-C(Me)H=C(4-Me-Ph)-S- | T1 | H | $n_D^{21.5}$1.6273 |
| IV-107 | -S-C(2-F-Ph)=C(Me)-O- | T1 | H | $n_D^{20.5}$1.6095 |
| IV-108 | -S-CH=C(2-F-4-MeO-Ph)-O- | T1 | H | m.p.91-92℃ |
| IV-109 | -S-CH=C(2,3-$Me_2$-Ph)-O- | T1 | H | m.p.78-80℃ |
| IV-110 | -S-CH=C(3,5-$Me_2$-Ph)-O- | T1 | H | m.p.88-91℃ |
| IV-111 | -S-CH=C(2-Cl-4-F-Ph)-S- | T1 | H | $n_D^{20.4}$1.6421 |
| IV-112 | -S-C(Me)H=C(4-Me-Ph)-O- | T1 | 4-Me | m.p.110-111℃ |

(Table 13)

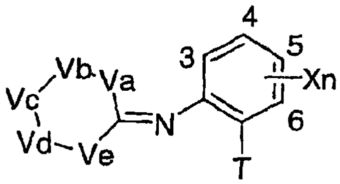

| No. | -Va-Vb-Vc-Vd-Ve- | T | X | Physical properties (m.p. etc.) |
|---|---|---|---|---|
| V-1 | $-S-CH_2-CH_2-CH_2-N(Ph)-$ | T1 | H | $n_D^{21.1}$ 1.5728 |
| V-2 | $-S-CH_2-C(Ph)=N-N(Me)-$ | T1 | H | m.p. 85-86℃ |
| V-3 | $-N(Me)-CH_2=CH_2-CH(Ph)-S-$ | T1 | H | $n_D^{21.3}$ 1.6292 |
| V-4 | -N=C(OMe)-CH=C(OMe)-N(Me)- | T1 | H | m.p. 122-123℃ |
| V-5 | -CH=CH-CH=C(F)-N(Me)- | T1 | H | $n_D^{21.7}$ 1.5286 |
| V-6 | $-S-CH_2-C(Me)=N-N(Ph)-$ | T1 | H | m.p. 101-104℃ |
| V-7 | -CH=CH-CH=CH-N(-OMe)- | T1 | H | $n_D^{21.0}$ 1.5728 |
| V-8 | $-S-CH_2-C(=O)-N(Me)-N(Me)-$ | T1 | H | $n_D^{20.7}$ 1.5728 |
| V-9 | $-CH_2-S-CH=C(Ph)-N(Me)-$ | T1 | H | $n_D^{20.4}$ 1.5728 |
| V-10 | $-CH_2-S-CH=C(Ph)-N(Me)-$ | T2-1 | H | Viscous oil |

(Table 14)

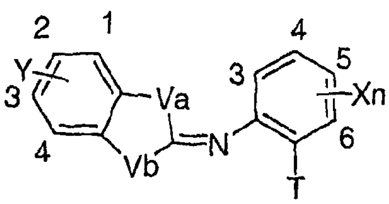

| No. | Va | Vb | Y | T | X | Physical properties (m.p. etc.) |
|---|---|---|---|---|---|---|
| VI-1 | S | NMe | H | T1 | H | $n_D^{21.5}$ 1.5512 |
| VI-2 | O | NMe | H | T1 | H | $n_D^{21.3}$ 1.5526 |
| VI-3 | S | S | H | T1 | H | $n_D^{21.4}$ 1.5032 |
| VI-4 | S | NMe | 2-Cl | T1 | H | $n_D^{21.2}$ 1.5232 |
| VI-5 | S | NMe | 2-MeO | T1 | H | $n_D^{21.3}$ 1.5276 |
| VI-6 | NMe | NMe | H | T1 | H | $n_D^{21.0}$ 1.5380 |
| VI-7 | S | O | H | T1 | H | $n_D^{21.2}$ 1.5459 |

(Table 15)

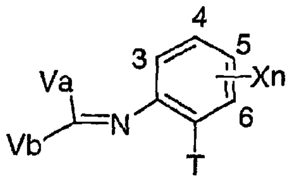

| No. | Va | Vb | T | X | Physical properties (m.p. etc.) |
|---|---|---|---|---|---|
| VII-1 | PhC(=O)$CH_2$S | $Me_2$N | T1 | H | $n_D^{21.7}$ 1.6084 |
| VII-2 | (2-MeO-Ph)C(=O)$CH_2$S | $Me_2$N | T1 | H | $n_D^{21.6}$ 1.5325 |
| VII-3 | (3-MeO-Ph)C(=O)$CH_2$S | $Me_2$N | T1 | H | $n_D^{21.5}$ 1.5274 |
| VII-4 | (4-MeO-Ph)C(=O)$CH_2$S | $Me_2$N | T1 | H | $n_D^{21.7}$ 1.5053 |
| VII-5 | (3-Cl-Ph)C(=O)$CH_2$S | $Me_2$N | T1 | H | $n_D^{21.5}$ 1.4970 |
| VII-6 | (4-Cl-Ph)C(=O)$CH_2$S | $Me_2$N | T1 | H | $n_D^{21.5}$ 1.5545 |
| VII-7 | (2-F-Ph)C(=O)$CH_2$S | $Me_2$N | T1 | H | $n_D^{21.5}$ 1.5166 |
| VII-8 | (3-F-Ph)C(=O)$CH_2$S | $Me_2$N | T1 | H | $n_D^{21.4}$ 1.5544 |
| VII-9 | (4-F-Ph)C(=O)$CH_2$S | $Me_2$N | T1 | H | $n_D^{21.5}$ 1.5281 |
| VII-10 | (2-Me-Ph)C(=O)$CH_2$S | $Me_2$N | T1 | H | $n_D^{21.6}$ 1.5382 |
| VII-11 | (3-Me-Ph)C(=O)$CH_2$S | $Me_2$N | T1 | H | $n_D^{21.5}$ 1.5022 |
| VII-12 | (4-Me-Ph)C(=O)$CH_2$S | $Me_2$N | T1 | H | $n_D^{21.5}$ 1.5221 |
| VII-13 | (2,6-$F_2$-Ph)C(=O)$CH_2$S | $Me_2$N | T1 | H | $n_D^{21.2}$ 1.5647 |
| VII-14 | (2-Cl-Ph)C(=O)$CH_2$S | $Me_2$N | T1 | H | $n_D^{21.3}$ 1.5364 |
| VII-15 | (1-naphthyl)C(=O)$CH_2$S | $Me_2$N | T1 | H | $n_D^{21.4}$ 1.5342 |
| VII-16 | (2,5-$F_2$-Ph)C(=O)$CH_2$S | $Me_2$N | T1 | H | $n_D^{21.3}$ 1.5860 |
| VII-17 | (4-$NO_2$-Ph)C(=O)$CH_2$S | $Me_2$N | T1 | H | $n_D^{20.9}$ 1.5216 |
| VII-18 | (2-Br-Ph)C(=O)$CH_2$S | $Me_2$N | T1 | H | $n_D^{20.9}$ 1.5756 |
| VII-19 | (4-MeS-Ph)C(=O)$CH_2$S | $Me_2$N | T1 | H | $n_D^{20.8}$ 1.5932 |
| VII-20 | (2,6-$Me_2$-Ph)C(=O)$CH_2$S | $Me_2$N | T1 | H | $n_D^{20.5}$ 1.5232 |
| VII-21 | (4-PhO-Ph)C(=O)$CH_2$S | $Me_2$N | T1 | H | $n_D^{20.4}$ 1.6214 |
| VII-22 | (4-Hex-Ph)C(=O)$CH_2$S | $Me_2$N | T1 | H | $n_D^{20.5}$ 1.5778 |
| VII-23 | (4-Bu-Ph)C(=O)$CH_2$S | $Me_2$N | T1 | H | $n_D^{20.4}$ 1.5884 |
| VII-24 | (4-Pr-Ph)C(=O)$CH_2$S | $Me_2$N | T1 | H | $n_D^{20.4}$ 1.5936 |
| VII-25 | (4-Me-Ph)C(=O)$CH_2$S | $Me_2$N | T1 | H | $n_D^{21.5}$ 1.5592 |
| VII-26 | (2,4-$F_2$-Ph)C(=O)$CH_2$S | $Me_2$N | T1 | H | $n_D^{21.5}$ 1.5104 |
| VII-27 | (4-$CF_3$O-Ph)C(=O)$CH_2$S | $Me_2$N | T1 | H | $n_D^{21.7}$ 1.5640 |
| VII-28 | (4-$CF_3$-Ph)C(=O)$CH_2$S | $Me_2$N | T1 | H | $n_D^{21.8}$ 1.5653 |
| VII-29 | (4-Et-Ph)C(=O)$CH_2$S | $Me_2$N | T1 | H | $n_D^{20.8}$ 1.5790 |
| VII-30 | MeS | $Me_3SiCH_2$S | T1 | H | $n_D^{21.0}$ 1.5676 |

(Table 16)

| No. | $^1$HNMR ($CDCl_3$ δ (ppm)) |
|---|---|
| I-50 | 2.38(s, 3H), 3.26(s, 3H), 3.58(s, 3H), 3.73(s, 3H), 5.71(s, 1H), 6.80-7.60(m, 9H) |
| I-51 | 2.39(s, 3H), 3.28(s, 3H), 3.55(s, 3H), 3.85(s, 3H), 5.70(s, 1H), 6.54(s, 1H), 6.90-7.40(m, 8H) |
| I-52 | 3.23(s, 3H), 3.56(s, 3H), 3.74(s, 3H), 5.73(s, 1H), 6.90-7.80(m, 9H) |
| I-53 | 3.28(s, 3H), 3.59(s, 3H), 3.86(s, 3H), 5.75(s, 1H), 6.56(s, 1H), 6.80-7.60(m, 8H) |

(Table 16) (continued)

| No. | $^1$HNMR ($CDCl_3$ δ (ppm)) |
|---|---|
| I-54 | 3.31(s, 3H), 3.59(s, 3H), 3.75(s, 3H), 5.79(s, 1H), 7.10-7.85(m, 14H) |
| I-58 | 3.30(s, 3H), 3.60(s, 3H), 3.90(s, 3H), 5.90(s, 1H), 6.55(s, 1H), 7.00-8.15(m, 11H) |
| I-59 | 3.38(s, 3H), 3.61(s, 3H), 3.78(s, 3H), 5.94(s, 1H), 7.00-7.60(m, 8H) |
| I-76 | 2.94(s, 3H), 3.32(s, 3H), 3.54(s, 3H), 3.82(s, 3H), 4.20(s, 2H), 5.57(s, 1H), 6.49(s, 1H), 6.65-7.45(m, 9H) |
| I-79 | 3.19(s, 3H), 3.61(s, 3H), 3.67(s, 2H), 5.85(s, 1H), 7.00-7.80(m, 7H) |
| I-118 | 3.24(s, 3H), 3.51(s, 3H), 3.76(s, 3H), 5.63(s, 1H), 6.45(s, 1H), 6.60-7.60(m, 13H) |
| I-I23 | 3.43(s, 3H), 3.55(s, 3H), 3.73(s, 3H), 6.00(s, 1H), 6.35-6.65(m, 2H), 6.90-7.60(m, 6H) |
| I-124 | 3.56(s, 3H), 3.63(s, 3H), 3.75(s, 3H), 6.28(s, 1H), 7.10-7.45(m, 6H), 7.86(d, J=3.6Hz, 1H) |
| I-129 | 3.22(s, 3H), 3.53(s, 3H), 3.72(s, 3H), 5.77(s, 1H), 6.90-7.50(m, 8H) |
| I-130 | 3.25(s, 3H), 3.54(s, 3H), 3.82(s, 3H), 5.77(s, 1H), 6.49(s, 1H), 6.80-7.55(m, 7H) |
| I-132 | 3.08(s, 3H), 3.52(s, 3H), 3.82(s, 3H), 5.82(s, 1H), 6.53(s, 1H), 6.80-7.55(m, 7H) |
| I-134 | 3.12(s, 3H), 3.52(s, 3H), 3.82(s, 3H), 5.75(s, 1H), 6.50(s, 1H), 6.90-7.55(m, 7H) |
| I-138 | 3.17(s, 3H), 3.60(s, 3H), 3.85(s, 3H), 6.53(s, 1H), 6.85-7.75(m, 9H) |
| 1-152 | 3.92(s, 3H), 3.35(s, 3H), 3.57(s, 3H), 3.61(s, 2H), 4.20(s, 2H), 5.61(s, 1H), 6.75-7.45(m, 9H) |
| I-161 | 0.94(t, J=5.4Hz, 3H), 1.20-1.75(m, 4H), 2.2-2.6(m, 2H), 3.30(s, 3H), 3.49(s, 3H), 3.80(s, 3H), 5.42(s, 1H), 6.47(s, 1H), 6.85-7.35(m, 4H) |
| I-168 | 3.44(s, 3H), 3.57(s, 3H), 3.76(s, 3H), 6.44(s, 1H), 7.05-7.35(m, 4H), 7.38(s, 1H) |
| I-169 | 3.47(s, 3H), 3.54(s, 3H), 3.87(s, 3H), 6.45(s, 1H), 6.54(s, 1H), 7.05-7.35(m, 4H), |
| 1-171 | 3.49(s, 3H), 3.53(s, 3H), 3.86(s, 3H), 4.85(s, 2H), 5.98(s, 1H), 6.56(s, 1H), 6.90-7.55(m, 7H) |
| 1-173 | 3.23(s, 3H), 3.44(s, 3H), 3.67(s, 3H), 4.91(s, 2H), 5.91(s, 1H), 6.85-7.45(m, 10H) |
| 1-174 | 3.31(s, 3H), 3.41(s, 3H), 3.86(s, 3H), 4.98(s, 2H), 5.99(s, 1H), 6.53(s, 1H), 7.05-7.50(m, 9H) |
| I-175 | 2.91(s, 3H), 3.30(s, 3H), 3.54(s, 3H), 3.72(s, 3H), 4.17(s, 2H), 5.57(s, 1H), 6.70-7.45(m, 10H) |
| I-177 | 2.10(s, 3H), 3.45(s, 3H), 3.55(s, 3H), 3.70(s, 3H), 5.15(s, 2H), 6.00(s, 1H), 6.90-7.70(m, 9H) |
| I-178 | 2.10(s, 3H), 3.50(s, 3H), 3.55(s, 3H), 3.90(s, 3H), 5.20(s, 2H), 6.10(s, 1H), 6.90-7.50(m, 9H) |
| I-179 | 0.55-1.15(m, 4H), 1.40-1.70(m, 1H), 3.43(s, 3H), 3.54(s, 3H), 3.76(s, 3H), 5.43(s, 1H), 6.95-7.50(m, 5H) |
| I-181 | 2.05(s, 3H), 3.38(s, 3H), 3.44(s, 2H), 3.55(s, 3H), |

(Table 16) (continued)

| No. | $^1$HNMR ($CDCl_3$ δ (ppm)) |
|---|---|
| | 3.75(s, 3H), 5.68(s, 1H), 6.95-7.37(m, 5H) |
| 1-183 | 2.04(s, 3H), 3.37(s, 3H), 3.58(s, 3H). 3.74(s, 3H), 6.85-7.65(m, 9H) |
| I-184 | 2.03(s, 3H), 3.40(s, 3H), 3.56(s, 3H), 3.82(s, 3H), 6.50(s, 1H), 6.90-7.45(m, 8H) |
| I-190 | 3.30(s, 3H), 3.60(s, 3H), 3.62(s, 2H), 5.82(s, 1H). 6.92-7.45(m, 8H) |
| I-224 | 3.57(s, 8H), 6.16(s, 1H), 6.90-7.60(m, 6H) |
| I-231 | 3.48(s, 3H), 3.59(s, 3H). 3.63(s, 3H), 5.12(d, J=48.4Hz, 2H), 6.06(d, J=6.6Hz, 1H), 7.00-7.10(m, 2H), 7.20-7.30(m, 2H) |
| IV-3 | 3.09(s, 3H), 3.51(s, 2H), 3.61(s, 3H), 4.00(s, 2H), 7.05-7.48(m, 4H) |
| IV-4 | 1.61(d, J=7.2Hz, 3H), 3.30(s, 3H), 3.57(s, 2H), 3.62(s, 3H), 4.08(q, J=7.2Hz, 1H), 6.90-7.45(m, 4H) |
| IV-5 | 1.58(d, J=7.8Hz, 3H), 3.21(s, 3H), 3.63(s, 3H), 3.77(s, 3H), 4.05(q, J=7.8Hz, 1H), 6.85-7.40(m, 5H) |
| IV-15 | 3.62(s, 3H), 3.68(s, 2H), 6.42(s, 1H), 7.06-7.53(m, 8H) |
| IV-20 | 3.64(s, 3H), 3.64(s, 2H), 6.59(s, 0.4H), 6.68(s, 0.6H), 7.04-7.32(m, 9H) |
| IV-28 | 3.38(dd, J=9.1, 11.0Hz, 1H), 3.60-3.65(m, 1H), 3.65(s, 3H), 3.66(s, 2H), 5.63(dd, J=5.8, 9.1Hz, 1H), 6.96-7.46(m, 9H) |
| IV-30 | 3.66(s, 3H), 3.72(s, 2H), 6.37(s, 1H), 7.10-8.35(m, 11H) |
| IV-54 | 1.34(s, 9H), 3.62(s, 3H), 3.69(s, 2H), 6.33(s, 1H), 7.05-7.65 (m, 8H) |
| IV-57 | 3.61(s, 3H), 3.68(s, 2H), 6.27(s, 1H), 6.95-7.7(m, 13H) |
| IV-66 | 3.65(s, 3H), 3.68(s, 2H), 6.9-8.15(m, 9H) |
| IV-71 | 3.28(s, 3H), 3.64(s, 3H), 3.73(s, 2H), 6.81(s, 1H), 6.95-7.5(m, 9H) |
| IV-78 | 3.67(s, 2H), 3.70(s, 3H), 7.05-7.32(m, 14H) |
| V-10 | 3.03(s, 3H), 3.25(s, 2H), 3.69(s, 3H), 3.82(s, 3H), 5.71(s, 1H), 6.79-7.41(m, 10H), 7.46(s, 1H) |

Now, Formulations Examples of fungicides and insecticides for agricultural and horticultural use containing the compounds of the present invention as active ingredients, will be specifically given. However, the present invention is not limited thereto. In the following Formulation Examples, "parts" means "parts by weight".

| FORMULATION EXAMPLE 1: Emulsifiable concentrate | |
|---|---|
| Compound No. I-1 of the present invention | 20 parts |
| Methyl naphthalene | 55 parts |
| Cyclohexanone | 20 parts |
| Sorpol 2680 (mixture of a nonionic surfactant and an anionic surfactant, tradename, Toho Chemical Industry Co., Ltd.) | 5 parts |

The above materials are uniformly mixed to obtain an emulsifiable concentrate. In use, the above emulsifiable concentrate is diluted from 50 to 20000 times and applied so that the amount of the active ingredient will be from 0.005 to 50 kg per hectare.

| FORMULATION EXAMPLE 2: Wettable powder | |
|---|---|
| Compound No. I-37 of the present invention | 25 parts |
| pyrophyllite | 66 parts |
| Sorpol 5039 (anionic surfactant, tradename, Toho Chemical Industry Co., Ltd.) | 4 parts |
| Carplex #80D (white carbon, tradename, Shionogi & Co., Ltd.) | 3 parts |
| Calcium ligninsulfonate | 2 parts |

The above materials are uniformly mixed and pulverized to obtain a wettable powder.

In use, the above wettable powder is diluted from 50 to 20000 times and applied so that the amount of the active ingredient will be from 0.005 to 50 kg per hectare.

| FORMULATION EXAMPLE 3: Dust | |
|---|---|
| Compound No. I-43 of the present invention | 3 parts |
| Carplex #80D (white carbon, tradename, Shionogi & Co., Ltd.) | 0.5 part |
| Kaolinite | 95 parts |
| Diisopropyl phosphate | 1.5 parts |

The above materials are uniformly mixed to obtain a dust. In use, the above dust is applied so that the amount of the active ingredient will be from 0.005 to 50 kg per hectare.

| FORMULATION EXAMPLE 4: Granule | |
|---|---|
| Compound No. 1-53 of the present invention | 5 parts |
| Bentonite | 30 parts |
| Talc | 64 parts |
| Calcium ligninsulfonate | 1 part |

The above materials are uniformly mixed and pulverized, and a small amount of water is added, followed by stirring and mixing, and the mixture is granulated by an extrusion granulator, followed by drying to obtain a granule. In use, the above granule is applied so that the amount of the active ingredient will be from 0.005 to 50 kg per hectare.

| FORMULATION EXAMPLE 5: Flowable | |
|---|---|
| Compound No. I-70 of the present invention | 25 parts |
| Sorpol 3353 (nonionic surfactant, tradename, Toho Chemical Industry Co., Ltd.) | 5 parts |
| Lunox 1000C (anionic surfactant, tradename, Toho Chemical Industry Co., Ltd.) | 0.5 part |
| Xanthan gum (natural polymer) | 0.2 part |
| Sodium benzoate | 0.4 part |
| Propylene glycol | 10 parts |
| Water | 58.9 parts |

The above components except for the active ingredient (the compound of the present invention) are uniformly dissolved, then the compound of the present invention is added, followed by stirring. Then, the mixture is wet-pulverized by a sand mill to obtain a flowable. In use, the flowable is diluted from 50 to 20000 times and applied so that the amount of the active ingredient will be from 0.005 to 50 kg per hectare.

| FORMULATION EXAMPLE 6: Granular wettable powder (dry flowable) | |
|---|---|
| Compound No. II-1 of the present invention | 75 parts |

(continued)

| FORMULATION EXAMPLE 6: Granular wettable powder (dry flowable) | |
|---|---|
| Hitenol NE-15 (anionic surfactant, tradename, Daiichi Kogyo Seiyaku Co., Ltd.) | 5 parts |
| Vanilex N (anionic surfactant, tradename, Nippon Paper Industries Co., Ltd.) | 10 parts |
| Carplex #80D (white carbon, tradename, Shionogi & Co., Ltd.) | 10 parts |

The above materials are uniformly mixed and finely pulverized, and a small amount of water was added, followed by stirring and mixing. Then, the mixture is granulated by an extrusion granulator and dried to obtain a dry flowable. In use, it is diluted with water from 50 to 20000 times and applied so that the amount of the active ingredient will be from 0.005 to 50 kg per hectare. TEST EXAMPLES: The usefulness of the compounds of the present invention will be described in detail with reference to the following Test Examples. However, the present invention is not limited thereto.

TEST EXAMPLE 1: Test on rice blast controlling effects (water surface application)

To rice (variety: Nihonbare) of 1.5 leaf stage planted in a beaker pot of 1/20,000 are, the emulsifiable concentrate of the compound of the present invention was diluted with water to obtain a solution adjusted to 500 ppm, which was applied for irrigation treatment of 10 m per pot.

After 7 days from the irrigation treatment, a suspension of spores of blast (Pyricularia oryzae) ($2\times10^5$ spores/ml) was sprayed and inoculated to the treated rice. The inoculated rice was put in an inoculation box at a temperature of from 20 to 25°C under a humidity of at least 95% for 1 day. Then, it was put in a greenhouse, and after 7 days from the inoculation, the proportion of the formed lesion area on the inoculated leaf, was measured, and the control value was calculated in accordance with the following formula.

Control value=

[1-(lesion area in treated section/lesion area in

non-treated section)]×100

As a result, the following compounds showed control values of 70 or higher.

Compound Nos. of the present invention: I-1,I-2, I-3,I-4,I-7,I-13,I-17,I-20,I-21,I-33,I-37,I-41,I-43, I-44,I-45,I-47,I-50,I-51,I-52,I-54,I-55,I-56,I-57,I-58,I-59,I-64,I-66,I-68,I-70,I-71,I-72,I-73,I-75,I-80, I-82,I-99,I-111,I-128,I-131,I-134,I-143,I-144,I-146, I-151,I-160,I-162,I-164,I-166,I-167,I-168,I-169,I-175, I-176,I-181,I-196,I-197,I-198,I-203,1-204,I-205,I-244, I-246,II-4,II-10,II-11,II-12,II-19,IV-2,IV-3,IV-5,IV-16, IV-17, IV-19, IV-20, IV-43, IV-44, IV-45,V-3,VII-18

TEST EXAMPLE 2: Test on rice blast controlling effects (spray test)

To rice (variety: Nihonbare) of 3 leaf stage grown in a pot having a diameter of 7 cm, a solution obtained by diluting the emulsifiable concentrate of the compound of the present invention with water to 500 ppm, was sprayed in an amount of 20 ml per pot by means of a spray gun.

One day after the spraying, a suspension of spores of rice blast (Pyricularia oryzae) ($2\times10^5$ spores/ml) was sprayed for inoculation. The inoculated rice was put in an inoculation box at a temperature of 25°C under a humidity of at least 95% for 1 day. Then, it was put in a greenhouse, and after 7 days from inoculation, the proportion of the formed lesion area on the inoculated leaf, was measured, and the control value was calculated in accordance with the following formula.

Control value=

[1-(lesion area in treated section/lesion area in

non-treated section)]×100

As a result, the following compounds showed control values of 70 or higher.

Compound Nos. of the present invention: I-1,I-2,I-3,I-4,I-5,I-6,I-8,I-9,I-10,I-11,I-12,I-13,I-14,I-15,1-16,I-17,I-18,I-19,I-20,I-21,I-25,I-26,I-27,I-28,I-29,I-30,I-31,I-33,I-36,I-37,I-38,I-39,I-40,I-41,I-42,I-43,I-44,I-45,I-46,I-47,I-48,I-49,I-50,I-51,I-52,I-53,I-54,1-55,I-56,I-57,I-58,I-59,I-60,I-61,I-62,I-63,I-64,I-65,I-66,I-67,I-68,I-69,I-70,I-71,I-72,I-74,I-76,I-79,I-80,1-82,I-92,I-93,I-94,I-98,I-99,I-100,I-102,I-105,I-106,1-107,I-108,I-109,I-110,I-111,I-112,I-113,I-114,I-115,I-116,I-117,I-119,I-126,I-127,I-128,I-129,I-133,I-134,I-137,I-139,I-140,I-141,I-143,I-144,I-145,I-146,I-149,1-150,I-152,I-155,I-160,I-161,I-162,I-164,I-165,I-166,1-168,I-169,I-171,I-175,I-176,I-179,I-183,I-186,I-187,I-196,I-197,I-198,I-201,II-1,II-2,II-3,II-4,II-12,IV-10

TEST EXAMPLE 3: Test on wheat powdery mildew controlling effects

To wheat (variety: Norin No. 61) of from 2.0 to 2.5 leaf stage grown in a pot having a diameter of 5.5 cm, a solution prepared by diluting the emulsifiable concentrate of the compound of the present invention with water to 500 ppm, was applied in an amount of 20 ml per pot by means of a spray gun.

After 1 day from the application, spores of wheat powdery mildew (Erysiphe graminis) were directly inoculated. Then, it was put in a greenhouse, and after 7 days from the inoculation, the proportion of the formed lesion area on the inoculated leaf, was measured, and the control value was calculated in accordance with the following formula:

Control value=

[1-(lesion area in treated section/lesion area in

non-treated section)]×100

As a result, the following compounds showed control values of 70 or higher.

Compound Nos. of the present invention: I-1,I-3,I-4,I-5,I-6,I-9,I-10,I-11,I-12,I-13,I-15,I-16,I-18,I-20,1-21,I-22,I-23,I-24,I-25,I-26,I-27,I-28,I-31,I-37,I-41,1-43,I-45,I-49,I-50,I-52,I-56,I-59,I-62,I-66,I-67,I-68,I-70,I-72,I-73,I-76,I-78,I-79,I-80,I-84,I-85,I-86,I-87,I-88,I-89,I-90,I-92,I-93,I-94,I-95,I-96,I-97,I-106,I-111,I-113,I-115,I-120,I-125,I-127,I-129,I-130,I-132,I-135,1-143,I-145,I-146,I-147,I-151,I-155,I-160,I-161,I-164,1-166,I-168,I-169,I-177,I-178,I-188,I-190,I-191,I-193,1-194,I-195,I-197,I-198,I-200,I-201,I-203,I-205,I-206,I-207,I-208,I-210,I-211,I-212,I-213,I-214,I-215,I-218,I-219,I-220,I-223,I-224,I-225,I-226,I-228,I-229,I-234,I-240,I-242,I-243,I-244,II-1,II-2,II-25,II-26,IV-1,IV-2,IV-5,IV-7,IV-14,IV-15,IV-16,IV-18, IV-19,IV-20,IV-21,IV-22,IV-23,IV-24,IV-25,IV-26,IV-27,IV-28,IV-29, IV-30,IV-31,IV-34,IV-35,IV-36,IV-37,IV-38,IV-39,IV-41, IV-42,IV-43,IV-44,IV-45,IV-46,IV-47,IV-48,IV-49,IV-50,IV-51,IV-52,IV-53,IV-54,IV-55,IV-56,IV-57,IV-58,IV-59,IV-60,IV-61,IV-62,IV-63,IV-64,IV-65,IV-66,IV-67, IV-68, IV-69,IV-70,IV-85,IV-86,VI-1,VI-3,VI-7,VII-1,VII-2,VII-3,VII-4,VII-5,VII-6,VII-8,VII-7,VII-9,VII-10,VII-11,VII-12,VII-13,VII-14,VII-16,VII-18,VII-22,VII-23,VII-24,VII-29

TEST EXAMPLE 4: Test on wheat scab controlling effects

To wheat (variety: Norin No. 61) of from 2.0 to 2.5 leaf stage grown in a pot having a diameter of 5.5 cm, a solution prepared by diluting the emulsifiable concentrate of the compound of the present invention with water to 500 ppm, was applied in an amount of 20 ml per pot by means of a spray gun.

After 1 day from the application, a suspension of spores of wheat scab (Puccinia recondita) ($2\times10^5$ spores/ml) was sprayed, and it was put in an inoculation box at a temperature of from 20 to 25°C under a humidity of at least 95% for 1 day. Then, it was put in a greenhouse, and after 10 days from the inoculation, the formed, lesion area was measured, and the control value was calculated in accordance with the following formula:

Control value=

[1-(lesion area in treated section/lesion area in

non-treated section)]×100

As a result, the following compounds showed control values of 70 or higher.

Compound Nos. of the present invention: I-1,I-2,I-3,I-4,I-5,I-6,I-7,I-8,I-9,I-10,I-11,I-12,I-13,I-14,I-15,I-16,I-18,I-19,I-20,I-21,I-24,I-25,I-26,I-27,I-28,I-29,I-30,I-31,I-33,I-35,I-36,I-37,I-38,I-39,I-40,I-41,I-42,I-43,I-44,I-45,I-46,I-47,I-48,I-49,I-50,I-51,I-52,I-53,I-54,I-55,I-56,I-57,I-59,I-60,I-62,I-63,I-64,I-65,I-66,I-67,I-68,I-69,I-70,I-71,I-72,I-73,I-74,I-76,I-77,I-78,I-79,I-80,I-82,I-83,I-84,I-85,I-86,I-87,I-88,I-89,I-91,I-92,I-93,I-94,I-95,I-96,I-97,I-98,I-99,I-100,I-102,I-104,I-105,I-106,I-107,I-108,I-109,I-110,I-111,I-112,I-114,I-115,I-116,I-117,I-118,I-119,I-120,I-121,I-123,I-124,I-125,I-126,I-127,I-128,I-129,I-130,I-131,I-132,I-133,I-134,I-135,I-136,I-137,I-139,I-143,I-144,I-145,I-146,I-147,I-148,I-149,I-151,I-152,I-153,I-155,I-160,I-161,I-162,I-163,I-164,I-165,I-166,I-167,I-168,I-170,1-171,I-173,I-174,I-175,I-176,I-177,I-178,I-179,I-180,1-181,I-182,I-183,I-187,I-188,I-189,I-190,I-191,I-193,1-194,I-195,I-196,I-197,I-198,I-199,I-200,I-203,I-205,I-206,I-207,I-208,I-209,I-211,I-212,I-214,I-215,I-216,I-217,I-218,I-219,I-220,I-221,I-223,I-224,I-225,I-226,I-227,I-228,I-229,I-230,I-232,I-233,I-234,I-236,I-238,I-239,I-240,I-241,I-242,I-243,I-244,I-245,I-246,I-247,II-1,II-2,II-4,II-5,II-12,II-16,II-17,II-18,II-22,II-23,II-25,II-26,II-28,III-1,IV-6,IV-14,IV-15,IV-16,IV-17,IV-18,IV-19,IV-20,IV-21,IV-22,IV-23,IV-24,IV-25,IV-26,IV-27,IV-28,IV-29,IV-30,IV-31,IV-32,IV-33,IV-34,IV-35,IV-36,IV-37,IV-38,IV-39,IV-42,IV-42,IV-43,IV-44,IV-45, IV-46,IV-47,IV-48,IV-49,IV-50,IV-51,IV-52,IV-53,IV-54,IV-55,IV-56,IV-57,IV-58,IV-59,IV-60,IV-61,IV-62,IV-63,IV-64,IV-65,IV-66,IV-67,IV-68,IV-69, IV-70,IV-85,IV-86,IV-87,V-10,VI-1,VI-2,VI-3,VI-5,VI-7,VII-1,VII-2,VII-3,VII-4,VII-5,VII-6,VII-7,VII-9,VII-10,VII-11,VII-12,VII-13,VII-14,VII-15,VII-16,VII-18,VII-19,VII-23,VII-24,VII-25,VII-26,VII-29

TEST EXAMPLE 5: Test on wheat glume-blotch control

To wheat (variety: altria) of from 2.0 to 2.5 leaf stage grown in a pot having a diameter of 5.5 cm, a solution prepared by diluting the emulsifiable concentrate of the compound of the present invention with water to 500 ppm, was applied in an amount of 20 ml per pot by means of a spray gun.

After 1 day from the application, a suspension of spores of glume-blotch (Leptosphaera nodorum) ($2\times10^5$ spores/ml) was sprayed for inoculation. The inoculated wheat was put in an inoculation box at a temperature of from 18 to 20°C under a humidity of at least 95% for from 7 to 10 days to promote the disease. The proportion of the formed lesion area on the inoculated leaf, was measured, and the control value was calculated in accordance with the following formula.

$$[1-(\text{lesion area in treated section/lesion area in non-treated section})]\times 100$$

As a result, the following compounds showed control values of 70 or higher.

Compound Nos. of the present invention: I-1,I-2, I-3,I-4,I-5,I-6,I-7,I-8,I-9,I-10,I-11,I-12,I-13,I-14, I-15,I-16,I-17,I-18,I-19,I-20,I-21,I-22,I-23,I-24,I-2 5,I-26,I-27,I-28,I-31,I-33,I-34,I-36,I-37,I-39,I-40, I-41,I-42,I-43,I-44,I-45,I-46,I-47,I-48,I-49,I-50,I-52,I-54,I-55,I-56,I-57,I-58,I-59,I-60,I-61,I-62,I-63, I-64,I-65,I-66,I-67,I-68,I-69,I-70,I-71,I-73,I-74,I-7 5,I-76,I-78,I-79,I-80,I-82,I-83,I-84,I-85,I-86,I-87, I-88,I-89,I-90,I-91,I-93,I-96,I-97,I-99,I-100,I-102, I-103,I-105,I-106,I-107,I-108,I-109,I-110,I-112,I-113, I-114,I-115,I-116,I-117,I-118,I-119,I-120,I-121,I-123, I-125,I-126,I-127,I-128,I-129,I-130,I-132,I-133,I-134, I-135,I-136,I-137,I-143,I-145,I-146,I-147,I-149,I-150, I-153,I-155,I-160,I-161,I-162,I-164,I-165,I-166,I-167, I-168,I-169,I-170,I-172,I-174,I-175,I-177,I-179,I-182, I-183,I-187,I-188,I-189,I-190,I-191,I-193,I-194,I-195, I-197,I-198,I-199,I-200,I-201,I-203,I-204,I-205,I-206, I-207,I-208,I-209,I-210,I-211,I-212,I-215,I-216,I-217, I-218,I-219,I-220,I-221,I-223,I-224,I-225,I-226,I-228, I-229,I-230,I-231,I-232,I-233,I-234,I-235,I-236,I-239, I-240,I-241,I-242,I-243,I-244,I-245,I-246,I-247,II-1, II-2,II-3,II-4,II-5,II-12,II-14,II-16,II-19,II-22,II-23,II-25,II-26,II-28,IV-1,IV-5,IV-14,IV-15,IV-16,IV-1 7,IV-18,IV-19,IV-20,IV-21,IV-22,IV-23,IV-24,IV-25,IV-26,IV-27,IV-28,IV-35,IV-36,IV-37,IV-38,IV-39,IV-40,I V-41,IV-42,IV-43, IV-44, IV-45, IV-46,IV-47, IV-48, IV-49, IV-50,IV-51,IV-52,IV-53,IV-54,IV-55,IV-56,IV-57,IV-58, IV-59,IV-60,IV-61, IV-62, IV-64,IV-65,IV-66, IV-67,IV-68, IV-69,IV-70,IV-85,IV-86,IV-87,V-6,V-7,VI-1,VI-2,VI-3, VI-4,VI-5,VI-7,VII-2,VII-3,VII-4,VII-5,VII-6,VII-7,VI I-8,VII-9,VII-10,VII-11,VII-12,VII-13,VII-14,VII-15,V II-16,VII-18,VII-19,VII-29

TEST EXAMPLE 6: Test on cucumber downy mildew controlling effects

To cucumber (variety: Sagamihanjiro) of 1.5 leaf stage grown in a pot having a diameter of 7 cm, a solution prepared by diluting the emulsifiable concentrate of the compound of the present invention with water to 500 ppm, was applied in an amount of 20 ml per pot by means of a spray gun.

After 1 day from the application, a suspension of spores of cucumber downy mildew (Pseudoperonospora cubensis) ($2\times10^5$ spores/ml) was sprayed, and it was put in an inoculation box at a temperature of from 20 to 25°C

under a humidity of at least 95% for 1 day. Then, it was put in a greenhouse, and after 7 days from the inoculation, the proportion of the formed lesion area on the inoculated leaf, was measured, and the control value was calculated in accordance with the following formula.

$$[1\text{-(lesion area in treated section/lesion area in non-treated section)}]\times 100$$

As a result, the following compounds showed control values of 70 or higher.
Compound Nos. of the present invention: I-1,I-3, I-6,I-9,I-10,I-11,I-12,I-13,I-16,I-17,I-18,I-19,I-20, I-21,I-23,I-25,I-28,I-33,I-37,I-38,I-43,I-44,I-45,I-46,I-47,I-48,I-49,I-51,I-52,I-53,I-54,I-55,I-56,I-57, I-59,I-62,I-64,I-65,I-66,I-68,I-70,I-71,I-72,I-74,I-76,I-80,I-81,I-87,I-89,I-91,I-92,I-102,I-107,I-109,I-110,I-111,I-113,I-114,I-115,I-117,I-118,I-119,I-120,I-125,I-127,I-128,I-129,I-131,I-133,I-134,I-135,I-136, I-137,I-139,I-140,I-144,I-145,I-147,I-149,I-155,I-161, I-162,I-163,I-164,I-165,I-166,I-167,I-168,I-169,I-171, I-172,I-173,I-175,I-176,I-177,I-178,I-179,I-181,I-183, I-187,I-188,I-189,I-190,I-191,I-194,I-195,I-197,I-198, I-200,I-208,I-211,I-217,I-218,I-219,I-219,I-220,I-223, I-225,I-228,I-229,I-232,I-236,I-239,I-240,I-241,I-244, I-245,I-247,II-2,IV-2,IV-15,IV-16,IV-17,IV-18,IV-19,IV-20, IV-21, IV-22,IV-23,IV-24,IV-25, IV-26, IV-27, IV-35, IV-38,IV-42,IV-46,IV-52,IV-53,IV-54,IV-56,IV-57,IV-60, IV-61,IV-72,IV-86,IV-87,VI-3,VII-2,VII-25

TEST EXAMPLE 7: Insecticidal test against brown rice plant hoppers (Nilaparvata lugens Stal)

A 5% emulsifiable concentrate of the compound of the present invention (depending upon the compound, a 25% emulsifiable concentrate was tested) was diluted with water containing a spreader to obtain a solution having a concentration of 500 ppm.
This solution was applied in a sufficient amount to the foliage of rice planted in a pot of 1/20,000 are. After drying it by air, a cylinder was put, and 10 larvae of 2 old of brown rice plant hoppers were released per pot. A cover was put, and the pot was stored in a constant temperature chamber. An inspection was carried out upon expiration of 6 days, and the mortality was obtained by the following calculation formula. The test was carried out in two sections.

$$\text{Mortality (\%)} = [\text{Number of dead larvae/(Number of dead larvae + alive larvae)}]\times 100$$

As a result, the following compounds showed a mortality of 70% or higher.
Compound Nos. of the present invention: I-50,I-184 TEST EXAMPLE 8: Insecticidal test against green rice leaf hoppers (Nephotellix cincticeps Vhler)
A rice foliage was dipped in an emulsified solution having a concentration of 500 ppm of the compound of the present invention, for about 10 seconds, and this foliage was put into a glass cylinder, and adults of green rice leaf hoppers showing resistance against organophosphorus insecticides, were released, and a perforated cover was put. The covered cylinder was put in a constant temperature chamber of 25°C. Six days later, the number of dead insects were investigated, and the mortality was obtained by the same calculation formula as in Test Example 7. The test was carried out in two sections. As a result, the following compounds showed a mortality of 70% or higher.
Compound Nos. of the present invention: I-10,I-16, I-37,I-45,I-47,I-50,I-52,I-54,I-55,I-68,I-72,I-83,I-89,I-91,I-109,I-113,I-137,I-160,I-162,I-166,I-168,I-197,II-5,IV-12

TEST EXAMPLE 9: Contact insecticidal test against diamond back moths (Plutella xylostella Linne)

A kohlrabi leaf was dipped in an emulsified aqueous solution having a concentration of 500 ppm of the compound of the present invention, for about 10 seconds, and after drying it by air, it was put in a petri dish, wherein 10 larvae of diamond back moths of 2 old were released. A perforated cover was put thereon, and the petri dish was put in a constant temperature chamber of 25°C. Six days later, the number of dead larvae was investigated, and the mortality was obtained by the same calculation formula as in Test Example 7. The test was carried out in two sections. As a result, the following compounds showed a mortality of 70% or higher.
Compound Nos. of the present invention: I-37,I-47, I-50,I-52,I-54,I-55,I-63,I-89,I-109,I-113,I-119,I-125, I-229, I-137, I- 2 0 3 , I-208, I-220

TEST EXAMPLE 10: Test on miticidal effects against two-spotted spider mite (Tetranychus ulticae Koch)

A bean leaf was cut into a circular shape having a diameter of 3.0 cm by means of a leaf punch and put on a wet filter paper on a styrol cup having a diameter of 7 cm. Ten larvae per leaf, of larvae of two-spotted spider mite, were inoculated thereto. A 5% emulsifiable concentrate of the compound of the present invention as described in the specification (depending upon the compound, a 25% emulsifiable concentrate was tested) was diluted with water containing a spreader, to obtain a solution having a concentration of 500 ppm. This solution was applied in an amount of 2 ml per styrol cut by means of a rotational applying column, and the cup was put in a constant temperature chamber of 25°C. Upon expiration of 96 hours, the mortality was obtained by the same calculation formula as in Test Example 7. The test was carried out in two sections. As a result, the following compounds showed a mortality of 70% or higher.

Compound Nos. of the present invention: I-37,I-45, I-47,I-50,I-52,I-54,I-55,I-64,I-95,I-105,I-109,I-111, I-113,I-115,I-117,I-125,I-126,I-127,I-128,I-129,I-131, I-133,I-137,I-139,I-161,I-162,I-164,I-165,I-166,I-167, I-168,I-169

INDUSTRIAL APPLICABILITY

These compounds of the present invention have excellent controlling effects against plant diseases and plant insect pests, and they are safe also against crop plants.

## Claims

**1.** A heterocyclic imino compound of the formula (1) and an agrochemically acceptable salt thereof:

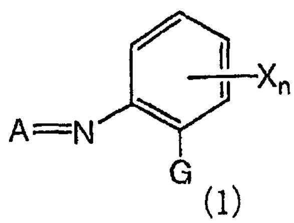

(1)

wherein G is a group selected from $G^1$ to $G^{14}$:

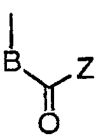

$G^1$

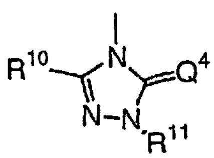

$G^2$

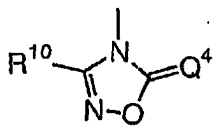

$G^3$

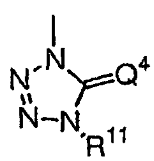

$G^4$.

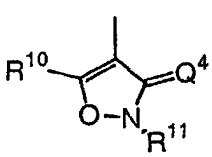

$G^5$

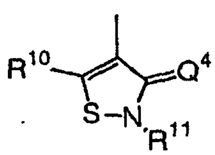

$G^6$.

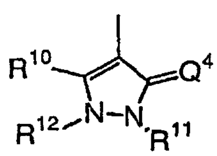

$G^7$

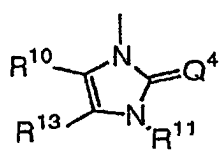

$G^8$

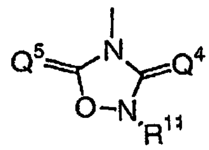
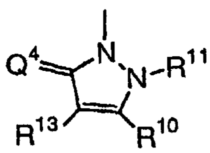

G9 G10

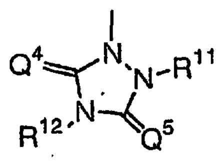
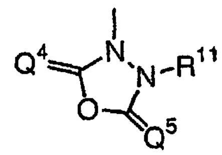
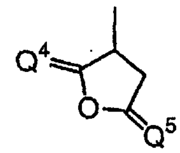
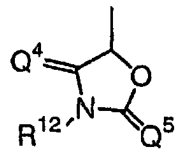

G11. G12. G13 G14

A is a 3- to 13-membered, mono-, di- or tri-cyclic ring which contains at least one hetero atom selected from among oxygen atoms, sulfur atoms and nitrogen atoms, which is composed of from 3 to 13 atoms arbitrarily selected from among carbon atoms, oxygen atoms, sulfur atoms and nitrogen atoms and which is substituted by from 0 to 13 Ys, provided that when A is a quinolone ring, the nitrogen atom in the quinolone ring is present at the α-position to the imino bond,

Z is $-OR^1$, $-SR^1$ or $-NR^2R^3$,

B is $-CH_2-$, $-C(=CH-OR^4)-$ or $-C(=N-OR^4)-$,

Y is $Y'-D-(CH_2)_p-$ or $=Q^1$ (provided that in the case of 2 or more Ys, they may be the same or different), or 2 Ys substituted on the same carbon atom of A, may, together with the carbon atom, form a 3- to 7-membered ring which may contain from 1 to 3 oxygen atoms, nitrogen atoms or sulfur atoms,

provided that when Y is a substituent on a carbon atom, Y may be a hydrogen atom,

D is a single bond, $-NR^5-$, $-C(=Q^2)-$, $-C(=Q^2)-C(=Q^3)-$, $-CR^6=N-$, $-N=C_R{}^6-$, $-CR^6=N-N=CR^6-$, $-N=CR^6-O-N=CR^6-$, $-CR^6=N-O-$, $-CR^6=N-O-CR^6=N-O-$, $-O-N=CR^6-CR^6=N-O-$, $-CR^6=N-NR^5-$ or $-O-N=CR^6-CR^6=N-NR^5-$,

$Q^1$, $Q^2$ and $Q^3$, each independently is =O, =S, $=N-R^7$ or $=C(R^8)(_R{}^9)$,

$Q^4$ and $Q^5$, each independently is =O or =S,

X is halogen, $C_1$-$C_6$ alkyl, $C_1$-$C_6$ haloalkyl, $C_1$-$C_6$ alkoxy, $C_1$-$C_6$ haloalkoxy, $C_1$-$C_6$ alkylthio, $C_1$-$C_6$ alkylamino, $(C_1$-$C_6$ alkyl$)_2$ amino, $NO_2$, CN, formyl, OH, SH, $NU^1U^2$, $C_1$-$C_6$ alkoxycarbonyl, $C_1$-$C_6$ alkylcarbonyl, $C_1$-$C_6$ haloalkylcarbonyl, phenylcarbonyl which may be substituted by $R^a$, or $C_1$-$C_6$ alkylcarbonyloxy (provided that in the case of two or more Xs substituted, they may be the same or different),

$R^1$, $R^2$ and $R^4$, each independently is a hydrogen atom, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_1$-$C_6$ alkylsulfenyl $C_1$-$C_6$ alkyl, phenyl $C_1$-$C_6$ alkyl which may be substituted by $R^a$, or heteroaryl $C_1$-$C_6$ alkyl which may be substituted by $R^a$,

$R^3$ is a hydrogen atom, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_1$-$C_6$ alkylsulfenyl $C_1$-$C_6$ alkyl, phenyl which may be substituted by $R^a$, phenyl $C_1$-$C_6$ alkyl which may be substituted by $R^a$, or heteroaryl $C_1$-$C_6$ alkyl which may be substituted by $R^a$,

$R^5$ and $R^6$, each independently is halogen, $C_1$-$C_6$ alkyl, $C_1$-$C_6$ haloalkyl, $C_3$-$C_6$ cycloalkyl, $C_1$-$C_6$ alkoxy, $C_1$-$C_6$ alkoxy $C_1$-$C_6$ alkyl, $C_1$-$C_6$ alkylsulfenyl $C_1$-$C_6$ alkyl, $C_1$-$C_6$ haloalkoxy, $C_1$-$C_6$ alkylsulfenyl, $C_1$-$C_6$ alkylsulfinyl, $C_1$-$C_6$ alkylsulfonyl, $C_1$-$C_6$ haloalkylsulfenyl, $C_1$-$C_6$ haloalkylsulfinyl, $C_1$-$C_6$ haloalkylsulfonyl, $C_2$-$C_6$ alkenyl, $C_2$-$C_6$ haloalkenyl, $C_2$-$C_6$ alkenyloxy, $C_2$-$C_6$ haloalkenyloxy, $C_2$-$C_6$ alkenylsulfenyl, $C_2$-$C_6$ alkenylsulfinyl, $C_2$-$C_6$ alkenylsulfonyl, $C_2$-$C_6$ haloalkenylsulfenyl, $C_2$-$C_6$ haloalkenylsulfinyl, $C_2$-$C_6$ haloalkenylsulfonyl, $C_2$-$C_6$ alkynyl, $C_2$-$C_6$ haloalkynyl, $C_2$-$C_6$ alkynyloxy, $C_2$-$C_6$ haloalkynyloxy, $C_2$-$C_6$ alkynylsulfenyl, $C_2$-$C_6$ alkynylsulfinyl, $C_2$-$C_6$ alkylsulfonyl, $C_2$-$C_6$ haloalkynylsulfenyl, $C_2$-$C_6$ haloalkynylsulfinyl, $C_2$-$C_6$ haloalkynylsulfonyl, $NO_2$, CN, formyl, OH, SH, SCN, $C_1$-$C_6$ alkoxycarbonyl, $C_1$-$C_6$ haloalkoxycarbonyl, $C_1$-$C_6$ alkylcarbonyl, $C_1$-$C_6$ haloalkylcarbonyl, $C_1$-$C_6$ alkylcarbonyloxy, phenyl which may be substituted by $R^a$, phenyl $C_1$-$C_6$ alkyl which may be substituted by $R^a$, phenylsulfonyl which may be substituted $R^a$, phenyl $C_1$-$C_6$ alkylsulfonyl which may be substituted by $R^a$, heteroaryl which may be substituted by $R^a$, heteroaryl $C_1$-$C_6$ alkyl which may be substituted by $R^a$, heteroarylsulfonyl which may be substituted by $R^a$, phenylcarbonyl which may be substituted by $R^a$, phenyl $C_1$-$C_6$ alkylcarbonyl which may be substituted by $R^a$, heteroarylcarbonyl which may be substituted by $R^a$, or $-NU^1U^2$, provided that $R^6$ may be a

hydrogen atom,

$R^7$ is hydrogen atom, $C_1$-$C_6$ alkyl, $C_1$-$C_6$ haloalkyl, $C_3$-$C_6$ cycloalkyl, $C_1$-$C_6$ alkoxy, $C_1$-$C_6$ alkoxy $C_1$-$C_6$ alkyl, $C_1$-$C_6$ alkylsulfenyl $C_1$-$C_6$ alkyl, $C_1$-$C_6$ alkylsulfonyl, $C_1$-$C_6$ haloalkylsulfonyl, $C_1$-$C_6$ alkylcarbonyl, $C_1$-$C_6$ haloalkylcarbonyl, phenyl which may be substituted by $R^a$, phenoxy which may be substituted by $R^a$, phenyl $C_1$-$C_6$ alkyl which may be substituted by $R^a$, phenyl $C_1$-$C_6$ alkoxy which may be substituted by $R^a$, phenylsulfonyl which may be substituted by $R^a$, phenyl $C_1$-$C_6$ alkylsulfonyl which may be substituted by $R^a$, heteroaryl which may be substituted by $R^a$, heteroaryloxy which may be substituted by $R^a$, heteroaryl $C_1$-$C_6$ alkyl which may be substituted by $R^a$, heteroarylsulfonyl which may be substituted by $R^a$, phenylcarbonyl which may be substituted by $R^a$, phenoxycarbonyl which may be substituted by $R^a$, phenyl $C_1$-$C_6$ alkylcarbonyl which may be substituted by $R^a$, heteroarylcarbonyl which may be substituted by $R^a$, heteroaryloxycarbonyl which may be substituted by $R^a$, or heteroaryl $C_1$-$C_6$ alkylcarbonyl which may be substituted by $R^a$,

$R^8$ and $R^9$, each independently is a hydrogen atom, halogen, $C_1$-$C_6$ alkyl, $C_1$-$C_6$ alkoxy, $C_1$-$C_6$ alkylsulfenyl, $C_2$-$C_6$ alkenyl, $NO_2$, CN, formyl, or $C_1$-$C_6$ alkoxycarbonyl,

$R^{10}$ is a hydrogen atom, halogen, $R^{14}$, $-OR^{14}$, $-SR^{14}$, $-SOR^{14}$, or $-SO_2R^{14}$,

$R^{11}$ is a hydrogen atom, $R^{14}$ or CN,

$R^{12}$ is a hydrogen atom or $R^{14}$,

$R^{13}$ is a hydrogen atom, halogen, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_2$-$C_6$ alkenyl, or $C_2$-$C_6$ alkynyl,

$R^{14}$ is $C_1$-$C_6$ alkyl, $C_1$-$C_6$ haloalkyl, $C_2$-$C_6$ alkenyl, $C_2$-$C_6$ haloalkenyl, $C_2$-$C_6$ alkynyl, $C_2$-$C_6$ haloalkynyl, $C_3$-$C_6$ cycloalkyl, $C_1$-$C_6$ alkylcarbonyl, or $C_1$-$C_6$ alkoxycarbonyl,

Y' is halogen, $C_1$-$C_{12}$ alkyl which may be substituted by $R^b$, $C_3$-$C_6$ cycloalkyl which may be substituted by $R^b$, $C_2$-$C_{12}$ alkenyl which may be substituted by $R^b$, $C_2$-$C_{12}$ alkynyl which may be substituted by $R^b$, $C_1$-$C_{12}$ alkoxy which may be substituted by $R^b$, $C_1$-$C_6$ alkoxy $C_1$-$C_6$ alkoxy which may be substituted by $R^b$, $C_2$-$C_6$ alkenyloxy which may be substituted by $R^b$, $C_2$-$C_6$ alkynyloxy which may be substituted by $R^b$, $C_1$-$C_6$ alkylsulfenyl which may be substituted by $R^b$, $C_2$-$C_6$ alkenylsulfenyl which may be substituted by $R^b$, $C_2$-$C_6$ alkynylsulfenyl which may be substituted by $R^b$, $C_1$-$C_6$ alkylsulfinyl which may be substituted by $R^b$, $C_2$-$C_6$ alkenylsulfinyl which may be substituted by $R^b$, $C_2$-$C_6$ alkynylsulfinyl which may be substituted by $R^b$, $C_1$-$C_6$ alkylsulfonyl which may be substituted by $R^b$, $C_2$-$C_6$ alkenylsulfonyl which may be substituted by $R^b$, $C_2$-$C_6$ alkynylsulfonyl which may be substituted by $R^b$, $C_1$-$C_6$ alkoxycarbonyl which may be substituted by $R^b$, $C_1$-$C_6$ alkylcarbonyl which may be substituted by $R^b$, $C_1$-$C_6$ alkylcarbonyloxy which may be substituted by $R^b$, phenyl which may be substituted by $R^c$, phenoxy which may be substituted by $R^c$, phenyl $C_1$-$C_6$ alkyl which may be substituted by $R^c$, phenyl $C_1$-$C_6$ alkoxy which may be substituted by $R^c$, phenylsulfonyl which may be substituted by $R^c$, phenylsulfinyl which may be substituted by $R^c$, phenylsulfenyl which may be substituted by $R^c$, phenyl $C_1$-$C_6$ alkylsulfenyl which may be substituted by $R^c$, phenyl $C_1$-$C_6$ alkylsulfinyl which may be substituted by $R^c$, phenyl $C_1$-$C_6$ alkylsulfonyl which may be substituted by $R^c$, heteroaryl which may be substituted by $R^c$, heteroaryloxy which may be substituted by $R^c$, heteroaryl $C_1$-$C_6$ alkyl which may be substituted by $R^c$, heteroaryl $C_1$-$C_6$ alkoxy which may be substituted by $R^c$, heteroarylsulfinyl which may be substituted by $R^c$, heteroarylsulfenyl which may be substituted by $R^c$, heteroarylsulfonyl which may be substituted by $R^c$, heteroaryl $C_1$-$C_6$ alkylsulfenyl which may be substituted by $R^c$, heteroaryl $C_1$-$C_6$ alkylsulfinyl which may be substituted by $R^c$, heteroaryl $C_1$-$C_6$ alkylsulfonyl which may be substituted by $R^c$, phenylcarbonyl which may be substituted by $R^c$, phenylcarbonylxoy which may be substituted by $R^c$, phenoxycarbonyl which may be substituted by $R^c$, phenyl $C_1$-$C_6$ alkylcarbonyl which may be substituted by $R^c$, phenyl $C_1$-$C_6$ alkylcarbonyloxy which may be substituted by $R^c$, heteroarylcarbonyl which may be substituted by $R^c$, heteroarylcarbonyloxy which may be substituted by $R^c$, heteroaryloxycarbonyl which may be substituted by $R^c$, heteroaryl $C_1$-$C_6$ alkylcarbonyl which may be substituted by $R^c$, heteroaryl $C_1$-$C_6$ alkylcarbonyloxy which may be substituted by $R^c$, $NO_2$, CN, formyl, or naphthyl,

$R^a$ is halogen, $C_1$-$C_6$ alkyl, $C_1$-$C_6$ haloalkyl, $C_3$-$C_6$ cycloalkyl, $C_1$-$C_6$ alkoxy, $C_1$-$C_6$ alkoxy $C_1$-$C_6$ alkyl, $C_1$-$C_6$ alkylsulfenyl $C_1$-$C_6$ alkyl, $C_1$-$C_6$ haloalkoxy, $C_1$-$C_6$ alkylsulfenyl, $C_1$-$C_6$ alkylsulfinyl, $C_1$-$C_6$ alkylsulfonyl, $C_1$-$C_6$ haloalkylsulfenyl, $C_1$-$C_6$ haloalkylsulfinyl, $C_1$-$C_6$ haloalkylsulfonyl, $C_2$-$C_6$ alkenyl, $C_2$-$C_6$ haloalkenyl, $C_2$-$C_6$ alkenyloxy, $C_2$-$C_6$ haloalkenyloxy, $C_2$-$C_6$ alkenylsulfenyl, $C_2$-$C_6$ alkenylsulfinyl, $C_2$-$C_6$ alkenylsulfonyl, $C_2$-$C_6$ haloalkenylsulfenyl, $C_2$-$C_6$ haloalkenylsulfinyl, $C_2$-$C_6$ haloalkenylsulfonyl, $C_2$-$C_6$ alkynyl, $C_2$-$C_6$ haloalkynyl, $C_2$-$C_6$ alkynyloxy, $C_2$-$C_6$ haloalkynyloxy, $C_2$-$C_6$ alkynylsulfenyl, $C_2$-$C_6$ alkynylsulfinyl, $C_2$-$C_6$ alkynylsulfonyl, $C_2$-$C_6$ haloalkynylsulfenyl, $C_2$-$C_6$ haloalkynylsulfinyl, $C_2$-$C_6$ haloalkynylsulfonyl, $NO_2$, CN, formyl, SH, OH, SCN, $C_1$-$C_6$ alkoxycarbonyl, $C_1$-$C_6$ alkylcarbonyl, $C_1$-$C_6$ haloalkylcarbonyl, $C_1$-$C_6$ alkylcarbonyloxy, phenyl, or $-NU^1U^2$, the number of $R^a$ for substitution being from 1 to 5 (provided that in the case of two or more $R^a$, they may be the same or different),

$R^b$ is halogen, $C_3$-$C_6$ cycloalkyl, $C_1$-$C_6$ alkoxy, $C_1$-$C_6$ alkoxy $C_1$-$C_6$ alkoxy, $C_1$-$C_6$ alkylsulfenyl $C_1$-$C_6$ alkoxy, $C_1$-$C_6$ haloalkoxy, $C_1$-$C_6$ alkylsulfenyl, $C_1$-$C_6$ alkylsulfinyl, $C_1$-$C_6$ alkylsulfonyl, $C_1$-$C_6$ haloalkylsulfenyl, $C_1$-$C_6$ haloalkylsulfinyl, $C_1$-$C_6$ haloalkylsulfonyl, $C_2$-$C_6$ alkenyloxy, $C_2$-$C_6$ haloalkenyloxy, $C_2$-$C_6$ alkenylsulfenyl, $C_2$-$C_6$ alkenylsulfinyl, $C_2$-$C_6$ alkenylsulfonyl, $C_2$-$C_6$ haloalkenylsulfenyl, $C_2$-$C_6$ haloalkenylsulfinyl, $C_2$-$C_6$ haloalkenylsul-

fonyl, $C_2$-$C_6$ alkynyloxy, $C_2$-$C_6$ haloalkynyloxy, $C_2$-$C_6$ alkynylsulfenyl, $C_2$-$C_6$ alkynylsulfinyl, $C_2$-$C_6$ alkynylsulfonyl, $C_2$-$C_6$ haloalkynylsulfenyl, $C_2$-$C_6$ haloalkynylsulfinyl, $C_2$-$C_6$ haloalkynylsulfonyl, $NO_2$, CN, formyl, OH, SH, SCN, $C_1$-$C_6$ alkoxycarbonyl, $C_1$-$C_6$ alkylcarbonyl, $C_1$-$C_6$ haloalkylcarbonyl, $C_1$-$C_6$ alkylcarbonyloxy, phenyl which may be substituted by $R^a$, phenoxy which may be substituted by $R^a$, phenyl $C_1$-$C_6$ alkoxy which may be substituted by $R^a$, phenylsulfonyl which may be substituted by $R^a$, phenyl $C_1$-$C_6$ alkylsulfonyl which may be substituted by $R^a$, heteroaryl which may be substituted by $R^a$, heteroaryloxy which may be substituted by $R^a$, heteroarylsulfonyl which may be substituted by $R^a$, phenylcarbonyl which may be substituted by $R^a$, phenoxycarbonyl which may be substituted by $R^a$, phenyl $C_1$-$C_6$ alkylcarbonyl which may be substituted by $R^a$, heteroarylcarbonyl which may be substituted by $R^a$, heteroaryloxycarbonyl which may be substituted by $R^a$, or heteroaryl $C_1$-$C_6$ alkylcarbonyl which may be substituted by $R^a$, or -$NU^1U^2$, or a 3- to 7-membered ring which may contain from 1 to 4 hetero atoms selected from among oxygen atoms, nitrogen atoms and sulfur atoms, the number of $R^b$ for substitution being from 1 to 8 (provided that in the case of two or more $R^b$, they may be the same or different),

$R^c$ is halogen, $C_1$-$C_{12}$ alkyl which may be substituted by $R^b$, $C_3$-$C_6$ cycloalkyl which may be substituted by $R^b$, $C_2$-$C_{12}$ alkenyl which may be substituted by $R^b$, $C_2$-$C_{12}$ alkynyl which may be substituted by $R^b$, $C_1$-$C_{12}$ alkoxy which may be substituted by $R^b$, $C_1$-$C_6$ alkoxy $C_1$-$C_6$ alkoxy which may be substituted by $R^b$, $C_2$-$C_6$ alkenyloxy which may be substituted by $R^b$, $C_2$-$C_6$ alkynyloxy which may be substituted by $R^b$, $C_1$-$C_6$ alkylsulfenyl which may be substituted by $R^b$, $C_2$-$C_6$ alkenylsulfenyl which may be substituted by $R^b$, $C_2$-$C_6$ alkynylsulfenyl which may be substituted by $R^b$, $C_1$-$C_6$ alkylsulfinyl which may be substituted by $R^b$, $C_2$-$C_6$ alkenylsulfinyl which may be substituted by $R^b$, $C_2$-$C_6$ alkynylsulfinyl which may be substituted by $R^b$, $C_1$-$C_6$ alkylsulfonyl which may be substituted by $R^b$, $C_2$-$C_6$ alkenylsulfonyl which may be substituted by $R^b$, $C_2$-$C_6$ alkynylsulfonyl which may be substituted by $R^b$, $C_1$-$C_6$ alkoxycarbonyl which may be substituted by $R^b$, $C_1$-$C_6$ alkylcarbonyl which may be substituted by $R^b$, $C_1$-$C_6$ alkylcarbonyloxy which may be substituted by $R^b$, $NO_2$, CN, formyl, OH, SH, SCN, $C_1$-$C_6$ alkoxycarbonyl, $C_1$-$C_6$ alkylcarbonyl, $C_1$-$C_6$ haloalkylcarbonyl, $C_1$-$C_6$ alkylcarbonyloxy, phenyl which may be substituted by $R^a$, phenoxy which may be substituted by $R^a$, phenyl $C_1$-$C_6$ alkyl which may be substituted by $R^a$, phenyl $C_1$-$C_6$ alkoxy which may be substituted by $R^a$, phenylsulfonyl which may be substituted by $R^a$, phenylsulfinyl which may be substituted by $R^a$, phenylsulfenyl which may be substituted by $R^a$, phenyl $C_1$-$C_6$ alkylsulfenyl which may be substituted by $R^a$, phenyl $C_1$-$C_6$ alkylsulfinyl which may be substituted by $R^a$, phenyl $C_1$-$C_6$ alkylsulfonyl which may be substituted by $R^a$, heteroaryl which may be substituted by $R^a$, heteroaryloxy which may be substituted by $R^a$, heteroaryl $C_1$-$C_6$ alkyl which may be substituted by $R^a$, heteroaryl $C_1$-$C_6$ alkoxy which may be substituted by $R^a$, heteroarylsulfinyl which may be substituted by $R^a$, heteroarylsulfenyl which may be substituted by $R^a$, heteroarylsulfonyl which may be substituted by $R^a$, heteroaryl $C_1$-$C_6$ alkylsulfenyl which may be substituted by $R^a$, heteroaryl $C_1$-$C_6$ alkylsulfinyl which may be substituted by $R^a$, heteroaryl $C_1$-$C_6$ alkylsulfonyl which may be substituted by $R^a$, phenylcarbonyl which may be substituted by $R^a$, phenylcarbonyloxy which may be substituted by $R^a$, phenoxycarbonyl which may be substituted by $R^a$, phenyl $C_1$-$C_6$ alkylcarbonyl which may be substituted by $R^a$, phenyl $C_1$-$C_6$ alkylcarbonyloxy which may be substituted by $R^a$, heteroarylcarbonyl which may be substituted by $R^a$, heteroarylcarbonyloxy which may be substituted by $R^a$, heteroaryloxycarbonyl which may be substituted by $R^a$, heteroaryl $C_1$-$C_6$ alkylcarbonyl which may be substituted by $R^a$, heteroaryl $C_1$-$C_6$ alkylcarbonyloxy which may be substituted by $R^a$, or -$NU^1U^2$, the number of $R^c$ for substitution being from 1 to 5 (provided that in the case of two or more $R^c$, they may be the same or different),

$U^1$ and $U^{2'}$ each independently is a hydrogen atom, $C_1$-$C_6$ alkyl, $C_1$-$C_6$ haloalkyl, $C_3$-$C_6$ cycloalkyl, $C_1$-$C_6$ alkoxy $C_1$-$C_6$ alkyl, $C_1$-$C_6$ alkylsulfenyl $C_1$-$C_6$ alkyl, formyl, $C_1$-$C_6$ alkylsulfonyl, $C_1$-$C_6$ haloalkylsulfonyl, $C_1$-$C_6$ alkoxycarbonyl, $C_1$-$C_6$ alkylcarbonyl, or $C_1$-$C_6$ haloalkylcarbonyl, or $U^1$ and $U^2$ together form a 3- to 7-membered ring which may contain from 1 to 4 hetero atoms selected from among oxygen atoms, nitrogen atoms and sulfur atoms,

n represents the number of substituents and is from 0 to 4, and

p represents the number of repeating units and is from 0 to 2.

2. A hydrochloride, a hydrobromide, a hydroiodide, a formate, an acetate or an oxalate of the heterocyclic imino compound according to Claim 1.

3. The heterocyclic imino compound and an agrochemically acceptable salt thereof, according to Claim 1, wherein A is

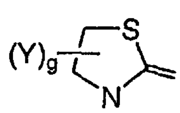 , 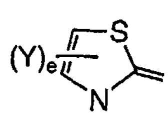 , 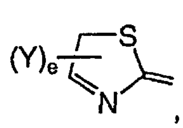 , 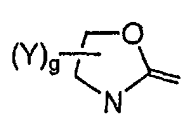 , 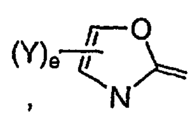 ,

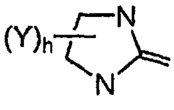 , 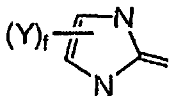 , 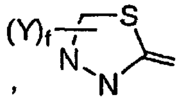 , 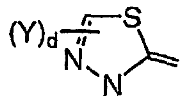 , 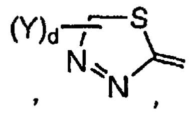 ,

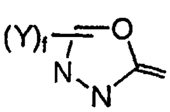 , 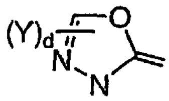 , 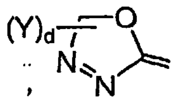 , 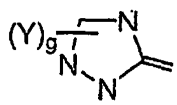 , 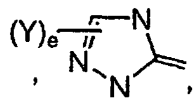 ,

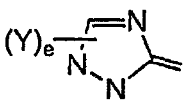 , 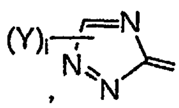 , 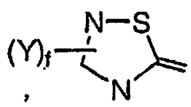 , 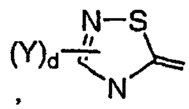 , 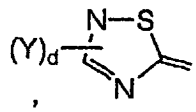 ,

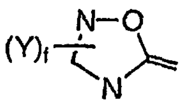 , 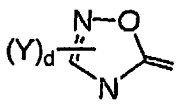 , 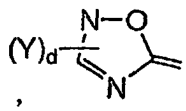 , 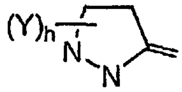 , 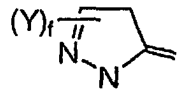 ,

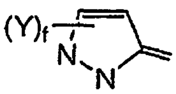 , 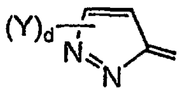 , 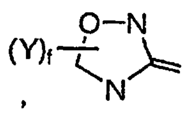 , 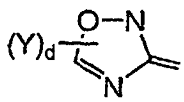 , 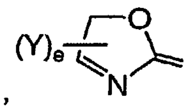 ,

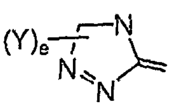 , 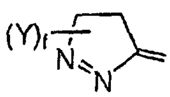 , 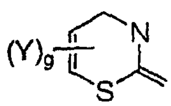 , 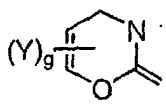 , 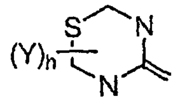 ,

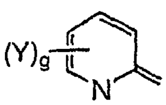 , 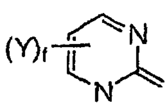 , 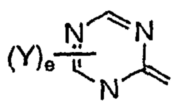 , 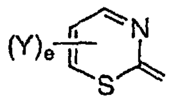 , 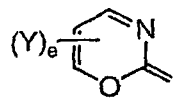 ,

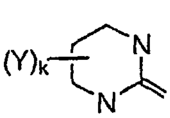 , 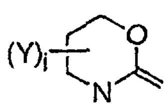 , 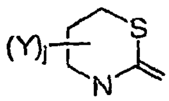 , 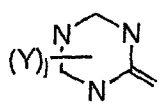 , 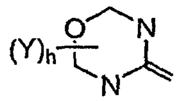 ,

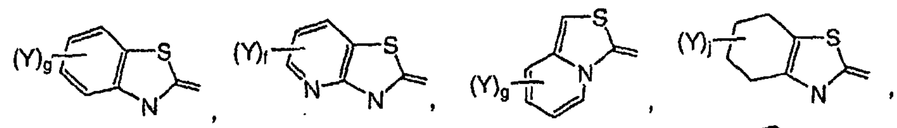

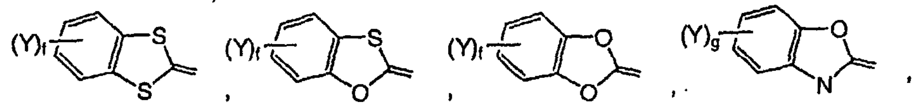

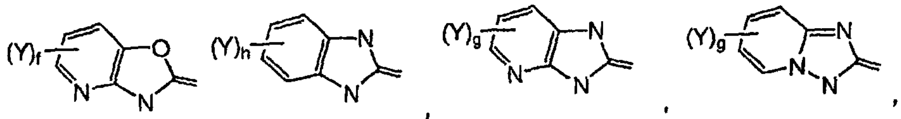

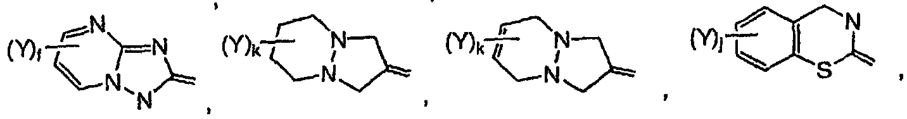

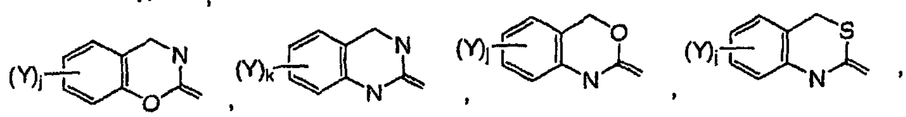

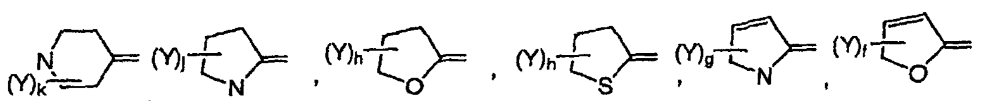

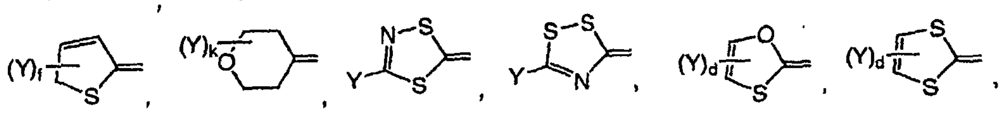

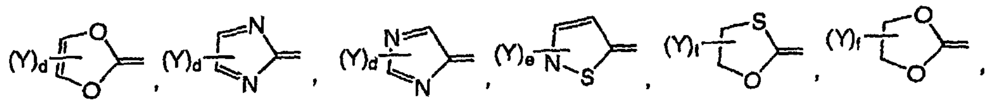

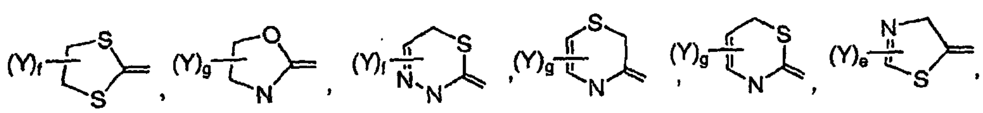

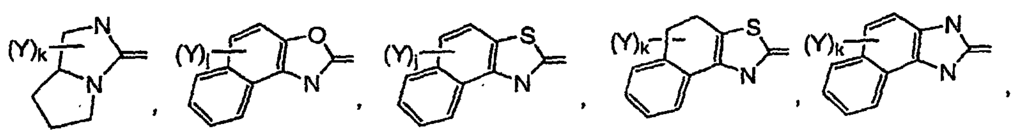

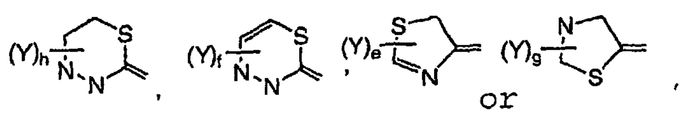

or

d represents the number of substituents and is from 0 to 2,
e represents the number of substituents and is from 0 to 3,
f represents the number of substituents and is from 0 to 4,
g represents the number of substituents and is from 0 to 5,
h represents the number of substituents and is from 0 to 6,
i represents the number of substituents and is from 0 to 1,
j represents the number of substituents and is from 0 to 7, and
k represents the number of substituents and is from 0 to 8.

**4.** The heterocyclic imino compound according to any one of Claims 1 to 3, wherein G is $G^1$.

**5.** An agricultural chemical containing at least one member selected from the heterocyclic imino compound and an agrochemically acceptable salt thereof according to any one of Claims 1 to 4, as an active ingredient.

**6.** A fungicide containing at least one member selected from the heterocyclic imino compound and an agrochemically acceptable salt thereof according to any one of Claims 1 to 4, as an active ingredient.

**7.** An insecticide containing at least one member selected from the heterocyclic imino compound and an agrochemically acceptable salt thereof according to any one of Claims 1 to 4, as an active ingredient.

## INTERNATIONAL SEARCH REPORT

International application No.
PCT/JP00/09411

**A. CLASSIFICATION OF SUBJECT MATTER**

Int.Cl[7] C07D213/74, 277/18, 42, 60, 82, 263/48, 58, 233/50, 88, 271/10, 285/12, 327/04, 339/06, 279/06, 12, 239/46, 235/30, 487/04, 417/04, 285/10, A01N43/78, 28, 86//C07C335/32

According to International Patent Classification (IPC) or to both national classification and IPC

**B. FIELDS SEARCHED**

Minimum documentation searched (classification system followed by classification symbols)
Int.Cl[7] C07D213/00-74, 277/00-82, 263/00-58, 233/00-88, 271/00-10, 285/00-12, 327/00-04, 339/00-06, 279/00-12, 239/00-46, 235/00-30, 487/00-04, 417/00-04, 285/00-10, A01N43/00-86

Documentation searched other than minimum documentation to the extent that such documents are included in the fields searched

Electronic data base consulted during the international search (name of data base and, where practicable, search terms used)
REGISTRY(STN), CAPLUS(STN)

**C. DOCUMENTS CONSIDERED TO BE RELEVANT**

| Category* | Citation of document, with indication, where appropriate, of the relevant passages | Relevant to claim No. |
|---|---|---|
| X<br>A | MAEDA, R.; OHSUGI, E.; FUJIOKA, T.; HIROSE, K. Studies on the synthesis and analgesic and antiinflammatory activities of 2-thiazolylamino- and 2-thiazolyloxyarylaceticacid derivatives. Chem. Pharm. Bull., 1983, Vol.31, No.10, pp.3424-3445 especially, p.3436, compounds. IXj, IXl; p.3438, compound IXk etc. | 1-4<br>5-7 |
| X<br>A | WERBEL, L. M.; ELSLAGER, E. F.; PHILLIPS, A. A.; WORTH, D. F.; ISLIP, P. J.; NEVILLE, M. C. Synthetic amebicides. IX. 2-(Alkyl- and arylamino)-5-nitrothiazole derivatives with antiamebic, antitrichomonal, and antimalarial properties. J. Med. Chem., 1969, Vol.12, pp.521-524 especially, p.522, compound No. 22, etc. | 1-4<br>5-7 |
| X<br>A | JP, 7-53527, A (TOA WOOL SPINNING & WEAVING CO.), 28 February, 1995 (28.02.95), especially, page 4, example, etc. (Family: none) | 1-4<br>5-7 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 28 March, 2001 (28.03.01) | 17 April, 2001 (17.04.01) |
| Name and mailing address of the ISA/<br>Japanese Patent Office | Authorized officer |
| Facsimile No. | Telephone No. |

Form PCT/ISA/210 (second sheet) (July 1992)

**INTERNATIONAL SEARCH REPORT**

International application No.
PCT/JP00/09411

C (Continuation). DOCUMENTS CONSIDERED TO BE RELEVANT

| Category* | Citation of document, with indication, where appropriate, of the relevant passages | Relevant to claim No. |
|---|---|---|
| A | GB, 1258920, A (GEIGY, J. R., A.-G.), 30 December, 1971 (30.12.71), entire description & DE, 1816700, C3 & FR, 1601535, A | 1-4 |
| A | JP, 6-157478, A (Nissan Chemical Industries, Ltd.), 03 June, 1994 (03.06.94), entire description (Family: none) | 1-7 |

Form PCT/ISA/210 (continuation of second sheet) (July 1992)

**INTERNATIONAL SEARCH REPORT**

International application No.
PCT/JP00/09411

**Box I Observations where certain claims were found unsearchable (Continuation of item 1 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 1-7
because they relate to parts of the international application that do not comply with the prescribed requirements to such an extent that no meaningful international search can be carried out, specifically:
(See extra sheet.)

3. ☐ Claims Nos.:
because they are dependent claims and are not drafted in accordance with the second and third sentences of Rule 6.4(a).

**Box II Observations where unity of invention is lacking (Continuation of item 2 of first sheet)**

This International Searching Authority found multiple inventions in this international application, as follows:
(See extra sheet.)

1. ☐ As all required additional search fees were timely paid by the applicant, this international search report covers all searchable claims.

2. ☒ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1992)

**INTERNATIONAL SEARCH REPORT**

International application No.
PCT/JP00/09411

Continuation of Box No.I and II of continuation of first sheet (1)

Most parts of the structure of the general formula set forth in claim 1 are variable, and the compounds represented by the general formula have only N-phenylated heterocyclic imine structure, which is a publicly known chemical structure in itself, as the common partial chemical structure.

Accordingly, an invention of unified chemical substances cannot be grasped from such disclosure of claim 1, so that the invention of claim 1 does not comply with the requirement of unity of invention. Further, claim 1 includes too wide a range of compounds to make worthwhile search of prior literature for the whole range of compounds.

In this International Search Report, therefore, prior art search has been made in the light of the disclosure of the description only for compounds of the general formula wherein A is attached to the imino nitrogen at a carbon atom thereof and is

1) a five-membered ring having heteroatoms at the positions adjacent to the carbon atom attached to the imino nitrogen with the other ring-constituting atoms being carbon atoms linked by a double bond, or a fused ring containing the five-membered ring,

2) a six-membered unsaturated heterocycle having a nitrogen atom at the α-position to the carbon atom attached to the imino nitrogen, or a fused ring containing the six-membered unsaturated heterocycle, or

3) a heterocycle not causing the imino group to form tautomers.

The same applies to claims 2 to 7.

Form PCT/ISA/210 (extra sheet) (July 1992)